# EUROPEAN PATENT SPECIFICATION

(11) **EP 3 965 824 B1**
(45) Date of publication and mention of the grant of the patent: **08.01.2025**
(21) Application number: 20802303.6
(22) Date of filing: 05.05.2020
(51) Int. Cl.: C07D 209/34, A61K 47/55, C07D 401/04, C07D 403/14

(54) **HETEROBIFUNCTIONAL COMPOUNDS AS DEGRADERS OF HPK1**
HETEROBIFUNKTIONELLE VERBINDUNGEN ALS DEGRADER VON HPK1
COMPOSÉS HÉTÉROBIFONCTIONNELS EN TANT QU'AGENTS DE DÉGRADATION DE HPK1

(30) Priority: 06.05.2019 US 201962843816 P
(43) Date of publication of application: 16.03.2022
(73) Proprietor: Icahn School of Medicine at Mount Sinai, New York, NY 10029 (US)
(72) Inventor: JIN, Jian, New York, New York 10029 (US); BURAKOFF, Steven, New York, New York 10029 (US); KANISKAN, H. Umit, New York, New York 10029 (US); SAWASDIKOSOL, Sansana, New York, New York 10029 (US); CHEN, He, New York, New York 10029 (US); BRODY, Joshua, New York, New York 10029 (US); BHARDWAJ, Nina, New York, New York 10029 (US)
(74) Representative: Grünecker Patent- und Rechtsanwälte PartG mbB
(86) International application number: PCT/US2020/031527
(87) International publication number: WO 2020/227325

(56) References cited:
- WO-A1-2017/197055
- WO-A1-2018/049200
- WO-A1-2018/106870
- WO-A1-2019/084030
- US-A1- 2004 063 773
- US-A1- 2018 072 741

## Description

### TECHNICAL FIELD

This disclosure relates to bivalent compounds (e.g., heterobifunctional compounds) which degrade and/or disrupt Hematopoietic Progenitor Kinase 1 (HPK1), compositions comprising one or more of the bivalent compounds, and these bivalent compounds and compositions for use in methods of treatment of HPK1 -mediated diseases in a subject in need thereof. The disclosure also relates to methods for designing such bivalent compounds.

### BACKGROUND OF THE INVENTION

Unlike traditional enzyme inhibitors, which only inhibit the catalytic activity of the target enzyme, heterobifunctional compounds, also known as proteolysis-targeted chimeras (PROTACs), bind and induce degradation of the enzyme, thus eliminating potential scaffolding functions of the protein, in addition to inhibiting its enzymatic activity (Buckley and Crews, 2014). The HPK1 degraders disclosed herein offer a novel mechanism for treating HPK1-mediated diseases. Additionally, the ability of the degraders to target HPK1 for degradation, as opposed to inhibiting the catalytic activity of HPK1, is expected to overcome resistance, regardless of whether due to the drugs used in prior treatments or whether acquired resistance was caused by gene mutation, amplification or otherwise.

Lewis Thomas and Frank Macfarlane Burnet are the first to introduce the concept that the immune system constantly surveil the host for the emergence of nascent cancer cells and eliminate them before they become tumors (Burnet, 1970). While several lines of evidence suggested that our immune system could accomplish such task (Corthay, 2014), the most direct support of such concept comes from the development of immuno-oncological drugs that target the inhibitory molecules that hinder the anti-tumor immunity effort, allowing immune system to vigorously engage and eliminate previously difficult to treat cancers (Ribas and Wolchok, 2018). The success of the immune checkpoint inhibitor approach provides the roadmap as to how the exhausted immune systems could be provoked to re-engage the cancer cells. This theoretical framework spurs the search for novel immune checkpoint receptors that could serve as novel immune checkpoint targets.

Hematopoietic Progenitor Kinase 1 (HPK1, also known as MAP4K1), an intracellular negative regulator of the T cell antigen receptor (TCR) signal transduction pathways is a member of the KHS subfamily of Ste20 serine/threonine kinases (Hu et al., 1996; Kiefer et al., 1996). It is a multimodular-domain protein comprising proline-rich motifs and a C-terminal citron homology domain, in addition to its kinase domain. These additional domains suggest that HPK1 could perform functions other than its kinase function. HPK1 transcripts are detected in all embryonic tissues examined, but its expression profile shifts to a hematopoietic cell-restricted pattern post-partum at neonatal day 1 (Kiefer et al., 1996), leading to the speculation that HPK1 may perform a specialized function in hematopoietic cells. This cytosolic Ste20 kinase is recruited to the TCR complex (Ling et al., 2001) and its kinase activity is induced upon the engagement of the TCR (Liou et al., 2000). Overexpression of HPK1 suppresses TCR-induced activation of AP-1-dependent gene transcription in a kinase dependent manner, suggesting that the kinase activity of HPK1 is required to inhibit the Erk MAPK pathway (Liou et al., 2000). This blockage of the Erk MAPK pathway is thought to be the inhibitory mechanism that negatively regulates TCR-induced IL-2 gene transcription.

WO 2018/049200 A1 describes pyrazolopyridine derivatives as HPK1 modulators and their uses for the treatment of cancer.

WO 2019/084030 A1 describes bifunctional compounds, which can be used as modulators of targeted ubiquitination.

WO 2018/106870 A1 describes methods for designing heterobifunctional small molecules which selectively degrade/disrupt CDK4/6 and compositions and methods of using such degraders/disruptors to treat CDK4/6-mediated cancer.

US 2004/063773 A1 describes pyrrole substituted 2-indolinone compounds and their pharmaceutically acceptable salts which modulate the activity of protein kinases.

US 2018/072741 A1 describes pyrazolopyrimidine compounds for inhibiting HPK1 activity and pharmaceutical compositions comprising these compounds and the use of these compounds in treating, preventing or ameliorating diseases or disorders associated with HPK1 activity such as cancer.

WO 2017/197055 A1 describes heterocyclic compounds that bind to E3 Ubiquitin Ligase, which can be linked to a Targeting Ligand for a selected Target Protein for therapeutic purposes and methods of use and compositions thereof.

### SUMMARY OF THE INVENTION

The invention is defined in the claims.

Any references in the description to methods of treatment are to be understood to refer to the respective compounds and compositions for use in those methods.

The present disclosure relates generally to bivalent compounds (e.g., bi-functional compounds) which degrade and/or disrupt HPK1 and to these bivalent compounds for use in methods of treatment of HPK1-mediated diseases (i.e., a disease which depends on HPK1; overexpresses HPK1; depends on HPK1 activity; or includes elevated levels of HPK1 activity relative to a wild-type tissue of the same species and tissue type). It is important to note, because the HPK1 degraders/disruptors have dual functions (enzyme inhibition plus protein degradation/disruption), the bivalent compounds of the present disclosure can be significantly more effective therapeutic agents than currently available HPK1 inhibitors, which inhibit the enzymatic activity of HPK1, but do not affect HPK1 protein levels. The present disclosure further provides methods for identifying HPK1 degraders/disruptors as described herein.

More specifically, the present disclosure provides a bivalent compound including a HPK1 ligand conjugated to a degradation/disruption tag.

The HPK1 degraders/disruptors of the present invention have the form **"PI-linker-EL"**, as shown below: 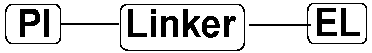 wherein **PI** (protein of interest) comprises an HPK1 ligand (*e.g.*, an HPK1 inhibitor) and **EL** (E3 ligase) comprises a degradation/disruption tag (*e.g.*, E3 ligase ligand). The HPK1 ligands (**PI**), degradation/disruption tags (**EL**), and linkers (**Linker**) of the present invention are illustrated below.

### HPK1 Ligands

The HPK1 ligand (**PI**) of the present invention is a moiety selected from the group consisting of:
(**I**) a moiety according to FORMULA 3L: 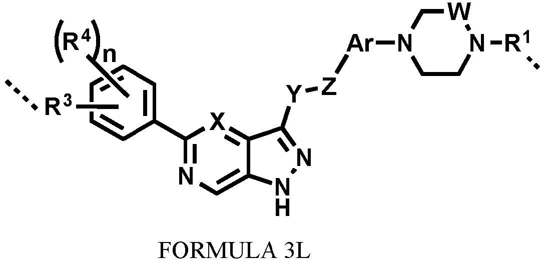 wherein
   the "Linker" moiety of the bivalent compound is attached independently to R¹ or R³;
   X is selected from CR² or N;
   R¹ and R³ are independently selected from null, hydrogen, C(O)R⁶, C(O)OR⁶, C(O)NR⁶R⁷, S(O)R⁶, S(O)₂R⁶, S(O)₂NR⁶R⁷, NR⁸C(O)OR⁶, NR⁸C(O)R⁶, NR⁸C(O)NR⁶R⁷, NR⁸S(O)R⁶, NR⁸S(O)₂R⁶, NR⁸S(O)₂NR⁶R⁷ , optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
   R⁶ is null, or a bivalent moiety selected from optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl;
      R⁷, and R⁸ are independently selected from hydrogen, optionally substituted C₁-C₈ alkyl, optionally substituted C₁-C₈alkoxyC₁-C₈alkyl, optionally substituted C₁-C₈alkylaminoC₁-Csalkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
      R⁶ and R⁷ together with the atom to which they are connected form an optionally substituted 3-20 membered cycloalkyl or heterocyclyl ring;
   R² is independently selected from hydrogen, halogen, oxo, CN, NO₂, OR⁹, SR⁹, NR⁹R¹⁰, C(O)R⁹, C(O)OR⁹, C(O)NR⁹R¹⁰, S(O)R⁹, S(O)₂R⁹, S(O)₂NR⁹R¹⁰, NR¹¹C(O)OR⁹, NR¹¹C(O)R⁹, NR¹¹C(O)NR⁹R¹⁰, NR¹¹S(O)R⁹, NRⁿS(O)₂R⁹, NR¹¹S(O)₂NR⁹R¹⁰, optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
      R⁹, R¹⁰, and R¹¹ are independently selected from hydrogen, optionally substituted C₁-C₈ alkyl, optionally substituted C₁-C₈alkoxyC₁-C₈alkyl, optionally substituted C₁-C₈alkylaminoC₁-Csalkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
      R⁹ and R¹⁰, R⁹ and R¹¹, R¹⁰ and R¹¹ together with the atom to which they are connected form an optionally substituted 3-20 membered cycloalkyl or heterocyclyl ring;
   each R⁴ is independently selected from null, hydrogen, halogen, oxo, CN, NO₂, OR¹⁸, SR¹⁸, NR¹⁸R¹⁹, OCOR¹⁸, OCO₂R¹⁸, OCONR¹⁸R¹⁹, COR¹⁸, CO₂R¹⁸, CONR¹⁸R¹⁹, SOR¹⁸, SO₂R¹⁸, SO₂NR¹⁸R¹⁹, NR²⁰CO₂R¹⁸, NR²⁰COR¹⁸, NR²⁰C(O)NR¹⁸R¹⁹, NR²⁰SOR¹⁸, NR²⁰SO₂R¹⁸, NR⁷SO₂NR⁵R⁶, optionally substituted Ci-Cs alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; wherein
      R¹⁸, R¹⁹ and R²⁰ are independently selected from hydrogen, optionally substituted Ci-Cs alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; or
      R¹⁸ and R¹⁹, R¹⁸ and R²⁰ together with the atom to which they are connected form a 4-20 membered heterocyclyl ring;
   Ar is selected from null, aryl and heteroaryl;
   n is independently selected from 0, 1, 2, 3, 4 and 5;
   Y, Z at each occurrence, are independently selected from null, CO, CO₂, CH₂, CR²⁴R²⁵, C(O)NR²⁴, C(S)NR²⁴, O, S, SO, SO₂, SO₂NR²⁴, NR²⁴, NR²⁴CO, NR²⁴CONR²⁴, NR²⁴C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
      R²⁴ and R²⁵ are independently selected from hydrogen, optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
   W, at each occurrence, is independently selected from null, CO, CH₂, (CH₂)ₘ CR²⁶R²⁷, (CR²⁶R²⁷)ₘ, SO, SO₂
   wherein
      R²⁶ and R²⁷ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
      and
   m = 0-5;
(II) a moiety according to FORMULA 3M: 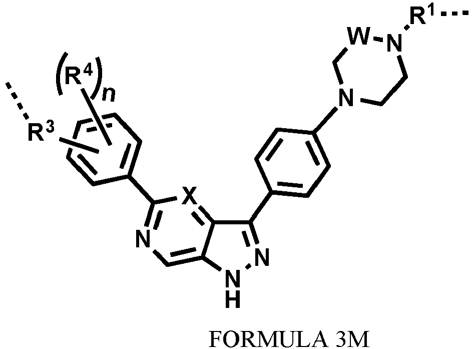 wherein
   the "Linker" moiety of the bivalent compound is attached independently to R¹ or R³; X is selected from CR² or N;
   the definitions of W, R¹, R², R³ and R⁴ are the same as for FORMULA 3L;
   n is independently selected from 0, 1, 2, 3, 4 and 5;
   and pharmaceutically acceptable salts thereof.

In an embodiment, **PI** is: 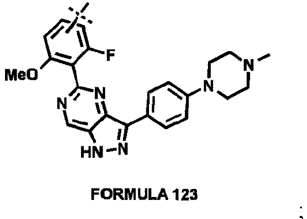 and pharmaceutically acceptable salts thereof.

### Degradation/Disruption Tags

The Degradation/Disruption tag **(EL)** of the present invention is a moiety selected from the group consisting of:
(I) a moiety according to FORMULAE 12A, 12B, 12C and 12D: 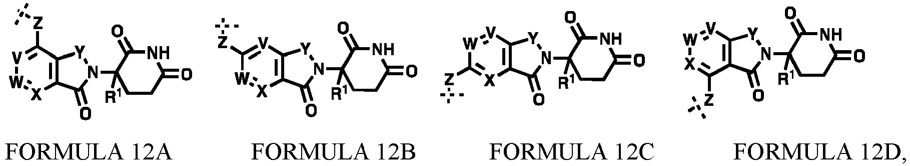 wherein
   V, W, and X are independently selected from CR² and N;
   Y is selected from CO, CR³R⁴, and N=N;
   Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
   R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
   R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
   R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
**(II)** a moiety according to one of FORMULAE 12E, 12F, 12G, 12H, and 12I: 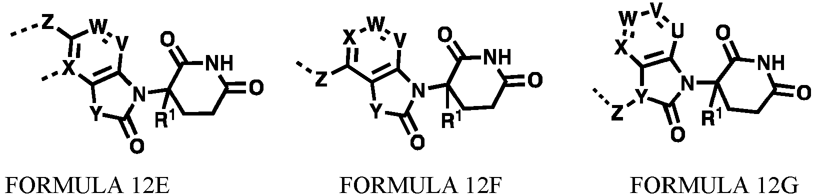 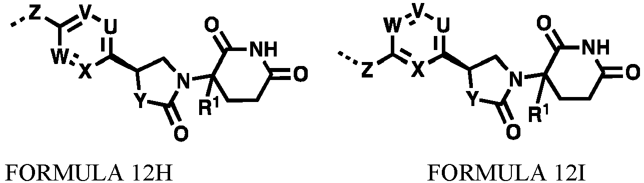 wherein
   U, V, W, and X are independently selected from CR² and N;
   Y is selected from CR³R⁴, NR³ and O; preferably, Y is selected from CH₂, NH, NCH₃ and O;
   Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
   R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
   R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
   R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
**(III)** a moiety according to FORMULA 13A: 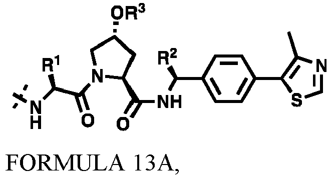 wherein
   R¹ and R² are independently selected from hydrogen, optionally substituted Ci-Cs alkyl,
   optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; and
   R³ is hydrogen, optionally substituted C(O)C₁-C₈ alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC₁-C₈alkoxyC₁-C₈alkyl, optionally substituted C(O)NC₁-C₈ haloalkyl, optionally substituted C(O)NC₁-C₈ hydroxyalkyl, optionally substituted C(O)NCi-C₈ aminoalkyl, optionally substituted C(O)NC₁-C₈alkylaminoC₁-C₈alkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂;
**(IV)** a moiety according to FORMULAE 13B, 13C, 13D, 13E and 13F: 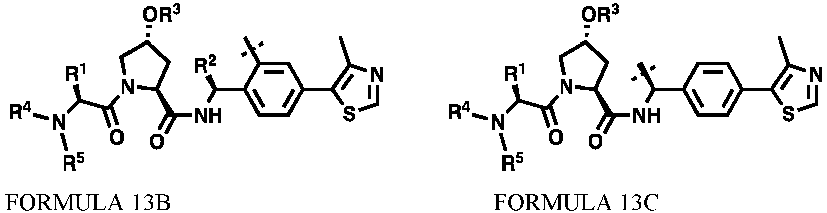 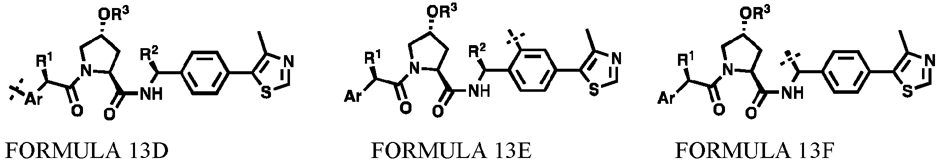 wherein
   R¹ and R² are independently selected from hydrogen, halogen, OH, NH₂, CN, optionally substituted C₁-C₈ alkyl, optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; (preferably, R¹ is selected from iso-propyl or tert-butyl; and R² is selected from hydrogen or methyl);
   R³ is hydrogen, optionally substituted C(O)C₁-C₈ alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC₁-C₈alkoxyC₁-C₈alkyl, optionally substituted C(O)NC₁-C₈ haloalkyl, optionally substituted C(O)NC₁-C₈ hydroxyalkyl, optionally substituted C(O)NCi-C₈ aminoalkyl, optionally substituted C(O)NC₁-C₈alkylaminoC₁-C₈alkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂; and
      R⁴ and R⁵ are independently selected from hydrogen, COR⁶, CO₂R⁶, CONR⁶R⁷, SOR⁶, SO₂R⁶, SO₂NR⁶R⁷, optionally substituted C₁-C₈ alkyl, optionally substituted C₁-C₈alkoxyC₁-Csalkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; wherein
      R⁶ and R⁷ are independently selected from hydrogen, optionally substituted C₁-C₈ alkyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; or
      R⁴ and R⁵; R⁶ and R⁷ together with the atom to which they are connected form a 4-8 membered cycloalkyl or heterocyclyl ring;
      Ar is selected from aryl and heteroaryl, each of which is optionally substituted with one or more substituents independently selected from F, Cl, CN, NO₂, OR⁸, NR⁸R⁹, COR⁸, CO₂R⁸, CONR⁸R⁹, SOR⁸, SO₂R⁸, SO₂NR⁹R¹⁰, NR⁹COR¹⁰, NR⁸C(O)NR⁹R¹⁰, NR⁹SOR¹⁰, NR⁹SO₂R¹⁰, optionally substituted C₁-C₆ alkyl, optionally substituted C₁-C₆ alkoxyalkyl, optionally substituted C₁-C₆ haloalkyl, optionally substituted C₁-C₆ hydroxyalkyl, optionally substituted C₁-C₆alkylaminoC₁-C₆alkyl, optionally substituted C₃-C₇ cycloalkyl, optionally substituted 3-7 membered heterocyclyl, optionally substituted C₂-C₆ alkenyl, optionally substituted C₂-C₆ alkynyl, optionally substituted aryl, and optionally substituted C₄-C₅ heteroaryl; wherein
      R⁸, R⁹, and R¹⁰ are independently selected from null, hydrogen, optionally substituted C₁-C₆ alkyl, optionally substituted C₂-C₆ alkenyl, optionally substituted C₂-C₆ alkynyl, optionally substituted C₃-C₇ cycloalkyl, optionally substituted 3-7 membered heterocyclyl, optionally substituted aryl, and optionally substituted heteroaryl; or
      R⁸ and R⁹; R⁹ and R¹⁰ together with the atom to which they are connected form a 4-8 membered cycloalkyl or heterocyclyl ring;
   and pharmaceutically acceptable salts thereof;

In an embodiment, degradation/disruption tags are selected from the group consisting of: 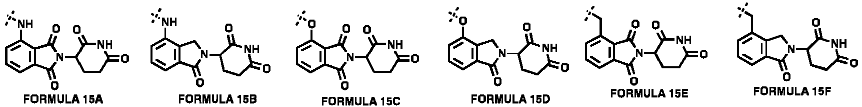 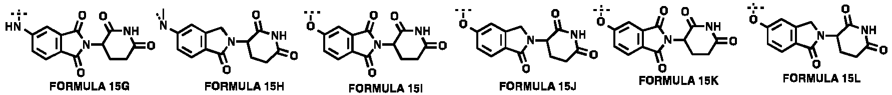 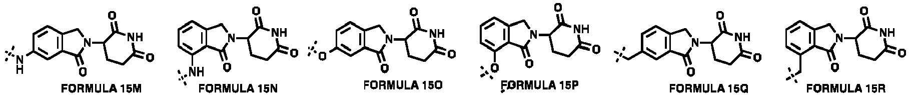 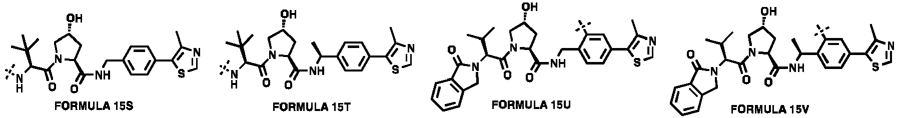 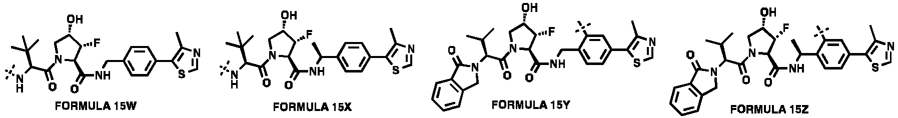 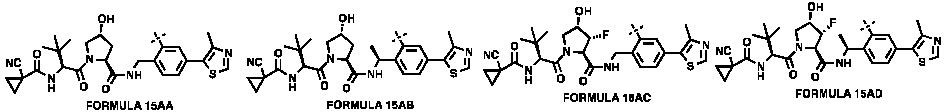 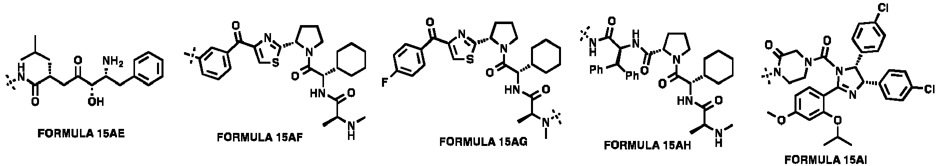 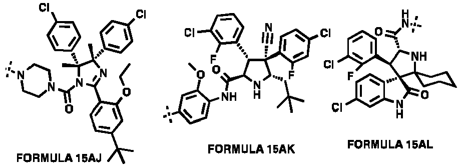 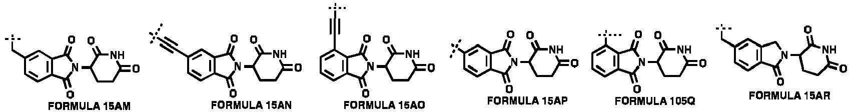 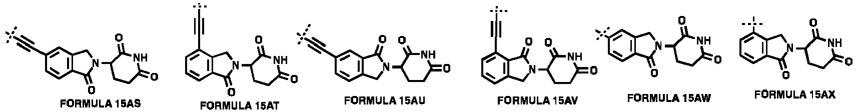 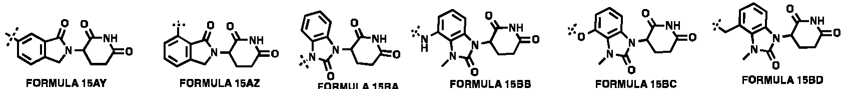 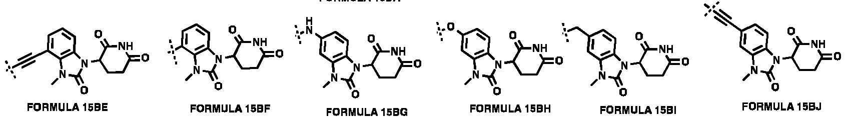 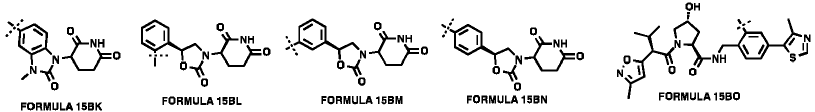 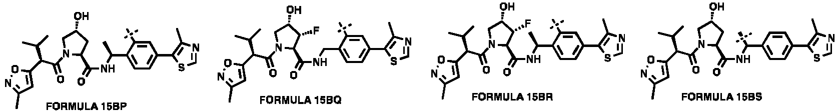 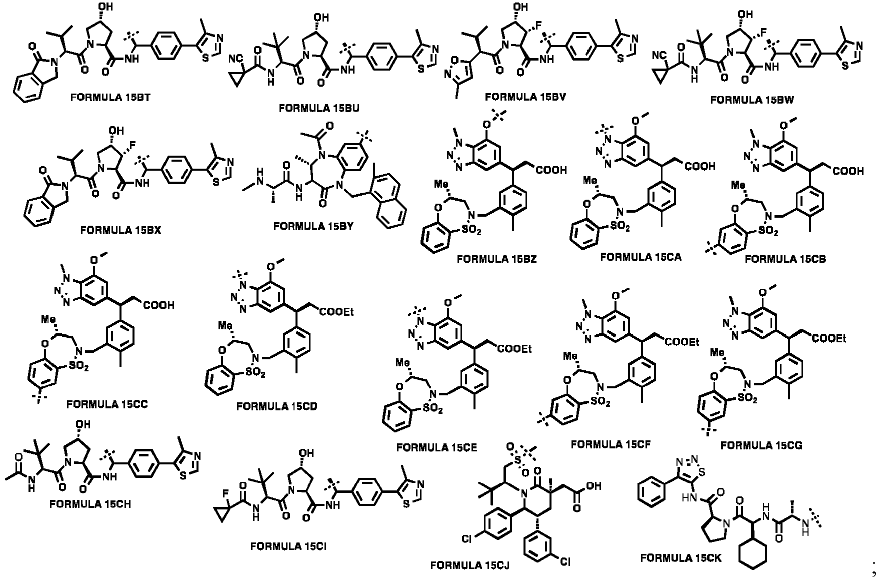 and pharmaceutically acceptable salts thereof.

### LINKERS

In any of the above-described compounds, the HPK1 ligand can be conjugated to the degradation/disruption tag through a linker. The linker can include, e.g., acyclic or cyclic saturated or unsaturated carbon, ethylene glycol, amide, amino, ether, urea, carbamate, aromatic, heteroaromatic, heterocyclic, and/or carbonyl containing groups with different lengths.

The linker of the present invention is:
**(I)** a moiety selected from the group consisting of:
   a moiety according to FORMULA 16: 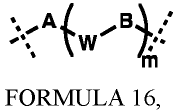
   wherein
   A, W, and B, at each occurrence, are independently selected from null, CO, CO₂, C(O)NR¹, C(S)NR¹, O, S, SO, SO₂, SO₂NR¹, NR¹, NR¹CO, NR¹CONR², NR¹C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
   R¹ and R² are independently selected from hydrogen, optionally substituted C₁-C₈ alkyl, optionally substituted C₃-C₈ cycloalkyl, optionally substituted 3-8 membered cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl; and
   m is 0 to 15;
   a moiety according to FORMULA 16A: 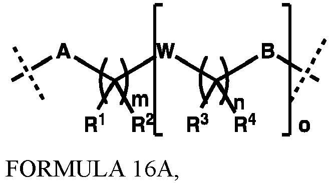
   wherein
   R¹, R², R³, and R⁴, at each occurrence, are independently selected from hydrogen, halogen, CN, OH, NH₂, optionally substituted C₁-C₈ alkyl, optionally substituted C₃-C₈ cycloalkyl, optionally substituted C₃-C₈ cycloalkoxy, optionally substituted 3-8 membered membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
   A, W, and B, at each occurrence, are independently selected from null, CO, CO₂, C(O)NR⁵, C(S)NR⁵, O, S, SO, SO₂, SO₂NR⁵, NR⁵, NR⁵CO, NR⁵CONR⁶, NR⁵C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
   R⁵ and R⁶ are independently selected from hydrogen, optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
   m is 0 to 15;
   n, at each occurrence, is 0 to 15; and
   o is 0 to 15;
   a moiety according to FORMULA 16B: 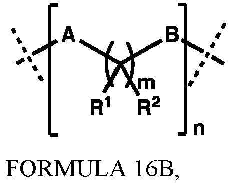
   wherein
   R¹ and R², at each occurrence, are independently selected from hydrogen, halogen, CN, OH, NH₂, and optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
   A and B, at each occurrence, are independently selected from null, CO, CO₂, C(O)NR³, C(S)NR³, O, S, SO, SO₂, SO₂NR³, NR³, NR³CO, NR³CONR⁴, NR³C(S), and optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, or C₃-C₁₃ spiro heterocyclyl; wherein
   R³ and R⁴ are independently selected from hydrogen, and optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
   each m is 0 to 15; and
   n is 0 to 15;
   a moiety according to FORMULA 16C: 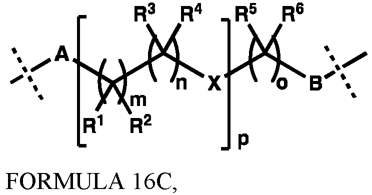
   wherein
   X is selected from O, NH, and NR⁷;
   R¹, R², R³, R⁴, R⁵, and R⁶, at each occurrence, are independently selected from hydrogen, halogen, CN, OH, NH₂, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
   A and B, at each occurrence, are independently selected from null, CO, NH, NH-CO, CO-NH, CH₂-NH-CO, CH₂-CO-NH, NH-CO-CH₂, CO-NH-CH₂, CH₂-NH-CH₂-CO-NH, CH₂-NH-CH₂-NH-CO, -CO-NH, CO-NH- CH₂-NH-CH₂, CH₂-NH-CH₂, CO₂, C(O)NR⁷, C(S)NR⁷, O, S, SO, SO₂, SO₂NR⁷, NR⁷, NR⁷CO, NR⁷CONR⁸, NR⁷C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
   R⁷ and R⁸ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
   m, at each occurrence, is 0 to 15;
   n, at each occurrence, is 0 to 15;
   o is 0 to 15; and
   p is 0 to 15; and
   pharmaceutically acceptable salts thereof; or
**(II)** a moiety selected from the group consisting of: a ring selected from the group consisting of a 3 to 13 membered ring; a 3 to 13 membered fused ring; a 3 to 13 membered bridged ring; and a 3 to13 membered spiro ring; and pharmaceutically acceptable salts thereof; or
(**III**) a moiety selected from the group consisting of one of FORMULAE C1, C2, C3, C4 and C5: 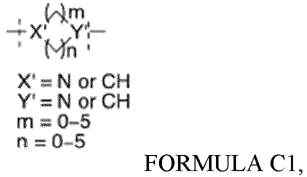 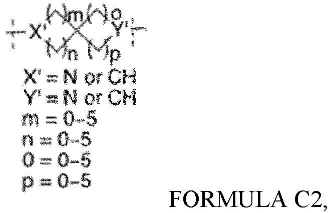 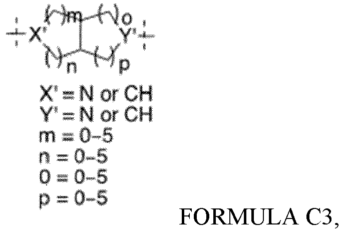 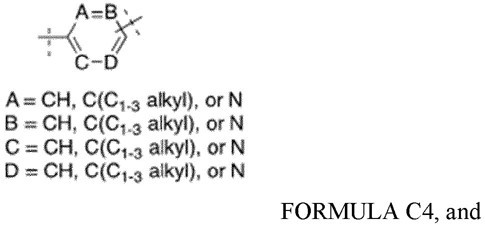 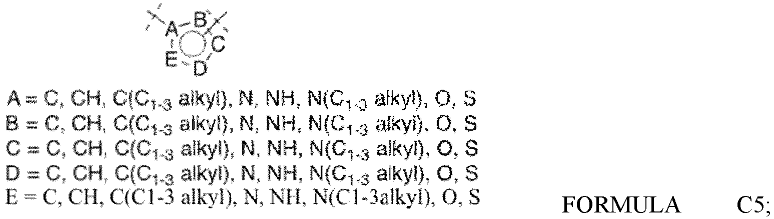 and pharmaceutically acceptable salts thereof.

In an embodiment, the bivalent compound according to the present invention is selected from the group consisting of:
HC58-18, HC58-19, HC58-20, HC58-22, HC58-23, HC58-24, HC58-25, HC58-26, HC58-27, HC58-28, HC58-29, HC58-30, HC58-31, HC58-32, HC58-33, HC58-34, HC58-35, HC58-36, HC58-37, HC58-38, HC58-39, HC58-40, HC58-41, HC58-43, HC58-44, HC58-45, HC58-46, HC58-53, HC58-57, HC58-58, HC58-59, HC58-60, HC58-63, HC58-64, HC58-65, HC58-66, HC58-67, HC58-68, HC58-69, HC58-70, HC58-71, HC58-73, HC58-74, HC58-75, HC58-76, HC58-77, HC58-78, HC58-133, HC58-134, HC58-135, HC58-136, HC58-137, HC58-138, HC58-139, HC58-144, HC58-145, HC58-146, HC58-147, HC58-148, HC58-149, HC58-150, HC58-158, HC58-159, HC58-160, HC58-161, HC58-164, HC58-165, HC58-167, HC58-178, HC58-179, HC58-180, HC58-181, HC58-182, HC58-183, HC58-184, HC58-185, HC65-2, HC65-3, HC65-4, HC65-5, HC65-6, HC65-7, HC65-8, HC65-13, HC65-14, HC65-15, HC65-16, HC65-17, HC65-18, HC65-19, HC65-24, HC65-25, HC65-26, HC65-27, HC65-28, HC65-29, HC65-30, HC65-33, HC65-34, HC65-35, HC65-37, HC65-175, HC65-183, HC65-184, HC65-185, HC65-186, HC75-1, HC75-2, HC75-3, HC75-4, HC75-5, HC75-6, HC75-7, HC75-8, HC75-9, HC75-10, HC75-11, HC75-12, HC75-13, HC75-14, HC75-15, HC75-16, HC75-17, HC75-18, HC75-18, HC75-20, HC75-21, HC75-22, HC75-23, HC75-24, HC75-29, HC75-31, HC75-34, HC75-35, HC75-36, HC75-37, HC75-38, HC75-39, HC75-40, HC75-41, HC75-42, HC75-43, HC75-44, HC75-45, HC75-46, HC75-47, HC75-48, HC75-49, HC75-50, HC75-52, HC75-53, HC75-54, HC75-55, HC75-56, HC75-57, HC75-58, HC75-59, HC75-60, HC75-61, HC75-62, HC90-33, HC90-34, HC90-35, HC90-36, HC90-37, HC90-41, HC90-42, HC90-43, HC90-44, HC90-45, HC90-46, HC90-47, HC90-49, HC90-50, HC90-51, HC90-52, HC90-53, HC90-54, HC90-55, HC90-56, HC90-57, HC90-58, HC90-59, HC90-61, HC90-66, HC90-69, HC90-70, HC90-71, HC90-72, HC90-73, HC90-74, HC90-84, HC90-85, HC90-86, HC90-87, HC90-88, HC90-89, HC90-90, HC90-91, HC90-92, HC90-93, HC90-94, HC90-95, HC90-96, HC90-97, HC90-102, HC90-103, HC90-104, HC90-105, HC90-106, HC90-107, HC90-108, HC90-109, HC90-110, HC90-111, HC90-112, HC90-113, HC90-117, HC90-119, HC90-120, HC90-121, HC90-122, HC90-123, HC90-124, HC90-125, HC90-126, HC90-127, HC90-128, HC90-129, HC90-130, HC90-131, HC90-132, HC90-133, HC90-134, HC90-135, HC90-136, HC90-60, HC90-62, HC90-63, HC90-64, HC90-65, HC90-67 and HC90-68 or analogs thereof; and pharmaceutically acceptable salts thereof.

In an embodiment, the bivalent compound according to the present invention is selected from the group consisting of: HC58-18, HC58-19, HC58-20, HC58-22, HC58-23, HC58-24, HC58-25, HC58-26, HC58-27, HC58-28, HC58-29, HC58-30, HC58-31, HC58-32, HC58-33, HC58-34, HC58-35, HC58-36, HC58-37, HC58-38, HC58-39, HC58-40, HC58-41, HC58-43, HC58-44, HC58-45, HC58-46, HC58-53, HC58-57, HC58-58, HC58-59, HC58-60, HC58-63, HC58-64, HC58-65, HC58-66, HC58-67, HC58-68, HC58-69, HC58-70, HC58-71, HC58-73, HC58-74, HC58-75, HC58-76, HC58-77, HC58-78, HC58-133, HC58-134, HC58-135, HC58-136, HC58-137, HC58-138, HC58-139, HC58-144, HC58-145, HC58-146, HC58-147, HC58-148, HC58-149, HC58-150, HC58-158, HC58-159, HC58-160, HC58-161, HC58-164, HC58-165, HC58-167, HC58-178, HC58-179, HC58-180, HC58-181, HC58-182, HC58-183, HC58-184,HC58-185, HC65-2, HC65-3, HC65-4, HC65-5, HC65-6, HC65-7, HC65-8, HC65-13, HC65-14, HC65-15, HC65-16, HC65-17, HC65-18, HC65-19, HC65-24, HC65-25, HC65-26, HC65-27, HC65-28, HC65-29, HC65-30, HC65-33, HC65-34, HC65-35 and HC65-37; and pharmaceutically acceptable salts thereof.

In an embodiment, the bivalent compound according to the present invention is selected from the group consisting of: HC65-175, HC65-183, HC65-184, HC65-185, HC65-186, HC75-1, HC75-2, HC75-3, HC75-4, HC75-5, HC75-6, HC75-7, HC75-8, HC75-9, HC75-10, HC75-11, HC75-12, HC75-13, HC75-14, HC75-15, HC75-16, HC75-17, HC75-18, HC75-18, HC75-20, HC75-21, HC75-22, HC75-23, HC75-24, HC75-29, HC75-31, HC75-34, HC75-35, HC75-36, HC75-37, HC75-38, HC75-39, HC75-40, HC75-41, HC75-42, HC75-43, HC75-44, HC75-45, HC75-46, HC75-47, HC75-48, HC75-49, HC75-50, HC75-52, HC75-53, HC75-54, HC75-55, HC75-56, HC75-57, HC75-58, HC75-59, HC75-60, HC75-61 and HC75-62; and pharmaceutically acceptable salts thereof.

In an embodiment, the bivalent compound according to the present invention is selected from the group consisting of:
HC90-33, HC90-34, HC90-35, HC90-36, HC90-37, HC90-41, HC90-42, HC90-43, HC90-44, HC90-45, HC90-46, HC90-47, HC90-49, HC90-50, HC90-51, HC90-52, HC90-53, HC90-54, HC90-55, HC90-56, HC90-57, HC90-58, HC90-59, HC90-61, HC90-66, HC90-69, HC90-70, HC90-71, HC90-72, HC90-73, HC90-74, HC90-84, HC90-85, HC90-86, HC90-87, HC90-88, HC90-89, HC90-90, HC90-91, HC90-92, HC90-93, HC90-94, HC90-95, HC90-96, HC90-97, HC90-102, HC90-103, HC90-104, HC90-105, HC90-106, HC90-107, HC90-108, HC90-109, HC90-110, HC90-111, HC90-112, HC90-113, HC90-117, HC90-119, HC90-120, HC90-121, HC90-122, HC90-123, HC90-124, HC90-125, HC90-126, HC90-127, HC90-128, HC90-129, HC90-130, HC90-131, HC90-132, HC90-133, HC90-134, HC90-135, HC90-136, HC90-60, HC90-62, HC90-63, HC90-64, HC90-65, HC90-67 and HC90-68; and pharmaceutically acceptable salts thereof.

In some aspects, this disclosure provides a method of treating the HPK1-mediated diseases, the method including administering to a subject in need thereof with an HPK1 -mediated disease one or more bivalent compounds including an HPK1 ligand conjugated to a degradation/disruption tag. The HPK1-mediated diseases may be a disease resulting from HPK1 amplification. The HPK1 -mediated diseases can have elevated HPK1 enzymatic activity relative to a wild-type tissue of the same species and tissue type. Non-limiting examples of HPK1-mediated diseases or diseases whose clinical symptoms could be treated by HPK1 degraders/disruptors-mediated therapy include: all solid and liquid cancer, chronic infections that produce exhausted immune response, infection-mediated immune suppression, age-related decline in immune response, age-related decline in cognitive function and infertility.

Exemplary types of cancer that could prevented, or therapeutically treated by manipulation of HPK1 level by degraders/disruptors should include all solid and liquid cancers, including, but not limited to, cancers of the breast, respiratory tract, brain, reproductive organs, digestive tract, urinary tract, eye, liver, skin, head and neck, thyroid, parathyroid and their distant metastases. Examples of liquid cancers include lymphomas, sarcomas, and leukaemias. Listed below are the type of cancers that immunotherapy using HPK1 degraders/disruptors should be able to prevent or treat.

Examples of breast cancers include, but are not limited to, triple negative breast cancer, invasive ductal carcinoma, invasive lobular carcinoma, ductal carcinoma in situ, and lobular carcinoma in situ.

Examples of cancers of the respiratory tract include, but are not limited to, small-cell and non- small-cell lung carcinoma, as well as bronchial adenoma and pleuropulmonary blastoma.

Examples of brain cancers include, but are not limited to, brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to, prostate and testicular cancer.

Tumors of the female reproductive organs include, but are not limited to, endometrial, cervical, ovarian, vaginal, and vulvar cancer, as well as sarcoma of the uterus.

Examples of ovarian cancer include, but are not limited to, serous tumor, endometrioid tumor, mucinous cystadenocarcinoma, granulosa cell tumor, Sertoli-Leydig cell tumor and arrhenoblastoma.

Examples of cervical cancer include, but are not limited to, squamous cell carcinoma, adenocarcinoma, adenosquamous carcinoma, small cell carcinoma, neuroendocrine tumor, glassy cell carcinoma and villoglandular adenocarcinoma. Tumors of the digestive tract include, but are not limited to, anal, colon, colorectal, esophageal, gallbladder, gastric, pancreatic, rectal, small-intestine, and salivary gland cancers.

Examples of esophageal cancer include, but are not limited to, esophageal cell carcinomas and adenocarcinomas, as well as squamous cell carcinomas, leiomyosarcoma, malignant melanoma, rhabdomyosarcoma and lymphoma.

Examples of gastric cancer include, but are not limited to, intestinal type and diffuse type gastric adenocarcinoma.

Examples of pancreatic cancer include, but are not limited to, ductal adenocarcinoma, adenosquamous carcinomas and pancreatic endocrine tumors.

Example of tumors of the urinary tract include, but are not limited to, bladder, penile, kidney, renal pelvis, ureter, urethral and human papillary renal cancers.

Examples of kidney cancer include, but are not limited to, renal cell carcinoma, urothelial cell carcinoma, juxtaglomerular cell tumor (reninoma), angiomyolipoma, renal oncocytoma, Bellini duct carcinoma, clear-cell sarcoma of the kidney, mesoblastic nephroma and Wilms' tumor.

Examples of bladder cancer include, but are not limited to, transitional cell carcinoma, squamous cell carcinoma, adenocarcinoma, sarcoma and small cell carcinoma. Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Example of skin cancers include, but are not limited to, squamous cell carcinoma, Kaposi's sarcoma, malignant melanoma, Merkel cell skin cancer, and non-melanoma skin cancer.

Example of head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.

Example of lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Example of sarcomas include, but are not limited to, sarcoma of the soft tissue, osteosarcoma, malignant fibrous histiocytoma, lymphosarcoma, and rhabdomyosarcoma.

Eample of leukemias include, but are not limited to, acute myeloid leukemia, acute lymphoblastic leukemia, chronic lymphocytic leukemia, chronic myelogenous leukemia, and hairy cell leukemia.

The HPK1 degraders/disruptors should be able to treat the above cancer types as stand alone agents or used as an agent in combination with existing standards of treatment therapy and other FDA-approved cancer therapy.

Therapeutic use of HPK1 extends to include diseases and therapies that are amenable to treatment by stimulation/augmentation of immune response, including the prolongation of immune responses during vaccination for immunizable diseases such as influenza and coronaviruses, including Covid 19. Also, because HPK1 is expressed at high level in two other anatomical locations - brain and testes - the HPK1 degraders/disruptors should be able to treat or prevent diseases related to brain and testes that were caused by HPK1 or could be treated by HPK1 degraders/disruptors. These potential diseases include, but are not limited to, Alzheimer's disease, age-related dementia and infertility, regardless whether these possible diseases were caused by HPK1 or by other etiological causes.

Therapeutic use of HPK1 further extends to include therapies involving *ex vivo* treatment of immune cells, including, but not limited to, all T cell subsets, genetically engineered T cells, Chimeric Antigen Receptor (CAR) T cells, tumor infiltrating lymphocytes, dendritic cells, macrophage, mast cells, granulocytes (include basophils, eosinophils, and neutrophils), natural killer cells, NK T cells and B cells. Such cells would be therapeutically treated by HPK1 degraders and then re-introduced back to the patient being treated for conditions that would benefit from reduction in HPK1 expression. The sources of cells for such *ex vivo* treatment include, but are not limited to, the autologous bone marrow cells from the patient him/herself, or from the patient's frozen banked cord blood stem cells, peripheral blood or bone marrow stem cells from MHC-matched or MHC-mismatched donors.

Treating patients by administering specific immune cells that had been treated with HPK1 degraders offers many added advantages over *in vivo* use. By treating specific immune cells type with HPK1 degraders *ex vivo,* it is possible to specifically target the immune cell type that would receive the benefit of having the endogenous HPK1 level reduced by HPK1 degraders while sparing the HPK1 expression level in other immune cell types that are not involved in the disease condition.

This therapeutic approach would provide cell type-specific targeting of immune cells in a way that is not possible with the use of HPK1 degrader in the *in vivo* setting. Thus, the *ex vivo* approach would likely limit potential toxicity that may result from reduction of HPK1 level in immune cell types that do not benefit from a reduction in HPK1 levels.

Furthermore, by administering HPK1 degraders in the *ex vivo* cell setting, the risk of patients experiencing the toxicity or undesirable outcome that might occur should the HPK1 degraders were to be administered systemically would be eliminated.

It is known that HPK1 is also expressed in non-hematopoietically-derived tissues such as the brain and testes. Because of this tissue-specific expression pattern of HPK1, HPK1 degraders might be able to treat or prevent diseases related to the brain and testes that were caused by HPK1. These potential treatments include, but are not limited to, treatment of Alzheimer's disease, age-related dementia and infertility, irrespective to whether these possible diseases were caused by HPK1 or by other etiological causes.

The use of HPK1 expression status of the tumor as the biomarker would enable stratification of patients into appropriate therapeutic groups that would receive HPK1 degraders *in vivo* or *ex vivo,* based on HPK1 expression in the tumors.

Furthermore, using HPK1 degraders in an *ex vivo* setting offers additional advantages over gene-editing approaches such as CRISPR in that it allows therapeutic use of HPK1 degraders as a non-permanent treatment that allows a therapeutic regimen to be adjusted temporally through dosing levels and through alteration of the administration schedule.

In addition, HPK1 degraders could be used in settings whereby stimulation/augmentation of the immune response is required, or when the prolongation of immune responses is needed. Improving immune response to vaccination is one of the settings in which HPK1 degraders could be used therapeutically. HPK1 degraders could also be used to enhance the antigen presentation capability of dendritic cell-based cancer vaccines.

Other utilities of HPK1 degraders include treatment of dendritic cells with HPK1 degraders to increase resistance to maturation-induced apoptosis, thus increasing the yield of dendritic cell production.

In an embodiment, HPK1 degraders of the present invention may be employed in combination with treatments using checkpoint inhibitors, including, but not limited to anti-programmed cell death protein (anti-PD-1) and anti-programmed death ligand-1 (anti-PD-L1). Examples of anti-PD-1 and anti-PD-L1 agents include monoclonal antibodies that target either PD-1 or PD-L1. Such antibodies include, but are not limited to pembrolizumab (Keytruda), nivolumab (Opdivo), and cemiplimab (Libtayo) (PD-1 inhibitors); and atezolizumab (Tecentriq), avelumab (Bavencio), and durvalumab (Imfinzi) (PD-L1 inhibitors). Use of such anti-PD1 and/or anti-PD-L1 agents in immunotherapy, particularly cancer immunotherapy, may be enhanced by concomitant therapy with HPK1 degraders of the present invention. Such combination therapy of anti-PD-1 agents with HPK1 degraders of the present invention is particularly useful in the treatment of melanoma, lung cancer, renal cell carcinoma, Hodgkin lymphoma, head and neck cancer, colon cancer and liver cancer. Such combination therapy of anti-PD-L1 agents with HPK1 degraders of the present invention are particularly useful in the treatment of non-small cell lung carcinoma, multiple myeloma, urothelial cancer and head and neck cancer. (Hernandez 2018).

Similar combination therapy may employ HPK1 degraders of the present invention with an anti-CTLA-4 (cytotoxic T-lymphocyte-associated protein 4) agent, such as the monoclonal antibody ilimumab, particularly for the treatment of melanoma, lung cancer, renal cell carcinoma, glioblastoma, hepatocellular carcinoma large B cell lymphoma, Hodgkin lymphoma, head and neck cancer, colon cancer and liver cancer.

In an embodiment, the HPK1 degraders of the present invention may be used in the treatment of tumor types having elevated expression of cyclooxygenase-2 (COX-2). COX-2 elevation leads to over production of prostaglandin E2 (PGE2). PGE2 made by these tumors is known to inhibit the anti-tumor immune response. T cells lacking HPK1 are resistant to PGE2-mediated inhibition. (Alzabin 2010). Cancer types known to have high expression levels of COX-2 include, but not are not limited to colon cancer, lung cancer, sarcoma and breast cancer.
In any of the above-described methods, the bivalent compounds can be HC58-18, HC58-19, HC58-20, HC58-22, HC58-23, HC58-24, HC58-25, HC58-26, HC58-27, HC58-28, HC58-29, HC58-30, HC58-31, HC58-32, HC58-33, HC58-34, HC58-35, HC58-36, HC58-37, HC58-38, HC58-39, HC58-40, HC58-41, HC58-43, HC58-44, HC58-45, HC58-46, HC58-53, HC58-57, HC58-58, HC58-59, HC58-60, HC58-63, HC58-64, HC58-65, HC58-66, HC58-67, HC58-68, HC58-69, HC58-70, HC58-71, HC58-73, HC58-74, HC58-75, HC58-76, HC58-77, HC58-78, HC58-133, HC58-134, HC58-135, HC58-136, HC58-137, HC58-138, HC58-139, HC58-144, HC58-145, HC58-146, HC58-147, HC58-148, HC58-149, HC58-150, HC58-158, HC58-159, HC58-160, HC58-161, HC58-164, HC58-165, HC58-167, HC58-178, HC58-179, HC58-180, HC58-181, HC58-182, HC58-183, HC58-184, HC58-185, HC65-2, HC65-3, HC65-4, HC65-5, HC65-6, HC65-7, HC65-8, HC65-13, HC65-14, HC65-15, HC65-16, HC65-17, HC65-18, HC65-19, HC65-24, HC65-25, HC65-26, HC65-27, HC65-28, HC65-29, HC65-30, HC65-33, HC65-34, HC65-35, HC65-37, HC65-175, HC65-183, HC65-184, HC65-185, HC65-186, HC75-1, HC75-2, HC75-3, HC75-4, HC75-5, HC75-6, HC75-7, HC75-8, HC75-9, HC75-10, HC75-11, HC75-12, HC75-13, HC75-14, HC75-15, HC75-16, HC75-17, HC75-18, HC75-18, HC75-20, HC75-21, HC75-22, HC75-23, HC75-24, HC75-29, HC75-31, HC75-34, HC75-35, HC75-36, HC75-37, HC75-38, HC75-39, HC75-40, HC75-41, HC75-42, HC75-43, HC75-44, HC75-45, HC75-46, HC75-47, HC75-48, HC75-49, HC75-50, HC75-52, HC75-53, HC75-54, HC75-55, HC75-56, HC75-57, HC75-58, HC75-59, HC75-60, HC75-61, HC75-62, HC90-33, HC90-34, HC90-35, HC90-36, HC90-37, HC90-41, HC90-42, HC90-43, HC90-44, HC90-45, HC90-46, HC90-47, HC90-49, HC90-50, HC90-51, HC90-52, HC90-53, HC90-54, HC90-55, HC90-56, HC90-57, HC90-58, HC90-59, HC90-61, HC90-66, HC90-69, HC90-70, HC90-71, HC90-72, HC90-73, HC90-74, HC90-84, HC90-85, HC90-86, HC90-87, HC90-88, HC90-89, HC90-90, HC90-91, HC90-92, HC90-93, HC90-94, HC90-95, HC90-96, HC90-97, HC90-102, HC90-103, HC90-104, HC90-105, HC90-106, HC90-107, HC90-108, HC90-109, HC90-110, HC90-111, HC90-112, HC90-113, HC90-117, HC90-119, HC90-120, HC90-121, HC90-122, HC90-123, HC90-124, HC90-125, HC90-126, HC90-127, HC90-128, HC90-129, HC90-130, HC90-131, HC90-132, HC90-133, HC90-134, HC90-135, HC90-136, HC90-60, HC90-62, HC90-63, HC90-64, HC90-65, HC90-67, HC90-68 or analogs thereof.

In some aspects of the disclosed methods, the bivalent compounds can be administered by any of several routes of administration including, *e*.*g*., orally, parenterally, intradermally, subcutaneously, topically, and/or rectally.

Any of the above-described methods can further include treating the subject with one or more additional therapeutic regimens for treating cancer. The one or more additional therapeutic regimens for treating cancer can be, *e.g.*, one or more of surgery, chemotherapy, radiation therapy, hormone therapy, or immunotherapy.

This disclosure additionally provides a method for identifying a bivalent compound which mediates degradation/disruption of HPK1, the method including providing a heterobifunctional test compound including a HPK1 ligand conjugated to a degradation/disruption tag, contacting the heterobifunctional test compound with a cell (*e.g.,* a cancer cell such as a HPK1-mediated cancer cell) including a ubiquitin ligase and HPK1.

As used herein, the terms "about" and "approximately" are defined as being within plus or minus 10% of a given value or state, preferably within plus or minus 5% of said value or state. Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used.

Other features and advantages of the invention will be apparent from the following detailed description and figures, and from the claims.

### BRIEF DESCRIPTION OF THE DRAWINGS

**Figure 1** is a series of graphs of screening of HC58 Series HPK1 Degraders for the enhancement of TCR-induced IL-2 production.
**Figure 2** is a Western blot analysis showing that HC58 series degraders could reduce the endogenous level of HPK1 in Jurkat T cells.
**Figure 3** is a Western blot analysis showing that the HC58 series degraders, HC58-75 and HC58-78 could reduce the endogenous level of HPK1 in Jurkat T cells on multiple day post exposure to HC58 series.
**Figure 4** is a Western blot analysis revealed that the HC58 series degraders could reduce the endogenous level of HPK1 in Jurkat T cells, relative to DMSO and other controls. (A) Amounts of IL-2 produced in response to TCR engagement. (B) Fold IL-2 increase after Degrader treatment.
**Figure 5** is a set of graphs showing that treating primary T cells with the lead HPK1 degraders from the HC58 series conferred murine primary T cells with elevated IL-2 response, as well as an enhanced proliferative response to the anti-CD28 X anti-CD28 mAb-mediated receptor crosslinking.
**Figure 6** is a graph showing CD28-independent IL-2 production by HC58-75-treated CD4⁺ T cells upon being stimulated by a fixed concentration of plate-bound anti-CD3ε and varying concentrations of soluble anti-CD28 mAb.
**Figure 7** is a graph showing treating primary GFP⁺ Tregs with the HC58-78 HPK1 degrader conferred Tregs with an elevated IL-2 production in response to the stimulation by TCR engagement.
**Figure 8** is a set of graphs showing screening the HC90 HPK1 degrader series for compounds that could elicit superior IL-2 production when compared to the level elicited by the lead HC58 series compounds.
**Figure 9** is a graph showing IL-2 response profiles elicited by varying concentrations of the lead HC90 compounds in Jurkat T cells.
**Figure 10** is a graph showing IL-2 produced by HC58-78-treated or HC90-50-treated human PBMC, shown as fold differences in IL-2 produced by the degrader-treated cells relative to IL-2 produced by the DMSO-treated cells.
**Figure 11** is a schematic depiction of how HPK1 degraders might be used as therapeutic agents to directly or indirectly treat various disease states.
**Figure 12** is a series of blots showing HPK1 degraders could effectively degrade endogenous HPK1 in murine T cells, murin TCR transgenic T cells and in human DC1 dendritic cells.
**Figure 13** is a set of graphs showing HPK1 degraders could effectively enhance Blinatumomab-mediated killing of the human CD19⁺ B Cell Acute Lymphoblastic Leukemia cells, Raji.
**Figure 14** is a set of graphs showing HPK1 degraders could effectively pro-inflammatory cytokine produced by the Blinatumomab-treated PBMC cells. (A) A representative intracellular cytokine staining pattern for the expression of IFNγ and TNFα by HPK1 degrader/Blinatumomab-treated PBMC cells. (B) Averaged percentage of cells that are stained positive for both IFNγ and TNFα in HPK1 degrader/Blinatumomab-treated PBMC cells.

### DETAILED DESCRIPTION

The present disclosure is based, in part, on the discovery that novel heterobifunctional molecules which degrade HPK1, HPK1 fusion proteins, and/or HPK1 mutant proteins are useful in the treatment of HPK1 -mediated diseases.

Non-limiting examples of HPK1-mediated diseases or diseases whose clinical symptoms could be treated by HPK1 degraders/disruptors-mediated therapy include: all solid and liquid cancer, chronic infections that produce exhausted immune response, infection-mediated immune suppression, age-related decline in immune response, age-related decline in cognitive function and infertility

Exemplary type of cancers that could be prevented, or therapeutically treated by manipulation of HPK1 level by degraders/disruptors should include all solid and liquid cancers, including, but not limited to, cancers of the breast, respiratory tract, brain, reproductive organs, digestive tract, urinary tract, eye, liver, skin, head and neck, thyroid, parathyroid and their distant metastases. Examples of liquid cancers include lymphomas, sarcomas, and leukaemias. Listed below are the type of cancers that immunotherapy using HPK1 degraders/disruptors should be able to prevent or treat.

Examples of breast cancers include, but are not limited to, triple negative breast cancer, invasive ductal carcinoma, invasive lobular carcinoma, ductal carcinoma in situ, and lobular carcinoma *in situ.*

Examples of cancers of the respiratory tract include, but are not limited to, small-cell and non- small-cell lung carcinoma, as well as bronchial adenoma and pleuropulmonary blastoma.

Examples of brain cancers include, but are not limited to, brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to, prostate and testicular cancer.

Tumors of the female reproductive organs include, but are not limited to, endometrial, cervical, ovarian, vaginal, and vulvar cancer, as well as sarcoma of the uterus.

Examples of ovarian cancer include, but are not limited to, serous tumor, endometrioid tumor, mucinous cystadenocarcinoma, granulosa cell tumor, Sertoli-Leydig cell tumor and arrhenoblastoma.

Examples of cervical cancer include, but are not limited to, squamous cell carcinoma, adenocarcinoma, adenosquamous carcinoma, small cell carcinoma, neuroendocrine tumor, glassy cell carcinoma and villoglandular adenocarcinoma. Tumors of the digestive tract include, but are not limited to, anal, colon, colorectal, esophageal, gallbladder, gastric, pancreatic, rectal, small-intestine, and salivary gland cancers.

Examples of esophageal cancer include, but are not limited to, esophageal cell carcinomas and adenocarcinomas, as well as squamous cell carcinomas, leiomyosarcoma, malignant melanoma, rhabdomyosarcoma and lymphoma.

Examples of gastric cancer include, but are not limited to, intestinal type and diffuse type gastric adenocarcinoma.

Examples of pancreatic cancer include, but are not limited to, ductal adenocarcinoma, adenosquamous carcinomas and pancreatic endocrine tumors.

Example of tumors of the urinary tract include, but are not limited to, bladder, penile, kidney, renal pelvis, ureter, urethral and human papillary renal cancers.

Examples of kidney cancer include, but are not limited to, renal cell carcinoma, urothelial cell carcinoma, juxtaglomerular cell tumor (reninoma), angiomyolipoma, renal oncocytoma, Bellini duct carcinoma, clear-cell sarcoma of the kidney, mesoblastic nephroma and Wilms' tumor.

Examples of bladder cancer include, but are not limited to, transitional cell carcinoma, squamous cell carcinoma, adenocarcinoma, sarcoma and small cell carcinoma. Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Example of skin cancers include, but are not limited to, squamous cell carcinoma, Kaposi's sarcoma, malignant melanoma, Merkel cell skin cancer, and non-melanoma skin cancer.

Example of head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.

Example of lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Example of sarcomas include, but are not limited to, sarcoma of the soft tissue, osteosarcoma, malignant fibrous histiocytoma, lymphosarcoma, and rhabdomyosarcoma.

Eample of leukemias include, but are not limited to, acute myeloid leukemia, acute lymphoblastic leukemia, chronic lymphocytic leukemia, chronic myelogenous leukemia, and hairy cell leukemia.

The HPK1 degraders/disruptors should be able to treat the above cancer type as stand alone agent or used as agent in combination with existing standard of treatment therapy and other FDA-approved cancer therapy.

Therapeutic uses of HPK1 include diseases and therapies that are amenable to treatment by stimulation/augmentation of immune response, including the prolongation of immune responses during vaccination for immunizable diseases. Also, because HPK1 is expressed at high level in two other anatomical locations - brain and testes - the HPK1 degraders/disruptors should be able to treat or prevent diseases related to brain and testes that were caused by HPK1 or could be treated by HPK1 degraders/disruptors. These potential diseases include, but is not limited to, Alzheimer's disease, age-related dementia and infertility, regardless whether these possible diseases were caused by HPK1 or by other etiological causes.

Successful strategies for selective degradation/disruption of the target protein induced by a bifunctional molecule include recruiting an E3 ubiquitin ligase and mimicking protein misfolding with a hydrophobic tag (Buckley and Crews, 2014). PROTACs (PROteolysis TArgeting Chimeras) are bivalent molecules with one moiety that binds an E3 ubiquitin ligase and another moiety that binds the protein target of interest (Buckley and Crews, 2014). The induced proximity leads to selective ubiquitination of the target followed by its degradation at the proteasome. Several types of high affinity small-molecule E3 ligase ligands have been identified/developed: They include (1) immunomodulatory drugs (IMiDs) such as thalidomide and pomalidomide, which bind cereblon (CRBN or CRL4^{CRBN}), a component of a cullin-RING ubiquitin ligase (CRL) complex (Bondeson et al., 2015; Chamberlain et al., 2014; Fischer et al., 2014; Ito et al., 2010; Winter et al., 2015); (2) VHL-1, a hydroxyproline-containing ligand, which binds van Hippel-Lindau protein (VHL or CRL2^{VHL}), a component of another CRL complex (Bondeson et al., 2015; Buckley et al., 2012a; Buckley et al., 2012b; Galdeano et al., 2014; Zengerle et al., 2015); (3) compound 7,which selectively binds KEAPI, a component of a CRL3 complex (Davies et al., 2016); (4) AMG232, which selectively binds MDM2, a heterodimeric RING E3 ligase (Sun et al., 2014); and (5) LCL161, which selectively binds IAP, a homodimeric RING E3 ligase (Ohoka et al., 2017; Okuhira et al, 2011; Shibata et al., 2017). The degrader technology has been successfully applied to degradation of multiple targets (Bondeson et al., 2015; Buckley et al., 2015; Lai et al., 2016; Lu et al., 2015; Winter et al., 2015; Zengerle et al., 2015), but not to degradation of HPK1. In addition, a hydrophobic tagging approach, which utilizes a bulky and hydrophobic adamantyl group, has been developed to mimic protein misfolding, leading to the degradation of the target protein by proteasome (Buckley and Crews, 2014). This approach has also been successfully applied to selective degradation of the pseudokinase Her3 (Xie et al., 2014), but not to degradation of HPK1 proteins.

As discussed in the following examples, this disclosure provides specific examples of novel HPK1 degraders/disruptors, and examined the effect of exemplary degraders/disruptors on reducing HPK1 protein levels, inhibiting/disrupting HPK1 activity and increasing the TCR-induced IL-2 production by Jurkat T cells. The results indicated that these novel compounds can be beneficial in treating cancer. Exemplary type of cancers that could be prevented, or therapeutically treated by manipulation of HPK1 level by degraders/disruptors should include all solid and liquid cancers, including, but not limited to, cancers of the breast, respiratory tract, brain, reproductive organs, digestive tract, urinary tract, eye, liver, skin, head and neck, thyroid, parathyroid and their distant metastases. Examples of liquid cancers include lymphomas, sarcomas, and leukaemias. Listed below are the type of cancers that immunotherapy using HPK1 degraders/disruptors should be able to prevent or treat as mentioned above.

Current compounds targeting HPK1 generally focus on inhibition of its catalytic activity. In the present disclosure a different approach was taken: to develop compounds that directly and selectively target not only the catalytic function of HPK1, but also its protein level in cells. Strategies for inducing protein degradation include recruiting E3 ubiquitin ligases, mimicking protein misfolding with hydrophobic tags, and inhibiting chaperones. For example, a thalidomide-JQ1 bivalent compound has been used to hijack the cereblon E3 ligase, inducing highly selective BET protein degradation *in vitro* and *in vivo* and resulting in a demonstrated delay in leukemia progression in mice (Winter et al., 2015). Similarly, BET protein degradation has also been induced via another E3 ligase, VHL (Zengerle et al., 2015). Partial degradation of the Her3 protein has been induced using an adamantane-modified compound (Xie et al., 2014). Such an approach, based on the use of bivalent molecules, permits more flexible regulation of protein levels *in vitro* and *in vivo* compared with techniques such as gene knockout or knockdown via RNA interference. Unlike gene knockout or knockdown, this chemical approach provides an opportunity to study dose and time dependency in a disease model by varying the concentrations and frequencies of administration of the relevant compound.

This disclosure includes all stereoisomers, geometric isomers, tautomers and isotopes of the structures depicted and compounds named herein. This disclosure also includes compounds described herein, regardless of how they are prepared, e.g., synthetically, through biological process (e.g., metabolism or enzyme conversion), or a combination thereof.

This disclosure includes pharmaceutically acceptable salts of the structures depicted and compounds named herein.

One or more constituent atoms of the compounds presented herein can be replaced or substituted with isotopes of the atoms in natural or non-natural abundance. In some embodiments, the compound includes at least one deuterium atom. In some embodiments, the compound includes two or more deuterium atoms. In some embodiments, the compound includes 1-2, 1-3, 1-4, 1-5, or 1-6 deuterium atoms. In some embodiments, all of the hydrogen atoms m a compound can be replaced or substituted by deuterium atoms. In some embodiments, the compound includes at least one fluorine atom. In some embodiments, the compound includes two or more fluorine atoms. In some embodiments, the compound includes 1-2, 1-3, 1-4, 1-5, or 1-6 fluorine atoms. In some embodiments, all of the hydrogen atoms in a compound can be replaced or substituted by fluorine atoms.

### Degraders

In some aspects, the present disclosure provides bivalent compounds, also referred to herein as degraders, comprising a HPK1 ligand (or targeting moiety) conjugated to a degradation tag. Linkage of the HPK1 ligand to the degradation tag can be direct, or indirect via a linker.

As used herein, the terms "protein arginine methyltransferase 5 (HPK1) ligand" or "HPK1 ligand" or "HPK1 targeting moiety" are to be construed broadly, and encompass a wide variety of molecules ranging from small molecules to large proteins that associate with or bind to HPK1. The HPK1 ligand or targeting moiety can be, for example, a small molecule compound (i.e., a molecule of molecular weight less than about 1.5 kilodaltons (kDa)), a peptide or polypeptide, nucleic acid or oligonucleotide, carbohydrate such as oligosaccharides, or an antibody or fragment thereof.

The HPK1 ligand or targeting moiety can be derived from a HPK1 inhibitor (e.g., sutent and analogs thereof), which is capable of interfering with the enzymatic activity of HPK1. As used herein, an "inhibitor" refers to an agent that restrains, retards, or otherwise causes inhibition of a physiological, chemical or enzymatic action or function. As used herein an inhibitor causes a decrease in enzyme activity of at least 5%. An inhibitor can also or alternatively refer to a drug, compound, or agent that prevents or reduces the expression, transcription, or translation of a gene or protein. An inhibitor can reduce or prevent the function of a protein, e.g., by binding to or activating/inactivating another protein or receptor.

As used herein, the term "degradation/disruption tag" refers to a compound, which associates with or binds to a ubiquitin ligase for recruitment of the corresponding ubiquitination machinery to HPK1 or induces HPK1 protein misfolding and subsequent degradation at the proteasome or loss of function.

In some aspects, the degradation/disruption tags of the present disclosure include, *e.g.*, thalidomide, pomalidomide, lenalidomide, VHL-1, adamantane, 1-((4,4,5,5,5-pentafluoropentyl)sulfinyl)nonane, nutlin-3a, RG7112, RG7338, AMG232, AA-115, bestatin, MV-1, LCL161, and/or analogs thereof.

As used herein, a "linker" is a bond, molecule, or group of molecules that binds two separate entities to one another. Linkers can provide for optimal spacing of the two entities. The term "linker" in some aspects refers to any agent or molecule that bridges the HPK1 ligand to the degradation/disruption tag. One of ordinary skill in the art recognizes that sites on the HPK1 ligand or the degradation/disruption tag, which are not necessary for the function of the degraders of the present disclosure, are ideal sites for attaching a linker, provided that the linker, once attached to the conjugate of the present disclosure, does not interfere with the function of the degrader, i.e., its ability to target HPK1 and its ability to recruit a ubiquitin ligase.

The length of the linker of the bivalent compound can be adjusted to minimize the molecular weight of the disruptors/degraders and avoid any potential clash of the HPK1 ligand or targeting moiety with either the ubiquitin ligase or the induction of HPK1 misfolding by the hydrophobic tag at the same time.

In some aspects, the degradation/disruption tags of the present disclosure include, for example, thalidomide, pomalidomide, lenalidomide, VHL-1, adamantane, 1-((4,4,5,5,5-pentafluoropentyl)sulfinyl)nonane, nutlin-3a, RG7112, RG7338, AMG 232, AA-115, bestatin, MV-1, LCL161, and analogs thereof. The degradation/disruption tags can be attached to any portion of the structure of a HPK1 ligand or targeting moiety (*e*.*g*., sutent) with linkers of different types and lengths in order to generate effective bivalent compounds. In particular, attaching VHL1, pomalidomide, or LCL161 to any portion of the molecule can recruit the E3 ligase to HPK1.

The bivalent compounds disclosed herein can increase the TCR-induced IL-2 production by Jurkat T cells.

Non-limiting examples of HPK1 disruptors/degraders (*e*.*g*., bivalent compounds) are shown in Table 1 (below). The left portion of each HPK1 disruptors/degrader compound as shown binds to HPK1 (as sutent (sunitinib) do), and the right portion of each compound recruits for the ubiquitination machinery to HPK1, which induces the poly-ubiquitination and degradation of HPK1 at the proteasome.

The present invention provides a bivalent compound including a HPK1 ligand conjugated to a degradation/disruption tag.

The HPK1 degraders/disruptors of the present invention have the form **"PI-linker-EL",** as shown below: 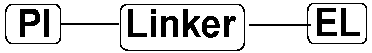 wherein **PI** (protein of interest) comprises an HPK1 ligand (*e.g.,* an HPK1 inhibitor) and **EL** (E3 ligase) comprises a degradation/disruption tag (*e.g.,* E3 ligase ligand). HPK1 ligands (**PI**), degradation/disruption tags **(EL)**, and linkers **(Linker)** of the present invention are illustrated below.

### HPK1 Ligands

The HPK1 ligand **(PI)** of the present invention is a moiety selected from the group consisting of:
**(I)** a moiety according to FORMULA 3L: 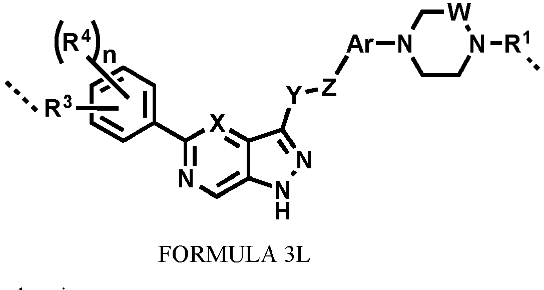 wherein
   the "Linker" moiety of the bivalent compound is attached independently to R¹ or R³;
   X is selected from CR² or N;
   R¹ and R³ are independently selected from null, hydrogen, C(O)R⁶, C(O)OR⁶, C(O)NR⁶R⁷, S(O)R⁶, S(O)₂R⁶, S(O)₂NR⁶R⁷, NR⁸C(O)OR⁶, NR⁸C(O)R⁶, NR⁸C(O)NR⁶R⁷, NR⁸S(O)R⁶, NR⁸S(O)₂R⁶, NR⁸S(O)₂NR⁶R⁷ , optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
   R⁶ is null, or a bivalent moiety selected from optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl;
      R⁷, and R⁸ are independently selected from hydrogen, optionally substituted C₁-C₈ alkyl, optionally substituted C₁-C₈alkoxyC₁-C₈alkyl, optionally substituted C₁-C₈alkylaminoC₁-Csalkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
      R⁶ and R⁷ together with the atom to which they are connected form an optionally substituted 3-20 membered cycloalkyl or heterocyclyl ring;
   R² is independently selected from hydrogen, halogen, oxo, CN, NO₂, OR⁹, SR⁹, NR⁹R¹⁰, C(O)R⁹, C(O)OR⁹, C(O)NR⁹R¹⁰, S(O)R⁹, S(O)₂R⁹, S(O)₂NR⁹R¹⁰, NR¹¹C(O)OR⁹, NR¹¹C(O)R⁹, NR¹¹C(O)NR⁹R¹⁰, NR¹¹S(O)R⁹, NR¹¹S(O)₂R⁹, NR¹¹S(O)₂NR⁹R¹⁰, optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
      R⁹, R¹⁰, and R¹¹ are independently selected from hydrogen, optionally substituted C₁-C₈ alkyl, optionally substituted C₁-C₈alkoxyC₁-C₈alkyl, optionally substituted C₁-C₈alkylaminoC₁-Csalkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
      R⁹ and R¹⁰, R⁹ and R¹¹, R¹⁰ and R¹¹ together with the atom to which they are connected form an optionally substituted 3-20 membered cycloalkyl or heterocyclyl ring;
   each R⁴ is independently selected from null, hydrogen, halogen, oxo, CN, NO₂, OR¹⁸, SR¹⁸, NR¹⁸R¹⁹, OCOR¹⁸, OCO₂R¹⁸, OCONR¹⁸R¹⁹, COR¹⁸, CO₂R¹⁸, CONR¹⁸R¹⁹, SOR¹⁸, SO₂R¹⁸, SO₂NR¹⁸R¹⁹, NR²⁰CO₂R¹⁸, NR²⁰COR¹⁸, NR²⁰C(O)NR¹⁸R¹⁹, NR²⁰SOR¹⁸, NR²⁰SO₂R¹⁸, NR⁷SO₂NR⁵R⁶, optionally substituted Ci-Cs alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; wherein
      R¹⁸, R¹⁹ and R²⁰ are independently selected from hydrogen, optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; or
      R¹⁸ and R¹⁹, R¹⁸ and R²⁰ together with the atom to which they are connected form a 4-20 membered heterocyclyl ring;
   Ar is selected from null, aryl and heteroaryl;
   n is independently selected from 0, 1, 2, 3, 4 and 5;
   Y, Z at each occurrence, are independently selected from null, CO, CO₂, CH₂, CR²⁴R²⁵, C(O)NR²⁴, C(S)NR²⁴, O, S, SO, SO₂, SO₂NR²⁴, NR²⁴, NR²⁴CO, NR²⁴CONR²⁴, NR²⁴C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
      R²⁴ and R²⁵ are independently selected from hydrogen, optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
   W, at each occurrence, is independently selected from null, CO, CH₂, (CH₂)ₘ CR²⁶R²⁷, (CR²⁶R²⁷)ₘ, SO, SO₂
   wherein
      R²⁶ and R²⁷ are independently selected from hydrogen, optionally substituted Ci-Cs alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
      and
   m = 0-5;
**(II)** a moiety according to FORMULA 3M: 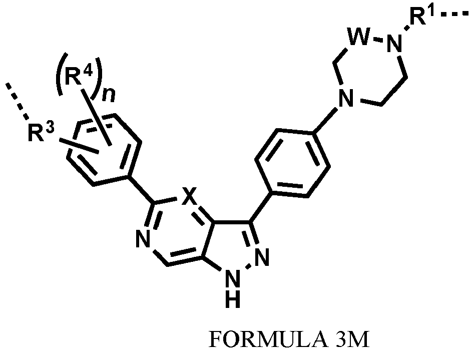 wherein
   the "Linker" moiety of the bivalent compound is attached independently to R¹ or R³; X is selected from CR² or N;
   the definitions of W, R¹, R², R³ and R⁴ are the same as for FORMULA 3L;
   n is independently selected from 0, 1, 2, 3, 4 and 5;
   and pharmaceutically acceptable salts thereof.

In an embodiment, **PI** is: 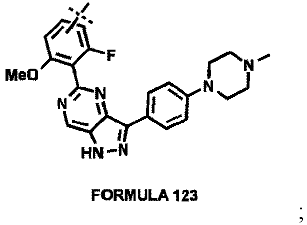 and pharmaceutically acceptable salts thereof.

### Degradation/Disruption Tags

The Degradation/Disruption tag **(EL)** of the present invention is a moiety selected from the group consisting of:
**(I)** a moiety according to FORMULAE 12A, 12B, 12C and 12D: 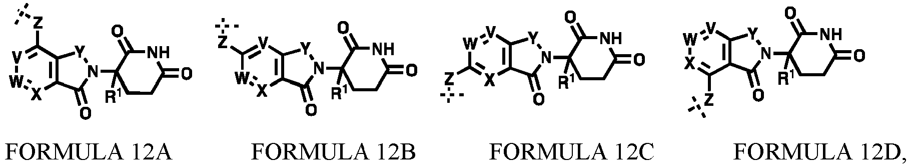 wherein
   V, W, and X are independently selected from CR² and N;
   Y is selected from CO, CR³R⁴, and N=N;
   Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
   R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
   R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
   R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
(II) a moiety according to one of FORMULAE 12E, 12F, 12G, 12H, and 12I: 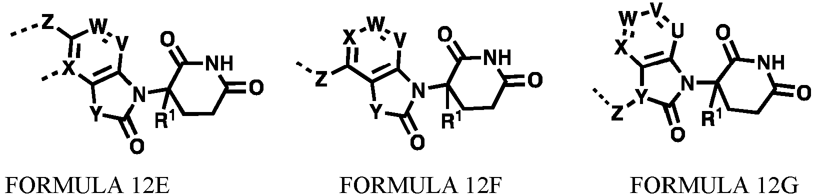 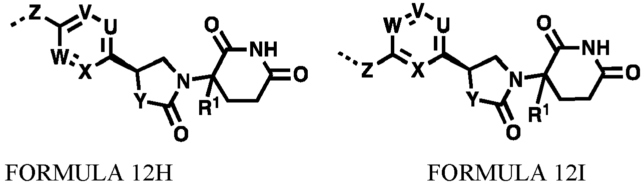 wherein
   U, V, W, and X are independently selected from CR² and N;
   Y is selected from CR³R⁴, NR³ and O; preferably, Y is selected from CH₂, NH, NCH₃ and O;
   Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
   R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
   R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
   R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
**(III)** a moiety according to FORMULA 13A: 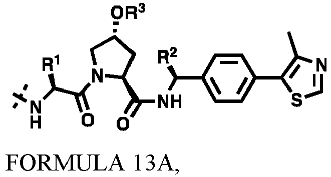 wherein
   R¹ and R² are independently selected from hydrogen, optionally substituted Ci-Cs alkyl, optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; and
   R³ is hydrogen, optionally substituted C(O)C₁-C₈ alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC₁-C₈alkoxyC₁-C₈alkyl, optionally substituted C(O)NC₁-C₈ haloalkyl, optionally substituted C(O)NC₁-C₈ hydroxyalkyl, optionally substituted C(O)NCi-C₈ aminoalkyl, optionally substituted C(O)NC₁-C₈alkylaminoC₁-C₈alkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂;
**(IV)** a moiety according to FORMULAE 13B, 13C, 13D, 13E and 13F: 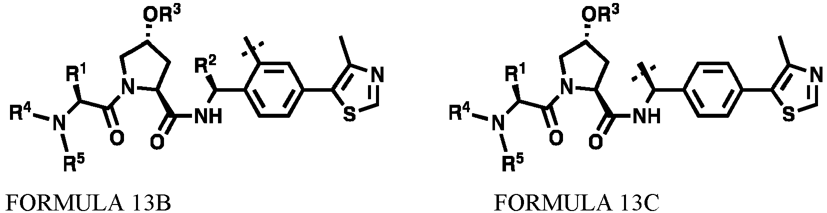 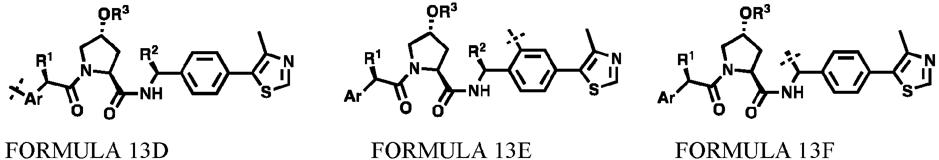 wherein
   R¹ and R² are independently selected from hydrogen, halogen, OH, NH₂, CN, optionally substituted C₁-C₈ alkyl, optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; (preferably, R¹ is selected from iso-propyl or tert-butyl; and R² is selected from hydrogen or methyl);
   R³ is hydrogen, optionally substituted C(O)C₁-C₈ alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC₁-C₈alkoxyC₁-C₈alkyl, optionally substituted C(O)NC₁-C₈ haloalkyl, optionally substituted C(O)NC₁-C₈ hydroxyalkyl, optionally substituted C(O)NCi-C₈ aminoalkyl, optionally substituted C(O)NC₁-C₈alkylaminoC₁-C₈alkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂; and
      R⁴ and R⁵ are independently selected from hydrogen, COR⁶, CO₂R⁶, CONR⁶R⁷, SOR⁶, SO₂R⁶, SO₂NR⁶R⁷, optionally substituted C₁-C₈ alkyl, optionally substituted C₁-C₈alkoxyC₁-Csalkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; wherein
      R⁶ and R⁷ are independently selected from hydrogen, optionally substituted C₁-C₈ alkyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; or
      R⁴ and R⁵; R⁶ and R⁷ together with the atom to which they are connected form a 4-8 membered cycloalkyl or heterocyclyl ring;
      Ar is selected from aryl and heteroaryl, each of which is optionally substituted with one or more substituents independently selected from F, Cl, CN, NO₂, OR⁸, NR⁸R⁹, COR⁸, CO₂R⁸, CONR⁸R⁹, SOR⁸, SO₂R⁸, SO₂NR⁹R¹⁰, NR⁹COR¹⁰, NR⁸C(O)NR⁹R¹⁰, NR⁹SOR¹⁰, NR⁹SO₂R¹⁰, optionally substituted C₁-C₆ alkyl, optionally substituted C₁-C₆ alkoxyalkyl, optionally substituted C₁-C₆ haloalkyl, optionally substituted C₁-C₆ hydroxyalkyl, optionally substituted C₁-C₆alkylaminoC₁-C₆alkyl, optionally substituted C₃-C₇ cycloalkyl, optionally substituted 3-7 membered heterocyclyl, optionally substituted C₂-C₆ alkenyl, optionally substituted C₂-C₆ alkynyl, optionally substituted aryl, and optionally substituted C₄-C₅ heteroaryl; wherein
      R⁸, R⁹, and R¹⁰ are independently selected from null, hydrogen, optionally substituted C₁-C₆ alkyl, optionally substituted C₂-C₆ alkenyl, optionally substituted C₂-C₆ alkynyl, optionally substituted C₃-C₇ cycloalkyl, optionally substituted 3-7 membered heterocyclyl, optionally substituted aryl, and optionally substituted heteroaryl; or
      R⁸ and R⁹; R⁹ and R¹⁰ together with the atom to which they are connected form a 4-8 membered cycloalkyl or heterocyclyl ring;
   and pharmaceutically acceptable salts thereof;

In an embodiment, degradation/disruption tags are selected from the group consisting of: 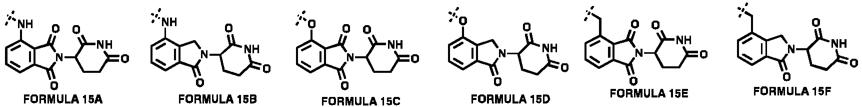 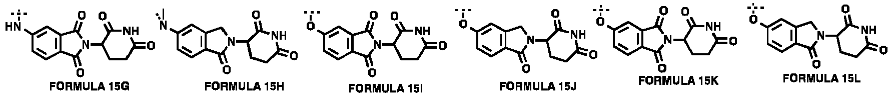 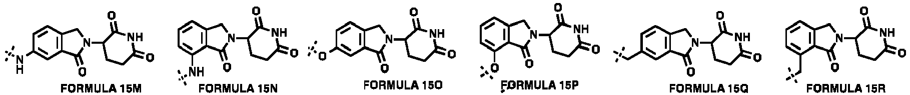 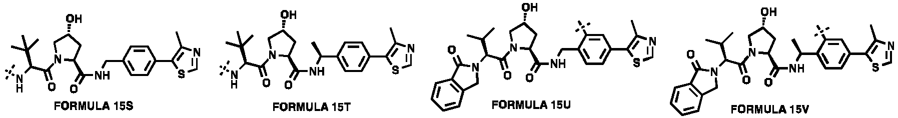 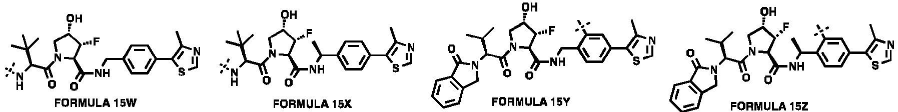 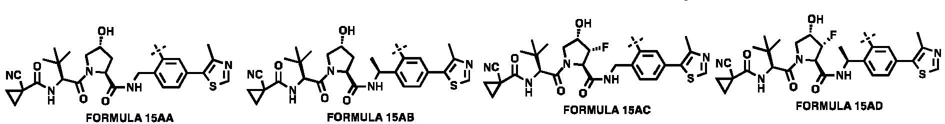 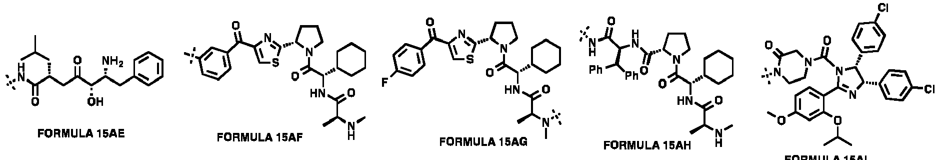 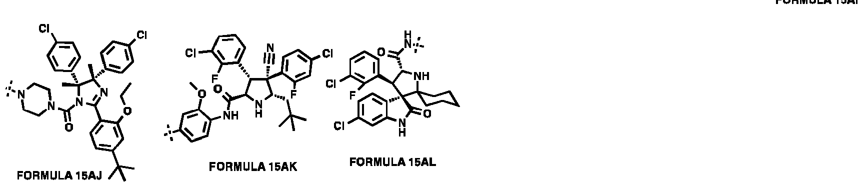 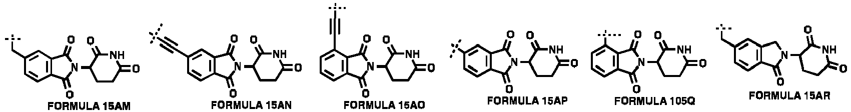 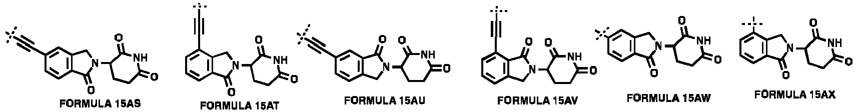 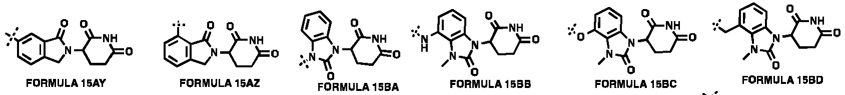 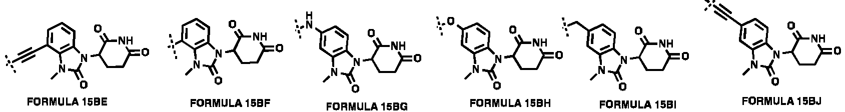 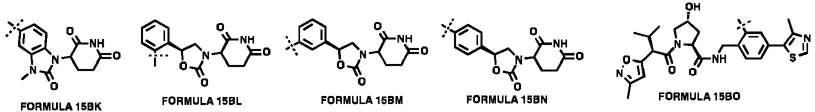 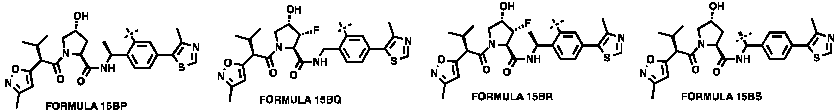 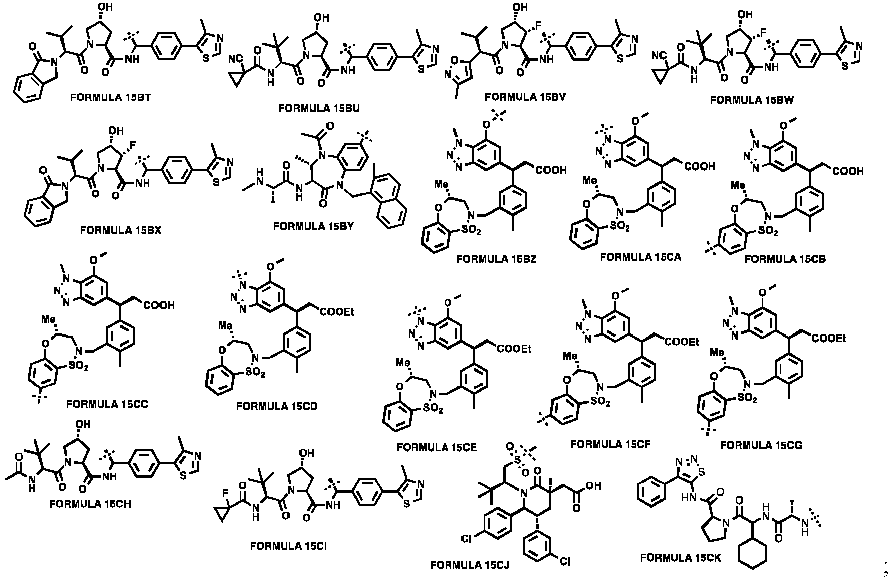 and pharmaceutically acceptable salts thereof.

### LINKERS

In any of the above-described compounds, the HPK1 ligand can be conjugated to the degradation/disruption tag through a linker. The linker can include, e.g., acyclic or cyclic saturated or unsaturated carbon, ethylene glycol, amide, amino, ether, urea, carbamate, aromatic, heteroaromatic, heterocyclic, and/or carbonyl containing groups with different lengths.

The linker of the present invention is:
**(I)** a moiety selected from the group consisting of:
   a moiety according to FORMULA 16: 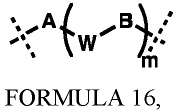 wherein
      A, W, and B, at each occurrence, are independently selected from null, CO, CO₂, C(O)NR¹, C(S)NR¹, O, S, SO, SO₂, SO₂NR¹, NR¹, NR¹CO, NR¹CONR², NR¹C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
      R¹ and R² are independently selected from hydrogen, optionally substituted C₁-C₈ alkyl, optionally substituted C₃-C₈ cycloalkyl, optionally substituted 3-8 membered cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl; and
      m is 0 to 15;
   a moiety according to FORMULA 16A: 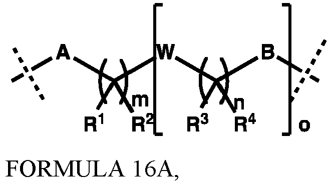 wherein
      R¹, R², R³, and R⁴, at each occurrence, are independently selected from hydrogen, halogen, CN, OH, NH₂, optionally substituted C₁-C₈ alkyl, optionally substituted C₃-C₈ cycloalkyl, optionally substituted C₃-C₈ cycloalkoxy, optionally substituted 3-8 membered membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
      A, W, and B, at each occurrence, are independently selected from null, CO, CO₂, C(O)NR⁵, C(S)NR⁵, O, S, SO, SO₂, SO₂NR⁵, NR⁵, NR⁵CO, NR⁵CONR⁶, NR⁵C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
   R⁵ and R⁶ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
      m is 0 to 15;
      n, at each occurrence, is 0 to 15; and
      o is 0 to 15;
   a moiety according to FORMULA 16B: 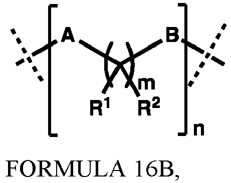 wherein
      R¹ and R², at each occurrence, are independently selected from hydrogen, halogen, CN, OH, NH₂, and optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
      A and B, at each occurrence, are independently selected from null, CO, CO₂, C(O)NR³, C(S)NR³, O, S, SO, SO₂, SO₂NR³, NR³, NR³CO, NR³CONR⁴, NR³C(S), and optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, or C₃-C₁₃ spiro heterocyclyl; wherein
      R³ and R⁴ are independently selected from hydrogen, and optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
      each m is 0 to 15; and
      n is 0 to 15;
   a moiety according to FORMULA 16C: 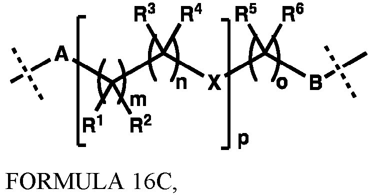 wherein
      X is selected from O, NH, and NR⁷;
      R¹, R², R³, R⁴, R⁵, and R⁶, at each occurrence, are independently selected from hydrogen, halogen, CN, OH, NH₂, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
      A and B, at each occurrence, are independently selected from null, CO, NH, NH-CO, CO-NH, CH₂-NH-CO, CH₂-CO-NH, NH-CO-CH₂, CO-NH-CH₂, CH₂-NH-CH₂-CO-NH, CH₂-NH-CH₂-NH-CO, -CO-NH, CO-NH- CH₂-NH-CH₂, CH₂-NH-CH₂, CO₂, C(O)NR⁷, C(S)NR⁷, O, S, SO, SO₂, SO₂NR⁷, NR⁷, NR⁷CO, NR⁷CONR⁸, NR⁷C(S), optionally substituted C₁-C₈ alkyl, optionally substituted C₁-C₈ alkoxy, optionally substituted C₁-C₈alkoxyC₁-C₈alkyl, optionally substituted C₁-Cs haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
      R⁷ and R⁸ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
      m, at each occurrence, is 0 to 15;
      n, at each occurrence, is 0 to 15;
      o is 0 to 15; and
      p is 0 to 15; and
      pharmaceutically acceptable salts thereof; or
**(II)** a moiety selected from the group consisting of: a ring selected from the group consisting of a 3 to 13 membered ring; a 3 to 13 membered fused ring; a 3 to 13 membered bridged ring; and a 3 to 13 membered spiro ring; and pharmaceutically acceptable salts thereof; or
**(III)** a moiety selected from the group consisting of one of FORMULAE C1, C2, C3, C4 and C5: 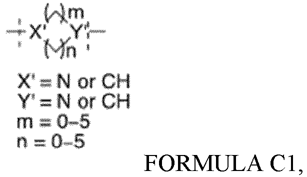 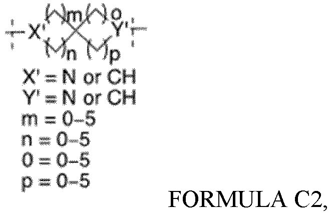 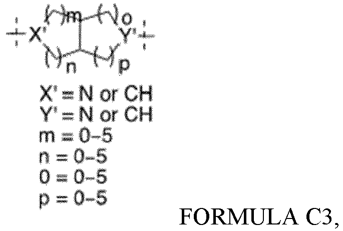 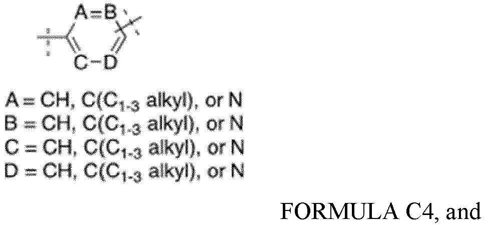 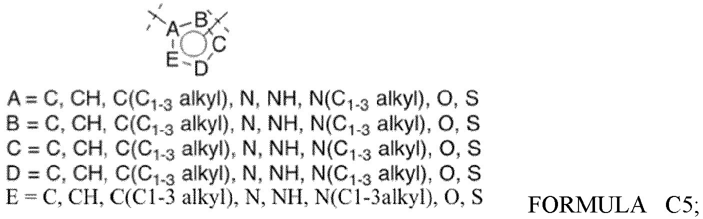 and pharmaceutically acceptable salts thereof.

### Synthesis and Testing of Bivalent Compounds

The binding affinity of novel synthesized bivalent compounds (i.e., HPK1 degraders/disruptors) can be assessed using standard biophysical assays known in the art (*e.g.,* isothermal titration calorimetry (ITC)). Cellular assays can then be used to assess the bivalent compound's ability to induce HPK1 degradation and inhibit cancer cell proliferation. Besides evaluating bivalent compound's -induced changes in the protein expression of HPK1, enzymatic activity can also be assessed. Assays suitable for use in any or all of these steps are known in the art, and include, e.g., Western blotting, quantitative mass spectrometry (MS) analysis, flow cytometry, enzymatic inhibition, ITC, SPR, cell growth inhibition and xenograft and PDX models. Suitable cell lines for use in any or all of these steps are known in the art and include, *e.g.,* HPK1 - deficient Jurkat clone that had been created by CRISPR-mediated frameshift mutation that resulted in the loss of HPK1 expression in Jurkat T cells. Such line could serve as a platform for counter screening the lead HPK1 degraders/disruptors for non-HPK1-specific effects. By way of non-limiting example, detailed synthesis protocols are described in the Examples for specific exemplary HPK1 degraders/disruptors.

Pharmaceutically acceptable isotopic variations of the compounds disclosed herein are contemplated and can be synthesized using conventional methods known in the art or methods corresponding to those described in the Examples (substituting appropriate reagents with appropriate isotopic variations of those reagents). Specifically, an isotopic variation is a compound in which at least one atom is replaced by an atom having the same atomic number, but an atomic mass different from the atomic mass usually found in nature. Useful isotopes are known in the art and include, for example, isotopes of hydrogen, carbon, nitrogen, oxygen, phosphorus, sulfur, fluorine, and chlorine. Exemplary isotopes thus include, *e.g.,* ²H, ³H, ¹³C, ¹⁴C, ¹⁵N, ¹⁷O, ¹⁸O, ³²P, ³⁵S, ¹⁸F, and ³⁶Cl.

Isotopic variations (*e.g.,* isotopic variations containing ²H) can provide therapeutic advantages resulting from greater metabolic stability, *e.g.,* increased *in vivo* half-life or reduced dosage requirements. In addition, certain isotopic variations (particularly those containing a radioactive isotope) can be used in drug or substrate tissue distribution studies. The radioactive isotopes tritium (³H) and carbon-14 (¹⁴C) are particularly useful for this purpose in view of their ease of incorporation and ready means of detection.

Pharmaceutically acceptable solvates of the compounds disclosed herein are contemplated. A solvate can be generated, *e.g.,* by substituting a solvent used to crystallize a compound disclosed herein with an isotopic variation (*e.g.,* D₂O in place of H₂O, *d*₆-acetone in place of acetone, or *d*₆-DMSO in place of DMSO).

Pharmaceutically acceptable fluorinated variations of the compounds disclosed herein are contemplated and can be synthesized using conventional methods known in the art or methods corresponding to those described in the Examples (substituting appropriate reagents with appropriate fluorinated variations of those reagents). Specifically, a fluorinated variation is a compound in which at least one hydrogen atom is replaced by a fluoro atom. Fluorinated variations can provide therapeutic advantages resulting from greater metabolic stability, *e.g.,* increased *in vivo* half-life or reduced dosage requirements

### Characterization of Exemplary HPK1 Degraders/Disruptors

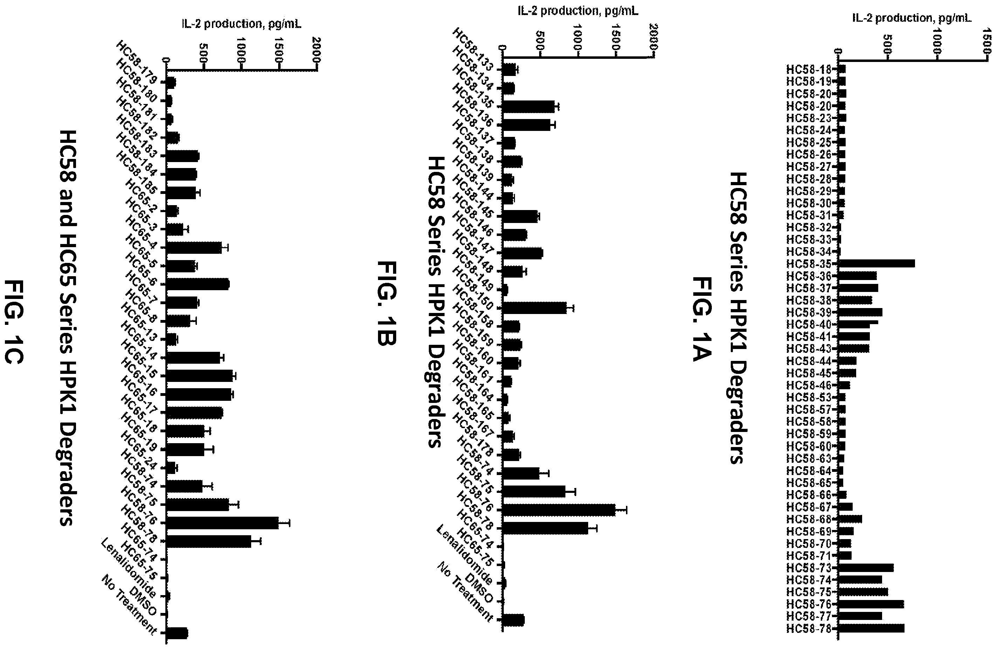

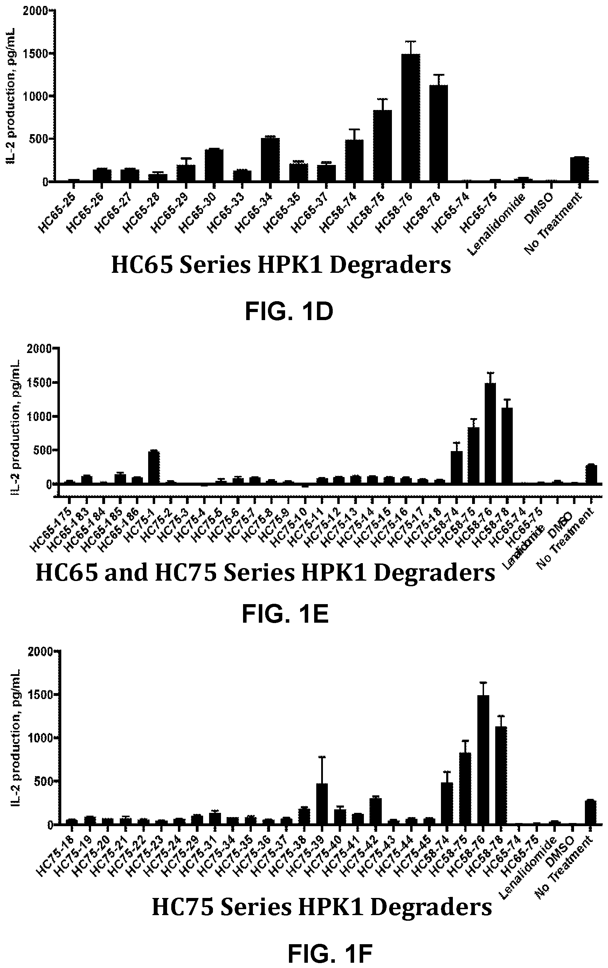

Specific exemplary HPK1 degraders/disruptors were characterized using (Figures 1-14). HC58-38, HC58-75, HC58-76, HC58-78, HC90-50, and HC90-51 in particular were found to be especially effective in reducing HPK1 protein levels.

### Definition of Terms

As used herein, the terms "comprising" and "including" are used in their open, non-limiting sense.

"Alkyl" refers to a straight or branched hydrocarbon chain radical consisting solely of carbon and hydrogen atoms, containing no unsaturation. An alkyl may comprise one, two, three, four, five, six, seven, eight, nine, ten, eleven, twelve, thirteen, fourteen, fifteen, or sixteen carbon atoms. In certain embodiments, an alkyl comprises one to fifteen carbon atoms (*e.g.,* C₁-C₁₅ alkyl). In certain embodiments, an alkyl comprises one to thirteen carbon atoms (*e.g.,* C₁-C₁₃ alkyl). In certain embodiments, an alkyl comprises one to eight carbon atoms (*e.g.,* C₁-C₈ alkyl). In other embodiments, an alkyl comprises five to fifteen carbon atoms (*e.g.,* C₅-C₁₅ alkyl). In other embodiments, an alkyl comprises five to eight carbon atoms (*e.g.,* C₅-C₈ alkyl). The alkyl is attached to the rest of the molecule by a single bond, for example, methyl (Me), ethyl (Et), *n*-propyl, 1-methylethyl (*iso*-propyl), *n*-butyl, *n*-pentyl, 1,1-dimethylethyl (*t*-butyl), pentyl, 3-methylhexyl, 2-methylhexyl, and the like.

"Alkenyl" refers to a straight or branched hydrocarbon chain radical group consisting solely of carbon and hydrogen atoms, containing at least one double bond. An alkenyl may comprise two, three, four, five, six, seven, eight, nine, ten, eleven, twelve, thirteen, fourteen, fifteen, or sixteen carbon atoms. In certain embodiments, an alkenyl comprises two to twelve carbon atoms (*e.g.,* C₂-C₁₂ alkenyl). In certain embodiments, an alkenyl comprises two to eight carbon atoms (*e.g.,* C₂-C₈ alkenyl). In certain embodiments, an alkenyl comprises two to six carbon atoms *(e.g.,* C₂-C₆ alkenyl). In other embodiments, an alkenyl comprises two to four carbon atoms (*e.g.,* C₂-C₄ alkenyl). The alkenyl is attached to the rest of the molecule by a single bond, for example, ethenyl (*i.e.,* vinyl), prop-1-enyl (*i.e.,* allyl), but-1-enyl, pent-1-enyl, penta-1,4-dienyl, and the like.

The term "allyl," as used herein, means a -CH₂CH=CH₂ group.

As used herein, the term "alkynyl" refers to a straight or branched hydrocarbon chain radical group consisting solely of carbon and hydrogen atoms, containing at least one triple bond. An alkynyl may comprise two, three, four, five, six, seven, eight, nine, ten, eleven, twelve, thirteen, fourteen, fifteen, or sixteen carbon atoms. In certain embodiments, an alkynyl comprises two to twelve carbon atoms (*e.g.,* C₂-C₁₂ alkynyl). In certain embodiments, an alkynyl comprises two to eight carbon atoms (*e.g.,* C₂-C₈ alkynyl). In other embodiments, an alkynyl has two to six carbon atoms *(e.g.,* C₂-C₆ alkynyl). In other embodiments, an alkynyl has two to four carbon atoms (*e.g.,* C₂-C₄ alkynyl). The alkynyl is attached to the rest of the molecule by a single bond. Examples of such groups include, but are not limited to, ethynyl, propynyl, 1-butynyl, 2-butynyl, 1-pentynyl, 2-pentynyl, 1-hexynyl, 2-hexynyl, 3-hexynyl, and the like.

The term " alkoxy", as used herein, means an alkyl group as defined herein witch is attached to the rest of the molecule via an oxygen atom. Examples of such groups include, but are not limited to, methoxy, ethoxy, n-propyloxy, iso-propyloxy, n-butoxy, iso-butoxy, tert-butoxy, pentyloxy, hexyloxy, and the like.

The term "aryl", as used herein, refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon atoms. An aryl may comprise from six to eighteen carbon atoms, where at least one of the rings in the ring system is fully unsaturated, *i.e.,* it contains a cyclic, delocalized (4n+2) π-electron system in accordance with the Hückel theory. In certain embodiments, an aryl comprises six to fourteen carbon atoms (C₆-C₁₄ aryl). In certain embodiments, an aryl comprises six to ten carbon atoms (C₆-C₁₀ aryl). Examples of such groups include, but are not limited to, phenyl, fluorenyl and naphthyl. The terms "Ph" and "phenyl," as used herein, mean a -C₆H₅ group.

The term "heteroaryl", refers to a radical derived from a 3- to 18-membered aromatic ring radical that comprises two to seventeen carbon atoms and from one to six heteroatoms selected from nitrogen, oxygen and sulfur. As used herein, the heteroaryl radical may be a monocyclic, bicyclic, tricyclic or tetracyclic ring system, wherein at least one of the rings in the ring system is fully unsaturated, *i.e.,* it contains a cyclic, delocalized (4n+2) π-electron system in accordance with the Hückel theory. Heteroaryl includes fused or bridged ring systems. The heteroatom(s) in the heteroaryl radical is optionally oxidized. One or more nitrogen atoms, if present, are optionally quaternized. The heteroaryl is attached to the rest of the molecule through any atom of the ring(s). Examples of such groups include, but not limited to, pyridinyl, imidazolyl, pyrimidinyl, pyrazolyl, triazolyl,pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, furopyridinyl, and the like. In certain embodiments, an heteroaryl is attached to the rest of the molecule via a ring carbon atom. In certain embodiments, an heteroaryl is attached to the rest of the molecule via a nitrogen atom (N-attached) or a carbon atom (C-attached). For instance, a group derived from pyrrole may be pyrrol-1-yl (N-attached) or pyrrol-3-yl (C-attached). Further, a group derived from imidazole may be imidazol-1-yl (N-attached) or imidazol-3-yl (C-attached).

The term "heterocyclyl", as used herein, means a non-aromatic, monocyclic, bicyclic, tricyclic, or tetracyclic radical having a total of from 4, 5, 6, 7, 8, 9, 10, 11, 12, or 13 atoms in its ring system, and containing from 3 to 12 carbon atoms and from 1 to 4 heteroatoms each independently selected from O, S and N, and with the proviso that the ring of said group does not contain two adjacent O atoms or two adjacent S atoms. A heterocyclyl group may include fused, bridged or spirocyclic ring systems. In certain embodiments, a hetercyclyl group comprises 3 to 10 ring atoms (3-10 membered heterocyclyl). In certain embodiments, a hetercyclyl group comprises 3 to 8 ring atoms (3-8 membered heterocyclyl). In certain embodiments, a hetercyclyl group comprises 4 to 8 ring atoms (4-8 membered heterocyclyl). In certain embodiments, a hetercyclyl group comprises 3 to 6 ring atoms (3-6 membered heterocyclyl). A heterocyclyl group may contain an oxo substituent at any available atom that will result in a stable compound. For example, such a group may contain an oxo atom at an available carbon or nitrogen atom. Such a group may contain more than one oxo substituent if chemically feasible. In addition, it is to be understood that when such a heterocyclyl group contains a sulfur atom, said sulfur atom may be oxidized with one or two oxygen atoms to afford either a sulfoxide or sulfone. An example of a 4 membered heterocyclyl group is azetidinyl (derived from azetidine). An example of a 5 membered cycloheteroalkyl group is pyrrolidinyl. An example of a 6 membered cycloheteroalkyl group is piperidinyl. An example of a 9 membered cycloheteroalkyl group is indolinyl. An example of a 10 membered cycloheteroalkyl group is 4H-quinolizinyl. Further examples of such heterocyclyl groups include, but are not limited to, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4*H*-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3*H*-indolyl, quinolizinyl, 3-oxopiperazinyl, 4-methylpiperazinyl, 4-ethylpiperazinyl, and 1-oxo-2,8,diazaspiro[4.5]dec-8-yl. A heteroaryl group may be attached to the rest of molecular via a carbon atom (C-attached) or a nitrogen atom (N-attached). For instance, a group derived from piperazine may be piperazin-1-yl (N-attached) or piperazin-2-yl (C-attached).

The term " cycloalkyl" means a saturated, monocyclic, bicyclic, tricyclic, or tetracyclic radical having a total of from 4, 5, 6, 7, 8, 9, 10, 11, 12, or 13 carbon atoms in its ring system. A cycloalkyl may be fused, bridged or spirocyclic. In certain embodiments, a cycloalkyl comprises 3 to 8 carbon ring atoms (C₃-C₈ cycloalkyl). In certain embodiments, a cycloalkyl comprises 3 to 6 carbon ring atoms (C₃-C₆ cycloalkyl). Examples of such groups include, but are not limited to, cyclopropyl, cyclobutyl, cyclopentyl, cyclopentenyl, cyclohexyl, cycloheptyl, adamantyl, and the like.

The term "cycloalkylene" is a bidentate radical obtained by removing a hydrogen atom from a cycloalkyl ring as defined above. Examples of such groups include, but are not limited to, cyclopropylene, cyclobutylene, cyclopentylene, cyclopentenylene, cyclohexylene, cycloheptylene, and the like.

The term "spirocyclic" as used herein has its conventional meaning, that is, any ring system containing two or more rings wherein two of the rings have one ring carbon in common. Each ring of the spirocyclic ring system, as herein defined, independently comprises 3 to 20 ring atoms. Preferably, they have 3 to 10 ring atoms. Non-limiting examples of a spirocyclic system include spiro[3.3]heptane, spiro[3.4]octane, and spiro[4.5]decane.

The term cyano" refers to a -C=N group.

An "aldehyde" group refers to a -C(O)H group.

An "alkoxy" group refers to both an -O-alkyl, as defined herein.

An "alkoxycarbonyl" refers to a -C(O)-alkoxy, as defined herein.

An "alkylaminoalkyl" group refers to an -alkyl-NR-alkyl group, as defined herein.

An "alkylsulfonyl" group refer to a -SO₂alkyl, as defined herein.

An "amino" group refers to an optionally substituted -NH₂.

An "aminoalkyl" group refers to an -alky-amino group, as defined herein.

An "aminocarbonyl" refers to a -C(O)-amino, as defined herein.

An "arylalkyl" group refers to -alkylaryl, where alkyl and aryl are defined herein.

An "aryloxy" group refers to both an -O-aryl and an -O-heteroaryl group, as defined herein.

An "aryloxycarbonyl" refers to -C(O)-aryloxy, as defined herein.

An "arylsulfonyl" group refers to a -SO₂aryl, as defined herein.

A "carbonyl" group refers to a -C(O)- group, as defined herein.

A "carboxylic acid" group refers to a -C(O)OH group.

A "cycloalkoxy" refers to a -O-cycloalkyl group, as defined herein.

A "halo" or "halogen" group refers to fluorine, chlorine, bromine or iodine.

A "haloalkyl" group refers to an alkyl group substituted with one or more halogen atoms.

A "hydroxy" group refers to an -OH group.

A "nitro" group refers to a -NO₂ group.

An "oxo" group refers to the =O substituent.

A "trihalomethyl" group refers to a methyl substituted with three halogen atoms.

The term "substituted," means that the specified group or moiety bears one or more substituents independently selected from C₁-C₄ alkyl, aryl, heteroaryl, aryl-C₁-C₄ alkyl-, heteroaryl-C₁-C₄ alkyl-, C₁-C₄ haloalkyl, -OC₁-C₄ alkyl, -OC₁-C₄ alkylphenyl, -C₁-C₄ alkyl-OH, -OC₁-C₄ haloalkyl, halo, -OH, -NH₂, -C₁-C₄ alkyl-NH₂, -N(C₁-C₄ alkyl)(C₁-C₄ alkyl), -NH(C₁-C₄ alkyl), -N(C₁-C₄ alkyl)(C₁-C₄ alkylphenyl), -NH(C₁-C₄ alkylphenyl), cyano, nitro, oxo, -CO₂H, -C(O)OC₁-C₄ alkyl, -CON(C₁-C₄ alkyl)(C₁-C₄ alkyl), -CONH(C₁-C₄ alkyl), -CONH₂, -NHC(O)(C₁-C₄ alkyl), -NHC(O)(phenyl), -N(C₁-C₄ alkyl)C(O)(C₁-C₄ alkyl), -N(C₁-C₄ alkyl)C(O)(phenyl), -C(O)C₁-C₄ alkyl, -C(O)C₁-C₄ alkylphenyl, -C(O)C₁-C₄ haloalkyl, -OC(O)C₁-C₄ alkyl, -SO₂(C₁-C₄ alkyl), -SO₂(phenyl), -SO₂(C₁-C₄ haloalkyl), -SO₂NH₂, -SO₂NH(C₁-C₄ alkyl), -SO₂NH(phenyl), -NHSO₂(C₁-C₄ alkyl), -NHSO₂(phenyl), and -NHSO₂(C₁-C₄ haloalkyl).

The term "optionally substituted" means that the specified group may be either unsubstituted or substituted by one or more substituents as defined herein. It is to be understood that in the compounds of the present invention when a group is said to be "unsubstituted," or is "substituted" with fewer groups than would fill the valencies of all the atoms in the compound, the remaining valencies on such a group are filled by hydrogen. For example, if a C₆ aryl group, also called "phenyl" herein, is substituted with one additional substituent, one of ordinary skill in the art would understand that such a group has 4 open positions left on carbon atoms of the C₆ aryl ring (6 initial positions, minus one at which the remainder of the compound of the present invention is attached to and an additional substituent, remaining 4 positions open). In such cases, the remaining 4 carbon atoms are each bound to one hydrogen atom to fill their valencies. Similarly, if a C₆ aryl group in the present compounds is said to be "disubstituted," one of ordinary skill in the art would understand it to mean that the C₆ aryl has 3 carbon atoms remaining that are unsubstituted. Those three unsubstituted carbon atoms are each bound to one hydrogen atom to fill their valencies.

"Pharmaceutically acceptable salt" includes both acid and base addition salts. A pharmaceutically acceptable salt of any one of the bivalent compounds described herein is intended to encompass any and all pharmaceutically suitable salt forms. Preferred pharmaceutically acceptable salts of the compounds described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S.M. et al., "Pharmaceutical Salts," Journal of Pharmaceutical Science, 66:1-19 (1997)). Acid addition salts of basic compounds may be prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts may be formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, *N,N*-dibenzylethylenediamine, chloroprocaine, hydrabamine, choline, betaine, ethylenediamine, ethylenedianiline, N-methylglucamine, glucosamine, methylglucamine, theobromine, purines, piperazine, piperidine, N ethylpiperidine, polyamine resins and the like. See Berge et al., *supra.*

### Pharmaceutical Compositions

In some aspects, the present invention includes pharmaceutical compositions and medicaments that include one or more bivalent compounds as disclosed herein for use in the methods described herein. Also included are the pharmaceutical compositions themselves.

In some aspects, the compositions disclosed herein can include other compounds, drugs, or agents used for the treatment of cancer. For example, in some instances, pharmaceutical compositions disclosed herein can be combined with one or more *(e.g.,* one, two, three, four, five, or less than ten) compounds. Such additional compounds can include, *e.g.,* conventional chemotherapeutic agents known in the art. When co-administered, HPK1 degraders/disruptors disclosed herein can operate in conjunction with conventional chemotherapeutic agents to produce mechanistically additive or synergistic therapeutic effects.

In some aspects, the pH of the compositions disclosed herein can be adjusted with pharmaceutically acceptable acids, bases, or buffers to enhance the stability of the HPK1 degraders/disruptor or its delivery form.

Pharmaceutical compositions typically include a pharmaceutically acceptable carrier, adjuvant, or vehicle. As used herein, the phrase "pharmaceutically acceptable" refers to molecular entities and compositions that are generally believed to be physiologically tolerable and do not typically produce an allergic or similar untoward reaction, such as gastric upset, dizziness and the like, when administered to a human. A pharmaceutically acceptable carrier, adjuvant, or vehicle is a composition that can be administered to a patient, together with a compound of the invention, and which does not destroy the pharmacological activity thereof and is nontoxic when administered in doses sufficient to deliver a therapeutic amount of the compound. Exemplary conventional nontoxic pharmaceutically acceptable carriers, adjuvants, and vehicles include saline, solvents, dispersion media, coatings, antibacterial and antifungal agents, isotonic and absorption delaying agents, and the like, compatible with pharmaceutical administration.

In particular, pharmaceutically acceptable carriers, adjuvants, and vehicles that can be used in the pharmaceutical compositions of this invention include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, may also be advantageously used to enhance delivery of compounds of the formulae described herein.
As used herein, the HPK1 degraders/disruptors disclosed herein are defined to include pharmaceutically acceptable derivatives thereof. A "pharmaceutically acceptable derivative" means any pharmaceutically acceptable salt, which upon administration to a recipient is capable of providing (directly or indirectly) a compound described herein, or an active metabolite or residue thereof. Particularly favored derivatives are those that increase the bioavailability of the compounds disclosed herein when such compounds are administered to a mammal (e.g., by allowing an orally administered compound to be more readily absorbed into the blood) or which enhance delivery of the parent compound to a biological compartment (e.g., the brain or lymphatic system) relative to the parent species. Reference is made to the teaching of Burger's Medicinal Chemistry and Drug Discovery, 5th Edition, Vol. 1: Principles and Practice.

The HPK1 degraders/disruptors disclosed herein include pure enantiomers, mixtures of enantiomers, pure diastereoisomers, mixtures of diastereoisomers, diastereoisomeric racemates, mixtures of diastereoisomeric racemates and the meso-form and pharmaceutically acceptable salts, solvent complexes, morphological forms, or deuterated derivative thereof.

In particular, pharmaceutically acceptable salts of the HPK1 degraders/disruptors disclosed herein include, *e.g.,* those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate, trifluoromethylsulfonate, and undecanoate. Salts derived from appropriate bases include, *e.g.,* HPK1 alkali metal *(e.g.,* sodium), HPK1 alkaline earth metal (*e.g.,* magnesium), ammonium and N-(HPK1yl)4+ salts. The invention also envisions the quaternization of any basic nitrogen-containing groups of the HPK1 degraders/disruptors disclosed herein. Water or oil-soluble or dispersible products can be obtained by such quaternization.

In some aspects, the pharmaceutical compositions disclosed herein can include an effective amount of one or more HPK1 degraders/disruptors. The terms "effective amount" and "effective to treat," as used herein, refer to an amount or a concentration of one or more compounds or a pharmaceutical composition described herein utilized for a period of time (including acute or chronic administration and periodic or continuous administration) that is effective within the context of its administration for causing an intended effect or physiological outcome (*e.g.,* treatment or prevention of cell growth, cell proliferation, or cancer). In some aspects, pharmaceutical compositions can further include one or more additional compounds, drugs, or agents used for the treatment of cancer (*e.g.,* conventional chemotherapeutic agents) in amounts effective for causing an intended effect or physiological outcome (*e.g.,* treatment or prevention of cell growth, cell proliferation, or cancer).

In some aspects, the pharmaceutical compositions disclosed herein can be formulated for sale in the United States, import into the United States, or export from the United States.

### Administration of Pharmaceutical Compositions

The pharmaceutical compositions disclosed herein can be formulated or adapted for administration to a subject via any route, *e.g.,* any route approved by the Food and Drug Administration (FDA). Exemplary methods are described in the FDA Data Standards Manual (DSM) (available at http://www.fda.gov/Drugs/DevelopmentApprovalProcess/ FormsSubmissionRequirements/ElectronicSubmissions/DataStandardsManualmonographs). In particular, the pharmaceutical compositions can be formulated for and administered via oral, parenteral, or transdermal delivery. The term "parenteral" as used herein includes subcutaneous, intracutaneous, intravenous, intramuscular, intraperitoneal, intra-articular, intra-arterial, intrasynovial, intrasternal, intrathecal, intralesional, and intracranial injection or infusion techniques.

For example, the pharmaceutical compositions disclosed herein can be administered, *e.g.,* topically, rectally, nasally (*e.g.,* by inhalation spray or nebulizer), buccally, vaginally, subdermally *(e.g.,* by injection or via an implanted reservoir), or ophthalmically.

For example, pharmaceutical compositions of this invention can be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient may be suspended or dissolved in an oily phase is combined with emulsifying or suspending agents. If desired, certain sweetening, flavoring, or coloring agents can be added.

For example, the pharmaceutical compositions of this invention can be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax, and polyethylene glycols.

For example, the pharmaceutical compositions of this invention can be administered by nasal aerosol or inhalation. Such compositions are prepared according to techniques well-known in the art of pharmaceutical formulation and can be prepared as solutions in saline, employing benzyl alcohol or other suitable preservatives, absorption promoters to enhance bioavailability, fluorocarbons, or other solubilizing or dispersing agents known in the art.

For example, the pharmaceutical compositions of this invention can be administered by injection (*e.g.,* as a solution or powder). Such compositions can be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, *e.g.,* as a solution in 1,3-butanediol. Among the acceptable vehicles and solvents that may be employed are mannitol, water, Ringer's solution, and isotonic sodium chloride solution. In addition, sterile, fixed oils are conventionally employed as a solvent or suspending medium. For this purpose, any bland fixed oil can be employed, including synthetic mono- or diglycerides. Fatty acids, such as oleic acid and its glyceride derivatives are useful in the preparation of injectables, as are natural pharmaceutically-acceptable oils, *e.g.,* olive oil or castor oil, especially in their polyoxyethylated versions. These oil solutions or suspensions can also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens, Spans, or other similar emulsifying agents or bioavailability enhancers which are commonly used in the manufacture of pharmaceutically acceptable solid, liquid, or other dosage forms can also be used for the purposes of formulation.

In some aspects, an effective dose of a pharmaceutical composition of this invention can include, but is not limited to, *e.g.,* about 0.00001, 0.0001, 0.001, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.25, 1.5, 1.75, 2, 2.5, 3, 4, 5, 6, 7, 8, 9, 10, 15, 20, 30, 40, 50, 60, 70, 80, 90, 100, 200, 300, 400, 500, 600, 700, 800, 900, 1000, 2500, 5000, or 10000 mg/kg/day, or according to the requirements of the particular pharmaceutical composition.

When the pharmaceutical compositions disclosed herein include a combination of a compound of the formulae described herein *(e.g.,* a HPK1 degraders/disruptors) and one or more additional compounds (*e.g.,* one or more additional compounds, drugs, or agents used for the treatment of cancer or any other condition or disease, including conditions or diseases known to be associated with or caused by cancer), both the compound and the additional compound should be present at dosage levels of between about 1 to 100%, and more preferably between about 5 to 95% of the dosage normally administered in a monotherapy regimen. The additional agents can be administered separately, as part of a multiple dose regimen, from the compounds of this invention. Alternatively, those agents can be part of a single dosage form, mixed together with the compounds of this invention in a single composition.

In some aspects, the pharmaceutical compositions disclosed herein can be included in a container, pack, or dispenser together with instructions for administration.

### Methods of Treatment

The methods disclosed herein contemplate administration of an effective amount of a compound or composition to achieve the desired or stated effect. Typically, the compounds or compositions of the invention will be administered from about 1 to about 6 times per day or, alternately or in addition, as a continuous infusion. Such administration can be used as a chronic or acute therapy. The amount of active ingredient that can be combined with the carrier materials to produce a single dosage form will vary depending upon the host treated and the particular mode of administration. A typical preparation will contain from about 5% to about 95% active compound (w/w). Alternatively, such preparations can contain from about 20% to about 80% active compound.

In some aspects, the present disclosure provides compositions comprising a HPK1 degrader/disruptor, including pharmaceutical compositions (indicated below as 'X') disclosed herein for use in the following methods:
Substance X for use as a medicament in the treatment of one or more diseases or conditions disclosed herein (e.g., cancer, referred to in the following examples as `Y'); and substance X for use in the treatment of Y.

In some aspects, the methods disclosed include the administration of a therapeutically effective amount of one or more of the compounds or compositions described herein to a subject *(e.g.,* a mammalian subject, *e.g.,* a human subject) who is in need of, or who has been determined to be in need of, such treatment. In some aspects, the methods disclosed include selecting a subject and administering to the subject an effective amount of one or more of the compounds or compositions described herein, and optionally repeating administration as required for the prevention or treatment of cancer.

In some aspects, subject selection can include obtaining a sample from a subject (*e.g.,* a candidate subject) and testing the sample for an indication that the subject is suitable for selection. In some aspects, the subject can be confirmed or identified, *e.g.* by a health care professional, as having had or having a condition or disease. In some aspects, suitable subjects include, for example, subjects who have or had a condition or disease but that resolved the disease or an aspect thereof, present reduced symptoms of disease *(e.g.,* relative to other subjects *(e.g.,* the majority of subjects) with the same condition or disease), or that survive for extended periods of time with the condition or disease *(e.g.,* relative to other subjects *(e.g.,* the majority of subjects) with the same condition or disease), *e.g.,* in an asymptomatic state *(e.g.,* relative to other subjects *(e.g.,* the majority of subjects) with the same condition or disease). In some aspects, exhibition of a positive immune response towards a condition or disease can be made from patient records, family history, or detecting an indication of a positive immune response. In some aspects, multiple parties can be included in subject selection. For example, a first party can obtain a sample from a candidate subject and a second party can test the sample. In some aspects, subjects can be selected or referred by a medical practitioner (*e.g.,* a general practitioner). In some aspects, subject selection can include obtaining a sample from a selected subject and storing the sample or using the in the methods disclosed herein. Samples can include, *e.g.,* cells or populations of cells.

In some aspects, methods of treatment can include a single administration, multiple administrations, and repeating administration of one or more compounds disclosed herein as required for the prevention or treatment of the disease or condition from which the subject is suffering (*e.g.,* an HPK1-mediated cancer). In some aspects, methods of treatment can include assessing a level of disease in the subject prior to treatment, during treatment, or after treatment. In some aspects, treatment can continue until a decrease in the level of disease in the subject is detected.

The term "subject," as used herein, refers to any animal. In some instances, the subject is a mammal. In some instances, the term "subject," as used herein, refers to a human (*e.g.,* a man, a woman, or a child).

The terms "administer," "administering," or "administration," as used herein, refer to implanting, ingesting, injecting, inhaling, or otherwise absorbing a compound or composition, regardless of form. For example, the methods disclosed herein include administration of an effective amount of a compound or composition to achieve the desired or stated effect.

The terms "treat", "treating," or "treatment," as used herein, refer to partially or completely alleviating, inhibiting, ameliorating, or relieving the disease or condition from which the subject is suffering. This means any manner in which one or more of the symptoms of a disease or disorder *(e.g.,* cancer) are ameliorated or otherwise beneficially altered. As used herein, amelioration of the symptoms of a particular disorder (*e.g.,* cancer) refers to any lessening, whether permanent or temporary, lasting or transient that can be attributed to or associated with treatment by the compositions and methods of the present invention. In some aspects, treatment can promote or result in, for example, a decrease in the number of tumor cells *(e.g.,* in a subject) relative to the number of tumor cells prior to treatment; a decrease in the viability *(e.g.,* the average/mean viability) of tumor cells *(e.g.,* in a subject) relative to the viability of tumor cells prior to treatment; a decrease in the rate of growth of tumor cells; a decrease in the rate of local or distant tumor metastasis; or reductions in one or more symptoms associated with one or more tumors in a subject relative to the subject's symptoms prior to treatment.

As used herein, the term "treating cancer" means causing a partial or complete decrease in the rate of growth of a tumor, and/or in the size of the tumor and/or in the rate of local or distant tumor metastasis, and/or the overall tumor burden in a subject, and/or any decrease in tumor survival, in the presence of a degrader/disruptor (*e.g.,* an HPK1 degrader/disruptor) described herein.

The terms "prevent," "preventing," and "prevention," as used herein, shall refer to a decrease in the occurrence of a disease or decrease in the risk of acquiring a disease or its associated symptoms in a subject. The prevention may be complete, *e.g.,* the total absence of disease or pathological cells in a subject. The prevention may also be partial, such that the occurrence of the disease or pathological cells in a subject is less than, occurs later than, or develops more slowly than that which would have occurred without the present invention.

Exemplary type of cancers that could be prevented, or therapeutically treated by manipulation of HPK1 level by degraders/disruptors should include all solid and liquid cancers, including, but not limited to, cancers of the breast, respiratory tract, brain, reproductive organs, digestive tract, urinary tract, eye, liver, skin, head and neck, thyroid, parathyroid and their distant metastases. Examples of liquid cancers include lymphomas, sarcomas, and leukaemias. Listed below are the type of cancers that immunotherapy using HPK1 degraders/disruptors should be able to prevent or treat.

Examples of breast cancers include, but are not limited to, triple negative breast cancer, invasive ductal carcinoma, invasive lobular carcinoma, ductal carcinoma in situ, and lobular carcinoma in situ.

Examples of cancers of the respiratory tract include, but are not limited to, small-cell and non- small-cell lung carcinoma, as well as bronchial adenoma and pleuropulmonary blastoma.

Examples of brain cancers include, but are not limited to, brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to, prostate and testicular cancer.

Tumors of the female reproductive organs include, but are not limited to, endometrial, cervical, ovarian, vaginal, and vulvar cancer, as well as sarcoma of the uterus.

Examples of ovarian cancer include, but are not limited to, serous tumor, endometrioid tumor, mucinous cystadenocarcinoma, granulosa cell tumor, Sertoli-Leydig cell tumor and arrhenoblastoma.

Examples of cervical cancer include, but are not limited to, squamous cell carcinoma, adenocarcinoma, adenosquamous carcinoma, small cell carcinoma, neuroendocrine tumor, glassy cell carcinoma and villoglandular adenocarcinoma. Tumors of the digestive tract include, but are not limited to, anal, colon, colorectal, esophageal, gallbladder, gastric, pancreatic, rectal, small-intestine, and salivary gland cancers.

Examples of esophageal cancer include, but are not limited to, esophageal cell carcinomas and adenocarcinomas, as well as squamous cell carcinomas, leiomyosarcoma, malignant melanoma, rhabdomyosarcoma and lymphoma.

Examples of gastric cancer include, but are not limited to, intestinal type and diffuse type gastric adenocarcinoma.

Examples of pancreatic cancer include, but are not limited to, ductal adenocarcinoma, adenosquamous carcinomas and pancreatic endocrine tumors.

Example of tumors of the urinary tract include, but are not limited to, bladder, penile, kidney, renal pelvis, ureter, urethral and human papillary renal cancers.

Examples of kidney cancer include, but are not limited to, renal cell carcinoma, urothelial cell carcinoma, juxtaglomerular cell tumor (reninoma), angiomyolipoma, renal oncocytoma, Bellini duct carcinoma, clear-cell sarcoma of the kidney, mesoblastic nephroma and Wilms' tumor.

Examples of bladder cancer include, but are not limited to, transitional cell carcinoma, squamous cell carcinoma, adenocarcinoma, sarcoma and small cell carcinoma. Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Example of skin cancers include, but are not limited to, squamous cell carcinoma, Kaposi's sarcoma, malignant melanoma, Merkel cell skin cancer, and non-melanoma skin cancer.

Example of head-and-neck cancers include, but are not limited to, squamous cell cancer of the head and neck, laryngeal, hypopharyngeal, nasopharyngeal, oropharyngeal cancer, salivary gland cancer, lip and oral cavity cancer and squamous cell.

Example of lymphomas include, but are not limited to, AIDS-related lymphoma, non-Hodgkin's lymphoma, cutaneous T-cell lymphoma, Burkitt lymphoma, Hodgkin's disease, and lymphoma of the central nervous system.

Example of sarcomas include, but are not limited to, sarcoma of the soft tissue, osteosarcoma, malignant fibrous histiocytoma, lymphosarcoma, and rhabdomyosarcoma.

Eample of leukemias include, but are not limited to, acute myeloid leukemia, acute lymphoblastic leukemia, chronic lymphocytic leukemia, chronic myelogenous leukemia, and hairy cell leukemia.

The HPK1 degraders/disruptors should be able to treat the above cancer type as stand alone agent or used as agent in combination with existing standard of treatment therapy and other FDA-approved cancer therapy.

Therapeutic uses of HPK1 include diseases and therapies that are amenable to treatment by stimulation/augmentation of immune response, including the prolongation of immune responses during vaccination for immunizable diseases. Also, because HPK1 is expressed at high level in two other anatomical locations - brain and testes - the HPK1 degraers/disruptors should be able to treat or prevent diseases related to brain and testes that were caused by HPK1 or could be treated by HPK1 degraders/disruptors. These potential diseases include, but is not limited to, Alzheimer's disease, age-related dementia and infertility, regarless whether these possible diseases were caused by HPK1 or by other eithiological causes.

As used herein, the term "preventing a disease" (*e.g.,* preventing cancer) in a subject means for example, to stop the development of one or more symptoms of a disease in a subject before they occur or are detectable, *e.g.,* by the patient or the patient's doctor. A blood test that measure the level of HPK1 in each of the immune cell sub-types, which could be achieved by intracellular staining by anti-HPK1 antibody and analyze by clinical FACS analysis. Such detection method could identify immune cell type possess aberrant level of HPK1 and may signify that such patient might be a good candidate for HPK1 degraders/disruptors-based therapy. This detection of aberrant expression level of HPK1 may be an early warning biomarker that may indicate which patient may respond well to their disease conditions if HPK1 degraders/disruptors were to used as stand alone or as part of combination therapy. Preferably, the disease (*e.g.,* cancer) does not develop at all, i.e., no symptoms of the disease are detectable. However, it can also mean delaying or slowing of the development of one or more symptoms of the disease. Alternatively, or in addition, it can mean decreasing the severity of one or more subsequently developed symptoms.

Specific dosage and treatment regimens for any particular patient will depend upon a variety of factors, including the activity of the specific compound employed, the age, body weight, general health status, sex, diet, time of administration, rate of excretion, drug combination, the severity and course of the disease, condition or symptoms, the patient's disposition to the disease, condition or symptoms, and the judgment of the treating physician.

An effective amount can be administered in one or more administrations, applications or dosages. A therapeutically effective amount of a therapeutic compound (i.e., an effective dosage) depends on the therapeutic compounds selected. Moreover, treatment of a subject with a therapeutically effective amount of the compounds or compositions described herein can include a single treatment or a series of treatments. For example, effective amounts can be administered at least once. The compositions can be administered one from one or more times per day to one or more times per week; including once every other day. The skilled artisan will appreciate that certain factors can influence the dosage and timing required to effectively treat a subject, including but not limited to the severity of the disease or disorder, previous treatments, the general health or age of the subject, and other diseases present.

Following administration, the subject can be evaluated to detect, assess, or determine their level of disease. In some instances, treatment can continue until a change (*e.g.,* reduction) in the level of disease in the subject is detected. Upon improvement of a patient's condition *(e.g.,* a change (*e.g.,* decrease) in the level of disease in the subject), a maintenance dose of a compound, or composition disclosed herein can be administered, if necessary. Subsequently, the dosage or frequency of administration, or both, can be reduced, *e.g.,* as a function of the symptoms, to a level at which the improved condition is retained. Patients may, however, require intermittent treatment on a long-term basis upon any recurrence of disease symptoms.

The HPK1 degraders/disruptors disclosed herein include pure enantiomers, mixtures of enantiomers, pure diastereoisomers, mixtures of diastereoisomers, diastereoisomeric racemates, mixtures of diastereoisomeric racemates and the meso-form and pharmaceutically acceptable salts, solvent complexes, morphological forms, or deuterated and fluoro derivatives thereof.

### EXAMPLES

The following Examples describe the synthesis of exemplary HPK1 degrader/disrupter compounds according to the present invention.

### EXAMPLES

### Example 1: Synthesis of intermediate 2

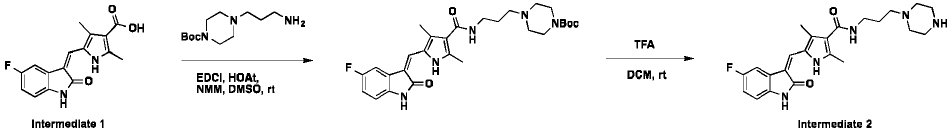

To a solution of Intermediate 1 (926 mg, 3.09 mmol) in DMSO (20 mL) were added tert-butyl 4-(3-aminopropyl)piperazine-1-carboxylate (899 mg, 3.70 mmol, 1.2 equiv), EDCI (1-ethyl-3-(3-dimethylaminopropyl)carbodiimide) (888 mg, 4.63 mmol, 1.5 equiv), HOAt (1-hydroxy-7-azabenzo-triazole) (630 mg, 4.63 mmol, 1.5 equiv), and NMM (N-Methylmorpholine) (625 mg, 6.18 mmol, 2.0 equiv). After being stirred overnight at room temperature, H₂O (100 mL) was added to the solution and precipitates was filtered. The solid was washed with water and dried under vacuum. The obtained solid was dissolved in DCM (10 mL). To the resulting solution was added TFA (5 ml). After being stirred for 1 h at room temperature, the reaction mixture was concentrated and the residue was purified by reverse phase C18 column (10% - 100% methanol / 0.1% TFA in H₂O) to afford intermediate 2 as yellow solid in TFA salt form (1.117 g, 55% yield for two step). ¹H NMR (600 MHz, DMSO-*d*₆) δ 13.72 (s, 1H), 10.93 (s, 1H), 7.80 - 7.75 (m, 2H), 7.73 (s, 1H), 6.94 (td, *J=* 9.1, 2.6 Hz, 1H), 6.86 (dd, *J=* 8.5, 4.5 Hz, 1H), 3.41 - 3.25 (m, 8H), 3.13 - 2.97 (m, 4H), 2.45 (s, 3H), 2.43 (s, 3H), 1.93 - 1.81 (m, 2H). ESI m/z =426.2 [M + H⁺].

### Example 2

### Synthesis of HC58-18

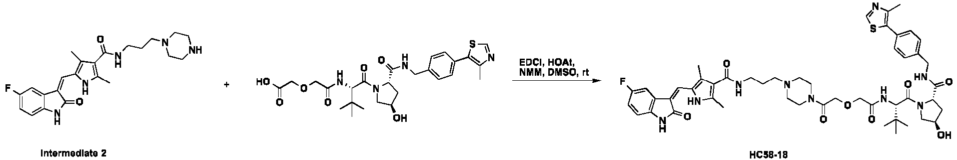

To a solution of Intermediate 2 (13.0 mg, 0.024 mmol, 1.2 equiv) in DMSO (1 mL) were added 2-(2-(((*S*)-1-((2*S*,4*R*)-4-hydroxy-2-((4-(4-methylthiazol-5-yl)benzyl)carbamoyl)pyrrolidin-l-yl)-3,3-dimethyl-1-oxobutan-2-yl)amino)-2-oxoethoxy)acetic acid (11 mg, 0.02 mmol, 1.0 equiv), EDCI (1-ethyl-3-(3-dimethylaminopropyl)carbodiimide) (5.8 mg, 0.03 mmol, 1.5 equiv), HOAt (1-hydroxy-7-azabenzo-triazole) (4.1 mg, 0.03 mmol, 1.5 equiv), and NMM (N-Methylmorpholine) (6.1 mg, 0.06 mmol, 3.0 equiv). After being stirred overnight at room temperature, the resulting mixture was purified by preparative HPLC (10%-100% methanol / 0.1% TFA in H₂O) to afford HC58-18 as white solid in TFA salt form (15.0 mg, 70%). ¹H NMR (600 MHz, Methanol-d4) δ 13.70 (s, 1H), 8.92 (s, 1H), 7.58 (s, 1H), 7.48 - 7.40 (m, 5H), 6.94 - 6.82 (m, 2H), 4.70 (s, 1H), 4.62 - 4.57 (m, 1H), 4.55 - 4.34 (m, 5H), 4.19 (d, J = 15.1 Hz, 1H), 4.11 (d, J = 15.0 Hz, 1H), 3.92 (d, J = 11.0 Hz, 1H), 3.83 (dd, J = 11.0, 3.8 Hz, 1H), 3.70 - 3.46 (m, 6H), 3.31 - 3.01 (m, 6H), 2.51 (s, 3H), 2.48 (s, 3H), 2.47 (s, 3H), 2.26 (dd, J = 13.3, 7.7 Hz, 1H), 2.15 - 2.02 (m, 3H), 1.08 (s, 9H). HRMS calcd for C₄₉H₆₁FN₉O₈S [M + H⁺] 954.4348, found 954.4363.

### Example 3

### Synthesis of HC58-19

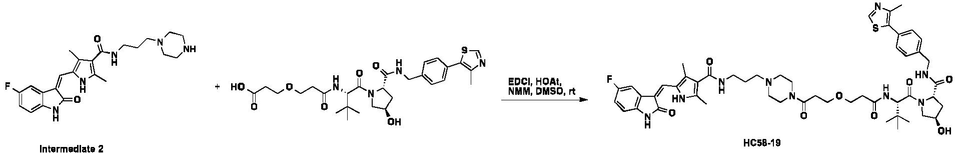

HC58-19 was synthesized following the standard procedure for preparing HC58-18 from Intermediate 2 (13.0 mg, 0.024 mmol, 1.2 equiv), 3-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)propanoic acid (11.5 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-19 was obtained as yellow solid in TFA salt form (13.8 mg, 63%). ¹H NMR (600 MHz, Methanol-*d*4) δ 13.69 (s, 1H), 8.94 (s, 1H), 7.56 (s, 1H), 7.42 (dt, *J=* 11.3, 7.2 Hz, 5H), 6.97 - 6.79 (m, 2H), 4.67 (s, 1H), 4.56 (t, *J=* 8.5 Hz, 1H), 4.51 (s, 1H), 4.47 (d, *J=* 15.5 Hz, 1H), 4.40 (d, *J=* 15.4 Hz, 1H), 3.91 (d, *J=* 11.0 Hz, 1H), 3.81 (dd, *J=* 11.0, 3.9 Hz, 1H), 3.79 - 3.57 (m, 8H), 3.53 (t, *J=* 6.5 Hz, 2H), 3.30 (t, *J* = 7.6 Hz, 3H), 3.23 - 2.98 (m, 4H), 2.55 (t, *J=* 5.8 Hz, 2H), 2.50 (s, 3H), 2.49 - 2.45 (m, 7H), 2.27 - 2.22 (m, 1H), 2.16 - 2.04 (m, 3H), 1.05 (s, 9H). HRMS calcd for C₅₁H₆₅FN₉O₈S [M + H⁺] 982.4661, found 982.4669.

### Example 4

### Synthesis of HC58-20

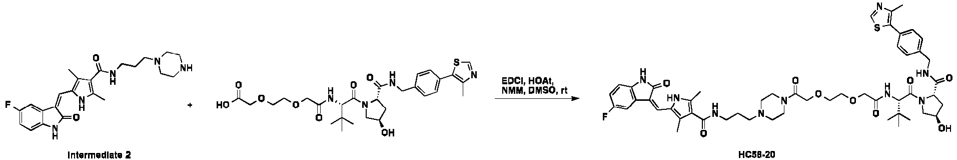

HC58-20 was synthesized following the standard procedure for preparing HC58-18 from Intermediate 2 (13.0 mg, 0.024 mmol, 1.2 equiv), 2-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)acetic acid (11.8 mg, 0.02 mmol, 1.0 equiv), EDCI (5.8 mg, 0.03 mmol, 1.5 equiv), HOAt (4.1 mg, 0.03 mmol, 1.5 equiv), andNMM(6.1 mg, 0.06 mmol, 3.0 equiv) in DMSO (1 mL). HC58-20 was obtained as yellow solid in TFA salt form (10.7 mg, 48%).¹H NMR (600 MHz, Methanol-*d*₄) δ 13.65 (s, 1H), 8.92 (s, 1H), 7.71 (d, *J=* 9.5 Hz, 1H), 7.52 (s, 1H), 7.46 - 7.33 (m, 6H), 6.95 - 6.78 (m, 2H), 4.76 - 4.70 (m, 1H), 4.62 - 4.55 (m, 1H), 4.51 (s, 1H), 4.42 (s, 2H), 4.08 (s, 2H), 3.90 (d, *J=* 11.1 Hz, 1H), 3.82 (dd, *J=* 11.1, 3.6 Hz, 1H), 3.80 - 3.72 (m, 6H), 3.68 - 3.45 (m, 6H), 3.31 - 3.02 (m, 6H), 2.49 (s, 3H), 2.47 (s, 3H), 2.44 (s, 3H), 2.29 - 2.22 (m, 1H), 2.16 - 2.03 (m, 3H), 1.06 (s, 9H). HRMS calcd for C₅₁H₆₅FN₉O₉S [M + H⁺] 998.4610, found 998.4625.

### Example 5

### Synthesis of HC58-22

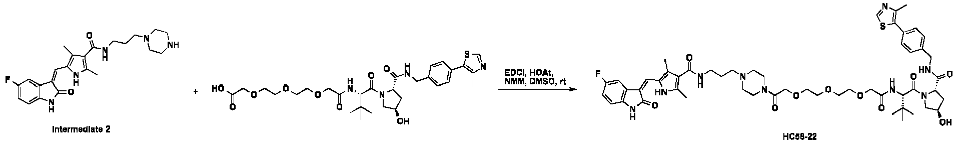

HC58-22 was synthesized following the standard procedure for preparing HC58-18 from Intermediate 2 (13.0 mg, 0.024 mmol, 1.2 equiv), (*S*)-13-((2*S*,4*R*)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecanoic acid (12.7 mg, 0.02 mmol, 1.0 equiv), EDCI (5.8 mg, 0.03 mmol, 1.5 equiv), HOAt (4.1 mg, 0.03 mmol, 1.5 equiv), andNMM(6.1 mg, 0.06 mmol, 3.0 equiv) in DMSO (1 mL). HC58-22 was obtained as yellow solid in TFA salt form (9.9 mg, 43%). ¹H NMR (600 MHz, Methanol-*d*₄) δ 13.68 (s, 1H), 8.98 (s, 1H), 7.71 (d, *J* = 9.3 Hz, 1H), 7.56 (s, 1H), 7.45 (d, *J* = 8.2 Hz, 2H), 7.43 - 7.39 (m, 3H), 6.89 - 6.83 (m, 2H), 4.72 - 4.68 (m, 1H), 4.62 - 4.49 (m, 3H), 4.39 (d, *J* = 15.4 Hz, 1H), 4.36 - 4.21 (m, 2H), 4.09 (s, 2H), 3.90 (d, *J* = 11.1 Hz, 1H), 3.81 (dd,*J* = 11.0, 3.7 Hz, 1H), 3.78 - 3.45 (m, 14H), 3.28 (t, *J=* 7.6 Hz, 2H), 3.24 - 3.03 (m, 4H), 2.51 (s, 3H), 2.49 (s, 3H), 2.47 (s, 3H), 2.29 - 2.22 (m, 1H), 2.10 (tt, *J=* 8.4, 4.3 Hz, 3H), 1.06 (d, *J* = 2.1 Hz, 9H). HRMS calcd for C₅₃H₆₉FN₉O₁₀S [M + H⁺] 1042.4872, found 1042.4868.

### Example 6

### Synthesis of HC58-23

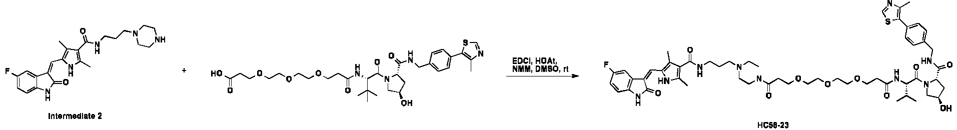

HC58-23 was synthesized following the standard procedure for preparing HC58-18 from Intermediate 2 (13.0 mg, 0.024 mmol, 1.2 equiv), (*S*)-15-((2*S*,4*R*)-4-hydroxy-2-((4-(4-methylthiazol-5-yl)benzyl)carbamoyl)pyrrolidine-1-carbonyl)-16,16-dimethyl-13-oxo-4,7,10-trioxa-14-azaheptadecanoic acid (13.3 mg, 0.02 mmol, 1.0 equiv), EDCI (5.8 mg, 0.03 mmol, 1.5 equiv), HOAt (4.1 mg, 0.03 mmol, 1.5 equiv), andNMM(6.1 mg, 0.06 mmol, 3.0 equiv) in DMSO (1 mL). HC58-23 was obtained as yellow solid in TFA salt form (9.9 mg, 43%). ¹H NMR (600 MHz, Methanol-*d*₄) δ 13.69 (s, 1H), 8.95 (s, 1H), 7.93 (d, *J=* 8.9 Hz, 1H), 7.57 (d, *J=* 2.7 Hz, 1H), 7.46 (d, *J=* 8.2 Hz, 2H), 7.43 - 7.39 (m, 3H), 6.93 - 6.83 (m, 2H), 4.65 (s, 1H), 4.61 - 4.53 (m, 1H), 4.53 - 4.49 (m, 2H), 4.37 (d, *J=* 15.5 Hz, 1H), 3.90 (d, *J* = 10.9 Hz, 1H), 3.81 (dd, *J* = 11.0, 3.9 Hz, 1H), 3.79 - 3.71 (m, 4H), 3.69 - 3.57 (m, 13H), 3.53 (t, *J=* 6.6 Hz, 2H), 3.28 (dd, *J* = 10.8, 4.6 Hz, 2H), 3.24 - 2.98 (m, 4H), 2.59 (ddd, *J=* 15.0, 7.1, 5.5 Hz, 1H), 2.55 - 2.45 (m, 11H), 2.28 - 2.20 (m, 1H), 2.17 - 2.06 (m, 3H), 1.05 (s, 9H). HRMS calcd for C₅₅H₇₃FN₉O₁₀S [M + H⁺] 1070.5185, found 1070.5197.

### Example 7

### Synthesis of HC58-24

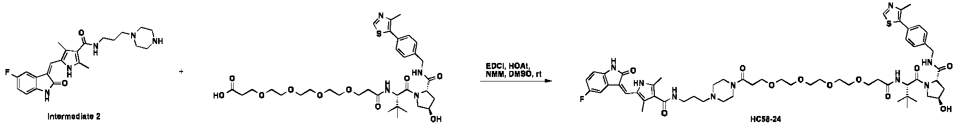

HC58-24 was synthesized following the standard procedure for preparing HC58-18 from Intermediate 2 (13.0 mg, 0.024 mmol, 1.2 equiv), (*S*)-18-((2*S*,4*R*)-4-hydroxy-2-((4-(4-methylthiazol-5-yl)benzyl)carbamoyl)pyrrolidine-1-carbonyl)-19,19-dimethyl-16-oxo-4,7,10,13-tetraoxa-17-azaicosanoic acid (14.1 mg, 0.02 mmol, 1.0 equiv), EDCI (5.8 mg, 0.03 mmol, 1.5 equiv), HOAt (4.1 mg, 0.03 mmol, 1.5 equiv), andNMM(6.1 mg, 0.06 mmol, 3.0 equiv) in DMSO (1 mL). HC58-24 was obtained as yellow solid in TFA salt form (9.6 mg, 39%). ¹H NMR (600 MHz, Methanol-*d*₄) δ 13.70 (s, 1H), 8.95 (s, 1H), 7.58 (s, 1H), 7.47 (d, *J=* 8.2 Hz, 2H), 7.44 - 7.38 (m, 3H), 6.91 - 6.83 (m, 2H), 4.65 (s, 1H), 4.61 - 4.49 (m, 3H), 4.37 (d, *J=* 15.6 Hz, 1H), 3.90 (d, *J=* 10.8 Hz, 1H), 3.84- 3.55 (m, 21H), 3.53 (t, *J=* 6.5 Hz, 2H), 3.28 (t, *J=* 7.6 Hz, 2H), 3.23 - 2.96 (m, 4H), 2.64 - 2.56 (m, 2H), 2.54 - 2.45 (m, 11H), 2.28 - 2.20 (m, 1H), 2.15 - 2.05 (m, 3H), 1.05 (s, 9H). HRMS calcd for C₅₇H₇₇FN₉O₁₁S [M + H⁺] 1114.5447, found 1114.5456.

### Example 8

### Synthesis of HC58-25

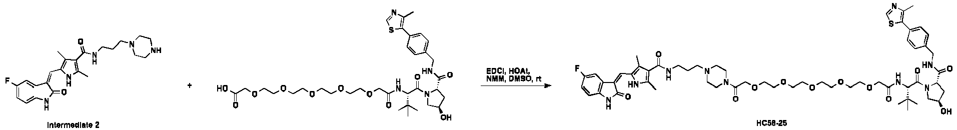

HC58-25 was synthesized following the standard procedure for preparing HC58-18 from Intermediate 2 (13.0 mg, 0.024 mmol, 1.2 equiv), (*S*)-19-((2*S*,4*R*)-4-hydroxy-2-((4-(4-methylthiazol-5-yl)benzyl)carbamoyl)pyrrolidine-1-carbonyl)-20,20-dimethyl-17-oxo-3,6,9,12,15-pentaoxa-18-azahenicosanoic acid (14.5 mg, 0.02 mmol, 1.0 equiv), EDCI (5.8 mg, 0.03 mmol, 1.5 equiv), HOAt(4.1 mg, 0.03 mmol, 1.5 equiv), and NMM (6.1 mg, 0.06 mmol, 3.0 equiv) in DMSO (1 mL). HC58-25 was obtained as yellow solid in TFA salt form (8.9 mg, 36%). 1H NMR (600 MHz, Methanol-d4) δ 13.67 (s, 1H), 9.00 (s, 1H), 7.88 (d, J = 8.6 Hz, 1H), 7.54 (s, 1H), 7.48 - 7.36 (m, 5H), 6.91 - 6.81 (m, 2H), 4.67 - 4.50 (m, 4H), 4.36 (d, J = 15.5 Hz, 1H), 4.18 - 4.06 (m, 2H), 3.89 (d, J = 10.9 Hz, 1H), 3.84 - 3.58 (m, 23H), 3.51 (t, J = 6.7 Hz, 2H), 3.31 - 2.98 (m, 6H), 2.50 (s, 3H), 2.48 (s, 3H), 2.46 (s, 3H), 2.26 (dd, J = 13.2, 7.4 Hz, 1H), 2.16 - 2.05 (m, 3H), 1.07 (s, 9H). HRMS calcd for C₅₇H₇₇FN₉O₁₂S [M + H⁺] 1130.5396, found 1130.5401.

### Example 9

### Synthesis of HC58-26

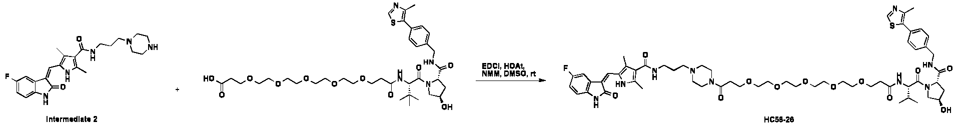

HC58-26 was synthesized following the standard procedure for preparing HC58-18 from Intermediate 2 (13.0 mg, 0.024 mmol, 1.2 equiv), (*S*)-21-((2*S*,4*R*)-4-hydroxy-2-((4-(4-methylthiazol-5-yl)benzyl)carbamoyl)pyrrolidine-1-carbonyl)-22,22-dimethyl-19-oxo-4,7,10,13,16-pentaoxa-20-azatricosanoic acid (15.0 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-26 was obtained as yellow solid in TFA salt form (12.2 mg, 48%). ¹H NMR (600 MHz, Methanol-*d*₄) δ 13.68 (s, 1H), 9.01 (s, 1H), 7.95 (d, *J=* 9.0 Hz, 1H), 7.55 (s, 1H), 7.50 - 7.34 (m, 5H), 6.92 - 6.80 (m, 2H), 4.65 (d, *J=* 2.6 Hz, 1H), 4.61 - 4.49 (m, 3H), 4.36 (d, *J=* 15.5 Hz, 1H), 3.90 (d, *J* = 10.8 Hz, 1H), 3.84- 3.56 (m, 25H), 3.52 (t, *J=* 6.6 Hz, 2H), 3.28 (t, *J=* 7.6 Hz, 2H), 3.24 - 2.99 (m, 4H), 2.64 - 2.55 (m, 2H), 2.53 - 2.43 (m, 11H), 2.28 - 2.20 (m, 1H), 2.10 (ddt, *J* = 17.4, 9.2, 5.2 Hz, 3H), 1.05 (s, 9H). HRMS calcd for C₅₉H₈₁FN₉O₁₂S [M + H⁺] 1158.5709, found 1158.5704.

### Example 10

### Synthesis of HC58-27

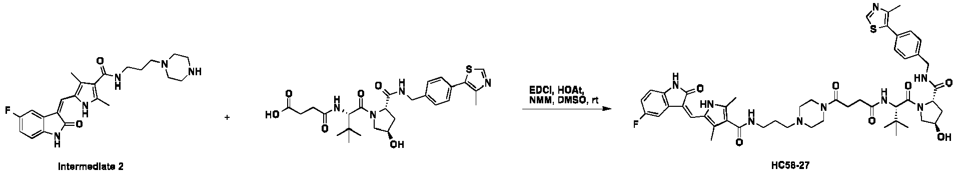

HC58-27 was synthesized following the standard procedure for preparing HC58-18 from Intermediate 2 (13.0 mg, 0.024 mmol, 1.2 equiv), 4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutanoic acid (10.6 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-27 was obtained as yellow solid in TFA salt form (11.2 mg, 53%).¹H NMR (600 MHz, Methanol-d4) δ 13.68 (s, 1H), 8.91 (s, 1H), 7.97 (d, J = 8.7 Hz, 1H), 7.56 (s, 1H), 7.47 - 7.39 (m, 5H), 6.96 - 6.79 (m, 2H), 4.63 - 4.56 (m, 2H), 4.56 - 4.47 (m, 2H), 4.36 (d, J = 15.6 Hz, 1H), 3.92 (d, J = 11.0 Hz, 1H), 3.81 (dd, J = 10.9, 3.9 Hz, 1H), 3.73 - 3.44 (m, 6H), 3.31 - 3.00 (m, 6H), 2.75 - 2.59 (m, 4H), 2.51 (s, 3H), 2.48 (s, 3H), 2.47 (s, 3H), 2.32 - 2.20 (m, 1H), 2.16 - 2.05 (m, 3H), 1.06 (s, 9H). HRMS calcd for C₄₉H₆₁FN₉O₇S [M + H⁺] 938.4399, found 938.4408.

### Example 11

### Synthesis of HC58-28

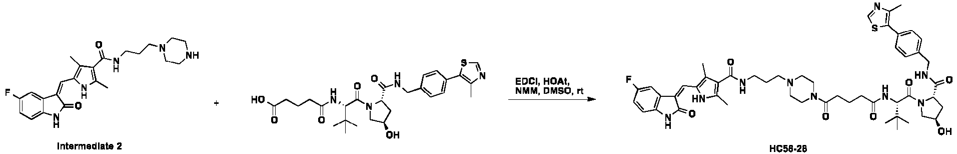

HC58-28 was synthesized following the standard procedure for preparing HC58-18 from Intermediate 2 (13.0 mg, 0.024 mmol, 1.2 equiv), 5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentanoic acid (10.9 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-28 was obtained as yellow solid in TFA salt form (11.9 mg, 56%). ¹H NMR (600 MHz, Methanol-*d*₄) δ 13.68 (s, 1H), 8.90 (s, 1H), 7.94 (d, *J* = 8.7 Hz, 1H), 7.56 (s, 1H), 7.49 - 7.37 (m, 5H), 6.97 - 6.83 (m, 2H), 4.64 - 4.61 (m, 1H), 4.57 (dd, *J=* 9.3, 7.4 Hz, 1H), 4.53 - 4.47 (m, 2H), 4.36 (d, *J* = 15.5 Hz, 1H), 3.95 (d, *J=* 11.0 Hz, 1H), 3.82 (dd, *J=* 10.9, 3.8 Hz, 1H), 3.72 - 3.46 (m, 6H), 3.30 - 2.95 (m, 6H), 2.54 - 2.44 (m, 11H), 2.38 (t, *J* = 7.1 Hz, 2H), 2.29 - 2.21 (m, 1H), 2.16 - 2.07 (m, 3H), 1.93 (p, *J=* 7.3 Hz, 2H), 1.06 (s, 9H). HRMS calcd for C₅₀H₆₃FN₉O₇S [M + H⁺] 952.4555, found 952.4568.

### Example 12

### Synthesis of HC58-29

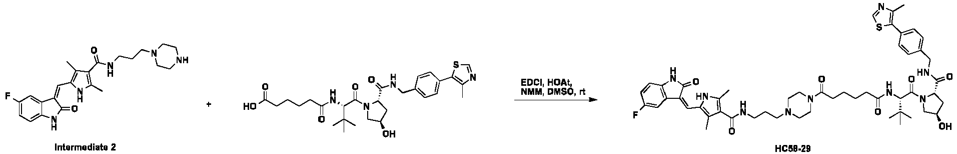

HC58-29 was synthesized following the standard procedure for preparing HC58-18 from Intermediate 2 (13.0 mg, 0.024 mmol, 1.2 equiv), 6-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-6-oxohexanoic acid (11.2 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-29 was obtained as yellow solid in TFA salt form (13.2 mg, 61%). ¹H NMR (600 MHz, Methanol-*d*₄) δ 13.68 (s, 1H), 9.02 (s, 1H), 7.56 (s, 1H), 7.47 (d, *J=* 8.3 Hz, 2H), 7.44 - 7.39 (m, 3H), 6.95 - 6.82 (m, 2H), 4.64 (s, 1H), 4.58 (dd, *J=* 9.1, 7.5 Hz, 1H), 4.55 - 4.48 (m, 2H), 4.37 (d, *J=* 15.5 Hz, 1H), 3.92 (d, *J=* 11.0 Hz, 1H), 3.82 (dd, *J=* 10.9, 3.9 Hz, 1H), 3.73 - 3.47 (m, 6H), 3.28 (t, J = 7.6 Hz, 2H), 3.22 - 3.00 (m, 4H), 2.55 - 2.45 (m, 11H), 2.39 - 2.29 (m, 2H), 2.27 - 2.21 (m, 1H), 2.15 - 2.05 (m, 3H), 1.78 - 1.56 (m, 4H), 1.05 (s, 9H). HRMS calcd for C₅₁H₆₅FN₉O₇S [M + H⁺] 966.4712, found 966.4723.

### Example 13

### Synthesis of HC58-30

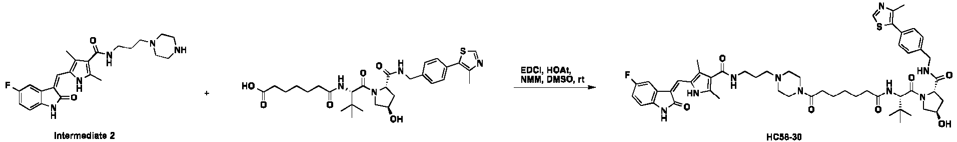

HC58-30 was synthesized following the standard procedure for preparing HC58-18 from Intermediate 2 (13.0 mg, 0.024 mmol, 1.2 equiv), 7-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-7-oxoheptanoic acid (11.5 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-30 was obtained as yellow solid in TFA salt form (8.3 mg, 38%). ¹H NMR (600 MHz, Methanol-*d*₄) δ 13.69 (s, 1H), 8.98 (d, *J=* 2.5 Hz, 1H), 7.85 (d, *J=* 8.6 Hz, 1H), 7.57 (d, *J=* 2.7 Hz, 1H), 7.49 - 7.45 (m, 2H), 7.45 - 7.39 (m, 3H), 7.01 - 6.76 (m, 2H), 4.65 *(d, J=* 2.7 Hz, 1H), 4.61 - 4.49 (m, 3H), 4.38 (dd, *J=* 15.4, 2.6 Hz, 1H), 3.92 (d, *J=* 10.9 Hz, 1H), 3.84 - 3.80 (m, 1H), 3.75 - 3.46 (m, 6H), 3.28 (t, *J* = 7.1 Hz, 2H), 3.16 (d, *J* = 61.8 Hz, 4H), 2.58 - 2.44 (m, 11H), 2.37 - 2.27 (m, 2H), 2.26 - 2.20 (m, 1H), 2.16 - 2.05 (m, 3H), 1.72 - 1.60 (m, 4H), 1.47 - 1.36 (m, 2H), 1.05 (s, 9H). HRMS calcd for C₅₂H₆₇FN₉O₇S [M + H⁺] 980.4868, found 980.4880.

### Example 14

### Synthesis of HC58-31

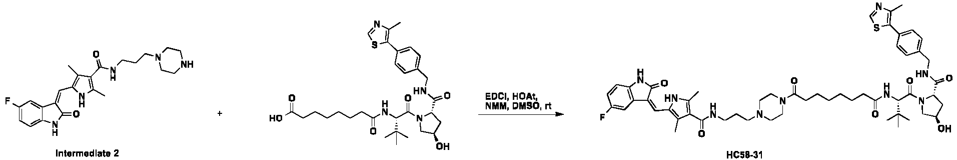

HC58-31 was synthesized following the standard procedure for preparing HC58-18 from Intermediate 2 (13.0 mg, 0.024 mmol, 1.2 equiv), 8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctanoic acid (11.7 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-31 was obtained as yellow solid in TFA salt form (9.6 mg, 43%). ¹H NMR (600 MHz, Methanol-d4) δ 13.69 (s, 1H), 9.04 (s, 1H), 7.56 (d, J = 3.2 Hz, 1H), 7.48 (d, J = 8.2 Hz, 2H), 7.45 - 7.40 (m, 3H), 6.92 - 6.80 (m, 2H), 4.65 (s, 1H), 4.60 - 4.50 (m, 3H), 4.38 (d, J = 15.5 Hz, 1H), 3.92 (d, J = 10.9 Hz, 1H), 3.82 (dd, J = 10.9, 3.9 Hz, 1H), 3.73 - 3.56 (m, 4H), 3.52 (t, J = 6.6 Hz, 2H), 3.28 (t, J = 7.5 Hz, 2H), 3.23 - 2.98 (m, 4H), 2.51 (s, 3H), 2.49 (s, 3H), 2.48 - 2.44 (m, 5H), 2.36 - 2.19 (m, 3H), 2.16 - 2.06 (m, 3H), 1.69 - 1.59 (m, 4H), 1.46 - 1.33 (m, 4H), 1.05 (s, 9H). HRMS calcd for C₅₃H₆₉FN₉O₇S [M + H⁺] 994.5025, found 994.5029.

### Example 15

### Synthesis of HC58-32

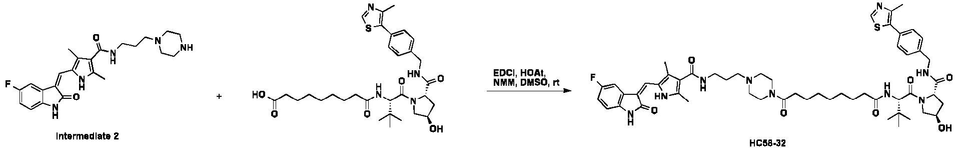

HC58-32 was synthesized following the standard procedure for preparing HC58-18 from Intermediate 2 (13.0 mg, 0.024 mmol, 1.2 equiv), 9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononanoic acid (12.0 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-32 was obtained as yellow solid in TFA salt form (10.8 mg, 48%). ¹H NMR (600 MHz, Methanol-*d*₄) δ 8.95 (d, *J* = 1.2 Hz, 1H), 7.84 (d, *J* = 9.0 Hz, 1H), 7.59 (d, *J* = 2.0 Hz, 1H), 7.48 (d, *J* = 7.9 Hz, 2H), 7.46 - 7.40 (m, 3H), 6.94 - 6.83 (m, 2H), 4.70 - 4.63 (m, 1H), 4.62 - 4.49 (m, 3H), 4.38 (d, *J* = 15.5 Hz, 1H), 3.93 (d, *J* = 10.9 Hz, 1H), 3.82 (dd, *J* = 10.9, 3.9 Hz, 1H), 3.74 - 3.46 (m, 6H), 3.28 (t, *J=* 7.6 Hz, 2H), 3.23 - 2.99 (m, 4H), 2.52 (s, 3H), 2.51 -2.44 (m, 8H), 2.37 - 2.21 (m, 3H), 2.17 - 2.07 (m, 3H), 1.69 - 1.58 (m, 4H), 1.44 - 1.33 (m, 6H), 1.05 (s, 9H). HRMS calcd for C₅₄H₇₁FN₉O₇S [M + H⁺] 1008.5181, found 1008.5189.

### Example 16

### Synthesis of HC58-33

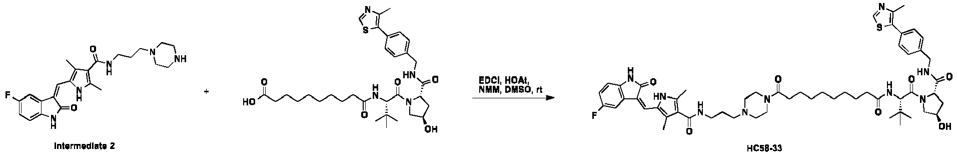

HC58-33 was synthesized following the standard procedure for preparing HC58-18 from Intermediate 2 (13.0 mg, 0.024 mmol, 1.2 equiv), 10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecanoic acid (12.3 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-33 was obtained as yellow solid in TFA salt form (13.2 mg, 58%). ¹H NMR (600 MHz, Methanol-*d*₄) δ 8.95 (s, 1H), 7.84 (d, *J=* 9.0 Hz, 1H), 7.59 (s, 1H), 7.48 (d, *J=* 8.2 Hz, 2H), 7.45 - 7.41 (m, 3H), 6.94 - 6.84 (m, 2H), 4.69 - 4.63 (m, 1H), 4.63 - 4.49 (m, 3H), 4.38 (d, *J=* 15.4 Hz, 1H), 3.92 (d, *J=* 10.9 Hz, 1H), 3.83 (dd, *J=* 11.0, 3.9 Hz, 1H), 3.70 - 3.50 (m, 6H), 3.28 (t, *J* = 7.6 Hz, 2H), 3.23 - 3.02 (m, 4H), 2.52 (s, 3H), 2.51 - 2.45 (m, 8H), 2.35 - 2.21 (m, 3H), 2.15 - 2.06 (m, 3H), 1.67 - 1.58 (m, 4H), 1.42 - 1.32 (m, 8H), 1.05 (s, 9H). HRMS calcd for C₅₅H₇₃FN₉O₇S [M + H⁺] 1022.5338, found 1022.5349.

### Example 17

### Synthesis of HC58-34

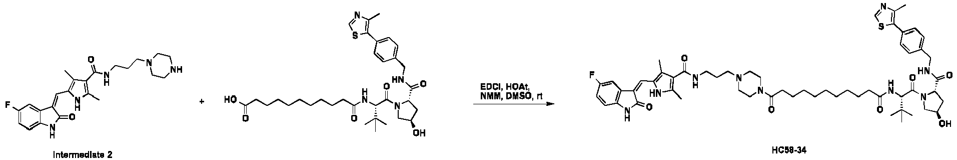

HC58-34 was synthesized following the standard procedure for preparing HC58-18 from Intermediate 2 (13.0 mg, 0.024 mmol, 1.2 equiv), 11-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecanoic acid (12.6 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-34 was obtained as yellow solid in TFA salt form (10.1 mg, 44%). ¹H NMR (600 MHz, Methanol-*d*₄) δ 13.68 (s, 1H), 9.04 (s, 1H), 7.55 (d, *J* = 3.2 Hz, 1H), 7.48 (d, J = 8.0 Hz, 2H), 7.44 - 7.37 (m, 3H), 6.90 - 6.83 (m, 2H), 4.65 (s, 1H), 4.62 - 4.48 (m, 3H), 4.37 (d, *J=* 15.5 Hz, 1H), 3.92 (d, *J* = 11.0 Hz, 1H), 3.82 (dd, *J=* 10.9, 3.9 Hz, 1H), 3.78 - 3.47 (m, 6H), 3.28 (t, *J=* 7.6 Hz, 2H), 3.22 - 2.97 (m, 4H), 2.52 - 2.43 (m, 11H), 2.36 - 2.20 (m, 3H), 2.14 - 2.05 (m, 3H), 1.67 - 1.55 (m, 4H), 1.42 - 1.28 (m, 10H), 1.05 (s, 9H). HRMS calcd for C₅₆H₇₅FN₉O₇S [M + H⁺] 1036.5494, found 1036.5499.

### Example 18

### Synthesis of HC58-35

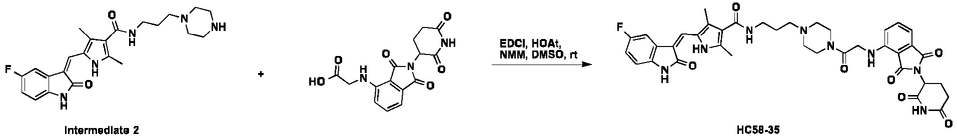

HC58-35 was synthesized following the standard procedure for preparing HC58-18 from Intermediate 2 (13.0 mg, 0.024 mmol, 1.2 equiv), (2-(2,6-dioxopiperidin-3-yl)-1,3-dioxoisoindolin-4-yl)glycine (6.6 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-35 was obtained as yellow solid in TFA salt form (9.7 mg, 57%). ¹H NMR (600 MHz, DMSO-*d*₆) δ 13.73 (d, *J*= 3.5 Hz, 1H), 11.13 (s, 1H), 10.94 (s, 1H), 7.81 (t, *J*= 5.8 Hz, 1H), 7.79 - 7.76 (m, 1H), 7.73 (s, 1H), 7.64 (dd, *J* = 8.5, 7.1 Hz, 1H), 7.11 (t, *J* = 7.4 Hz, 2H), 7.03 (t, *J* =4.6Hz, 1H), 6.94 (td, *J* = 9.1, 2.6 Hz, 1H), 6.86 (dd, *J* = 8.4, 4.6 Hz, 1H), 5.08 (dd, *J* = 12.8, 5.5 Hz, 1H), 4.49 (s, 1H), 4.38 - 4.06 (m, 3H), 3.57 (s, 2H), 3.32 (q, *J=* 6.3 Hz, 2H), 3.24 - 3.12 (m, 2H), 3.11 - 2.97 (m, 4H), 2.89 (ddd, *J=* 17.0, 13.9, 5.4 Hz, 1H), 2.65 - 2.52 (m, 2H), 2.46 (s, 3H), 2.44 (s, 3H), 2.07 - 2.01 (m, 1H), 1.99 - 1.87 (m, 2H). HRMS calcd for C₃₈H₄₀FN₈O₇ [M + H⁺] 739.3004, found 739.3017.

### Example 19

### Synthesis of HC58-36

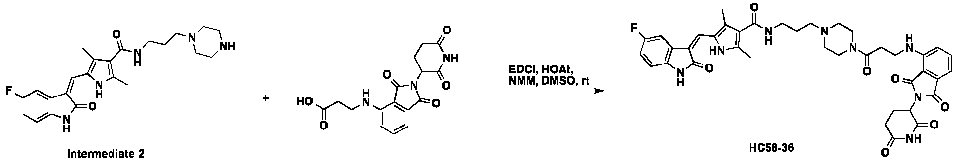

HC58-36 was synthesized following the standard procedure for preparing HC58-18 from Intermediate 2 (13.0 mg, 0.024 mmol, 1.2 equiv), 3-((2-(2,6-dioxopiperidin-3-yl)-1,3-dioxoisoindolin-4-yl)amino)propanoic acid (6.9 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-36 was obtained as yellow solid in TFA salt form (9.0 mg, 52%). 1H NMR (600 MHz, DMSO-d6) δ 13.72 (s, 1H), 11.11 (s, 1H), 10.93 (s, 1H), 7.79 - 7.75 (m, 2H), 7.73 (s, 1H), 7.64 - 7.55 (m, 1H), 7.16 (d, J = 8.6 Hz, 1H), 7.04 (d, J = 7.0 Hz, 1H), 6.94 (td, J = 9.1, 2.6 Hz, 1H), 6.86 (dd, J = 8.4, 4.5 Hz, 1H), 6.78 (t, J = 6.3 Hz, 1H), 5.05 (dd, J = 12.9, 5.5 Hz, 1H), 4.50 (s, 1H), 4.08 (d, J = 6.9 Hz, 1H), 3.63 - 3.34 (m, 7H), 3.32 - 3.26 (m, 2H), 3.22 - 2.92 (m, 5H), 2.88 (ddd, J = 17.1, 13.9, 5.4 Hz, 1H), 2.72 (s, 2H), 2.64 - 2.57 (m, 1H), 2.56 - 2.47 (m, 1H), 2.45 (s, 3H), 2.43 (s, 3H), 2.07 - 2.00 (m, 1H), 1.96 - 1.85 (m, 2H). HRMS calcd for C₃₉H₄₂FN₈O₇ [M + H⁺] 753.3160, found 753.3178.

### Example 20

### Synthesis of HC58-37

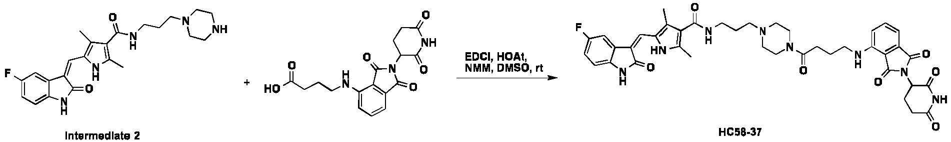

HC58-37 was synthesized following the standard procedure for preparing HC58-18 from Intermediate 2 (13.0 mg, 0.024 mmol, 1.2 equiv), 4-((2-(2,6-dioxopiperidin-3-yl)-1,3-dioxoisoindolin-4-yl)amino)butanoic acid (7.2 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-37 was obtained as yellow solid in TFA salt form (11.3 mg, 64%). ¹H NMR (600 MHz, DMSO-*d*₆) δ 13.73 (s, 1H), 11.11 (s, 1H), 10.93 (s, 1H), 7.85 - 7.75 (m, 2H), 7.73 (s, 1H), 7.60 (t, *J* = 7.8 Hz, 1H), 7.18 (d, *J* = 8.6 Hz, 1H), 7.04 (d, *J* = 7.0 Hz, 1H), 6.94 (td, *J=* 9.1, 2.6 Hz, 1H), 6.86 (dd, *J=* 8.5, 4.5 Hz, 1H), 6.68 (t, *J=* 6.2 Hz, 1H), 5.06 (dd, *J=* 12.8, 5.4 Hz, 1H), 4.56 - 4.44 (m, 1H), 4.17 - 4.01 (m, 1H), 3.51 (s, 2H), 3.43 - 3.27 (m, 7H), 3.19 - 3.10 (m, 2H), 3.10 - 3.00 (m, 1H), 3.00 - 2.84 (m, 3H), 2.63 - 2.57 (m, 1H), 2.57 - 2.52 (m, 1H), 2.46 (s, 3H), 2.43 (s, 3H), 2.07 - 1.99 (m, 1H), 1.96 - 1.86 (m, 2H), 1.85 - 1.76 (m, 2H). HRMS calcd for C₄₀H₄₄FN₈O₇ [M + H⁺] 767.3317, found 767.3332.

### Example 21

### Synthesis of HC58-38

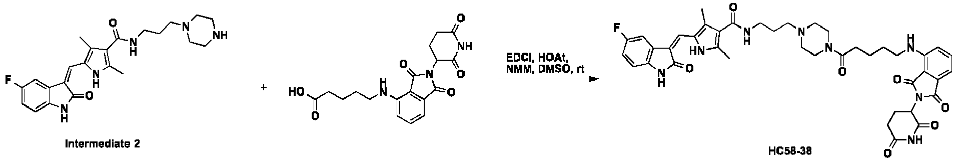

HC58-38 was synthesized following the standard procedure for preparing HC58-18 from Intermediate 2 (13.0 mg, 0.024 mmol, 1.2 equiv), 5-((2-(2,6-dioxopiperidin-3-yl)-1,3-dioxoisoindolin-4-yl)amino)pentanoic acid (7.5 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-38 was obtained as yellow solid in TFA salt form (11.5 mg, 61%). ¹H NMR (600 MHz, DMSO-*d*₆) δ 13.72 (s, 1H), 11.11 (s, 1H), 10.93 (s, 1H), 7.86 - 7.74 (m, 2H), 7.73 (s, 1H), 7.64 - 7.54 (m, 1H), 7.11 (d, J = 8.6 Hz, 1H), 7.03 (d, *J* = 7.0 Hz, 1H), 6.94 (td, *J=* 9.1, 2.7 Hz, 1H), 6.86 (dd, *J=* 8.4, 4.5 Hz, 1H), 6.57 (t, *J=* 5.9 Hz, 1H), 5.06 (dd, *J=* 12.9, 5.4 Hz, 1H), 4.48 (s, 1H), 4.18 - 3.98 (m, 1H), 3.62 - 3.24 (m, 7H), 3.16 (d, *J=* 15.7 Hz, 2H), 3.05 (s, 1H), 2.99 - 2.83 (m, 3H), 2.64 - 2.56 (m, 1H), 2.53 (d, *J=* 4.6 Hz, 3H), 2.45 (s, 3H), 2.43 (s, 3H), 2.08 - 2.00 (m, 1H), 1.97 - 1.87 (m, 2H), 1.66 - 1.52 (m, 4H). HRMS calcd for C₄₁H₄₆FN₈O₇ [M + H⁺] 781.3473, found 781.3478.

### Example 22

### Synthesis of HC58-39

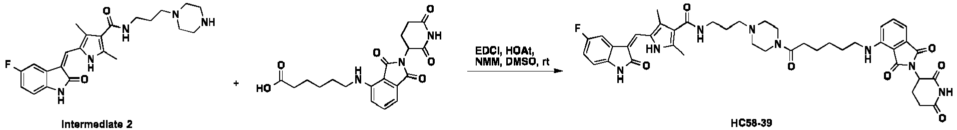

HC58-39 was synthesized following the standard procedure for preparing HC58-18 from Intermediate 2 (13.0 mg, 0.024 mmol, 1.2 equiv), 6-((2-(2,6-dioxopiperidin-3-yl)-1,3-dioxoisoindolin-4-yl)amino)hexanoic acid (7.8 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-39 was obtained as yellow solid in TFA salt form (9.8 mg, 54%). ¹H NMR (600 MHz, DMSO-*d*₆) δ 13.72 (s, 1H), 11.11 (s, 1H), 10.93 (s, 1H), 7.84 - 7.76 (m, 2H), 7.73 (s, 1H), 7.59 (t, J = 7.8 Hz, 1H), 7.10 (d, *J=* 8.6 Hz, 1H), 7.03 (d, *J* = 7.0 Hz, 1H), 6.94 (td, *J=* 9.0, 2.6 Hz, 1H), 6.86 (dd, *J=* 8.4, 4.5 Hz, 1H), 6.54 (d, *J=* 5.9 Hz, 1H), 5.05 (dd, *J=* 12.9, 5.5 Hz, 1H), 4.55 - 4.42 (m, 1H), 4.16 - 4.04 (m, 1H), 3.57 - 3.42 (m, 2H), 3.40 - 3.26 (m, 5H), 3.20 - 3.10 (m, 2H), 3.09 - 2.99 (m, 1H), 2.98 - 2.84 (m, 3H), 2.64 - 2.57 (m, 1H), 2.56 - 2.50 (m, 1H), 2.45 (s, 3H), 2.43 (s, 3H), 2.40 - 2.34 (m, 2H), 2.06 - 1.99 (m, 1H), 1.92 (p, *J* = 7.0 Hz, 2H), 1.66 - 1.50 (m, 4H), 1.37 (p, *J* = 7.8 Hz, 2H). HRMS calcd for C₄₂H₄₈FN₈O₇ [M + H⁺] 795.3630, found 795.3645.

### Example 23

### Synthesis of HC58-40

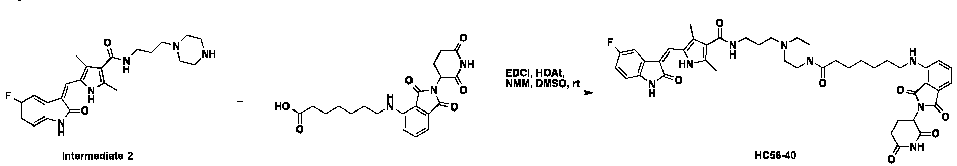

HC58-40 was synthesized following the standard procedure for preparing HC58-18 from Intermediate 2 (13.0 mg, 0.024 mmol, 1.2 equiv), 7-((2-(2,6-dioxopiperidin-3-yl)-1,3-dioxoisoindolin-4-yl)amino)heptanoic acid (8.0 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-40 was obtained as yellow solid in TFA salt form (7.8 mg, 42%). ¹H NMR (600 MHz, DMSO-*d*₆) δ 13.73 (s, 1H), 11.11 (s, 1H), 10.93 (s, 1H), 7.84 - 7.76 (m, 2H), 7.73 (s, 1H), 7.58 (dd, *J* = 9.2, 6.5 Hz, 1H), 7.10 (d, *J=* 8.6 Hz, 1H), 7.03 (d, *J* = 7.0 Hz, 1H), 6.94 (td, *J=* 8.9, 2.5 Hz, 1H), 6.86 (dd, *J=* 8.4, 4.5 Hz, 1H), 6.54 (s, 1H), 5.05 (dd, *J=* 12.9, 5.5 Hz, 1H), 4.47 (s, 1H), 4.08 (d, *J=* 14.3 Hz, 1H), 3.30 (p, *J=* 6.1 Hz, 7H), 3.21 - 3.11 (m, 2H), 3.09-3.00 (m, 1H), 2.98 - 2.84 (m, 3H), 2.64 - 2.51 (m, 2H), 2.46 (s, 3H), 2.43 (s, 3H), 2.40 - 2.31 (m, 2H), 2.07 - 1.99 (m, 1H), 1.96 - 1.87 (m, 2H), 1.64 - 1.45 (m, 4H), 1.34 (d, *J=* 10.1 Hz, 4H). HRMS calcd for C₄₃H₅₀FN₈O₇ [M + H⁺] 809.3786, found 809.3796.

### Example 24

### Synthesis of HC58-41

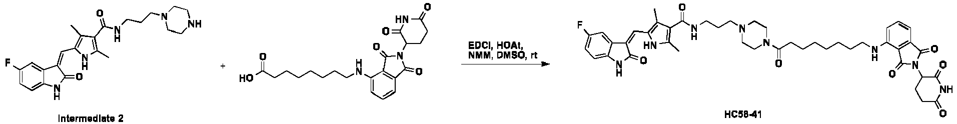

HC58-41 was synthesized following the standard procedure for preparing HC58-18 from Intermediate 2 (13.0 mg, 0.024 mmol, 1.2 equiv), 8-((2-(2,6-dioxopiperidin-3-yl)-1,3-dioxoisoindolin-4-yl)amino)octanoic acid (8.3 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-41 was obtained as yellow solid in TFA salt form (8.1 mg, 43%). ¹H NMR (600 MHz, Methanol-*d*₄) δ 13.60 (s, 1H), 7.86 (s, 1H), 7.56 - 7.52 (m, 2H), 7.40 - 7.36 (m, 1H), 7.03 (d, *J* = 7.1 Hz, 1H), 7.01 (d, *J* = 8.6 Hz, 1H), 6.87 - 6.84 (m, 2H), 5.05 (dd, *J* = 12.7, 5.5 Hz, 1H), 4.60 (s, 2H), 3.62 (s, 2H), 3.56 (t, *J=* 5.2 Hz, 2H), 3.44 (t, *J=* 6.8 Hz, 2H), 3.33 - 3.28 (m, 6H), 2.86 (ddd, *J=* 17.3, 13.9, 5.2 Hz, 1H), 2.79 - 2.68 (m, 2H), 2.53 (t, *J=* 7.3 Hz, 2H), 2.49 (s, 3H), 2.45 (s, 3H), 2.39 (t, *J=* 7.6 Hz, 2H), 2.16 - 2.09 (m, 1H), 1.86 (p, *J=* 7.0 Hz, 2H), 1.67 (p, *J=* 7.0 Hz, 2H), 1.64 - 1.57 (m, 2H), 1.49 - 1.35 (m, 4H). HRMS calcd for C₄₄H₅₂FN₈O₇ [M + H⁺] 823.3943, found 823.3952.

### Example 25

### Synthesis of HC58-43

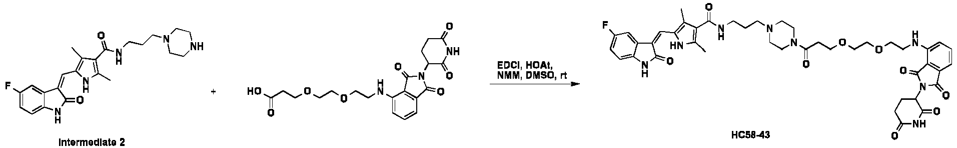

HC58-43 was synthesized following the standard procedure for preparing HC58-18 from Intermediate 2 (13.0 mg, 0.024 mmol, 1.2 equiv), 3-(2-(2-((2-(2,6-dioxopiperidin-3-yl)-1,3-dioxoisoindolin-4-yl)amino)ethoxy)ethoxy)propanoic acid (8.7 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-43 was obtained as yellow solid in TFA salt form (6.9 mg, 36%). ¹H NMR (600 MHz, DMSO-*d*₆) δ 13.72 (s, 1H), 11.11 (s, 1H), 10.93 (s, 1H), 7.84 - 7.75 (m, 2H), 7.73 (s, 1H), 7.59 (dd, *J* = 8.6, 7.1 Hz, 1H), 7.15 (d, *J=* 8.6 Hz, 1H), 7.05 (d, *J=* 7.0 Hz, 1H), 6.94 (td, *J=* 9.0, 2.6 Hz, 1H), 6.86 (dd, *J=* 8.5, 4.5 Hz, 1H), 6.61 (s, 1H), 5.06 (dd, *J=* 12.9, 5.5 Hz, 1H), 4.51- 4.42 (m, 1H), 4.09 (d, *J* = 14.6 Hz, 1H), 3.68 - 3.59 (m, 4H), 3.59 - 3.27 (m, 11H), 3.18 - 3.10 (m, 2H), 3.07 - 2.98 (m, 1H), 2.96 - 2.83 (m, 3H), 2.71 - 2.47 (m, 4H), 2.45 (s, 3H), 2.43 (s, 3H), 2.08 - 2.00 (m, 1H), 1.96 - 1.86 (m, 2H). HRMS calcd for C₄₃H₅₀FN₈O₉ [M + H⁺] 841.3685, found 841.3692.

### Example 26

### Synthesis of HC58-44

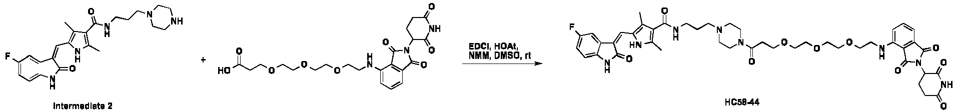

HC58-44 was synthesized following the standard procedure for preparing HC58-18 from Intermediate 2 (13.0 mg, 0.024 mmol, 1.2 equiv), 3-(2-(2-(2-((2-(2,6-dioxopiperidin-3-yl)-1,3-dioxoisoindolin-4-yl)amino)ethoxy)ethoxy)ethoxy)propanoic acid (9.6 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-44 was obtained as yellow solid in TFA salt form (90 mg, 45%). ¹H NMR (600 MHz, DMSO-*d*₆) δ 13.72 (s, 1H), 11.11 (s, 1H), 10.93 (s, 1H), 7.82 - 7.75 (m, 2H), 7.73 (s, 1H), 7.58 (dd, *J=* 8.5, 7.1 Hz, 1H), 7.14 (d, *J=* 8.6 Hz, 1H), 7.04 (d, *J=* 7.0 Hz, 1H), 6.94 (td, *J=* 9.1, 2.6 Hz, 1H), 6.86 (dd, *J=* 8.4, 4.5 Hz, 1H), 6.60 (s, 1H), 5.06 (dd, *J=* 12.9, 5.4 Hz, 1H), 4.47 (s, 1H), 4.11 (d, *J=* 14.4 Hz, 1H), 3.72 - 3.26 (m, 19H), 3.19 - 3.11 (m, 2H), 3.10 - 3.00 (m, 1H), 2.98 - 2.84 (m, 3H), 2.70 - 2.49 (m, 4H), 2.45 (s, 3H), 2.43 (s, 3H), 2.08 - 1.99 (m, 1H), 1.98 - 1.87 (m, 2H). HRMS calcd for C₄₅H₅₄FN₈O₁₀ [M + H⁺] 885.3947, found 885.3955.

### Example 27

### Synthesis of HC58-45

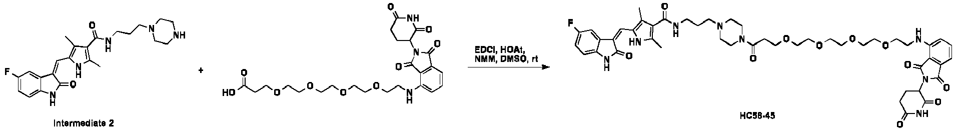

HC58-45 was synthesized following the standard procedure for preparing HC58-18 from Intermediate 2 (13.0 mg, 0.024 mmol, 1.2 equiv), 1-((2-(2,6-dioxopiperidin-3-yl)-1,3-dioxoisoindolin-4-yl)amino)-3,6,9,12-tetraoxapentadecan-15-oic acid (10.4 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-45 was obtained as yellow solid in TFA salt form (6.9 mg, 33%). ¹H NMR (600 MHz, DMSO-*d*₆) δ 13.72 (s, 1H), 11.11 (s, 1H), 10.93 (s, 1H), 7.82 - 7.75 (m, 2H), 7.73 (s, 1H), 7.58 (t, *J* = 7.8 Hz, 1H), 7.14 (d, *J* = 8.6 Hz, 1H), 7.04 (d, *J* = 7.0 Hz, 1H), 6.94 (td, *J=* 9.1, 2.5 Hz, 1H), 6.86 (dd, *J=* 8.5, 4.5 Hz, 1H), 6.60 (s, 1H), 5.06 (dd, *J=* 12.9, 5.4 Hz, 1H), 4.47 (s, 1H), 4.12 (d, *J=* 14.6 Hz, 1H), 3.65 - 3.42 (m, 20H), 3.42 - 3.26 (m, 3H), 3.19 - 3.12 (m, 2H), 3.09 - 2.98 (m, 1H), 2.98 - 2.83 (m, 3H), 2.73 - 2.52 (m, 4H), 2.45 (s, 3H), 2.43 (s, 3H), 2.06 - 1.99 (m, 1H), 1.96 - 1.87 (m, 2H). HRMS calcd for C₄₇H₅₈FN₈O₁₁ [M + H⁺] 929.4209, found 929.4216.

### Example 28

### Synthesis of HC58-46

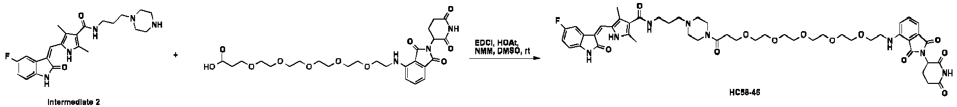

HC58-46 was synthesized following the standard procedure for preparing HC58-18 from Intermediate 2 (13.0 mg, 0.024 mmol, 1.2 equiv), 1-((2-(2,6-dioxopiperidin-3-yl)-1,3-dioxoisoindolin-4-yl)amino)-3,6,9,12,15-pentaoxaoctadecan-18-oic acid (11.3 mg, 0.02 mmol, 1.0 equiv), EDCI (5.8 mg, 0.03 mmol, 1.5 equiv), HOAt (4.1 mg, 0.03 mmol, 1.5 equiv), and NMM (6.1 mg, 0.06 mmol, 3.0 equiv) in DMSO (1 mL). HC58-46 was obtained as yellow solid in TFA salt form (8.9 mg, 41%). ¹H NMR (600 MHz, DMSO-*d*₆) δ 13.72 (s, 1H), 11.11 (s, 1H), 10.93 (s, 1H), 7.82 - 7.75 (m, 2H), 7.73 (s, 1H), 7.58 (dd, *J* = 8.6, 7.0 Hz, 1H), 7.14 (d, *J* = 8.7 Hz, 1H), 7.04 (d, *J* = 7.1 Hz, 1H), 6.96 - 6.90 (m, 1H), 6.86 (dd, *J* = 8.4, 4.6 Hz, 1H), 6.60 (t, *J* = 5.9 Hz, 1H), 5.06 (dd, *J=* 12.9, 5.5 Hz, 1H), 4.47 (s, 1H), 4.12 (d, *J=* 14.5 Hz, 1H), 3.62 (td, *J=* 6.0, 5.4, 3.9 Hz, 4H), 3.59 - 3.34 (m, 21H), 3.30 (q, *J* = 6.5 Hz, 2H), 3.21 - 3.11 (m, 2H), 3.09 - 2.99 (m, 1H), 2.98 - 2.84 (m, 3H), 2.71 - 2.51 (m, 4H), 2.45 (s, 3H), 2.43 (s, 3H), 2.09 - 1.98 (m, 1H), 1.97 - 1.88 (m, 2H). HRMS calcd for C₄₉H₆₂FN₈O₁₂ [M + H⁺] 973.4471, found 973.4479.

### Example 29

### Synthesis of intermediate 3

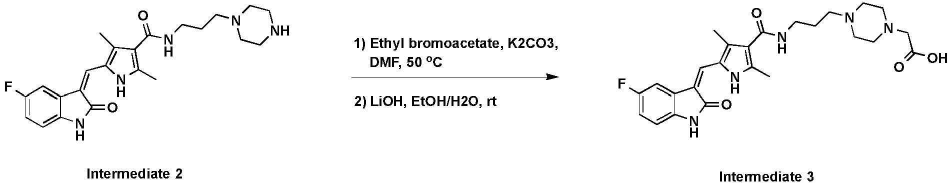

To a solution of intermediate 2 (540 mg, 0.83 mmol) in DMF (10 mL) was added potassium carbonate (280 mg, 2.03 mmol, 2.4 equiv) and ethyl bromoacetate (334 mg, 2.00 mmol, 2.41 equiv). Then the mixture was stirred at 50 °C overnight. Water (20 mL) was added to quench the reaction. The mixture was extracted with ethyl acetate (3 x 20 mL). The organic layer was combined and washed with brine. Dried over Na₂SO₄, filtered and evaporated. The resulting residue was purified by reverse phase C18 column (10% - 100% methanol / 0.1% TFA in H₂O) to afford yellow solid in TFA salt form. The obtained yellow solid was dissolved in EtOH/H₂O (5:1). To the resulting solution was added lithium hydroxide (60 mg, 2.5 mmol, 3 equiv). After being stirred overnight at room temperature, the reaction mixture was concentrated and the residue was purified by reverse phase C18 column (10% - 100% methanol / 0.1% TFA in H₂O) to afford intermediate 3 as yellow solid in TFA salt form (395 mg, 67% yield for two steps). ¹H NMR (600 MHz, Methanol-*d*₄) δ 13.73 (s, 1H), 7.63 (s, 1H), 7.46 (d, *J=* 8.9 Hz, 1H), 6.93 - 6.85 (m, 2H), 3.51 (t, *J* = 6.6 Hz, 2H), 3.45 (s, 2H), 3.26 - 3.19 (m, 2H), 3.30 - 2.80 (m, 8H), 2.53 (s, 3H), 2.49 (s, 3H), 2.11 - 2.03 (m, 2H). ESI m/z =512.3 [M + H⁺].

### Example 30

### Synthesis of HC58-53

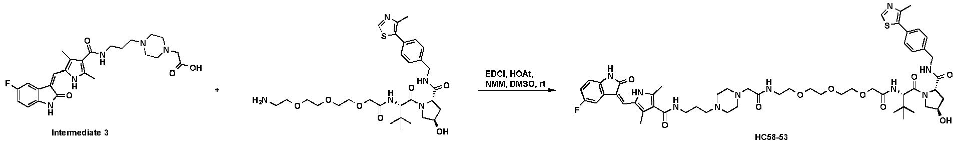

To a solution of Intermediate 3 (14.2 mg, 0.02 mmol, 1.0 equiv) in DMSO (1 mL) were added (2*S*,4*R*)-1-((*S*)-14-amino-2-(*tert*-butyl)-4-oxo-6,9,12-trioxa-3-azatetradecanoyl)-4-hydroxy-*N*-(4-(4-methylthiazol-5-yl)benzyl)pyrrolidine-2-carboxamide (16.9 mg, 0.02 mmol, 1.0 equiv), EDCI (1-ethyl-3-(3-dimethylaminopropyl)carbodiimide) (5.8 mg, 0.03 mmol, 1.5 equiv), HOAt (1-hydroxy-7-azabenzo-triazole) (4.1 mg, 0.03 mmol, 1.5 equiv), and NMM (N-Methylmorpholine) (12.1 mg, 0.12 mmol, 6.0 equiv). After being stirred overnight at room temperature, the resulting mixture was purified by preparative HPLC (10%-100% methanol / 0.1% TFA in H₂O) to afford HC58-53 as yellow solid in TFA salt form (11.8 mg, 45%). ¹H NMR (600 MHz, Methanol-*d*₄) δ 13.68 (s, 1H), 8.97 (s, 1H), 7.69 (d, *J* = 9.3 Hz, 1H), 7.56 (s, 1H), 7.47 - 7.38 (m, 5H), 6.97 - 6.79 (m, 2H), 4.74 - 4.67 (m, 1H), 4.59 (dd, *J=* 9.4, 7.5 Hz, 1H), 4.55 - 4.48 (m, 2H), 4.38 (d, *J=* 15.4 Hz, 1H), 4.12 - 4.06 (m, 2H), 3.89 (d, *J=* 11.1 Hz, 1H), 3.81 (dt, *J=* 11.0, 3.2 Hz, 1H), 3.77 - 3.60 (m, 10H), 3.59 - 3.39 (m, 8H), 3.31 - 3.20 (m, 4H), 3.12 - 2.86 (m, 4H), 2.50 (s, 3H), 2.49 (s, 3H), 2.47 (s, 3H), 2.31 - 2.22 (m, 1H), 2.09 (tdd, *J=* 13.2, 9.7, 4.1 Hz, 3H), 1.06 (d, *J=* 1.7 Hz, 9H). HRMS calcd for C₅₅H₇₄FN₁₀O₁₀S [M + H⁺] 1085.5294, found 1085.5299.

### Example 31

### Synthesis of HC58-57

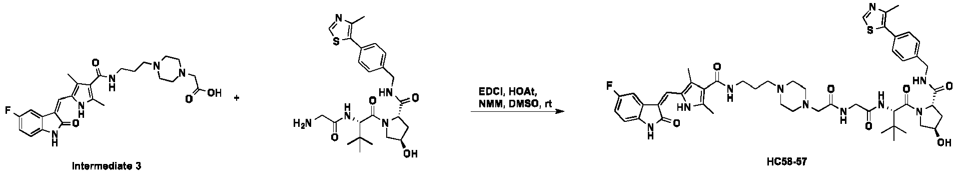

HC58-57 was synthesized following the standard procedure for preparing HC58-53 from Intermediate 3 (14.2 mg, 0.02 mmol, 1.0 equiv), (2*S*,4*R*)-1-((*S*)-2-(2-aminoacetamido)-3,3-dimethylbutanoyl)-4-hydroxy-*N*-(4-(4-methylthiazol-5-yl)benzyl)pyrrolidine-2-carboxamide (14.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-57 was obtained as yellow solid in TFA salt form (10.2 mg, 43%). ¹H NMR (600 MHz, Methanol-*d*₄) δ 13.67 (s, 1H), 9.07 (s, 1H), 7.96 (s, 1H), 7.55 (s, 1H), 7.51 - 7.37 (m, 5H), 6.96 - 6.80 (m, 2H), 4.67 - 4.63 (m, 1H), 4.57 (t, *J =* 8.2 Hz, 1H), 4.54 - 4.48 (m, 2H), 4.38 *(d, J=* 15.5 Hz, 1H), 4.03 *(d, J=* 16.8 Hz, 1H), 4.00 - 3.95 (m, 1H), 3.89 (d, *J* = 10.9 Hz, 1H), 3.81 (dd, *J=* 11.0, 3.8 Hz, 1H), 3.56 - 3.38 (m, 8H), 3.25 (t, *J=* 7.6 Hz, 2H), 3.14 - 2.90 (m, 4H), 2.50 (s, 3H), 2.49 (s, 3H), 2.47 (s, 3H), 2.29 - 2.21 (m, 1H), 2.14 - 2.01 (m, 3H), 1.06 (s, 9H). HRMS calcd for C₄₉H₆₂FN₁₀O₇S [M + H⁺] 953.4508, found 953.4516.

### Example 32

### Synthesis of HC58-58

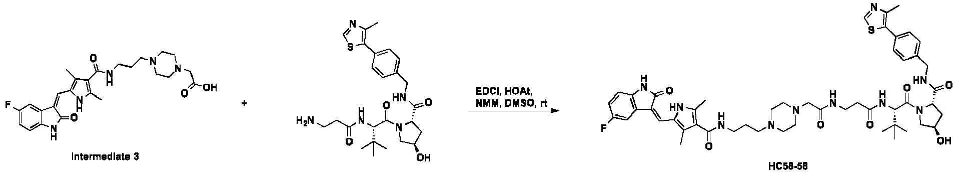

HC58-58 was synthesized following the standard procedure for preparing HC58-53 from Intermediate 3 (14.2 mg, 0.02 mmol, 1.0 equiv), (2*S*,4*R*)-1-((*S*)-2-(3-aminopropanamido)-3,3-dimethylbutanoyl)-4-hydroxy-*N*-(4-(4-methylthiazol-5-yl)benzyl)pyrrolidine-2-carboxamide (14.6 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-58 was obtained as yellow solid in TFA salt form (12.2 mg, 51%). ¹H NMR (600 MHz, Methanol-*d*₄) δ 13.68 (s, 1H), 9.01 (s, 1H), 8.00 (d, *J* = 8.6 Hz, 1H), 7.55 (s, 1H), 7.50 -7.38 (m, 5H), 6.94 - 6.82 (m, 2H), 4.65 - 4.56 (m, 2H), 4.54 - 4.48 (m, 2H), 4.37 (d, *J=* 15.5 Hz, 1H), 3.95 (d, *J=* 10.9 Hz, 1H), 3.81 (dd, *J =* 10.8, 3.9 Hz, 1H), 3.58 - 3.36 (m, 8H), 3.29 - 3.20 (m, 4H), 3.14 - 2.81 (m, 4H), 2.58 - 2.51 (m, 2H), 2.50 (s, 3H), 2.48 (s, 3H), 2.46 (s, 3H), 2.32 - 2.23 (m, 1H), 2.14 - 2.05 (m, 3H), 1.06 (s, 9H). HRMS calcd for C₅₀H₆₄FN₁₀O₇S [M + H⁺] 967.4664, found 967.4672.

### Example 33

### Synthesis of HC58-59

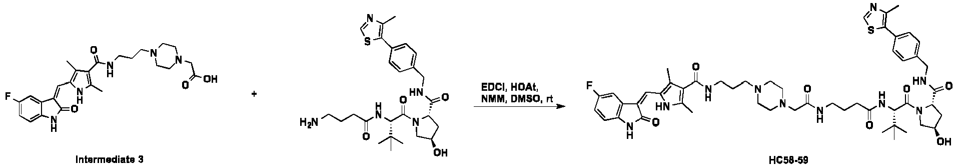

HC58-59 was synthesized following the standard procedure for preparing HC58-53 from Intermediate 3 (14.2 mg, 0.02 mmol, 1.0 equiv), (2*S*,4*R*)-1-((*S*)-2-(4-aminobutanamido)-3,3-dimethylbutanoyl)-4-hydroxy-*N*-(4-(4-methylthiazol-5-yl)benzyl)pyrrolidine-2-carboxamide (14.9 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-59 was obtained as yellow solid in TFA salt form (8.9 mg, 37%). ¹H NMR (600 MHz, Methanol-*d*₄) δ 13.68 (s, 1H), 9.03 (s, 1H), 7.57 (s, 1H), 7.47 (d, *J=* 8.0 Hz, 2H), 7.44 - 7.38 (m, 3H), 6.92 - 6.84 (m, 2H), 4.64 (s, 1H), 4.59 (dd, *J* = 9.1, 7.6 Hz, 1H), 4.56 - 4.49 (m, 2H), 4.38 (d, *J* = 15.5 Hz, 1H), 3.93 (d, *J=* 11.0 Hz, 1H), 3.82 (dd, *J=* 11.0, 3.9 Hz, 1H), 3.51 (t, *J=* 6.4 Hz, 2H), 3.49 - 3.39 (m, 4H), 3.31 - 3.21 (m, 6H), 3.09 - 2.90 (m, 4H), 2.51 (s, 3H), 2.49 (s, 3H), 2.47 (s, 3H), 2.41 - 2.28 (m, 2H), 2.27 - 2.22 (m, 1H), 2.13 - 2.05 (m, 3H), 1.91 - 1.78 (m, 2H), 1.06 (s, 9H). HRMS calcd for C₅₁H₆₆FN₁₀O₇S [M + H⁺] 981.4821, found 981.4833.

### Example 34

### Synthesis of HC58-60

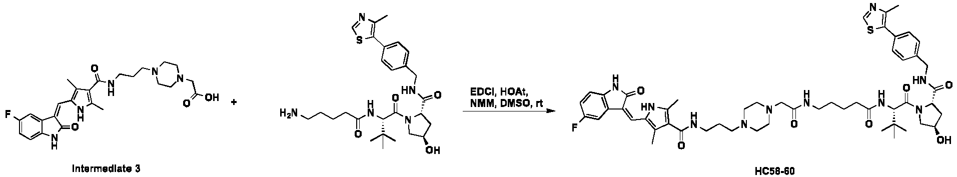

HC58-60 was synthesized following the standard procedure for preparing HC58-53 from Intermediate 3 (14.2 mg, 0.02 mmol, 1.0 equiv), (2*S*,4*R*)-1-((*S*)-2-(5-aminopentanamido)-3,3-dimethylbutanoyl)-4-hydroxy-N-(4-(4-methylthiazol-5-yl)benzyl)pyrrolidine-2-carboxamide (11.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-60 was obtained as yellow solid in TFA salt form (11.7 mg, 48%). ¹H NMR (600 MHz, Methanol-*d*₄) δ 13.71 (s, 1H), 8.94 (s, 1H), 7.87 (d, *J* = 8.9 Hz, 1H), 7.59 (s, 1H), 7.46 (d, *J* = 8.2 Hz, 2H), 7.45 - 7.40 (m, 3H), 6.95 - 6.79 (m, 2H), 4.66 - 4.62 (m, 1H), 4.57 (t, *J* = 8.3 Hz, 1H), 4.54 - 4.48 (m, 2H), 4.37 (d, *J* = 15.4 Hz, 1H), 3.91 (d, *J=* 11.0 Hz, 1H), 3.82 (dd, *J* = 10.9, 3.9 Hz, 1H), 3.57 - 3.35 (m, 6H), 3.29 - 3.21 (m, 6H), 3.11 - 2.83 (m, 4H), 2.52 (s, 3H), 2.48 (s, 3H), 2.48 (s, 3H), 2.36 - 2.29 (m, 2H), 2.26 - 2.20 (m, 1H), 2.14 - 2.05 (m, 3H), 1.74 - 1.60 (m, 2H), 1.59 - 1.52 (m, 2H), 1.05 (s, 9H). HRMS calcd for C₅₂H₆₈FN₁₀O₇S [M + H⁺] 995.4977, found 995.4990.

### Example 35

### Synthesis of HC58-63

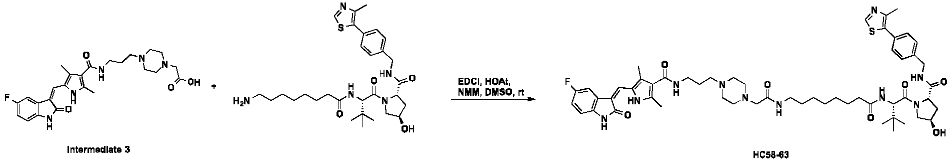

HC58-63 was synthesized following the standard procedure for preparing HC58-53 from Intermediate 3 (14.2 mg, 0.02 mmol, 1.0 equiv), (2*S*,4*R*)-1-((*S*)-2-(8-aminooctanamido)-3,3-dimethylbutanoyl)-4-hydroxy-*N*-(4-(4-methylthiazol-5-yl)benzyl)pyrrolidine-2-carboxamide (16.0 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-63 was obtained as yellow solid in TFA salt form (8.3 mg, 33%). ¹H NMR (600 MHz, Methanol-*d*₄) δ 13.70 (s, 1H), 8.90 (s, 1H), 7.83 *(d, J=* 9.0 Hz, 1H), 7.59 (s, 1H), 7.47 (d, *J* = 8.1 Hz, 2H), 7.45 - 7.40 (m, 3H), 6.96 - 6.81 (m, 2H), 4.69 - 4.63 (m, 1H), 4.61 - 4.49 (m, 3H), 4.37 (d, *J* = 15.4 Hz, 1H), 3.92 (d, *J=* 11.0 Hz, 1H), 3.82 (dd, *J=* 10.9, 3.9 Hz, 1H), 3.51 (t, *J=* 6.6 Hz, 2H), 3.49 - 3.36 (m, 4H), 3.28 - 2.89 (m, 10H), 2.52 (s, 3H), 2.48 (s, 6H), 2.34 - 2.20 (m, 3H), 2.14 - 2.05 (m, 3H), 1.69 - 1.58 (m, 2H), 1.57 - 1.50 (m, 2H), 1.42 - 1.29 (m, 6H), 1.05 (s, 9H). HRMS calcd for C₅₅H₇₄FN₁₀O₇S [M + H⁺] 1037.5447, found 1037.5449.

### Example 36

### Synthesis of HC58-64

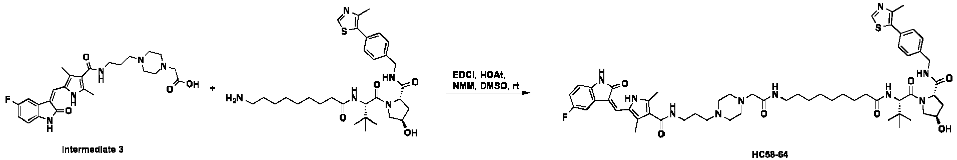

HC58-64 was synthesized following the standard procedure for preparing HC58-53 from Intermediate 3 (14.2 mg, 0.02 mmol, 1.0 equiv), (2*S*,4*R*)-1-((*S*)-2-(9-aminononanamido)-3,3-dimethylbutanoyl)-4-hydroxy-*N*-(4-(4-methylthiazol-5-yl)benzyl)pyrrolidine-2-carboxamide (12.4 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-64 was obtained as yellow solid in TFA salt form (10.2 mg, 39%). ¹H NMR (600 MHz, Methanol-*d*₄) δ 13.70 (s, 1H), 8.91 (s, 1H), 7.84 (d, *J=* 9.0 Hz, 0H), 7.58 (s, 1H), 7.47 (d, *J=* 8.2 Hz, 2H), 7.44 - 7.40 (m, 3H), 6.92 - 6.83 (m, 2H), 4.67 - 4.63 (m, 1H), 4.62 - 4.49 (m, 3H), 4.37 (d, *J* = 15.5 Hz, 1H), 3.92 (d, *J=* 11.0 Hz, 1H), 3.82 (dd, *J=* 10.9, 3.9 Hz, 1H), 3.59 - 3.37 (m, 6H), 3.28 - 3.19 (m, 6H), 3.07 - 2.78 (m, 4H), 2.55 - 2.45 (m, 9H), 2.36 - 2.21 (m, 3H), 2.13 - 2.05 (m, 3H), 1.67 - 1.58 (m, 2H), 1.57 - 1.48 (m, 2H), 1.34 (s, 8H), 1.05 (s, 9H). HRMS calcd for C₅₆H₇₆FN₁₀O₇S [M + H⁺] 1051.5603, found 1051.5620.

### Example 37

### Synthesis of HC58-65

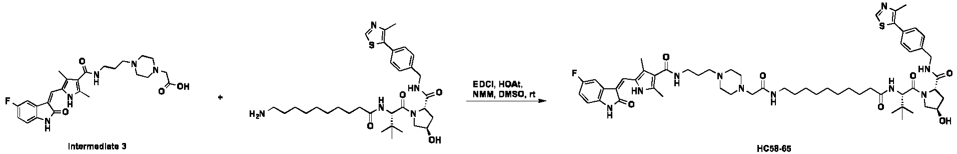

HC58-65 was synthesized following the standard procedure for preparing HC58-53 from Intermediate 3 (14.2 mg, 0.02 mmol, 1.0 equiv), (2*S*,4*R*)-1-((*S*)-2-(10-aminodecanamido)-3,3-dimethylbutanoyl)-4-hydroxy-*N*-(4-(4-methylthiazol-5-yl)benzyl)pyrrolidine-2-carboxamide (16.6 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-65 was obtained as yellow solid in TFA salt form (10.9 mg, 42%). ¹H NMR (600 MHz, Methanol-*d*₄) δ 13.71 (s, 1H), 8.91 (s, 1H), 7.84 (d, *J* = 9.0 Hz, 1H), 7.59 (s, 1H), 7.47 (d, *J* = 8.3 Hz, 2H), 7.45 -7.39 (m, 3H), 6.98 - 6.83 (m, 2H), 4.67 - 4.64 (m, 1H), 4.61 - 4.49 (m, 3H), 4.37 (d, *J=* 15.4 Hz, 1H), 3.92 (d, *J=* 11.0 Hz, 1H), 3.82 (dd, *J=* 10.9, 3.9 Hz, 1H), 3.58 - 3.37 (m, 6H), 3.23 (dd, *J=* 12.7, 5.3 Hz, 6H), 3.04 - 2.76 (m, 4H), 2.52 (s, 3H), 2.48 (s, 6H), 2.35 - 2.20 (m, 3H), 2.15 - 2.05 (m, 3H), 1.66 - 1.57 (m, 2H), 1.56 - 1.50 (m, 2H), 1.33 (s, 10H), 1.05 (s, 9H). HRMS calcd for C₅₇H₇₈FN₁₀O₇S [M + H⁺] 1065.5760, found 1065.5767.

### Example 38

### Synthesis of HC58-66

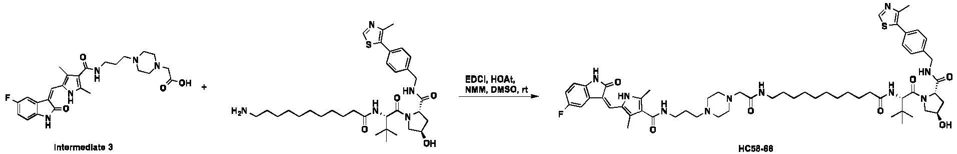

HC58-66 was synthesized following the standard procedure for preparing HC58-53 from Intermediate 3 (14.2 mg, 0.02 mmol, 1.0 equiv), (2*S*,4*R*)-1-((*S*)-2-(11-aminoundecanamido)-3,3-dimethylbutanoyl)-4-hydroxy-*N*-(4-(4-methylthiazol-5-yl)benzyl)pyrrolidine-2-carboxamide (14.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-66 was obtained as yellow solid in TFA salt form (8.4 mg, 32%). ¹H NMR (600 MHz, Methanol-*d*₄) δ 13.73 (s, 1H), 8.89 (s, 1H), 7.83 (d, *J=* 9.0 Hz, 1H), 7.62 (s, 1H), 7.49 - 7.41 (m, 5H), 6.95 - 6.85 (m, 2H), 4.69 - 4.63 (m, 1H), 4.60 - 4.49 (m, 3H), 4.37 (d, *J=* 15.4 Hz, 1H), 3.92 (d, *J=* 10.8 Hz, 1H), 3.82 (dd, *J=* 10.9, 3.9 Hz, 1H), 3.65 - 3.55 (m, 4H), 3.51 (t, *J=* 6.5 Hz, 2H), 3.28 - 3.18 (m, 6H), 3.16 - 3.05 (m, 4H), 2.53 (s, 3H), 2.49 (s, 3H), 2.48 (s, 3H), 2.36 - 2.20 (m, 3H), 2.13 - 2.04 (m, 3H), 1.62 (s, 2H), 1.56 - 1.49 (m, 2H), 1.37 - 1.27 (m, 12H), 1.05 (s, 9H). HRMS calcd for C₅₈H₈₀FN₁₀O₇S [M + H⁺] 1079.5916, found 1079.5928.

### Example 39

### Synthesis of HC58-67

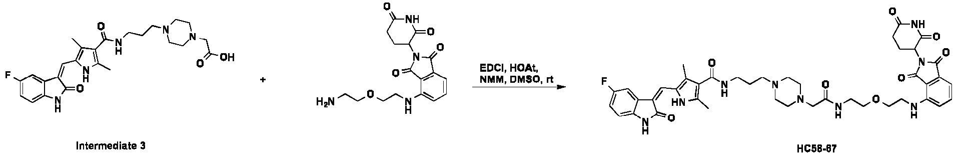

HC58-67 was synthesized following the standard procedure for preparing HC58-53 from Intermediate 3 (14.2 mg, 0.02 mmol, 1.0 equiv), 4-((2-(2-aminoethoxy)ethyl)amino)-2-(2,6-dioxopiperidin-3-yl)isoindoline-1,3-dione (9.5 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-67 was obtained as yellow solid in TFA salt form (11.0 mg, 52%). ¹H NMR (600 MHz, Methanol-*d*₄) δ 13.68 (s, 1H), 7.59 - 7.54 (m, 2H), 7.42 (dd, *J* = 8.9, 2.3 Hz, 1H), 7.10 (d, *J* = 8.6 Hz, 1H), 7.07 (d, *J* = 7.0 Hz, 1H), 6.91 - 6.84 (m, 2H), 5.06 (dd, *J* = 12.8, 5.5 Hz, 1H), 3.73 (t, *J* = 5.1 Hz, 2H), 3.63 (t, *J* = 5.2 Hz, 2H), 3.49 (dt, *J* = 13.9, 5.1 Hz, 6H), 3.41 - 3.31 (m, 4H), 3.25 (s, 2H), 3.17 (t, *J* = 7.5 Hz, 2H), 3.02 - 2.80 (m, 5H), 2.79 - 2.69 (m, 2H), 2.50 (s, 3H), 2.46 (s, 3H), 2.16 - 2.10 (m, 1H), 2.07 - 1.99 (m, 2H). HRMS calcd for C₄₂H₄₉FN₉O₈ [M + H⁺] 826.3688, found 826.3697.

### Example 40

### Synthesis of HC58-68

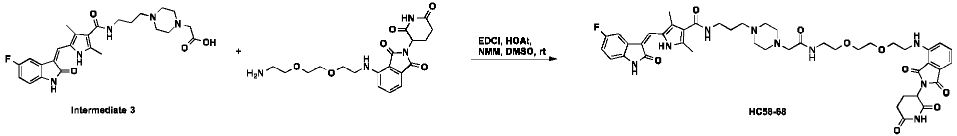

HC58-68 was synthesized following the standard procedure for preparing HC58-53 from Intermediate 3 (14.2 mg, 0.02 mmol, 1.0 equiv), 4-((2-(2-(2-aminoethoxy)ethoxy)ethyl)amino)-2-(2,6-dioxopiperidin-3-yl)isoindoline-1,3-dione (10.4 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-68 was obtained as yellow solid in TFA salt form (10.8 mg, 49%). ¹H NMR (600 MHz, Methanol-*d*₄) δ 13.64 (s, 1H), 7.56 - 7.51 (m, 2H), 7.40 (dd, *J* = 8.9, 2.3 Hz, 1H), 7.06 (d, *J* = 8.6 Hz, 1H), 7.03 (d, *J* = 7.0 Hz, 1H), 6.90 - 6.83 (m, 2H), 5.07 (dd, *J* = 12.7, 5.4 Hz, 1H), 3.73 (t, *J* = 5.1 Hz, 2H), 3.69 - 3.63 (m, 4H), 3.59 (t, *J* = 5.3 Hz, 2H), 3.52 - 3.39 (m, 6H), 3.39 - 3.28 (m, 4H), 3.21 (d, *J* = 2.6 Hz, 2H), 3.17 (t, *J* = 7.5 Hz, 2H), 3.01 - 2.81 (m, 5H), 2.80 - 2.67 (m, 2H), 2.49 (s, 3H), 2.44 (s, 3H), 2.17 - 2.10 (m, 1H), 2.04 (p, *J* = 6.7 Hz, 2H). HRMS calcd for C₄₄H₅₃FN₉O₉ [M + H⁺] 870.3950, found 870.3958.

### Example 41

### Synthesis of HC58-69

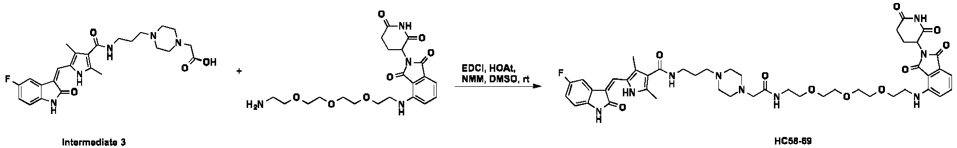

HC58-69 was synthesized following the standard procedure for preparing HC58-53 from Intermediate 3 (14.2 mg, 0.02 mmol, 1.0 equiv), 4-((2-(2-(2-(2-aminoethoxy)ethoxy)ethoxy)ethyl)amino)-2-(2,6-dioxopiperidin-3-yl)isoindoline-1,3-dione (11.2 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-69 was obtained as yellow solid in TFA salt form (13.2 mg, 58%). ¹H NMR (600 MHz, Methanol-*d*₄) δ 13.65 (s, 1H), 7.55 - 7.50 (m, 2H), 7.40 (dd, *J* = 9.0, 2.3 Hz, 1H), 7.05 (d, *J* = 8.5 Hz, 1H), 7.01 (d, *J* = 7.1 Hz, 1H), 6.90 - 6.82 (m, 2H), 5.06 (dd, *J* = 12.7, 5.5 Hz, 1H), 3.71 (t, *J* = 5.2 Hz, 2H), 3.68 - 3.63 (m, 6H), 3.60 (dt, *J* = 6.4, 2.2 Hz, 2H), 3.56 (t, *J* = 5.4 Hz, 2H), 3.52 - 3.46 (m, 4H), 3.42 (t, *J* = 5.3 Hz, 2H), 3.41-3.35 (m, 4H), 3.26-3.19 (m, 4H), 3.04-2.81 (m, 5H), 2.79-2.66 (m, 2H), 2.49 (s, 3H), 2.45 (s, 3H), 2.15 - 2.10 (m, 1H), 2.06 (p, *J* = 6.7 Hz, 2H). HRMS calcd for C₄₆H₅₇FN₉O₁₀ [M + H⁺] 914.4212, found 914.4226.

### Example 42

### Synthesis of HC58-70

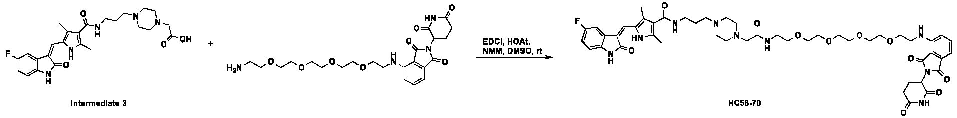

HC58-70 was synthesized following the standard procedure for preparing HC58-53 from Intermediate 3 (14.2 mg, 0.02 mmol, 1.0 equiv), 4-((14-amino-3,6,9,12-tetraoxatetradecyl)amino)-2-(2,6-dioxopiperidin-3-yl)isoindoline-1,3-dione (11.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-70 was obtained as yellow solid in TFA salt form (11.9 mg, 50%). ¹H NMR (600 MHz, Methanol-*d*₄) δ 13.68 (s, 1H), 7.56 (s, 1H), 7.52 (dd, *J* = 8.6, 7.1 Hz, 1H), 7.41 (dd, *J* = 8.9, 2.4 Hz, 1H), 7.05 (d, *J* = 8.6 Hz, 1H), 7.02 (d, *J* = 7.0 Hz, 1H), 6.91 - 6.83 (m, 2H), 5.06 (dd, *J* = 12.7, 5.5 Hz, 1H), 3.71 (t, *J* = 5.2 Hz, 2H), 3.68 - 3.58 (m, 12H), 3.56 (t, *J* = 5.3 Hz, 2H), 3.50 (t, *J* = 6.5 Hz, 2H), 3.47 (t, *J* = 5.2 Hz, 2H), 3.45 - 3.36 (m, 6H), 3.27 - 3.19 (m, 4H), 3.05 - 2.80 (m, 5H), 2.78 - 2.67 (m, 2H), 2.51 (s, 3H), 2.46 (s, 3H), 2.17 - 2.03 (m, 3H). HRMS calcd for C₄₈H₆₁FN₉O₁₁ [M + H⁺] 958.4475, found 958.4488.

### Example 43

### Synthesis of HC58-71

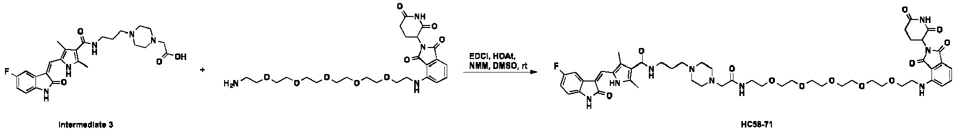

HC58-71 was synthesized following the standard procedure for preparing HC58-53 from Intermediate 3 (14.2 mg, 0.02 mmol, 1.0 equiv), 4-((17-amino-3,6,9,12,15-pentaoxaheptadecyl)amino)-2-(2,6-dioxopiperidin-3-yl)isoindoline-1,3-dione (12.2 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-71 was obtained as yellow solid in TFA salt form (11.8 mg, 48%). ¹H NMR (600 MHz, Methanol-*d*₄) δ 13.65 (s, 1H), 7.54 - 7.49 (m, 2H), 7.39 (dd, *J* = 8.9, 2.4 Hz, 1H), 7.04 (d, *J* = 8.6 Hz, 1H), 7.01 (d, *J* = 7.0 Hz, 1H), 6.90 - 6.82 (m, 3H), 5.05 (dd, *J* = 12.8, 5.5 Hz, 1H), 3.70 (t, *J* = 5.2 Hz, 2H), 3.67 - 3.59 (m, 16H), 3.56 (t, *J* = 5.3 Hz, 2H), 3.50 (t, *J* = 6.4 Hz, 2H), 3.48 - 3.38 (m, 8H), 3.28 - 3.21 (m, 4H), 3.08 - 2.82 (m, 5H), 2.79 - 2.66 (m, 2H), 2.49 (s, 3H), 2.45 (s, 3H), 2.17 - 2.04 (m, 3H). HRMS calcd for C₅₀H₆₅FN₉O₁₂ [M + H⁺] 1002.4737, found 1002.4752.

### Example 44

### Synthesis of HC58-73

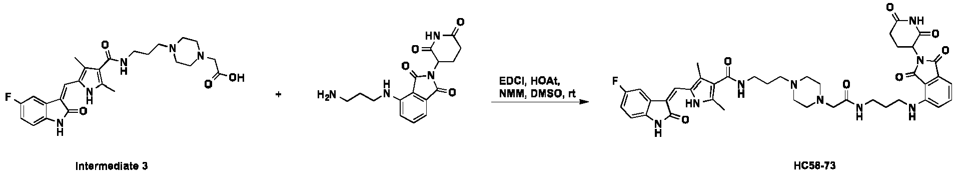

HC58-73 was synthesized following the standard procedure for preparing HC58-53 from Intermediate 3 (14.2 mg, 0.02 mmol, 1.0 equiv), 4-((3-aminopropyl)amino)-2-(2,6-dioxopiperidin-3-yl)isoindoline-1,3-dione (8.9 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-73 was obtained as yellow solid in TFA salt form (13.9 mg, 68%). ¹H NMR (600 MHz, Methanol-*d*₄) δ 13.67 (s, 1H), 7.57 - 7.53 (m, 2H), 7.40 (dd, *J* = 8.9, 2.4 Hz, 1H), 7.06 (d, *J* = 5.6 Hz, 1H), 7.04 (d, *J* = 4.1 Hz, 1H), 6.91 - 6.83 (m, 2H), 5.06 (dd, *J* = 12.7, 5.5 Hz, 1H), 3.50 (t, *J* = 6.5 Hz, 2H), 3.43 - 3.36 (m, 8H), 3.28 (s, 2H), 3.22 (dd, *J* = 8.5, 6.6 Hz, 2H), 3.05 - 2.81 (m, 5H), 2.78 - 2.67 (m, 2H), 2.50 (s, 3H), 2.46 (s, 3H), 2.16 - 2.03 (m, 3H), 1.89 (p, *J* = 6.6 Hz, 2H). HRMS calcd for C₄₁H₄₇FN₉O₇ [M + H⁺] 796.3582, found 796.3590.

### Example 45

### Synthesis of HC58-74

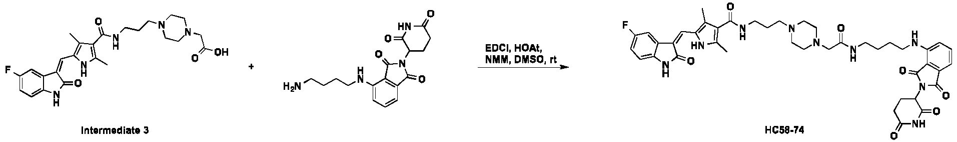

HC58-74 was synthesized following the standard procedure for preparing HC58-53 from Intermediate 3 (14.2 mg, 0.02 mmol, 1.0 equiv), 4-((4-aminobutyl)amino)-2-(2,6-dioxopiperidin-3-yl)isoindoline-1,3-dione (9.2 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-74 was obtained as yellow solid in TFA salt form (14.9 mg, 72%). ¹H NMR (600 MHz, Methanol-*d*₄) δ 13.67 (s, 1H), 7.59 - 7.52 (m, 2H), 7.41 (dd, *J* = 8.9, 2.3 Hz, 1H), 7.08 - 7.02 (m, 2H), 6.91 - 6.83 (m, 2H), 5.05 (dd, *J* = 12.7, 5.5 Hz, 1H), 3.50 (t, *J* = 6.6 Hz, 2H), 3.40 - 3.31 (m, 8H), 3.24 (s, 2H), 3.21 (t, *J* = 7.6 Hz, 2H), 3.01 - 2.80 (m, 5H), 2.79 - 2.68 (m, 2H), 2.50 (s, 3H), 2.46 (s, 3H), 2.16 - 2.01 (m, 3H), 1.73 - 1.62 (m, 4H). HRMS calcd for C₄₂H₄₉FN₉O₇ [M + H⁺] 810.3739, found 810.3745.

### Example 46

### Synthesis of HC58-75

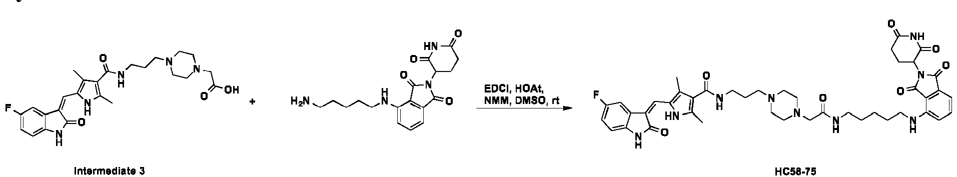

HC58-75 was synthesized following the standard procedure for preparing HC58-53 from Intermediate 3 (14.2 mg, 0.02 mmol, 1.0 equiv), 4-((5-aminopentyl)amino)-2-(2,6-dioxopiperidin-3-yl)isoindoline-1,3-dione (9.5 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-75 was obtained as yellow solid in TFA salt form (12.2 mg, 58%). ¹H NMR (600 MHz, Methanol-*d*₄) δ 13.69 (s, 1H), 7.58 (s, 1H), 7.55 (dd, *J* = 8.6, 7.1 Hz, 1H), 7.43 (dd, *J* = 8.9, 2.3 Hz, 1H), 7.05 (d, *J* = 6.4 Hz, 1H), 7.04 (d, *J* = 4.9 Hz, 1H), 6.91 - 6.84 (m, 2H), 5.05 (dd, *J* = 12.7, 5.5 Hz, 1H), 3.50 (t, *J* = 6.5 Hz, 2H), 3.37 - 3.33 (m, 6H), 3.29 (t, *J* = 6.8 Hz, 2H), 3.23 - 3.18 (m, 4H), 3.01 - 2.80 (m, 5H), 2.78 - 2.67 (m, 2H), 2.51 (s, 3H), 2.47 (s, 3H), 2.16 - 2.03 (m, 3H), 1.71 (p, *J* = 6.9 Hz, 2H), 1.61 (p, *J* = 7.0 Hz, 2H), 1.52 - 1.43 (m, 2H). HRMS calcd for C₄₃H₅₁FN₉O₇ [M + H⁺] 824.3895, found 824.3898.

### Example 47

### Synthesis of HC58-76

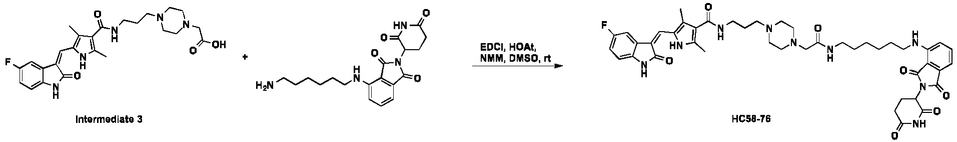

HC58-76 was synthesized following the standard procedure for preparing HC58-53 from Intermediate 3 (14.2 mg, 0.02 mmol, 1.0 equiv), 4-((6-aminohexyl)amino)-2-(2,6-dioxopiperidin-3-yl)isoindoline-1,3-dione (8.2 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-76 was obtained as yellow solid in TFA salt form (10.0 mg, 47%). ¹H NMR (600 MHz, Methanol-*d*₄) δ 13.71 (s, 1H), 7.59 (s, 1H), 7.55 (dd, *J* = 8.6, 7.1 Hz, 1H), 7.44 (dd, *J* = 8.9, 2.3 Hz, 1H), 7.04 (dd, *J* = 7.8, 4.2 Hz, 2H), 6.93 - 6.84 (m, 2H), 5.06 (dd, *J* = 12.7, 5.5 Hz, 1H), 3.51 (t, *J* = 6.5 Hz, 2H), 3.39 - 3.32 (m, 6H), 3.26 (t, *J* = 6.8 Hz, 2H), 3.23 - 3.19 (m, 4H), 3.01 - 2.81 (m, 5H), 2.79 - 2.66 (m, 2H), 2.52 (s, 3H), 2.48 (s, 3H), 2.16 - 2.02 (m, 3H), 1.69 (p, *J* = 7.1 Hz, 2H), 1.57 (p, *J* = 7.2 Hz, 2H), 1.48 (p, *J* = 7.1 Hz, 2H), 1.45 - 1.37 (m, 2H). HRMS calcd for C₄₄H₅₃FN₉O₇ [M + H⁺] 838.4052, found 838.4030.

### Example 48

### Synthesis of HC58-77

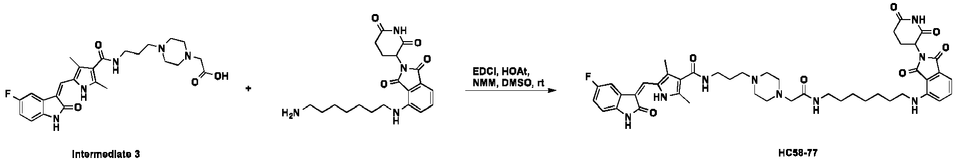

HC58-77 was synthesized following the standard procedure for preparing HC58-53 from Intermediate 3 (14.2 mg, 0.02 mmol, 1.0 equiv), 4-((7-aminoheptyl)amino)-2-(2,6-dioxopiperidin-3-yl)isoindoline-1,3-dione (10.0 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-77 was obtained as yellow solid in TFA salt form (9.7 mg, 45%). ¹H NMR (600 MHz, Methanol-*d*₄) δ 13.70 (s, 1H), 7.58 (s, 1H), 7.54 (dd, *J* = 8.6, 7.1 Hz, 1H), 7.43 (dd, *J* = 9.0, 2.3 Hz, 1H), 7.03 (dd, *J* = 7.8, 3.1 Hz, 2H), 6.92 - 6.85 (m, 2H), 5.06 (dd, *J* = 12.7, 5.5 Hz, 1H), 3.51 (t, *J* = 6.6 Hz, 2H), 3.33 - 3.29 (m, 6H), 3.27 - 3.19 (m, 6H), 3.02 - 2.81 (m, 5H), 2.78 - 2.66 (m, 2H), 2.52 (s, 3H), 2.48 (s, 3H), 2.16 - 2.04 (m, 3H), 1.67 (p, *J* = 6.9 Hz, 2H), 1.55 (q, *J* = 7.2 Hz, 2H), 1.48 - 1.33 (m, 6H). HRMS calcd for C₄₅H₅₅FN₉O₇ [M + H⁺] 852.4208, found 852.4201.

### Example 49

### Synthesis of HC58-78

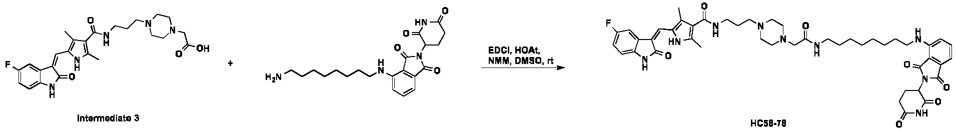

HC58-78 was synthesized following the standard procedure for preparing HC58-53 from Intermediate 3 (14.2 mg, 0.02 mmol, 1.0 equiv), 4-((8-aminooctyl)amino)-2-(2,6-dioxopiperidin-3-yl)isoindoline-1,3-dione (10.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-78 was obtained as yellow solid in TFA salt form (12.5 mg, 57%). ¹H NMR (600 MHz, Methanol-*d*₄) δ 13.69 (s, 1H), 7.58 (s, 1H), 7.54 (dd, *J* = 8.6, 7.1 Hz, 1H), 7.42 (dd, *J* = 8.9, 2.3 Hz, 1H), 7.05 - 7.00 (m, 2H), 6.92 - 6.84 (m, 2H), 5.06 (dd, *J* = 12.8, 5.5 Hz, 1H), 3.51 (t, *J* = 6.6 Hz, 2H), 3.33 - 3.29 (m, 6H), 3.23 (dd, *J* = 15.3, 8.1 Hz, 6H), 3.04 - 2.81 (m, 5H), 2.80 - 2.68 (m, 2H), 2.52 (s, 3H), 2.47 (s, 3H), 2.15 - 2.05 (m, 3H), 1.66 (p, *J* = 7.1 Hz, 2H), 1.57 - 1.50 (m, 2H), 1.48 - 1.30 (m, 8H). HRMS calcd for C₄₆H₅₇FN₉O₇ [M + H⁺] 866.4365, found 866.4346.

### Example 50

### Synthesis of intermediate 4

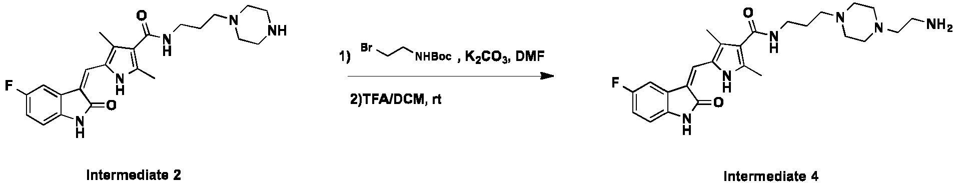

To a solution of intermediate 2 (196 mg, 0.3 mmol, 1.0 equiv) in DMF (4 mL) was added potassium carbonate (124 mg, 0.9 mmol, 3.0 equiv) and *tert*-butyl (2-bromoethyl)carbamate (140 mg, 0.6 mmol, 2.0 equiv). Then the mixture was stirred at 50 °C overnight. Water (10 mL) was added to quench the reaction. The mixture was extracted with ethyl acetate (3 x 15 mL). The organic layer was combined and washed with brine. Dried over Na₂SO₄, filtered, evaporated and afforded oil. The obtained oil was treated with DCM/TFA (2:1). After being stirred for 1 h at room temperature, the reaction mixture was concentrated and the residue was purified by reverse phase C18 column (10% - 100% methanol / 0.1% TFA in H₂O) to afford intermediate 4 as yellow solid in TFA salt form (164 mg, 67% yield for two steps). ¹H NMR (600 MHz, DMSO-*d*₆) δ 13.73 (s, 1H), 10.93 (s, 1H), 7.81 (t, *J* = 5.9 Hz, 1H), 7.77 (dd, *J* = 9.4, 2.6 Hz, 1H), 7.73 (s, 1H), 6.98 - 6.92 (m, 1H), 6.86 (dd, *J* = 8.5, 4.6 Hz, 1H), 3.54 - 3.27 (m, 10H), 3.17 - 3.11 (m, 2H), 3.02 - 2.92 (m, 4H), 2.46 (s, 3H), 2.43 (s, 3H), 1.96 - 1.87 (m, 2H). ESI m/z =469.3 [M + H⁺].

### Example 51

### Synthesis of HC58-133

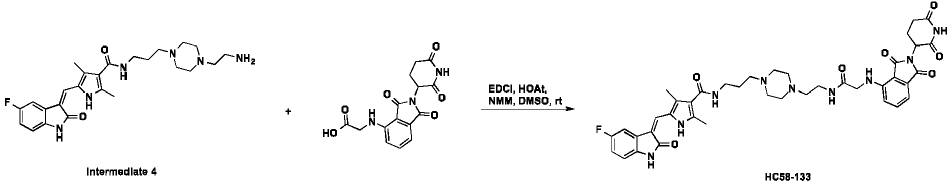

To a solution of Intermediate 4 (16.2 mg, 0.02 mmol, 1.0 equiv) in DMSO (1 mL) were added (2-(2,6-dioxopiperidin-3-yl)-1,3-dioxoisoindolin-4-yl)glycine (6.6 mg, 0.02 mmol, 1.0 equiv), EDCI (1-ethyl-3-(3-dimethylaminopropyl)carbodiimide) (5.8 mg, 0.03 mmol, 1.5 equiv), HOAt (1-hydroxy-7-azabenzo-triazole) (4.1 mg, 0.03 mmol, 1.5 equiv), and NMM (N-Methylmorpholine) (12.1 mg, 0.12 mmol, 6.0 equiv). After being stirred overnight at room temperature, the resulting mixture was purified by preparative HPLC (10%-100% MeCN/ 0.1% TFA in H₂O) to afford HC58-133 as yellow solid in TFA salt form (11.3 mg, 56%). ¹H NMR (600 MHz, Methanol-*d*₄) δ 13.65 (s, 1H), 7.63 - 7.5 7 (m, 1H), 7.55 (s, 1H), 7.40 (d, *J* = 8.7 Hz, 1H), 7.15 (d, *J* = 7.1 Hz, 1H), 6.93 (d, *J* = 8.6 Hz, 1H), 6.90 - 6.78 (m, 2H), 5.08 (dd, *J* = 12.6, 5.5 Hz, 1H), 4.04 (s, 2H), 3.54 - 3.41 (m, 6H), 3.36 - 3.22 (m, 4H), 3.17 (t, *J* = 7.6 Hz, 2H), 3.11 - 2.92 (m, 4H), 2.87 - 2.70 (m, 3H), 2.50 (s, 3H), 2.46 (s, 3H), 2.18 - 2.07 (m, 1H), 2.08 - 1.96 (m, 2H). HRMS calcd for C₄₀H₄₅FN₉O₇ [M + H⁺] 782.3426, found 782.3418.

### Example 52

### Synthesis of HC58-134

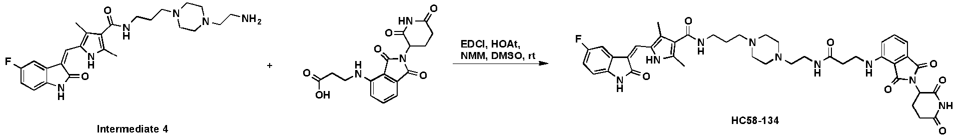

HC58-134 was synthesized following the standard procedure for preparing HC58-133 from Intermediate 4 (16.2 mg, 0.02 mmol, 1.0 equiv), 3-((2-(2,6-dioxopiperidin-3-yl)-1,3-dioxoisoindolin-4-yl)amino)propanoic acid (6.9 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-134 was obtained as yellow solid in TFA salt form (8.6 mg, 42%). ¹H NMR (600 MHz, Methanol-*d*₄) δ 13.64 (s, 1H), 7.57 (dd, *J* = 8.6, 7.1 Hz, 1H), 7.54 (s, 1H), 7.41 - 7.36 (m, 1H), 7.12 (d, *J* = 8.5 Hz, 1H), 7.06 (d, *J* = 7.1 Hz, 1H), 6.85 (q, *J* = 5.0, 3.8 Hz, 2H), 5.06 (dd, *J* = 12.7, 5.5 Hz, 1H), 3.66 (t, *J* = 6.1 Hz, 2H), 3.52 - 3.35 (m, 8H), 3.27 - 3.12 (m, 6H), 2.92 (t, *J* = 6.0 Hz, 2H), 2.84 (ddd, *J* = 17.4, 13.9, 5.3 Hz, 1H), 2.77 - 2.67 (m, 2H), 2.57 (t, *J* = 6.1 Hz, 2H), 2.50 (s, 3H), 2.46 (s, 3H), 2.14 - 2.08 (m, 1H), 2.04 (p, *J* = 6.6 Hz, 2H). HRMS calcd for C₄₁H₄₇FN₉O₇ [M + H⁺] 796.3582, found 796.3598.

### Example 53

### Synthesis of HC58-135

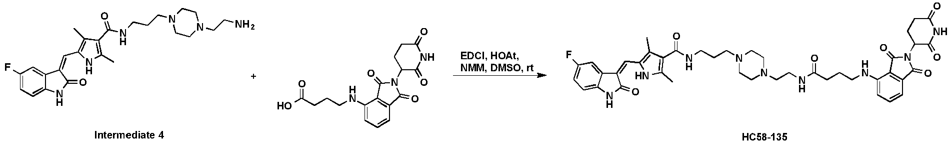

HC58-135 was synthesized following the standard procedure for preparing HC58-133 from Intermediate 4 (16.2 mg, 0.02 mmol, 1.0 equiv), 4-((2-(2,6-dioxopiperidin-3-yl)-1,3-dioxoisoindolin-4-yl)amino)butanoic acid (7.2 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-135 was obtained as yellow solid in TFA salt form (12.9 mg, 62%). ¹H NMR (600 MHz, Methanol-*d*₄) δ 13.65 (s, 1H), 7.59 - 7.52 (m, 2H), 7.40 (d, *J* = 8.9 Hz, 1H), 7.07 (d, *J* = 8.6 Hz, 1H), 7.04 (d, *J* = 7.1 Hz, 1H), 6.91 - 6.82 (m, 2H), 5.06 (ddd, *J* = 12.8, 5.5, 1.9 Hz, 1H), 3.49 (t, *J* = 6.4 Hz, 2H), 3.45 - 3.36 (m, 4H), 3.36 - 3.28 (m, 4H), 3.18 - 3.02 (m, 6H), 2.88 - 2.82 (m, 2H), 2.79 - 2.67 (m, 3H), 2.49 (s, 3H), 2.45 (s, 3H), 2.38 - 2.32 (m, 2H), 2.16 - 2.08 (m, 1H), 2.08 - 1.94 (m, 4H). HRMS calcd for C₄₂H₄₉FN₉O₇ [M + H⁺] 810.3739, found 810.3728.

### Example 54

### Synthesis of HC58-136

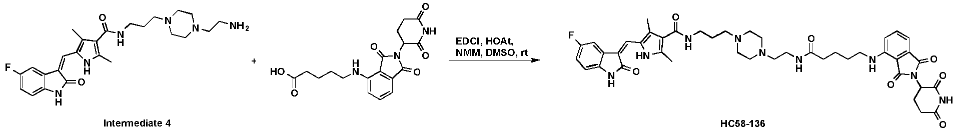

HC58-136 was synthesized following the standard procedure for preparing HC58-133 from Intermediate 4 (16.2 mg, 0.02 mmol, 1.0 equiv), 5-((2-(2,6-dioxopiperidin-3-yl)-1,3-dioxoisoindolin-4-yl)amino)pentanoic acid (7.5 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-136 was obtained as yellow solid in TFA salt form (9.9 mg, 47%). ¹H NMR (600 MHz, Acetone-*d*₆) δ 13.54 (s, 1H), 9.80 (s, 1H), 9.74 (s, 1H), 7.51 - 7.44 (m, 2H), 7.34 (dd, *J* = 9.1, 6.6 Hz, 1H), 7.30 (dd, *J* = 9.1, 2.3 Hz, 1H), 7.04 (t, *J* = 6.0 Hz, 1H), 6.84 (d, *J* = 8.5 Hz, 1H), 6.79 (d, *J* = 7.0 Hz, 1H), 6.70 (dd, *J* = 8.4, 4.5 Hz, 1H), 6.68 - 6.63 (m, 1H), 6.20 (s, 1H), 4.85 (dd, *J* = 12.4, 5.4 Hz, 1H), 3.62 - 3.45 (m, 8H), 3.43 (q, *J* = 5.8 Hz, 2H), 3.31 (q, *J* = 6.3 Hz, 2H), 3.19 - 3.03 (m, 6H), 2.78 - 2.67 (m, 1H), 2.59 - 2.48 (m, 2H), 2.29 (s, 3H), 2.25 (s, 3H), 2.03 (t, *J* = 6.8 Hz, 2H), 2.00 - 1.88 (m, 3H), 1.55 - 1.41 (m, 4H). HRMS calcd for C₄₃H₅₁FN₉O₇ [M + H⁺] 824.3895, found 824.3889.

### Example 55

### Synthesis of HC58-137

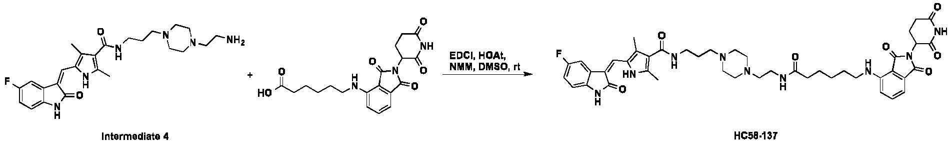

HC58-137 was synthesized following the standard procedure for preparing HC58-133 from Intermediate 4 (16.2 mg, 0.02 mmol, 1.0 equiv), 6-((2-(2,6-dioxopiperidin-3-yl)-1,3-dioxoisoindolin-4-yl)amino)hexanoic acid (7.8 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-137 was obtained as yellow solid in TFA salt form (11.3 mg, 53%). ¹H NMR (600 MHz, Acetone-*d*₆) δ 13.78 (s, 1H), 10.03 (s, 1H), 9.98 (s, 1H), 7.74 - 7.67 (m, 2H), 7.58 (dd, *J* = 8.6, 7.0 Hz, 1H), 7.54 (dd, *J* = 9.2, 2.5 Hz, 1H), 7.31 - 7.25 (m, 1H), 7.08 (d, *J* = 8.5 Hz, 1H), 7.03 (d, *J* = 7.0 Hz, 1H), 6.94 (dd, *J* = 8.4, 4.5 Hz, 1H), 6.90 (td, *J* = 8.9, 2.5 Hz, 1H), 6.40 (s, 1H), 5.08 (dd, *J* = 12.5, 5.5 Hz, 1H), 3.90 - 3.71 (m, 8H), 3.70 - 3.63 (m, 2H), 3.60 - 3.52 (m, 2H), 3.41 - 3.30 (m, 6H), 3.03 - 2.91 (m, 1H), 2.83 - 2.72 (m, 2H), 2.53 (s, 3H), 2.49 (s, 3H), 2.27 - 2.13 (m, 5H), 1.74 - 1.62 (m, 4H), 1.50 - 1.40 (m, 2H). HRMS calcd for C₄₄H₅₃FN₉O₇ [M + H⁺] 838.4052, found 838.4070.

### Example 56

### Synthesis of HC58-138

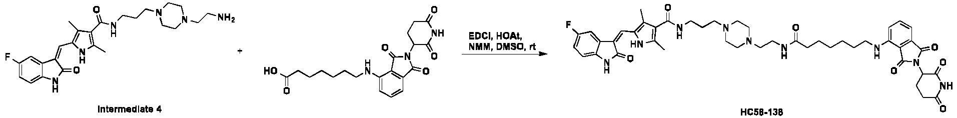

HC58-138 was synthesized following the standard procedure for preparing HC58-133 from Intermediate 4 (16.2 mg, 0.02 mmol, 1.0 equiv), 7-((2-(2,6-dioxopiperidin-3-yl)-1,3-dioxoisoindolin-4-yl)amino)heptanoic acid (8.0 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-138 was obtained as yellow solid in TFA salt form (11.7 mg, 54%). ¹H NMR (600 MHz, Acetone-*d*₆) δ 13.78 (s, 1H), 10.01 (s, 1H), 9.98 (s, 1H), 7.71 (s, 1H), 7.61 - 7.5 3 (m, 2H), 7.24 (t, *J* = 6.0 Hz, 1H), 7.09 (d, *J* = 8.6 Hz, 1H), 7.03 (d, *J* = 7.0 Hz, 1H), 6.94 (dd, *J* = 8.4, 4.5 Hz, 1H), 6.90 (td, *J* = 8.9, 2.5 Hz, 1H), 6.41 (s, 1H), 5.08 (dd, *J* = 12.5, 5.5 Hz, 1H), 3.67 - 3.44 (m, 12H), 3.37 (t, *J* = 7.1 Hz, 2H), 3.25 (t, *J* = 7.7 Hz, 2H), 3.14 (t, *J* = 5.8 Hz, 2H), 3.02 - 2.92 (m, 1H), 2.84-2.72 (m, 2H), 2.53 (s, 3H), 2.50 (s, 3H), 2.28 - 2.15 (m, 3H), 2.15 - 2.10 (m, 2H), 1.69 (p, *J* = 7.3 Hz, 2H), 1.61 (p, *J* = 7.5 Hz, 2H), 1.45 (p, *J* = 7.1 Hz, 2H), 1.41 - 1.34 (m, 2H). HRMS calcd for C₄₅H₅₅FN₉O₇ [M + H⁺] 852.4208, found 852.4224.

### Example 57

### Synthesis of HC58-139

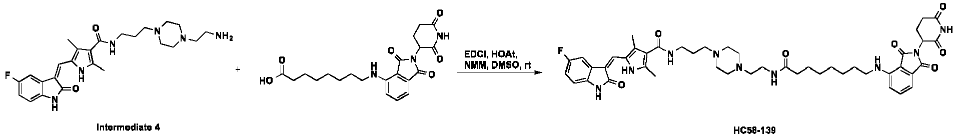

HC58-139 was synthesized following the standard procedure for preparing HC58-133 from Intermediate 4 (16.2 mg, 0.02 mmol, 1.0 equiv), 8-((2-(2,6-dioxopiperidin-3-yl)-1,3-dioxoisoindolin-4-yl)amino)octanoic acid (8.3 mg, 0.02 mmol, 1.0 equiv), EDCI (5.8 mg, 0.03 mmol, 1.5 equiv), HOAt (4.1 mg, 0.03 mmol, 1.5 equiv), and NMM (12.1 mg, 0.12 mmol, 6.0 equiv) in DMSO (1 mL). HC58-139 was obtained as yellow solid in TFA salt form (11.8 mg, 54%). ¹H NMR (600 MHz, Acetone-*d*₆) δ 13.79 (s, 1H), 9.99 (s, 1H), 9.96 (s, 1H), 7.71 (s, 1H), 7.64 - 7.52 (m, 2H), 7.26 (s, 1H), 7.09 (d, *J* = 8.6 Hz, 1H), 7.03 (d, *J* = 7.1 Hz, 1H), 6.97 - 6.87 (m, 2H), 6.41 (s, 1H), 5.15 - 4.99 (m, 1H), 3.81 - 3.67 (m, 8H), 3.66 - 3.61 (m, 2H), 3.58 - 3.52 (m, 2H), 3.39 - 3.32 (m, 4H), 3.31 - 3.23 (m, 2H), 3.01 - 2.92 (m, 1H), 2.84 - 2.72 (m, 2H), 2.53 (s, 3H), 2.50 (s, 3H), 2.26 - 2.12 (m, 5H), 1.75 - 1.64 (m, 2H), 1.63 - 1.55 (m, 2H), 1.49 - 1.29 (m, 6H). HRMS calcd for C₄₆H₅₇FN₉O₇ [M + H⁺] 866.4365, found 866.4380.

### Example 58

### Synthesis of intermediate 5

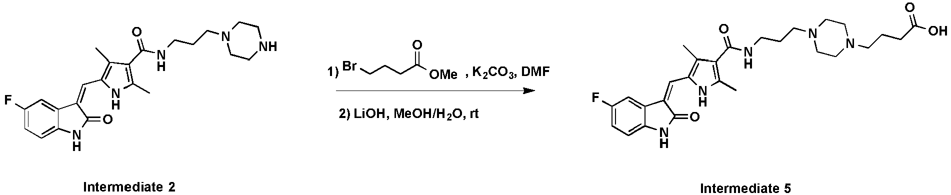

To a solution of intermediate 2 (196 mg, 0.3 mmol, 1.0 equiv) in DMF (4 mL) was added potassium carbonate (124 mg, 0.9 mmol, 3.0 equiv) and methyl 4-bromobutanoate (109 mg, 0.6 mmol, 2.0 equiv). Then the mixture was stirred at 60 °C overnight. Water (10 mL) was added to quench the reaction. The mixture was extracted with ethyl acetate (3 x 15 mL). The organic layer was combined and washed with brine. Dried over Na₂SO₄, filtered, evaporated and afforded solid. The obtained solid was dissolved in MeOH/H₂O (2:1). To the solution was added LiOH (22 mg, 0.9 mmol, 3 equiv). After stirring overnight at room temperature, the reaction mixture was concentrated and the residue was purified by reverse phase C18 column (10% - 100% methanol / 0.1% TFA in H₂O) to afford intermediate 5 as yellow solid in TFA salt form (154 mg, 69% yield for two steps). ¹H NMR (600 MHz, Methanol-*d*₄) δ 7.59 (s, 1H), 7.42 (d, *J* = 8.3 Hz, 1H), 6.92 - 6.85 (m, 2H), 3.51 (t, *J* = 6.6 Hz, 2H), 3.48 - 3.36 (m, 8H), 3.15 (t, *J* = 7.5 Hz, 2H), 3.09 (t, *J* = 7.9 Hz, 2H), 2.52 (s, 3H), 2.50 - 2.44 (m, 5H), 2.09 - 1.96 (m, 4H).
ESI m/z =512.3 [M + H⁺].

### Example 59

### Synthesis of HC58-144

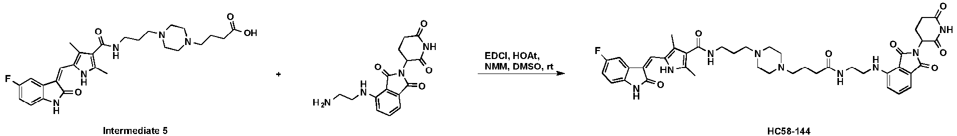

To a solution of Intermediate 5 (14.8 mg, 0.02 mmol, 1.0 equiv) in DMSO (1 mL) were added 4-((2-aminoethyl)amino)-2-(2,6-dioxopiperidin-3-yl)isoindoline-1,3-dione (9.5 mg, 0.022 mmol, 1.1 equiv), EDCI (1-ethyl-3-(3-dimethylaminopropyl)carbodiimide) (5.8 mg, 0.03 mmol, 1.5 equiv), HOAt (1-hydroxy-7-azabenzo-triazole) (4.1 mg, 0.03 mmol, 1.5 equiv), and NMM (N-Methylmorpholine) (10.1 mg, 0.1 mmol, 5.0 equiv). After being stirred overnight at room temperature, the resulting mixture was purified by preparative HPLC (10%-100% MeOH/ 0.1% TFA in H₂O) to afford HC58-144 as yellow solid in TFA salt form (11.6 mg, 56%). ¹H NMR (600 MHz, Acetone-*d*₆) δ 13.82 (s, 1H), 10.10 (s, 1H), 9.95 (s, 1H), 7.99 (s, 1H), 7.72 (s, 1H), 7.60 (dd, *J* = 8.6, 7.0 Hz, 1H), 7.55 (dd, *J* = 9.1, 2.5 Hz, 1H), 7.35 (s, 1H), 7.16 (d, *J* = 8.5 Hz, 1H), 7.06 (d, *J* = 7.0 Hz, 1H), 7.00 - 6.86 (m, 2H), 5.11 (dd, *J* = 12.7, 5.5 Hz, 1H), 3.99 - 3.72 (m, 8H), 3.64 - 3.50 (m, 6H), 3.49 - 3.38 (m, 4H), 3.02 - 2.91 (m, 1H), 2.81- 2.71 (m, 2H), 2.54 (s, 3H), 2.51 (s, 3H), 2.27 - 2.16 (m, 2H), 2.14 - 2.01 (m, 5H). HRMS calcd for C₄₂H₄₉FN₉O₇ [M + H⁺] 810.3739, found 810.3747.

### Example 60

### Synthesis of HC58-145

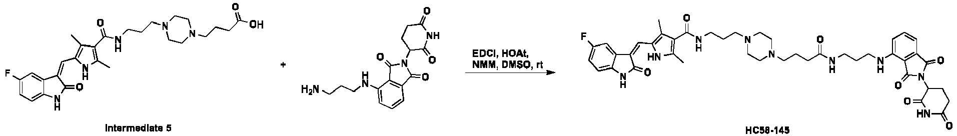

HC58-145 was synthesized following the standard procedure for preparing HC58-144 from Intermediate 5 (14.8 mg, 0.02 mmol, 1.0 equiv), 4-((3-aminopropyl)amino)-2-(2,6-dioxopiperidin-3-yl)isoindoline-1,3-dione (9.8 mg, 0.022 mmol, 1.1 equiv), EDCI (5.8 mg, 0.03 mmol, 1.5 equiv), HOAt (4.1 mg, 0.03 mmol, 1.5 equiv), and NMM (10.1 mg, 0.1 mmol, 5.0 equiv) in DMSO (1 mL). HC58-145 was obtained as yellow solid in TFA salt form (10.3 mg, 49%). ¹H NMR (600 MHz, Acetone-*d*₆) δ 13.77 (s, 1H), 10.07 (s, 1H), 10.00 (s, 1H), 7.70 (s, 1H), 7.63 (s, 1H), 7.58 (dd, *J* = 8.6, 7.0 Hz, 1H), 7.54 (dd, *J* = 9.1, 2.5 Hz, 1H), 7.24 - 7.18 (m, 1H), 7.09 (d, *J* = 8.6 Hz, 1H), 7.03 (d, *J* = 7.1 Hz, 1H), 6.94 (dd, *J* = 8.5, 4.5 Hz, 1H), 6.90 (td, *J* = 8.9, 2.5 Hz, 1H), 6.61 (s, 1H), 5.09 (dd, *J* = 12.5, 5.6 Hz, 1H), 3.65 - 3.48 (m, 10H), 3.46- 3.40 (m, 2H), 3.38- 3.32 (m, 2H), 3.23 (t, *J* = 7.7 Hz, 2H), 3.18 (t, *J* = 7.4 Hz, 2H), 2.96 (ddd, *J* = 14.8, 10.5, 7.4 Hz, 1H), 2.82 - 2.71 (m, 2H), 2.52 (s, 3H), 2.49 (s, 3H), 2.41 (t, *J* = 6.8 Hz, 2H), 2.26 - 2.18 (m, 1H), 2.12 - 2.08 (m, 4H), 1.89 - 1.77 (m, 2H). HRMS calcd for C₄₃H₅₁FN₉O₇ [M + H⁺] 824.3895, found 824.3889.

### Example 61

### Synthesis of HC58-146

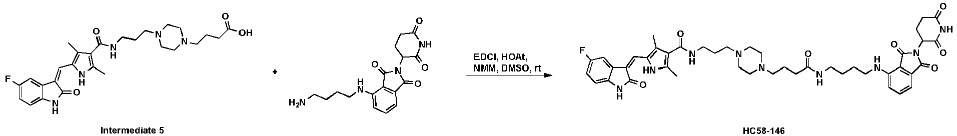

HC58-146 was synthesized following the standard procedure for preparing HC58-144 from Intermediate 5 (14.8 mg, 0.02 mmol, 1.0 equiv), 4-((4-aminobutyl)amino)-2-(2,6-dioxopiperidin-3-yl)isoindoline-1,3-dione (10.0 mg, 0.022 mmol, 1.1 equiv), EDCI (5.8 mg, 0.03 mmol, 1.5 equiv), HOAt (4.1 mg, 0.03 mmol, 1.5 equiv), and NMM (10.1 mg, 0.1 mmol, 5.0 equiv) in DMSO (1 mL). HC58-146 was obtained as yellow solid in TFA salt form (8.3 mg, 39%). ¹H NMR (600 MHz, Acetone-*d*₆) δ 13.77 (s, 1H), 10.06 (s, 1H), 10.00 (s, 1H), 7.70 (s, 1H), 7.62-7.56 (m, 1H), 7.54 (dd, *J* = 9.2, 2.5 Hz, 1H), 7.48 (t, *J* = 5.8 Hz, 1H), 7.23 (t, *J* = 6.0 Hz, 1H), 7.10 (d, *J* = 8.6 Hz, 1H), 7.03 (d, *J* = 7.1 Hz, 1H), 6.94 (dd, *J* = 8.4, 4.5 Hz, 1H), 6.90 (td, *J* = 9.0, 2.5 Hz, 1H), 6.42 (s, 1H), 5.09 (dd, *J* = 12.5, 5.5 Hz, 1H), 3.68 - 3.49 (m, 10H), 3.40 (t, *J* = 7.1 Hz, 2H), 3.28 - 3.20 (m, 4H), 3.15 (t, *J* = 7.5 Hz, 2H), 2.97 (ddd, *J* = 18.8, 14.7, 5.2 Hz, 1H), 2.83 - 2.72 (m, 2H), 2.52 (s, 3H), 2.49 (s, 3H), 2.36 (t, *J* = 6.9 Hz, 2H), 2.21 (dt, *J* = 10.8, 5.3 Hz, 1H), 2.14-2.02 (m, 4H), 1.72 (p, *J* = 7.1 Hz, 2H), 1.63 (p, *J* = 7.0 Hz, 2H). HRMS calcd for C₄₄H₅₃FN₉O₇ [M + H⁺] 838.4052, found 838.4068.

### Example 62

### Synthesis of HC58-147

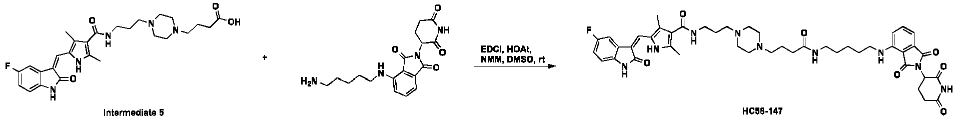

HC58-147 was synthesized following the standard procedure for preparing HC58-144 from Intermediate 5 (14.8 mg, 0.02 mmol, 1.0 equiv), 4-((5-aminopentyl)amino)-2-(2,6-dioxopiperidin-3-yl)isoindoline-1,3-dione (11.0 mg, 0.022 mmol, 1.1 equiv), EDCI (5.8 mg, 0.03 mmol, 1.5 equiv), HOAt (4.1 mg, 0.03 mmol, 1.5 equiv), and NMM (10.1 mg, 0.1 mmol, 5.0 equiv) in DMSO (1 mL). HC58-147 was obtained as yellow solid in TFA salt form (9.5 mg, 44%). ¹H NMR (600 MHz, Acetone-*d*₆) δ 13.79 (s, 1H), 10.02 (s, 1H), 9.97 (s, 1H), 7.72 (s, 1H), 7.59 (dd, *J* = 8.6, 7.0 Hz, 1H), 7.55 (dd, *J* = 9.2, 2.5 Hz, 1H), 7.42 - 7.36 (m, 1H), 7.25 - 7.18 (m, 1H), 7.09 (d, *J* = 8.5 Hz, 1H), 7.04 (d, *J* = 7.1 Hz, 1H), 6.94 (dd, *J* = 8.4, 4.5 Hz, 1H), 6.90 (td, *J* = 8.9, 2.5 Hz, 1H), 6.41 (s, 1H), 5.09 (dd, *J* = 12.5, 5.4 Hz, 1H), 3.74 - 3.49 (m, 10H), 3.37 (t, *J* = 7.1 Hz, 2H), 3.28 - 3.20 (m, 4H), 3.17 (t, *J* = 7.5 Hz, 2H), 2.97 (ddd, *J* = 18.1, 14.8, 5.3 Hz, 1H), 2.84 - 2.73 (m, 2H), 2.53 (s, 3H), 2.50 (s, 3H), 2.36 (t, *J* = 6.8 Hz, 2H), 2.25 - 2.19 (m, 1H), 2.15 - 2.06 (m, 4H), 1.78 - 1.67 (m, 2H), 1.63 - 1.53 (m, 2H), 1.51 - 1.43 (m, 2H). HRMS calcd for C₄₅H₅₅FN₉O₇ [M + H⁺] 852.4208, found 852.4201.

### Example 63

### Synthesis of HC58-148

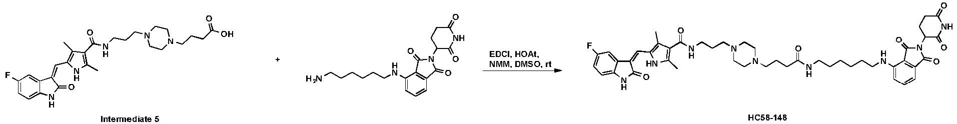

HC58-148 was synthesized following the standard procedure for preparing HC58-144 from Intermediate 5 (14.8 mg, 0.02 mmol, 1.0 equiv), 4-((6-aminohexyl)amino)-2-(2,6-dioxopiperidin-3-yl)isoindoline-1,3-dione (9.0 mg, 0.022 mmol, 1.1 equiv), EDCI (5.8 mg, 0.03 mmol, 1.5 equiv), HOAt (4.1 mg, 0.03 mmol, 1.5 equiv), and NMM (10.1 mg, 0.1 mmol, 5.0 equiv) in DMSO (1 mL). HC58-148 was obtained as yellow solid in TFA salt form (9.2 mg, 42%).¹H NMR (600 MHz, Acetone-*d*₆) δ 13.78 (s, 1H), 10.02 (s, 1H), 10.00 (s, 1H), 7.71 (s, 1H), 7.59 (dd, *J* = 8.5, 7.1 Hz, 1H), 7.55 (dd, *J* = 9.2, 2.5 Hz, 1H), 7.35 (s, 1H), 7.20 (t, *J* = 5.9 Hz, 1H), 7.09 (d, *J* = 8.5 Hz, 1H), 7.03 (d, *J* = 7.1 Hz, 1H), 6.94 (dd, *J* = 8.4, 4.5 Hz, 1H), 6.90 (td, *J* = 9.0, 2.5 Hz, 1H), 6.42 (s, 1H), 5.08 (dd, *J* = 12.4, 5.5 Hz, 1H), 3.59 - 3.43 (m, 10H), 3.40 - 3.34 (m, 2H), 3.23 - 3.15 (m, 4H), 3.08 (t, *J* = 7.5 Hz, 2H), 2.97 (ddd, *J* = 18.5, 15.0, 5.2 Hz, 1H), 2.82- 2.72 (m, 2H), 2.53 (s, 3H), 2.50 (s, 3H), 2.33 (t, *J* = 6.9 Hz, 2H), 2.26 - 2.18 (m, 1H), 2.11 - 2.07 (m, 2H), 2.04 - 1.99 (m, 2H), 1.69 (p, *J* = 7.2 Hz, 2H), 1.55 - 1.35 (m, 6H). HRMS calcd for C₄₆H₅₇FN₉O₇ [M + H⁺] 866.4365, found 866.4381.

### Example 64

### Synthesis of HC58-149

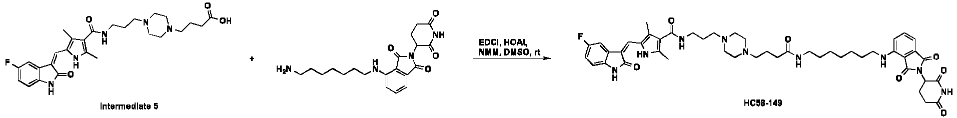

HC58-149 was synthesized following the standard procedure for preparing HC58-144 from Intermediate 5 (14.8 mg, 0.02 mmol, 1.0 equiv), 4-((7-aminoheptyl)amino)-2-(2,6-dioxopiperidin-3-yl)isoindoline-1,3-dione (11.0 mg, 0.022 mmol, 1.1 equiv), EDCI (5.8 mg, 0.03 mmol, 1.5 equiv), HOAt (4. 1 mg, 0.03 mmol, 1.5 equiv), and NMM (10.1 mg, 0.1 mmol, 5.0 equiv) in DMSO (1 mL). HC58-149 was obtained as yellow solid in TFA salt form (9.8 mg, 44%). ¹H NMR (600 MHz, Acetone-*d*₆) δ 13.78 (s, 1H), 10.02 (s, 1H), 9.99 (s, 1H), 7.71 (s, 1H), 7.59 (t, *J* = 7.8 Hz, 1H), 7.55 (dd, *J* = 9.1, 2.4 Hz, 1H), 7.34 (s, 1H), 7.20 (s, 1H), 7.09 (d, *J* = 8.5 Hz, 1H), 7.03 (d, *J* = 7.0 Hz, 1H), 6.94 (dd, *J* = 8.5, 4.5 Hz, 1H), 6.90 (td, *J* = 9.0, 2.5 Hz, 1H), 6.41 (s, 1H), 5.08 (dd, *J* = 12.4, 5.4 Hz, 1H), 3.60 - 3.43 (m, 10H), 3.37 (t, *J* = 7.2 Hz, 2H), 3.22-3.16 (m, 4H), 3.09 (t, *J* = 7.6 Hz, 2H), 2.97 (ddd, *J* = 19.7, 15.2, 5.3 Hz, 1H), 2.84 - 2.72 (m, 2H), 2.53 (s, 3H), 2.50 (s, 3H), 2.33 (t, *J* = 6.9 Hz, 2H), 2.22 (dt, *J* = 10.9, 5.8 Hz, 1H), 2.11 - 2.00 (m, 4H), 1.69 (p, *J* = 7.3 Hz, 2H), 1.53 - 1.31 (m, 8H). HRMS calcd for C₄₇H₅₉FN₉O₇ [M+ H⁺] 880.4521, found 880.4529.

### Example 65

### Synthesis of HC58-150

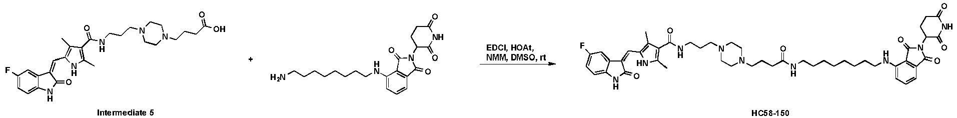

HC58-150 was synthesized following the standard procedure for preparing HC58-144 from Intermediate 5 (14.8 mg, 0.02 mmol, 1.0 equiv), 4-((8-aminooctyl)amino)-2-(2,6-dioxopiperidin-3-yl)isoindoline-1,3-dione (11.3 mg, 0.022 mmol, 1.1 equiv), EDCI (5.8 mg, 0.03 mmol, 1.5 equiv), HOAt (4.1 mg, 0.03 mmol, 1.5 equiv), and NMM (10.1 mg, 0.1 mmol, 5.0 equiv) in DMSO (1 mL). HC58-150 was obtained as yellow solid in TFA salt form (8.5 mg, 38%). ¹H NMR (600 MHz, Acetone-*d*₆) δ 13.78 (s, 1H), 10.02 (s, 1H), 10.00 (s, 1H), 7.71 (s, 1H), 7.59 (dd, *J* = 8.6, 7.0 Hz, 1H), 7.55 (dd, *J* = 9.2, 2.5 Hz, 1H), 7.34 (s, 1H), 7.20 (t, *J* = 5.9 Hz, 1H), 7.09 (d, *J* = 8.5 Hz, 1H), 7.03 (d, *J* = 7.0 Hz, 1H), 6.95 (dd, *J* = 8.5, 4.5 Hz, 1H), 6.90 (td, *J* = 9.0, 2.5 Hz, 1H), 6.42 (s, 1H), 5.08 (dd, *J* = 12.6, 5.5 Hz, 1H), 3.56 - 3.32 (m, 12H), 3.21 - 3.13 (m, 4H), 3.07 (t, *J* = 7.5 Hz, 2H), 3.01 - 2.92 (m, 1H), 2.83 - 2.73 (m, 2H), 2.53 (s, 3H), 2.50 (s, 3H), 2.33 (t, *J* = 6.9 Hz, 2H), 2.27 - 2.19 (m, 1H), 2.13 - 2.06 (m, 2H), 2.01 (p, *J* = 7.0 Hz, 2H), 1.69 (p, *J* = 7.2 Hz, 2H), 1.53 - 1.28 (m, 10H). HRMS calcd for C₄₈H₆₁FN₉O₇ [M + H⁺] 894.4678, found 894.4689.

### Example 66

### Synthesis of HC58-158

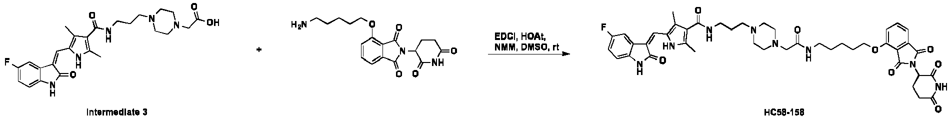

HC58-158 was synthesized following the standard procedure for preparing HC58-53 from Intermediate 3 (12.0 mg, 0.017 mmol, 1.0 equiv), 4-((5-aminopentyl)oxy)-2-(2,6-dioxopiperidin-3-yl)isoindoline-1,3-dione (9.5 mg, 0.02 mmol, 1.2 equiv), EDCI (5.0 mg, 0.026 mmol, 1.5 equiv), HOAt (4.0 mg, 0.026 mmol, 1.5 equiv), and NMM (8.6 mg, 0.085 mmol, 5.0 equiv) in DMSO (1 mL). HC58-158 was obtained as yellow solid in TFA salt form (8.2 mg, 46%). ¹H NMR (600 MHz, Methanol-*d*₄) δ 13.63 (s, 1H), 7.74 (dd, *J* = 8.4, 7.3 Hz, 1H), 7.51 (s, 1H), 7.43 - 7.35 (m, 3H), 6.89 - 6.81 (m, 2H), 5.10 (dd, *J* = 12.7, 5.5 Hz, 1H), 4.20 (t, *J* = 6.1 Hz, 2H), 3.49 (t, *J* = 6.6 Hz, 2H), 3.44 - 3.29 (m, 8H), 3.22 (t, *J* = 7.5 Hz, 2H), 3.07 - 2.91 (m, 4H), 2.87 (ddd, *J* = 17.3, 13.9, 5.4 Hz, 1H), 2.79 - 2.68 (m, 2H), 2.48 (s, 3H), 2.44 (s, 3H), 2.19-2.10 (m, 1H), 2.06 (p, *J* = 6.7 Hz, 2H), 1.87 (p, *J* = 6.3 Hz, 2H), 1.68 - 1.55 (m, 4H). HRMS calcd for C₄₃H₅₀FN₈O₈ [M + H⁺] 825.3736, found 825.3749.

### Example 67

### Synthesis of HC58-159

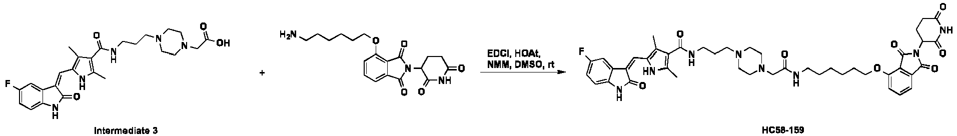

HC58-159 was synthesized following the standard procedure for preparing HC58-53 from Intermediate 3 (12.0 mg, 0.017 mmol, 1.0 equiv), 4-((6-aminohexyl)oxy)-2-(2,6-dioxopiperidin-3-yl)isoindoline-1,3-dione (9.7 mg, 0.02 mmol, 1.2 equiv), EDCI (5.0 mg, 0.026 mmol, 1.5 equiv), HOAt (4.0 mg, 0.026 mmol, 1.5 equiv), and NMM (8.6 mg, 0.085 mmol, 5.0 equiv) in DMSO (1 mL). HC58-159 was obtained as yellow solid in TFA salt form (8.0 mg, 44%). ¹H NMR (600 MHz, Methanol-*d*₄) δ 13.63 (s, 1H), 7.73 (dd, *J* = 8.5, 7.4 Hz, 1H), 7.52 (s, 1H), 7.43 - 7.34 (m, 3H), 6.90 - 6.81 (m, 2H), 5.10 (dd, *J* = 12.8, 5.5 Hz, 1H), 4.20 (t, *J* = 6.2 Hz, 2H), 3.58 - 3.38 (m, 6H), 3.35 - 3.33 (m, 2H), 3.29 - 3.21 (m, 4H), 3.03 (s, 4H), 2.87 (ddd, *J* = 17.7, 14.1, 5.3 Hz, 1H), 2.79 - 2.67 (m, 2H), 2.48 (s, 3H), 2.44 (s, 3H), 2.18 - 2.03 (m, 3H), 1.85 (p, *J* = 6.3 Hz, 2H), 1.63 - 1.53 (m, 4H), 1.49 - 1.40 (m, 2H). HRMS calcd for C₄₄H₅₂FN₈O₈ [M + H⁺] 839.3892, found 839.3881.

### Example 68

### Synthesis of HC58-160

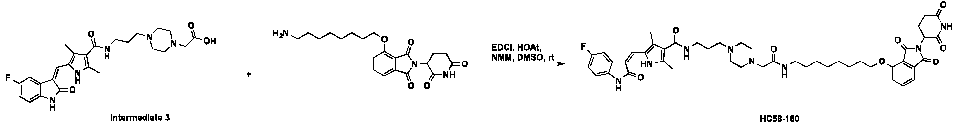

HC58-160 was synthesized following the standard procedure for preparing HC58-53 from Intermediate 3 (5.9 mg, 0.0083 mmol, 1.0 equiv), 4-((8-aminooctyl)oxy)-2-(2,6-dioxopiperidin-3-yl)isoindoline-1,3-dione (4.7 mg, 0.009 mmol, 1.1 equiv), EDCI (2.3 mg, 0.012 mmol, 1.5 equiv), HOAt (2.0 mg, 0.012 mmol, 1.5 equiv), and NMM (4.2 mg, 0.042 mmol, 5.0 equiv) in DMSO (0.6 mL). HC58-160 was obtained as yellow solid in TFA salt form (4.5 mg, 49%). ¹H NMR (600 MHz, Methanol-*d*₄) δ 13.68 (s, 1H), 7.76 - 7.72 (m, 1H), 7.57 (s, 1H), 7.44 - 7.39 (m, 3H), 6.90 - 6.84 (m, 2H), 5.11 (dd, *J* = 12.8, 5.5 Hz, 1H), 4.20 (t, *J* = 6.3 Hz, 2H), 3.50 (t, *J* = 6.5 Hz, 2H), 3.47 - 3.36 (m, 4H), 3.29 - 3.21 (m, 6H), 3.08 - 2.84 (m, 5H), 2.79 - 2.66 (m, 2H), 2.50 (s, 3H), 2.46 (s, 3H), 2.16 - 2.05 (m, 3H), 1.84 (p, *J* = 6.5 Hz, 2H), 1.59 - 1.49 (m, 4H), 1.46 - 1.34 (m, 6H). HRMS calcd for C₄₆H₅₆FN₈O₈ [M + H⁺] 867.4205, found 867.4221.

### Example 69

### Synthesis of HC58-161

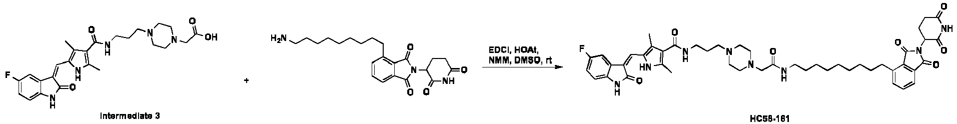

HC58-161 was synthesized following the standard procedure for preparing HC58-53 from Intermediate 3 (12.0 mg, 0.017 mmol, 1.0 equiv), 4-((5-aminopentyl)oxy)-2-(2,6-dioxopiperidin-3-yl)isoindoline-1,3-dione (9.4 mg, 0.018 mmol, 1.1 equiv), EDCI (5.0 mg, 0.026 mmol, 1.5 equiv), HOAt (4.0 mg, 0.026 mmol, 1.5 equiv), and NMM (8.6 mg, 0.085 mmol, 5.0 equiv) in DMSO (1 mL). HC58-161 was obtained as yellow solid in TFA salt form (6.5 mg, 35%). ¹H NMR (600 MHz, Methanol-*d*₄) δ 13.66 (s, 1H), 7.71 - 7.67 (m, 2H), 7.62 - 7.58 (m, 1H), 7.53 (s, 1H), 7.39 (dd, *J* = 8.9, 2.4 Hz, 1H), 6.90 - 6.82 (m, 2H), 5.13 (dd, *J* = 12.7, 5.5 Hz, 1H), 3.55 - 3.39 (m, 6H), 3.28 - 3.21 (m, 4H), 3.14 - 2.95 (m, 6H), 2.89 (ddd, *J* = 17.3, 13.8, 5.3 Hz, 1H), 2.80 - 2.69 (m, 2H), 2.50 (s, 3H), 2.46 (s, 3H), 2.19 - 2.04 (m, 3H), 1.65 (p, *J* = 7.5 Hz, 2H), 1.57- 1.49 (m, 2H), 1.43 - 1.27 (m, 12H). HRMS calcd for C₄₇H₅₈FN₈O₇ [M + H⁺] 865.4412, found 865.4430.

### Example 70

### Synthesis of HC58-164

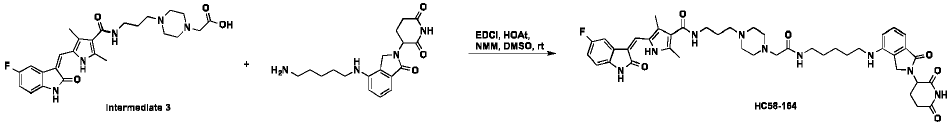

HC58-164 was synthesized following the standard procedure for preparing HC58-53 from Intermediate 3 (14.0 mg, 0.02 mmol, 1.0 equiv), 3-(4-((5-aminopentyl)amino)-1-oxoisoindolin-2-yl)piperidine-2,6-dione (10.0 mg, 0.022 mmol, 1.1 equiv), EDCI (5.8 mg, 0.03mmol, 1.5 equiv), HOAt (4.1 mg, 0.03 mmol, 1.5 equiv), and NMM (10.1 mg, 0.1 mmol, 5.0 equiv) in DMSO (1 mL). HC58-164 was obtained as yellow solid in TFA salt form (8.9 mg, 35%). ¹H NMR (600 MHz, Methanol-*d*₄) δ 13.70 (s, 1H), 7.58 (s, 1H), 7.46 - 7.41 (m, 2H), 7.39 (d, *J* = 7.3 Hz, 1H), 7.20 - 7.15 (m, 1H), 6.92 - 6.84 (m, 2H), 5.17 (dd, *J* = 13.5, 5.2 Hz, 1H), 4.46 (d, *J* = 16.9 Hz, 1H), 4.38 (d, *J* = 16.9 Hz, 1H), 3.86 - 3.73 (m, 2H), 3.51 - 3.44 (m, 2H), 3.40 - 3.21 (m, 8H), 3.15 (t, *J* = 7.6 Hz, 2H), 3.06 - 2.88 (m, 7H), 2.51 (s, 3H), 2.48 (s, 3H), 2.24 - 2.16 (m, 1H), 2.02 (p, *J* = 6.4 Hz, 2H), 1.65 - 1.51 (m, 4H), 1.39 - 1.27 (m, 2H). HRMS calcd for C₄₃H₅₃FN₉O₆ [M + H⁺] 810.4103, found 810.4119.

### Example 71

### Synthesis of HC58-165

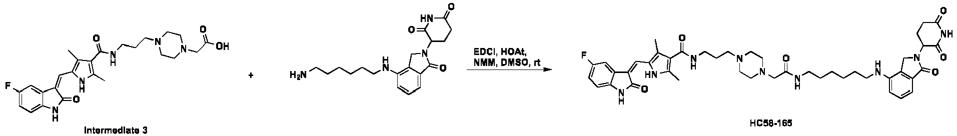

HC58-165 was synthesized following the standard procedure for preparing HC58-53 from Intermediate 3 (5.7 mg, 0.0085 mmol, 1.0 equiv), 3-(4-((6-aminohexyl)amino)-1-oxoisoindolin-2-yl)piperidine-2,6-dione (4.0 mg, 0.0085 mmol, 1.0 equiv), EDCI (2.5 mg, 0.013 mmol, 1.5 equiv), HOAt (2.0 mg, 0.013 mmol, 1.5 equiv), and NMM (4.3 mg, 0.043 mmol, 5.0 equiv) in DMSO (0.6 mL). HC58-165 was obtained as yellow solid in TFA salt form (3.4 mg, 38%). ¹H NMR (600 MHz, Methanol-*d*₄) δ 13.73 (s, 1H), 7.62 (s, 1H), 7.48 - 7.43 (m, 1H), 7.32 (t, *J* = 7.7 Hz, 1H), 7.20 (d, *J* = 7.4 Hz, 1H), 7.00 (d, *J* = 7.8 Hz, 1H), 6.95 - 6.85 (m, 2H), 5.15 (dd, *J* = 13.5, 5.1 Hz, 1H), 4.38 (d, *J* = 16.7 Hz, 1H), 4.29 (d, *J* = 16.8 Hz, 1H), 3.85 - 3.75 (m, 2H), 3.50 (t, *J* = 6.6 Hz, 2H), 3.41 - 3.33 (m, 4H), 3.26 (t, *J* = 6.6 Hz, 2H), 3.22 - 3.16 (m, 4H), 3.03 - 2.79 (m, 7H), 2.53 (s, 3H), 2.49 (s, 3H), 2.24 - 2.17 (m, 1H), 2.10 - 2.02 (m, 2H), 1.63 - 1.50 (m, 4H), 1.45 - 1.28 (m, 4H). HRMS calcd for C₄₄H₅₅FN₉O₆ [M + H⁺] 824.4259, found 824.4267.

### Example 72

### Synthesis of HC58-167

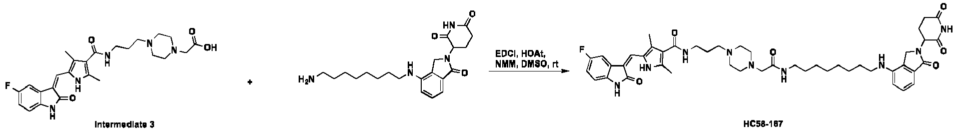

HC58-167 was synthesized following the standard procedure for preparing HC58-53 from Intermediate 3 (11.4 mg, 0.016 mmol, 1.0 equiv), 3-(4-((8-aminooctyl)amino)-1-oxoisoindolin-2-yl)piperidine-2,6-dione (9.0 mg, 0.018 mmol, 1.1 equiv), EDCI (4.6 mg, 0.024 mmol, 1.5 equiv), HOAt (3.3 mg, 0.024 mmol, 1.5 equiv), and NMM (8.1 mg, 0.08 mmol, 5.0 equiv) in DMSO (1 mL). HC58-167 was obtained as yellow solid in TFA salt form (9.3 mg, 54%). ¹H NMR (600 MHz, Methanol-*d*₄) δ 13.69 (s, 1H), 7.57 (s, 1H), 7.45 - 7.40 (m, 1H), 7.40 - 7.34 (m, 1H), 7.33 - 7.29 (m, 1H), 7.10 (d, *J* = 8.0 Hz, 1H), 6.93 - 6.83 (m, 2H), 5.15 (dd, *J* = 13.3, 5.1 Hz, 1H), 4.41 (d, *J* = 16.5 Hz, 1H), 4.33 (d, *J* = 17.3 Hz, 1H), 3.83 - 3.69 (m, 2H), 3.56 - 3.28 (m, 8H), 3.27 - 3.20 (m, 4H), 3.10 - 2.87 (m, 7H), 2.51 (s, 3H), 2.47 (s, 3H), 2.24 - 2.14 (m, 1H), 2.12- 2.01 (m, 2H), 1.53 (s, 4H), 1.45 - 1.26 (m, 8H). HRMS calcd for C₄₆H₅₉FN₉O₆ [M + H⁺] 852.4572, found 852.4561.

### Example 73

### Synthesis of HC58-178

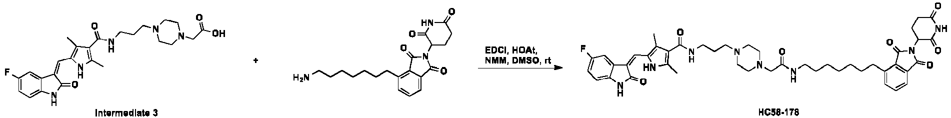

HC58-178 was synthesized following the standard procedure for preparing HC58-53 from Intermediate 3 (12.0 mg, 0.017 mmol, 1.0 equiv), 4-(7-aminoheptyl)-2-(2,6-dioxopiperidin-3-yl)isoindoline-1,3-dione (9.7 mg, 0.02 mmol, 1.2 equiv), EDCI (5.0 mg, 0.026 mmol, 1.5 equiv), HOAt (4.0 mg, 0.026 mmol, 1.5 equiv), and NMM (8.6 mg, 0.085 mmol, 5.0 equiv) in DMSO (1 mL). HC58-178 was obtained as yellow solid in TFA salt form (6.8 mg, 38%). ¹H NMR (600 MHz, Methanol-d4) δ 13.65 (s, 1H), 7.71 - 7.67 (m, 2H), 7.63 - 7.59 (m, 1H), 7.53 (s, 1H), 7.39 (dd, J = 9.0, 2.4 Hz, 1H), 6.89 - 6.82 (m, 2H), 5.13 (dd, J = 12.8, 5.5 Hz, 1H), 3.51 (t, J = 6.5 Hz, 2H), 3.47 - 3.37 (m, 4H), 3.27 - 3.21 (m, 4H), 3.12 - 2.94 (m, 6H), 2.88 (ddd, J = 17.4, 13.9, 5.3 Hz, 1H), 2.80 - 2.69 (m, 2H), 2.50 (s, 3H), 2.45 (s, 3H), 2.18 - 2.04 (m, 3H), 1.71 - 1.62 (m, 2H), 1.52 (p, J = 7.3 Hz, 2H), 1.44 - 1.30 (m, 8H). HRMS calcd for C₄₅H₅₄FN₈O₇ [M + H⁺] 837.4099, found 837.4112.

### Example 74

### Synthesis of intermediate 7

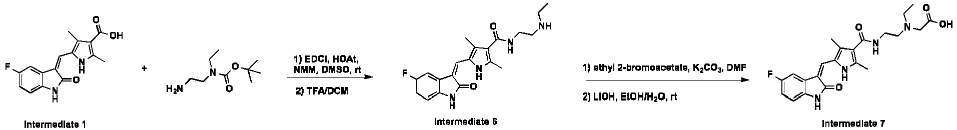

To a solution of Intermediate 1 (150 mg, 0.5 mmol) in DMSO (5 mL) were added *tert*-butyl (2-aminoethyl)(ethyl)carbamate (141.2 mg, 0.75 mmol, 1.5 equiv), EDCI (1-ethyl-3-(3-dimethylaminopropyl)carbodiimide) (144 mg, 0.75 mmol, 1.5 equiv), HOAt (1-hydroxy-7-azabenzo-triazole) (102 mg, 0.75 mmol, 1.5 equiv), and NMM (N-Methylmorpholine) (152 mg, 1.5 mmol, 3.0 equiv). After being stirred overnight at room temperature, the resulting mixture was purified by preparative HPLC (10%-100% methanol / 0.1% TFA in H₂O) to afford yellow solid in TFA salt form. The obtained solid was dissolved in DCM (4 mL). To the resulting solution was added TFA (2 ml). After being stirred for 1 h at room temperature, the reaction mixture was concentrated and the residue was purified preparative HPLC (10%-100% methanol / 0.1% TFA in H₂O) to afford intermediate 6 as yellow solid in TFA salt form (138 mg, 57% yield for two step). ESI m/z =371.2 [M + H⁺]. To a solution of intermediate 6 (70 mg, 0.145 mmol) in DMF (2 mL) was added potassium carbonate (40 mg, 0.29 mmol, 2.0 equiv) and ethyl bromoacetate (48.3 mg, 0.29 mmol, 2.0 equiv). Then the mixture was stirred at 70 °C for 3h. Water (20 mL) was added to quench the reaction. The mixture was extracted with ethyl acetate (3 x 20 mL). The organic layer was combined and washed with brine. Dried over Na₂SO₄, filtered and evaporated to afford yellow solid. The obtained yellow solid was dissolved in EtOH/H₂O (2.5 mL/0.5 mL). To the resulting solution was added lithium hydroxide (11 mg, 0.46 mmol, 3 equiv). After being stirred overnight at room temperature, the reaction mixture was concentrated and the residue was purified by preparative HPLC (10% - 100% methanol / 0.1% TFA in H₂O) to afford intermediate 7 as yellow solid in TFA salt form (58.7 mg, 75% yield for two steps). ¹H NMR (600 MHz, Methanol-*d*₄) δ 7.60 - 7.54 (m, 1H), 7.44 - 7.38 (m, 1H), 6.92 - 6.83 (m, 2H), 4.08 (s, 2H), 3.81 - 3.72 (m, 2H), 3.51 - 3.43 (m, 4H), 2.53 (s, 3H), 2.49 (s, 3H), 1.44 (t, *J* = 7.2 Hz, 3H). ESI m/z =429.2 [M + H⁺].

### Example 75

### Synthesis of HC58-179

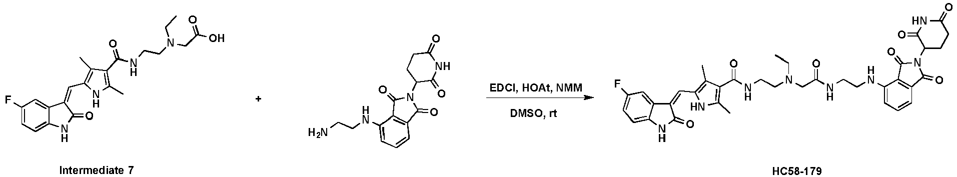

To a solution of Intermediate 7 (7.0 mg, 0.0129 mmol, 1.0 equiv) in DMSO (1 mL) were added 4-((2-aminoethyl)amino)-2-(2,6-dioxopiperidin-3-yl)isoindoline-1,3-dione (6.2 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv). After being stirred overnight at room temperature, the resulting mixture was purified by preparative HPLC (10%-100% MeOH/ 0.1% TFA in H₂O) to afford HC58-179 as yellow solid in TFA salt form (4.7 mg, 43%). ¹H NMR (600 MHz, Methanol-d4) δ 13.63 (s, 1H), 7.52 - 7.47 (m, 2H), 7.37 (dd, J = 8.9, 2.3 Hz, 1H), 7.10 (d, J = 8.5 Hz, 1H), 6.97 (d, J = 7.1 Hz, 1H), 6.90 - 6.82 (m, 2H), 5.04 (dd, J = 12.8, 5.5 Hz, 1H), 4.16 - 3.94 (m, 2H), 3.93 - 3.75 (m, 1H), 3.73 - 3.34 (m, 9H), 2.84 (ddd, J = 18.4, 13.7, 5.3 Hz, 1H), 2.78 - 2.62 (m, 2H), 2.47 (s, 3H), 2.42 (s, 3H), 2.14 - 2.04 (m, 1H), 1.41 (t, J = 7.2 Hz, 3H). HRMS calcd for C₃₇H₄₀FN₈O₇ [M + H⁺] 727.3004, found 727.3013.

### Example 76

### Synthesis of HC58-180

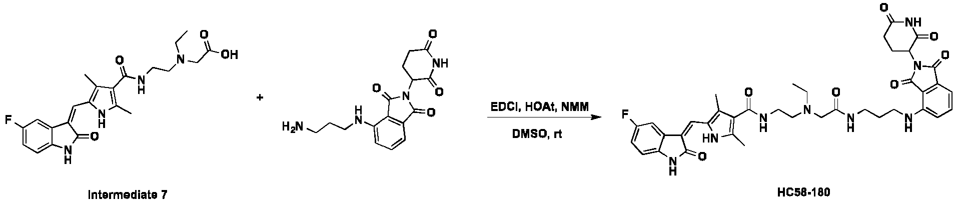

HC58-180 was synthesized following the standard procedure for preparing HC58-179 from Intermediate 7 (7.0 mg, 0.0129 mmol, 1.0 equiv), 4-((3-aminopropyl)amino)-2-(2,6-dioxopiperidin-3-yl)isoindoline-1,3-dione (6.4 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-180 was obtained as yellow solid in TFA salt form (6.0 mg, 54%). ¹H NMR (600 MHz, Methanol-d4) δ 13.64 (s, 1H), 7.51 - 7.45 (m, 2H), 7.37 (d, J = 8.9 Hz, 1H), 7.00 (d, J = 8.5 Hz, 1H), 6.94 - 6.83 (m, 3H), 5.05 (dd, J = 12.8, 5.4 Hz, 1H), 4.18 - 3.98 (m, 2H), 3.91 - 3.77 (m, 1H), 3.73 - 3.61 (m, 1H), 3.58 - 3.40 (m, 8H), 2.86 (ddd, J = 19.1, 14.0, 5.3 Hz, 1H), 2.79 - 2.68 (m, 2H), 2.53 (s, 3H), 2.46 (s, 3H), 2.19 - 2.10 (m, 1H), 1.93 - 1.79 (m, 2H), 1.44 (t, J = 7.2 Hz, 3H). HRMS calcd for C₃₈H₄₂FN₈O₇ [M + H⁺] 741.3160, found 741.3152.

### Example 77

### Synthesis of HC58-181

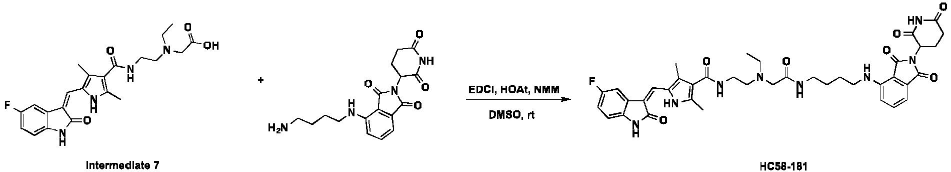

HC58-181 was synthesized following the standard procedure for preparing HC58-179 from Intermediate 7 (7.0 mg, 0.0129 mmol, 1.0 equiv), 4-((4-aminobutyl)amino)-2-(2,6-dioxopiperidin-3-yl)isoindoline-1,3-dione (6.6 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-181 was obtained as yellow solid in TFA salt form (3.6 mg, 32%). ¹H NMR (600 MHz, Methanol-*d*₄) δ 13.64 (s, 1H), 7.52 - 7.44 (m, 2H), 7.40 - 7.32 (m, 1H), 7.02 - 6.91 (m, 2H), 6.89 - 6.76 (m, 2H), 5.12 - 5.00 (m, 1H), 4.17 - 3.96 (m, 2H), 3.92 - 3.58 (m, 2H), 3.43 (s, 4H), 3.33 - 3.25 (m, 4H), 2.84 (ddd, *J* = 18.0, 13.9, 5.3 Hz, 1H), 2.77 - 2.66 (m, 2H), 2.51 (s, 3H), 2.45 (s, 3H), 2.17 - 2.05 (m, 1H), 1.66 (s, 4H), 1.46 - 1.39 (m, 3H). HRMS calcd for C₃₉H₄₄FN₈O₇ [M + H⁺] 755.3317, found 755.3332.

### Example 78

### Synthesis of HC58-182

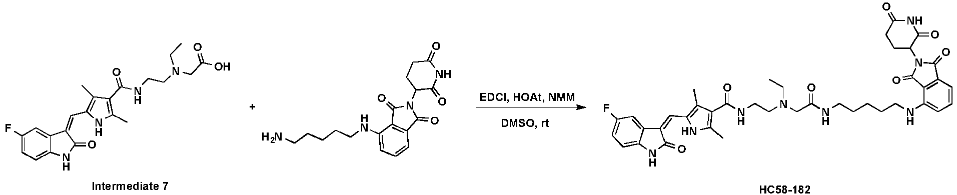

HC58-182 was synthesized following the standard procedure for preparing HC58-179 from Intermediate 7 (7.0 mg, 0.0129 mmol, 1.0 equiv), 4-((5-aminopentyl)amino)-2-(2,6-dioxopiperidin-3-yl)isoindoline-1,3-dione (6.8 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-182 was obtained as yellow solid in TFA salt form (4.5 mg, 40%). ¹H NMR (600 MHz, Methanol-*d*₄) δ 13.62 (s, 1H), 7.48 - 7.42 (m, 2H), 7.35 (dd, *J =* 8.9, 2.4 Hz, 1H), 6.96 - 6.92 (m, 2H), 6.89 - 6.79 (m, 2H), 5.04 (dd, *J =* 12.7, 5.5 Hz, 1H), 4.17 - 3.97 (m, 2H), 3.86 (s, 1H), 3.63 (s, 1H), 3.57 - 3.37 (m, 4H), 3.25 (t, *J* = 6.9 Hz, 4H), 2.85 (ddd, *J* = 17.0, 13.7, 5.2 Hz, 1H), 2.78 - 2.66 (m, 2H), 2.51 (s, 3H), 2.45 (s, 3H), 2.15 - 2.05 (m, 1H), 1.71 - 1.56 (m, 4H), 1.49 - 1.38 (m, 5H). HRMS calcd for C₄₀H₄₆FN₈O₇ [M + H⁺] 769.3473, found 769.3489.

### Example 79

### Synthesis of HC58-183

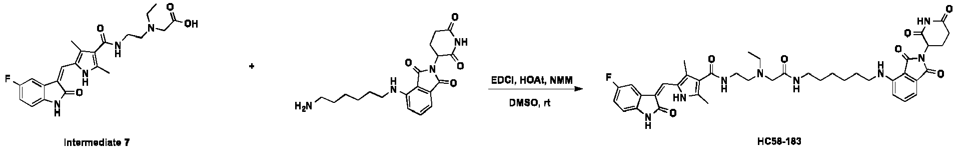

HC58-183 was synthesized following the standard procedure for preparing HC58-179 from Intermediate 7 (7.0 mg, 0.0129 mmol, 1.0 equiv), 4-((6-aminohexyl)amino)-2-(2,6-dioxopiperidin-3-yl)isoindoline-1,3-dione (5.9 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-183 was obtained as yellow solid in TFA salt form (5.9 mg, 51%). ¹H NMR (600 MHz, Methanol-*d*₄) δ 13.66 (s, 1H), 7.50 (s, 1H), 7.46 - 7.43 (m, 1H), 7.37 (dd, *J* = 9.0, 2.5 Hz, 1H), 6.93 (s, 1H), 6.91 (s, 1H), 6.88 - 6.80 (m, 2H), 5.04 (dd, *J* = 12.8, 5.4 Hz, 1H), 4.17 - 3.95 (m, 2H), 3.87 (s, 1H), 3.63 (s, 1H), 3.57 - 3.37 (m, 4H), 3.27 - 3.17 (m, 4H), 2.92 - 2.81 (m, 1H), 2.77 - 2.64 (m, 2H), 2.53 (s, 3H), 2.46 (s, 3H), 2.16 - 2.07 (m, 1H), 1.67 - 1.51 (m, 4H), 1.48 - 1.36 (m, 7H). HRMS calcd for C₄₁H₄₈FN₈O₇ [M + H⁺] 783.3630, found 783.3639.

### Example 80

### Synthesis of HC58-184

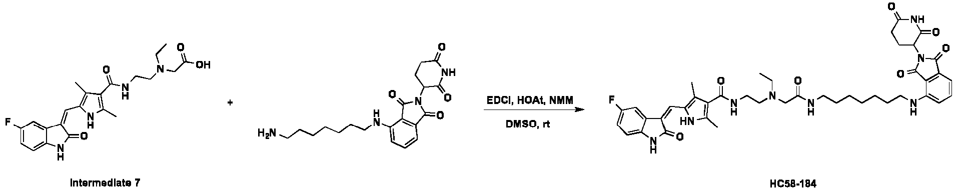

HC58-184 was synthesized following the standard procedure for preparing HC58-179 from Intermediate 7 (7.0 mg, 0.0129 mmol, 1.0 equiv), 4-((7-aminoheptyl)amino)-2-(2,6-dioxopiperidin-3-yl)isoindoline-1,3-dione (7.2 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-184 was obtained as yellow solid in TFA salt form (4.8 mg, 41%). ¹H NMR (600 MHz, Methanol-*d*₄) δ 13.64 (s, 1H), 7.49 - 7.42 (m, 2H), 7.34 (dd, *J* = 8.7, 2.7 Hz, 1H), 6.94 (dd, *J* = 7.3, 2.9 Hz, 1H), 6.91 (dd, *J* = 8.7, 2.8 Hz, 1H), 6.87 - 6.77 (m, 2H), 5.08 - 5.02 (m, 1H), 4.13 - 3.96 (m, 2H), 3.86 (s, 1H), 3.63 (s, 1H), 3.56 - 3.36 (m, 4H), 3.30 - 3.15 (m, 4H), 2.91 - 2.81 (m, 1H), 2.78 - 2.65 (m, 2H), 2.52 (s, 3H), 2.45 (s, 3H), 2.16-2.07 (m, 1H), 1.56 (d, *J* = 30.8 Hz, 4H), 1.49 - 1.30 (m, 9H). HRMS calcd for C₄₂H₅₀FN₈O₇ [M + H⁺] 797.3786, found 797.3774.

### Example 81

### Synthesis of HC58-185

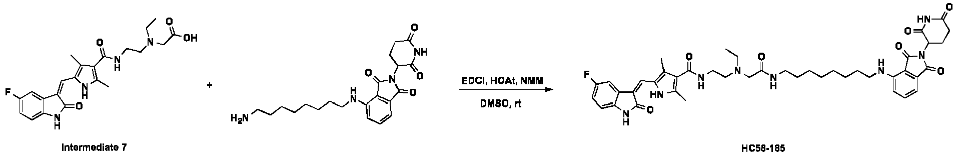

HC58-185 was synthesized following the standard procedure for preparing HC58-179 from Intermediate 7 (7.0 mg, 0.0129 mmol, 1.0 equiv), 4-((8-aminooctyl)amino)-2-(2,6-dioxopiperidin-3-yl)isoindoline-1,3-dione (7.4 mg, 0.0143 mmol, 1.1 equiv), EDCI (1-ethyl-3-(3-dimethylaminopropyl)carbodiimide) (4.0 mg, 0.02 mmol, 1.5 equiv), HOAt (1-hydroxy-7-azabenzo-triazole) (3.0 mg, 0.02 mmol, 1.5 equiv), and NMM (N-Methylmorpholine) (5.3 mg, 0.052 mmol, 4.0 equiv) in DMSO (1 mL). HC58-185 was obtained as yellow solid in TFA salt form (5.6 mg, 47%). ¹H NMR (600 MHz, Methanol-*d*₄) δ 13.67 (s, 1H), 7.50 (s, 1H), 7.46 (dd, *J* = 8.6, 7.1 Hz, 1H), 7.36 (dd, *J* = 8.9, 2.4 Hz, 1H), 6.95 (d, *J* = 7.1 Hz, 1H), 6.91 (d, *J* = 8.6 Hz, 1H), 6.87 - 6.78 (m, 2H), 5.04 (dd, *J* = 12.8, 5.5 Hz, 1H), 4.14 - 3.98 (m, 2H), 3.87 (s, 1H), 3.63 (s, 1H), 3.56 - 3.37 (m, 4H), 3.29 - 3.17 (m, 4H), 2.85 (ddd, *J* = 17.6, 14.0, 5.3 Hz, 1H), 2.78 - 2.65 (m, 2H), 2.53 (s, 3H), 2.47 (s, 3H), 2.15 - 2.05 (m, 1H), 1.61 - 1.50 (m, 4H), 1.43 (t, *J* = 7.2 Hz, 3H), 1.41 - 1.26 (m, 8H). HRMS calcd for C₄₃H₅₂FN₈O₇ [M+ H⁺] 811.3943, found 811.3959.

### Example 82

### Synthesis of intermediate 8

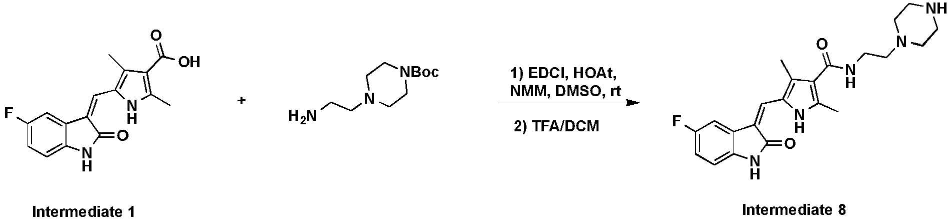

To a solution of intermediate 1 (150 mg, 0.5 mmol) in DMSO (5 mL) were added tert-butyl 4-(2-aminoethyl)piperazine-1-carboxylate (149 mg, 0.65 mmol, 1.3 equiv), EDCI (1-ethyl-3-(3-dimethylaminopropyl)carbodiimide) (144 mg, 0.75 mmol, 1.5 equiv), HOAt (1-hydroxy-7-azabenzo-triazole) (102 mg, 0.75 mmol, 1.5 equiv), and NMM (N-Methylmorpholine) (152 mg, 1.5 mmol, 3.0 equiv). After being stirred overnight at room temperature, the resulting mixture was purified by preparative HPLC (10%-100% methanol / 0.1% TFA in H₂O) to afford yellow solid in TFA salt form. The obtained solid was dissolved in DCM (4 mL). To the resulting solution was added TFA (2 ml). After being stirred for 1 h at room temperature, the reaction mixture was concentrated and the residue was purified preparative HPLC (10%-100% methanol / 0.1% TFA in H₂O) to afford intermediate 8 as yellow solid in TFA salt form (170 mg, 53% yield for two step). ¹H NMR (600 MHz, Methanol-*d*₄) δ 7.60 (s, 1H), 7.46 - 7.42 (m, 1H), 6.93 - 6.86 (m, 2H), 3.62 (t, *J* = 6.2 Hz, 2H), 3.39 - 3.34 (m, 4H), 3.05 (s, 4H), 2.94 - 2.87 (m, 2H), 2.53 (s, 3H), 2.49 (s, 3H). ESI m/z =412.2 [M + H⁺].

### Example 83

### Synthesis of HC65-2

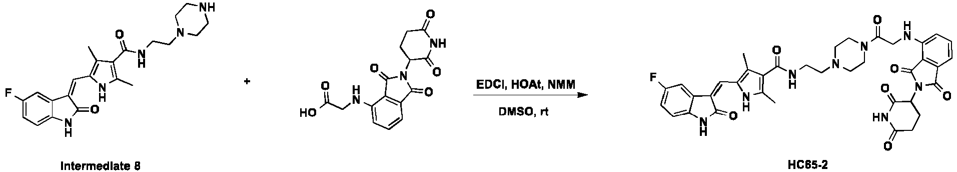

To a solution of Intermediate 8 (10 mg, 0.0156 mmol, 1.1 equiv) in DMSO (1 mL) were added (2-(2,6-dioxopiperidin-3-yl)-1,3-dioxoisoindolin-4-yl)glycine (4.9 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv). After being stirred overnight at room temperature, the resulting mixture was purified by preparative HPLC (10%-100% MeOH/ 0.1% TFA in H₂O) to afford HC65-2 as yellow solid in TFA salt form (7.0 mg, 56%). ¹H NMR (600 MHz, Acetone-*d*₆) δ 13.84 (s, 1H), 10.01 (s, 1H), 9.97 (s, 1H), 7.73 (s, 1H), 7.68 (s, 1H), 7.62 - 7.52 (m, 2H), 7.18 (s, 1H), 7.08 - 7.01 (m, 2H), 6.97 - 6.89 (m, 2H), 5.11 (dd, *J* = 12.7, 5.4 Hz, 1H), 4.52 - 4.21 (m, 6H), 4.01 - 3.68 (m, 6H), 3.64 - 3.53 (m, 2H), 2.99 (ddd, *J* = 17.4, 14.4, 5.8 Hz, 1H), 2.86 - 2.75 (m, 2H), 2.55 (s, 3H), 2.52 (s, 3H), 2.27 - 2.22 (m, 1H). HRMS calcd for C₃₇H₃₈FN₈O₇ [M + H⁺] 725.2847, found 725.2842.

### Example 84

### Synthesis of HC65-3

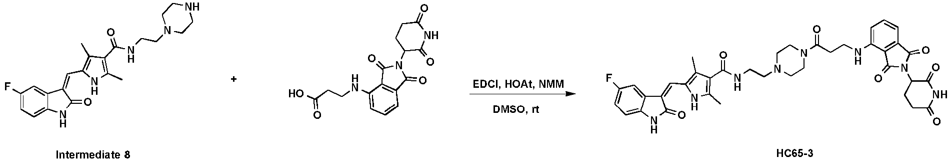

HC65-3 was synthesized following the standard procedure for preparing HC65-2 from Intermediate 8 (10 mg, 0.0156 mmol, 1.1 equiv), 3-((2-(2,6-dioxopiperidin-3-yl)-1,3-dioxoisoindolin-4-yl)amino)propanoic acid (5.1 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-3 was obtained as yellow solid in TFA salt form (7.4 mg, 58%). ¹H NMR (600 MHz, Acetone-*d*₆) δ 13.83 (s, 1H), 10.04 (s, 1H), 9.98 (s, 1H), 7.71 (s, 1H), 7.63 - 7.53 (m, 2H), 7.15 (d, *J* = 8.5 Hz, 1H), 7.04 (d, *J* = 7.0 Hz, 1H), 6.98 - 6.87 (m, 2H), 6.73 (s, 1H), 5.09 (dd, *J* = 12.5, 5.4 Hz, 1H), 4.50 - 4.02 (m, 8H), 4.00 - 3.81 (m, 2H), 3.70 (s, 2H), 3.55 (s, 2H), 2.97 (ddd, *J* = 18.1, 14.8, 5.0 Hz, 1H), 2.86 (s, 2H), 2.82 - 2.71 (m, 2H), 2.54 (s, 3H), 2.50 (s, 3H), 2.28 - 2.17 (m, 1H). HRMS calcd for C₃₈H₄₀FN₈O₇ [M + H⁺] 739.3004, found 739.3021.

### Example 85

### Synthesis of HC65-4

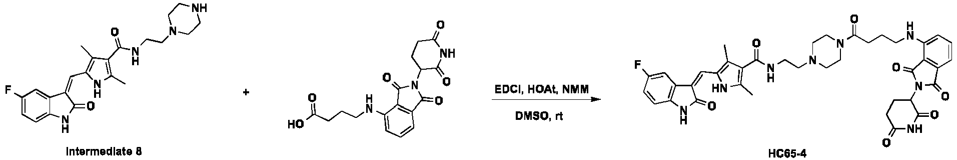

HC65-4 was synthesized following the standard procedure for preparing HC65-2 from Intermediate 8 (10 mg, 0.0156 mmol, 1.1 equiv), 4-((2-(2,6-dioxopiperidin-3-yl)-1,3-dioxoisoindolin-4-yl)amino)butanoic acid (5.4 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-4 was obtained as yellow solid in TFA salt form (6.5 mg, 50%). ¹H NMR (600 MHz, Acetone-*d*₆) δ 13.84 (s, 1H), 10.04 (s, 1H), 9.97 (s, 1H), 7.74 (s, 1H), 7.70 (s, 1H), 7.64 - 7.55 (m, 2H), 7.19 (d, *J* = 8.5 Hz, 1H), 7.05 (d, *J* = 7.1 Hz, 1H), 6.98 - 6.88 (m, 2H), 6.53 (s, 1H), 5.09 (dd, *J* = 12.6, 5.4 Hz, 1H), 4.02 - 3.72 (m, 10H), 3.58 - 3.51 (m, 2H), 3.45 (t, *J* = 7.0 Hz, 2H), 3.01 - 2.93 (m, 1H), 2.83 - 2.71 (m, 2H), 2.61 (s, 2H), 2.55 (s, 3H), 2.52 (s, 3H), 2.25 - 2.19 (m, 1H), 2.02 - 1.98 (m, 2H). HRMS calcd for C₃₉H₄₂FN₈O₇ [M + H⁺] 753.3160, found 753.3167.

### Example 86

### Synthesis of HC65-5

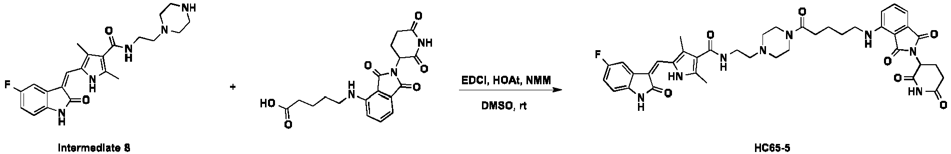

HC65-5 was synthesized following the standard procedure for preparing HC65-2 from Intermediate 8 (10 mg, 0.0156 mmol, 1.1 equiv), 5-((2-(2,6-dioxopiperidin-3-yl)-1,3-dioxoisoindolin-4-yl)amino)pentanoic acid (5.6 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-5 was obtained as yellow solid in TFA salt form (5.0 mg, 38%). ¹H NMR (600 MHz, Acetone-*d*₆) δ 13.85 (s, 1H), 10.04 (s, 1H), 9.98 (s, 1H), 7.73 (d, *J* = 8.8 Hz, 1H), 7.62 - 7.52 (m, 2H), 7.10 (d, *J* = 8.5 Hz, 1H), 7.04 (d, *J* = 6.9 Hz, 1H), 6.98 - 6.88 (m, 2H), 5.10 (dd, *J* = 12.7, 5.4 Hz, 1H), 4.08 - 3.64 (m, 6H), 3.61 - 3.51 (m, 2H), 3.47 - 3.08 (m, 6H), 3.02 - 2.91 (m, 1H), 2.81 - 2.70 (m, 4H), 2.55 (s, 3H), 2.52 (s, 3H), 2.28 - 2.18 (m, 1H), 1.81 - 1.70 (m, 4H). HRMS calcd for C₄₀H₄₄FN₈O₇ [M + H⁺] 767.3317, found 767.3331.

### Example 87

### Synthesis of HC65-6

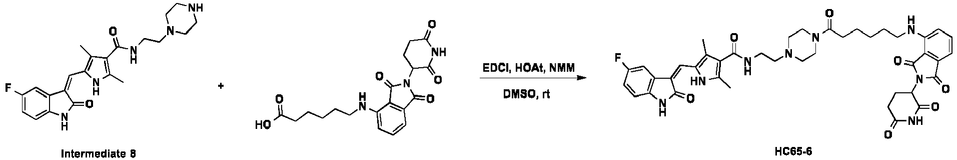

HC65-6 was synthesized following the standard procedure for preparing HC65-2 from Intermediate 8 (10 mg, 0.0156 mmol, 1.1 equiv), 6-((2-(2,6-dioxopiperidin-3-yl)-1,3-dioxoisoindolin-4-yl)amino)hexanoic acid (5.8 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-6 was obtained as yellow solid in TFA salt form (5.7 mg, 43%). ¹H NMR (600 MHz, Acetone-d6) δ 13.85 (s, 1H), 10.02 (s, 1H), 9.98 (s, 1H), 7.74 (s, 1H), 7.73 - 7.68 (m, 1H), 7.62 - 7.55 (m, 2H), 7.10 (d, J = 8.5 Hz, 1H), 7.04 (d, J = 7.0 Hz, 1H), 6.97 - 6.88 (m, 2H), 5.09 (dd, J = 12.7, 5.5 Hz, 1H), 3.99 - 3.66 (m, 6H), 3.56 (t, J = 5.6 Hz, 2H), 3.39 (t, J = 7.0 Hz, 2H), 3.34 - 3.10 (m, 4H), 3.04 - 2.92 (m, 1H), 2.83 - 2.73 (m, 2H), 2.55 (s, 3H), 2.52 (s, 3H), 2.48 (t, J = 7.3 Hz, 2H), 2.27 - 2.19 (m, 1H), 1.79 - 1.63 (m, 4H), 1.49 (p, J = 7.7 Hz, 2H). HRMS calcd for C₄₁H₄₆FN₈O₇ [M + H⁺] 781.3473, found 781.3465.

### Example 88

### Synthesis of HC65-7

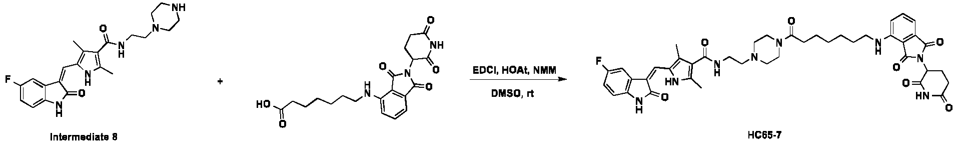

HC65-7 was synthesized following the standard procedure for preparing HC65-2 from Intermediate 8 (10 mg, 0.0156 mmol, 1.1 equiv), 7-((2-(2,6-dioxopiperidin-3-yl)-1,3-dioxoisoindolin-4-yl)amino)heptanoic acid (6.0 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-7 was obtained as yellow solid in TFA salt form (6.6 mg, 49%). ¹H NMR (600 MHz, Acetone-*d*₆) δ 13.86 (s, 1H), 10.01 (s, 1H), 9.98 (s, 1H), 7.75 (s, 1H), 7.71 (s, 1H), 7.62 - 7.56 (m, 2H), 7.10 (d, *J* = 8.5 Hz, 1H), 7.04 (d, *J* = 7.0 Hz, 1H), 6.98 - 6.89 (m, 2H), 5.09 (dd, *J* = 12.6, 5.5 Hz, 1H), 3.94 - 3.86 (m, 2H), 3.83 - 3.61 (m, 4H), 3.57 (s, 2H), 3.38 (t, *J* = 7.0 Hz, 2H), 3.32 - 3.07 (m, 4H), 2.97 (ddd, *J* = 17.9, 14.8, 5.3 Hz, 1H), 2.84 - 2.73 (m, 2H), 2.55 (s, 3H), 2.52 (s, 3H), 2.45 (t, *J* = 7.4 Hz, 2H), 2.26 - 2.18 (m, 1H), 1.70 (p, *J* = 7.2 Hz, 2H), 1.63 (p, *J* = 7.4 Hz, 2H), 1.52 - 1.37 (m, 4H). HRMS calcd for C₄₂H₄₈FN₈O₇ [M + H⁺] 795.3630, found 795.3642.

### Example 89

### Synthesis of HC65-8

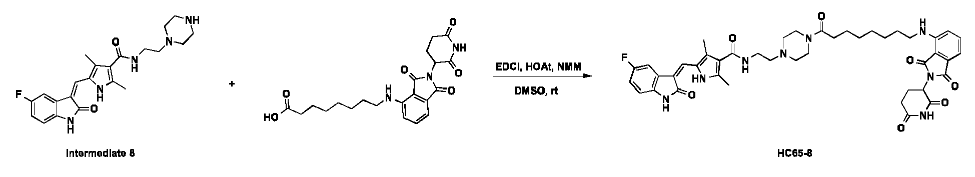

HC65-8 was synthesized following the standard procedure for preparing HC65-2 from Intermediate 8 (10 mg, 0.0156 mmol, 1.1 equiv), 8-((2-(2,6-dioxopiperidin-3-yl)-1,3-dioxoisoindolin-4-yl)amino)octanoic acid (6.2 mg, 0.0149 mmol, 1.0 equiv), EDCI (1-ethyl-3-(3-dimethylaminopropyl)carbodiimide) (4.3 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv) in DMSO (1 mL). HC65-8 was obtained as yellow solid in TFA salt form (7.3 mg, 53%). ¹H NMR (600 MHz, Acetone-*d*₆) δ 13.86 (s, 1H), 9.99 (s, 1H), 9.98 (s, 1H), 7.75 (s, 1H), 7.71 (t, *J* = 5.6 Hz, 1H), 7.63 - 7.55 (m, 2H), 7.10 (d, *J* = 8.6 Hz, 1H), 7.04 (d, *J* = 7.0 Hz, 1H), 6.98 - 6.88 (m, 2H), 5.09 (dd, *J* = 12.6, 5.5 Hz, 1H), 3.92 (q, *J* = 5.5 Hz, 6H), 3.58 (t, *J* = 5.6 Hz, 2H), 3.38 (t, *J* = 7.1 Hz, 2H), 3.34 - 3.15 (m, 4H), 2.97 (ddd, *J* = 18.0, 14.7, 5.2 Hz, 1H), 2.83 2.73 (m, 2H), 2.55 (s, 3H), 2.52 (s, 3H), 2.44 (t, *J* = 7.5 Hz, 2H), 2.28 - 2.19 (m, 1H), 1.70 (p, *J* = 7.2 Hz, 2H), 1.61 (p, *J* = 7.5 Hz, 2H), 1.48 - 1.34 (m, 6H). HRMS calcd for C₄₃H₅₀FN₈O₇ [M + H⁺] 809.3786, found 809.3799.

### Example 90

### Synthesis of intermediate 9

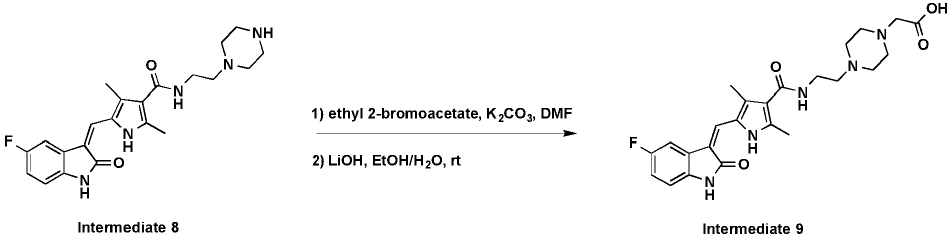

To a solution of intermediate 8 (80 mg, 0.125 mmol, 1.0 equiv) in DMF (2 mL) was added potassium carbonate (52 mg, 0.375 mmol, 3.0 equiv) and ethyl bromoacetate (42 mg, 0.25 mmol, 2.0 equiv). Then the mixture was stirred at 60 °C for 3h. Water (10 mL) was added to quench the reaction. The mixture was extracted with ethyl acetate (3 x 10 mL). The organic layer was combined and washed with brine. Dried over Na₂SO₄, filtered and evaporated to afford yellow solid. The obtained yellow solid was dissolved in EtOH/H₂O (2.5 mL/0.5 mL). To the resulting solution was added lithium hydroxide (9 mg, 0.375 mmol, 3 equiv). After being stirred overnight at room temperature, the reaction mixture was concentrated and the residue was purified by preparative HPLC (10% - 100% methanol / 0.1% TFA in H₂O) to afford intermediate 9 as yellow solid in TFA salt form (54.2 mg, 62% yield for two steps). ¹H NMR (600 MHz, Methanol-*d*₄) δ 7.61 (s, 1H), 7.44 (dd, *J* = 8.8, 2.2 Hz, 1H), 6.93 - 6.85 (m, 2H), 3.73 (t, *J* = 6.1 Hz, 2H), 3.60 (s, 2H), 3.36 (s, 4H), 3.26 (t, *J* = 6.0 Hz, 2H), 3.12 (s, 4H), 2.54 (s, 3H), 2.50 (s, 3H). ESI m/z =470.2 [M + H⁺].

### Example 91

### Synthesis of HC65-13

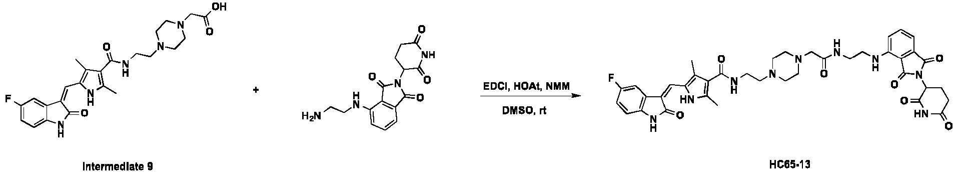

To a solution of Intermediate 9 (7 mg, 0.01 mmol, 1.1 equiv) in DMSO (1 mL) were added 4-((2-aminoethyl)amino)-2-(2,6-dioxopiperidin-3-yl)isoindoline-1,3-dione (4.8 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv). After being stirred overnight at room temperature, the resulting mixture was purified by preparative HPLC (10%-100% MeOH/ 0.1% TFA in H₂O) to afford HC65-13 as yellow solid in TFA salt form (5.8 mg, 58%). ¹H NMR (600 MHz, Methanol-*d*₄) δ 13.70 (s, 1H), 7.60 - 7.55 (m, 2H), 7.43 (d, *J* = 8.4 Hz, 1H), 7.14 (dd, *J=* 8.7, 3.1 Hz, 1H), 7.08 (dd, *J* = 7.1, 3.1 Hz, 1H), 6.92 - 6.84 (m, 2H), 5.17 - 5.06 (m, 1H), 3.80 - 3.70 (m, 2H), 3.60 - 3.51 (m, 2H), 3.46 - 3.28 (m, 10H), 3.02 - 2.80 (m, 5H), 2.79 - 2.61 (m, 2H), 2.52 (s, 3H), 2.47 (s, 3H), 2.21 - 2.08 (m, 1H). HRMS calcd for C₃₉H₄₃FN₉O₇ [M + H⁺] 768.3269, found 768.3282.

### Example 92

### Synthesis of HC65-14

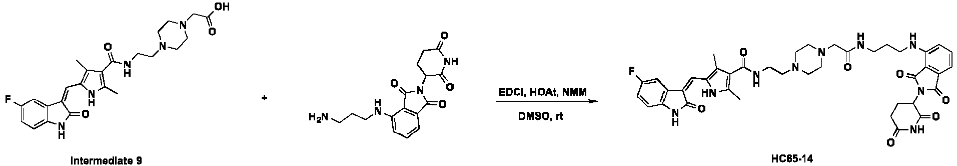

HC65-14 was synthesized following the standard procedure for preparing HC65-13 from Intermediate 9 (7 mg, 0.01 mmol, 1.1 equiv), 4-((3-aminopropyl)amino)-2-(2,6-dioxopiperidin-3-yl)isoindoline-1,3-dione (4.9 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-14 was obtained as yellow solid in TFA salt form (5.1 mg, 51%). ¹H NMR (600 MHz, Methanol-*d*₄) δ 13.70 (s, 1H), 7.59 - 7.53 (m, 2H), 7.42 (d, *J* = 8.9 Hz, 1H), 7.08 - 7.02 (m, 2H), 6.93 - 6.84 (m, 2H), 5.09 - 5.04 (m, 1H), 3.77 - 3.69 (m, 2H), 3.50 - 3.27 (m, 12H), 3.02 (s, 4H), 2.90 - 2.81 (m, 1H), 2.79 - 2.66 (m, 2H), 2.52 (s, 3H), 2.47 (s, 1H), 2.17 - 2.07 (m, 1H), 1.95 - 1.84 (m, 2H). HRMS calcd for C₄₀H₄₅FN₉O₇ [M + H⁺] 782.3426, found 782.3421.

### Example 93

### Synthesis of HC65-15

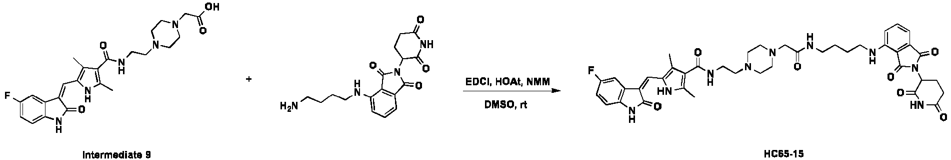

HC65-15 was synthesized following the standard procedure for preparing HC65-13 from Intermediate 9 (7 mg, 0.01 mmol, 1.1 equiv), 4-((4-aminobutyl)amino)-2-(2,6-dioxopiperidin-3-yl)isoindoline-1,3-dione (5.1 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-15 was obtained as yellow solid in TFA salt form (4.6 mg, 45%). ¹H NMR (600 MHz, Methanol-*d*₄) δ 13.73 (s, 1H), 7.62 - 7.54 (m, 2H), 7.46 - 7.41 (m, 1H), 7.10 - 7.02 (m, 2H), 6.92 - 6.85 (m, 2H), 5.07 (dd, *J* = 12.7, 5.7 Hz, 1H), 3.73 (t, *J* = 6.0 Hz, 2H), 3.48 - 3.33 (m, 12H), 3.08 - 2.81 (m, 5H), 2.78 - 2.66 (m, 2H), 2.53 (s, 3H), 2.49 (s, 3H), 2.17 - 2.09 (m, 1H), 1.79 - 1.62 (m, 4H). HRMS calcd for C₄₁H₄₇FN₉O₇ [M + H⁺] 796.3582, found 796.3595.

### Example 94

### Synthesis of HC65-16

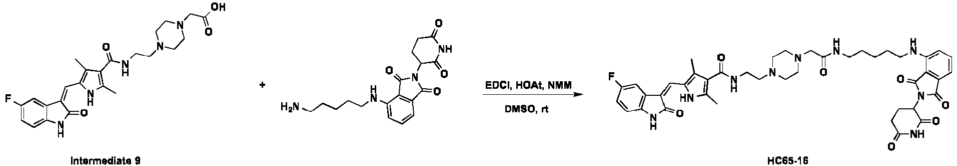

HC65-16 was synthesized following the standard procedure for preparing HC65-13 from Intermediate 9 (7 mg, 0.01 mmol, 1.1 equiv), 4-((5-aminopentyl)amino)-2-(2,6-dioxopiperidin-3-yl)isoindoline-1,3-dione (5.2 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-16 was obtained as yellow solid in TFA salt form (5.1 mg, 49%). ¹H NMR (600 MHz, Methanol-*d*₄) δ 13.72 (s, 1H), 7.58 (s, 1H), 7.55 (dd, *J =* 8.6, 7.1 Hz, 1H), 7.43 (dd, *J* = 8.9, 2.4 Hz, 1H), 7.05 (d, *J* = 4.7 Hz, 1H), 7.03 (d, *J* = 3.2 Hz, 1H), 6.91 - 6.84 (m, 2H), 5.05 (dd, *J* = 12.8, 5.5 Hz, 1H), 3.73 (t, *J* = 6.0 Hz, 2H), 3.48 - 3.34 (m, 8H), 3.32 - 3.26 (m, 4H), 3.01 (s, 4H), 2.85 (ddd, *J* = 17.6, 14.0, 5.3 Hz, 1H), 2.79-2.68 (m, 2H), 2.53 (s, 3H), 2.48 (s, 3H), 2.16-2.07 (m, 1H), 1.70 (p, *J* = 6.9 Hz, 2H), 1.61 (p, *J* = 7.0 Hz, 2H), 1.52 - 1.42 (m, 2H). HRMS calcd for C₄₂H₄₉FN₉O₇ [M + H⁺] 810.3739, found 810.3747.

### Example 95

### Synthesis of HC65-17

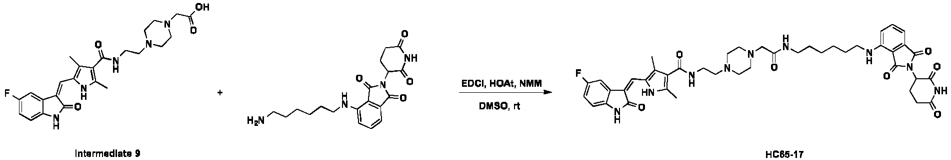

HC65-17 was synthesized following the standard procedure for preparing HC65-13 from Intermediate 9 (7 mg, 0.01 mmol, 1.1 equiv), 4-((6-aminohexyl)amino)-2-(2,6-dioxopiperidin-3-yl)isoindoline-1,3-dione (4.5 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-17 was obtained as yellow solid in TFA salt form (4.7 mg, 45%). ¹H NMR (600 MHz, Methanol-*d*₄) δ 13.74 (s, 1H), 7.60 (s, 1H), 7.58 - 7.53 (m, 1H), 7.49 - 7.42 (m, 1H), 7.11 - 7.00 (m, 2H), 6.95 - 6.85 (m, 2H), 5.12 - 5.03 (m, 1H), 3.79 - 3.70 (m, 2H), 3.52 - 3.24 (m, 12H), 3.00 (s, 4H), 2.90 - 2.82 (m, 1H), 2.80 - 2.68 (m, 2H), 2.53 (s, 3H), 2.49 (d, *J* = 1.4 Hz, 3H), 2.19 - 2.04 (m, 1H), 1.76 - 1.65 (m, 2H), 1.62 - 1.53 (m, 2H), 1.53 - 1.38 (m, 4H). HRMS calcd for C₄₃H₅₁FN₉O₇ [M + H⁺] 824.3895, found 824.3888.

### Example 96

### Synthesis of HC65-18

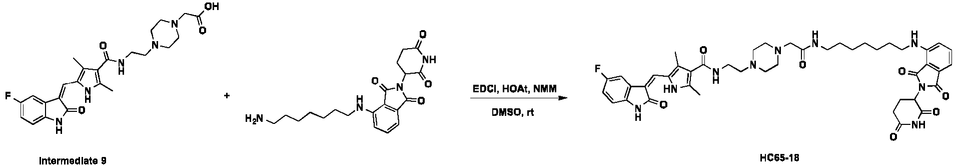

HC65-18 was synthesized following the standard procedure for preparing HC65-13 from Intermediate 9 (7 mg, 0.01 mmol, 1.1 equiv), 4-((7-aminoheptyl)amino)-2-(2,6-dioxopiperidin-3-yl)isoindoline-1,3-dione (5.5 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-18 was obtained as yellow solid in TFA salt form (4.2 mg, 39%). ¹H NMR (600 MHz, Methanol-*d*₄) δ 13.71 (s, 1H), 7.58 (s, 1H), 7.56 - 7.52 (m, 1H), 7.43 (dd, *J* = 8.9, 2.4 Hz, 1H), 7.03 (s, 1H), 7.02 (s, 1H), 6.92 - 6.84 (m, 2H), 5.06 (dd, *J* = 12.8, 5.5 Hz, 1H), 3.74 (t, *J* = 6.0 Hz, 2H), 3.43 (s, 4H), 3.39 - 3.28 (m, 6H), 3.25 (t, *J* = 7.0 Hz, 2H), 3.02 (s, 4H), 2.86 (ddd, *J* = 17.5, 13.9, 5.3 Hz, 1H), 2.79 - 2.67 (m, 2H), 2.52 (s, 3H), 2.48 (s, 3H), 2.16 - 2.08 (m, 1H), 1.67 (p, *J* = 7.0 Hz, 2H), 1.55 (p, *J* = 7.1 Hz, 2H), 1.48 - 1.34 (m, 6H). HRMS calcd for C₄₄H₅₃FN₉O₇ [M + H⁺] 838.4052, found 838.4069.

### Example 97

### Synthesis of HC65-19

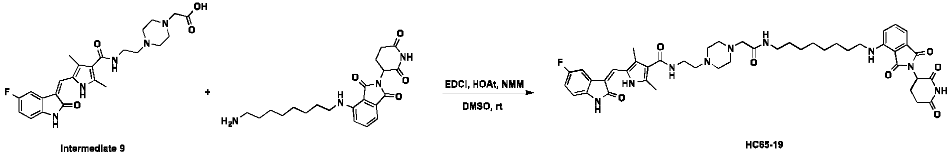

HC65-19 was synthesized following the standard procedure for preparing HC65-13 from Intermediate 9 (7 mg, 0.01 mmol, 1.1 equiv), 4-((8-aminooctyl)amino)-2-(2,6-dioxopiperidin-3-yl)isoindoline-1,3-dione (5.7 mg, 0.011 mmol, 1.1 equiv), EDCI (1-ethyl-3-(3-dimethylaminopropyl)carbodiimide) (2.9 mg, 0.015 mmol, 1.5 equiv), HOAt (1-hydroxy-7-azabenzo-triazole) (2.0 mg, 0.015 mmol, 1.5 equiv), and NMM (N-Methylmorpholine) (5.0 mg, 0.05 mmol, 5.0 equiv) in DMSO (1 mL). HC65-19 was obtained as yellow solid in TFA salt form (5.9 mg, 46%). ¹H NMR (600 MHz, Methanol-*d*₄) δ 13.74 (s, 1H), 7.60 (s, 1H), 7.57 - 7.52 (m, 1H), 7.45 (dd, *J* = 8.9, 2.5 Hz, 1H), 7.03 (s, 1H), 7.02 (s, 2H), 6.92 - 6.84 (m, 2H), 5.07 (dd, *J* = 12.7, 5.5 Hz, 1H), 3.73 (t, *J* = 6.1 Hz, 2H), 3.51- 3.27 (m, 10H), 3.24 (t, *J* = 7.1 Hz, 2H), 3.01 (s, 4H), 2.91 - 2.83 (m, 1H), 2.80 - 2.67 (m, 2H), 2.53 (s, 3H), 2.49 (s, 3H), 2.19 - 2.07 (m, 1H), 1.72 - 1.61 (m, 2H), 1.59 - 1.50 (m, 2H), 1.48 - 1.31 (m, 8H). HRMS calcd for C₄₅H₅₅FN₉O₇ [M + H⁺] 852.4208, found 852.4216.

### Example 98

### Synthesis of intermediate 10

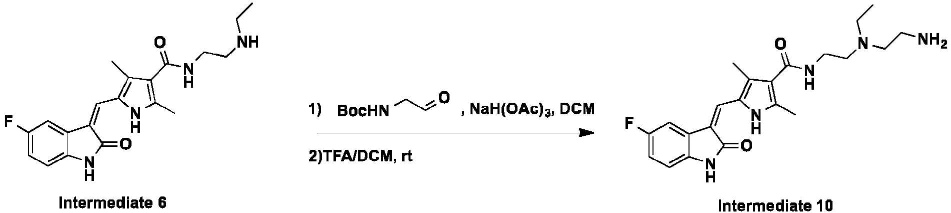

To a solution of intermediate 6 (60 mg, 0.124 mmol, 1.0 equiv) in DCM (2 mL) were added Et₃N (19 mg, 0.186 mmol, 1.5 equiv), *tert*-butyl (2-oxoethyl)carbamate (30 mg, 0.186 mmol, 1.5 equiv), NaH(OAc)₃ (1-ethyl-3-(3-dimethylaminopropyl)carbodiimide) (144 mg, 0.75 mmol, 1.5 equiv), HOAt (1-hydroxy-7-azabenzo-triazole) (102 mg, 0.75 mmol, 1.5 equiv), and NMM (N-Methylmorpholine) (52 mg, 0.248 mmol, 2.0 equiv). After being stirred overnight at room temperature for 3 h, the reaction was quenched with water (1 mL) and concentrated under vaccum. the resulting mixture was purified by preparative HPLC (10%-100% methanol / 0.1% TFA in H₂O) to afford yellow solid in TFA salt form. The obtained solid was dissolved in DCM (4 mL). To the resulting solution was added TFA (2 ml). After being stirred for 1 h at room temperature, the reaction mixture was concentrated and the residue was purified preparative HPLC (10%-100% methanol / 0.1% TFA in H₂O) to afford intermediate 10 as yellow solid in TFA salt form (51 mg, 64% yield for two step). ¹H NMR (600 MHz, Methanol-*d*₄) δ 7.61 (s, 1H), 7.45 (dd, *J* = 8.8, 2.1 Hz, 1H), 6.94 - 6.86 (m, 2H), 3.76 (t, *J* = 6.2 Hz, 2H), 3.55 (s, 2H), 3.50 - 3.35 (m, 6H), 2.55 (s, 3H), 2.51 (s, 3H), 1.40 (t, *J* = 7.2 Hz, 3H). ESI m/z =414.2 [M + H⁺].

### Example 99

### Synthesis of HC65-24

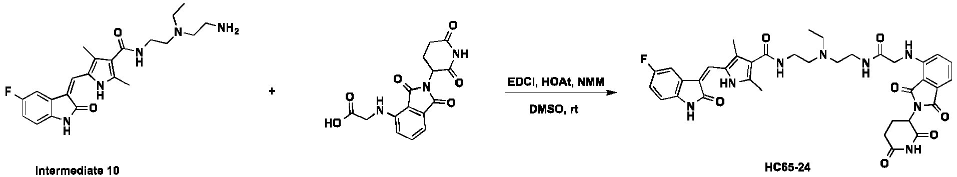

To a solution of Intermediate 10 (9.5 mg, 0.0148 mmol, 1.06 equiv) in DMSO (1 mL) were added (2-(2,6-dioxopiperidin-3-yl)-1,3-dioxoisoindolin-4-yl)glycine (4.6 mg, 0.014 mmol, 1.0 equiv), EDCI (1-ethyl-3-(3-dimethylaminopropyl)carbodiimide) (4.0 mg, 0.021 mmol, 1.5 equiv), HOAt (1-hydroxy-7-azabenzo-triazole) (2.9 mg, 0.021 mmol, 1.5 equiv), and NMM (N-Methylmorpholine) (5.7 mg, 0.056 mmol, 4.0 equiv). After being stirred overnight at room temperature, the resulting mixture was purified by preparative HPLC (10%-100% MeOH/ 0.1% TFA in H₂O) to afford HC65-24 as yellow solid in TFA salt form (7.4 mg, 63%). ¹H NMR (600 MHz, Methanol-*d*₄) δ 13.47 (s, 1H), 7.36 (t, *J* = 7.8 Hz, 1H), 7.33 - 7.27 (m, 2H), 6.87 (dtd, *J* = 19.1, 8.6, 3.3 Hz, 2H), 6.73 - 6.66 (m, 2H), 5.04 - 4.99 (m, 1H), 3.86 (s, 2H), 3.79 - 3.61 (m, 4H), 3.59 - 3.38 (m, 6H), 2.93 - 2.83 (m, 1H), 2.81 - 2.69 (m, 2H), 2.53 (s, 3H), 2.41 (s, 3H), 2.30 - 2.21 (m, 1H), 1.46 (t, *J* = 7.2 Hz, 3H). HRMS calcd for C₃₇H₄₀FN₈O₇ [M + H⁺] 727.3004, found 727.3013.

### Example 100

### Synthesis of HC65-25

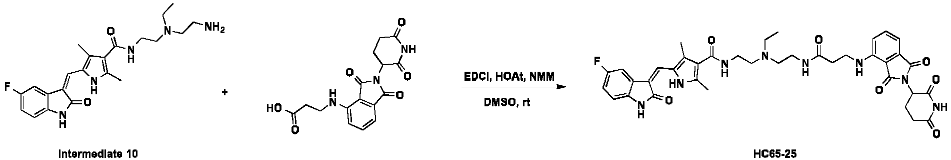

HC65-25 was synthesized following the standard procedure for preparing HC65-24 from Intermediate 10 (9.5 mg, 0.0148 mmol, 1.06 equiv), 3-((2-(2,6-dioxopiperidin-3-yl)-1,3-dioxoisoindolin-4-yl)amino)propanoic acid (4.9 mg, 0.014 mmol, 1.0 equiv), EDCI (1-ethyl-3-(3-dimethylaminopropyl)carbodiimide) (4.0 mg, 0.021 mmol, 1.5 equiv), HOAt (1-hydroxy-7-azabenzo-triazole) (2.9 mg, 0.021 mmol, 1.5 equiv), and NMM (N-Methylmorpholine) (5.7 mg, 0.056 mmol, 4.0 equiv) in DMSO (1 mL). HC65-25 was obtained as yellow solid in TFA salt form (8.0 mg, 67%). ¹H NMR (600 MHz, Methanol-*d*₄) δ 13.62 (s, 1H), 7.43 (s, 1H), 7.37 - 7.30 (m, 2H), 6.92 - 6.81 (m, 3H), 6.77 - 6.72 (m, 1H), 5.03 (d, *J* = 13.2 Hz, 1H), 3.81 - 3.67 (m, 2H), 3.67 - 3.55 (m, 2H), 3.52 - 3.35 (m, 8H), 2.92 - 2.81 (m, 1H), 2.79 - 2.64 (m, 2H), 2.61 - 2.55 (m, 2H), 2.51 (s, 3H), 2.44 (s, 3H), 2.19 - 2.09 (m, 1H), 1.40 (t, *J* = 7.2 Hz, 3H). HRMS calcd for C₃₈H₄₂FN₈O₇ [M + H⁺] 741.3160, found 741.3168.

### Example 101

### Synthesis of HC65-26

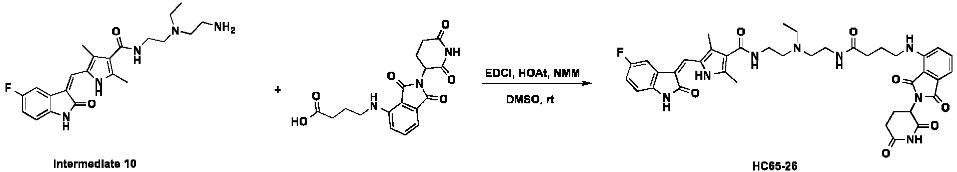

HC65-26 was synthesized following the standard procedure for preparing HC65-24 from Intermediate 10 (11.0 mg, 0.0172 mmol, 1.06 equiv), 4-((2-(2,6-dioxopiperidin-3-yl)-1,3-dioxoisoindolin-4-yl)amino)butanoic acid (5.8 mg, 0.016 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.3 mg, 0.024 mmol, 1.5 equiv), and NMM (N-Methylmorpholine) (8.1 mg, 0.08 mmol, 5.0 equiv) in DMSO (1 mL). HC65-26 was obtained as yellow solid in TFA salt form (7.9 mg, 57%). ¹H NMR (600 MHz, Methanol-*d*₄) δ 13.65 (s, 1H), 7.44 (s, 1H), 7.39 - 7.31 (m, 2H), 6.90 - 6.78 (m, 4H), 5.03 (dd, *J* = 12.7, 5.4 Hz, 1H), 3.79 - 3.70 (m, 2H), 3.63 - 3.55 (m, 2H), 3.52 - 3.32 (m, 6H), 3.17 - 3.09 (m, 2H), 2.86 (ddd, *J* = 18.5, 13.8, 5.3 Hz, 1H), 2.79 - 2.64 (m, 2H), 2.53 (s, 3H), 2.47 (s, 3H), 2.36 - 2.30 (m, 2H), 2.16- 2.08 (m, 1H), 1.88 - 1.69 (m, 2H), 1.47 - 1.36 (m, 3H). HRMS calcd for C₃₉H₄₄FN₈O₇ [M + H⁺] 755.3317, found 755.3312.

### Example 102

### Synthesis of HC65-27

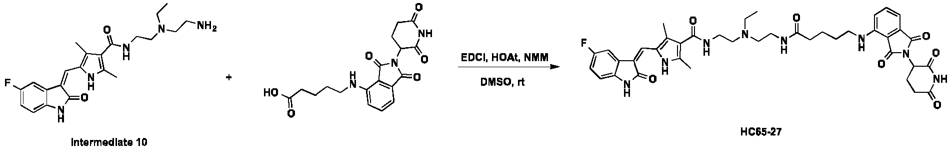

HC65-27 was synthesized following the standard procedure for preparing HC65-24 from Intermediate 10 (11.0 mg, 0.0172 mmol, 1.06 equiv), 5-((2-(2,6-dioxopiperidin-3-yl)-1,3-dioxoisoindolin-4-yl)amino)pentanoic acid (6.0 mg, 0.016 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.3 mg, 0.024 mmol, 1.5 equiv), and NMM (N-Methylmorpholine) (8.1 mg, 0.08 mmol, 5.0 equiv) in DMSO (1 mL). HC65-27 was obtained as yellow solid in TFA salt form (5.4 mg, 38%). ¹H NMR (600 MHz, Methanol-*d*₄) δ 13.70 (s, 1H), 7.48 (s, 1H), 7.36 - 7.29 (m, 2H), 6.82 (td, *J* = 8.9, 2.5 Hz, 1H), 6.80 - 6.74 (m, 4H), 5.05 (dd, *J* = 12.7, 5.6 Hz, 1H), 3.82 - 3.70 (m, 2H), 3.65 - 3.50 (m, 2H), 3.49 - 3.34 (m, 6H), 3.08 - 3.00 (m, 2H), 2.88 (ddd, *J* = 17.0, 13.8, 5.2 Hz, 1H), 2.81 - 2.68 (m, 2H), 2.55 (s, 3H), 2.49 (s, 3H), 2.27 (t, *J* = 7.0 Hz, 2H), 2.18 - 2.10 (m, 1H), 1.64 - 1.46 (m, 4H), 1.40 (t, *J* = 7.2 Hz, 3H). HRMS calcd for C₄₀H₄₆FN₈O₇ [M + H⁺] 769.3473, found 769.3489.

### Example 103

### Synthesis of HC65-28

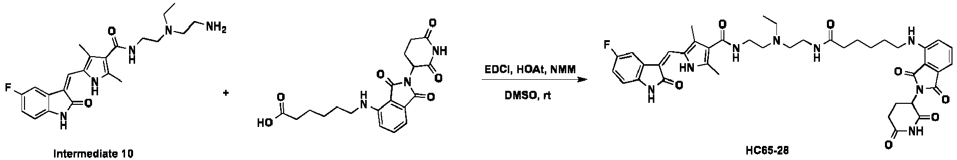

HC65-28 was synthesized following the standard procedure for preparing HC65-24 from Intermediate 10 (11.0 mg, 0.0172 mmol, 1.06 equiv), 6-((2-(2,6-dioxopiperidin-3-yl)-1,3-dioxoisoindolin-4-yl)amino)hexanoic acid (6.2 mg, 0.016 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.3 mg, 0.024 mmol, 1.5 equiv), and NMM (N-Methylmorpholine) (8.1 mg, 0.08 mmol, 5.0 equiv) in DMSO (1 mL). HC65-28 was obtained as yellow solid in TFA salt form (6.9 mg, 48%). ¹H NMR (600 MHz, Methanol-*d*₄) δ 13.72 (s, 1H), 7.50 (s, 1H), 7.38 (dd, *J* = 8.5, 7.1 Hz, 1H), 7.33 (d, *J* = 9.0 Hz, 1H), 6.86 (d, *J* = 7.1 Hz, 1H), 6.83 - 6.72 (m, 3H), 5.05 (dd, *J* = 12.7, 5.5 Hz, 1H), 3.74 (t, *J* = 5.3 Hz, 2H), 3.66 - 3.53 (m, 3H), 3.52 - 3.30 (m, 5H), 3.05 (t, *J* = 7.2 Hz, 2H), 2.87 (ddd, *J* = 17.2, 13.8, 5.3 Hz, 1H), 2.80 - 2.66 (m, 2H), 2.56 (s, 3H), 2.50 (s, 3H), 2.23 (t, *J* = 7.6 Hz, 2H), 2.18-2.10 (m, 1H), 1.60-1.43 (m, 4H), 1.41 (t, *J* = 7.2 Hz, 3H), 1.34 - 1.25 (m, 2H). HRMS calcd for C₄₁H₄₈FN₈O₇ [M + H⁺] 783.3630, found 783.3639.

### Example 104

### Synthesis of HC65-29

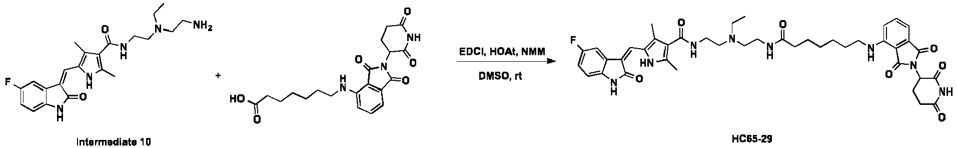

HC65-29 was synthesized following the standard procedure for preparing HC65-24 from Intermediate 10 (11.0 mg, 0.0172 mmol, 1.06 equiv), 7-((2-(2,6-dioxopiperidin-3-yl)-1,3-dioxoisoindolin-4-yl)amino)heptanoic acid (6.4 mg, 0.016 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.3 mg, 0.024 mmol, 1.5 equiv), and NMM (N-Methylmorpholine) (8.1 mg, 0.08 mmol, 5.0 equiv) in DMSO (1 mL). HC65-29 was obtained as yellow solid in TFA salt form (7.7 mg, 53%). ¹H NMR (600 MHz, Methanol-d4) δ 13.75 (s, 1H), 7.55 (s, 1H), 7.41 - 7.35 (m, 2H), 6.92 - 6.87 (m, 1H), 6.82 - 6.73 (m, 3H), 5.06 (dd, J = 12.8, 5.5 Hz, 1H), 3.80 - 3.68 (m, 2H), 3.67 - 3.52 (m, 1H), 3.52 - 3.32 (m, 7H), 3.05 (t, J = 7.1 Hz, 2H), 2.88 (ddd, J = 17.5, 14.0, 5.3 Hz, 1H), 2.80 - 2.68 (m, 2H), 2.56 (s, 3H), 2.50 (s, 3H), 2.21 (t, J = 7.7 Hz, 2H), 2.16 - 2.10 (m, 1H), 1.56 - 1.43 (m, 4H), 1.40 (t, J = 7.2 Hz, 3H), 1.30 - 1.20 (m, 4H). HRMS calcd for C₄₂H₅₀FN₈O₇ [M + H⁺] 797.3786, found 797.3798.

### Example 105

### Synthesis of HC65-30

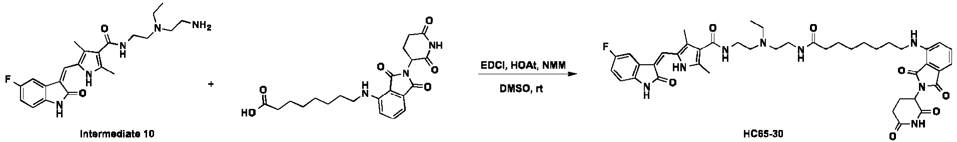

HC65-30 was synthesized following the standard procedure for preparing HC65-24 from Intermediate 10 (11.0 mg, 0.0172 mmol, 1.06 equiv), 8-((2-(2,6-dioxopiperidin-3-yl)-1,3-dioxoisoindolin-4-yl)amino)octanoic acid (6.7 mg, 0.016 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.3 mg, 0.024 mmol, 1.5 equiv), and NMM (N-Methylmorpholine) (8.1 mg, 0.08 mmol, 5.0 equiv) in DMSO (1 mL). HC65-30 was obtained as yellow solid in TFA salt form (6.2 mg, 42%). ¹H NMR (600 MHz, Methanol-*d*₄) δ 13.75 (s, 1H), 7.55 (s, 1H), 7.44 (dd, *J* = 8.6, 7.1 Hz, 1H), 7.37 (dd, *J* = 8.9, 2.4 Hz, 1H), 6.95 (d, *J* = 7.1 Hz, 1H), 6.82 (d, *J* = 8.6 Hz, 1H), 6.81 - 6.74 (m, 2H), 5.06 (dd, *J* = 12.8, 5.5 Hz, 1H), 3.74 (t, *J* = 5.4 Hz, 2H), 3.60 (dd, *J* = 13.9, 9.0 Hz, 1H), 3.52 - 3.30 (m, 7H), 3.07 (t, *J* = 7.2 Hz, 2H), 2.88 (ddd, *J* = 17.6, 13.9, 5.3 Hz, 1H), 2.81 - 2.66 (m, 2H), 2.55 (s, 3H), 2.50 (s, 3H), 2.20 (t, *J* = 7.7 Hz, 2H), 2.16 - 2.10 (m, 1H), 1.55 - 1.42 (m, 4H), 1.40 (t, *J* = 7.2 Hz, 3H), 1.29 - 1.17 (m, 6H). HRMS calcd for C₄₃H₅₂FN₈O₇ [M + H⁺] 811.3943, found 811.3939.

### Example 106

### Synthesis of HC65-33

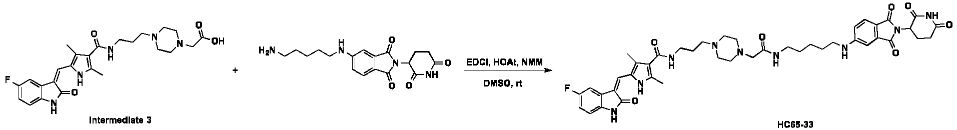

HC65-33 was synthesized following the standard procedure for preparing HC58-53 from Intermediate 3 (10.0 mg, 0.014 mmol, 1.0 equiv), 5-((5-aminopentyl)amino)-2-(2,6-dioxopiperidin-3-yl)isoindoline-1,3-dione (7.1 mg, 0.015 mmol, 1.1 equiv), EDCI (4.0 mg, 0.021 mmol, 1.5 equiv), HOAt (3.0 mg, 0.021 mmol, 1.5 equiv), and NMM (7.1 mg, 0.07 mmol, 5.0 equiv) in DMSO (1 mL). HC65-33 was obtained as yellow solid in TFA salt form (8.7 mg, 59%). ¹H NMR (600 MHz, Methanol-*d*₄) δ 13.67 (s, 1H), 7.60 - 7.51 (m, 2H), 7.40 (d, *J* = 8.3 Hz, 1H), 6.95 (s, 1H), 6.90-6.79 (m, 4H), 5.04 (dd, *J* = 12.8, 6.3 Hz, 1H), 3.62-3.35 (m, 6H), 3.31-3.17 (m, 8H), 2.99 (s, 4H), 2.90 - 2.80 (m, 1H), 2.79 - 2.65 (m, 2H), 2.50 (s, 3H), 2.46 (s, 3H), 2.15 - 2.03 (m, 3H), 1.77 - 1.65 (m, 2H), 1.63 - 1.54 (m, 2H), 1.52 - 1.39 (m, 2H). HRMS calcd for C₄₃H₅₁FN₉O₇ [M + H⁺] 824.3895, found 824.3889.

### Example 107

### Synthesis of HC65-34

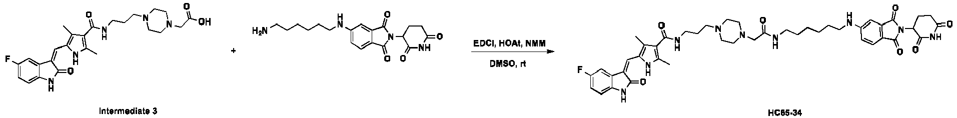

HC65-34 was synthesized following the standard procedure for preparing HC58-53 from Intermediate 3 (10.0 mg, 0.014 mmol, 1.0 equiv), 5-((6-aminohexyl)amino)-2-(2,6-dioxopiperidin-3-yl)isoindoline-1,3-dione (7.3 mg, 0.015 mmol, 1.1 equiv), EDCI (4.0 mg, 0.021 mmol, 1.5 equiv), HOAt (3.0 mg, 0.021 mmol, 1.5 equiv), and NMM (7.1 mg, 0.07 mmol, 5.0 equiv) in DMSO (1 mL). HC65-34 was obtained as yellow solid in TFA salt form (7.4 mg, 50%). ¹H NMR (600 MHz, Methanol-*d*₄) δ 13.65 (s, 1H), 7.56 - 7.51 (m, 2H), 7.42 - 7.36 (m, 1H), 6.94 (d, *J* = 2.1 Hz, 1H), 6.91 - 6.83 (m, 2H), 6.81 (dd, *J* = 8.4, 2.1 Hz, 1H), 5.04 (dd, *J* = 12.8, 5.5 Hz, 1H), 3.59 - 3.37 (m, 8H), 3.29 - 3.22 (m, 4H), 3.19 (t, *J* = 7.0 Hz, 2H), 3.03 (s, 4H), 2.85 (ddd, *J* = 18.6, 14.1, 5.3 Hz, 1H), 2.77 - 2.65 (m, 2H), 2.50 (s, 3H), 2.46 (s, 3H), 2.15 - 2.04 (m, 3H), 1.66 (p, *J* = 7.2 Hz, 2H), 1.57 (p, *J* = 7.2 Hz, 2H), 1.51 - 1.34 (m, 4H). HRMS calcd for C₄₄H₅₃FN₉O₇ [M + H⁺] 838.4052, found 838.4064.

### Example 108

### Synthesis of HC65-35

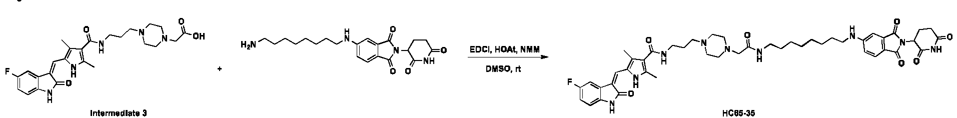

HC65-35 was synthesized following the standard procedure for preparing HC58-53 from Intermediate 3 (10.0 mg, 0.014 mmol, 1.0 equiv), 5-((8-aminooctyl)amino)-2-(2,6-dioxopiperidin-3-yl)isoindoline-1,3-dione (7.7 mg, 0.015 mmol, 1.1 equiv), EDCI (4.0 mg, 0.021 mmol, 1.5 equiv), HOAt (3.0 mg, 0.021 mmol, 1.5 equiv), and NMM (7.1 mg, 0.07 mmol, 5.0 equiv) in DMSO (1 mL). HC65-35 was obtained as yellow solid in TFA salt form (6.6 mg, 43%). ¹H NMR (600 MHz, Methanol-*d*₄) δ 13.66 (s, 1H), 7.55 - 7.51 (m, 2H), 7.39 (dd, *J* = 8.8, 2.3 Hz, 1H), 6.93 (d, *J* = 2.2 Hz, 1H), 6.89 - 6.83 (m, 2H), 6.80 (dd, *J* = 8.4, 2.2 Hz, 1H), 5.04 (dd, *J* = 12.8, 5.5 Hz, 1H), 3.57 - 3.37 (m, 8H), 3.29 - 3.21 (m, 4H), 3.18 (t, *J* = 7.1 Hz, 2H), 3.10 (s, 4H), 2.85 (ddd, *J* = 17.6, 14.0, 5.3 Hz, 1H), 2.79 - 2.64 (m, 2H), 2.50 (s, 3H), 2.46 (s, 3H), 2.14 - 2.05 (m, 3H), 1.64 (p, *J* = 7.1 Hz, 2H), 1.54 (p, *J* = 6.9 Hz, 2H), 1.40 (d, *J* = 39.7 Hz, 8H). HRMS calcd for C₄₆H₅₇FN₉O₇ [M + H⁺] 866.4365, found 866.4379.

### Example 109

### Synthesis of HC65-37

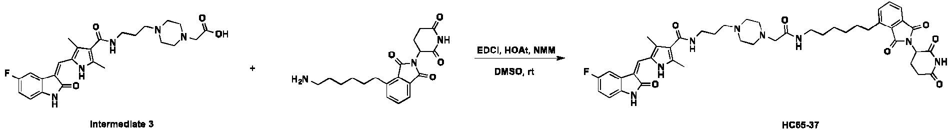

HC65-37 was synthesized following the standard procedure for preparing HC58-53 from Intermediate 3 (12.0 mg, 0.0169 mmol, 1.0 equiv), 4-(6-aminohexyl)-2-(2,6-dioxopiperidin-3-yl)isoindoline-1,3-dione (8.9 mg, 0.0185 mmol, 1.1 equiv), EDCI (4.9 mg, 0.0255 mmol, 1.5 equiv), HOAt (3.5 mg, 0.0255 mmol, 1.5 equiv), and NMM (8.6 mg, 0.085mmol, 5.0 equiv) in DMSO (1 mL). HC65-37 was obtained as yellow solid in TFA salt form (9.3 mg, 52%). ¹H NMR (600 MHz, Methanol-*d*₄) δ 13.64 (s, 1H), 7.70 (d, *J* = 4.4 Hz, 2H), 7.61 (t, *J* = 4.4 Hz, 1H), 7.52 (d, *J* = 3.2 Hz, 1H), 7.38 (dd, *J* = 8.9, 2.3 Hz, 1H), 6.91- 6.81 (m, 2H), 5.13 (dd, *J* = 12.8, 5.5 Hz, 1H), 3.55 - 3.37 (m, 6H), 3.24 (t, *J* = 7.2 Hz, 4H), 3.13 - 2.97 (m, 6H), 2.88 (ddd, *J* = 18.7, 13.9, 5.3 Hz, 1H), 2.81 - 2.67 (m, 2H), 2.48 (s, 3H), 2.44 (s, 3H), 2.19 - 2.00 (m, 3H), 1.67 (p, *J* = 7.3 Hz, 2H), 1.54 (p, *J* = 7.1 Hz, 2H), 1.40 (dt, *J* = 9.8, 5.7 Hz, 6H). HRMS calcd for C₄₄H₅₂FN₈O₇ [M + H⁺] 823.3943, found 823.3954.

### Example 110

### Synthesis of HC65-74

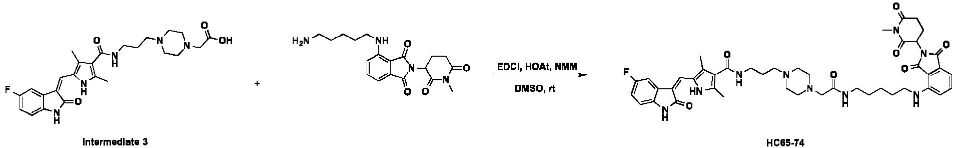

HC65-74 was synthesized following the standard procedure for preparing HC58-53 from Intermediate 3 (12.0 mg, 0.0169 mmol, 1.0 equiv), 4-((5-aminopentyl)amino)-2-(1-methyl-2,6-dioxopiperidin-3-yl)isoindoline-1,3-dione (9.0 mg, 0.0185 mmol, 1.1 equiv), EDCI (4.9 mg, 0.0255 mmol, 1.5 equiv), HOAt (3.5 mg, 0.0255 mmol, 1.5 equiv), and NMM (8.5 mg, 0.085mmol, 5.0 equiv) in DMSO (1 mL). HC65-74 was obtained as yellow solid in TFA salt form (14.8 mg, 82%). ¹H NMR (600 MHz, Acetone-*d*₆) δ 13.57 (s, 1H), 9.68 (s, 1H), 7.50 - 7.40 (m, 2H), 7.29 (dd, *J* = 8.5, 7.0 Hz, 1H), 7.25 (dd, *J* = 9.2, 2.2 Hz, 1H), 7.20 (t, *J* = 6.0 Hz, 1H), 6.79 (d, *J* = 8.5 Hz, 1H), 6.73 (d, *J* = 7.0 Hz, 1H), 6.67 - 6.59 (m, 2H), 4.79 (dd, *J* = 13.0, 5.4 Hz, 1H), 3.45 - 3.23 (m, 8H), 3.16 - 3.04 (m, 8H), 2.98 (q, *J* = 6.6 Hz, 2H), 2.79 (s, 3H), 2.69 (ddd, *J* = 18.6, 13.8, 5.3 Hz, 1H), 2.58 (dt, *J* = 17.3, 3.6 Hz, 1H), 2.43 (qd, *J* = 13.1, 4.5 Hz, 1H), 2.26 (s, 3H), 2.22 (s, 3H), 1.95 - 1.85 (m, 3H), 1.42 (p, *J* = 7.3 Hz, 2H), 1.29 (p, *J* = 7.2 Hz, 2H), 1.17 (p, *J* = 7.7 Hz, 2H). HRMS calcd for C₄₄H₅₃FN₉O₇ [M + H⁺] 838.4052, found 838.4059.

### Example 111

### Synthesis of HC65-75

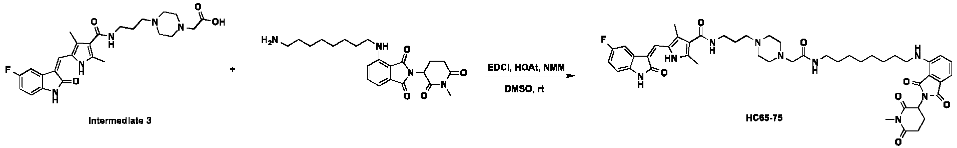

HC65-75 was synthesized following the standard procedure for preparing HC58-53 from Intermediate 3 (12.0 mg, 0.0169 mmol, 1.0 equiv), 4-((8-aminooctyl)amino)-2-(1-methyl-2,6-dioxopiperidin-3-yl)isoindoline-1,3-dione (9.9 mg, 0.0185 mmol, 1.1 equiv), EDCI (4.9 mg, 0.0255 mmol, 1.5 equiv), HOAt (3.5 mg, 0.0255 mmol, 1.5 equiv), and NMM (8.6 mg, 0.085mmol, 5.0 equiv) in DMSO (1 mL). HC65-75 was obtained as yellow solid in TFA salt form (13.0 mg, 69%). ¹H NMR (600 MHz, Acetone-*d*₆) δ 13.86 (s, 1H), 9.98 (s, 1H), 7.78 - 7.66 (m, 2H), 7.64 - 7.53 (m, 2H), 7.51 - 7.45 (m, 1H), 7.12 - 7.06 (m, 1H), 7.05 - 6.99 (m, 1H), 6.97 - 6.88 (m, 2H), 5.15 - 5.05 (m, 1H), 3.81 - 3.49 (m, 8H), 3.47 - 3.30 (m, 8H), 3.27 - 3.19 (m, 2H), 3.09 (s, 3H), 3.03 - 2.94 (m, 1H), 2.92 - 2.84 (m, 1H), 2.80 - 2.69 (m, 1H), 2.56 (s, 3H), 2.52 (s, 3H), 2.28 - 2.14 (m, 3H), 1.74 - 1.65 (m, 2H), 1.55 - 1.26 (m, 10H). HRMS calcd for C₄₇H₅₉FN₉O₇ [M + H⁺] 880.4521, found 880.4533.

### Example 112

### Synthesis of tert-butyl N-tert-butoxycarbonyl-N-[4-methyl-5-(4,4,5,5-tetramethyl-1,3,2-dioxaborolan-2-yl)-3-pyridyl] carbamate

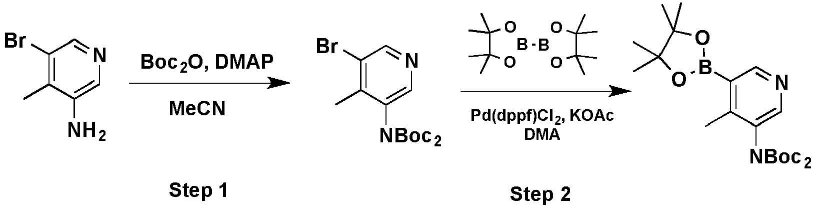

### Step 1: N-tert-butoxycarbonyl-N-(4-methyl-5-bromo-3-pyridyl)carbamate

To a solution of 5-bromo-4-methylpyridin-3-amine (1.88 g, 10 mmol, 1.0 equiv) in MeCN (20 mL) were added DMAP (122 mg, 1.0 mmol, 0.1 equiv), di-*tert*-butyl dicarbonate (6.55 g, 30 mmol, 3.0 equiv). The mixture was heated to 60 °C and stirred overnight. The reaction was quenched with water (20 mL) and extracted with ethyl acetate (3 x 20 mL), dried over Na₂SO₄, filtered and evaporated. The resulting residue was purified by silica gel column (hexane/ ethyl acetate = 4:1) to afford **N-tert-butoxycarbonyl-N-(4-methyl-5-bromo-3-pyridyl)carbamate** (2.383 g, 62%). ESI m/z =387.2 [M + H⁺].

### Step 2: tert-butyl N-tert-butoxycarbonyl-N-[4-methyl-5-(4,4,5,5-tetramethyl-1,3,2-dioxaborolan-2-yl)-3-pyridyl] carbamate

Dissolved the obtained **N-tert-butoxycarbonyl-N-(4-methyl-5-bromo-3-pyridyl)carbamate** (2.383 g, 6.16 mmol, 1 equiv) in dimethyl acetamide (24 mL). To the solution were added Bis(pinacolato)diboron (7.823 g, 30.8 mmol, 5 equiv), KOAc (1.813 g, 18.5 mmol, 3 equiv) and Pd(dppf)Cl₂ (454 mg, 0.62 mmol, 0.1 equiv) under N₂. The mixture was heated to 90 °C and stirred for 2 h. Cooled to room temperature, quenched with water (20 mL) and extracted with ethyl acetate (3 x 20 mL). Combined the organic layers, dried over Na₂SO₄, filtered and evaporated. The resulting residue was purified by silica gel column (hexane/ ethyl acetate = 1:2) to afford ***tert-*butyl N-*tert*-butoxycarbonyl-N-[4-methyl-5-(4,4,5,5-tetramethyl-1,3,2-dioxaborolan-2-yl)-3-pyridyl]carbamate** (2.077 g, 78%). ESI m/z =353.2 [M(Corresponding boric acid) + H⁺].

### Example 113

### Synthesis of intermediate 11

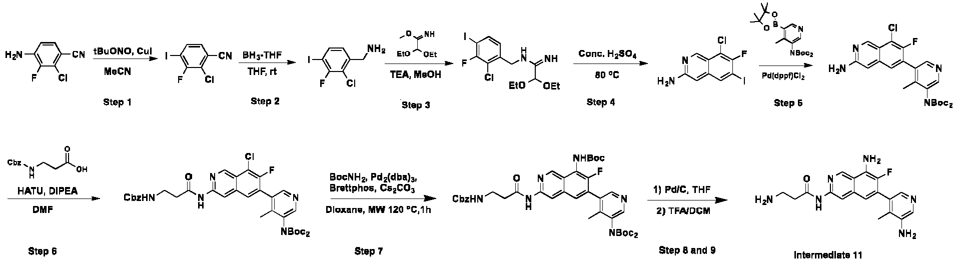

### Step 1: 2-chloro-3-fluoro-4-iodobenzonitrile

To a suspension of CuI (9.24 g, 49 mmol) in acetonitrile (200 mL) was dropwise added 90% tert-butyl nitrite (6.95 mg, 60 mmol) at 65 °C. The mixture was stirred at 65 °C for 10 minutes. Then 4-amino-2-chloro-3-fluorobenzonitrile (6.9 g, 40.4 mmol) was added to the reaction mixture for 1 h. The mixture was stirred at 65 °C for 2 h. The mixture was quenched with saturated aqueous Na₂S₂O₄ and extracted with ethyl acetate (3 x 300 mL). The combined organic layers was washed with brine, dried over Na₂SO₄ and filtered. The filtrate was concentrated to give crude product, which was purified by silica gel column (hexane/ ethyl acetate = 9:1) to give 2-chloro-3-fluoro-4-iodo-benzonitrile (6.66 g, 59%) as a yellow solid. ¹H NMR (500 MHz, Chloroform-*d*) δ 7.82 (dd, *J* = 8.3, 5.5 Hz, 1H), 7.24 (dd, *J* = 8.3, 1.4 Hz, 1H).

### Step 2: (2-chloro-3-fluoro-4-iodophenyl)methanamine

2-Chloro-3-fluoro-4-iodobenzonitrile (2 g, 7 mmol) was added to a solution of borane-THF (35mL, 1 M in THF, 35 mmol) in THF (20 mL) at 0 °C. The mixture was then warmed to room temperature and stirred overnight. The reaction solution was quenched by the dropwise addition of a solution of HCl (3 M, 8 mL). The mixture was neutralized with an aqueous solution of NaHCOs to pH=8 and extracted with dichloromethane (2 x 200 mL). The combined organic layer was washed with water and brine, dried over Na₂SO₄, filtered and concentrated to give (2-chloro-3-fluoro-4-iodophenyl)methanamine (1.287 g, crude yield 65%), which was used directly in the next step without further purification. ESI m/z =285.9 [M + H⁺].

### Step 3: N-(2-chloro-3-fluoro-4-iodobenzyl)-2,2-diethoxyacetimidamide

To a solution of methyl 2,2-diethoxyacetimidate (1 g, 6.4 mmol) in methanol (15 mL) was added (2-chloro-3-fluoro-4-iodophenyl)methanamine (1.287 mg, 4.5 mmol). The reaction mixture was stirred at room temperature for 1 hours, and then concentrated to dryness. The residue was diluted with DCM (50 mL), washed with water (50 mL) and brine (50 mL), dried over Na₂SO₄, filtered and concentrated to give N-(2-chloro-3-fluoro-4-iodobenzyl)-2,2-diethoxyacetimidamide as a yellow solid. It was directly used to the next step without purification. ESI m/z =415.0 [M + H⁺].

### Step 4: 8-chloro-7-fluoro-6-iodoisoquinolin-3-amine

A solution of N-(2-chloro-3-fluoro-4-iodobenzyl)-2,2-diethoxyacetimidamide (from Step 3) and cone. H2SO4 (8 mL) was stirred at 60 °C for overnight. The mixture was cooled to 0 °C and NaOH was added to adjust to pH=9. The residue was extracted with dichloromethane (4 x 80 mL). The combined organic layers were washed with brine, dried over Na₂SO₄, filtered and concentrated to give 8-chloro-7-fluoro-6-iodoisoquinolin-3-amine (1.01 g, 70% for two steps) as a yellow solid, which was directly used to the next step. ESI m/z =322.9 [M + H⁺].

### Step 5: tert-butyl N-[5-(3-amino-8-chloro-7-fluoro-6-isoquinolyl)-4-methyl-3-pyridyl]-N-tert-butoxycarbonyl-carbamate

A mixture of 8-chloro-7-fluoro-6-iodo-isoquinolin-3-amine (636 mg, 2 mmol, 1.0 equiv), *tert-*butyl N-*tert*-butoxycarbonyl-N-[4-methyl-5-(4,4,5,5-tetramethyl-1,3,2-dioxaborolan-2-yl)-3-pyridyl]carbamate (1.04 g, 2.4 mmol, 1.2 equiv), Pd(dppf)Cl₂ (0.29 g, 0.4 mmol, 0.2 equiv), potassium carbonate (830 mg, 6 mmol, 3 equiv) in 1,4-dioxane (10 mL) and water (5 mL) was stirred at 80 °C (Microwave) for 30 min. The reaction was filtered. The product was extracted with ethyl acetate. The combined organic extracts were combined and concentrated under vacuum. The residue was purified by silica gel column (hexane/ ethyl acetate = 1:4) to give the *tert*-butyl N-[5-(3-amino-8-chloro-7-fluoro-6-isoquinolyl)-4-methyl-3-pyridyl]-N-*tert*-butoxycarbonyl-carbamate as a yellow solid (690 mg, 62%). ESI m/z =503.2 [M + H⁺].

### Step 6: tert-butyl (5-(3-(3-(((benzyloxy)carbonyl)amino)propanamido)-8-chloro-7-fluoroisoquinolin-6-yl)-4-methylpyridin-3-yl)(tert-butoxycarbonyl)carbamate

A mixture of *tert*-butyl N-[5-(3-amino-8-chloro-7-fluoro-6-isoquinolyl)-4-methyl-3-pyridyl]-N-*tert*-butoxycarbonyl-carbamate (300 mg, 0.6 mmol, 1.0 equiv), 3-(((benzyloxy)carbonyl)amino)propanoic acid (201 mg, 0.9 mmol, 1.5 equiv), HATU (342 mg, 0.9 mmol, 1.5 equiv) and DIPEA (232.2 mg, 1.8 mmol, 3 equiv) in DMF (6 mL) was stirred at 50 °C overnight. Cooled down and the mixture was purified preparative HPLC (10%-100% methanol / 0.1% TFA in H₂O) to afford *tert*-butyl (5-(3-(3-(((benzyloxy)carbonyl)amino)propanamido)-8-chloro-7-fluoroisoquinolin-6-yl)-4-methylpyridin-3-yl)(tert-butoxycarbonyl)carbamate as yellow solid in TFA salt form (346 mg, 62%). ESI m/z =708.3 [M + H⁺].

### Step 7: tert-butyl (5-(3-(3-(((benzyloxy)carbonyl)amino)propanamido)-8-((tert-butoxycarbonyl)amino)-7-fluoroisoquinolin-6-yl)-4-methylpyridin-3-yl)(tert-butoxycarbonyl)carbamate

A mixture of *tert*-butyl (5-(3-(3-(((benzyloxy)carbonyl)amino)propanamido)-8-chloro-7-fluoroisoquinolin-6-yl)-4-methylpyridin-3-yl)(*tert*-butoxycarbonyl)carbamate (276 mg, 0.3 mmol, 1.0 equiv), *tert*-butyl carbamate (518 mg, 4.4 mmol, 15 equiv), Pd₂(dba)₂ (61 mg, 0.06 mmol, 0.2 equiv), Brettphos (64 mg, 0.12 mmol, 0.4 equiv), cesium carbonate (482 mg, 1.5 mmol, 5 equiv) in 1,4-dioxane (10 mL) was stirred at 120 °C (Microwave) for 1 h. Cooled to room temperature and water was added. The product was extracted with ethyl acetate. The combined organic extracts were combined and concentrated under vacuum. The residue was purified by silica gel column (hexane/ ethyl acetate = 1:4) to give the *tert*-butyl (5-(3-(3-(((benzyloxy)carbonyl)amino)propanamido)-8-((*tert*-butoxycarbonyl)amino)-7-fluoroisoquinolin-6-yl)-4-methylpyridin-3-yl)(*tert*-butoxycarbonyl)carbamate as a yellow solid (114 mg, 48%). ESI m/z =789.4 [M + H⁺].

### Step 8 and 9: intermediate 11

A mixture of *tert*-butyl (5-(3-(3-(((benzyloxy)carbonyl)amino)propanamido)-8-((*tert-*butoxycarbonyl)amino)-7-fluoroisoquinolin-6-yl)-4-methylpyridin-3-yl)(*tert-*butoxycarbonyl)carbamate (553 mg, 0.7 mmol, 1 equiv) and Pd/C (55 mg, 0.1 equiv) in THF (10 mL) under H₂ was stirred at room temperature overnight. Filtered and concentrated under vacuum to give a yellow solid. ESI m/z =655.3 [M + H⁺]. Dissolved the obtained solid in DCM (4 mL). To the solution was added TFA (2 mL). Stirred at room temperature for 1 h and concentrated under vacuum. The residue was purified preparative HPLC (10%-100% methanol / 0.1% TFA in H₂O) to afford intermediate 11 as a yellow solid (395 mg, 81% for two steps) in TFA salt form. ¹H NMR (600 MHz, Methanol-*d*₄) δ 9.41 (s, 1H), 8.38 (s, 1H), 8.12 (s, 1H), 8.02 (s, 1H), 7.10 (d, *J* = 5.9 Hz, 1H), 3.37 - 3.34 (m, 2H), 2.97 (t, *J* = 6.4 Hz, 2H), 2.26 (s, 3H). ESI m/z =355.2 [M + H⁺].

### Example 114

### Synthesis of intermediate 12

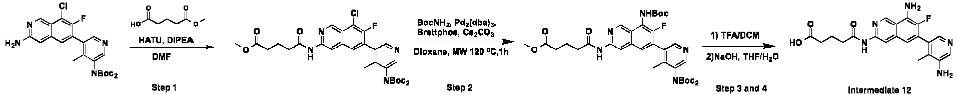

### Step 1: methyl 5-((6-(5-(bis(tert-butoxycarbonyl)amino)-4-methylpyridin-3-yl)-8-chloro-7-fluoroisoquinolin-3-yl)amino)-5-oxopentanoate

A mixture of *tert*-butyl N-[5-(3-amino-8-chloro-7-fluoro-6-isoquinolyl)-4-methyl-3-pyridyl]-N-*tert*-butoxycarbonyl-carbamate (800 mg, 1.6 mmol, 1.0 equiv), 5-methoxy-5-oxopentanoic acid (701 mg, 4.8 mmol, 3 equiv), HATU (1.82 g, 4.8 mmol, 3 equiv) and DIPEA (1.03 g, 8.0 mmol, 5 equiv) in DMF (8 mL) was stirred at room temperature overnight. Cooled down and the mixture was purified preparative HPLC (10%-100% methanol / 0.1% TFA in H₂O) to afford methyl 5-((6-(5-(bis(*tert*-butoxycarbonyl)amino)-4-methylpyridin-3-yl)-8-chloro-7-fluoroisoquinolin-3-yl)amino)-5-oxopentanoate as yellow solid in TFA salt form (864 mg, 63%). ESI m/z =631.2 [M +H⁺].

### Step 2: methyl 5-((6-(5-(bis(tert-butoxycarbonyl)amino)-4-methylpyridin-3-yl)-8-((tert-butoxycarbonyl)amino)-7-fluoroisoquinolin-3-yl)amino)-5-oxopentanoate

A mixture of methyl 5-((6-(5-(bis(*tert*-butoxycarbonyl)amino)-4-methylpyridin-3-yl)-8-chloro-7-fluoroisoquinolin-3-yl)amino)-5-oxopentanoate (864 mg, 1 mmol, 1.0 equiv), *tert*-butyl carbamate (1.76 mg, 15 mmol, 15 equiv), Pd₂(dba)₂·chloroform (207 mg, 0.2 mmol, 0.2 equiv), Brettphos (215 mg, 0.4 mmol, 0.4 equiv), cesium carbonate (1.63 mg, 5 mmol, 5 equiv) in 1,4-dioxane (15 mL) was stirred at 120 °C (Microwave) for 1 h. Cooled to room temperature and water was added. The product was extracted with ethyl acetate. The combined organic extracts were combined and concentrated under vacuum. The residue was purified by silica gel column (hexane/ ethyl acetate = 1:3) to give the methyl 5-((6-(5-(bis(*tert*-butoxycarbonyl)amino)-4-methylpyridin-3-yl)-8-((*tert-*butoxycarbonyl)amino)-7-fluoroisoquinolin-3-yl)amino)-5-oxopentanoate as a yellow solid (442 mg, 62%). ESI m/z =712.3 [M + H⁺].

### Step 3 and 4: synthesis of intermediate 12

To a solution of methyl 5-((6-(5-(bis(*tert*-butoxycarbonyl)amino)-4-methylpyridin-3-yl)-8-((*tert-*butoxycarbonyl)amino)-7-fluoroisoquinolin-3-yl)amino)-5-oxopentanoate (442 mg, 0.62 mmol, 1 equiv) in THF (10 mL) was added TFA (5 mL) and stirred at room temperature for 1 h. Concentrated under vacuum to remove all of the solvents and afforded yellow oil. ESI m/z =412.2 [M + H⁺]. Dissolved the obtained solid in THF/H₂O (4:2 mL). To the solution was added NaOH (124 mg, 3.1 mmol, 5 equiv). Stirred at room temperature for 2 h and concentrated under vacuum. The residue was purified preparative HPLC (10%-100% MeCN / 0.1% TFA in H₂O) to afford intermediate 12 as a yellow solid (302 mg, 78% for two steps) in TFA salt form. ¹H NMR (600 MHz, Methanol-*d*₄) δ 9.39 (s, 1H), 8.28 (s, 1H), 8.11 (s, 1H), 8.03 (s, 1H), 7.11 (d, *J* = 5.9 Hz, 1H), 2.60 (t, *J* = 7.5 Hz, 2H), 2.46 (t, *J* = 7.4 Hz, 2H), 2.26 (s, 3H), 2.04 (p, *J* = 7.4 Hz, 2H). ESI m/z =398.2 [M + H⁺].

### Example 115

### Synthesis of HC65-175

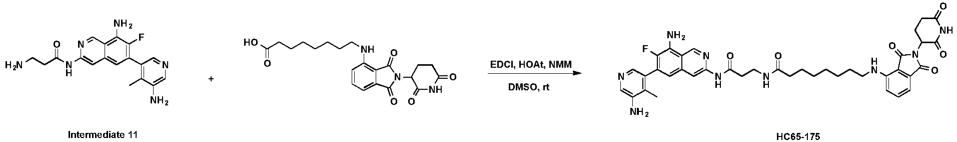

To a solution of Intermediate 11 (11 mg, 0.0158 mmol, 1.0 equiv) in DMSO (1 mL) were added 8-((2-(2,6-dioxopiperidin-3-yl)-1,3-dioxoisoindolin-4-yl)amino)octanoic acid (6.5 mg, 0.0158 mmol, 1.0 equiv), EDCI (1-ethyl-3-(3-dimethylaminopropyl)carbodiimide) (4.6 mg, 0.024 mmol, 1.5 equiv), HOAt (1-hydroxy-7-azabenzo-triazole) (3.2 mg, 0.024 mmol, 1.5 equiv), and NMM (N-Methylmorpholine) (8.0 mg, 0.079 mmol, 5.0 equiv). After being stirred overnight at room temperature, the resulting mixture was purified by preparative HPLC (10%-100% MeOH/ 0.1% TFA in H₂O) to afford HC65-175 as yellow solid in TFA salt form (7.6 mg, 49%). ¹H NMR (600 MHz, Methanol-*d*₄) δ 9.35 (s, 1H), 8.34 (s, 1H), 8.06 (s, 1H), 7.98 (s, 1H), 7.50 (t, *J* = 7.8 Hz, 1H), 7.05 (d, *J* = 5.8 Hz, 1H), 6.98 (d, *J* = 7.1 Hz, 1H), 6.91 (d, *J* = 8.6 Hz, 1H), 5.06 (dd, *J* = 12.7, 5.5 Hz, 1H), 3.63 - 3.54 (m, 2H), 3.14 (t, *J* = 7.1 Hz, 2H), 2.91 - 2.82 (m, 1H), 2.81 - 2.64 (m, 4H), 2.20 (s, 5H), 2.15-2.07 (m, 1H), 1.61 (t, *J* = 7.0 Hz, 2H), 1.53 (t, *J* = 6.9 Hz, 2H), 1.34-1.27 (m, 6H). HRMS calcd for C₃₉H₄₃FN₉O₆ [M + H⁺] 752.3320, found 752.3328.

### Example 116

### Synthesis of HC65-183

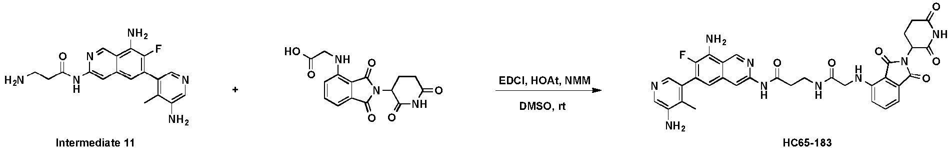

**HC65-183** was synthesized following the standard procedure for preparing HC65-175 from Intermediate 11 (9.1 mg, 0.013 mmol, 1.0 equiv), (2-(2,6-dioxopiperidin-3-yl)-1,3-dioxoisoindolin-4-yl)glycine (4.3 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC65-183 was obtained as yellow solid in TFA salt form (5.8 mg, 50%). ¹H NMR (600 MHz, Methanol-*d*₄) δ 9.34 (s, 1H), 8.09 (s, 1H), 8.06 (s, 1H), 8.03 (s, 1H), 7.36 - 7.24 (m, 1H), 6.99 (d, *J =* 5.7 Hz, 1H), 6.80 (d, *J =* 8.5 Hz, 1H), 6.69 (d, *J =* 19.9 Hz, 1H), 5.02 (dd, *J =* 12.9, 5.4 Hz, 1H), 4.00 (s, 2H), 3.71- 3.56 (m, 2H), 2.85 (ddd, *J* = 18.4, 13.9, 5.3 Hz, 1H), 2.79 - 2.63 (m, 4H), 2.27 (s, 3H), 2.12 - 2.04 (m, 1H). HRMS calcd for C₃₃H₃₁FN₉O₆ [M + H⁺] 668.2381, found 668.2388.

### Example 117

### Synthesis of HC65-184

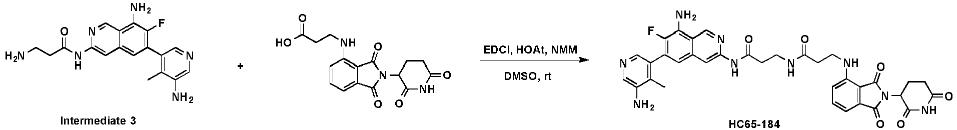

HC65-184 was synthesized following the standard procedure for preparing HC65-175 from Intermediate 11 (9.1 mg, 0.013 mmol, 1.0 equiv), 3-((2-(2,6-dioxopiperidin-3-yl)-1,3-dioxoisoindolin-4-yl)amino)propanoic acid (4.5 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC65-184 was obtained as yellow solid in TFA salt form (2.2 mg, 19%). ¹H NMR (600 MHz, Methanol-*d*₄) δ 9.30 (s, 1H), 8.31 (s, 1H), 8.09 (s, 0H), 8.04 (s, 1H), 7.52 (t, *J* = 7.8 Hz, 1H), 7.08 (d, *J* = 8.6 Hz, 1H), 7.02 (d, *J =* 5.9 Hz, 1H), 6.97 (d, *J* = 7.1 Hz, 1H), 5.07 (dd, *J =* 12.8, 5.4 Hz, 1H), 3.67 - 3.64 (m, 3H), 3.52 - 3.47 (m, 1H), 2.87 (ddd, *J* = 18.4, 13.8, 5.3 Hz, 1H), 2.80 - 2.67 (m, 1H), 2.71 - 2.68 (m, 3H), 2.62 - 2.49 (m, 2H), 2.26 (s, 3H), 2.13 - 2.07 (m, 1H). HRMS calcd for C₃₄H₃₃FN₉O₆ [M + H⁺] 682.2538, found 682.2551.

### Example 118

### Synthesis of HC65-185

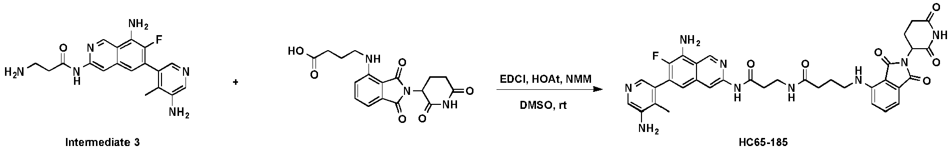

HC65-185 was synthesized following the standard procedure for preparing HC65-175 from Intermediate 11 (9.1 mg, 0.013 mmol, 1.0 equiv), 4-((2-(2,6-dioxopiperidin-3-yl)-1,3-dioxoisoindolin-4-yl)amino)butanoic acid (4.7 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC65-185 was obtained as yellow solid in TFA salt form (4.1 mg, 34%). ¹H NMR (600 MHz, Methanol-*d*₄) δ 9.30 (s, 1H), 8.24 (s, 1H), 8.09 (s, 1H), 8.01 (s, 1H), 7.40 (dd, *J =* 8.5, 7.1 Hz, 1H), 6.97 (d, *J* = 6.1 Hz, 1H), 6.95 (d, *J =* 8.6 Hz, 1H), 6.87 (d, *J* = 7.0 Hz, 1H), 5.04 (dd, *J =* 12.7, 5.4 Hz, 1H), 3.64 - 3.56 (m, 2H), 3.30 (t, *J =* 7.1 Hz, 2H), 2.85 (ddd, *J =* 17.2, 13.8, 5.2 Hz, 1H), 2.79 - 2.65 (m, 4H), 2.33 (t, *J* = 6.9 Hz, 2H), 2.24 (s, 3H), 2.14 - 2.06 (m, 1H), 1.95 (p, *J =* 7.0 Hz, 2H). HRMS calcd for C₃₅H₃₅FN₉O₆ [M + H⁺] 696.2694, found 696.2688.

### Example 119

### Synthesis of HC65-186

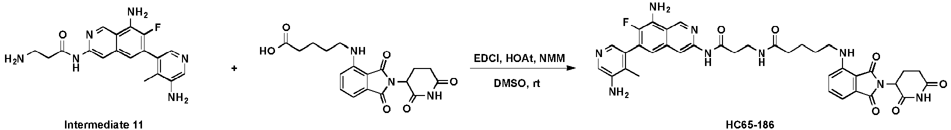

HC65-186 was synthesized following the standard procedure for preparing HC65-175 from Intermediate 11 (9.1 mg, 0.013 mmol, 1.0 equiv), 5-((2-(2,6-dioxopiperidin-3-yl)-1,3-dioxoisoindolin-4-yl)amino)pentanoic acid (4.9 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC65-186 was obtained as yellow solid in TFA salt form (5.5 mg, 45%). ¹H NMR (600 MHz, Methanol-*d*₄) δ 9.31 (s, 1H), 8.27 (s, 1H), 8.09 (s, 1H), 8.00 (s, 1H), 7.45 (t, *J* = 7.8 Hz, 1H), 7.00 (d, *J* = 5.8 Hz, 1H), 6.91 (d, *J* = 2.3 Hz, 1H), 6.90 (d, *J* = 3.7 Hz, 1H), 5.05 (dd, *J =* 12.7, 5.5 Hz, 1H), 3.61 (t, *J =* 6.5 Hz, 2H), 3.22 (t, *J =* 7.0 Hz, 2H), 2.86 (ddd, *J =* 18.3, 13.7, 5.3 Hz, 1H), 2.80 - 2.63 (m, 4H), 2.28 (t, *J =* 7.0 Hz, 2H), 2.22 (s, 3H), 2.17 - 2.04 (m, 1H), 1.74 (p, *J* = 7.0 Hz, 2H), 1.66 (p, *J* = 7.1 Hz, 2H). HRMS calcd for C₃₆H₃₇FN₉O₆ [M + H⁺] 710.2851, found 710.2867.

### Example 120

### Synthesis of HC75-1

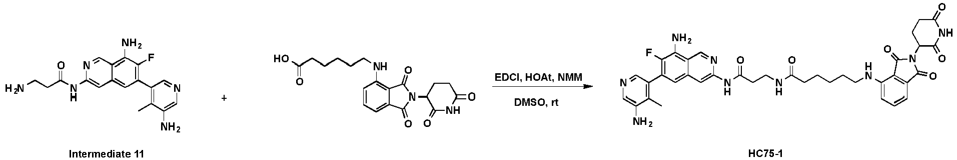

HC75-1 was synthesized following the standard procedure for preparing HC65-175 from Intermediate 11 (9.1 mg, 0.013 mmol, 1.0 equiv), 6-((2-(2,6-dioxopiperidin-3-yl)-1,3-dioxoisoindolin-4-yl)amino)hexanoic acid (5.1 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC75-1 was obtained as yellow solid in TFA salt form (7.1 mg, 51%). ¹H NMR (600 MHz, Methanol-*d*₄) δ 9.33 (s, 1H), 8.27 (s, 1H), 8.08 (s, 1H), 8.00 (s, 1H), 7.45 (t, *J =* 7.8 Hz, 1H), 7.03 (d, *J =* 5.8 Hz, 1H), 6.93 (d, *J =* 7.1 Hz, 1H), 6.87 (d, *J =* 8.5 Hz, 1H), 5.05 (dd, *J =* 12.8, 5.4 Hz, 1H), 3.60 (t, *J =* 6.4 Hz, 2H), 3.17 (t, *J* = 7.0 Hz, 2H), 2.85 (ddd, *J =* 18.6, 14.0, 5.3 Hz, 1H), 2.72 (ddt, *J* = 37.1, 13.5, 6.4 Hz, 4H), 2.24 (t, *J =* 7.1 Hz, 2H), 2.21 (s, 3H), 2.15 - 2.06 (m, 1H), 1.68 (p, *J =* 7.2 Hz, 2H), 1.61 (p, *J =* 7.2 Hz, 2H), 1.45 - 1.37 (m, 2H). HRMS calcd for C₃₇H₃₉FN₉O₆ [M + H⁺] 724.3007, found 724.3016.

### Example 121

### Synthesis of HC75-2

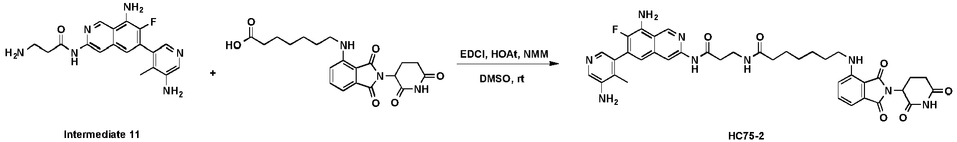

HC75-2 was synthesized following the standard procedure for preparing HC65-175 from Intermediate 11 (9.1 mg, 0.013 mmol, 1.0 equiv), 7-((2-(2,6-dioxopiperidin-3-yl)-1,3-dioxoisoindolin-4-yl)amino)heptanoic acid (5.2 mg, 0.013 mmol, 1.0 equiv), EDCI (3.8 mg, 0.02 mmol, 1.5 equiv), HOAt (2.7 mg, 0.02 mmol, 1.5 equiv), and NMM (6.6 mg, 0.065 mmol, 5.0 equiv) in DMSO (1 mL). HC75-2 was obtained as yellow solid in TFA salt form (4.5 mg, 36%). ¹H NMR (600 MHz, Methanol-*d*₄) δ 9.32 (s, 1H), 8.34 (s, 1H), 8.07 (s, 1H), 7.99 (s, 1H), 7.47 (dd, *J =* 8.5, 7.0 Hz, 1H), 7.03 (d, *J* = 5.8 Hz, 1H), 6.96 (d, *J =* 7.0 Hz, 1H), 6.89 (d, *J* = 8.5 Hz, 1H), 5.06 (dd, *J =* 12.7, 5.4 Hz, 1H), 3.60 (t, *J =* 6.4 Hz, 2H), 3.17 (t, *J =* 7.0 Hz, 2H), 2.87 (ddd, *J =* 17.0, 13.8, 5.2 Hz, 1H), 2.80 - 2.66 (m, 4H), 2.25 - 2.18 (m, 5H), 2.16 - 2.06 (m, 1H), 1.68 - 1.59 (m, 2H), 1.59 - 1.52 (m, 2H), 1.38 (h, *J =* 5.4, 4.5 Hz, 4H). HRMS calcd for C₃₈H₄₁FN₉O₆ [M + H⁺] 738.3164, found 738.3172.

### Example 122

### Synthesis of HC75-3

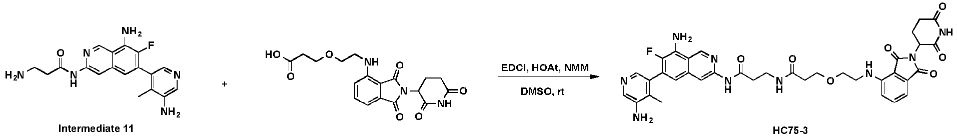

HC75-3 was synthesized following the standard procedure for preparing HC65-175 from Intermediate 11 (11.0 mg, 0.015 mmol, 1.0 equiv), 3-(2-((2-(2,6-dioxopiperidin-3-yl)-1,3-dioxoisoindolin-4-yl)amino)ethoxy)propanoic acid (6.0 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-3 was obtained as yellow solid in TFA salt form (3.6 mg, 25%). ¹H NMR (600 MHz, Methanol-d4) δ 9.24 (s, 1H), 8.26 (s, 1H), 8.09 (s, 1H), 8.04 (s, 1H), 7.38 (t, J = 7.9 Hz, 1H), 6.99 (s, 1H), 6.90 (d, J = 8.6 Hz, 1H), 6.80 (s, 1H), 5.07 (dd, J = 12.8, 5.5 Hz, 1H), 3.85 - 3.74 (m, 2H), 3.74 - 3.56 (m, 4H), 3.48 - 3.40 (m, 2H), 2.86 (ddd, J = 17.5, 14.0, 5.4 Hz, 1H), 2.80 - 2.63 (m, 4H), 2.49 (s, 2H), 2.23 (s, 3H), 2.19 - 2.05 (m, 1H). HRMS calcd for C₃₆H₃₇FN₉O₇ [M + H⁺] 726.2800, found 726.2810.

### Example 123

### Synthesis of HC75-4

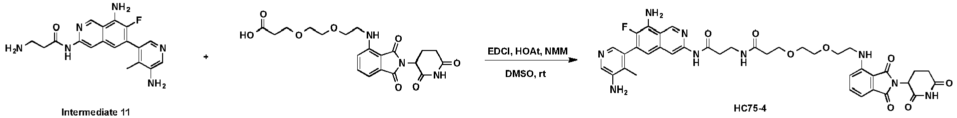

HC75-4 was synthesized following the standard procedure for preparing HC65-175 from Intermediate 11 (11.0 mg, 0.015 mmol, 1.0 equiv), 3-(2-(2-((2-(2,6-dioxopiperidin-3-yl)-1,3-dioxoisoindolin-4-yl)amino)ethoxy)ethoxy)propanoic acid (6.5 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-4 was obtained as yellow solid in TFA salt form (10.1 mg, 67%). ¹H NMR (600 MHz, Methanol-*d*₄) δ 9.31 (s, 1H), 8.15 (s, 1H), 8.10 (s, 1H), 8.04 (s, 1H), 7.41 (t, *J =* 7.9 Hz, 1H), 7.07 - 7.02 (m, 1H), 6.93 (d, *J =* 8.5 Hz, 1H), 6.88 - 6.75 (m, 1H), 5.05 (dd, *J =* 12.7, 5.5 Hz, 1H), 3.78 - 3.68 (m, 4H), 3.68 - 3.57 (m, 6H), 3.42 (t, *J =* 5.4 Hz, 2H), 2.87 (ddd, *J* = 17.5, 13.8, 5.3 Hz, 1H), 2.80 - 2.69 (m, 4H), 2.54 - 2.41 (m, 2H), 2.23 (s, 3H), 2.18 - 2.09 (m, 1H). HRMS calcd for C₃₈H₄₁FN₉O₈ [M + H⁺] 770.3062, found 770.3073.

### Example 124

### Synthesis of HC75-5

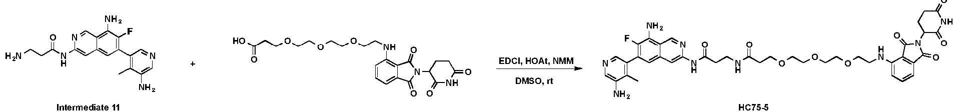

HC75-5 was synthesized following the standard procedure for preparing HC65-175 from Intermediate 11 (11.0 mg, 0.015 mmol, 1.0 equiv), 3-(2-(2-(2-((2-(2,6-dioxopiperidin-3-yl)-1,3-dioxoisoindolin-4-yl)amino)ethoxy)ethoxy)ethoxy)propanoic acid (7.2 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-5 was obtained as yellow solid in TFA salt form (10.5 mg, 67%). ¹H NMR (600 MHz, Methanol-*d*₄) δ 9.34 (s, 1H), 8.16 (s, 1H), 8.09 (s, 1H), 8.03 (s, 1H), 7.44 (t, *J* = 7.7 Hz, 1H), 7.11 - 7.04 (m, 1H), 6.97 (d, *J* = 8.6 Hz, 1H), 6.92 - 6.84 (m, 1H), 5.05 (dd, *J =* 12.8, 5.5 Hz, 1H), 3.76 - 3.64 (m, 8H), 3.64 - 3.55 (m, 6H), 3.45 (q, *J =* 4.8 Hz, 2H), 2.87 (ddd, *J =* 17.5, 13.8, 5.4 Hz, 1H), 2.80 - 2.66 (m, 4H), 2.44 (t, *J =* 5.9 Hz, 2H), 2.23 (s, 3H), 2.17 - 2.08 (m, 1H). HRMS calcd for C₄₀H₄₅FN₉O₉ [M + H⁺] 814.3324, found 814.3318.

### Example 125

### Synthesis of HC75-6

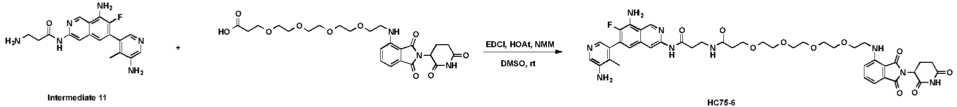

HC75-6 was synthesized following the standard procedure for preparing HC65-175 from Intermediate 11 (11.0 mg, 0.015 mmol, 1.0 equiv), 1-((2-(2,6-dioxopiperidin-3-yl)-1,3-dioxoisoindolin-4-yl)amino)-3,6,9,12-tetraoxapentadecan-15-oic acid (7.8 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-6 was obtained as yellow solid in TFA salt form (9.3 mg, 57%). ¹H NMR (600 MHz, Methanol-*d*₄) δ 9.35 (s, 1H), 8.19 (s, 1H), 8.09 (s, 1H), 8.03 (s, 1H), 7.46 (t, *J* = 7.8 Hz, 1H), 7.08 (d, *J* = 5.7 Hz, 1H), 6.98 (d, *J* = 8.5 Hz, 1H), 6.92 (d, *J* = 7.0 Hz, 1H), 5.05 (dd, *J =* 12.8, 5.5 Hz, 1H), 3.71 (q, *J =* 5.7 Hz, 4H), 3.67 (s, 4H), 3.65 - 3.57 (m, 6H), 3.55 (s, 4H), 3.49 - 3.40 (m, 2H), 2.87 (ddd, *J =* 18.4, 13.8, 5.3 Hz, 1H), 2.80 - 2.65 (m, 4H), 2.45 (t, *J =* 5.9 Hz, 2H), 2.23 (s, 3H), 2.16 - 2.07 (m, 1H). HRMS calcd for C₄₂H₄₉FN₉O₁₀ [M + H⁺] 858.3586, found 858.3597.

### Example 126

### Synthesis of HC75-7

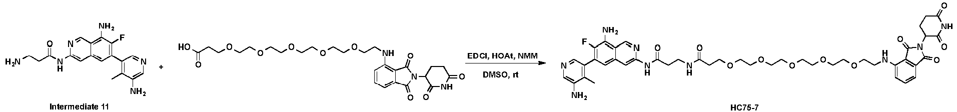

HC75-7 was synthesized following the standard procedure for preparing HC65-175 from Intermediate 11 (11.0 mg, 0.015 mmol, 1.0 equiv), 1-((2-(2,6-dioxopiperidin-3-yl)-1,3-dioxoisoindolin-4-yl)amino)-3,6,9,12,15-pentaoxaoctadecan-18-oic acid (8.5 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-7 was obtained as yellow solid in TFA salt form (11.0 mg, 65%). ¹H NMR (600 MHz, Methanol-*d*₄) δ 9.35 (s, 1H), 8.26 (s, 1H), 8.09 (s, 1H), 8.03 (s, 1H), 7.49 (t, *J* = 7.6 Hz, 1H), 7.07 (d, *J* = 5.8 Hz, 1H), 7.00 (d, *J* = 8.6 Hz, 1H), 6.96 (d, *J* = 7.0 Hz, 1H), 5.06 (dd, *J =* 12.7, 5.5 Hz, 1H), 3.71 (td, *J =* 6.6, 5.3, 2.8 Hz, 4H), 3.68 - 3.59 (m, 9H), 3.56 (ddt, *J =* 9.9, 6.2, 3.6 Hz, 9H), 3.44 (t, *J =* 5.1 Hz, 2H), 2.87 (ddd, *J =* 18.3, 13.9, 5.3 Hz, 1H), 2.79 - 2.66 (m, 4H), 2.51 - 2.41 (m, 2H), 2.23 (s, 3H), 2.15 - 2.09 (m, 1H). HRMS calcd for C₄₄H₅₃FN₉O₁₁ [M + H⁺] 902.3849, found 902.3860.

### Example 127

### Synthesis of HC75-8

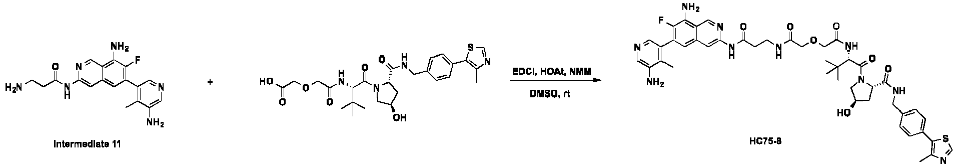

HC75-8 was synthesized following the standard procedure for preparing HC65-175 from Intermediate 11 (11.0 mg, 0.015 mmol, 1.0 equiv), 2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)acetic acid (8.2 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-8 was obtained as yellow solid in TFA salt form (11.2 mg, 67%). ¹H NMR (600 MHz, Methanol-*d*₄) δ 9.37 (s, 1H), 8.98 (s, 1H), 8.22 (s, 1H), 8.10 (s, 1H), 7.99 (s, 1H), 7.45 (d, *J* = 8.3 Hz, 2H), 7.38 (d, *J =* 7.9 Hz, 2H), 7.02 (d, *J =* 5.8 Hz, 1H), 4.72 (s, 1H), 4.66 - 4.56 (m, 2H), 4.53 (d, *J =* 4.0 Hz, 1H), 4.32 (d, *J =* 15.6 Hz, 1H), 4.21 - 4.11 (m, 2H), 4.10 (s, 2H), 3.92 (d, *J =* 11.0 Hz, 1H), 3.82 (dd, *J =* 11.0, 3.8 Hz, 1H), 3.74 - 3.62 (m, 2H), 2.84 - 2.74 (m, 2H), 2.46 (s, 3H), 2.33 - 2.26 (m, 1H), 2.23 (s, 3H), 2.11 (ddd, *J=* 13.4, 9.5, 4.4 Hz, 1H), 1.03 (s, 9H). HRMS calcd for C₄₄H₅₂FN₁₀O₇S [M + H⁺] 883.3725, found 883.3733.

### Example 128

### Synthesis of HC75-9

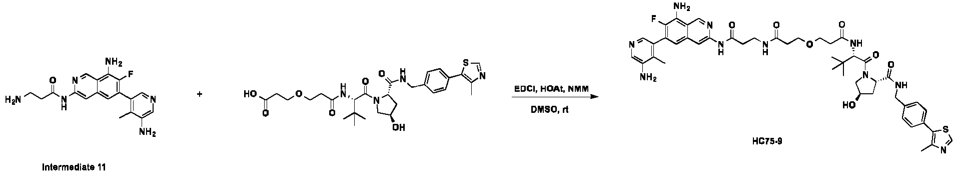

HC75-9 was synthesized following the standard procedure for preparing HC65-175 from Intermediate 11 (11.0 mg, 0.015 mmol, 1.0 equiv), 3-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)propanoic acid (8.6 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-9 was obtained as yellow solid in TFA salt form (8.9 mg, 52%). ¹H NMR (600 MHz, Methanol-*d*₄) δ 9.46 (s, 1H), 9.13 (s, 1H), 8.14 (s, 1H), 8.11 (s, 1H), 8.02 (s, 1H), 7.47 (d, *J =* 7.7 Hz, 2H), 7.39 (d, *J =* 8.0 Hz, 2H), 7.09 (d, *J =* 5.7 Hz, 1H), 4.68 (d, *J =* 3.6 Hz, 1H), 4.59 (d, *J =* 15.6 Hz, 1H), 4.54 (s, 1H), 4.34 (d, *J =* 15.5 Hz, 1H), 3.95 (d, *J =* 11.1 Hz, 1H), 3.83 (dd, *J =* 11.0, 3.9 Hz, 1H), 3.74 - 3.66 (m, 5H), 3.60 (t, *J* = 6.4 Hz, 2H), 2.83 - 2.70 (m, 2H), 2.56 - 2.41 (m, 7H), 2.33 - 2.22 (m, 4H), 2.16 - 2.06 (m, 1H), 1.05 (s, 9H). HRMS calcd for C₄₆H₅₆FN₁₀O₇S [M + H⁺] 911.4038, found 911.4049.

### Example 129

### Synthesis of HC75-10

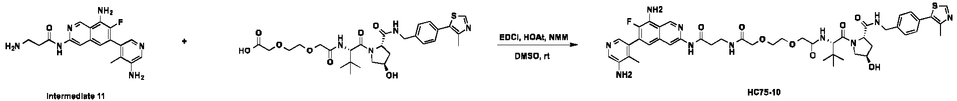

HC75-10 was synthesized following the standard procedure for preparing HC65-175 from Intermediate 11 (11.0 mg, 0.015 mmol, 1.0 equiv), 2-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)acetic acid (8.9 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-10 was obtained as yellow solid in TFA salt form (10.8 mg, 62%). ¹H NMR (600 MHz, Methanol-*d*₄) δ 9.44 (s, 1H), 9.14 (s, 1H), 8.17 (s, 1H), 8.11 (s, 1H), 8.03 (s, 1H), 7.46 (d, *J* = 8.4 Hz, 2H), 7.41 (d, *J =* 8.0 Hz, 2H), 7.11 (d, *J* = 5.7 Hz, 1H), 4.72 (s, 1H), 4.58 (d, *J =* 22.0 Hz, 1H), 4.53 (d, *J =* 8.1 Hz, 2H), 4.40 (d, *J =* 15.4 Hz, 1H), 4.12 - 4.05 (m, 2H), 4.00 (d, *J* = 15.5 Hz, 1H), 3.88 (d, *J =* 11.4 Hz, 1H), 3.82 (dd, *J =* 11.0, 3.9 Hz, 1H), 3.79 - 3.60 (m, 7H), 2.84 - 2.70 (m, 2H), 2.49 (s, 3H), 2.30 - 2.20 (m, 4H), 2.11 (ddd, *J =* 13.3, 9.2, 4.5 Hz, 1H), 1.04 (s, 9H). HRMS calcd for C₄₆H₅₆FN₁₀O₈S [M + H⁺] 927.3987, found 927.3978.

### Example 130

### Synthesis of HC75-11

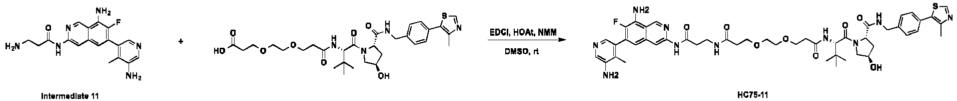

HC75-11 was synthesized following the standard procedure for preparing HC65-175 from Intermediate 11 (11.0 mg, 0.015 mmol, 1.0 equiv), 3-(2-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)ethoxy)propanoic acid (9.3 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-11 was obtained as yellow solid in TFA salt form (9.9 mg, 56%). ¹H NMR (600 MHz, Methanol-*d*₄) δ 9.42 (s, 1H), 9.04 (s, 1H), 8.26 (s, 1H), 8.10 (s, 1H), 8.01 (s, 1H), 7.48 (d, *J =* 7.9 Hz, 2H), 7.40 (d, *J =* 7.9 Hz, 2H), 7.08 (d, *J =* 5.8 Hz, 1H), 4.71 (s, 1H), 4.63 (t, *J* = 8.4 Hz, 1H), 4.57 (d, *J* = 15.5 Hz, 1H), 4.55 - 4.51 (m, 1H), 4.38 (d, *J =* 15.5 Hz, 1H), 3.93 (d, *J =* 11.0 Hz, 1H), 3.83 (dd, *J =* 11.0, 3.9 Hz, 1H), 3.77 - 3.65 (m, 4H), 3.62 - 3.52 (m, 6H), 2.79 (dt, *J =* 15.6, 6.7 Hz, 1H), 2.67 (d, *J =* 15.8 Hz, 1H), 2.56 - 2.38 (m, 7H), 2.30 - 2.22 (m, 4H), 2.11 (ddd, *J =* 13.3, 9.2, 4.5 Hz, 1H), 1.05 (s, 9H). HRMS calcd for C₄₈H₆₀FN₁₀O₈S [M + H⁺] 955.4300, found 955.4313.

### Example 131

### Synthesis of HC75-12

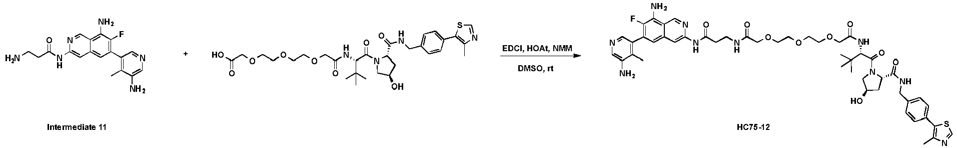

HC75-12 was synthesized following the standard procedure for preparing HC65-175 from Intermediate 11 (11.0 mg, 0.015 mmol, 1.0 equiv), (*S*)-13-((2*S*,4*R*)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecanoic acid (9.5 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-12 was obtained as yellow solid in TFA salt form (10.5 mg, 58%). ¹H NMR (600 MHz, Methanol-*d*₄) δ 9.40 (s, 1H), 9.02 (s, 1H), 8.27 (s, 1H), 8.10 (s, 1H), 8.02 (s, 1H), 7.47 (d, *J* = 7.8 Hz, 2H), 7.40 (d, *J* = 8.2 Hz, 2H), 7.07 (d, *J* = 5.8 Hz, 1H), 4.71 (d, *J* = 3.6 Hz, 1H), 4.65 - 4.60 (m, 1H), 4.58 (d, *J=* 15.3 Hz, 1H), 4.53 (s, 1H), 4.35 (d, *J =* 15.5 Hz, 1H), 4.04 (d, *J =* 12.1 Hz, 1H), 3.98 (d, *J =* 11.4 Hz, 1H), 3.90 (d, *J =* 11.0 Hz, 1H), 3.82 (dd, *J =* 11.0, 3.8 Hz, 1H), 3.75 - 3.62 (m, 12H), 2.82 - 2.72 (m, 2H), 2.47 (s, 3H), 2.29 - 2.21 (m, 4H), 2.12 (ddd, *J =* 13.4, 9.4, 4.3 Hz, 1H), 1.04 (s, 9H). HRMS calcd for C₄₈H₆₀FN₁₀O₉S [M + H⁺] 971.4249, found 971.4262.

### Example 132

### Synthesis of HC75-13

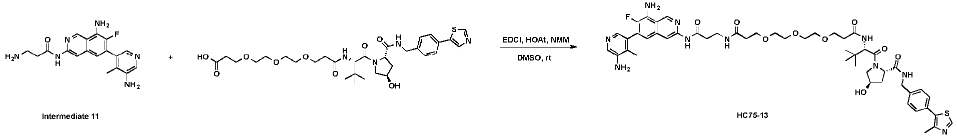

HC75-13 was synthesized following the standard procedure for preparing HC65-175 from Intermediate 11 (11.0 mg, 0.015 mmol, 1.0 equiv), (*S*)-15-((2*S*,4*R*)-4-hydroxy-2-((4-(4-methylthiazol-5-yl)benzyl)carbamoyl)pyrrolidine-1-carbonyl)-16,16-dimethyl-13-oxo-4,7,10-trioxa-14-azaheptadecanoic acid (9.9 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-13 was obtained as yellow solid in TFA salt form (8.4 mg, 46%). ¹H NMR (600 MHz, Methanol-*d*₄) δ 9.44 (s, 1H), 9.08 (s, 1H), 8.22 (s, 1H), 8.11 (s, 1H), 8.01 (s, 1H), 7.48 (d, *J* = 7.9 Hz, 2H), 7.40 (d, *J* = 8.3 Hz, 2H), 7.09 (d, *J* = 5.8 Hz, 1H), 4.69 (s, 1H), 4.63 - 4.60 (m, 1H), 4.58 (d, *J* = 15.7 Hz, 1H), 4.54 - 4.50 (m, 1H), 4.37 (d, *J =* 15.5 Hz, 1H), 3.92 (d, *J* = 11.0 Hz, 1H), 3.83 (dd, *J =* 11.0, 3.8 Hz, 1H), 3.75 - 3.67 (m, 4H), 3.62 - 3.54 (m, 10H), 2.75 (t, *J =* 6.7 Hz, 2H), 2.62 - 2.53 (m, 1H), 2.52 - 2.43 (m, 6H), 2.30 - 2.22 (m, 4H), 2.10 (ddd, *J =* 13.3, 9.3, 4.4 Hz, 1H), 1.05 (s, 9H). HRMS calcd for C₅₀H₆₄FN₁₀O₉S [M + H⁺] 999.4562, found 999.4569.

### Example 133

### Synthesis of HC75-14

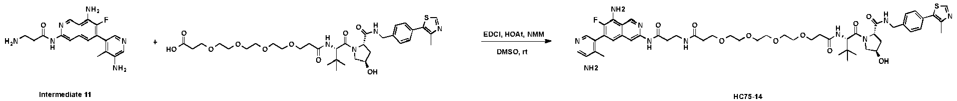

HC75-14 was synthesized following the standard procedure for preparing HC65-175 from Intermediate 11 (11.0 mg, 0.015 mmol, 1.0 equiv), (*S*)-18-((2*S*,4*R*)-4-hydroxy-2-((4-(4-methylthiazol-5-yl)benzyl)carbamoyl)pyrrolidine-1-carbonyl)-19,19-dimethyl-16-oxo-4,7,10,13-tetraoxa-17-azaicosanoic acid (11.0 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-14 was obtained as yellow solid in TFA salt form (6.4 mg, 34%). ¹H NMR (600 MHz, Methanol-*d*₄) δ 9.41 (s, 1H), 9.00 (s, 1H), 8.29 (s, 1H), 8.10 (s, 1H), 8.01 (s, 1H), 7.48 (d, *J =* 7.9 Hz, 2H), 7.41 (d, *J =* 8.2 Hz, 2H), 7.07 (d, *J =* 5.9 Hz, 1H), 4.68 (s, 1H), 4.61 (d, *J =* 7.5 Hz, 1H), 4.57 (d, *J* = 16.0 Hz, 1H), 4.54 - 4.51 (m, 1H), 4.37 (d, *J =* 15.4 Hz, 1H), 3.91 (d, *J* = 11.0 Hz, 1H), 3.82 (dd, *J =* 11.0, 3.8 Hz, 1H), 3.71 (td, *J =* 5.9, 3.2 Hz, 4H), 3.63 - 3.55 (m, 14H), 2.74 (t, *J* = 6.6 Hz, 2H), 2.57 (ddd, *J =* 14.9, 7.2, 5.4 Hz, 1H), 2.51 - 2.43 (m, 6H), 2.30 - 2.22 (m, 4H), 2.10 (ddd, *J =* 13.4, 9.2, 4.5 Hz, 1H), 1.05 (s, 9H). HRMS calcd for C₅₂H₆₈FN₁₀O₁₀S [M + H⁺] 1043.4825, found 1043.4839.

### Example 134

### Synthesis of HC75-15

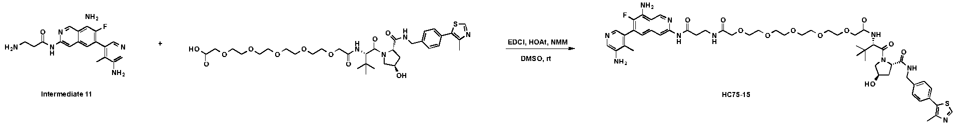

HC75-15 was synthesized following the standard procedure for preparing HC65-175 from Intermediate 11 (11.0 mg, 0.015 mmol, 1.0 equiv), (*S*)-19-((2*S*,4*R*)-4-hydroxy-2-((4-(4-methylthiazol-5-yl)benzyl)carbamoyl)pyrrolidine-1-carbonyl)-20,20-dimethyl-17-oxo-3,6,9,12,15-pentaoxa-18-azahenicosanoic acid (11 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-15 was obtained as yellow solid in TFA salt form (9.8 mg, 51%). ¹H NMR (600 MHz, Methanol-*d*₄) δ 9.46 (s, 1H), 9.15 (s, 1H), 8.19 (s, 1H), 8.12 (s, 1H), 8.01 (s, 1H), 7.49 (d, *J* = 8.0 Hz, 2H), 7.43 (d, *J =* 7.9 Hz, 2H), 7.11 (d, *J* = 5.7 Hz, 1H), 4.71 (s, 1H), 4.61 (t, *J* = 8.4 Hz, 1H), 4.57 (d, *J =* 15.5 Hz, 1H), 4.54 - 4.51 (m, 1H), 4.38 (d, *J =* 15.5 Hz, 1H), 4.06 (d, *J* = 15.6 Hz, 1H), 4.02 (d, *J* = 16.7 Hz, 1H), 3.91 (d, *J* = 11.0 Hz, 1H), 3.82 (dd, *J =* 11.0, 3.8 Hz, 1H), 3.70 - 3.60 (m, 20H), 2.79 (t, *J* = 6.5 Hz, 2H), 2.50 (s, 3H), 2.29 - 2.21 (m, 4H), 2.11 (ddd, *J* = 13.4, 9.4, 4.4 Hz, 1H), 1.05 (s, 9H). HRMS calcd for C₅₂H₆₈FN₁₀O₁₁S [M + H⁺] 1059.4774, found 1059.4788.

### Example 135

### Synthesis of HC75-16

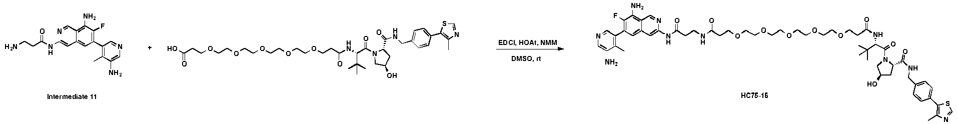

HC75-16 was synthesized following the standard procedure for preparing HC65-175 from Intermediate 11 (11.0 mg, 0.015 mmol, 1.0 equiv), (*S*)-21-((2*S*,4*R*)-4-hydroxy-2-((4-(4-methylthiazol-5-yl)benzyl)carbamoyl)pyrrolidine-1-carbonyl)-22,22-dimethyl-19-oxo-4,7,10,13,16-pentaoxa-20-azatricosanoic acid (11.3 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-16 was obtained as yellow solid in TFA salt form (8.9 mg, 45%). ¹H NMR (600 MHz, Methanol-*d*₄) δ 9.44 (s, 1H), 9.06 (s, 1H), 8.25 (s, 1H), 8.11 (s, 1H), 8.02 (s, 1H), 7.49 (d, *J =* 7.9 Hz, 2H), 7.42 (d, *J =* 7.9 Hz, 2H), 7.10 (d, *J =* 5.8 Hz, 1H), 4.68 (s, 1H), 4.60 (dd, *J* = 9.4, 7.7 Hz, 1H), 4.57 (d, *J =* 15.5 Hz, 1H), 4.54 - 4.50 (m, 1H), 4.38 (d,*J* = 15.6 Hz, 1H), 3.92 (d, *J* = 11.0 Hz, 1H), 3.82 (dd, *J =* 11.0, 3.9 Hz, 1H), 3.76 - 3.67 (m, 4H), 3.62 - 3.55 (m, 18H), 2.75 (t, *J =* 6.5 Hz, 2H), 2.57 (ddd, *J =* 15.0, 7.6, 5.2 Hz, 1H), 2.52 - 2.43 (m, 6H), 2.25 (s, 4H), 2.10 (ddd, *J* = 13.4, 9.3, 4.5 Hz, 1H), 1.05 (s, 9H). HRMS calcd for C₅₄H₇₂FN₁₀O₁₁S [M + H⁺] 1087.5087, found 1087.5096.

### Example 136

### Synthesis of HC75-17

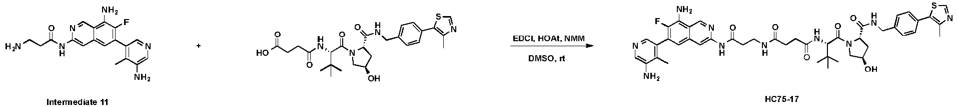

HC75-17 was synthesized following the standard procedure for preparing HC65-175 from Intermediate 11 (11.0 mg, 0.015 mmol, 1.0 equiv), 4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutanoic acid (8.0 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-17 was obtained as yellow solid in TFA salt form (9.6 mg, 58%). ¹H NMR (600 MHz, Methanol-*d*₄) δ 9.42 (s, 1H), 9.04 (s, 1H), 8.25 (s, 1H), 8.11 (s, 1H), 8.02 (s, 1H), 7.48 (d, *J* = 8.0 Hz, 2H), 7.40 (d, *J =* 8.0 Hz, 2H), 7.08 (d, *J =* 5.8 Hz, 1H), 4.65 - 4.54 (m, 3H), 4.53 - 4.49 (m, 1H), 4.36 (d, *J* = 15.5 Hz, 1H), 3.93 (d, *J* = 11.0 Hz, 1H), 3.80 (dd, *J* = 11.0, 3.9 Hz, 1H), 3.61 (dt, *J =* 13.5, 6.5 Hz, 1H), 3.54 (dt, *J =* 13.4, 6.5 Hz, 1H), 2.76 - 2.70 (m, 2H), 2.63 - 2.55 (m, 2H), 2.52 - 2.46 (m, 5H), 2.24 (d, *J* = 5.1 Hz, 4H), 2.10 (ddd, *J* = 13.4, 9.3, 4.4 Hz, 1H), 1.05 (s, 9H). HRMS calcd for C₄₄H₅₂FN₁₀O₆S [M + H⁺] 867.3776, found 867.3789.

### Example 137

### Synthesis of HC75-18

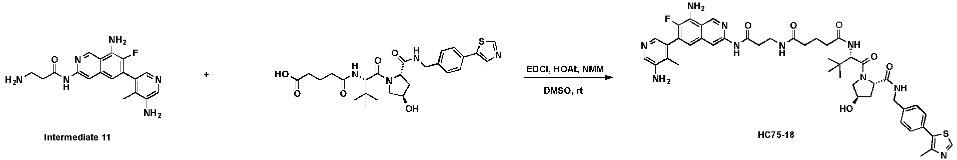

HC75-18 was synthesized following the standard procedure for preparing HC65-175 from Intermediate 11 (11.0 mg, 0.015 mmol, 1.0 equiv), 5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentanoic acid (8.2 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-18 was obtained as yellow solid in TFA salt form (9.1 mg, 55%). ¹H NMR (600 MHz, Methanol-*d*₄) δ 9.45 (s, 1H), 9.10 (s, 1H), 8.17 (s, 1H), 8.11 (s, 0H), 8.01 (s, 1H), 7.47 (d, *J* = 7.9 Hz, 2H), 7.38 (d, *J* = 7.9 Hz, 1H), 7.09 (d, *J* = 5.8 Hz, 1H), 4.66 (s, 1H), 4.64 - 4.56 (m, 2H), 4.55 - 4.51 (m, 1H), 4.36 (d, *J =* 15.5 Hz, 1H), 3.95 (d, *J* = 11.0 Hz, 1H), 3.83 (dd, *J =* 11.0, 3.8 Hz, 1H), 3.68 - 3.61 (m, 1H), 3.59 - 3.51 (m, 1H), 2.79 - 2.69 (m, 2H), 2.47 (s, 3H), 2.37 - 2.20 (m, 8H), 2.11 (ddd, *J* = 13.4, 9.4, 4.4 Hz, 1H), 1.91 (p, *J =* 7.3 Hz, 2H), 1.05 (s, 9H). HRMS calcd for C₄₅H₅₄FN₁₀O₆S [M + H⁺] 881.3933, found 881.3949.

### Example 138

### Synthesis of HC75-19

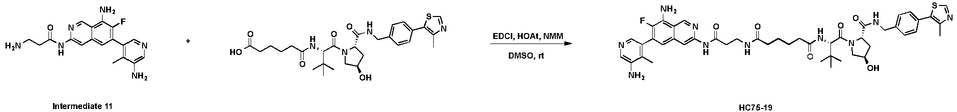

HC75-19 was synthesized following the standard procedure for preparing HC65-175 from Intermediate 11 (11.0 mg, 0.015 mmol, 1.0 equiv), 6-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-6-oxohexanoic acid (8.4 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-19 was obtained as yellow solid in TFA salt form (8.1 mg, 48%). ¹H NMR (600 MHz, Methanol-*d*₄) δ 9.43 (s, 1H), 9.01 (s, 1H), 8.26 (s, 1H), 8.11 (s, 1H), 8.03 (s, 1H), 7.48 (d, *J =* 7.9 Hz, 2H), 7.38 (d, *J =* 8.0 Hz, 2H), 7.06 (d, *J =* 5.6 Hz, 1H), 4.71 - 4.62 (m, 2H), 4.59 (d, *J =* 15.4 Hz, 1H), 4.54 - 4.50 (m, 1H), 4.34 (d, *J* = 15.5 Hz, 1H), 3.95 (d, *J* = 11.0 Hz, 1H), 3.84 (dd, *J* = 11.0, 3.9 Hz, 1H), 3.62 - 3.52 (m, 2H), 2.82 (dt, *J =* 14.3, 6.9 Hz, 1H), 2.60 (d, *J =* 14.4 Hz, 1H), 2.46 (s, 3H), 2.36 - 2.16 (m, 8H), 2.11 (ddd, *J =* 13.3, 9.3, 4.5 Hz, 1H), 1.69 - 1.54 (m, 4H), 1.06 (s, 9H). HRMS calcd for C₄₆H₅₆FN₁₀O₆S [M + H⁺] 895.4089, found 895.4096.

### Example 139

### Synthesis of HC75-20

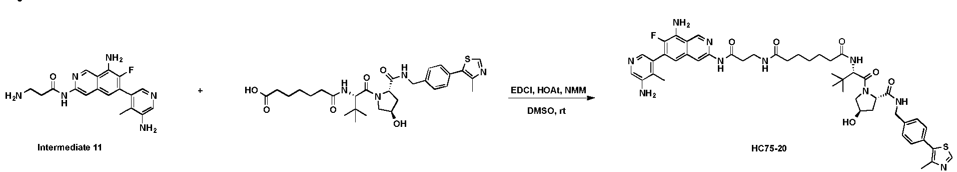

HC75-20 was synthesized following the standard procedure for preparing HC65-175 from Intermediate 11 (11.0 mg, 0.015 mmol, 1.0 equiv), 7-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-7-oxoheptanoic acid (8.6 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-20 was obtained as yellow solid in TFA salt form (9.5 mg, 56%). ¹H NMR (600 MHz, Methanol-*d*₄) δ 9.47 (s, 1H), 9.15 (s, 1H), 8.20 (s, 1H), 8.11 (s, 1H), 8.03 (s, 1H), 7.50 (d, *J* = 7.9 Hz, 2H), 7.42 (d, *J =* 8.2 Hz, 2H), 7.11 (d, *J* = 5.8 Hz, 1H), 4.67 (s, 1H), 4.62 (dd, *J =* 9.3, 7.4 Hz, 1H), 4.58 (d, *J* = 15.5 Hz, 1H), 4.54 - 4.50 (m, 1H), 4.37 (d, *J =* 15.5 Hz, 1H), 3.94 (d, *J* = 11.0 Hz, 1H), 3.83 (dd, *J=* 11.0, 3.9 Hz, 1H), 3.58 (t, *J =* 6.5 Hz, 2H), 2.85 - 2.74 (m, 1H), 2.71 - 2.64 (m, 1H), 2.49 (s, 3H), 2.34 - 2.16 (m, 8H), 2.11 (ddd, *J* = 13.3, 9.2, 4.5 Hz, 1H), 1.66-1.57 (m, 4H), 1.32 (p, *J* = 7.8 Hz, 2H), 1.05 (s, 9H). HRMS calcd for C₄₇H₅₈FN₁₀O₆S [M + H⁺] 909.4246, found 909.4258.

### Example 140

### Synthesis of HC75-21

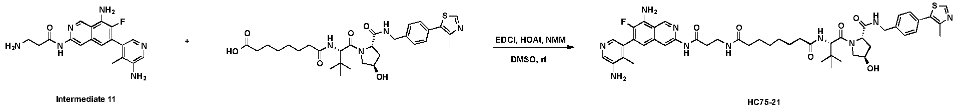

HC75-21 was synthesized following the standard procedure for preparing HC65-175 from Intermediate 11 (11.0 mg, 0.015 mmol, 1.0 equiv), 8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctanoic acid (8.8 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-21 was obtained as yellow solid in TFA salt form (9.4 mg, 54%). ¹H NMR (600 MHz, Methanol-*d*₄) δ 9.43 (s, 1H), 9.04 (s, 1H), 8.29 (s, 1H), 8.10 (s, 1H), 8.02 (s, 1H), 7.49 (d, *J* = 7.9 Hz, 2H), 7.42 (d, *J* = 8.0 Hz, 2H), 7.08 (d, *J* = 5.8 Hz, 1H), 4.69 (s, 1H), 4.65 - 4.61 (m, 1H), 4.57 (d, *J =* 15.5 Hz, 1H), 4.54 - 4.49 (m, 1H), 4.39 (d, *J* = 15.5 Hz, 1H), 3.94 (d, *J* = 10.9 Hz, 1H), 3.83 (dd, *J* = 10.9, 3.9 Hz, 1H), 3.56 (t, *J* = 6.4 Hz, 2H), 2.79 (dt, *J =* 14.1, 6.7 Hz, 1H), 2.66 - 2.59 (m, 1H), 2.48 (s, 3H), 2.33 - 2.15 (m, 8H), 2.10 (ddd, *J* = 13.4, 9.2, 4.5 Hz, 1H), 1.70 - 1.51 (m, 4H), 1.33 - 1.23 (m, 4H), 1.06 (s, 9H). HRMS calcd for C₄₈H₆₀FN₁₀O₆S [M + H⁺] 923.4402, found 923.4415.

### Example 141

### Synthesis of HC75-22

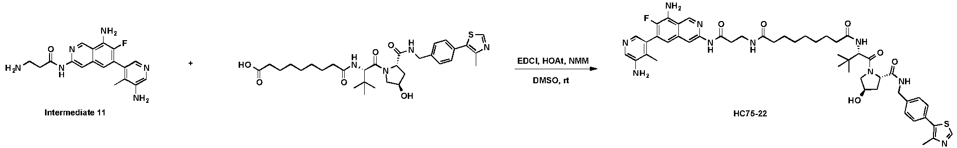

HC75-22 was synthesized following the standard procedure for preparing HC65-175 from Intermediate 11 (11.0 mg, 0.015 mmol, 1.0 equiv), 9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononanoic acid (9.0 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-22 was obtained as yellow solid in TFA salt form (7.8 mg, 45%). ¹H NMR (600 MHz, Methanol-*d*₄) δ 9.45 (s, 1H), 9.09 (s, 1H), 8.27 (s, 1H), 8.11 (s, 1H), 8.02 (s, 1H), 7.50 (d, *J =* 7.8 Hz, 2H), 7.45 - 7.42 (m, 2H), 7.10 (d, *J* = 5.2 Hz, 1H), 4.69 (s, 1H), 4.62 (t, *J* = 8.5 Hz, 1H), 4.58 (d, *J* = 15.9 Hz, 1H), 4.53 (s, 1H), 4.41 - 4.37 (m, 1H), 3.95 (d, *J* = 10.9 Hz, 1H), 3.83 (dd, *J* = 11.0, 3.6 Hz, 1H), 3.56 (q, *J =* 9.5, 6.9 Hz, 2H), 2.80 (dt, *J =* 14.6, 6.9 Hz, 1H), 2.65 - 2.58 (m, 1H), 2.49 (d, *J =* 3.0 Hz, 3H), 2.30 - 2.15 (m, 8H), 2.10 (ddt, *J =* 13.1, 8.3, 3.8 Hz, 1H), 1.64 - 1.48 (m, 4H), 1.34 - 1.19 (m, 6H), 1.06 (s, 9H). HRMS calcd for C₄₉H₆₂FN₁₀O₆S [M + H⁺] 937.4559, found 937.4567.

### Example 142

### Synthesis of HC75-23

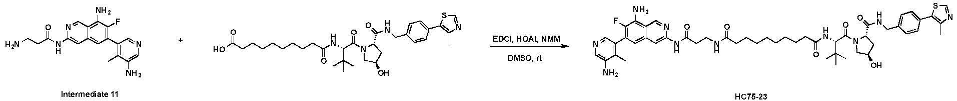

HC75-23 was synthesized following the standard procedure for preparing HC65-175 from Intermediate 11 (11.0 mg, 0.015 mmol, 1.0 equiv), 10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecanoic acid (9.2 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-23 was obtained as yellow solid in TFA salt form (8.0 mg, 45%). ¹H NMR (600 MHz, Methanol-*d*₄) δ 9.43 (s, 1H), 9.02 (s, 1H), 8.35 (s, 1H), 8.10 (s, 1H), 8.02 (s, 1H), 7.50 (d, *J* = 8.0 Hz, 2H), 7.43 (d, *J* = 8.0 Hz, 2H), 7.08 (d, *J* = 5.8 Hz, 1H), 4.74 (s, 1H), 4.65 - 4.57 (m, 2H), 4.56 - 4.52 (m, 1H), 4.39 (d, *J* = 15.4 Hz, 1H), 3.95 (d, *J =* 11.0 Hz, 1H), 3.85 (dd, *J =* 10.9, 3.9 Hz, 1H), 3.52 (t, *J =* 6.3 Hz, 2H), 2.84 (dt, *J =* 15.6, 6.8 Hz, 1H), 2.58 - 2.51 (m, 1H), 2.47 (s, 3H), 2.32 - 2.15 (m, 8H), 2.12 (ddd, *J =* 13.3, 9.0, 4.5 Hz, 1H), 1.71 - 1.44 (m, 4H), 1.31 - 1.15 (m, 8H), 1.08 (s, 9H). HRMS calcd for C₅₀H₆₄FN₁₀O₆S [M + H⁺] 951.4715, found 951.4730.

### Example 143

### Synthesis of HC75-24

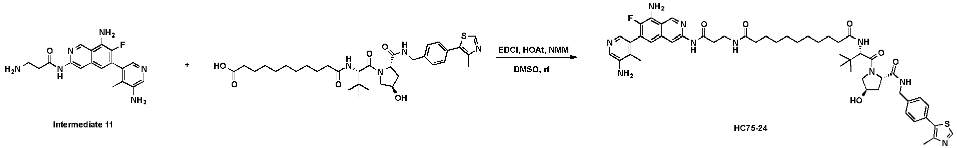

HC75-24 was synthesized following the standard procedure for preparing HC65-175 from Intermediate 11 (11.0 mg, 0.015 mmol, 1.0 equiv), 11-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecanoic acid (9.4 mg, 0.015 mmol, 1.0 equiv), EDCI (4.3 mg, 0.0225 mmol, 1.5 equiv), HOAt (3.1 mg, 0.0225 mmol, 1.5 equiv), and NMM (7.6 mg, 0.075 mmol, 5.0 equiv) in DMSO (1 mL). HC75-24 was obtained as yellow solid in TFA salt form (9.1 mg, 51%). ¹H NMR (600 MHz, Methanol-*d*₄) δ 9.42 (s, 1H), 9.05 (s, 1H), 8.28 (s, 1H), 8.10 (s, 1H), 8.02 (s, 1H), 7.49 (d, *J* = 8.0 Hz, 2H), 7.43 (d, *J =* 8.1 Hz, 2H), 7.09 (d, *J =* 5.8 Hz, 1H), 4.66 (s, 1H), 4.63 - 4.55 (m, 2H), 4.54 - 4.51 (m, 1H), 4.37 (d, *J=* 15.5 Hz, 1H), 3.93 (d, *J* = 11.0 Hz, 1H), 3.83 (dd, *J =* 11.0, 3.9 Hz, 1H), 3.57 (t, *J =* 6.5 Hz, 2H), 2.81 - 2.69 (m, 2H), 2.49 (s, 3H), 2.33 - 2.15 (m, 8H), 2.11 (ddd, *J =* 13.4, 9.2, 4.5 Hz, 1H), 1.63 - 1.50 (m, 4H), 1.33 - 1.16 (m, 10H), 1.05 (s, 9H). HRMS calcd for C₅₁H₆₆FN₁₀O₆S [M + H⁺] 965.4872, found 965.4881.

### Example 144

### Synthesis of HC75-29

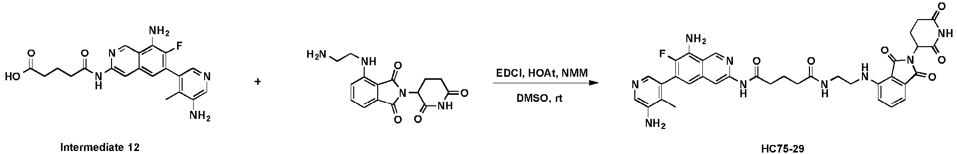

To a solution of Intermediate 12 (12 mg, 0.0192 mmol, 1.0 equiv) in DMSO (1 mL) were added 4-((2-aminoethyl)amino)-2-(2,6-dioxopiperidin-3-yl)isoindoline-1,3-dione (9.5 mg, 0.022 mmol, 1.0 equiv), EDCI (1-ethyl-3-(3-dimethylaminopropyl)carbodiimide) (5.8 mg, 0.03 mmol, 1.5 equiv), HOAt (1-hydroxy-7-azabenzo-triazole) (4.1 mg, 0.03 mmol, 1.5 equiv), and NMM (N-Methylmorpholine) (10.2 mg, 0.1 mmol, 5.0 equiv). After being stirred overnight at room temperature, the resulting mixture was purified by preparative HPLC (10%-100% MeOH/ 0.1% TFA in H₂O) to afford HC75-29 as yellow solid in TFA salt form (8.1 mg, 58%). ¹H NMR (600 MHz, Methanol-*d*₄) δ 9.37 (s, 1H), 8.19 (s, 1H), 8.10 (s, 1H), 8.05 (s, 1H), 7.52 (t, *J =* 7.8 Hz, 1H), 7.12 - 7.05 (m, 2H), 6.96 (d, *J =* 7.1 Hz, 1H), 5.04 (dd, *J* = 12.3, 5.4 Hz, 1H), 3.48 (s, 4H), 2.86 - 2.75 (m, 1H), 2.74 - 2.64 (m, 2H), 2.55 (t, *J =* 7.4 Hz, 2H), 2.34 (t, *J =* 7.1 Hz, 2H), 2.25 (s, 3H), 2.13 - 2.00 (m, 3H). HRMS calcd for C₃₅H₃₅FN₉O₆ [M + H⁺] 696.2694, found 696.2687.

### Example 145

### Synthesis of HC75-31

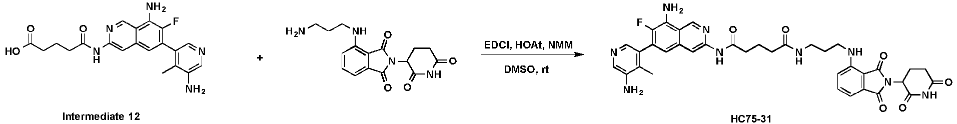

HC75-31 was synthesized following the standard procedure for preparing HC75-29 from Intermediate 12 (6.6 mg, 0.011 mmol, 1.0 equiv), 4-((3-aminopropyl)amino)-2-(2,6-dioxopiperidin-3-yl)isoindoline-1,3-dione (5.2 mg, 0.012 mmol, 1.1equiv), EDCI (3.2 mg, 0.0165 mmol, 1.5 equiv), HOAt (2.2 mg, 0.0165 mmol, 1.5 equiv), and NMM (5.6 mg, 0.055 mmol, 5.0 equiv) in DMSO (0.8 mL). HC75-31 was obtained as yellow solid in TFA salt form (6.9 mg, 67%). ¹H NMR (600 MHz, Methanol-*d*₄) δ 9.38 (s, 1H), 8.19 (s, 1H), 8.10 (s, 1H), 8.03 (s, 1H), 7.50 (dd, *J =* 8.6, 7.0 Hz, 1H), 7.08 (d, *J* = 5.8 Hz, 1H), 7.01 (d, *J =* 8.6 Hz, 1H), 6.97 (d, *J* = 7.1 Hz, 1H), 5.06 (dd, *J* = 12.8, 5.5 Hz, 1H), 3.38 (t, *J* = 6.7 Hz, 2H), 3.33 (s, 2H), 2.86 (ddd, *J* = 17.5, 13.9, 5.4 Hz, 1H), 2.79 - 2.66 (m, 2H), 2.58 (t, *J =* 7.2 Hz, 2H), 2.36 (t, *J =* 7.3 Hz, 2H), 2.24 (s, 3H), 2.16 - 2.02 (m, 3H), 1.85 (p, *J =* 6.6 Hz, 2H). HRMS calcd for C₃₆H₃₇FN₉O₆ [M + H⁺] 710.2851, found 710.2859.

### Example 146

### Synthesis of HC75-34

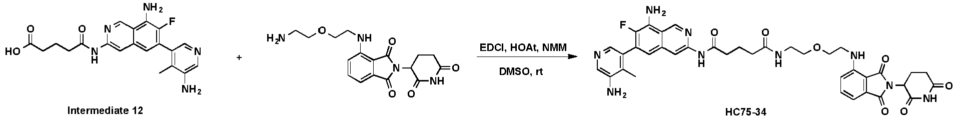

HC75-34 was synthesized following the standard procedure for preparing HC75-29 from Intermediate 12 (9.0 mg, 0.0144 mmol, 1.0 equiv), 4-((2-(2-aminoethoxy)ethyl)amino)-2-(2,6-dioxopiperidin-3-yl)isoindoline-1,3-dione (7.6 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-34 was obtained as yellow solid in TFA salt form (7.7 mg, 55%). ¹H NMR (600 MHz, Methanol-*d*₄) δ 9.21 (s, 1H), 8.30 (s, 1H), 8.10 (s, 1H), 8.06 (s, 1H), 7.41 (t, *J* = 7.8 Hz, 1H), 7.05 - 6.89 (m, 2H), 6.76 (s, 1H), 5.05 (dd, *J =* 12.7, 5.5 Hz, 1H), 3.82 - 3.72 (m, 2H), 3.66 (t, *J =* 5.0 Hz, 2H), 3.35 - 3.60 (m, 4H), 2.89 (ddd, *J =* 17.5, 13.9, 5.4 Hz, 1H), 2.82 - 2.66 (m, 2H), 2.54 (t, *J =* 7.0 Hz, 2H), 2.40 (s, 2H), 2.24 (s, 3H), 2.12 - 1.99 (m, 3H). HRMS calcd for C₃₇H₃₉FN₉O₇ [M + H⁺] 740.2956, found 740.2965.

### Example 147

### Synthesis of HC75-35

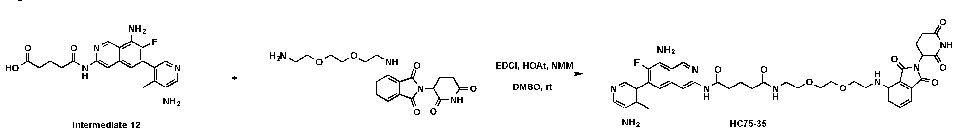

HC75-35 was synthesized following the standard procedure for preparing HC75-29 from Intermediate 12 (9.0 mg, 0.0144 mmol, 1.0 equiv), 4-((2-(2-(2-aminoethoxy)ethoxy)ethyl)amino)-2-(2,6-dioxopiperidin-3-yl)isoindoline-1,3-dione (8.3 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-35 was obtained as yellow solid in TFA salt form (9.7 mg, 67%). ¹H NMR (600 MHz, Methanol-*d*₄) δ 9.29 (s, 1H), 8.24 (s, 1H), 8.09 (s, 1H), 8.03 (s, 1H), 7.45 (t, *J =* 7.8 Hz, 1H), 7.02 (d, *J =* 5.8 Hz, 1H), 6.98 (d, *J =* 8.5 Hz, 1H), 6.89 (d, *J* = 7.0 Hz, 1H), 5.05 (dd, *J =* 12.7, 5.5 Hz, 1H), 3.77 - 3.70 (m, 2H), 3.70 - 3.63 (m, 4H), 3.59 (t, *J* = 5.3 Hz, 2H), 3.47 (t, *J =* 5.2 Hz, 2H), 3.39 (t, *J =* 5.3 Hz, 2H), 2.88 (ddd, *J =* 18.6, 13.8, 5.4 Hz, 1H), 2.81 - 2.66 (m, 2H), 2.55 (t, *J =* 7.3 Hz, 2H), 2.33 (t, *J =* 7.3 Hz, 2H), 2.23 (s, 3H), 2.17 - 2.10 (m, 1H), 2.05 (p, *J =* 7.3 Hz, 2H). HRMS calcd for C₃₉H₄₃FN₉O₈ [M + H⁺] 784.3219, found 784.3211.

### Example 148

### Synthesis of HC75-36

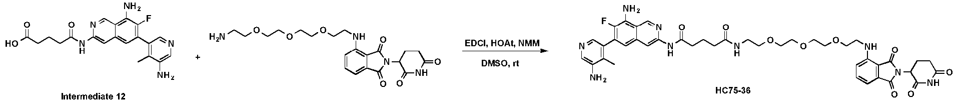

HC75-36 was synthesized following the standard procedure for preparing HC75-29 from Intermediate 12 (9.0 mg, 0.0144 mmol, 1.0 equiv), 4-((2-(2-(2-(2-aminoethoxy)ethoxy)ethoxy)ethyl)amino)-2-(2,6-dioxopiperidin-3-yl)isoindoline-1,3-dione (9.0 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-36 was obtained as yellow solid in TFA salt form (9.6 mg, 63%). ¹H NMR (600 MHz, Methanol-*d*₄) δ 9.36 (s, 1H), 8.15 (s, 1H), 8.10 (s, 1H), 8.03 (s, 1H), 7.47 (t, *J =* 7.8 Hz, 1H), 7.07 (s, 1H), 6.99 (d, *J* = 8.5 Hz, 1H), 6.93 (s, 1H), 5.06 (dd, *J =* 12.8, 5.5 Hz, 1H), 3.71 (t, *J =* 5.2 Hz, 2H), 3.69 - 3.64 (m, 6H), 3.64 - 3.60 (m, 2H), 3.55 (t, *J =* 5.3 Hz, 2H), 3.46 (t, *J =* 5.2 Hz, 2H), 3.40 - 3.36 (m, 2H), 2.88 (ddd, *J* = 18.5, 13.9, 5.4 Hz, 1H), 2.80 - 2.65 (m, 2H), 2.58 (s, 2H), 2.35 (t, *J* = 7.2 Hz, 2H), 2.23 (s, 3H), 2.16 - 2.10 (m, 1H), 2.06 (t, *J* = 7.3 Hz, 2H). HRMS calcd for C₄₁H₄₇FN₉O₉ [M + H⁺] 828.3481, found 828.3488.

### Example 149

### Synthesis of HC75-37

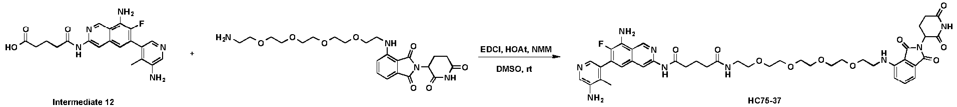

HC75-37 was synthesized following the standard procedure for preparing HC75-29 from Intermediate 12 (9.0 mg, 0.0144 mmol, 1.0 equiv), 4-((14-amino-3,6,9,12-tetraoxatetradecyl)amino)-2-(2,6-dioxopiperidin-3-yl)isoindoline-1,3-dione (9.0 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-37 was obtained as yellow solid in TFA salt form (5.6 mg, 35%). ¹H NMR (600 MHz, Methanol-*d*₄) δ 9.34 (s, 1H), 8.27 (s, 1H), 8.08 (s, 1H), 8.02 (s, 1H), 7.50 (t, *J =* 7.8 Hz, 1H), 7.08 - 7.00 (m, 2H), 6.97 (d, *J* = 7.0 Hz, 1H), 5.06 (dd, *J =* 12.7, 5.5 Hz, 1H), 3.71 (t, *J* = 5.1 Hz, 2H), 3.67 - 3.57 (m, 12H), 3.55 (t, *J* = 5.2 Hz, 2H), 3.46 (t, *J* = 5.2 Hz, 2H), 3.38 (t, *J* = 5.2 Hz, 2H), 2.87 (ddd, *J* = 18.3, 13.9, 5.3 Hz, 1H), 2.80 - 2.66 (m, 2H), 2.57 (s, 2H), 2.36 (s, 2H), 2.23 (s, 3H), 2.16 - 2.09 (m, 1H), 2.09 - 2.00 (m, 2H). HRMS calcd for C₄₃H₅₁FN₉O₁₀ [M + H⁺] 872.3743, found 872.3749.

### Example 150

### Synthesis of HC75-38

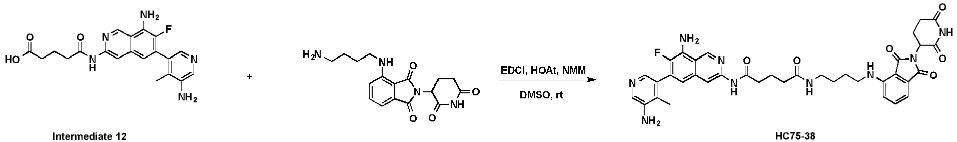

HC75-38 was synthesized following the standard procedure for preparing HC75-29 from Intermediate 12 (9.0 mg, 0.0144 mmol, 1.0 equiv), 4-((4-aminobutyl)amino)-2-(2,6-dioxopiperidin-3-yl)isoindoline-1,3-dione (7.4 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-38 was obtained as yellow solid in TFA salt form (8.1 mg, 59%). ¹H NMR (600 MHz, Methanol-*d*₄) δ 9.37 (s, 1H), 8.23 (s, 1H), 8.10 (s, 1H), 8.03 (s, 1H), 7.51 (dd, *J =* 8.6, 7.1 Hz, 1H), 7.08 (d, *J* = 5.8 Hz, 1H), 7.02 (d, *J =* 8.5 Hz, 1H), 6.96 (d, *J* = 7.1 Hz, 1H), 5.04 (dd, *J =* 12.7, 5.5 Hz, 1H), 3.35 - 3.31 (m, 2H), 3.26 (t, *J =* 6.6 Hz, 2H), 2.85 (ddd, *J =* 17.4, 13.9, 5.3 Hz, 1H), 2.79 - 2.63 (m, 2H), 2.55 (t, *J =* 7.4 Hz, 2H), 2.34 (t, *J =* 7.2 Hz, 2H), 2.24 (s, 3H), 2.14 - 2.00 (m, 3H), 1.74 - 1.59 (m, 4H). HRMS calcd for C₃₇H₃₉FN₉O₆ [M + H⁺] 724.3007, found 724.3014.

### Example 151

### Synthesis of HC75-39

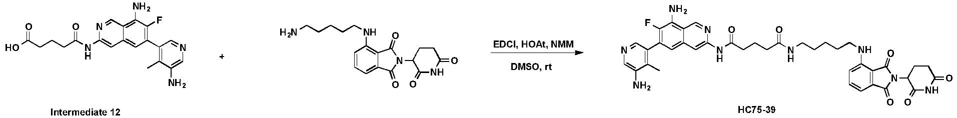

HC75-39 was synthesized following the standard procedure for preparing HC75-29 from Intermediate 12 (9.0 mg, 0.0144 mmol, 1.0 equiv), 4-((5-aminopentyl)amino)-2-(2,6-dioxopiperidin-3-yl)isoindoline-1,3-dione (7.6 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-39 was obtained as yellow solid in TFA salt form (9.6 mg, 69%). ¹H NMR (600 MHz, Methanol-*d*₄) δ 9.36 (s, 1H), 8.24 (s, 1H), 8.10 (s, 1H), 8.03 (s, 1H), 7.52 - 7.48 (m, 1H), 7.07 (d, *J =* 5.8 Hz, 1H), 6.99 (d, *J =* 8.6 Hz, 1H), 6.95 (d, *J =* 7.1 Hz, 1H), 5.05 (dd, *J =* 12.8, 5.5 Hz, 1H), 3.30 (t, *J =* 6.9 Hz, 2H), 3.23 (t, *J =* 6.8 Hz, 2H), 2.86 (ddd, *J =* 17.7, 14.0, 5.4 Hz, 1H), 2.79 - 2.67 (m, 2H), 2.55 (t, *J =* 7.4 Hz, 2H), 2.33 (t, *J =* 7.3 Hz, 2H), 2.24 (s, 3H), 2.15 - 2.09 (m, 1H), 2.05 (p, *J =* 7.4 Hz, 2H), 1.68 (p, *J* = 7.1 Hz, 2H), 1.58 (p, *J =* 7.0 Hz, 2H), 1.52 - 1.40 (m, 2H). HRMS calcd for C₃₈H₄₁FN₉O₆ [M + H⁺] 738.3164, found 738.3169.

### Example 152

### Synthesis of HC75-40

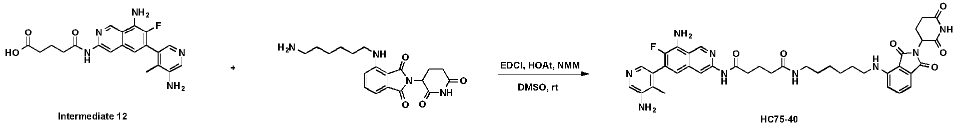

HC75-40 was synthesized following the standard procedure for preparing HC75-29 from Intermediate 12 (9.0 mg, 0.0144 mmol, 1.0 equiv), 4-((6-aminohexyl)amino)-2-(2,6-dioxopiperidin-3-yl)isoindoline-1,3-dione (6.5 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-40 was obtained as yellow solid in TFA salt form (9.8 mg, 69%). ¹H NMR (600 MHz, Methanol-*d*₄) δ 9.40 (s, 1H), 8.17 (s, 1H), 8.10 (s, 1H), 8.03 (s, 1H), 7.55 - 7.46 (m, 1H), 7.09 (d, *J =* 5.9 Hz, 1H), 6.99 (d, *J =* 8.6 Hz, 1H), 6.95 (d, *J =* 7.0 Hz, 1H), 5.05 (dd, *J =* 12.6, 5.5 Hz, 1H), 3.29 (t, *J =* 7.1 Hz, 2H), 3.21 (t, *J =* 6.9 Hz, 2H), 2.87 (ddd, *J =* 18.2, 13.8, 5.4 Hz, 1H), 2.80 - 2.66 (m, 2H), 2.57 (t, *J =* 7.4 Hz, 2H), 2.34 (t, *J =* 7.2 Hz, 2H), 2.24 (s, 3H), 2.16 - 2.09 (m, 1H), 2.06 (p, *J =* 7.4 Hz, 2H), 1.66 (p, *J =* 7.1 Hz, 2H), 1.59 - 1.49 (m, 2H), 1.49 - 1.33 (m, 4H). HRMS calcd for C₃₉H₄₃FN₉O₆ [M + H⁺] 752.3320, found 752.3329.

### Example 153

### Synthesis of HC75-41

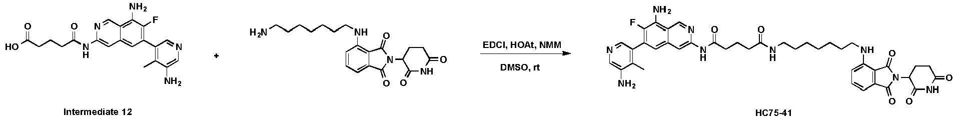

HC75-41 was synthesized following the standard procedure for preparing HC75-29 from Intermediate 12 (9.0 mg, 0.0144 mmol, 1.0 equiv), 4-((7-aminoheptyl)amino)-2-(2,6-dioxopiperidin-3-yl)isoindoline-1,3-dione (8.0 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-41 was obtained as yellow solid in TFA salt form (5.3 mg, 37%). ¹H NMR (600 MHz, Methanol-*d*₄) δ 9.36 (s, 1H), 8.26 (s, 1H), 8.09 (s, 1H), 8.02 (s, 1H), 7.54 - 7.46 (m, 1H), 7.07 (d, *J =* 5.8 Hz, 1H), 6.98 (d, *J =* 8.6 Hz, 1H), 6.96 (d, *J =* 7.1 Hz, 1H), 5.06 (dd, *J =* 12.7, 5.5 Hz, 1H), 3.28 (t, *J =* 7.0 Hz, 2H), 3.20 (t, *J* = 6.9 Hz, 2H), 2.87 (ddd, *J =* 17.4, 13.9, 5.3 Hz, 1H), 2.79 - 2.66 (m, 2H), 2.56 (t, *J =* 7.4 Hz, 2H), 2.33 (t, *J =* 7.3 Hz, 2H), 2.24 (s, 3H), 2.16 - 2.09 (m, 1H), 2.05 (p, *J =* 7.4 Hz, 2H), 1.66 (p, *J =* 7.1 Hz, 2H), 1.52 (p, *J =* 7.0 Hz, 2H), 1.47 - 1.32 (m, 6H). HRMS calcd for C₄₀H₄₅FN₉O₆ [M + H⁺] 766.3477, found 766.3489.

### Example 154

### Synthesis of HC75-42

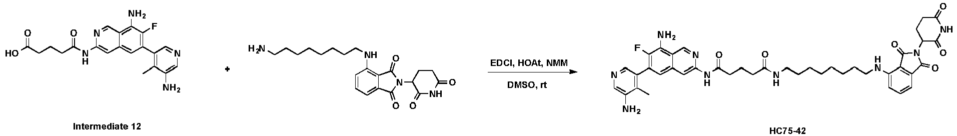

HC75-42 was synthesized following the standard procedure for preparing HC75-29 from Intermediate 12 (9.0 mg, 0.0144 mmol, 1.0 equiv), 4-((8-aminooctyl)amino)-2-(2,6-dioxopiperidin-3-yl)isoindoline-1,3-dione (8.2 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-42 was obtained as yellow solid in TFA salt form (9.9 mg, 68%). ¹H NMR (600 MHz, Methanol-*d*₄) δ 9.40 (s, 1H), 8.17 (s, 1H), 8.10 (s, 1H), 8.02 (s, 1H), 7.50 (t, *J =* 7.8 Hz, 1H), 7.09 (d, *J =* 5.8 Hz, 1H), 7.00 - 6.94 (m, 2H), 5.06 (dd, *J =* 12.7, 5.5 Hz, 1H), 3.28 (t, *J* = 7.0 Hz, 2H), 3.19 (t, *J =* 6.9 Hz, 2H), 2.87 (ddd, *J =* 18.2, 13.7, 5.5 Hz, 1H), 2.80 - 2.65 (m, 2H), 2.58 (t, *J =* 7.4 Hz, 2H), 2.34 (t, *J =* 7.3 Hz, 2H), 2.24 (s, 3H), 2.17 - 2.09 (m, 1H), 2.06 (p, *J =* 7.5 Hz, 2H), 1.63 (p, *J =* 7.1 Hz, 2H), 1.56 - 1.49 (m, 2H), 1.46 - 1.30 (m, 8H). HRMS calcd for C₄₁H₄₇FN₉O₆ [M + H⁺] 780.3633, found 780.3639.

### Example 155

### Synthesis of HC75-43

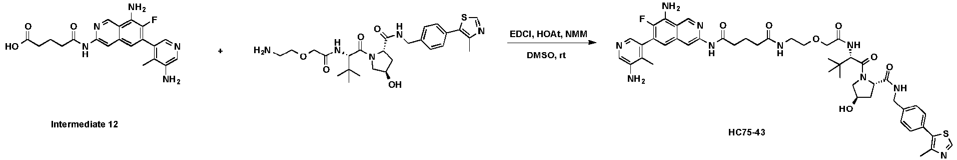

HC75-43 was synthesized following the standard procedure for preparing HC75-29 from Intermediate 12 (9.0 mg, 0.0144 mmol, 1.0 equiv), (2*S*,4*R*)-1-((*S*)-2-(2-(2-aminoethoxy)acetamido)-3,3-dimethylbutanoyl)-4-hydroxy-7V-(4-(4-methylthiazol-5-yl)benzyl)pyrrolidine-2-carboxamide (8.1 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-43 was obtained as yellow solid in TFA salt form (4.9 mg, 30%). ¹H NMR (600 MHz, Methanol-*d*₄) δ 9.36 (s, 1H), 8.95 (s, 1H), 8.28 (s, 1H), 8.09 (s, 1H), 7.99 (s, 1H), 7.45 (d, *J =* 7.4 Hz, 2H), 7.40 (d, *J =* 8.0 Hz, 2H), 7.01 (d, *J =* 5.9 Hz, 1H), 4.72 (s, 1H), 4.62 (dd, *J* = 9.3, 7.6 Hz, 1H), 4.57 - 4.51 (m, 2H), 4.35 (d, *J* = 15.6 Hz, 1H), 4.09 (d, *J* = 15.3 Hz, 1H), 4.00 (d, *J =* 15.2 Hz, 1H), 3.90 (d, *J =* 11.1 Hz, 1H), 3.82 (dd, *J =* 11.0, 3.8 Hz, 1H), 3.71 - 3.57 (m, 2H), 3.54 - 3.39 (m, 2H), 2.59 - 2.52 (m, 2H), 2.46 (s, 3H), 2.38 (t, *J =* 7.4 Hz, 2H), 2.30 - 2.22 (m, 4H), 2.11 (ddd, *J =* 13.4, 9.4, 4.4 Hz, 1H), 2.07 - 2.01 (m, 2H), 1.04 (s, 9H). HRMS calcd for C₄₆H₅₆FN₁₀O₇S [M + H⁺] 911.4038, found 911.4047.

### Example 156

### Synthesis of HC75-44

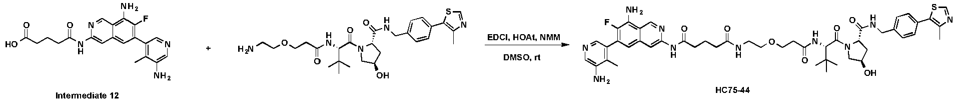

HC75-44 was synthesized following the standard procedure for preparing HC75-29 from Intermediate 12 (9.0 mg, 0.0144 mmol, 1.0 equiv), (2*S*,4*R*)-1-((*S*)-2-(3-(2-aminoethoxy)propanamido)-3,3-dimethylbutanoyl)-4-hydroxy-7V-(4-(4-methylthiazol-5-yl)benzyl)pyrrolidine-2-carboxamide (12.4 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-44 was obtained as yellow solid in TFA salt form (11 mg, 73%). ¹H NMR (600 MHz, Methanol-*d*₄) δ 9.41 (s, 1H), 9.01 (s, 1H), 8.24 (s, 1H), 8.10 (s, 1H), 8.00 (s, 1H), 7.46 (d, *J* = 7.8 Hz, 2H), 7.39 (d, *J* = 8.2 Hz, 2H), 7.06 (d, *J* = 5.8 Hz, 1H), 4.70 (s, 1H), 4.63 (dd, *J* = 9.2, 7.6 Hz, 1H), 4.57 - 4.49 (m, 2H), 4.40 (d, *J* = 15.4 Hz, 1H), 3.92 (d, *J* = 11.1 Hz, 1H), 3.82 (dd, *J =* 11.0, 3.9 Hz, 1H), 3.78 - 3.69 (m, 2H), 3.59 - 3.51 (m, 2H), 3.46 - 3.35 (m, 2H), 2.62 - 2.51 (m, 4H), 2.47 (s, 3H), 2.35 (t, *J =* 7.2 Hz, 2H), 2.30 - 2.21 (m, 4H), 2.09 (ddd, *J =* 13.3, 9.3, 4.4 Hz, 1H), 2.06 - 1.99 (m, 2H), 1.05 (s, 9H). HRMS calcd for C₄₇H₅₈FN₁₀O₇S [M + H⁺] 925.4195, found 925.4191.

### Example 157

### Synthesis of HC75-45

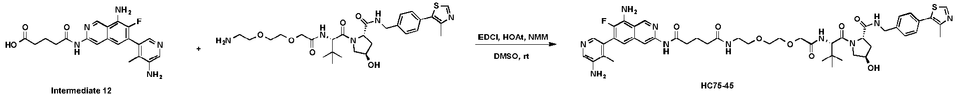

HC75-45 was synthesized following the standard procedure for preparing HC75-29 from Intermediate 12 (9.0 mg, 0.0144 mmol, 1.0 equiv), (2*S*,4*R*)-1-((*S*)-2-(2-(2-(2-aminoethoxy)ethoxy)acetamido)-3,3-dimethylbutanoyl)-4-hydroxy-*N*-(4-(4-methylthiazol-5-yl)benzyl)pyrrolidine-2-carboxamide (9.8 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-45 was obtained as yellow solid in TFA salt form (10.7 mg, 63%). ¹H NMR (600 MHz, Methanol-*d*₄) δ 9.43 (s, 1H), 9.10 (s, 1H), 8.17 (s, 1H), 8.11 (s, 1H), 8.02 (s, 1H), 7.46 (d, *J =* 8.2 Hz, 2H), 7.41 (d, *J =* 8.0 Hz, 2H), 7.10 (d, *J =* 5.8 Hz, 1H), 4.78 (s, 1H), 4.65 (t, *J =* 8.2 Hz, 1H), 4.59 - 4.50 (m, 2H), 4.38 (d, *J =* 15.5 Hz, 1H), 4.10 - 3.97 (m, 2H), 3.88 (d, *J* = 11.0 Hz, 1H), 3.83 (dd, *J =* 11.0, 3.8 Hz, 1H), 3.75 - 3.52 (m, 8H), 3.32 - 3.26 (m, 1H), 2.55 (t, *J =* 7.3 Hz, 2H), 2.50 (s, 3H), 2.43 - 2.21 (m, 6H), 2.14 - 1.97 (m, 3H), 1.07 (s, 9H). HRMS calcd for C₄₈H₆₀FN₁₀O₈S [M + H⁺] 955.4300, found 955.4308.

### Example 158

### Synthesis of HC75-46

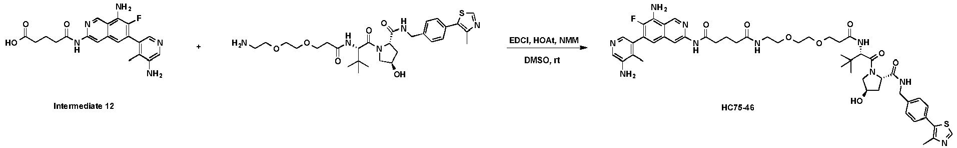

HC75-46 was synthesized following the standard procedure for preparing HC75-29 from Intermediate 12 (9.0 mg, 0.0144 mmol, 1.0 equiv), (2*S*,4*R*)-1-((*S*)-2-(3-(2-(2-aminoethoxy)ethoxy)propanamido)-3,3-dimethylbutanoyl)-4-hydroxy-*N*-(4-(4-methylthiazol-5-yl)benzyl)pyrrolidine-2-carboxamide (13.1 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-46 was obtained as yellow solid in TFA salt form (7.1 mg, 41%). ¹H NMR (600 MHz, Methanol-*d*₄) δ 9.42 (s, 1H), 9.02 (s, 1H), 8.23 (s, 1H), 8.10 (s, 1H), 8.01 (s, 1H), 7.48 (d, *J =* 8.0 Hz, 2H), 7.40 (d, *J =* 8.2 Hz, 2H), 7.08 (d, *J =* 5.8 Hz, 1H), 4.70 (s, 1H), 4.61 (dd, *J* = 9.2, 7.5 Hz, 1H), 4.56 (d, *J =* 15.5 Hz, 1H), 4.53 - 4.50 (m, 1H), 4.38 (d, *J =* 15.5 Hz, 1H), 3.92 (d, *J =* 11.0 Hz, 1H), 3.82 (dd, *J =* 11.0, 3.9 Hz, 1H), 3.75 (t, *J =* 5.9 Hz, 2H), 3.62 (s, 4H), 3.56 (t, *J =* 5.5 Hz, 2H), 3.45 - 3.35 (m, 2H), 2.63 - 2.46 (m, 7H), 2.39 - 2.31 (m, 2H), 2.29 - 2.22 (m, 4H), 2.10 (ddd, *J =* 13.4, 9.2, 4.5 Hz, 1H), 2.03 (p, *J =* 7.9, 7.4 Hz, 2H), 1.05 (s, 9H). HRMS calcd for C₄₉H₆₂FN₁₀O₈S [M + H⁺] 969.4457, found 969.4451.

### Example 159

### Synthesis of HC75-47

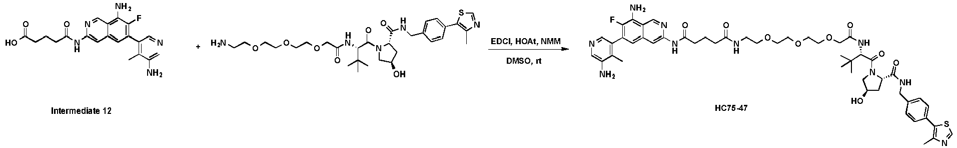

HC75-47 was synthesized following the standard procedure for preparing HC75-29 from Intermediate 12 (9.0 mg, 0.0144 mmol, 1.0 equiv), (2*S*,4*R*)-1-((*S*)-14-amino-2-(*tert*-butyl)-4-oxo-6,9,12-trioxa-3-azatetradecanoyl)-4-hydroxy-*N*-(4-(4-methylthiazol-5-yl)benzyl)pyrrolidine-2-carboxamide (13.6 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-47 was obtained as yellow solid in TFA salt form (9.8 mg, 55%). ¹H NMR (600 MHz, Methanol-*d*₄) δ 9.42 (s, 1H), 9.04 (s, 1H), 8.23 (s, 1H), 8.11 (s, 1H), 8.01 (s, 1H), 7.48 (d, *J=* 8.0 Hz, 2H), 7.42 (d, *J* = 8.1 Hz, 2H), 7.09 (d, *J =* 5.8 Hz, 1H), 4.72 (s, 1H), 4.64 - 4.59 (m, 1H), 4.57 - 4.50 (m, 2H), 4.38 (d, *J* = 15.5 Hz, 1H), 4.11- 4.01 (m, 2H), 3.90 (d, *J* = 11.1 Hz, 1H), 3.82 (dd, *J =* 11.0, 3.8 Hz, 1H), 3.75 - 3.61 (m, 8H), 3.54 (t, *J=* 5.4 Hz, 2H), 3.39 - 3.35 (m, 2H), 2.56 (t, *J* = 7.5 Hz, 2H), 2.49 (s, 3H), 2.34 (t, *J =* 7.5 Hz, 2H), 2.30 - 2.22 (m, 4H), 2.11 (ddd, *J =* 13.4, 9.3, 4.4 Hz, 1H), 2.04 (p, *J* = 7.4 Hz, 2H), 1.05 (s, 9H). HRMS calcd for C₅₀H₆₄FN₁₀O₉S [M + H⁺] 999.4562, found 999.4575.

### Example 160

### Synthesis of HC75-48

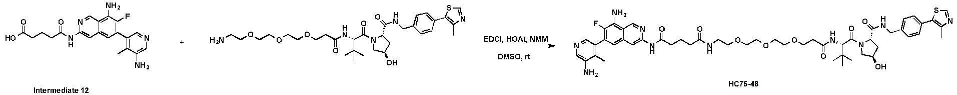

HC75-48 was synthesized following the standard procedure for preparing HC75-29 from Intermediate 12 (9.0 mg, 0.0144 mmol, 1.0 equiv), (2*S*,4*R*)-1-((*S*)-1-amino-14-(*tert*-butyl)-12-oxo-3,6,9-trioxa-13-azapentadecan-15-oyl)-4-hydroxy-*N*-(4-(4-methylthiazol-5-yl)benzyl)pyrrolidine-2-carboxamide (13.8 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-48 was obtained as yellow solid in TFA salt form (6.5 mg, 36%). ¹H NMR (600 MHz, Methanol-*d*₄) δ 9.42 (s, 1H), 9.02 (s, 1H), 8.25 (s, 1H), 8.10 (s, 1H), 8.01 (s, 1H), 7.48 (d, *J* = 8.0 Hz, 2H), 7.41 (d, *J* = 8.1 Hz, 2H), 7.08 (d, *J* = 5.9 Hz, 1H), 4.68 (s, 1H), 4.60 (dd, *J* = 9.3, 7.5 Hz, 1H), 4.57 (d, *J* = 15.5 Hz, 1H), 4.53 - 4.50 (m, 1H), 4.37 (d, *J* = 15.5 Hz, 1H), 3.92 (d, *J* = 10.9 Hz, 1H), 3.82 (dd, *J =* 11.0, 3.9 Hz, 1H), 3.75 - 3.70 (m, 2H), 3.68 - 3.59 (m, 8H), 3.56 (t, *J* = 5.4 Hz, 2H), 3.40 - 3.36 (m, 2H), 2.62 - 2.53 (m, 3H), 2.54 - 2.44 (m, 4H), 2.35 (t, *J* = 7.3 Hz, 2H), 2.25 (s, 4H), 2.10 (ddd, *J* = 13.3, 9.2, 4.4 Hz, 1H), 2.07 - 2.00 (m, 2H), 1.05 (s, 9H). HRMS calcd for C₅₁H₆₆FN₁₀O₉S [M + H⁺] 1013.4719, found 1013.4727.

### Example 161

### Synthesis of HC75-49

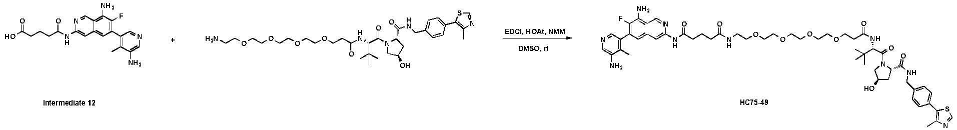

HC75-49 was synthesized following the standard procedure for preparing HC75-29 from Intermediate 12 (9.0 mg, 0.0144 mmol, 1.0 equiv), (2*S*,4*R*)-1-((*S*)-1-amino-17-(*tert*-butyl)-15-oxo-3,6,9,12-tetraoxa-16-azaoctadecan-18-oyl)-4-hydroxy-*N*-(4-(4-methylthiazol-5-yl)benzyl)pyrrolidine-2-carboxamide (11.5 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-49 was obtained as yellow solid in TFA salt form (9.3 mg, 50%). ¹H NMR (600 MHz, Methanol-*d*₄) δ 9.43 (s, 1H), 9.05 (s, 1H), 8.22 (s, 1H), 8.11 (s, 1H), 8.01 (s, 1H), 7.49 (d, *J =* 8.1 Hz, 2H), 7.42 (d, *J =* 8.0 Hz, 2H), 7.10 (d, *J =* 5.9 Hz, 1H), 4.67 (s, 1H), 4.63 - 4.50 (m, 3H), 4.38 (d, *J =* 15.5 Hz, 1H), 3.92 (d, *J =* 11.0 Hz, 1H), 3.82 (dd, *J =* 11.0, 3.9 Hz, 1H), 3.77 - 3.68 (m, 2H), 3.66 - 3.58 (m, 12H), 3.56 (t, *J =* 5.4 Hz, 2H), 3.39 (t, *J =* 5.4 Hz, 2H), 2.62 - 2.54 (m, 3H), 2.53 - 2.45 (m, 4H), 2.35 (t, *J =* 7.3 Hz, 2H), 2.25 (s, 4H), 2.16 - 2.00 (m, 3H), 1.05 (s, 9H). HRMS calcd for C₅₃H₇₀FN₁₀O₁₀S [M + H⁺] 1057.4981, found 1057.4987.

### Example 162

### Synthesis of HC75-50

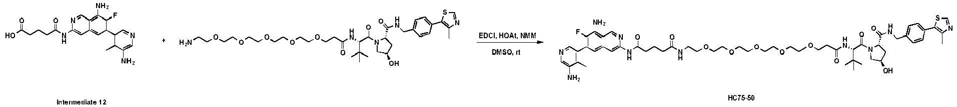

HC75-50 was synthesized following the standard procedure for preparing HC75-29 from Intermediate 12 (9.0 mg, 0.0144 mmol, 1.0 equiv), (2*S*,4*R*)-1-((*S*)-1-amino-20-(*tert*-butyl)-18-oxo-3,6,9,12,15-pentaoxa-19-azahenicosan-21-oyl)-4-hydroxy-*N*-(4-(4-methylthiazol-5-yl)benzyl)pyrrolidine-2-carboxamide (15.2 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-50 was obtained as yellow solid in TFA salt form (6.8 mg, 36%). ¹H NMR (600 MHz, Methanol-*d*₄) δ 9.45 (s, 1H), 9.11 (d, *J =* 8.1 Hz, 1H), 8.19 (s, 1H), 8.11 (s, 1H), 8.02 (s, 1H), 7.49 (d, *J =* 8.0 Hz, 2H), 7.43 (d, *J =* 7.9 Hz, 2H), 7.11 (d, *J =* 5.7 Hz, 1H), 4.67 (s, 1H), 4.62 - 4.50 (m, 3H), 4.38 (d, *J =* 15.6 Hz, 1H), 3.92 (d, *J =* 10.9 Hz, 1H), 3.82 (dd, *J =* 11.0, 3.9 Hz, 1H), 3.74 (ddq, *J =* 19.4, 10.1, 4.7 Hz, 2H), 3.62 (dd, *J =* 9.5, 6.3 Hz, 16H), 3.56 (t, *J=* 5.4 Hz, 2H), 3.39 (t, *J* = 5.4 Hz, 2H), 2.62 - 2.54 (m, 3H), 2.54 - 2.43 (m, 4H), 2.36 (t, *J* = 7.2 Hz, 2H), 2.29 - 2.21 (m, 4H), 2.15 - 2.01 (m, 3H), 1.05 (s, 9H). HRMS calcd for C₅₅H₇₄FN₁₀O₁₁S [M + H⁺] 1101.5243, found 1101.5252.

### Example 163

### Synthesis of HC75-52

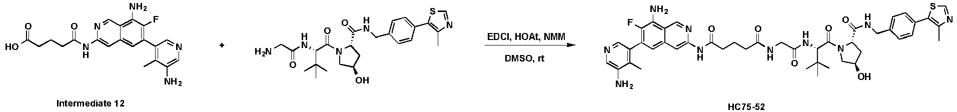

HC75-52 was synthesized following the standard procedure for preparing HC75-29 from Intermediate 12 (9.0 mg, 0.0144 mmol, 1.0 equiv), (2*S*,4*R*)-1-((*S*)-2-(2-aminoacetamido)-3,3-dimethylbutanoyl)-4-hydroxy-*N*-(4-(4-methylthiazol-5-yl)benzyl)pyrrolidine-2-carboxamide (10.3 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-52 was obtained as yellow solid in TFA salt form (4.3 mg, 27%). ¹H NMR (600 MHz, Methanol-*d*₄) δ 9.40 (s, 1H), 9.02 (s, 1H), 8.21 (s, 1H), 8.10 (s, 1H), 8.01 (s, 1H), 7.43 (d, *J =* 8.1 Hz, 2H), 7.37 (d, *J =* 8.2 Hz, 2H), 7.08 (d, *J =* 5.9 Hz, 1H), 4.72 - 4.64 (m, 2H), 4.60 - 4.50 (m, 2H), 4.34 (d, *J* = 15.4 Hz, 1H), 4.01 - 3.91 (m, 2H), 3.90 - 3.82 (m, 2H), 2.67 - 2.54 (m, 2H), 2.49 - 2.37 (m, 5H), 2.30 - 2.21 (m, 4H), 2.19 - 2.06 (m, 3H), 1.07 (s, 9H). HRMS calcd for C₄₄H₅₂FN₁₀O₆S [M + H⁺] 867.3776, found 867.3788.

### Example 164

### Synthesis of HC75-53

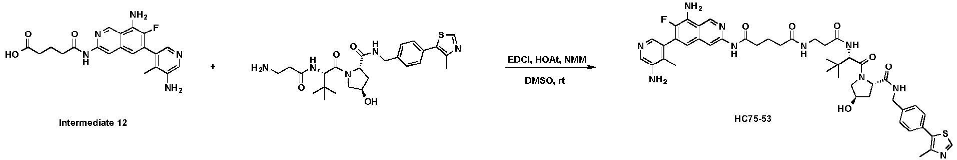

HC75-53 was synthesized following the standard procedure for preparing HC75-29 from Intermediate 12 (9.0 mg, 0.0144 mmol, 1.0 equiv), (2*S*,4*R*)-1-((*S*)-2-(3-aminopropanamido)-3,3-dimethylbutanoyl)-4-hydroxy-*N*-(4-(4-methylthiazol-5-yl)benzyl)pyrrolidine-2-carboxamide (10.5 mg, 0.016 mmol, 1.1equiv), EDCI (4.2 mg, 0.0216 mmol, 1.5 equiv), HOAt(3.0 mg, 0.0216 mmol, 1.5 equiv), and NMM (9.0 mg, 0.0864 mmol, 6.0 equiv) in DMSO (1 mL). HC75-53 was obtained as yellow solid in TFA salt form (4.9 mg, 31%). ¹H NMR (600 MHz, Methanol-*d*₄) δ 9.38 (s, 1H), 8.97 (s, 1H), 8.25 (s, 1H), 8.09 (s, 1H), 8.00 (s, 1H), 7.45 (d, *J* = 7.7 Hz, 2H), 7.36 (d, *J* = 8.2 Hz, 2H), 7.01 (d, *J* = 5.8 Hz, 1H), 4.69 - 4.51 (m, 4H), 4.35 (d, *J =* 15.8 Hz, 1H), 3.98 (d, *J =* 11.1 Hz, 1H), 3.81 (dd, *J =* 11.0, 3.9 Hz, 1H), 3.56 - 3.48 (m, 1H), 3.45 - 3.37 (m, 1H), 2.57 - 2.43 (m, 7H), 2.33 - 2.29 (m, 2H), 2.28 - 2.21 (m, 4H), 2.16 - 1.98 (m, 3H), 1.06 (s, 9H). HRMS calcd for C₄₅H₅₄FN₁₀O₆S [M + H⁺] 881.3933, found 881.3939.

### Example 165

### Synthesis of HC75-54

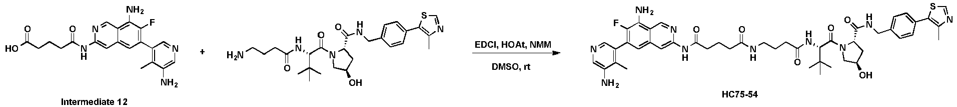

HC75-54 was synthesized following the standard procedure for preparing HC75-29 from Intermediate 12 (9.0 mg, 0.0144 mmol, 1.0 equiv), (2*S*,4*R*)-1-((*S*)-2-(4-aminobutanamido)-3,3-dimethylbutanoyl)-4-hydroxy-*N*-(4-(4-methylthiazol-5-yl)benzyl)pyrrolidine-2-carboxamide (10.7 mg, 0.016 mmol, 1.1equiv), EDCI (4.2 mg, 0.0216 mmol, 1.5 equiv), HOAt(3.0 mg, 0.0216 mmol, 1.5 equiv), and NMM (9.0 mg, 0.0864 mmol, 6.0 equiv) in DMSO (1 mL). HC75-54 was obtained as yellow solid in TFA salt form (5.6 mg, 35%). ¹H NMR (600 MHz, Methanol-*d*₄) δ 9.40 (s, 1H), 9.00 (s, 1H), 8.25 (s, 1H), 8.10 (d, *J =* 1.1 Hz, 1H), 8.01 (s, 1H), 7.47 (d, *J* = 8.1 Hz, 2H), 7.40 (d, *J=* 8.3 Hz, 2H), 7.07 (d, *J =* 5.9 Hz, 1H), 4.66 (s, 1H), 4.61 (dd, *J =* 9.3, 7.5 Hz, 1H), 4.56 (d, *J* = 15.6 Hz, 1H), 4.54 - 4.50 (m, 1H), 4.36 (d, *J* = 15.5 Hz, 1H), 3.94 (d, *J =* 11.0 Hz, 1H), 3.83 (dd, *J =* 11.0, 4.0 Hz, 1H), 3.23 (t, *J =* 6.8 Hz, 2H), 2.57 (d, *J =* 8.7 Hz, 2H), 2.47 (s, 3H), 2.39 - 2.30 (m, 4H), 2.26 - 2.21 (m, 4H), 2.15 - 2.02 (m, 3H), 1.85 - 1.76 (m, 2H), 1.06 (s, 9H). HRMS calcd for C₄₆H₅₆FN₁₀O₆S [M + H⁺] 895.4089, found 895.4098.

### Example 166

### Synthesis of HC75-55

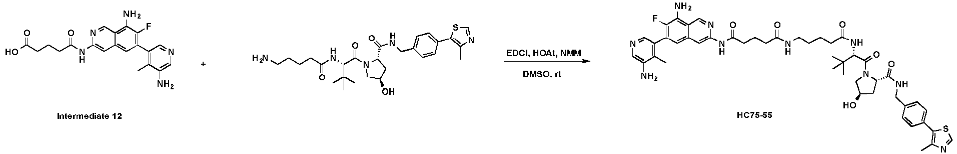

HC75-55 was synthesized following the standard procedure for preparing HC75-29 from Intermediate 12 (9.0 mg, 0.0144 mmol, 1.0 equiv), (2*S*,4*R*)-1-((*S*)-2-(5-aminopentanamido)-3,3-dimethylbutanoyl)-4-hydroxy-*N*-(4-(4-methylthiazol-5-yl)benzyl)pyrrolidine-2-carboxamide (8.5 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-55 was obtained as yellow solid in TFA salt form (3.7 mg, 23%). ¹H NMR (600 MHz, Methanol-*d*₄) δ 9.41 (s, 1H), 9.02 (s, 1H), 8.26 (s, 1H), 8.10 (d,J= 1.2 Hz, 1H), 8.02 (s, 1H), 7.51 - 7.46 (m, 2H), 7.42 (d, *J =* 8.2 Hz, 2H), 7.08 (d, *J =* 5.9 Hz, 1H), 4.67 (s, 1H), 4.63 - 4.49 (m, 3H), 4.37 (d, *J =* 15.5 Hz, 1H), 3.92 (d, *J =* 10.9 Hz, 1H), 3.82 (dd, *J =* 10.9, 3.9 Hz, 1H), 3.26 - 3.15 (m, 2H), 2.60 - 2.51 (m, 2H), 2.49 (s, 3H), 2.35 - 2.28 (m, 4H), 2.25 (d, *J =* 1.5 Hz, 4H), 2.14 - 1.99 (m, 3H), 1.70 - 1.62 (m, 2H), 1.53 (p, *J* = 7.1 Hz, 2H), 1.05 (s, 9H). HRMS calcd for C₄₇H₅₈FN₁₀O₆S [M + H⁺] 909.4246, found 909.4257.

### Example 167

### Synthesis of HC75-56

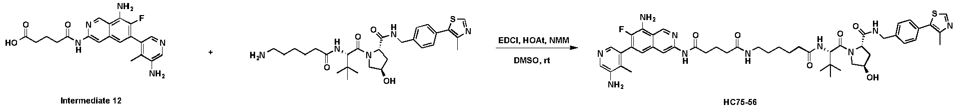

HC75-56 was synthesized following the standard procedure for preparing HC75-29 from Intermediate 12 (9.0 mg, 0.0144 mmol, 1.0 equiv), (2*S*,4*R*)-1-((*S*)-2-(6-aminohexanamido)-3,3-dimethylbutanoyl)-4-hydroxy-*N*-(4-(4-methylthiazol-5-yl)benzyl)pyrrolidine-2-carboxamide (8.7 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-56 was obtained as yellow solid in TFA salt form (4.7 mg, 28%). ¹H NMR (600 MHz, Methanol-*d*₄) δ 9.43 (s, 1H), 9.05 (s, 1H), 8.26 (s, 1H), 8.10 (s, 1H), 8.02 (d, *J =* 3.2 Hz, 1H), 7.49 (d, *J =* 7.9 Hz, 2H), 7.42 (d, *J* = 7.9 Hz, 2H), 7.09 (d, *J* = 5.8 Hz, 1H), 4.70 (s, 1H), 4.66 - 4.61 (m, 1H), 4.56 (d, *J* = 15.5 Hz, 1H), 4.52 (s, 1H), 4.39 (d, *J =* 15.4 Hz, 1H), 3.95 (d, *J =* 11.0 Hz, 1H), 3.82 (dd, *J =* 10.9, 3.9 Hz, 1H), 3.28 - 3.22 (m, 1H), 3.19 - 3.11 (m, 1H), 2.65 - 2.56 (m, 1H), 2.49 (s, 4H), 2.38 - 2.22 (m, 8H), 2.10 (ddd, *J =* 13.3, 9.2, 4.4 Hz, 1H), 2.06 - 1.99 (m, 2H), 1.69 - 1.60 (m, 2H), 1.56 - 1.47 (m, 2H), 1.41 - 1.29 (m, 2H), 1.06 (s, 9H). HRMS calcd for C₄₈H₆₀FN₁₀O₆S [M + H⁺] 923.4402, found 923.4410.

### Example 168

### Synthesis of HC75-57

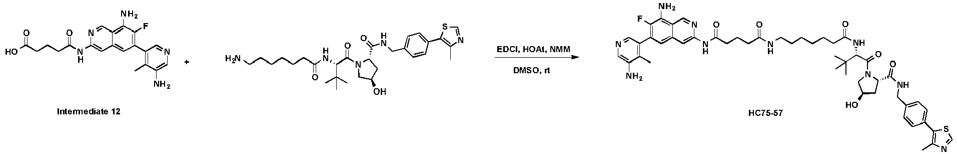

HC75-57 was synthesized following the standard procedure for preparing HC75-29 from Intermediate 12 (9.0 mg, 0.0144 mmol, 1.0 equiv), (2*S*,4*R*)-1-((*S*)-2-(7-aminoheptanamido)-3,3-dimethylbutanoyl)-4-hydroxy-*N*-(4-(4-methylthiazol-5-yl)benzyl)pyrrolidine-2-carboxamide (9.0 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-57 was obtained as yellow solid in TFA salt form (3.1 mg, 18%). ¹H NMR (600 MHz, Methanol-*d*₄) δ 9.38 (s, 1H), 8.95 (s, 1H), 8.32 (s, 1H), 8.09 (s, 1H), 8.01 (s, 1H), 7.48 (d, *J =* 8.0 Hz, 2H), 7.41 (d, *J =* 8.2 Hz, 2H), 7.06 (d, *J =* 5.9 Hz, 1H), 4.66 (s, 1H), 4.61 (dd, *J =* 9.1, 7.5 Hz, 1H), 4.56 (d, *J =* 15.4 Hz, 1H), 4.52 (s, 1H), 4.37 (d, *J =* 15.5 Hz, 1H), 3.93 (d, *J =* 11.0 Hz, 1H), 3.82 (dd, *J =* 10.9, 3.9 Hz, 1H), 3.24 - 3.13 (m, 2H), 2.59 - 2.49 (m, 2H), 2.48 (s, 3H), 2.35 - 2.21 (m, 8H), 2.10 (ddd, *J =* 13.3, 9.1, 4.4 Hz, 1H), 2.03 (p, *J =* 7.4 Hz, 2H), 1.68 - 1.59 (m, 2H), 1.55 - 1.47 (m, 2H), 1.40 - 1.30 (m, 4H), 1.05 (s, 9H). HRMS calcd for C₄₉H₆₂FN₁₀O₆S [M + H⁺] 937.4559, found 937.4568.

### Example 169

### Synthesis of HC75-58

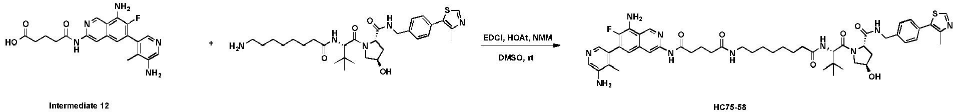

HC75-58 was synthesized following the standard procedure for preparing HC75-29 from Intermediate 12 (9.0 mg, 0.0144 mmol, 1.0 equiv), (2*S*,4*R*)-1-((*S*)-2-(8-aminooctanamido)-3,3-dimethylbutanoyl)-4-hydroxy-*N*-(4-(4-methylthiazol-5-yl)benzyl)pyrrolidine-2-carboxamide (11.5 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-58 was obtained as yellow solid in TFA salt form (4.2 mg, 25%). ¹H NMR (600 MHz, Methanol-*d*₄) δ 9.42 (s, 1H), 9.01 (s, 1H), 8.26 (s, 1H), 8.10 (s, 1H), 8.03 (s, 1H), 7.50 (d, *J =* 8.2 Hz, 2H), 7.41 (d, *J =* 8.3 Hz, 2H), 7.07 (d, *J* = 5.8 Hz, 1H), 4.71 (s, 1H), 4.65 (dd, *J =* 9.1, 7.5 Hz, 1H), 4.61 (d, *J =* 15.5 Hz, 1H), 4.55 - 4.50 (m, 1H), 4.36 (d, *J* = 15.5 Hz, 1H), 3.94 (d, *J =* 11.0 Hz, 1H), 3.83 (dd, *J =* 11.0, 3.9 Hz, 1H), 3.32 - 3.25 (m, 1H), 3.10 (dt, *J =* 13.4, 6.9 Hz, 1H), 2.60 - 2.52 (m, 1H), 2.52 - 2.42 (m, 4H), 2.40 - 2.22 (m, 8H), 2.10 (ddd, *J =* 13.3, 9.2, 4.4 Hz, 1H), 2.03 - 1.97 (m, 2H), 1.66 -1.57 (m, 2H), 1.54 - 1.42 (m, 2H), 1.37 -1.26 (m, 6H), 1.06 (s, 9H). HRMS calcd for C₅₀H₆₄FN₁₀O₆S [M + H⁺] 951.4715, found 951.4719.

### Example 170

### Synthesis of HC75-59

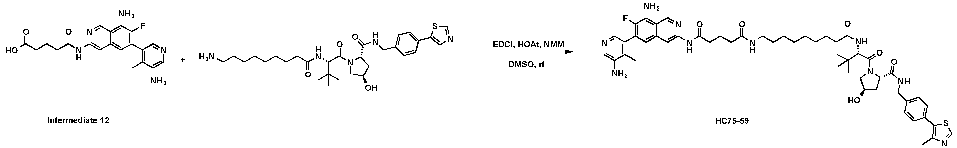

HC75-59 was synthesized following the standard procedure for preparing HC75-29 from Intermediate 12 (9.0 mg, 0.0144 mmol, 1.0 equiv), (2*S*,4*R*)-1-((*S*)-2-(9-aminononanamido)-3,3-dimethylbutanoyl)-4-hydroxy-*N*-(4-(4-methylthiazol-5-yl)benzyl)pyrrolidine-2-carboxamide (9.3 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-59 was obtained as yellow solid in TFA salt form (5.9 mg, 34%). ¹H NMR (600 MHz, Methanol-*d*₄) δ 9.41 (s, 1H), 9.01 (s, 1H), 8.26 (s, 1H), 8.10 (s, 1H), 8.02 (s, 1H), 7.49 (d, *J=* 8.0 Hz, 2H), 7.46 - 7.37 (m, 2H), 7.08 (d, *J* = 5.9 Hz, 1H), 4.67 (s, 1H), 4.64 - 4.54 (m, 2H), 4.54 - 4.51 (m, 1H), 4.37 (d, *J =* 15.5 Hz, 1H), 3.93 (d, *J =* 11.0 Hz, 1H), 3.83 (dd, *J =* 11.0, 3.9 Hz, 1H), 3.24 - 3.14 (m, 2H), 2.60 - 2.51 (m, 2H), 2.48 (s, 3H), 2.37 - 2.20 (m, 8H), 2.10 (ddd, *J =* 13.3, 9.2, 4.5 Hz, 1H), 2.06 - 1.98 (m, 2H), 1.66 -1.56 (m, 2H), 1.54 -1.47 (m, 2H), 1.38 -1.28 (m, 8H), 1.05 (s, 9H). HRMS calcd for C₅₁H₆₆FN₁₀O₆S [M + H⁺] 965.4872, found 965.4884.

### Example 171

### Synthesis of HC75-60

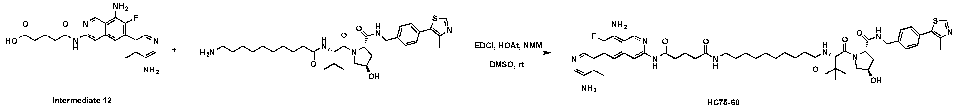

HC75-60 was synthesized following the standard procedure for preparing HC75-29 from Intermediate 12 (9.0 mg, 0.0144 mmol, 1.0 equiv), (2*S*,4*R*)-1-((*S*)-2-(10-aminodecanamido)-3,3-dimethylbutanoyl)-4-hydroxy-*N*-(4-(4-methylthiazol-5-yl)benzyl)pyrrolidine-2-carboxamide (11.9 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-60 was obtained as yellow solid in TFA salt form (4.3 mg, 25%). ¹H NMR (600 MHz, Methanol-*d*₄) δ 9.43 (s, 1H), 9.05 (s, 1H), 8.24 (s, 1H), 8.10 (s, 1H), 8.02 (s, 1H), 7.50 (d, *J =* 8.1 Hz, 2H), 7.43 (d, *J =* 8.2 Hz, 2H), 7.09 (d, *J =* 5.9 Hz, 1H), 4.67 (s, 1H), 4.63 - 4.55 (m, 2H), 4.54 - 4.49 (m, 1H), 4.37 (d, *J =* 15.6 Hz, 1H), 3.93 (d, *J =* 11.0 Hz, 1H), 3.83 (dd, *J =* 11.0, 3.9 Hz, 1H), 3.25 - 3.12 (m, 2H), 2.55 (t, *J =* 7.3 Hz, 2H), 2.49 (s, 3H), 2.38 - 2.20 (m, 8H), 2.10 (ddd, *J =* 13.2, 9.1, 4.5 Hz, 1H), 2.03 (p, *J* = 7.4 Hz, 2H), 1.65 - 1.56 (m, 2H), 1.55 - 1.45 (m, 2H), 1.38 - 1.25 (m, 10H), 1.06 (s, 9H). HRMS calcd for C₅₂H₆₈FN₁₀O₆S [M + H⁺] 979.5028, found 979.5042.

### Example 172

### Synthesis of HC75-61

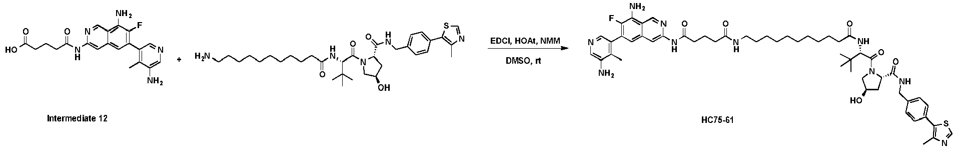

HC75-61 was synthesized following the standard procedure for preparing HC75-29 from Intermediate 12 (9.0 mg, 0.0144 mmol, 1.0 equiv), (2*S*,4*R*)-1-((*S*)-2-(11-aminoundecanamido)-3,3-dimethylbutanoyl)-4-hydroxy-*N*-(4-(4-methylthiazol-5-yl)benzyl)pyrrolidine-2-carboxamide (9.8 mg, 0.016 mmol, 1.1 equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-61 was obtained as yellow solid in TFA salt form (6.3 mg, 36%). ¹H NMR (600 MHz, Methanol-*d*₄) δ 9.42 (s, 1H), 9.04 (s, 1H), 8.25 (s, 1H), 8.10 (s, 1H), 8.02 (s, 1H), 7.49 (d, *J=* 8.0 Hz, 2H), 7.43 (d, *J =* 8.2 Hz, 2H), 7.10 (d, *J=* 5.8 Hz, 1H), 4.66 (s, 1H), 4.63 - 4.54 (m, 2H), 4.54 - 4.49 (m, 1H), 4.37 (d, *J =* 15.5 Hz, 1H), 3.93 (d, *J =* 11.0 Hz, 1H), 3.82 (dd, *J =* 11.0, 3.9 Hz, 1H), 3.22 - 3.13 (m, 2H), 2.56 (t, *J* = 7.4 Hz, 2H), 2.50 (s, 3H), 2.35 - 2.21 (m, 8H), 2.10 (ddd, *J* = 13.3, 9.2, 4.5 Hz, 1H), 2.06 - 2.00 (m, 2H), 1.65 - 1.56 (m, 2H), 1.54 - 1.47 (m, 2H), 1.37 - 1.26 (m, 12H), 1.05 (s, 9H). HRMS calcd for C₅₃H₇₀FN₁₀O₆S [M + H⁺] 993.5185, found 993.5189.

### Example 173

### Synthesis of HC75-62

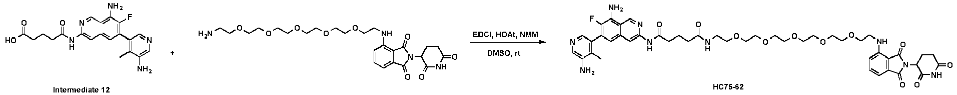

HC75-62 was synthesized following the standard procedure for preparing HC75-29 from Intermediate 12 (9.0 mg, 0.0144 mmol, 1.0 equiv), 4-((17-amino-3,6,9,12,15-pentaoxaheptadecyl)amino)-2-(2,6-dioxopiperidin-3-yl)isoindoline-1,3-dione (10.4 mg, 0.016 mmol, 1.1equiv), EDCI (4.2 mg, 0.0216 mmol, 1.5 equiv), HOAt (3.0 mg, 0.0216 mmol, 1.5 equiv), and NMM (9.0 mg, 0.0864 mmol, 6.0 equiv) in DMSO (1 mL). HC75-62 was obtained as yellow solid in TFA salt form (8.2 mg, 48%). ¹H NMR (600 MHz, Methanol-*d*₄) δ 9.35 (s, 1H), 8.24 (s, 1H), 8.09 (s, 1H), 8.03 (s, 1H), 7.50 (dd, *J =* 8.5, 7.0 Hz, 1H), 7.07 (d, *J =* 5.8 Hz, 1H), 7.02 (d, *J =* 8.6 Hz, 1H), 6.97 (d, *J =* 7.0 Hz, 1H), 5.06 (dd, *J =* 12.7, 5.5 Hz, 1H), 3.71 (t, *J =* 5.2 Hz, 2H), 3.65 (s, 4H), 3.64 - 3.58 (m, 12H), 3.55 (t, *J =* 5.4 Hz, 2H), 3.46 (t, *J =* 5.2 Hz, 2H), 3.38 (t, *J =* 5.4 Hz, 2H), 2.88 (ddd, *J* = 17.1, 13.8, 5.3 Hz, 1H), 2.81 - 2.65 (m, 2H), 2.57 (t, *J* = 7.4 Hz, 2H), 2.35 (t, *J* = 7.3 Hz, 2H), 2.23 (d, *J =* 1.5 Hz, 3H), 2.16 - 2.09 (m, 1H), 2.05 (p, *J* = 7.3 Hz, 2H). HRMS calcd for C₄₅H₅₅FN₉O₁₁ [M + H⁺] 916.4005, found 916.4016.

### Example 174

### Synthesis of intermediate 13

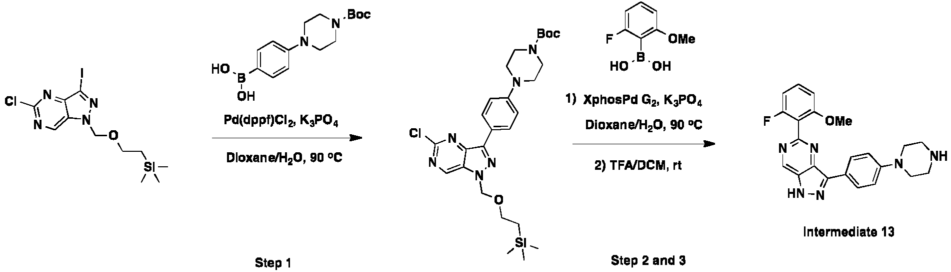

### Step 1: tert-butyl 4-(4-(5-chloro-1-((2-(trimethylsilyl)ethoxy)methyl)-1H pyrazolo[4,3-d]pyrimidin-3-yl)phenyl)piperazine-1-carboxylate

A mixture of 5-chloro-3-iodo-1-((2-(trimethylsilyl)ethoxy)methyl)-1*H*-pyrazolo[4,3-*d*]pyrimidine (411 mg, 1 mmol, 1.0 equiv), (4-(4-(*tert*-butoxycarbonyl)piperazin-1-yl)phenyl)boronic acid (306 mg, 1 mmol, 1.0 equiv), Pd(dppf)Cl₂ (110 mg, 0.15 mmol, 0.15 equiv), K₃PO₄ (424 mg, 2.0 mmol, 2.0 equiv) in 1,4-dioxane (8 mL) and water (1.3 mL) was stirred at 90 °C for 5 h. The reaction was filtered and extracted with ethyl acetate. The combined organic extracts were combined and concentrated under vacuum. The residue was purified by silica gel column (hexane/ ethyl acetate = 3:2) to give the *tert*-butyl 4-(4-(5-chloro-1-((2-(trimethylsilyl)ethoxy)methyl)-1*H*-pyrazolo[4,3-*d*]pyrimidin-3-yl)phenyl)piperazine-1-carboxylate as a yellow solid (345 mg, 63%). ESI m/z =545.3 [M + H⁺].

### Step 2 and 3: intermediate 13

A mixture of *tert*-butyl 4-(4-(5-chloro-1-((2-(trimethylsilyl)ethoxy)methyl)-1*H*-pyrazolo[4,3-*d*]pyrimidin-3-yl)phenyl)piperazine-1-carboxylate (345 mg, 0.63 mmol, 1.0 equiv), (2-fluoro-6-methoxyphenyl)boronic acid (306 mg, 1 mmol, 1.0 equiv), XphosPd G2 (50 mg, 0.06 mmol, 0.1 equiv), K₃PO₄ (400 mg, 1.9 mmol, 3.0 equiv) in 1,4-dioxane (6.4 mL) and water (0.8 mL) was stirred at 90 °C for 1.5 h. The reaction was filtered and extracted with ethyl acetate. The combined organic extracts were combined and concentrated under vacuum. The obtained residue was dissolved in DCM (4 mL). To the solution was added TFA (2 mL). Stirred at room temperature for 1 h and concentrated under vacuum. The residue was purified preparative HPLC (10%-100% methanol / 0.1% TFA in H₂O) to afford intermediate 13 as a yellow solid (280 mg, 86% for two steps) in TFA salt form. ¹H NMR (800 MHz, Methanol-*d*₄) δ 9.32 (s, 1H), 8.43 (d*, J =* 8.4 Hz, 2H), 7.50 (q, *J =* 7.9 Hz, 1H), 7.17 (d, *J =* 8.4 Hz, 2H), 7.02 (d, *J =* 8.5 Hz, 1H), 6.90 (t, *J =* 8.6 Hz, 1H), 3.81 (s, 3H), 3.53 (t, *J =* 5.2 Hz, 4H), 3.41 (t, *J* = 5.2 Hz, 4H). ESI m/z =405.3 [M + H⁺].

### Example 175

### Synthesis of HC90-33

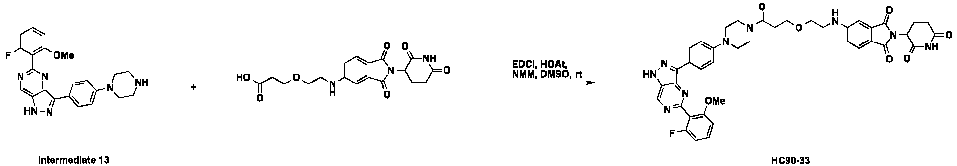

To a solution of Intermediate 13 (7.8 mg, 0.015 mmol, 1.0 equiv) in DMSO (1 mL) were added 3-(2-((2-(2,6-dioxopiperidin-3-yl)-1,3-dioxoisoindolin-5-yl)amino)ethoxy)propanoic acid (7.6 mg, 0.015 mmol, 1.0 equiv), EDCI (1-ethyl-3-(3-dimethylaminopropyl)carbodiimide) (4.2 mg, 0.0225 mmol, 1.5 equiv), HOAt (1-hydroxy-7-azabenzo-triazole) (3.1 mg, 0.0225 mmol, 1.5 equiv), and NMM (N-Methylmorpholine) (6.1 mg, 0.06 mmol, 4.0 equiv). After being stirred overnight at room temperature, the resulting mixture was purified by preparative HPLC (10%-100% methanol / 0.1% TFA in H₂O) to afford HC90-33 as yellow solid in TFA salt form (8.2 mg, 61%). ¹H NMR (600 MHz, Acetone-*d*₆) δ 9.88 (s, 1H), 9.41 (s, 1H), 8.45 (d, *J =* 8.9 Hz, 2H), 7.57 (d, *J=* 8.3 Hz, 1H), 7.50 (td, *J* = 8.5, 6.7 Hz, 1H), 7.11 (d, *J =* 8.9 Hz, 2H), 7.08 (d, *J* = 2.1 Hz, 1H), 7.02 (d, *J =* 8.5 Hz, 1H), 6.99 (dd, *J =* 8.3, 2.2 Hz, 1H), 6.91 (t, *J=* 8.7 Hz, 1H), 5.05 (dd, *J =* 12.6, 5.4 Hz, 1H), 3.90 - 3.71 (m, 11H), 3.45 (t, *J =* 5.2 Hz, 2H), 3.33 (dd, *J=* 6.4, 4.0 Hz, 2H), 3.28 (t, *J =* 5.3 Hz, 2H), 3.00 - 2.90 (m, 1H), 2.82 - 2.69 (m, 4H), 2.20 - 2.13 (m, 1H). HRMS calcd for C₄₀H₃₉FN₉O₇⁺ [M + H⁺] 776.2951, found 776.2940.

### Example 176

### Synthesis of HC90-34

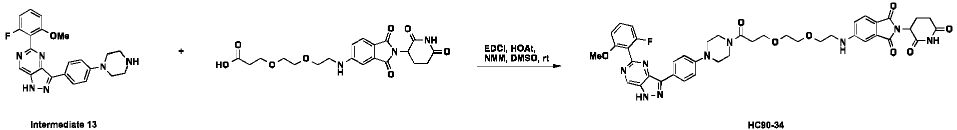

HC90-34 was synthesized following the standard procedure for preparing HC90-33 from Intermediate 13 (7.8 mg, 0.015 mmol, 1.0 equiv), 3-(2-(2-((2-(2,6-dioxopiperidin-3-yl)-1,3-dioxoisoindolin-5-yl)amino)ethoxy)ethoxy)propanoic acid (8.2 mg, 0.015 mmol, 1.0 equiv), EDCI (4.2 mg, 0.0225 mmol, 1.5 equiv), HOAt (3.1 mg, 0.0225 mmol, 1.5 equiv), and NMM (6.1 mg, 0.06 mmol, 4.0 equiv) in DMSO (1 mL). HC90-34 was obtained as yellow solid in TFA salt form (8.4 mg, 60%).¹H NMR (600 MHz, Acetone-*d*₆) δ 9.89 (s, 1H), 9.41 (s, 1H), 8.45 (d, *J=* 8.9 Hz, 2H), 7.55 (d, *J =* 8.3 Hz, 1H), 7.50 (td, *J =* 8.5, 6.7 Hz, 1H), 7.14 (d, *J =* 8.9 Hz, 2H), 7.10 (d, *J =* 2.1 Hz, 1H), 7.02 (d, *J* = 8.5 Hz, 1H), 6.98 (dd, *J =* 8.3, 2.2 Hz, 1H), 6.91 (t, *J* = 8.7 Hz, 1H), 5.05 (dd, *J =* 12.7, 5.4 Hz, 1H), 3.84 - 3.73 (m, 9H), 3.72 (t, *J* = 5.2 Hz, 2H), 3.66 - 3.57 (m, 4H), 3.44 (t, *J =* 5.3 Hz, 2H), 3.35 (t, *J =* 5.2 Hz, 2H), 3.28 (t, *J =* 5.3 Hz, 2H), 2.99 - 2.91 (m, 1H), 2.82 - 2.71 (m, 2H), 2.68 (t, *J =* 6.3 Hz, 2H), 2.19 - 2.14 (m, 1H). HRMS calcd for C₄₂H₄₃FN₉O₈⁺ [M + H⁺] 820.3213, found 820.3201.

### Example 177

### Synthesis of HC90-35

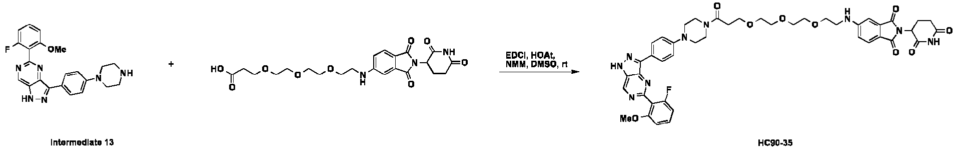

HC90-35 was synthesized following the standard procedure for preparing HC90-33 from Intermediate 13 (7.8 mg, 0.015 mmol, 1.0 equiv), 3-(2-(2-(2-((2-(2,6-dioxopiperidin-3-yl)-1,3-dioxoisoindolin-5-yl)amino)ethoxy)ethoxy)ethoxy)propanoic acid (8.9 mg, 0.015 mmol, 1.0 equiv), EDCI (4.2 mg, 0.0225 mmol, 1.5 equiv), HOAt (3.1 mg, 0.0225 mmol, 1.5 equiv), and NMM (6.1 mg, 0.06 mmol, 4.0 equiv) in DMSO (1 mL). HC90-35 was obtained as yellow solid in TFA salt form (9.6 mg, 74%). ¹H NMR (600 MHz, Acetone-*d*₆) δ 9.89 (s, 1H), 9.42 (s, 1H), 8.46 (d, *J=* 8.9 Hz, 2H), 7.55 (d, *J =* 8.3 Hz, 1H), 7.50 (td, *J =* 8.4, 6.6 Hz, 1H), 7.15 (d, *J =* 8.9 Hz, 2H), 7.06 (d, *J* = 2.2 Hz, 1H), 7.03 (d, *J* = 8.5 Hz, 1H), 6.95 (dd, *J =* 8.3, 2.2 Hz, 1H), 6.91 (t, *J =* 8.7 Hz, 1H), 5.06 (dd, *J =* 12.7, 5.5 Hz, 1H), 3.81 - 3.73 (m, 9H), 3.70 (t, *J =* 5.4 Hz, 2H), 3.60 (s, 8H), 3.44 (t, *J =* 5.4 Hz, 2H), 3.35 (t, *J =* 5.2 Hz, 2H), 3.28 (t, *J =* 5.2 Hz, 2H), 3.00 - 2.89 (m, 1H), 2.83 - 2.72 (m, 2H), 2.68 (t, *J* = 6.5 Hz, 2H), 2.25 - 2.13 (m, 1H). HRMS calcd for C₄₄H₄₇FN₉O₉⁺ [M + H⁺] 864.3475, found 864.3458.

### Example 178

### Synthesis of HC90-36

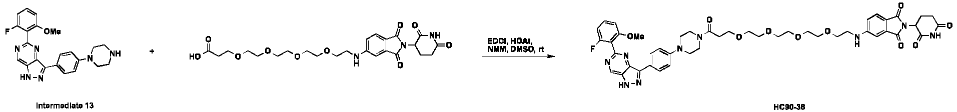

HC90-36 was synthesized following the standard procedure for preparing HC90-33 from Intermediate 13 (7.8 mg, 0.015 mmol, 1.0 equiv), 1-((2-(2,6-dioxopiperidin-3-yl)-1,3-dioxoisoindolin-5-yl)amino)-3,6,9,12-tetraoxapentadecan-15-oic acid (9.5 mg, 0.015 mmol, 1.0 equiv), EDCI (4.2 mg, 0.0225 mmol, 1.5 equiv), HOAt (3.1 mg, 0.0225 mmol, 1.5 equiv), and NMM (6.1 mg, 0.06 mmol, 4.0 equiv) in DMSO (1 mL). HC90-36 was obtained as yellow solid in TFA salt form (9.5 mg, 62%). ¹H NMR (600 MHz, Acetone-*d*₆) δ 9.89 (s, 1H), 9.41 (s, 1H), 8.46 (d, *J =* 8.9 Hz, 2H), 7.57 (d, *J =* 8.3 Hz, 1H), 7.50 (td, *J =* 8.5, 6.7 Hz, 1H), 7.13 (d, *J =* 9.0 Hz, 2H), 7.07 (d, *J =* 2.1 Hz, 1H, 7.02 (d, *J =* 8.5 Hz, 1H), 6.96 (dd, *J =* 8.3, 2.2 Hz, 1H), 6.93 - 6.89 (m, 1H), 5.06 (dd, *J =* 12.7, 5.4 Hz, 1H), 3.79 (s, 3H), 3.77 - 3.70 (m, 8H), 3.62 - 3.57 (m, 12H), 3.46 (t, *J =* 5.3 Hz, 2H), 3.34 (t, *J =* 5.1 Hz, 2H), 3.27 (t, *J =* 5.2 Hz, 2H), 3.00 - 2.92 (m, 1H), 2.82 - 2.72 (m, 2H), 2.67 (t, *J* = 6.5 Hz, 2H), 2.20 - 2.14 (m, 1H). HRMS calcd for C₄₆H₅₁FN₉O₁₀⁺ [M + H⁺] 908.3737, found 908.3721.

### Example 179

### Synthesis of HC90-37

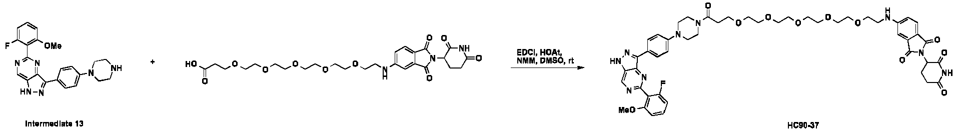

HC90-37 was synthesized following the standard procedure for preparing HC90-33 from Intermediate 13 (7.8 mg, 0.015 mmol, 1.0 equiv), 1-((2-(2,6-dioxopiperidin-3-yl)-1,3-dioxoisoindolin-5-yl)amino)-3,6,9,12,15-pentaoxaoctadecan-18-oic acid (10.2 mg, 0.015 mmol, 1.0 equiv), EDCI (4.2 mg, 0.0225 mmol, 1.5 equiv), HOAt (3.1 mg, 0.0225 mmol, 1.5 equiv), and NMM (6.1 mg, 0.06 mmol, 4.0 equiv) in DMSO (1 mL). HC90-37 was obtained as yellow solid in TFA salt form (8.9 mg, 56%). ¹H NMR (600 MHz, Acetone-*d*₆) δ 9.90 (s, 1H), 9.40 (s, 1H), 8.46 (d, *J =* 9.0 Hz, 2H), 7.57 (d, *J =* 8.3 Hz, 1H), 7.49 (td, *J =* 8.5, 6.7 Hz, 1H), 7.13 (d, *J =* 9.0 Hz, 2H), 7.07 (d, *J* = 2.2 Hz, 1H), 7.02 (d, *J* = 8.5 Hz, 1H), 6.97 (dd, *J =* 8.3, 2.2 Hz, 1H), 6.91 (t, *J =* 8.7 Hz, 1H), 5.06 (dd, *J =* 12.6, 5.5 Hz, 1H), 3.79 (s, 3H), 3.77 - 3.71 (m, 8H), 3.63 - 3.56 (m, 16H), 3.46 (t, *J =* 5.3 Hz, 2H), 3.34 (t, *J =* 5.2 Hz, 2H), 3.27 (t, *J =* 5.4 Hz, 2H), 3.02 - 2.92 (m, 1H), 2.84 - 2.73 (m, 2H), 2.68 (t, *J* = 6.4 Hz, 2H), 2.23 - 2.14 (m, 1H). HRMS calcd for C₄₈H₅₅FN₉O₁₁⁺ [M + H⁺] 952.4000, found 952.3988.

### Example 180

### Synthesis of HC90-41

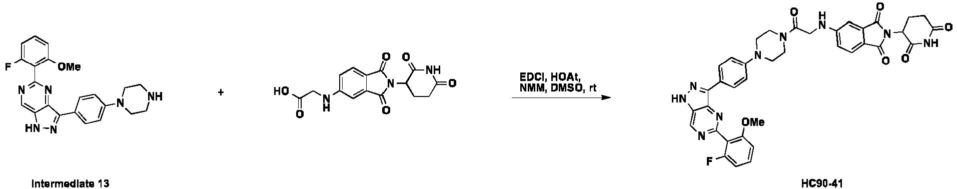

HC90-41 was synthesized following the standard procedure for preparing HC90-33 from Intermediate 13 (6.5 mg, 0.0125 mmol, 1.0 equiv), (2-(2,6-dioxopiperidin-3-yl)-1,3-dioxoisoindolin-5-yl)glycine (5.6 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-41 was obtained as yellow solid in TFA salt form (4.8 mg, 46%). ¹H NMR (800 MHz, Acetone-*d*₆) δ 9.40 (s, 1H), 8.47 (d, *J =* 8.2 Hz, 2H), 7.62 (d, *J =* 8.1 Hz, 1H), 7.50 (q, *J* = 8.3 Hz, 1H), 7.20 (s, 1H), 7.18 - 7.11 (m, 3H), 7.03 (d, *J* = 8.4 Hz, 1H), 6.91 (t, *J* = 8.5 Hz, 1H), 5.07 (dd, *J =* 12.9, 5.8 Hz, 1H), 4.30 (s, 3H), 3.91 - 3.76 (m, 6H), 3.43 (t, *J =* 5.1 Hz, 2H), 3.39 - 3.31 (m, 2H), 3.05 - 2.92 (m, 1H), 2.85 - 2.74 (m, 2H), 2.27 - 2.18 (m, 1H). HRMS calcd for C₃₇H₃₃FN₉O₆⁺ [M + H⁺] 718.2532, found 718.2514.

### Example 181

### Synthesis of HC90-42

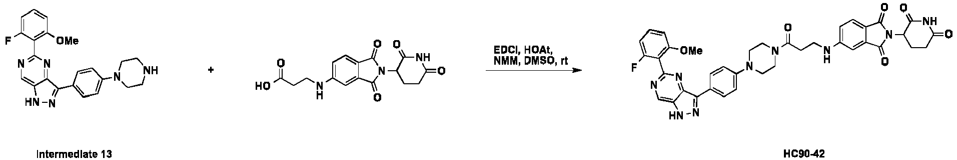

HC90-42 was synthesized following the standard procedure for preparing HC90-33 from Intermediate 13 (6.5 mg, 0.0125 mmol, 1.0 equiv), 3-((2-(2,6-dioxopiperidin-3-yl)-1,3-dioxoisoindolin-5-yl)amino)propanoic acid (5.7 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-42 was obtained as yellow solid in TFA salt form (3.3 mg, 28%). ¹H NMR (800 MHz, Acetone-*d*₆) δ 9.40 (s, 1H), 8.45 (d, *J =* 8.5 Hz, 2H), 7.59 (d, *J =* 8.3 Hz, 1H), 7.50 (q, *J* = 8.0 Hz, 1H), 7.11 (d, *J* = 8.5 Hz, 2H), 7.08 (s, 1H), 7.03 (d, *J* = 8.5 Hz, 1H), 7.00 (d, *J =* 8.6 Hz, 1H), 6.91 (t, *J =* 8.6 Hz, 1H), 5.06 (dd, *J =* 12.7, 5.7 Hz, 1H), 3.79 (s, 3H), 3.78 - 3.71 (m, 4H), 3.65 (t, *J =* 6.3 Hz, 2H), 3.35 - 3.29 (m, 2H), 3.27 (t, *J =* 5.4 Hz, 2H), 2.98 - 2.74 (m, 5H), 2.20 - 2.14 (m, 1H). HRMS calcd for C₃₈H₃₅FN₉O₆⁺ [M + H⁺] 732.2689, found 732.2672.

### Example 182

### Synthesis of HC90-43

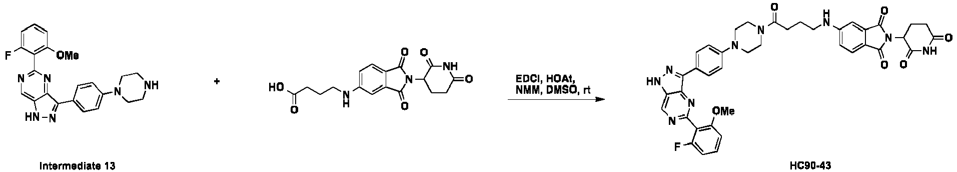

HC90-43 was synthesized following the standard procedure for preparing HC90-33 from Intermediate 13 (6.5 mg, 0.0125 mmol, 1.0 equiv), 4-((2-(2,6-dioxopiperidin-3-yl)-1,3-dioxoisoindolin-5-yl)amino)butanoic acid (5.9 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-43 was obtained as yellow solid in TFA salt form (3.6 mg, 33%). ¹H NMR (600 MHz, Acetone-*d*₆) δ 9.87 (s, 1H), 9.41 (d, *J =* 1.7 Hz, 1H), 8.45 (d, *J =* 7.7 Hz, 2H), 7.61 - 7.56 (m, 1H), 7.53 - 7.46 (m, 1H), 7.11 (d, *J =* 7.1 Hz, 2H), 7.06 - 7.01 (m, 2H), 6.99 - 6.95 (m, 1H), 6.91 (t, *J =* 8.6 Hz, 1H), 6.52 (s, 1H), 5.04 (dd, *J =* 12.7, 5.6 Hz, 1H), 3.79 (s, 3H), 3.77 - 3.72 (m, 4H), 3.42 - 3.37 (m, 2H), 3.32 - 3.25 (m, 4H), 2.81 - 2.71 (m, 3H), 2.62 (t, *J =* 6.9 Hz, 2H), 2.20 - 2.13 (m, 1H), 2.04 - 1.99 (m, 2H). HRMS calcd for C₃₉H₃₇FN₉O₆⁺ [M + H⁺] 746.2845, found 746.2832.

### Example 183

### Synthesis of HC90-44

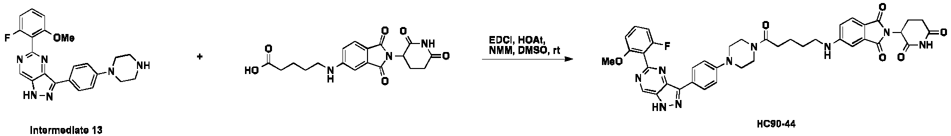

HC90-44 was synthesized following the standard procedure for preparing HC90-33 from Intermediate 13 (6.5 mg, 0.0125 mmol, 1.0 equiv), 5-((2-(2,6-dioxopiperidin-3-yl)-1,3-dioxoisoindolin-5-yl)amino)pentanoic acid (6.1 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-44 was obtained as yellow solid in TFA salt form (5.2 mg, 46%). ¹H NMR (800 MHz, Acetone-*d*₆) δ 9.40 (s, 1H), 8.46 (d, *J =* 8.3 Hz, 1H), 7.58 (d, *J =* 8.4 Hz, 1H), 7.50 (q, *J =* 8.3 Hz, 1H), 7.12 (d, *J =* 8.3 Hz, 2H), 7.06 - 7. 01 (m, 3H), 6.96 (d, *J =* 8.0 Hz, 1H), 6.91 (t, *J =* 8.5 Hz, 1H), 5.05 (dd, *J =* 12.6, 5.7 Hz, 1H), 3.79 (s, 3H), 3.76 - 3.69 (m, 4H), 3.40 - 3.30 (m, 4H), 3.26 (t, *J =* 5.6 Hz, 2H), 3.01 - 2.92 (m, 1H), 2.84 - 2.73 (m, 2H), 2.53 (t, *J =* 6.6 Hz, 2H), 2.21 - 2.15 (m, 1H), 1.84 - 1.74 (m, 4H). HRMS calcd for C₄₀H₃₉FN₉O₆⁺ [M + H⁺] 760.3002, found 760.2988.

### Example 184

### Synthesis of HC90-45

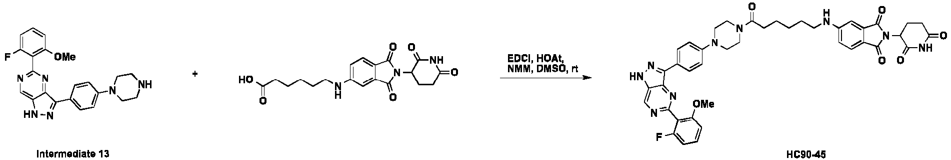

HC90-45 was synthesized following the standard procedure for preparing HC90-33 from Intermediate 13 (6.5 mg, 0.0125 mmol, 1.0 equiv), 6-((2-(2,6-dioxopiperidin-3-yl)-1,3-dioxoisoindolin-5-yl)amino)hexanoic acid (6.3 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-45 was obtained as yellow solid in TFA salt form (5.8 mg, 52%). ¹H NMR (800 MHz, Acetone-*d*₆) δ 9.40 (s, 1H), 8.46 (d, *J* = 8.4 Hz, 2H), 7.58 (d, *J* = 8.2 Hz, 1H), 7.54 - 7.46 (m, 1H), 7.12 (d, *J =* 8.3 Hz, 2H), 7.07 - 7.01 (m, 2H), 6.95 (d, *J* = 8.3 Hz, 1H), 6.91 (t, *J =* 8.4 Hz, 1H), 5.05 (dd, *J* = 12.8, 5.7 Hz, 1H), 3.79 (s, 3H), 3.76 - 3.71 (m, 4H), 3.36 - 3.29 (m, 4H), 3.29 - 3.24 (m, 2H), 3.02 - 2.93 (m, 1H), 2.82 - 2.73 (m, 2H), 2.46 (t, *J =* 7.3 Hz, 2H), 2.22 - 2.15 (m, 1H), 1.72 (dq, *J=* 28.5, 7.3 Hz, 4H), 1.52 (t, *J =* 7.7 Hz, 2H). HRMS calcd for C₄₁H₄₁FN₉O₆⁺ [M + H⁺] 774.3158, found 774.3144.

### Example 185

### Synthesis of HC90-46

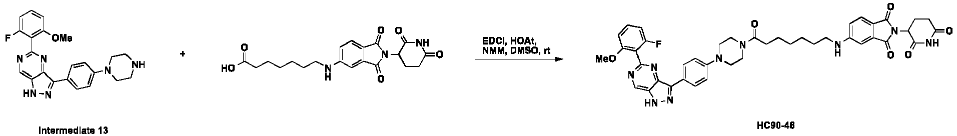

HC90-46 was synthesized following the standard procedure for preparing HC90-33 from Intermediate 13 (6.5 mg, 0.0125 mmol, 1.0 equiv), 7-((2-(2,6-dioxopiperidin-3-yl)-1,3-dioxoisoindolin-5-yl)amino)heptanoic acid (6.4 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-46 was obtained as yellow solid in TFA salt form (4.7 mg, 42%). ¹H NMR (600 MHz, Acetone-*d*₆) δ 9.87 (s, 1H), 9.41 (s, 1H), 8.46 (d, *J =* 8.9 Hz, 2H), 7.58 (d, *J =* 8.4 Hz, 1H), 7.50 (td, *J =* 8.5, 6.7 Hz, 1H), 7.13 (d, *J =* 9.0 Hz, 2H), 7.03 (dd, *J =* 5.3, 3.2 Hz, 2H), 6.94 (dd, *J* = 8.4, 2.2 Hz, 1H), 6.91 (t, *J* = 8.7 Hz, 1H), 5.05 (dd, *J =* 12.7, 5.5 Hz, 1H), 3.79 (s, 3H), 3.75 - 3.69 (m, 4H), 3.34 - 3.22 (m, 6H), 3.01 - 2.92 (m, 1H), 2.82 - 2.73 (m, 2H), 2.44 (t, *J =* 7.4 Hz, 2H), 2.22 - 2.14 (m, 1H), 1.72 (p, *J =* 7.2 Hz, 2H), 1.66 (p, *J =* 7.5 Hz, 2H), 1.51 (p, *J =* 7.2 Hz, 2H), 1.47 - 1.41 (m, 2H). HRMS calcd for C₄₂H₄₃FN₉O₆⁺ [M + H⁺] 788.3315, found 788.3302.

### Example 186

### Synthesis of HC90-47

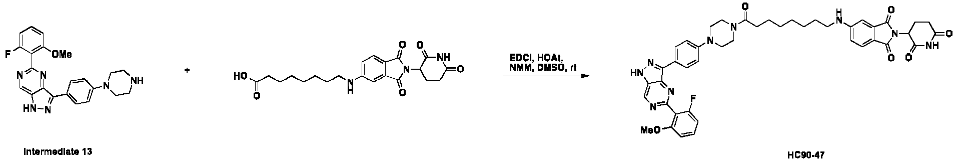

HC90-47 was synthesized following the standard procedure for preparing HC90-33 from Intermediate 13 (6.5 mg, 0.0125 mmol, 1.0 equiv), 8-((2-(2,6-dioxopiperidin-3-yl)-1,3-dioxoisoindolin-5-yl)amino)octanoic acid (6.6 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-47 was obtained as yellow solid in TFA salt form (4.2 mg, 37%). ¹H NMR (800 MHz, Acetone-*d*₆) δ 9.40 (s, 1H), 8.46 (d, *J* = 8.5 Hz, 2H), 7.57 (d, *J* = 8.2 Hz, 1H), 7.50 (q, *J =* 8.0 Hz, 1H), 7.13 (d, *J =* 8.5 Hz, 2H), 7.07 - 7.01 (m, 2H), 6.98 - 6.89 (m, 2H), 5.06 (dd, *J =* 12.8, 5.7 Hz, 1H), 3.79 (s, 3H), 3.76 - 3.70 (m, 4H), 3.36 - 3.24 (m, 6H), 3.01 - 2.93 (m, 1H), 2.81 - 2.73 (m, 2H), 2.43 (t, *J* = 7.4 Hz, 2H), 2.22 - 2.15 (m, 1H), 1.71 (t, *J* = 7.5 Hz, 2H), 1.64 (t, *J* = 7.3 Hz, 2H), 1.48 (t, *J* = 7.6 Hz, 2H), 1.45 - 1.39 (m, 4H). HRMS calcd for C₄₃H₄₅FN₉O₆⁺ [M + H⁺] 802.3471, found 802.3457.

### Example 187

### Synthesis of HC90-49

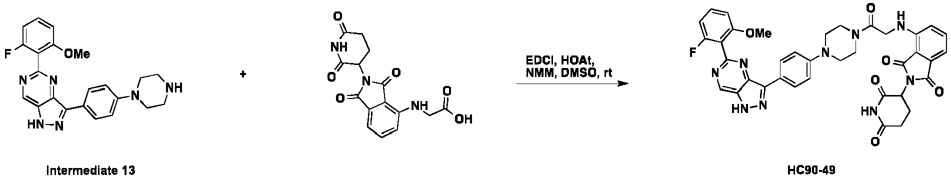

HC90-49 was synthesized following the standard procedure for preparing HC90-33 from Intermediate 13 (6.5 mg, 0.0125 mmol, 1.0 equiv), (2-(2,6-dioxopiperidin-3-yl)-1,3-dioxoisoindolin-4-yl)glycine (4.2 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-49 was obtained as yellow solid in TFA salt form (5.3 mg, 51%). ¹H NMR (800 MHz, Acetone-*d*₆) δ 9.40 (s, 1H), 8.47 (d, *J =* 8.4 Hz, 2H), 7.62 (t, *J =* 7.7 Hz, 1H), 7.50 (q, *J =* 7.9 Hz, 1H), 7.21 - 7.12 (m, 3H), 7.09 (d, *J =* 6.9 Hz, 1H), 7.03 (d, *J =* 8.4 Hz, 1H), 6.92 (t, *J* = 8.6 Hz, 1H), 5.11 (dd, *J =* 12.6, 5.7 Hz, 1H), 4.35 (s, 2H), 3.87 - 3.77 (m, 7H), 3.43 (t, *J =* 5.5 Hz, 2H), 3.36 (t, *J =* 5.5 Hz, 2H), 3.03 - 2.96 (m, 1H), 2.88 - 2.76 (m, 2H), 2.31 - 2.20 (m, 1H). HRMS calcd for C₃₇H₃₃FN₉O₆⁺ [M + H⁺] 718.2532, found 718.2520.

### Example 188

### Synthesis of HC90-50

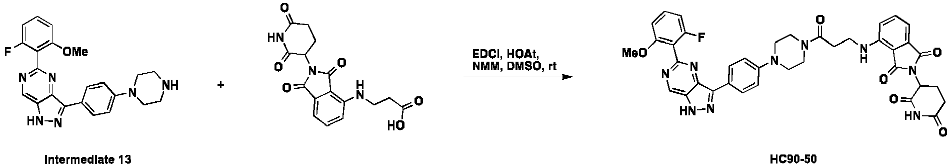

HC90-50 was synthesized following the standard procedure for preparing HC90-33 from Intermediate 13 (6.5 mg, 0.0125 mmol, 1.0 equiv), 3-((2-(2,6-dioxopiperidin-3-yl)-1,3-dioxoisoindolin-4-yl)amino)propanoic acid (4.4 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-50 was obtained as yellow solid in TFA salt form (6.4 mg, 61%). ¹H NMR (800 MHz, Acetone-*d*₆) δ 9.40 (s, 1H), 8.45 (d, *J =* 8.5 Hz, 2H), 7.61 (t, *J =* 7.8 Hz, 1H), 7.54 - 7.46 (m, 1H), 7.18 (d*, J =* 8.6 Hz, 1H), 7.12 (d, *J* = 8.4 Hz, 2H), 7.08 - 7.00 (m, 2H), 6.91 (t, *J* = 8.6 Hz, 1H), 5.06 (dd, *J* = 12.9, 5.7 Hz, 1H), 3.84 - 3.69 (m, 9H), 3.31 (t, *J* = 5.4 Hz, 2H), 3.27 (t, *J =* 5.5 Hz, 2H), 3.00 - 2.91 (m, 1H), 2.85 (t, *J =* 6.3 Hz, 2H), 2.81 - 2.72 (m, 2H), 2.26 - 2.16 (m, 1H). HRMS calcd for C₃₈H₃₅FN₉O₆⁺ [M + H⁺] 732.2689, found 732.2671.

### Example 189

### Synthesis of HC90-51

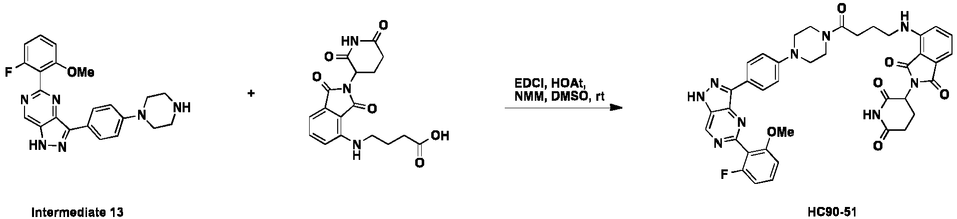

HC90-51 was synthesized following the standard procedure for preparing HC90-33 from Intermediate 13 (6.5 mg, 0.0125 mmol, 1.0 equiv), 4-((2-(2,6-dioxopiperidin-3-yl)-1,3-dioxoisoindolin-4-yl)amino)butanoic acid (4.5 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-51 was obtained as yellow solid in TFA salt form (6.8 mg, 63%). ¹H NMR (800 MHz, Acetone-*d*₆) δ 9.40 (s, 1H), 8.46 (d, *J =* 8.4 Hz, 2H), 7.61 (t, *J =* 7.8 Hz, 1H), 7.54 - 7.45 (m, 1H), 7.23 (d, *J* = 8.6 Hz, 1H), 7.12 (d, *J =* 8.4 Hz, 2H), 7.07 - 7.00 (m, 2H), 6.91 (t, *J =* 8.6 Hz, 1H), 5.08 (dd, *J* = 12.8, 5.8 Hz, 1H), 3.79 (s, 3H), 3.78 - 3.71 (m, 4H), 3.48 (t, *J =* 7.2 Hz, 2H), 3.34 - 3.23 (m, 4H), 3.00 - 2.91 (m, 1H), 2.84 - 2.77 (m, 2H), 2.59 (t, *J* = 6.8 Hz, 2H), 2.24 - 2.19 (m, 1H), 2.02 (t, *J =* 7.0 Hz, 2H). HRMS calcd for C₃₉H₃₇FN₉O₆⁺ [M + H⁺] 746.2845, found 746.2828.

### Example 190

### Synthesis of HC90-52

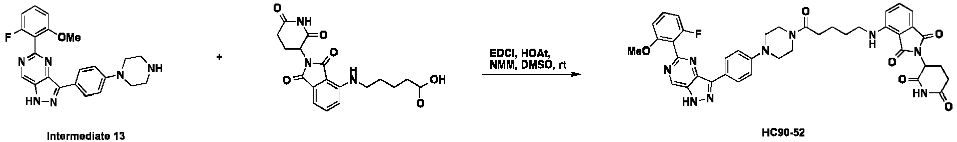

HC90-52 was synthesized following the standard procedure for preparing HC90-33 from Intermediate 13 (6.5 mg, 0.0125 mmol, 1.0 equiv), 5-((2-(2,6-dioxopiperidin-3-yl)-1,3-dioxoisoindolin-4-yl)amino)pentanoic acid (4.7 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-52 was obtained as yellow solid in TFA salt form (6.2 mg, 57%). ¹H NMR (800 MHz, Acetone-*d*₆) δ 9.40 (s, 1H), 8.46 (d, *J =* 8.4 Hz, 2H), 7.60 (t, *J =* 7.8 Hz, 1H), 7.50 (q, *J =* 8.4 Hz, 1H), 7.17 - 7.09 (m, 3H), 7.07 - 7.00 (m, 2H), 6.91 (t, *J=* 8.7 Hz, 1H), 5.07 (dd, *J =* 12.7, 5.7 Hz, 1H), 3.79 (s, 3H), 3.76 - 3.70 (m, 4H), 3.48 - 3.42 (m, 2H), 3.35 - 3.29 (m, 2H), 3.29 - 3.22 (m, 2H), 3.00 - 2.92 (m, 1H), 2.81 - 2.74 (m, 2H), 2.57 - 2.50 (m, 2H), 2.24 - 2.19 (m, 1H), 1.84 - 1.75 (m, 4H). HRMS calcd for C₄₀H₃₉FN₉O₆⁺ [M + H⁺] 760.3002, found 760.2988.

### Example 191

### Synthesis of HC90-53

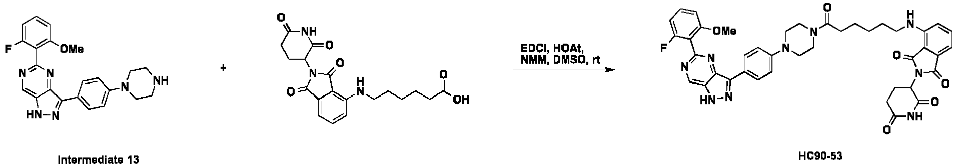

HC90-53 was synthesized following the standard procedure for preparing HC90-33 from Intermediate 13 (6.5 mg, 0.0125 mmol, 1.0 equiv), 6-((2-(2,6-dioxopiperidin-3-yl)-1,3-dioxoisoindolin-4-yl)amino)hexanoic acid (4.9 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-53 was obtained as yellow solid in TFA salt form (4.9 mg, 44%). ¹H NMR (800 MHz, Acetone-*d*₆) δ 9.40 (s, 1H), 8.46 (d, *J =* 8.4 Hz, 2H), 7.60 (t, *J =* 7.8 Hz, 1H), 7.50 (q, *J =* 8.3 Hz, 1H), 7.19 - 7.10 (m, 3H), 7.03 (t, *J =* 8.2 Hz, 2H), 6.91 (t, *J =* 8.6 Hz, 1H), 5.08 (dd, *J =* 12.8, 5.7 Hz, 1H), 3.79 (s, 3H), 3.75 - 3.70 (m, 4H), 3.41 (t, *J* = 7.1 Hz, 2H), 3.35 - 3.29 (m, 2H), 3.28 - 3.23 (m, 2H), 3.00 - 2.92 (m, 1H), 2.83 - 2.75 (m, 2H), 2.47 (t, *J =* 7.4 Hz, 2H), 2.27 - 2.19 (m, 1H), 1.80 - 1.70 (m, 4H), 1.57 - 1.50 (m, 2H). HRMS calcd for C₄₁H₄₁FN₉O₆⁺ [M + H⁺] 774.3158, found 774.3141.

### Example 192

### Synthesis of HC90-54

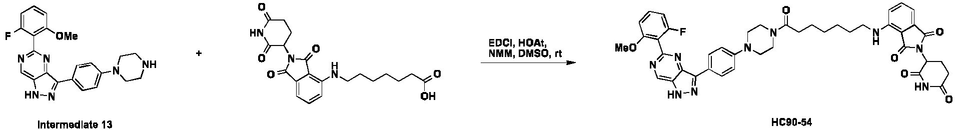

HC90-54 was synthesized following the standard procedure for preparing HC90-33 from Intermediate 13 (6.5 mg, 0.0125 mmol, 1.0 equiv), 7-((2-(2,6-dioxopiperidin-3-yl)-1,3-dioxoisoindolin-4-yl)amino)heptanoic acid (5.1 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-54 was obtained as yellow solid in TFA salt form (5.5 mg, 49%). ¹H NMR (800 MHz, Acetone-*d*₆) δ 9.40 (s, 1H), 8.46 (d, *J =* 8.5 Hz, 2H), 7.60 (t, *J =* 7.8 Hz, 1H), 7.50 (d, *J* = 7.7 Hz, 1H), 7.16 - 7.09 (m, 3H), 7.07 - 7.01 (m, 2H), 6.91 (t, *J* = 8.6 Hz, 1H), 5.08 (dd, *J =* 12.8, 5.8 Hz, 1H), 3.79 (s, 3H), 3.75 - 3.70 (m, 4H), 3.40 (t, *J =* 7.1 Hz, 2H), 3.34 - 3.29 (m, 2H), 3.28 - 3.23 (m, 2H), 3.00 - 2.94 (m, 1H), 2.81 - 2.76 (m, 2H), 2.44 (t, *J =* 7.4 Hz, 2H), 2.23 - 2.20 (m, 1H), 1.77 - 1.71 (m, 2H), 1.67 (t, *J* = 7.5 Hz, 2H), 1.54 - 1.45 (m, 4H). HRMS calcd for C₄₂H₄₃FN₉O₆⁺ [M + H⁺] 788.3315, found 788.3301.

### Example 193

### Synthesis of HC90-55

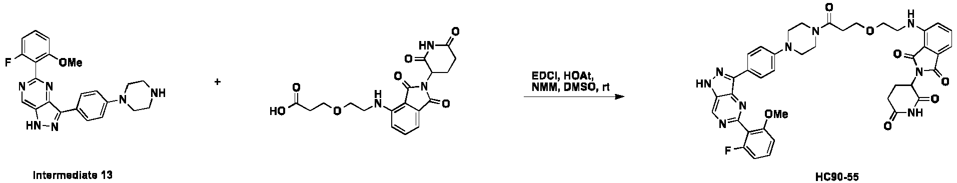

HC90-55 was synthesized following the standard procedure for preparing HC90-33 from Intermediate 13 (6.5 mg, 0.0125 mmol, 1.0 equiv), 3-(2-((2-(2,6-dioxopiperidin-3-yl)-1,3-dioxoisoindolin-4-yl)amino)ethoxy)propanoic acid (4.9 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-55 was obtained as yellow solid in TFA salt form (5.4 mg, 49%). ¹H NMR (800 MHz, Acetone-*d*₆) δ 9.40 (s, 1H), 8.43 (d, *J =* 8.4 Hz, 2H), 7.58 (t, *J =* 7.8 Hz, 1H), 7.55 - 7.48 (m, 1H), 7.13 (d, *J* = 8.6 Hz, 1H), 7.07 (d, *J* = 8.3 Hz, 2H), 7.03 (dd, *J =* 7.9, 4.3 Hz, 2H), 6.92 (t, *J =* 8.5 Hz, 1H), 5.07 (dd, *J =* 12.9, 5.7 Hz, 1H), 3.83 (t, *J =* 6.4 Hz, 2H), 3.80 (s, 3H), 3.77 - 3.72 (m, 6H), 3.59 - 3.54 (m, 2H), 3.32 - 3.23 (m, 4H), 2.96 - 2.90 (m, 1H), 2.82 - 2.69 (m, 4H), 2.23 - 2.18 (m, 1H). HRMS calcd for C₄₀H₃₉FN₉O₇⁺ [M + H⁺] 776.2951, found 776.2935.

### Example 194

### Synthesis of HC90-56

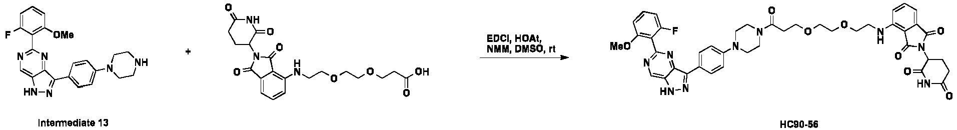

HC90-56 was synthesized following the standard procedure for preparing HC90-33 from Intermediate 13 (6.5 mg, 0.0125 mmol, 1.0 equiv), 3-(2-(2-((2-(2,6-dioxopiperidin-3-yl)-1,3-dioxoisoindolin-4-yl)amino)ethoxy)ethoxy)propanoic acid (5.5 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-56 was obtained as yellow solid in TFA salt form (6.7 mg, 57%). ¹H NMR (600 MHz, Acetone-*d*₆) δ 9.94 (s, 1H), 9.39 (s, 1H), 8.47 - 8.41 (m, 2H), 7.61 - 7.52 (m, 1H), 7.52 - 7.46 (m, 1H), 7.17 - 7.06 (m, 2H), 7.02 (dd, *J =* 7.8, 4.3 Hz, 2H), 6.91 (t, *J* = 8.4 Hz, 1H), 6.62 (s, 1H), 5.19 - 5.05 (m, 1H), 3.79 (s, 3H), 3.77 - 3.69 (m, 8H), 3.68 - 3.59 (m, 4H), 3.54 - 3.47 (m, 2H), 3.38 - 3.19 (m, 4H), 2.99 - 2.92 (m, 1H), 2.84 - 2.71 (m, 2H), 2.69 - 2.62 (m, 2H), 2.28 - 2.17 (m, 1H). HRMS calcd for C₄₂H₄₃FN₉O₈⁺ [M + H⁺] 820.3213, found 820.3201.

### Example 195

### Synthesis of HC90-57

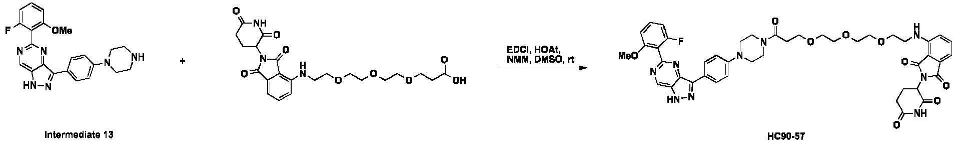

HC90-57 was synthesized following the standard procedure for preparing HC90-33 from Intermediate 13 (6.5 mg, 0.0125 mmol, 1.0 equiv), 3-(2-(2-(2-((2-(2,6-dioxopiperidin-3-yl)-1,3-dioxoisoindolin-4-yl)amino)ethoxy)ethoxy)ethoxy)propanoic acid (6.0 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-57 was obtained as yellow solid in TFA salt form (6.1 mg, 57%). ¹H NMR (800 MHz, Acetone-*d*₆) δ 9.39 (s, 1H), 8.45 (d, *J =* 8.5 Hz, 2H), 7.56 (t, *J =* 7.8 Hz, 1H), 7.50 (q, *J =* 8.0 Hz, 1H), 7.16 - 7.08 (m, 3H), 7.03 (t, *J =* 7.4 Hz, 2H), 6.91 (t, *J =* 8.6 Hz, 1H), 5.09 (dd, *J =* 13.0, 5.7 Hz, 1H), 3.79 (s, 3H), 3.78 - 3.70 (m, 8H), 3.65 - 3.57 (m, 8H), 3.50 (t, *J =* 5.4 Hz, 2H), 3.37 - 3.30 (m, 2H), 3.25 (t, *J =* 5.4 Hz, 2H), 3.01 - 2.94 (m, 1H), 2.84 - 2.74 (m, 2H), 2.66 (t, *J* = 6.5 Hz, 2H), 2.26 - 2.20 (m, 1H). HRMS calcd for C₄₄H₄₇FN₉O₉⁺ [M + H⁺] 864.3475, found 864.3459.

### Example 196

### Synthesis of HC90-58

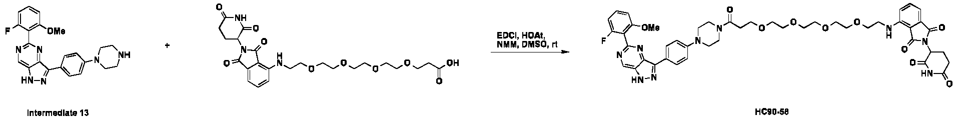

HC90-58 was synthesized following the standard procedure for preparing HC90-33 from Intermediate 13 (6.5 mg, 0.0125 mmol, 1.0 equiv), 1-((2-(2,6-dioxopiperidin-3-yl)-1,3-dioxoisoindolin-4-yl)amino)-3,6,9,12-tetraoxapentadecan-15-oic acid (6.6 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-58 was obtained as yellow solid in TFA salt form (6.5 mg, 51%). ¹H NMR (600 MHz, Acetone-*d*₆) δ 9.95 (s, 1H), 9.40 (s, 1H), 8.45 (d, *J* = 8.8 Hz, 2H), 7.59 - 7.55 (m, 1H), 7.53 - 7.47 (m, 1H), 7.14 - 7.09 (m, 2H), 7.07 - 7.01 (m, 2H), 6.91 (t, *J =* 8.7 Hz, 1H), 6.61 (s, 1H), 5.08 (dd, *J =* 12.8, 5.5 Hz, 1H), 3.79 (s, 3H), 3.77 - 3.48 (m, 22H), 3.33 (d, *J =* 5.9 Hz, 2H), 3.24 (t, *J =* 5.3 Hz, 2H), 3.01 - 2.92 (m, 1H), 2.84 - 2.74 (m, 2H), 2.68 (t, *J =* 6.4 Hz, 2H), 2.29 - 2.19 (m, 1H). HRMS calcd for C₄₆H₅₁FN₉O₁₀⁺ [M + H⁺] 908.3737, found 908.3721.

### Example 197

### Synthesis of HC90-59

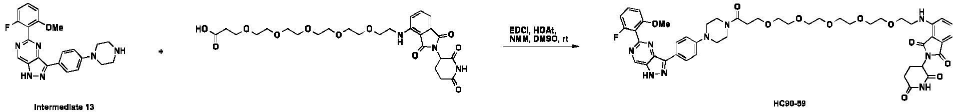

HC90-59 was synthesized following the standard procedure for preparing HC90-33 from Intermediate 13 (6.5 mg, 0.0125 mmol, 1.0 equiv), 1-((2-(2,6-dioxopiperidin-3-yl)-1,3-dioxoisoindolin-4-yl)amino)-3,6,9,12,15-pentaoxaoctadecan-18-oic acid (6.6 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-59 was obtained as yellow solid in TFA salt form (5.9 mg, 44%). ¹H NMR (800 MHz, Acetone-*d*₆) δ 9.40 (s, 1H), 8.45 (d, *J =* 8.5 Hz, 2H), 7.58 (t, *J =* 7.8 Hz, 1H), 7.54 - 7.48 (m, 1H), 7.12 (t, *J =* 9.7 Hz, 3H), 7.07 - 7.02 (m, 2H), 6.91 (t, *J =* 8.4 Hz, 1H), 5.09 (dd, *J =* 12.6, 5.7 Hz, 1H), 3.79 (s, 3H), 3.78 - 3.72 (m, 8H), 3.69 - 3.52 (m, 18H), 3.37 - 3.31 (m, 2H), 3.30 - 3.24 (m, 2H), 3.01 - 2.92 (m, 1H), 2.85 - 2.75 (m, 2H), 2.69 (t, *J =* 6.4 Hz, 2H), 2.27 - 2.20 (m, 1H). HRMS calcd for C₄₈H₅₅FN₉O₁₁⁺ [M + H⁺] 952.4000, found 952.3983.

### Example 198

### Synthesis of HC90-61

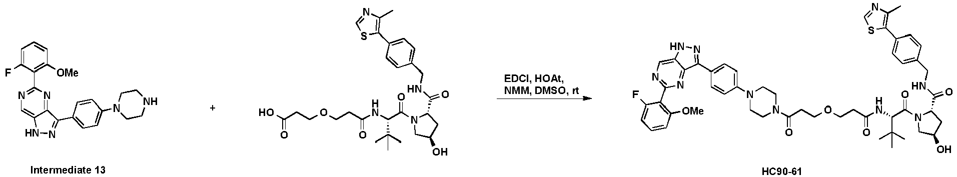

HC90-61 was synthesized following the standard procedure for preparing HC90-33 from Intermediate 13 (6.5 mg, 0.0125 mmol, 1.0 equiv), 3-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)propanoic acid (7.2 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-61 was obtained as yellow solid in TFA salt form (7.1 mg, 53%). ¹H NMR (800 MHz, Methanol-*d*₄) δ 9.29 (s, 1H), 8.84 (s, 1H), 8.36 (d, *J =* 8.3 Hz, 2H), 7.49 (q, *J =* 7.8 Hz, 1H), 7.40 (d, *J =* 7.9 Hz, 2H), 7.34 (d, *J =* 7.8 Hz, 2H), 7.08 (d, *J =* 8.5 Hz, 2H), 7.00 (d, *J =* 8.4 Hz, 1H), 6.89 (t, *J =* 8.5 Hz, 1H), 4.68 (s, 1H), 4.57 (t, *J=* 8.4 Hz, 1H), 4.51 (s, 2H), 4.48 (d, *J* = 15.4 Hz, 1H), 4.32 (d, *J* = 15.4 Hz, 1H), 3.90 (d, *J =* 11.1 Hz, 1H), 3.83 - 3.70 (m, 10H), 3.31 - 3.23 (m, 2H), 2.74 (t, *J =* 6.2 Hz, 2H), 2.59 - 2.50 (m, 2H), 2.43 (s, 3H), 2.26 - 2.19 (m, 1H), 2.08 (ddd, *J* = 13.2, 9.1, 4.6 Hz, 1H), 1.05 (s, 9H). HRMS calcd for C₅₀H₅₈FN₁₀O₇S⁺ [M+H⁺] 961.4189, found 961.4172.

### Example 199

### Synthesis of HC90-66

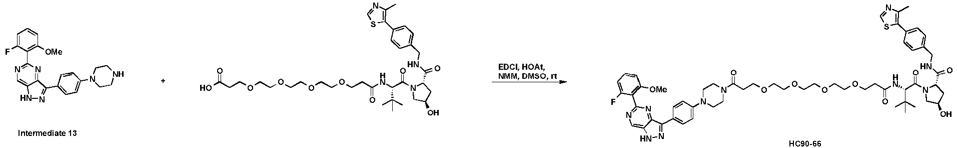

HC90-66 was synthesized following the standard procedure for preparing HC90-33 from Intermediate 13 (6.5 mg, 0.0125 mmol, 1.0 equiv), (*S*)-18-((2*S,*4*R*)-4-hydroxy-2-((4-(4-methylthiazol-5-yl)benzyl)carbamoyl)pyrrolidine-1-carbonyl)-19,19-dimethyl-16-oxo-4,7,10,13-tetraoxa-17-azaicosanoic acid (8.9 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-66 was obtained as yellow solid in TFA salt form (7.5 mg, 50%).
¹H NMR (800 MHz, Methanol-*d*₄) δ 9.29 (s, 1H), 8.90 (s, 1H), 8.38 (d, *J* = 8.3 Hz, 2H), 7.53 - 7.48 (m, 1H), 7.47 (d, *J =* 8.0 Hz, 2H), 7.41 (d, *J =* 7.8 Hz, 2H), 7.12 (d, *J =* 8.4 Hz, 3H), 7.00 (d, *J =* 8.4 Hz, 1H), 6.89 (t, *J* = 8.6 Hz, 1H), 4.65 (s, 1H), 4.60 - 4.57 (m, 1H), 4.55 (d, *J* = 15.3 Hz, 1H), 4.51 (s, 1H), 4.35 (d, *J* = 15.4 Hz, 1H), 3.90 (d, *J* = 11.2 Hz, 2H), 3.85 - 3.76 (m, 6H), 3.72 - 3.52 (m, 21H), 2.72 (t, *J* = 6.1 Hz, 2H), 2.55 (ddd, *J* = 13.7, 7.8, 4.7 Hz, 1H), 2.49 - 2.44 (m, 4H), 2.23 (t, *J* = 10.4 Hz, 1H), 2.09 (ddd, *J* = 13.2, 8.8, 4.4 Hz, 1H), 1.04 (s, 9H). HRMS calcd for C₅₆H₇₀FN₁₀O₁₀S⁺ [M + H⁺] 1093.4976, found 1093.4960.

### Example 200

### Synthesis of HC90-69

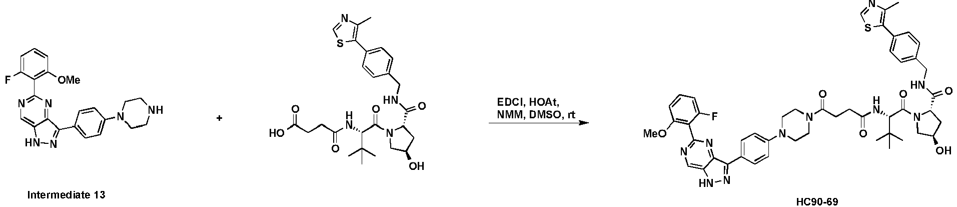

HC90-69 was synthesized following the standard procedure for preparing HC90-33 from Intermediate 13 (6.5 mg, 0.0125 mmol, 1.0 equiv), 4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutanoic acid (6.7 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-69 was obtained as yellow solid in TFA salt form (6.8 mg, 53%). ¹H NMR (800 MHz, Methanol-*d*₄) δ 9.29 (s, 1H), 8.90 (s, 1H), 8.37 (d, *J=* 8.4 Hz, 2H), 7.48 (d, *J* = 8.3 Hz, 1H), 7.45 (d, *J* = 8.1 Hz, 2H), 7.41 (d, *J* = 7.8 Hz, 2H), 7.11 (d, *J* = 8.5 Hz, 2H), 7.01 (d, *J* = 8.5 Hz, 1H), 6.90 (t, *J* = 8.6 Hz, 1H), 4.62 (s, 1H), 4.59 - 4.49 (m, 3H), 4.36 (d, *J* = 15.4 Hz, 1H), 3.92 (d, *J* = 11.0 Hz, 1H), 3.83 - 3.77 (m, 4H), 3.75 - 3.71 (m, 4H), 3.33 - 3.26 (m, 4H), 2.79- 2.70 (m, 2H), 2.69 - 2.58 (m, 2H), 2.49 (s, 3H), 2.28 - 2.20 (m, 1H), 2.13 - 2.04 (m, 1H), 1.06 (s, 9H). HRMS calcd for C₄₈H₅₄FN₁₀O₆S⁺ [M + H⁺] 917.3927, found 917.3911.

### Example 201

### Synthesis of HC90-70

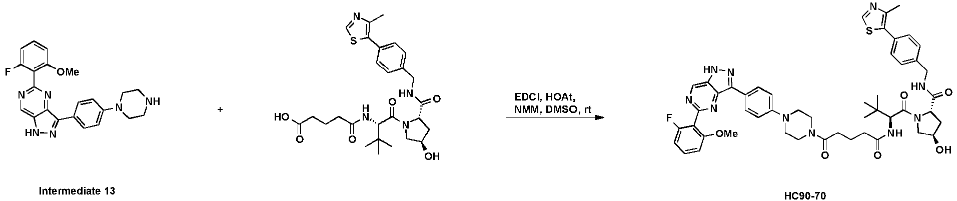

HC90-70 was synthesized following the standard procedure for preparing HC90-33 from Intermediate 13 (6.5 mg, 0.0125 mmol, 1.0 equiv), 5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentanoic acid (6.8 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-70 was obtained as yellow solid in TFA salt form (6.8 mg, 52%). ¹H NMR (800 MHz, Methanol-*d*₄) δ 9.29 (s, 1H), 8.89 (s, 1H), 8.36 (d, *J* = 8.4 Hz, 2H), 7.51 - 7.48 (m, 1H), 7.44 (d, *J* = 7.8 Hz, 2H), 7.38 (d, *J =* 7.8 Hz, 2H), 7.08 (d, *J =* 8.4 Hz, 2H), 7.01 (d, *J =* 8.4 Hz, 1H), 6.90 (t, *J* = 8.5 Hz, 1H), 4.64 (s, 1H), 4.57-4.49 (m, 3H), 4.33 (d, *J* = 15.5 Hz, 1H), 3.96 (d, *J* = 11.2 Hz, 1H), 3.86 - 3.79 (m, 4H), 3.76 - 3.68 (m, 4H), 3.32 - 3.26 (m, 4H), 2.53 - 2.47 (m, 2H), 2.46 (s, 3H), 2.38 (t, *J* = 7.1 Hz, 2H), 2.24 (dd, *J* = 12.2, 7.6 Hz, 1H), 2.12 - 2.05 (m, 1H), 2.00 - 1.90 (m, 2H), 1.07 (s, 9H). HRMS calcd for C₄₉H₅₆FN₁₀O₆S⁺ [M + H⁺] 931.4084, found 931.4071.

### Example 202

### Synthesis of HC90-71

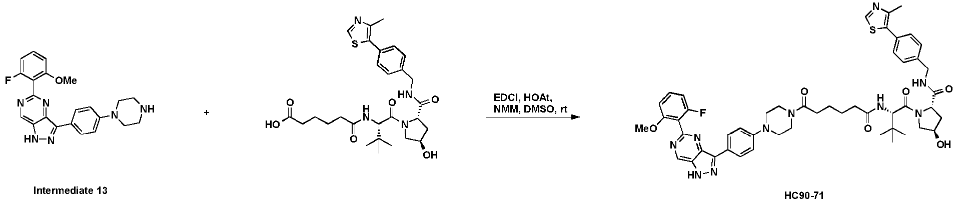

HC90-71 was synthesized following the standard procedure for preparing HC90-33 from Intermediate 13 (6.5 mg, 0.0125 mmol, 1.0 equiv), 6-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-6-oxohexanoic acid (7.0 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-71 was obtained as yellow solid in TFA salt form (9.2 mg, 69%). ¹H NMR (800 MHz, Methanol-*d*₄) δ 9.29 (s, 1H), 8.90 (s, 1H), 8.38 (d, *J* = 8.4 Hz, 2H), 7.54 - 7.47 (m, 1H), 7.45 (d, *J* = 7.7 Hz, 2H), 7.39 (d, *J =* 7.8 Hz, 2H), 7.11 (d, *J =* 8.4 Hz, 2H), 7.00 (d, *J =* 8.4 Hz, 1H), 6.89 (t, *J* = 8.5 Hz, 1H), 4.64 (s, 1H), 4.57 (t, *J* = 8.4 Hz, 1H), 4.54 - 4.50 (m, 2H), 4.35 (d, *J* = 15.4 Hz, 1H), 3.92 (d, *J* = 10.8 Hz, 1H), 3.83 - 3.78 (m, 4H), 3.78 - 3.69 (m, 4H), 3.32 - 3.25 (m, 4H), 2.50 - 2.44 (m, 5H), 2.38 - 2.30 (m, 2H), 2.23 (dd, *J* = 13.3, 7.3 Hz, 1H), 2.08 (ddd, *J* = 13.7, 9.3, 4.9 Hz, 1H), 1.74 - 1.62 (m, 4H), 1.05 (s, 9H). HRMS calcd for C₅₀H₅₈FN₁₀O₆S⁺ [M + H⁺] 945.4240, found 945.4222.

### Example 203

### Synthesis of HC90-72

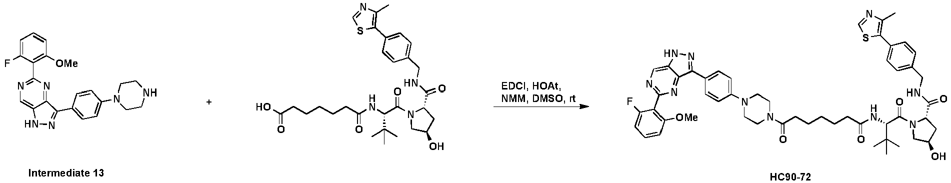

HC90-72 was synthesized following the standard procedure for preparing HC90-33 from Intermediate 13 (6.5 mg, 0.0125 mmol, 1.0 equiv), 7-(((*S*)-1-((2*S*,4*S*)-4-hydroxy-2-((4-(4-methylthiazol-5-yl)benzyl)carbamoyl)pyrrolidin-1-yl)-3,3-dimethyl-1-oxobutan-2-yl)amino)-7-oxoheptanoic acid (7.2 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-72 was obtained as yellow solid in TFA salt form (7.2 mg, 54%). ¹H NMR (800 MHz, Methanol-*d*₄) δ 9.30 (s, 1H), 8.89 (s, 1H), 8.38 (d, *J* = 8.4 Hz, 2H), 7.51 - 7.45 (m, 3H), 7.41 (d, *J* = 7.9 Hz, 2H), 7.11 (d, *J* = 8.6 Hz, 2H), 7.00 (d, *J* = 8.4 Hz, 1H), 6.89 (t, *J* = 8.6 Hz, 1H), 4.65 (s, 1H), 4.58 (t, *J* = 8.3 Hz, 1H), 4.54 (d, *J* = 15.4 Hz, 1H), 4.52 - 4.50 (m, 1H), 4.36 (d, *J* = 15.3 Hz, 1H), 3.95 - 3.90 (m, 1H), 3.86 - 3.78 (m, 4H), 3.78 - 3.71 (m, 4H), 3.32 - 3.23 (m, 4H), 2.49 - 2.44 (m, 5H), 2.36 - 2.28 (m, 2H), 2.23 (dd, *J* = 13.4, 7.4 Hz, 1H), 2.12 - 2.06 (m, 1H), 1.67 (hept, *J* = 7.1 Hz, 4H), 1.41 (p, *J* = 7.8 Hz, 2H), 1.05 (s, 9H). HRMS calcd for C₅₁H₆₀FN₁₀O₆S⁺ [M + H⁺] 959.4397, found 959.4381.

### Example 204

### Synthesis of HC90-73

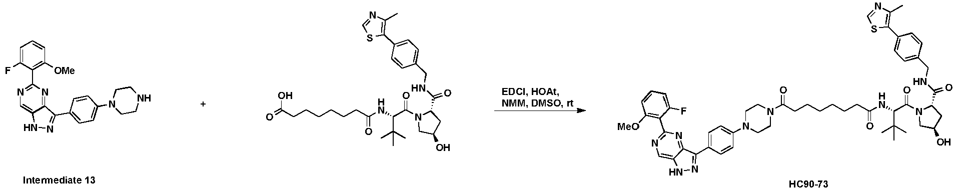

HC90-73 was synthesized following the standard procedure for preparing HC90-33 from Intermediate 13 (6.5 mg, 0.0125 mmol, 1.0 equiv), 8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctanoic acid (7.4 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-73 was obtained as yellow solid in TFA salt form (7.4 mg, 54%). ¹H NMR (800 MHz, Methanol-*d*₄) δ 9.30 (s, 1H), 8.90 (s, 1H), 8.38 (d, *J* = 8.4 Hz, 2H), 7.52 - 7.46 (m, 3H), 7.42 (d, *J* = 7.9 Hz, 2H), 7.11 (d, *J* = 8.6 Hz, 2H), 7.00 (d, *J* = 8.4 Hz, 1H), 6.89 (t, *J* = 8.6 Hz, 1H), 4.65 (s, 1H), 4.59 (t, *J =* 8.2 Hz, 1H), 4.55 (d, *J =* 15.4 Hz, 1H), 4.51 (s, 1H), 4.36 (d, *J* = 15.4 Hz, 1H), 3.93 (d, *J* = 11.1 Hz, 1H), 3.85 - 3.79 (m, 4H), 3.77 - 3.71 (m, 4H), 3.31 - 3.24 (m, 4H), 2.49 - 2.43 (m, 5H), 2.34 - 2.26 (m, 2H), 2.24 - 2.21 (m, 1H), 2.13 - 2.07 (m, 1H), 1.68 - 1.61 (m, 4H), 1.45 - 1.34 (m, 4H), 1.05 (s, 9H). HRMS calcd for C₅₂H₆₂FN₁₀O₆S⁺ [M + H⁺] 973.4553, found 973.4538.

### Example 205

### Synthesis of HC90-74

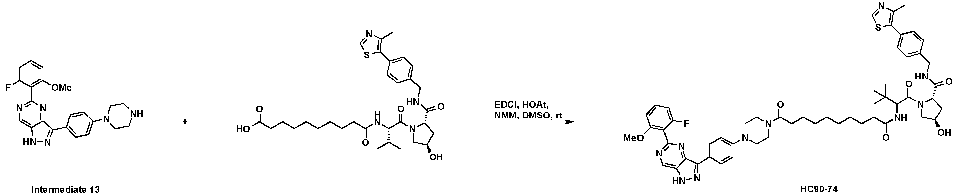

HC90-74 was synthesized following the standard procedure for preparing HC90-33 from Intermediate 13 (6.5 mg, 0.0125 mmol, 1.0 equiv), 10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecanoic acid (7.7 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-74 was obtained as yellow solid in TFA salt form (5.5 mg, 39%). ¹H NMR (800 MHz, Methanol-*d*₄) δ 9.30 (s, 1H), 8.90 (s, 1H), 8.38 (d, *J* = 8.4 Hz, 2H), 7.52 - 7.46 (m, 3H), 7.43 (d, *J* = 7.8 Hz, 2H), 7.11 (d, *J* = 8.6 Hz, 2H), 7.01 (d, *J* = 8.6 Hz, 1H), 6.90 (t, *J* = 8.6 Hz, 1H), 4.66 (s, 1H), 4.59 (t, *J* = 8.7 Hz, 1H), 4.55 (d, *J* = 15.5 Hz, 1H), 4.51 (s, 1H), 4.37 (d, *J* = 15.6 Hz, 1H), 3.92 (d, *J =* 11.0 Hz, 1H), 3.85 - 3.79 (m, 4H), 3.78 - 3.71 (m, 4H), 3.32 - 3.22 (m, 4H), 2.52 - 2.43 (m, 5H), 2.34-2.21 (m, 3H), 2.10 (ddd, *J* = 13.1, 8.7, 4.4 Hz, 1H), 1.68-1.60 (m, 4H), 1.44 - 1.32 (m, 8H), 1.05 (s, 9H). HRMS calcd for C₅₄H₆₆FN₁₀O₆S⁺ [M + H⁺] 1001.4866, found 1001.4850.

### Example 206

### Synthesis of HC90-84

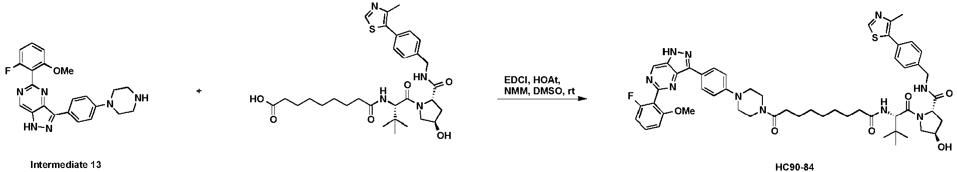

HC90-84 was synthesized following the standard procedure for preparing HC90-33 from Intermediate 13 (6.5 mg, 0.0125 mmol, 1.0 equiv), 9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononanoic acid (7.5 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-84 was obtained as yellow solid in TFA salt form (7.2 mg, 50%). ¹H NMR (800 MHz, Methanol-*d*₄) δ 9.30 (s, 1H), 8.92 (s, 1H), 8.38 (d, *J=* 8.2 Hz, 2H), 7.51 - 7.47 (m, 3H), 7.42 (d, *J* = 8.0 Hz, 2H), 7.10 (d, *J =* 8.5 Hz, 2H), 7.00 (d, *J =* 8.5 Hz, 1H), 6.90 (t, *J =* 8.5 Hz, 1H), 4.65 (s, 1H), 4.60 (t, *J* = 8.4 Hz, 1H), 4.55 (d, *J* = 15.3 Hz, 1H), 4.51 (s, 1H), 4.37 (d, *J =* 15.4 Hz, 1H), 3.92 (d, *J =* 10.9 Hz, 1H), 3.85 - 3.79 (m, 4H), 3.78 - 3.70 (m, 4H), 3.32 - 3.21 (m, 4H), 2.54 - 2.40 (m, 5H), 2.37 - 2.21 (m, 3H), 2.10 (ddd, *J=* 13.3, 9.2, 4.7 Hz, 1H), 1.69 - 1.60 (m, 4H), 1.42 - 1.33 (m, 6H), 1.05 (s, 9H). HRMS calcd for C₅₃H₆₄FN₁₀O₆S⁺ [M + H⁺] 987.4710, found 987.4693.

### Example 207

### Synthesis of HC90-85

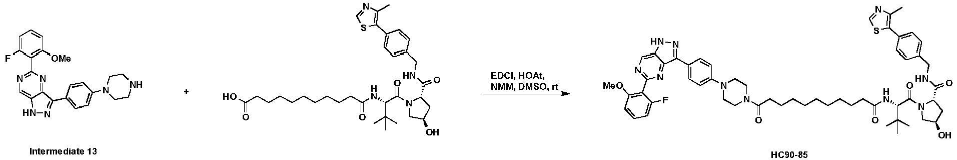

HC90-85 was synthesized following the standard procedure for preparing HC90-33 from Intermediate 13 (6.5 mg, 0.0125 mmol, 1.0 equiv), 11-(((*S*)-1-((2S*,*4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecanoic acid (7.8 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-85 was obtained as yellow solid in TFA salt form (4.3 mg, 30%). ¹H NMR (800 MHz, Methanol-*d*₄) δ 9.30 (s, 1H), 8.91 (s, 1H), 8.38 (d, *J* = 8.4 Hz, 2H), 7.52 - 7.46 (m, 3H), 7.43 (d, *J* = 7.8 Hz, 2H), 7.12 (d, *J =* 8.3 Hz, 2H), 7.01 (d, *J =* 8.6 Hz, 1H), 6.90 (t, *J =* 8.6 Hz, 1H), 4.66 (s, 1H), 4.59 (t, *J* = 8.0 Hz, 1H), 4.55 (d, *J* = 15.3 Hz, 1H), 4.51 (s, 1H), 4.37 (d, *J* = 15.6 Hz, 1H), 3.92 (d, *J* = 10.9 Hz, 1H), 3.85 - 3.79 (m, 4H), 3.78 - 3.73 (m, 4H), 3.33 - 3.23 (m, 4H), 2.49 (s, 3H), 2.46 (t, *J =* 7.6 Hz, 2H), 2.31 (dt, *J =* 14.9, 7.5 Hz, 1H), 2.29 - 2.20 (m, 2H), 2.10 (ddd, *J =* 13.2, 8.9, 4.6 Hz, 1H), 1.69 - 1.60 (m, 4H), 1.43 - 1.29 (m, 10H), 1.05 (s, 9H). HRMS calcd for C₅₅H₆₈FN₁₀O₆S⁺ [M + H⁺] 1015.5023, found 1015.5009.

### Example 208

### Synthesis of intermediate 14

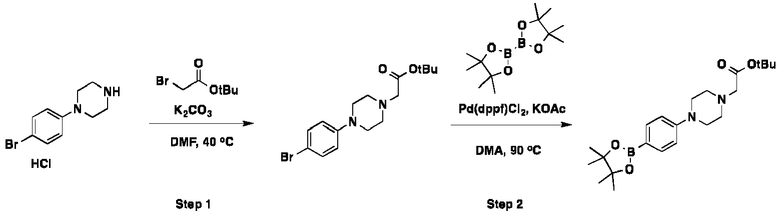

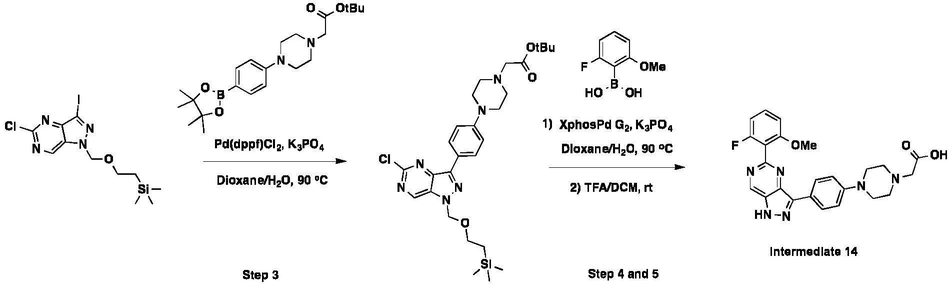

### Step 1: tert-butyl 2-(4-(4-bromophenyl)piperazin-1-yl)acetate

To a solution of 1-(4-Bromophenyl)piperazine hydrochloride (813 mg, 2.93 mmol, 1.0 equiv) in DMF (8 mL) was added potassium carbonate (1.7 g, 11.72 mmol, 4.0 equiv) and tert-butyl bromoacetate (686 mg, 3.52 mmol, 1.2 equiv). Then the mixture was stirred at 40 °C for 2h. Water (20 mL) was added to quench the reaction. The mixture was extracted with ethyl acetate (3 x 20 mL). The organic layer was combined and washed with brine. Dried over Na₂SO₄, filtered and evaporated to afford oil. ESI m/z =355.1 [M + H⁺]. The oil was not further purified and directly used in next step.

### Step 2: tert-butyl 2-(4-(4-(4,4,5,5-tetramethyl-1,3,2-dioxaborolan-2-yl)phenyl)piperazin-1-yl)acetate

A mixture of *tert*-butyl 2-(4-(4-bromophenyl)piperazin-1-yl)acetate (3.0 mmol, 1.0 equiv), 4,4,4',4',5,5,5',5'-octamethyl-2,2'-bi(1,3,2-dioxaborolane) (4.6 g, 18 mmol, 6.0 equiv), Pd(dppf)Ch (330 mg, 0.45 mmol, 0.15 equiv), KOAc (1.2 mg, 12 mmol, 4.0 equiv) in DMA (10 mL) was stirred at 90 °C for 18 h. The mixture was purified preparative HPLC (10%-100% methanol / 0.1% TFA in H₂O) to afford *tert*-butyl 2-(4-(4-(4,4,5,5-tetramethyl-1,3,2-dioxaborolan-2-yl)phenyl)piperazin-1-yl)acetate as a white solid (1.43 g, 91% for two steps) in TFA salt form. ESI m/z =403.4 [M + H⁺].

### Step 3: tert-butyl 2-(4-(4-(5-chloro-1-((2-(trimethylsilyl)ethoxy)methyl)-1H-pyrazolo[4,3-d]pyrimidin-3-yl)phenyl)piperazin-1-yl)acetate

A mixture of 5-chloro-3-iodo-1-((2-(trimethylsilyl)ethoxy)methyl)-1*H*-pyrazolo[4,3-*d*]pyrimidine (411 mg, 1 mmol, 1.0 equiv), *tert*-butyl 2-(4-(4-(4,4,5,5-tetramethyl-1,3,2-dioxaborolan-2-yl)phenyl)piperazin-1-yl)acetate (516 mg, 1 mmol, 1.0 equiv), Pd(dppf)Cl₂ (110 mg, 0.15 mmol, 0.15 equiv), K₃PO₄ (530 mg, 2.5 mmol, 2.5 equiv) in 1,4-dioxane (8 mL) and water (1.3 mL) was stirred at 90 °C for 18 h. The reaction was filtered and extracted with ethyl acetate. The combined organic extracts were combined and concentrated under vacuum. The residue was purified by silica gel column (hexane/ ethyl acetate = 3:2) to give *tert*-butyl 2-(4-(4-(5-chloro-1-((2-(trimethylsilyl)ethoxy)methyl)-1*H-*pyrazolo[4,3-*d*]pyrimidin-3-yl)phenyl)piperazin-1-yl)acetate as a yellow solid (306mg, 55%). ESI m/z =559.3 [M + H⁺].

### Step 4 and 5: intermediate 14

A mixture of *tert*-butyl 2-(4-(4-(5-chloro-1-((2-(trimethylsilyl)ethoxy)methyl)-1*H-*pyrazolo[4,3-*d*]pyrimidin-3-yl)phenyl)piperazin-1-yl)acetate (207 mg, 0.31 mmol, 1.0 equiv), (2-fluoro-6-methoxyphenyl)boronic acid (105 mg, 0.62 mmol, 2.0 equiv), XphosPd G2 (37 mg, 0.05 mmol, 0.15 equiv), K₃PO₄ (255 mg, 1.2 mmol, 4.0 equiv) in 1,4-dioxane (4.0 mL) and water (0.5 mL) was stirred at 90 °C for 1.5 h. The reaction was filtered and extracted with ethyl acetate. The combined organic extracts were combined and concentrated under vacuum. The obtained residue was dissolved in DCM (4 mL). To the solution was added TFA (2 mL). Stirred at room temperature for 3 h and concentrated under vacuum. The residue was purified preparative HPLC (10%-100% acetonitrile/ 0.1% TFA in H₂O) to afford intermediate 14 as a yellow solid (154 mg, 86% for two steps) in TFA salt form. ¹H NMR (800 MHz, Methanol-d4) δ 9.32 (s, 1H), 8.43 (d, J = 8.3 Hz, 2H), 7.50 (q, J = 7.9 Hz, 1H), 7.18 (d, J = 8.4 Hz, 2H), 7.02 (d, J *=* 8.5 Hz, 1H), 6.90 (t, J = 8.6 Hz, 1H), 4.20 (s, 2H), 3.81 (s, 3H), 3.74 - 3.49 (m, 8H). ESI m/z =463.3 [M + H⁺].

### Example 209

### Synthesis of HC90-86

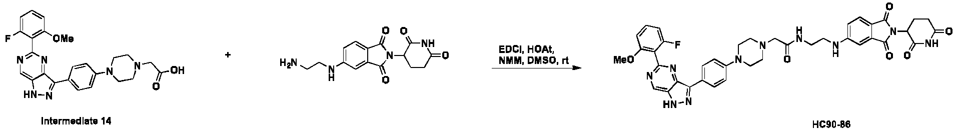

To a solution of Intermediate 14 (5.8 mg, 0.01 mmol, 1.0 equiv) in DMSO (1 mL) were added 5-((2-aminoethyl)amino)-2-(2,6-dioxopiperidin-3-yl)isoindoline-1,3-dione (4.8 mg, 0.011 mmol, 1.1 equiv), EDCI (1-ethyl-3-(3-dimethylaminopropyl)carbodiimide) (3.8 mg, 0.02 mmol, 2.0 equiv), HOAt (1-hydroxy-7-azabenzo-triazole) (2.7 mg, 0.02 mmol, 2.0 equiv), and NMM (N-Methylmorpholine) (5.1 mg, 0.05 mmol, 5.0 equiv). After being stirred overnight at room temperature, the resulting mixture was purified by preparative HPLC (10%-100% methanol / 0.1% TFA in H₂O) to afford HC90-86 as yellow solid in TFA salt form (5.3 mg, 61%). ¹H NMR (800 MHz, Methanol-*d*₄) δ 9.32 (s, 1H), 8.42 (d, *J=* 8.3 Hz, 2H), 7.62 (d, *J* = 8.3 Hz, 1H), 7.50 (q, *J =* 7.9 Hz, 1H), 7.15 (d, *J* = 8.4 Hz, 2H), 7.08 (s, 1H), 7.01 (d, *J* = 8.5 Hz, 1H), 6.94 (d, *J* = 8.5 Hz, 1H), 6.90 (t, *J* = 8.6 Hz, 1H), 5.06 (dd, *J* = 12.5, 5.3 Hz, 1H), 3.93 - 3.88 (m, 2H), 3.81 (s, 3H), 3.54 (t, *J =* 6.0 Hz, 2H), 3.51 - 3.40 (m, 10H), 2.89 - 2.80 (m, 1H), 2.76 - 2.68 (m, 2H), 2.13 - 2.05 (m, 1H). HRMS calcd for C₃₉H₃₈FN₁₀O₆⁺ [M + H⁺] 761.2954, found 761.2940.

### Example 210

### Synthesis of HC90-87

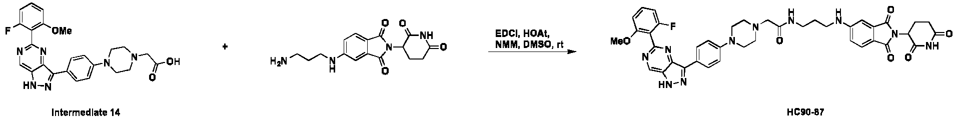

HC90-87 was synthesized following the standard procedure for preparing HC90-86 from Intermediate 14 (5.8 mg, 0.01 mmol, 1.0 equiv), 5-((3-aminopropyl)amino)-2-(2,6-dioxopiperidin-3-yl)isoindoline-1,3-dione (4.9 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-87 was obtained as yellow solid in TFA salt form (5.9 mg, 66%). ¹H NMR (800 MHz, Methanol-*d*₄) δ 9.32 (s, 1H), 8.42 (d, *J* = 8.3 Hz, 2H), 7.60 (d, *J* = 8.3 Hz, 1H), 7.50 (q, *J* = 7.9 Hz, 1H), 7.15 (d, *J* = 8.4 Hz, 2H), 7.03 - 7.00 (m, 2H), 6.96 - 6.86 (m, 2H), 5.06 (dd, *J* = 12.9, 5.4 Hz, 1H), 4.03 - 3.96 (m, 2H), 3.81 (s, 3H), 3.57 - 3.38 (m, 10H), 3.32 (t, *J =* 7.2 Hz, 2H), 2.89 - 2.80 (m, 1H), 2.76 - 2.67 (m, 2H), 2.13 - 2.07 (m, 1H), 1.92 (p, *J =* 7.2 Hz, 2H). HRMS calcd for C₄₀H₄₀FN₁₀O₆⁺ [M + H⁺] 775.3111, found 775.3096.

### Example 211

### Synthesis of HC90-88

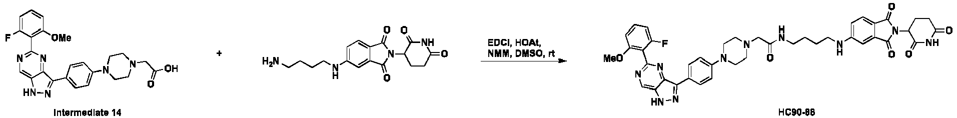

HC90-88 was synthesized following the standard procedure for preparing HC90-86 from Intermediate 14 (5.8 mg, 0.01 mmol, 1.0 equiv), 5-((4-aminobutyl)amino)-2-(2,6-dioxopiperidin-3-yl)isoindoline-1,3-dione (5.1 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-88 was obtained as yellow solid in TFA salt form (5.9 mg, 65%). ¹H NMR (800 MHz, Methanol-*d*₄) δ 9.32 (s, 1H), 8.42 (d, *J* = 8.3 Hz, 2H), 7.59 (d, *J* = 8.3 Hz, 1H), 7.50 (q, *J* = 8.0 Hz, 1H), 7.16 (d, *J =* 8.4 Hz, 2H), 7.03 - 6.99 (m, 2H), 6.90 (t, *J =* 8.6 Hz, 1H), 6.87 (d, *J =* 8.5 Hz, 1H), 5.05 (dd, *J* = 12.4, 5.4 Hz, 1H), 3.98 (s, 2H), 3.81 (s, 3H), 3.56 - 3.43 (m, 8H), 3.37 (t, *J =* 6.6 Hz, 2H), 3.30 (t, *J* = 6.7 Hz, 2H), 2.87 - 2.78 (m, 1H), 2.76 - 2.65 (m, 2H), 2.12 - 2.05 (m, 1H), 1.78 - 1.67 (m, 4H). HRMS calcd for C₄₁H₄₂FN₁₀O₆⁺ [M + H⁺] 789.3267, found 789.3251.

### Example 212

### Synthesis of HC90-89

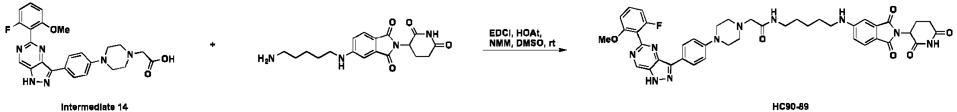

HC90-89 was synthesized following the standard procedure for preparing HC90-86 from Intermediate 14 (5.8 mg, 0.01 mmol, 1.0 equiv), 5-((5-aminopentyl)amino)-2-(2,6-dioxopiperidin-3-yl)isoindoline-1,3-dione (5.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-89 was obtained as yellow solid in TFA salt form (4.2 mg, 46%). ¹H NMR (800 MHz, Methanol-*d*₄) δ 9.32 (s, 1H), 8.42 (d, *J=* 8.3 Hz, 2H), 7.59 (d, *J* = 8.3 Hz, 1H), 7.50 (q, *J* = 7.9 Hz, 1H), 7.16 (d, *J =* 8.4 Hz, 2H), 7.08 - 6.98 (m, 2H), 6.90 (t, *J =* 8.6 Hz, 1H), 6.87 (d, *J =* 8.6 Hz, 1H), 5.05 (dd, *J* = 12.8, 5.3 Hz, 1H), 3.92 (s, 2H), 3.81 (s, 3H), 3.67 - 3.33 (m, 10H), 3.26 (t, *J =* 7.1 Hz, 2H), 2.86 - 2.79 (m, 1H), 2.76 - 2.66 (m, 2H), 2.11 - 2.04 (m, 1H), 1.73 (p, *J* = 7.2 Hz, 2H), 1.64 (p, *J =* 7.3 Hz, 2H), 1.52 (q, *J =* 7.9 Hz, 2H). HRMS calcd for C₄₂H₄₄FN₁₀O₆⁺[M + H⁺] 803.3424, found 803.3410.

### Example 213

### Synthesis of HC90-90

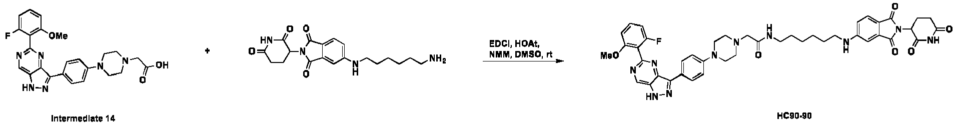

HC90-90 was synthesized following the standard procedure for preparing HC90-86 from Intermediate 14 (5.8 mg, 0.01 mmol, 1.0 equiv), 5-((6-aminohexyl)amino)-2-(2,6-dioxopiperidin-3-yl)isoindoline-1,3-dione (5.4 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-90 was obtained as yellow solid in TFA salt form (7.1 mg, 76%). ¹H NMR (800 MHz, Methanol-*d*₄) δ 9.32 (s, 1H), 8.42 (d, *J* = 8.3 Hz, 2H), 7.58 (d, *J* = 8.3 Hz, 1H), 7.50 (q, *J* = 8.0 Hz, 1H), 7.16 (d, *J* = 8.4 Hz, 2H), 7.01 (d, *J =* 8.5 Hz, 1H), 6.99 (s, 1H), 6.90 (t, *J =* 8.6 Hz, 1H), 6.86 (d, *J* = 8.5 Hz, 1H), 5.05 (dd, *J* = 12.9, 5.3 Hz, 1H), 4.00 (s, 2H), 3.81 (s, 3H), 3.62 - 3.45 (m, 8H), 3.31 (t, *J =* 7.2 Hz, 2H), 3.24 (t, *J =* 7.1 Hz, 2H), 2.88 - 2.80 (m, 1H), 2.76 - 2.65 (m, 2H), 2.12-2.06 (m, 1H), 1.70 (p, *J =* 7.3 Hz, 2H), 1.60 (p, *J =* 7.3 Hz, 2H), 1.54 -1.42 (m, 4H). HRMS calcd for C₄₃H₄₆FN₁₀O₆⁺ [M + H⁺] 817.3580, found 817.3566.

### Example 214

### Synthesis of HC90-91

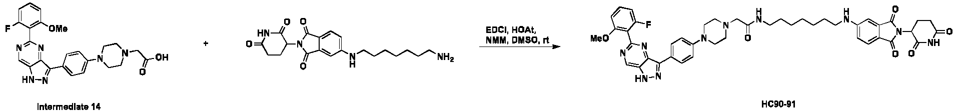

HC90-91 was synthesized following the standard procedure for preparing HC90-86 from Intermediate 14 (5.8 mg, 0.01 mmol, 1.0 equiv), 5-((7-aminoheptyl)amino)-2-(2,6-dioxopiperidin-3-yl)isoindoline-1,3-dione (5.5 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-91 was obtained as yellow solid in TFA salt form (6.4 mg, 68%). ¹H NMR (800 MHz, Methanol-*d*₄) δ 9.32 (s, 1H), 8.42 (d, *J =* 8.3 Hz, 2H), 7.58 (d, *J =* 8.3 Hz, 1H), 7.50 (q, *J =* 8.0 Hz, 1H), 7.17 (d, *J =* 8.4 Hz, 2H), 7.02 (d, *J =* 8.5 Hz, 1H), 7.00 (s, 1H), 6.90 (t, *J =* 8.6 Hz, 1H), 6.85 (d, *J =* 8.5 Hz, 1H), 5.05 (dd, *J =* 12.8, 5.4 Hz, 1H), 3.99 (s, 2H), 3.81 (s, 3H), 3.63 - 3.39 (m, 8H), 3.30 (t, *J =* 7.2 Hz, 2H), 3.24 (t, *J* = 7.1 Hz, 2H), 2.92-2.81 (m, 1H), 2.79 - 2.66 (m, 2H), 2.14-2.07 (m, 1H), 1.69 (p, *J* = 7.2 Hz, 2H), 1.59 (p, *J* = 7.3 Hz, 2H), 1.52 -1.38 (m, 6H). HRMS calcd for C₄₄H₄₈FN₁₀O₆⁺ [M + H⁺] 831.3737, found 831.3721.

### Example 215

### Synthesis of HC90-92

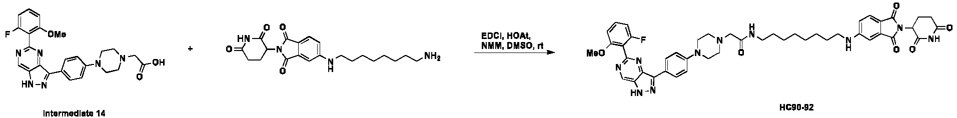

HC90-92 was synthesized following the standard procedure for preparing HC90-86 from Intermediate 14 (5.8 mg, 0.01 mmol, 1.0 equiv), 5-((8-aminooctyl)amino)-2-(2,6-dioxopiperidin-3-yl)isoindoline-1,3-dione (5.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-92 was obtained as yellow solid in TFA salt form (5.3 mg, 55%). ¹H NMR (800 MHz, Methanol-*d*₄) δ 9.31 (s, 1H), 8.42 (d, *J* = 8.3 Hz, 2H), 7.57 (d, *J* = 8.3 Hz, 1H), 7.50 (q, *J* = 7.9 Hz, 1H), 7.16 (d, *J* = 8.4 Hz, 2H), 7.02 (d, *J* = 8.5 Hz, 1H), 6.99 (s, 1H), 6.90 (t, *J* = 8.6 Hz, 1H), 6.85 (d, *J* = 8.4 Hz, 1H), 5.05 (dd, *J* = 12.9, 5.3 Hz, 1H), 3.95 (s, 2H), 3.81 (s, 3H), 3.60 - 3.40 (m, 8H), 3.29 (t, *J =* 7.2 Hz, 2H), 3.23 (t, *J =* 7.1 Hz, 2H), 2.92 - 2.81 (m, 1H), 2.78 - 2.66 (m, 2H), 2.18 - 2.04 (m, 1H), 1.69 (p, *J* = 7.2 Hz, 2H), 1.57(t, *J* =7.0Hz,2H), 1.47*(*t*, J =*7.4Hz*,*2H*),* 1.41 (q, *J* = 7.3, 5.1 Hz, 6H). HRMS calcd for C₄₅H₅₀FN₁₀O₆⁺ [M + H⁺] 845.3893, found 845.3879.

### Example 216

### Synthesis of HC90-93

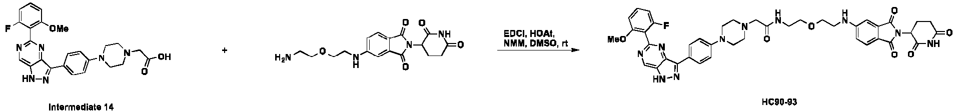

HC90-93 was synthesized following the standard procedure for preparing HC90-86 from Intermediate 14 (5.8 mg, 0.01 mmol, 1.0 equiv), 5-((2-(2-aminoethoxy)ethyl)amino)-2-(2,6-dioxopiperidin-3-yl)isoindoline-1,3-dione (5.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-93 was obtained as yellow solid in TFA salt form (7.4 mg, 81%). ¹H NMR (800 MHz, Methanol-*d*₄) δ 9.32 (s, 1H), 8.41 (d, *J* = 8.3 Hz, 2H), 7.61 (d, *J* = 8.3 Hz, 1H), 7.50 (q, *J* = 7.9 Hz, 1H), 7.13 (d, *J =* 8.4 Hz, 2H), 7.11 (s, 1H), 7.01 (d, *J* = 8.5Hz, 1H), 6.93 (d, *J* = 8.4 Hz, 1H), 6.90 (t, *J =* 8.6 Hz, 1H), 5.03 (dd, *J* = 12.8, 5.5 Hz, 1H), 3.98 (s, 2H), 3.81 (s, 3H), 3.73 (t, *J* = 5.2 Hz, 2H), 3.63 (t, *J* = 5.2 Hz, 2H), 3.56 - 3.42 (m, 12H), 2.85 - 2.74 (m, 1H), 2.72 - 2.62 (m, 2H), 2.14 - 2.00 (m, 1H). HRMS calcd for C₄₁H₄₂FN₁₀O₇⁺ [M + H⁺] 805.3216, found 805.3202.

### Example 217

### Synthesis of HC90-94

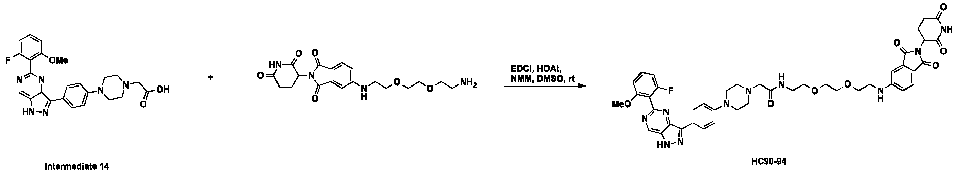

HC90-94 was synthesized following the standard procedure for preparing HC90-86 from Intermediate 14 (5.8 mg, 0.01 mmol, 1.0 equiv), 5-((2-(2-(2-aminoethoxy)ethoxy)ethyl)amino)-2-(2,6-dioxopiperidin-3-yl)isoindoline-1,3-dione (5.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-94 was obtained as yellow solid in TFA salt form (7.1 mg, 74%). ¹H NMR (800 MHz, Methanol-*d*₄) δ 9.32 (s, 1H), 8.41 (d, *J* = 8.4 Hz, 2H), 7.58 (d, *J* = 8.3 Hz, 1H), 7.50 (q, *J =* 7.9 Hz, 1H), 7.14 (d, *J =* 8.4 Hz, 2H), 7.07 (s, 1H), 7.01 (d, *J =* 8.6 Hz, 1H), 6.95 - 6.86 (m, 2H), 5.03 (dd, *J =* 12.9, 5.4 Hz, 1H), 3.98 (s, 2H), 3.81 (s, 3H), 3.73 (t, *J =* 5.3 Hz, 2H), 3.70 - 3.66 (m, 4H), 3.61 (t, *J* = 5.3 Hz, 2H), 3.53 - 3.41 (m, 12H), 2.85 - 2.77 (m, 1H), 2.74 - 2.65 (m, 2H), 2.20 - 1.92 (m, 1H). HRMS calcd for C₄₃H₄₆FN₁₀O₈⁺ [M + H⁺] 849.3479, found 849.3463.

### Example 218

### Synthesis of HC90-95

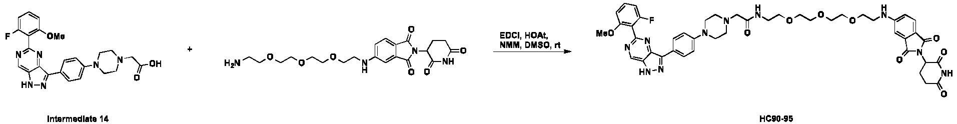

HC90-95 was synthesized following the standard procedure for preparing HC90-86 from Intermediate 14 (5.8 mg, 0.01 mmol, 1.0 equiv), 5-((2-(2-(2-(2-aminoethoxy)ethoxy)ethoxy)ethyl)amino)-2-(2,6-dioxopiperidin-3-yl)isoindoline-1,3-dione (6.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-95 was obtained as yellow solid in TFA salt form (6.5 mg, 65%). ¹H NMR (800 MHz, Methanol-*d*₄) δ 9.31 (s, 1H), 8.40 (d, *J =* 8.4 Hz, 2H), 7.55 (d, *J =* 8.3 Hz, 1H), 7.50 (q, *J =* 7.9 Hz, 1H), 7.14 (d, *J =* 8.4 Hz, 2H), 7.04 (s, 1H), 7.01 (d, *J =* 8.5 Hz, 1H), 6.90 (t, *J =* 8.6 Hz, 1H), 6.87 (d, *J =* 8.5 Hz, 1H), 5.04 (dd, *J =* 12.8, 5.4 Hz, 1H), 3.93 (s, 2H), 3.81 (s, 3H), 3.70 (t, *J =* 5.3 Hz, 2H), 3.69 - 3.63 (m, 8H), 3.60 (t, *J* = 5.3 Hz, 2H), 3.48 (t, *J* = 5.3 Hz, 2H), 3.46 - 3.43 (m, 8H), 3.41 (t, *J* = 5.3 Hz, 2H), 2.82 (ddd, *J* = 18.5, 13.8, 5.3 Hz, 1H), 2.75 - 2.65 (m, 2H), 2.13 - 2.05 (m, 1H). HRMS calcd for C₄₅H₅₀FN₁₀O₉⁺ [M + H⁺] 893.3741, found 893.3730.

### Example 219

### Synthesis of HC90-96

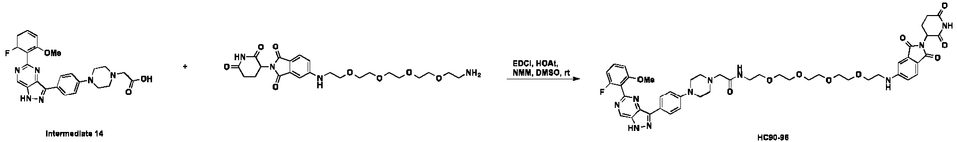

HC90-96 was synthesized following the standard procedure for preparing HC90-86 from Intermediate 14 (5.8 mg, 0.01 mmol, 1.0 equiv), 5-((14-amino-3,6,9,12-tetraoxatetradecyl)amino)-2-(2,6-dioxopiperidin-3-yl)isoindoline-1,3-dione (6.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-96 was obtained as yellow solid in TFA salt form (6.7 mg, 64%). ¹H NMR (800 MHz, Methanol-*d*₄) δ 9.31 (s, 1H), 8.40 (d, *J* = 8.3 Hz, 2H), 7.55 (d, *J* = 8.3 Hz, 1H), 7.50 (q, *J* = 7.9 Hz, 1H), 7.14 (d, *J* = 8.4 Hz, 2H), 7.02 (s, 1H), 7.01 (d, *J* = 8.6 Hz, 1H), 6.90 (t, *J* = 8.6 Hz, 1H), 6.86 (d, *J* = 8.4 Hz, 1H), 5.04 (dd, *J* = 12.9, 5.4 Hz, 1H), 3.98 (s, 2H), 3.81 (s, 3H), 3.68 (t, *J* = 5.2 Hz, 2H), 3.67 - 3.63 (m, 12H), 3.59 (t, *J* = 5.2 Hz, 2H), 3.53 - 3.44 (m, 10H), 3.39 (t, *J* = 5.3 Hz, 2H), 2.83 (ddd, *J* = 18.5, 13.9, 5.4 Hz, 1H), 2.76 - 2.62 (m, 2H), 2.18 - 1.99 (m, 1H). HRMS calcd for C₄₇H₅₄FN₁₀O₁₀⁺ [M + H⁺] 937.4003, found 937.3987.

### Example 220

### Synthesis of HC90-97

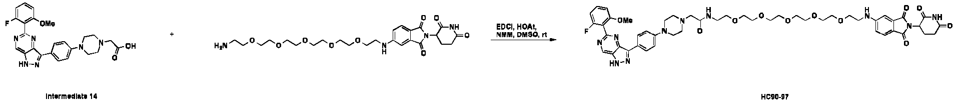

HC90-97 was synthesized following the standard procedure for preparing HC90-86 from Intermediate 14 (5.8 mg, 0.01 mmol, 1.0 equiv), 5-((17-amino-3,6,9,12,15-pentaoxaheptadecyl)amino)-2-(2,6-dioxopiperidin-3-yl)isoindoline-1,3-dione (7.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-97 was obtained as yellow solid in TFA salt form (6.8 mg, 62%). ¹H NMR (800 MHz, Methanol-*d*₄) δ 9.31 (s, 1H), 8.41 (d, *J =* 8.4 Hz, 2H), 7.55 (d, *J* = 8.3 Hz, 1H), 7.50 (q, *J* = 8.0 Hz, 1H), 7.14 (d, *J* = 8.4 Hz, 2H), 7.02 (s, 1H), 7.01 (d, *J* = 8.6 Hz, 1H), 6.90 (t, *J* = 8.6 Hz, 1H), 6.86 (d, *J* = 8.5 Hz, 1H), 5.05 (dd, *J =* 13.0, 5.3 Hz, 1H), 4.00 (s, 2H), 3.81 (s, 3H), 3.68 (t, *J* = 5.3 Hz, 2H), 3.64 (d, *J* = 6.4 Hz, 16H), 3.60 (t, *J =* 5.2 Hz, 2H), 3.52 - 3.44 (m, 10H), 3.38 (t, *J =* 5.3 Hz, 2H), 2.84 (ddd, *J =* 18.6, 14.0, 5.3 Hz, 1H), 2.78 - 2.60 (m, 2H), 2.18 - 1.95 (m, 1H). HRMS calcd for C₄₉H₅₈FN₁₀O₁₁⁺ [M + H⁺] 981.4265, found 981.4250.

### Example 221

### Synthesis of HC90-102

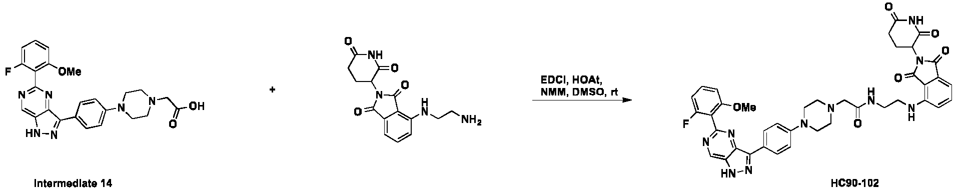

HC90-102 was synthesized following the standard procedure for preparing HC90-86 from Intermediate 14 (5.8 mg, 0.01 mmol, 1.0 equiv), 4-((2-aminoethyl)amino)-2-(2,6-dioxopiperidin-3-yl)isoindoline-1,3-dione (4.8 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-102 was obtained as yellow solid in TFA salt form (5.7 mg, 65%). ¹H NMR (800 MHz, Methanol-*d*₄) δ 9.32 (s, 1H), 8.42 (d, *J* = 8.3 Hz, 2H), 7.61 (t, *J* = 7.8 Hz, 1H), 7.50 (q, *J =* 7.9 Hz, 1H), 7.18 (d, *J* = 8.5 Hz, 1H), 7.15 (d, *J =* 8.4 Hz, 2H), 7.12 (d, *J =* 7.2 Hz, 1H), 7.01 (d,*J* = 8.5 Hz, 1H), 6.90 (t, *J =* 8.6 Hz, 1H), 5.09 (dd, *J =* 12.9, 5.4 Hz, 1H), 3.98 (s, 2H), 3.81 (s, 3H), 3.62 - 3.58 (m, 2H), 3.57 - 3.54 (m, 2H), 3.52 - 3.42 (m, 8H), 2.86 (ddd, *J* = 18.4, 13.8, 5.4 Hz, 1H), 2.78 -2.62 (m, 2H), 2.18 -2.04 (m, 1H). HRMS calcd for C₃₉H₃₈FN₁₀O₆⁺ [M + H⁺] 761.2954, found 761.2939.

### Example 222

### Synthesis of HC90-103

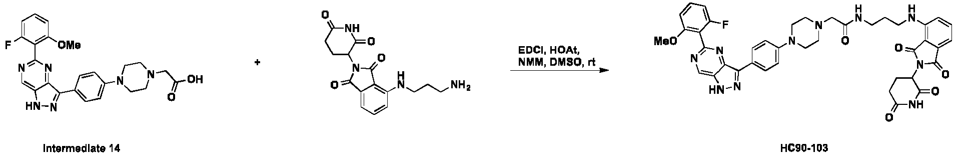

HC90-103 was synthesized following the standard procedure for preparing HC90-86 from Intermediate 14 (5.8 mg, 0.01 mmol, 1.0 equiv), 4-((3-aminopropyl)amino)-2-(2,6-dioxopiperidin-3-yl)isoindoline-1,3-dione (4.9 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-103 was obtained as yellow solid in TFA salt form (4.4 mg, 50%). ¹H NMR (800 MHz, Methanol-*d*₄) δ 9.32 (s, 1H), 8.42 (d, *J* = 8.3 Hz, 2H), 7.60 (t, *J* = 7.8 Hz, 1H), 7.50 (q, *J =* 7.9 Hz, 1H), 7.16 (d, *J* = 8.4 Hz, 2H), 7.10 (d, *J* = 7.9 Hz, 2H), 7.02 (d, *J =* 8.5 Hz, 1H), 6.90 (t, *J* = 8.6 Hz, 1H), 5.09 (dd, *J=* 12.9, 5.5 Hz, 1H), 3.96 (s, 2H), 3.81 (s, 3H), 3.56 - 3.39 (m, 12H), 2.86 (ddd, *J =* 18.5, 13.9, 5.4 Hz, 1H), 2.79 - 2.67 (m, 2H), 2.15 - 2.10 (m, 1H), 1.98 - 1.89 (m, 2H). HRMS calcd for C₄₀H₄₀FN₁₀O₆⁺ [M + H⁺] 775.3111, found 775.3094.

### Example 223

### Synthesis of HC90-104

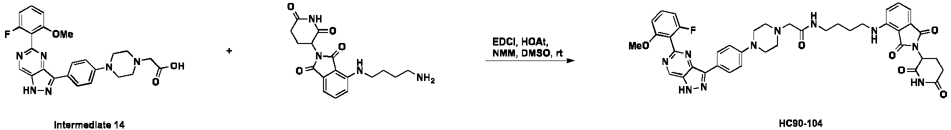

HC90-104 was synthesized following the standard procedure for preparing HC90-86 from Intermediate 14 (5.8 mg, 0.01 mmol, 1.0 equiv), 4-((4-aminobutyl)amino)-2-(2,6-dioxopiperidin-3-yl)isoindoline-1,3-dione (5.1 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-104 was obtained as yellow solid in TFA salt form (6.1 mg, 68%). ¹H NMR (800 MHz, Methanol-*d*₄) δ 9.32 (s, 1H), 8.42 (d, *J=* 8.3 Hz, 2H), 7.58 (t, *J=* 7.8 Hz, 1H), 7.50 (q, *J =* 7.9 Hz, 1H), 7.16 (d, *J* = 8.4 Hz, 2H), 7.12 - 7.06 (m, 2H), 7.01 (d, *J* = 8.5 Hz, 1H), 6.90 (t, *J =* 8.6 Hz, 1H), 5.07 (dd, *J* = 12.8, 5.4 Hz, 1H), 3.98 (s, 2H), 3.81 (s, 3H), 3.58 - 3.44 (m, 8H), 3.40 (t, *J* = 6.7 Hz, 2H), 3.36 (t, *J* = 6.7 Hz, 2H), 2.85 (ddd, *J* = 18.3, 13.7, 5.4 Hz, 1H), 2.78 - 2.68 (m, 2H), 2.14 - 2.08 (m, 1H), 1.77 -1.68 (m, 4H). HRMS calcd for C₄₁H₄₂FN₁₀O₆⁺ [M + H⁺] 789.3267, found 789.3251.

### Example 224

### Synthesis of HC90-105

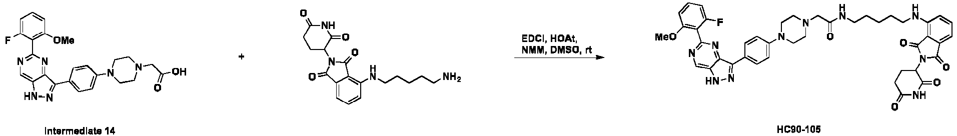

HC90-105 was synthesized following the standard procedure for preparing HC90-86 from Intermediate 14 (5.8 mg, 0.01 mmol, 1.0 equiv), 4-((5-aminopentyl)amino)-2-(2,6-dioxopiperidin-3-yl)isoindoline-1,3-dione (5.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-105 was obtained as yellow solid in TFA salt form (6.9 mg, 75%). ¹H NMR (800 MHz, Methanol-*d*₄) δ 9.32 (s, 1H), 8.43 (d, *J* = 8.4 Hz, 2H), 7.57 (t, *J* = 7.8 Hz, 1H), 7.50 (q, *J =* 7.9 Hz, 1H), 7.17 (d, *J =* 8.4 Hz, 2H), 7.09 - 7.05 (m, 2H), 7.01 (d, *J* = 8.5 Hz, 1H), 6.90 (t, *J =* 8.6 Hz, 1H), 5.18 - 4.97 (m, 1H), 3.98 (q, *J* = 15.3 Hz, 2H), 3.81 (s, 3H), 3.61 - 3.45 (m, 8H), 3.42 - 3.32 (m, 4H), 2.79 - 2.65 (m, 3H), 2.15 - 2.05 (m, 1H), 1.79 - 1.70 (m, 2H), 1.67 - 1.58 (m, 2H), 1.57 - 1.45 (m, 2H). HRMS calcd for C₄₂H₄₄FN₁₀O₆⁺ [M + H⁺] 803.3424, found 803.3411.

### Example 225

### Synthesis of HC90-106

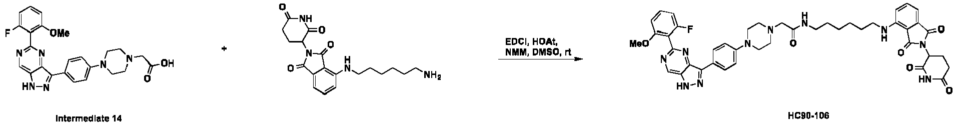

HC90-106 was synthesized following the standard procedure for preparing HC90-86 from Intermediate 14 (5.8 mg, 0.01 mmol, 1.0 equiv), 4-((6-aminohexyl)amino)-2-(2,6-dioxopiperidin-3-yl)isoindoline-1,3-dione (5.4 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-106 was obtained as yellow solid in TFA salt form (6.2 mg, 67%). ¹H NMR (800 MHz, Methanol-*d*₄) δ 9.32 (s, 1H), 8.42 (d, *J* = 8.3 Hz, 2H), 7.57 (t, *J* = 7.8 Hz, 1H), 7.50 (q, *J =* 7.9 Hz, 1H), 7.16 (d, *J =* 8.4 Hz, 2H), 7.09 - 7.04 (m, 2H), 7.01 (d, *J =* 8.5 Hz, 1H), 6.90 (t, *J =* 8.6 Hz, 1H), 5.07 (dd, *J* = 12.9, 5.3 Hz, 1H), 3.98 (s, 2H), 3.81 (s, 3H), 3.56 - 3.45 (m, 8H), 3.36 (t, *J* = 7.1 Hz, 2H), 3.30 (t, *J* = 7.1 Hz, 2H), 2.86 (ddd, *J* = 18.4, 13.8, 5.4 Hz, 1H), 2.78 - 2.69 (m, 2H), 2.17 - 2.09 (m, 1H), 1.75 - 1.67 (m, 2H), 1.64 - 1.57 (m, 2H), 1.54 - 1.43 (m, 4H). HRMS calcd for C₄₃H₄₆FN₁₀O₆⁺ [M + H⁺] 817.3580, found 817.3567.

### Example 226

### Synthesis of HC90-107

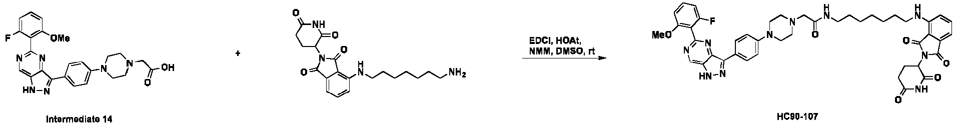

HC90-107 was synthesized following the standard procedure for preparing HC90-86 from Intermediate 14 (5.8 mg, 0.01 mmol, 1.0 equiv), 4-((7-aminoheptyl)amino)-2-(2,6-dioxopiperidin-3-yl)isoindoline-1,3-dione (5.5 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-107 was obtained as yellow solid in TFA salt form (6.6 mg, 70%). ¹H NMR (800 MHz, Methanol-*d*₄) δ 9.32 (s, 1H), 8.42 (d, *J* = 8.3 Hz, 2H), 7.56 (t, *J* = 7.8 Hz, 1H), 7.50 (q, *J =* 7.8 Hz, 1H), 7.16 (d, *J* = 8.4 Hz, 2H), 7.08 - 7.03 (m, 2H), 7.01 (d, *J* = 8.5 Hz, 1H), 6.90 (t, *J =* 8.6 Hz, 1H), 5.05 (dd, *J* = 12.2, 5.1 Hz, 1H), 3.98 (s, 2H), 3.81 (s, 3H), 3.63 - 3.43 (m, 8H), 3.35 (t, *J* = 7.3 Hz, 2H), 3.29 (t, *J* = 7.1 Hz, 2H), 2.83 (ddd, *J* = 18.8, 13.9, 5.3 Hz, 1H), 2.77 - 2.67 (m, 2H), 2.18 - 2.05 (m, 1H), 1.75 - 1.66 (m, 2H), 1.61 - 1.55 (m, 2H), 1.52 - 1.37 (m, 6H). HRMS calcd for C₄₄H₄₈FN₁₀O₆⁺ [M + H⁺] 831.3737, found 831.3725.

### Example 227

### Synthesis of HC90-108

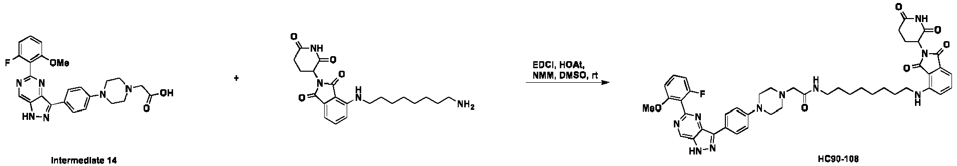

HC90-108 was synthesized following the standard procedure for preparing HC90-86 from Intermediate 14 (5.8 mg, 0.01 mmol, 1.0 equiv), 4-((8-aminooctyl)amino)-2-(2,6-dioxopiperidin-3-yl)isoindoline-1,3-dione (5.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-108 was obtained as yellow solid in TFA salt form (5.9 mg, 62%). ¹H NMR (800 MHz, Methanol-*d*₄) δ 9.31 (s, 1H), 8.42 (d, *J* = 8.3 Hz, 2H), 7.55 (t, *J* = 7.8 Hz, 1H), 7.50 (q, *J =* 7.9 Hz, 1H), 7.16 (d, *J =* 8.4 Hz, 2H), 7.04 (d, *J =* 7.8 Hz, 2H), 7.01 (d, *J =* 8.5 Hz, 1H), 6.90 (t, *J* = 8.6 Hz, 1H), 5.07 (dd, *J* = 12.9, 5.3 Hz, 1H), 3.97 (s, 2H), 3.81 (s, 3H), 3.60 - 3.42 (m, 8H), 3.36 - 3.33 (m, 2H), 3.29 (t, *J* = 7.1 Hz, 2H), 2.86 (ddd, *J =* 18.5, 13.8, 5.4 Hz, 1H), 2.80 - 2.68 (m, 2H), 2.16 - 2.06 (m, 1H), 1.69 (p, *J =* 7.2 Hz, 2H), 1.57 (t, *J* = 7.0 Hz, 2H), 1.47 (t, *J* = 7.5 Hz, 2H), 1.44 - 1.36 (m, 6H). HRMS calcd for C₄₅H₅₀FN₁₀O₆⁺ [M + H⁺] 845.3893, found 845.3881.

### Example 228

### Synthesis of HC90-109

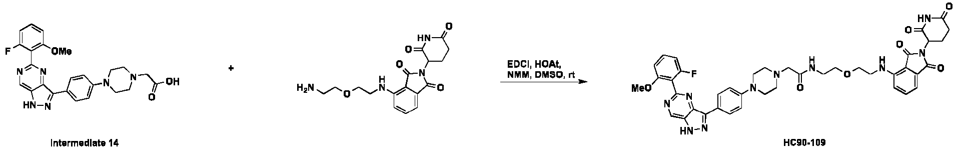

HC90-109 was synthesized following the standard procedure for preparing HC90-86 from Intermediate 14 (5.8 mg, 0.01 mmol, 1.0 equiv), 4-((2-(2-aminoethoxy)ethyl)amino)-2-(2,6-dioxopiperidin-3-yl)isoindoline-1,3-dione (5.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-109 was obtained as yellow solid in TFA salt form (5.5 mg, 60%). ¹H NMR (800 MHz, Methanol-*d*₄) δ 9.32 (s, 1H), 8.41 (d, *J* = 8.3 Hz, 2H), 7.59 (t*, J* = 7.8 Hz, 1H), 7.50 (q, *J =* 7.9 Hz, 1H), 7.14 (d, *J =* 8.6 Hz, 2H), 7.12 (d, *J =* 8.7 Hz, 1H), 7.09 (d, *J =* 7.1 Hz, 1H), 7.01 (d, *J =* 8.5 Hz, 1H), 6.90 (t, *J =* 8.6 Hz, 1H), 5.05 (dd, *J =* 12.5, 5.5 Hz, 1H), 3.96 (s, 2H), 3.81 (s, 3H), 3.75 (t, *J =* 5.0 Hz, 2H), 3.66 (t, *J =* 5.2 Hz, 2H), 3.58 - 3.40 (m, 12H), 2.78 (ddd, *J =* 18.7, 14.8, 6.0 Hz, 1H), 2.73 - 2.66 (m, 2H), 2.12 - 2.07 (m, 1H). HRMS calcd for C₄₁H₄₂FN₁₀O₇⁺ [M + H⁺] 805.3216, found 805.3201.

### Example 229

### Synthesis of HC90-110

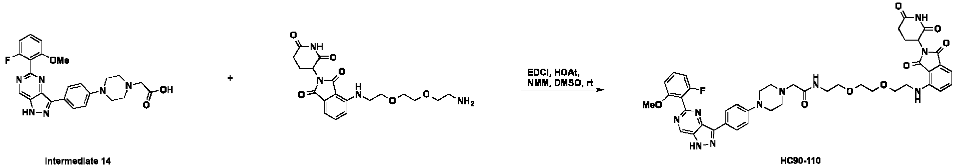

HC90-110 was synthesized following the standard procedure for preparing HC90-86 from Intermediate 14 (5.8 mg, 0.01 mmol, 1.0 equiv), 4-((2-(2-(2-aminoethoxy)ethoxy)ethyl)amino)-2-(2,6-dioxopiperidin-3-yl)isoindoline-1,3-dione (5.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-110 was obtained as yellow solid in TFA salt form (6.8 mg, 71%). ¹HNMR (800 MHz, Methanol-*d*₄) δ 9.32 (s, 1H), 8.40 (d*, J* = 8.3 Hz, 2H), 7.54 (t*, J* = 7.8 Hz, 1H), 7.50 (q, *J* = 7.9 Hz, 1H), 7.13 (d, *J* = 8.4 Hz, 2H), 7.08 (d, *J* = 8.5 Hz, 1H), 7.05 (d, *J* = 7.2 Hz, 1H), 7.01 (d, *J =* 8.5 Hz, 1H), 6.90 (t, *J =* 8.6 Hz, 1H), 5.07 (dd, *J =* 12.9, 5.4 Hz, 1H), 3.95 (s, 2H), 3.81 (s, 3H), 3.75 (t, *J* = 5.1 Hz, 2H), 3.71 - 3.67 (m, 4H), 3.63 (t, *J* = 5.3 Hz, 2H), 3.54 - 3.42 (m, 12H), 2.85 (ddd, *J* = 18.7, 13.9, 5.4 Hz, 1H), 2.78 - 2.68 (m, 2H), 2.18 - 2.10 (m, 1H). HRMS calcd for C₄₃H₄₆FN₁₀O₈⁺ [M + H⁺] 849.3479, found 849.3465.

### Example 230

### Synthesis of HC90-111

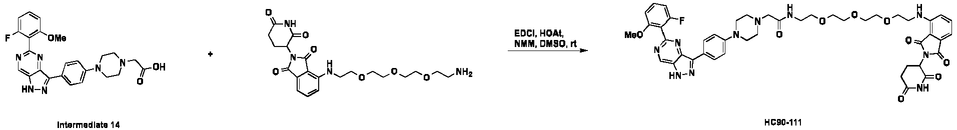

HC90-111 was synthesized following the standard procedure for preparing HC90-86 from Intermediate 14 (5.8 mg, 0.01 mmol, 1.0 equiv), 4-((2-(2-(2-(2-aminoethoxy)ethoxy)ethoxy)ethyl)amino)-2-(2,6-dioxopiperidin-3-yl)isoindoline-1,3 -dione (6.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-111 was obtained as yellow solid in TFA salt form (8.6 mg, 85%). ¹H NMR (800 MHz, Methanol-*d*₄) δ 9.32 (s, 1H), 8.39 (d, *J =* 8.3 Hz, 2H), 7.52 - 7.47 (m, 2H), 7.11 (d, *J =* 8.4 Hz, 2H), 7.06 - 7.02 (m, 2H), 7.01 (d, *J* = 8.6 Hz, 1H), 6.90 (t, *J* = 8.6 Hz, 1H), 5.06 (dd, *J =* 12.7, 5.4 Hz, 1H), 3.99 (s, 2H), 3.81 (s, 3H), 3.72 (t, *J* = 5.1 Hz, 2H), 3.68 (d, *J* = 7.9 Hz, 6H), 3.65 - 3.63 (m, 2H), 3.59 (t, *J=* 5.2 Hz, 2H), 3.53 - 3.41 (m, 12H), 2.86 (ddd, *J =* 18.1, 13.7, 5.3 Hz, 1H), 2.78 - 2.68 (m, 2H), 2.15 - 2.09 (m, 1H). HRMS calcd for C₄₅H₅₀FN₁₀O₉⁺ [M + H⁺] 893.3741, found 893.3724.

### Example 231

### Synthesis of HC90-112

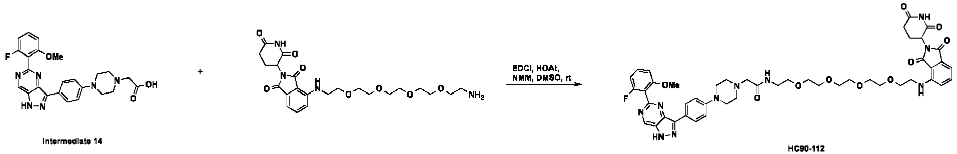

HC90-112 was synthesized following the standard procedure for preparing HC90-86 from Intermediate 14 (5.8 mg, 0.01 mmol, 1.0 equiv), 4-((14-amino-3,6,9,12-tetraoxatetradecyl)amino)-2-(2,6-dioxopiperidin-3-yl)isoindoline-1,3-dione (6.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-112 was obtained as yellow solid in TFA salt form (4.8 mg, 46%). ¹H NMR (800 MHz, Methanol-*d*₄) δ 9.32 (s, 1H), 8.38 (d, *J* = 8.3 Hz, 2H), 7.55 - 7.46 (m, 2H), 7.11 (d, *J =* 8.4 Hz, 2H), 7.05 - 7.00 (m, 3H), 6.90 (t, *J* = 8.6 Hz, 1H), 5.06 (dd, *J* = 12.8, 5.3 Hz, 1H), 4.01 (s, 2H), 3.81 (s, 3H), 3.72 - 3.62 (m, 14H), 3.60 (t, *J =* 5.2 Hz, 2H), 3.51 - 3.44 (m, 12H), 2.85 (ddd, *J =* 18.1, 13.7, 5.3 Hz, 1H), 2.80 - 2.66 (m, 2H), 2.17 - 2.07 (m, 1H). HRMS calcd for C₄₇H₅₄FN₁₀O₁₀⁺ [M + H⁺] 937.4003, found 937.3991.

### Example 232

### Synthesis of HC90-113

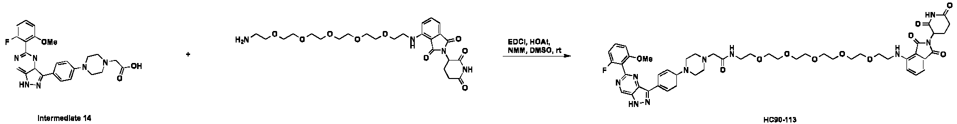

HC90-113 was synthesized following the standard procedure for preparing HC90-86 from Intermediate 14 (5.8 mg, 0.01 mmol, 1.0 equiv), 4-((17-amino-3,6,9,12,15-pentaoxaheptadecyl)amino)-2-(2,6-dioxopiperidin-3-yl)isoindoline-1,3-dione (7.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-113 was obtained as yellow solid in TFA salt form (5.7 mg, 52%). ¹H NMR (800 MHz, Methanol-*d*₄) δ 9.31 (s, 1H), 8.38 (d, *J* = 8.3 Hz, 2H), 7.53 - 7.45 (m, 2H), 7.11 (d, *J* = 8.4 Hz, 2H), 7.06 - 6.99 (m, 3H), 6.90 (t, *J* = 8.6 Hz, 1H), 5.06 (dd, *J* = 12.7, 5.4 Hz, 1H), 4.03 (s, 2H), 3.81 (s, 3H), 3.69 (t, *J* = 5.1 Hz, 2H), 3.65 (d, *J* = 7.9 Hz, 16H), 3.61 (t, *J =* 5.2 Hz, 2H), 3.51 - 3.43 (m, 12H), 2.85 (ddd, *J* = 18.1, 13.7, 5.3 Hz, 1H), 2.79 - 2.64 (m, 2H), 2.16 - 2.08 (m, 1H). HRMS calcd for C₄₉H₅₈FN₁₀O₁₁⁺ [M + H⁺] 981.4265, found 981.4251.

### Example 233

### Synthesis of HC90-117

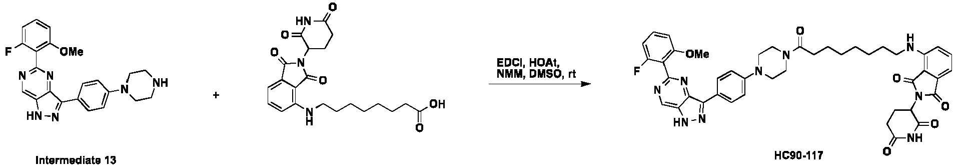

HC90-117 was synthesized following the standard procedure for preparing HC90-33 from Intermediate 13 (6.5 mg, 0.0125 mmol, 1.0 equiv), 8-((2-(2,6-dioxopiperidin-3-yl)-1,3-dioxoisoindolin-4-yl)amino)octanoic acid (6.6 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-117 was obtained as yellow solid in TFA salt form (4.2 mg, 37%). ¹H NMR (800 MHz, Acetone-d₆) δ 9.40 (s, 1H), 8.46 (d, *J* = 8.4 Hz, 2H), 7.60 (t, *J* = 7.8 Hz, 1H), 7.50 (q, *J =* 8.1 Hz, 1H), 7.12 (t, *J* = 8.9 Hz, 3H), 7.07 - 7.00 (m, 2H), 6.91 (t, *J =* 8.7 Hz, 1H), 5.08 (dd, *J* = 12.8, 5.6 Hz, 1H), 3.79 (s, 3H), 3.76- 3.64 (m, 4H), 3.40 (t, *J* = 7.1 Hz, 2H), 3.32 (s, 2H), 3.26 (s, 2H), 2.88 - 2.76 (m, 3H), 2.43 (t, *J* = 7.5 Hz, 2H), 2.29 - 2.20 (m, 1H), 1.73 (t, *J =* 7.3 Hz, 2H), 1.65 (t, *J* = 7.2 Hz, 2H), 1.54 - 1.37 (m, 6H). HRMS calcd for C₄₃H₄₅FN₉O₆⁺ [M + H⁺] 802.3471, found 802.3456.

### Example 234

### Synthesis of HC90-119

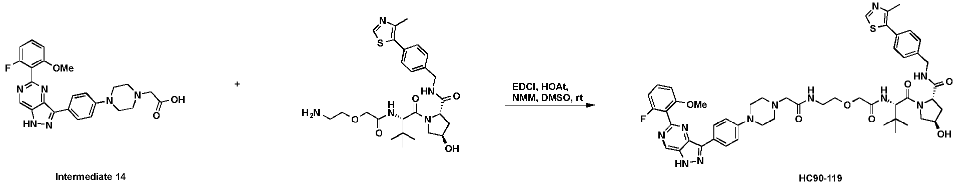

HC90-119 was synthesized following the standard procedure for preparing HC90-86 from Intermediate 14 (5.8 mg, 0.01 mmol, 1.0 equiv), (2*S*,4*R*)-1-((*S*)-2-(2-(2-aminoethoxy)acetamido)-3,3-dimethylbutanoyl)-4-hydroxy-N-(4-(4-methylthiazol-5-yl)benzyl)pyrrolidine-2-carboxamide (7.1 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-119 was obtained as yellow solid in TFA salt form (5.8 mg, 53%). ¹H NMR (800 MHz, Methanol-*d*₄) δ 9.31 (s, 1H), 8.85 (s, 1H), 8.41 (d, *J* = 8.2 Hz, 2H), 7.52 - 7.45 (m, 1H), 7.32 (s, 4H), 7.14 (d, *J* = 8.4 Hz, 2H), 7.01 (d, *J* = 8.5 Hz, 1H), 6.89 (t, *J* = 8.6 Hz, 1H), 4.77 (s, 1H), 4.58 (t, *J* = 8.3 Hz, 1H), 4.52 (t, *J* = 3.6 Hz, 1H), 4.43 (d, *J* = 15.4 Hz, 1H), 4.29 (d, *J* = 15.4 Hz, 1H), 4.15 -4.10 (m, 3H), 4.01 (d, *J* = 15.3 Hz, 1H), 3.93 (d, *J* = 11.1 Hz, 1H), 3.85 (dd, *J* = 11.2, 3.8 Hz, 1H), 3.79 (s, 3H), 3.73 (dd, *J* = 10.3, 3.9 Hz, 1H), 3.68 - 3.55 (m, 10H), 3.48 (dd, *J* = 11.7, 5.6 Hz, 1H), 2.41 (s, 3H), 2.30 (dd, *J* = 12.8, 8.0 Hz, 1H), 2.08 (ddd, *J* = 13.3, 9.0, 4.4 Hz, 1H), 1.10 (s, 9H). HRMS calcd for C₅₀H₅₉FN₁₁O₇S⁺ [M + H⁺] 976.4298, found 976.4273.

### Example 235

### Synthesis of HC90-120

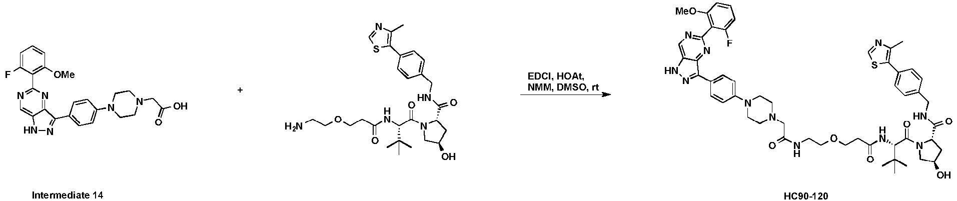

HC90-120 was synthesized following the standard procedure for preparing HC90-86 from Intermediate 14 (5.8 mg, 0.01 mmol, 1.0 equiv), (2*S*,4*R*)-1-((*S*)-2-(3-(2-aminoethoxy)propanamido)-3,3-dimethylbutanoyl)-4-hydroxy-*N*-(4-(4-methylthiazol-5-yl)benzyl)pyrrolidine-2-carboxamide (8.5 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-120 was obtained as yellow solid in TFA salt form (8.2 mg, 74%). ¹H NMR (800 MHz, Methanol-*d*₄) δ 9.31 (s, 1H), 8.88 (s, 1H), 8.42 (d, *J* = 8.4 Hz, 2H), 7.50 (q, *J* = 7.9 Hz, 1H), 7.42 (d, *J =* 8.7 Hz, 2H), 7.39 (d, *J =* 7.9 Hz, 2H), 7.15 (d, *J =* 8.4 Hz, 2H), 7.01 (d, *J =* 8.5 Hz, 1H), 6.90 (t, *J* = 8.6 Hz, 1H), 4.69 (s, 1H), 4.59 (t, *J* = 8.4 Hz, 1H), 4.52 (s, 1H), 4.46 (d*, J =* 15.4 Hz, 1H), 4.41 (d, *J* = 15.4 Hz, 1H), 4.06 (s, 2H), 3.94 (d, *J* = 11.0 Hz, 1H), 3.84 (dd, *J* = 11.0, 3.8 Hz, 1H), 3.80 (s, 3H), 3.77 - 3.74 (m, 2H), 3.66 - 3.46 (m, 12H), 2.61- 2.52 (m, 2H), 2.46 (s, 3H), 2.27 (dd, *J* = 13.2, 7.6 Hz, 1H), 2.10 (ddd, *J* = 13.4, 9.2, 4.5 Hz, 1H), 1.07 (s, 9H). HRMS calcd for C₅₁H₆₁FN₁₁O₇S⁺ [M + H⁺] 990.4455, found 990.4441.

### Example 236

### Synthesis of HC90-121

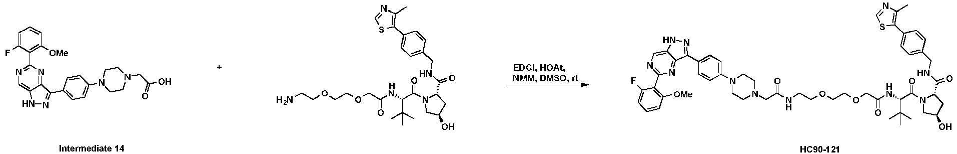

HC90-121 was synthesized following the standard procedure for preparing HC90-86 from Intermediate 14 (5.8 mg, 0.01 mmol, 1.0 equiv), (2*S*,4*R*)-1-((*S*)-2-(2-(2-(2-aminoethoxy)ethoxy)acetamido)-3,3-dimethylbutanoyl)-4-hydroxy-*N*-(4-(4-methylthiazol-5-yl)benzyl)pyrrolidine-2-carboxamide (6.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-121 was obtained as yellow solid in TFA salt form (7.5 mg, 66%). ¹H NMR (800 MHz, Methanol-*d*₄) δ 9.31 (s, 1H), 8.89 (s, 1H), 8.42 (d, *J* = 8.3 Hz, 2H), 7.50 (q, *J* = 7.9, 7.3 Hz, 1H), 7.41 (d, *J* = 8.0 Hz, 2H), 7.39 (d, *J* = 8.0 Hz, 2H), 7.15 (d, *J =* 8.4 Hz, 2H), 7.01 (d*, J =* 8.5 Hz, 1H), 6.90 (t, *J* = 8.6 Hz, 1H), 4.79 (s, 1H), 4.56 (t, *J* = 8.7 Hz, 1H), 4.52 (t, *J* = 3.5 Hz, 1H), 4.45 (d, *J =* 15.3 Hz, 1H), 4.41 (d, *J =* 15.4 Hz, 1H), 4.15 - 4.06 (m, 4H), 3.92 (d, *J =* 11.2 Hz, 1H), 3.86 (dd, *J* = 11.1, 3.7 Hz, 1H), 3.81 (s, 3H), 3.76 - 3.38 (m, 16H), 2.47 (s, 3H), 2.30 (dd, *J* = 13.4, 7.5 Hz, 1H), 2.14 - 2.07 (m, 1H), 1.07 (s, 9H). HRMS calcd for C₅₂H₆₃FN₁₁O₈S⁺ [M + H⁺] 1020.4560, found 1020.4549.

### Example 237

### Synthesis of HC90-122

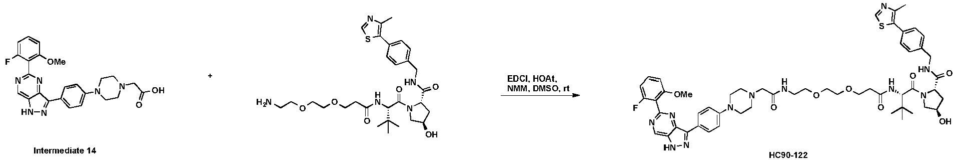

HC90-122 was synthesized following the standard procedure for preparing HC90-86 from Intermediate 14 (5.8 mg, 0.01 mmol, 1.0 equiv), (2S,4R)-1-((S)-2-(3-(2-(2-aminoethoxy)ethoxy)propanamido)-3,3-dimethylbutanoyl)-4-hydroxy-N-(4-(4-methylthiazol-5-yl)benzyl)pyrrolidine-2-carboxamide (9.0 mg, 0.011 mmol, 1.1 equiv), EDCI (3.8 mg, 0.02mmol, 2.0 equiv), HOAt (2.7 mg, 0.02 mmol, 2.0 equiv), and NMM (5.1 mg, 0.05 mmol, 5.0 equiv) in DMSO (1 mL). HC90-122 was obtained as yellow solid in TFA salt form (8.9 mg, 78%). ¹H NMR (800 MHz, Methanol-*d*₄) δ 9.32 (s, 1H), 8.89 (s, 1H), 8.42 (d, *J* = 8.4 Hz, 2H), 7.50 (q, *J* = 7.9 Hz, 1H), 7.45 (d, *J =* 7.8 Hz, 2H), 7.41 (d, *J =* 7.8 Hz, 2H), 7.16 (d, *J =* 8.4 Hz, 2H), 7.01 (d, *J =* 8.5 Hz, 1H), 6.90 (t, *J* = 8.6 Hz, 1H), 4.68 (s, 1H), 4.57 (t, *J* = 8.4 Hz, 1H), 4.53 - 4.48 (m, 1H), 4.40 (d, *J* = 15.4 Hz, 1H), 4.06 (s, 1H), 3.92 (d, *J* = 10.9 Hz, 1H), 3.85 - 3.74 (m, 6H), 3.70 - 3.46 (m, 18H), 2.63 - 2.58 (m, 1H), 2.55 - 2.50 (m, 1H), 2.47 (s, 3H), 2.25 (dd, *J* = 13.1, 7.6 Hz, 1H), 2.09 (ddd, *J* = 13.3, 9.0, 4.5 Hz, 1H), 1.06 (s, 9H). HRMS calcd for C₅₃H₆₅FN₁₁O₈S⁺ [M + H⁺] 1034.4717, found1034.4707.

### Example 238

### Synthesis of HC90-123

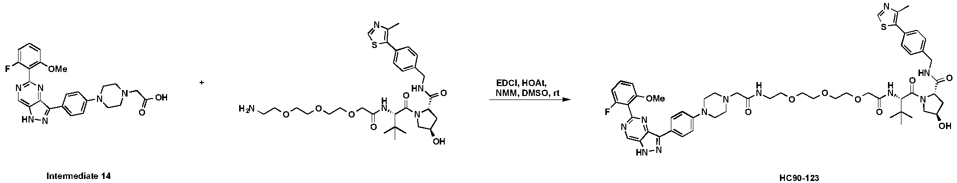

HC90-123 was synthesized following the standard procedure for preparing HC90-86 from Intermediate 14 (5.8 mg, 0.01 mmol, 1.0 equiv), (2*S*,4*R*)-1-((*S*)-14-amino-2-(*tert*-butyl)-4-oxo-6,9,12-trioxa-3-azatetradecanoyl)-4-hydroxy-N (4-(4-methylthiazol-5-yl)benzyl)pyrrolidine-2-carboxamide (9.3 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-123 was obtained as yellow solid in TFA salt form (7.3 mg, 62%). ¹H NMR (800 MHz, Methanol-*d*₄) δ 9.31 (s, 1H), 8.90 (s, 1H), 8.42 (d, *J* = 8.4 Hz, 2H), 7.50 (q, *J* = 8.0 Hz, 1H), 7.45 (d, *J* = 7.9 Hz, 2H), 7.41 (d, *J =* 7.9 Hz, 2H), 7.16 (d, *J =* 8.4 Hz, 2H), 7.01 (d, *J =* 8.5 Hz, 1H), 6.90 (t, *J =* 8.6 Hz, 1H), 4.71 (s, 1H), 4.61- 4.56 (m, 1H), 4.54 - 4.48 (m, 2H), 4.40 (d, *J* = 15.4 Hz, 1H), 4.15 - 4.07 (m, 2H), 4.05 (s, 2H), 3.89 (d, *J =* 10.9 Hz, 1H), 3.83 - 3.79 (m, 4H), 3.75 (t, *J =* 4.4 Hz, 2H), 3.74 - 3.71 (m, 2H), 3.70 - 3.68 (m, 2H), 3.67 - 3.64 (m, 2H), 3.63 - 3.44 (m, 12H), 2.48 (s, 3H), 2.26 (dd, *J* = 13.4, 7.6 Hz, 1H), 2.09 (ddd, *J* = 13.4, 9.3, 4.5 Hz, 1H), 1.06 (s, 9H). HRMS calcd for C₅₄H₆₇FN₁₁O₉S⁺ [M + H⁺] 1064.4822, found1064.4808.

### Example 239

### Synthesis of HC90-124

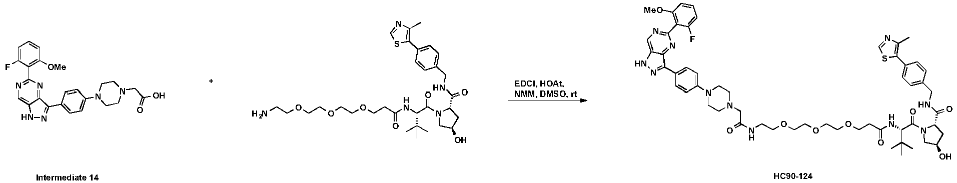

HC90-124 was synthesized following the standard procedure for preparing HC90-86 from Intermediate 14 (5.8 mg, 0.01 mmol, 1.0 equiv), (2*S*,4*R*)-1-((S)-1-amino-14-(*tert*-butyl)-12-oxo-3,6,9-trioxa-13-azapentadecan-15-oyl)-4-hydroxy-*N*-(4-(4-methylthiazol-5-yl)benzyl)pyrrolidine-2-carboxamide (9.5 mg, 0.011 mmol, 1.1 equiv), EDCI (3.8 mg, 0.02mmol, 2.0 equiv), HOAt (2.7 mg, 0.02 mmol, 2.0 equiv), and NMM (5.1 mg, 0.05 mmol, 5.0 equiv) in DMSO (1 mL). HC90-124 was obtained as yellow solid in TFA salt form (7.4 mg, 62%). ¹H NMR (800 MHz, Methanol-*d*₄) δ 9.31 (s, 1H), 8.90 (s, 1H), 8.42 (d, *J* = 8.3 Hz, 2H), 7.50 (q, *J* = 8.0 Hz, 1H), 7.45 (d, *J =* 7.9 Hz, 2H), 7.41 (d, *J =* 7.8 Hz, 2H), 7.16 (d, *J =* 8.4 Hz, 2H), 7.01 (d, *J =* 8.5 Hz, 1H), 6.90 (t, *J* = 8.6 Hz, 1H), 4.67 (s, 1H), 4.58 (t, *J* = 8.3 Hz, 1H), 4.54 - 4.48 (m, 2H), 4.38 (d, *J* = 15.4 Hz, 1H), 4.05 (s, 2H), 3.91 (d, *J* = 11.0 Hz, 1H), 3.84 - 3.73 (m, 6H), 3.70 - 3.46 (m, 20H), 2.64 - 2.59 (m, 1H), 2.53 - 2.45 (m, 4H), 2.27 - 2.22 (m, 1H), 2.09 (ddd, *J* = 13.3, 9.1, 4.6 Hz, 1H), 1.06 (s, 10H). HRMS calcd for C₅₅H₆₉FN₁₁O₉S⁺ [M + H⁺] 1078.4979, found 1078.4966.

### Example 240

### Synthesis of HC90-125

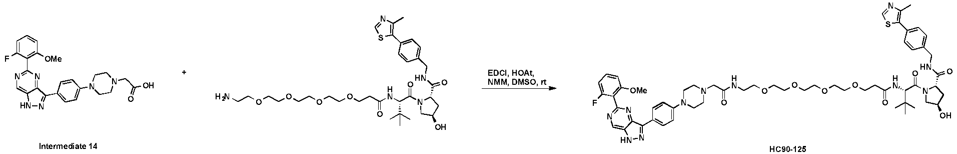

HC90-125 was synthesized following the standard procedure for preparing HC90-86 from Intermediate 14 (5.8 mg, 0.01 mmol, 1.0 equiv), (2*S*,4*R*)-1-((*S*)-1-amino-17-(*tert*-butyl)-15-oxo-3,6,9,12-tetraoxa-16-azaoctadecan-18-oyl)-4-hydroxy-*N*-(4-(4-methylthiazol-5-yl)benzyl)pyrrolidine-2-carboxamide (8.0 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-125 was obtained as yellow solid in TFA salt form (9.8 mg, 79%). ¹H NMR (800 MHz, Methanol-*d*₄) δ 9.31 (s, 1H), 8.90 (s, 1H), 8.42 (d, *J* = 8.4 Hz, 2H), 7.50 (q, *J* = 8.0 Hz, 1H), 7.46 (d, *J =* 7.8 Hz, 2H), 7.41 (d, *J =* 7.8 Hz, 2H), 7.17 (d, *J =* 8.4 Hz, 2H), 7.01 (d, *J =* 8.5 Hz, 1H), 6.90 (t, *J=* 8.6 Hz, 1H), 4.67 (s, 1H), 4.58 (t, *J=* 8.3 Hz, 1H), 4.54 - 4.50 (m, 2H), 4.38 (d, *J* = 15.4 Hz, 1H), 4.05 (s, 2H), 3.91 (d, *J* = 11.0 Hz, 1H), 3.83 - 3.78 (m, 4H), 3.74 (ddd, *J =* 16.0, 8.7, 4.6 Hz, 2H), 3.69 - 3.56 (m, 22H), 3.49 (t, *J* = 5.2 Hz, 2H), 2.64 - 2.56 (m, 1H), 2.51 - 2.46 (m, 4H), 2.24 (dd, *J* = 13.3, 7.6 Hz, 1H), 2.09 (ddd, *J* = 13.3, 9.0, 4.5 Hz, 1H), 1.05 (s, 9H). HRMS calcd for C₅₇H₇₃FN₁₁O₁₀S⁺ [M + H⁺] 1122.5241, found 1122.5230.

### Example 241

### Synthesis of HC90-126

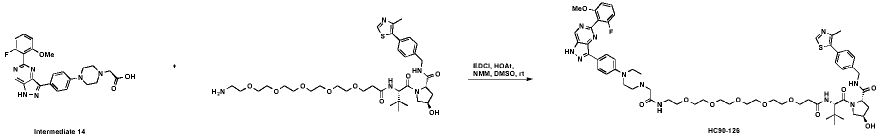

HC90-126 was synthesized following the standard procedure for preparing HC90-86 from Intermediate 14 (5.8 mg, 0.01 mmol, 1.0 equiv), (2*S*,4*R*)-1-((*S*)-1-amino-20-(*tert*-butyl)-18-oxo-3,6,9,12,15-pentaoxa-19-azahenicosan-21-oyl)-4-hydroxy-*N*-(4-(4-methylthiazol-5-yl)benzyl)pyrrolidine-2-carboxamide (10.5 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-126 was obtained as yellow solid in TFA salt form (6.3 mg, 49%). ¹H NMR (800 MHz, Methanol-*d*₄) δ 9.31 (s, 1H), 8.90 (s, 1H), 8.43 (d, *J=* 8.4 Hz, 2H), 7.50 (q, *J* = 8.0 Hz, 1H), 7.46 (d, *J =* 7.8 Hz, 2H), 7.41 (d, *J =* 7.8 Hz, 2H), 7.17 (d, *J* = 8.5 Hz, 2H), 7.01 (d, *J* = 8.5 Hz, 1H), 6.90 (t, *J* = 8.6 Hz, 1H), 4.67 (s, 1H), 4.58 (t, *J =* 8.5 Hz, 1H), 4.53 (d, *J* = 15.8 Hz, 1H), 4.52 - 4.50 (m, 1H), 4.38 (d, *J =* 15.4 Hz, 1H), 4.06 (s, 2H), 3.91 (d, *J =* 11.1 Hz, 1H), 3.81 - 3.78 (m, 4H), 3.74 (ddt, *J* = 19.3, 9.9, 4.1 Hz, 2H), 3.69 - 3.54 (m, 26H), 3.49 (t, *J* = 5.2 Hz, 2H), 2.59 (dt,*J* = 13.5, 6.6 Hz, 1H), 2.52- 2.46 (m, 4H), 2.24 (dd,*J* = 13.2, 7.7 Hz, 1H), 2.09 (ddd, *J* = 13.2, 8.8, 4.5 Hz, 1H), 1.05 (s, 9H). HRMS calcd for C₅₉H₇₇FN₁₁O₁₁S⁺ [M + H⁺] 1166.5503, found 1166.5491.

### Example 242

### Synthesis of HC90-127

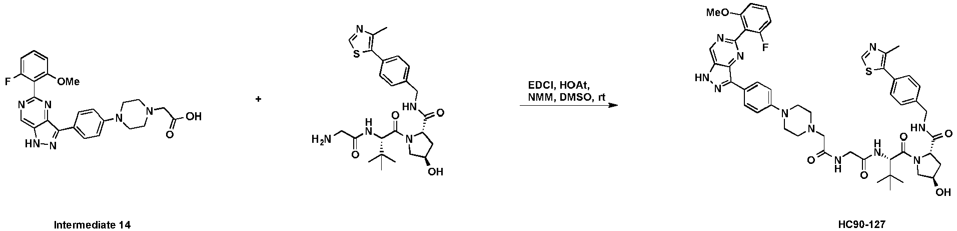

HC90-127 was synthesized following the standard procedure for preparing HC90-86 from Intermediate 14 (5.8 mg, 0.01 mmol, 1.0 equiv), (2*S*,4*R*)-1-((*S*)-2-(2-aminoacetamido)-3,3-dimethylbutanoyl)-4-hydroxy-*N*-(4-(4-methylthiazol-5-yl)benzyl)pyrrolidine-2-carboxamide (7.9 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-127 was obtained as yellow solid in TFA salt form (6.1 mg, 58%). ¹H NMR (800 MHz, Methanol-*d*₄) δ 9.32 (s, 1H), 8.89 (s, 1H), 8.41 (d, *J* = 8.3 Hz, 2H), 7.49 (dd, *J =* 21.6, 7.6 Hz, 3H), 7.41 (d, *J* = 7.8 Hz, 2H), 7.11 (d, *J =* 8.4 Hz, 2H), 7.01 (d, *J =* 8.5 Hz, 1H), 6.90 (t, *J =* 8.6 Hz, 1H), 4.69 (s, 1H), 4.61 - 4.55 (m, 2H), 4.54 (d, *J* = 4.3 Hz, 1H), 4.36 (d*, J* = 15.4 Hz, 1H), 4.13 - 4.02 (m, 4H), 3.90 (d, *J* = 10.9 Hz, 1H), 3.84 (dd, *J* = 11.1, 3.9 Hz, 1H), 3.81 (s, 3H), 3.67 - 3.49 (m, 8H), 2.46 (s, 3H), 2.25 (dd, *J* = 13.2, 7.8 Hz, 1H), 2.12 (ddd, *J* = 13.2, 8.9, 4.5 Hz, 1H), 1.08 (s, 9H). HRMS calcd for C₄₈H₅₅FN₁₁O₆S⁺ [M + H⁺] 932.4036, found 932.4026.

### Example 243

### Synthesis of HC90-128

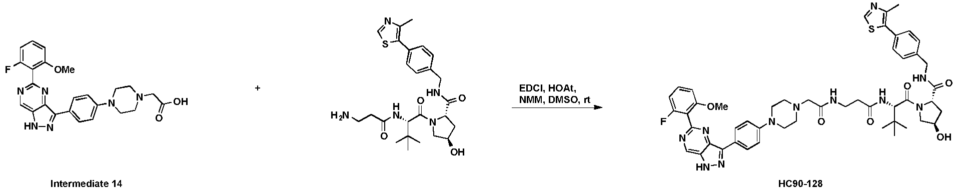

HC90-128 was synthesized following the standard procedure for preparing HC90-86 from Intermediate 14 (5.8 mg, 0.01 mmol, 1.0 equiv), (2*S*,4*R*)-1-((*S*)-2-(3-aminopropanamido)-3,3-dimethylbutanoyl)-4-hydroxy-*N*-(4-(4-methylthiazol-5-yl)benzyl)pyrrolidine-2-carboxamide (8.1 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-128 was obtained as yellow solid in TFA salt form (7.2 mg, 68%). ¹H NMR (800 MHz, Methanol-*d*₄) δ 9.31 (s, 1H), 8.86 (s, 1H), 8.40 (d, *J* = 8.3 Hz, 2H), 7.53 - 7.46 (m, 1H), 7.41 (d, *J* = 7.8 Hz, 2H), 7.36 (d, *J* = 7.8 Hz, 2H), 7.12 (d, *J* = 8.4 Hz, 2H), 7.01 (d, *J* = 8.3 Hz, 1H), 6.90 (t, *J* = 8.4 Hz, 1H), 4.64 (s, 1H), 4.60 - 4.53 (m, 2H), 4.48 (d, *J* = 15.5 Hz, 1H), 4.34 (d, *J* = 15.2 Hz, 1H), 4.03 - 3.93 (m, 3H), 3.84 - 3.78 (m, 4H), 3.68 - 3.41 (m, 10H), 2.70 - 2.60 (m, 1H), 2.53 - 2.48 (m, 1H), 2.44 (s, 3H), 2.31 - 2.24 (m, 1H), 2.15 - 2.08 (m, 1H), 1.08 (s, 9H). HRMS calcd for C₄₉H₅₇FN₁₁O₆S⁺ [M + H⁺] 946.4193, found 946.4182.

### Example 244

### Synthesis of HC90-129

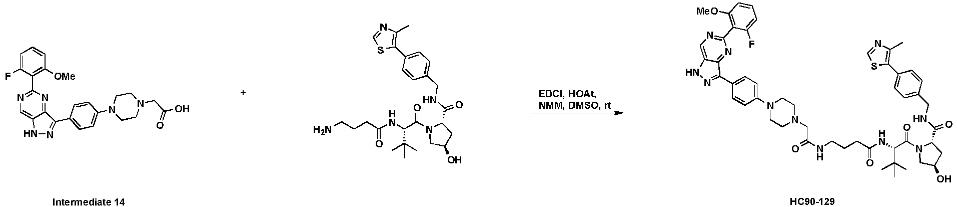

HC90-129 was synthesized following the standard procedure for preparing HC90-86 from Intermediate 14 (5.8 mg, 0.01 mmol, 1.0 equiv), (2S,4R)-1-((S)-2-(4-aminobutanamido)-3,3-dimethylbutanoyl)-4-hydroxy-*N*-(4-(4-methylthiazol-5-yl)benzyl)pyrrolidine-2-carboxamide (8.2 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-129 was obtained as yellow solid in TFA salt form (5.8 mg, 54%). ¹H NMR (800 MHz, Methanol-*d*₄) δ 9.32 (s, 1H), 8.88 (s, 1H), 8.42 (d, *J* = 8.3 Hz, 2H), 7.50 (q, *J =* 8.3 Hz, 1H), 7.46 (d, *J* = 7.8 Hz, 2H), 7.41 (d, *J* = 7.8 Hz, 2H), 7.15 (d, *J =* 8.4 Hz, 2H), 7.01 (d, *J* = 8.6 Hz, 1H), 6.90 (t, *J* = 8.6 Hz, 1H), 4.65 (s, 1H), 4.61 - 4.57 (m, 1H), 4.56 - 4.51 (m, 2H), 4.37 (d, *J* = 15.5 Hz, 1H), 4.03 (s, 2H), 3.95 (d, *J* = 11.0 Hz, 1H), 3.84 (dd,*J* = 10.9, 3.8 Hz, 1H), 3.81 (s, 3H), 3.70 -3.42 (m, 10H), 2.47 (s, 3H), 2.41 - 2.32 (m, 2H), 2.29 - 2.22 (m, 1H), 2.11 (ddd, *J* = 13.3, 8.9, 4.5 Hz, 1H), 1.92 - 1.82 (m, 2H), 1.08 (s, 9H). HRMS calcd for C₅₀H₅₉FN₁₁O₆S⁺ [M + H⁺] 960.4349, found 960.4336.

### Example 245

### Synthesis of HC90-130

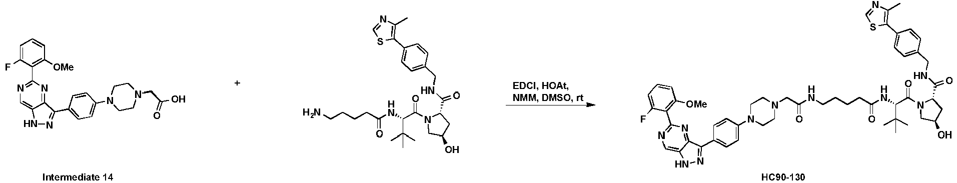

HC90-130 was synthesized following the standard procedure for preparing HC90-86 from Intermediate 14 (5.8 mg, 0.01 mmol, 1.0 equiv), (2*S*,4*R*)-1-((*S*)-2-(5-aminopentanamido)-3,3-dimethylbutanoyl)-4-hydroxy-N-(4-(4-methylthiazol-5-yl)benzyl)pyrrolidine-2-carboxamide (7.1 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-130 was obtained as yellow solid in TFA salt form (6.0 mg, 55%). ¹H NMR (800 MHz, Methanol-*d*₄) δ 9.31 (s, 1H), 8.87 (s, 1H), 8.42 (d, *J* = 8.5 Hz, 2H), 7.50 (q, *J* = 7.9 Hz, 1H), 7.45 (d, *J* = 7.8 Hz, 2H), 7.40 (d, *J =* 7.9 Hz, 2H), 7.16 (d, *J =* 8.4 Hz, 2H), 7.01 (dd, *J =* 8.4, 3.8 Hz, 1H), 6.89 (t, *J =* 8.6 Hz, 1H), 4.66 (s, 1H), 4.60 - 4.56 (m, 1H), 4.54 - 4.52 (m, 1H), 4.49 (d, *J* = 15.6 Hz, 1H), 4.39 (d, *J* = 15.5 Hz, 1H), 4.01 (s, 2H), 3.93 (d, *J =* 11.0 Hz, 1H), 3.83 (dd, *J =* 11.2, 3.9 Hz, 1H), 3.81 (d, *J* =4.3 Hz, 3H), 3.62 - 3.44 (m, 8H), 3.33 - 3.28 (m, 2H), 2.47 (s, 3H), 2.39 - 2.31 (m, 2H), 2.26 (dd, *J* = 12.7, 7.5 Hz, 1H), 2.10 (ddd, *J* = 13.2, 8.8, 4.4 Hz, 1H), 1.74 -1.55 (m, 4H), 1.07 (s, 9H). HRMS calcd for C₅₁H₆₁FN₁₁O₆S⁺ [M + H⁺] 974.4506, found 974.4498.

### Example 246

### Synthesis of HC90-131

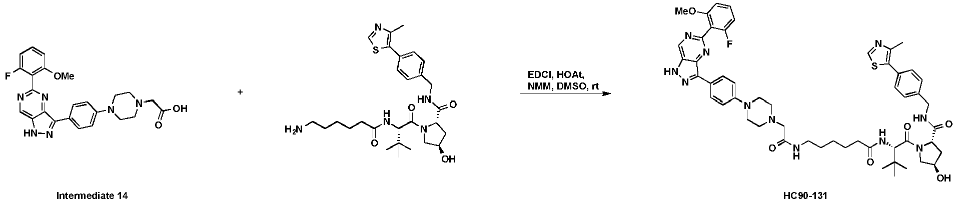

HC90-131 was synthesized following the standard procedure for preparing HC90-86 from Intermediate 14 (5.8 mg, 0.01 mmol, 1.0 equiv), (2*S*,4*R*)-1-((*S*)-2-(6-aminohexanamido)-3,3-dimethylbutanoyl)-4-hydroxy-N-(4-(4-methylthiazol-5-yl)benzyl)pyrrolidine-2-carboxamide (7.3 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-131 was obtained as yellow solid in TFA salt form (7.7 mg, 70%). ¹H NMR (800 MHz, Methanol-*d*₄) δ 9.31 (s, 1H), 8.88 (s, 1H), 8.42 (d, *J* = 8.3 Hz, 2H), 7.55 - 7.48 (m, 1H), 7.46 (d, *J =* 7.7 Hz, 2H), 7.41 (d*, J =* 7.8 Hz, 2H), 7.16 (d, *J =* 8.4 Hz, 2H), 7.01 (d, *J =* 8.5 Hz, 1H), 6.90 (t, *J =* 8.6 Hz, 1H), 4.66 (s, 1H), 4.59 (t, *J* = 8.3 Hz, 1H), 4.55 - 4.50 (m, 2H), 4.38 (d, *J* = 15.5 Hz, 1H), 4.02 (s, 2H), 3.93 (d, *J =* 11.0 Hz, 1H), 3.85 - 3.78 (m, 4H), 3.64- 3.45 (m, 8H), 3.32 - 3.28 (m, 2H), 2.48 (s, 3H), 2.37 - 2.28 (m, 2H), 2.25 (dd, *J* = 13.4, 7.7 Hz, 1H), 2.11 (ddd, *J* = 13.3, 9.0, 4.6 Hz, 1H), 1.67 (p*, J* = 7.5 Hz, 2H), 1.59 (p, *J =* 7.1 Hz, 2H), 1.40 (p, *J =* 7.7 Hz, 2H), 1.06 (s, 9H). HRMS calcd for C₅₂H₆₃FN₁₁O₆S⁺ [M + H⁺] 988.4662, found 988.4649.

### Example 247

### Synthesis of HC90-132

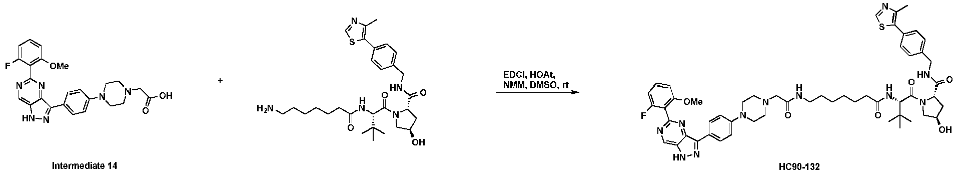

HC90-132 was synthesized following the standard procedure for preparing HC90-86 from Intermediate 14 (5.8 mg, 0.01 mmol, 1.0 equiv), (2*S*,4*R*)-1-((*S*)-2-(7-aminoheptanamido)-3,3-dimethylbutanoyl)-4-hydroxy-*N*-(4-(4-methylthiazol-5-yl)benzyl)pyrrolidine-2-carboxamide (7.4 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-132 was obtained as yellow solid in TFA salt form (4.3 mg, 39%). ¹H NMR (800 MHz, Methanol-*d*₄) δ 9.31 (s, 1H), 8.89 (s, 1H), 8.42 (d, *J* = 8.3 Hz, 2H), 7.50 (q, *J* = 8.1 Hz, 1H), 7.47 (d, *J* = 7.9 Hz, 2H), 7.42 (d, *J* = 7.9 Hz, 2H), 7.16 (d, *J* = 8.5 Hz, 2H), 7.01 (d, *J* = 8.5 Hz, 1H), 6.90 (t, *J* = 8.6 Hz, 1H), 4.67 (s, 1H), 4.59 (t, *J* = 8.3 Hz, 1H), 4.55 - 4.51 (m, 2H), 4.39 (d, *J* = 15.5 Hz, 1H), 4.00 (s, 2H), 3.93 (d, *J* = 10.9Hz, 1H), 3.83 (dd,*J* = 10.9, 3.8 Hz, 1H), 3.81 (s, 3H), 3.62-3.42 (m, 8H), 3.31-3.28 (m, 2H), 2.48 (s, 3H), 2.36 - 2.27 (m, 2H), 2.27 - 2.21 (m, 1H), 2.11 (ddd, *J* = 13.2, 8.9, 4.4 Hz, 1H), 1.71 - 1.62 (m, 2H), 1.58 (t, *J =* 7.3 Hz, 2H), 1.39 (d, *J =* 5.8 Hz, 4H), 1.06 (s, 9H). HRMS calcd for C₅₃H₆₅FN₁₁O₆S⁺ [M + H⁺] 1002.4819, found 1002.4808.

### Example 248

### Synthesis of HC90-133

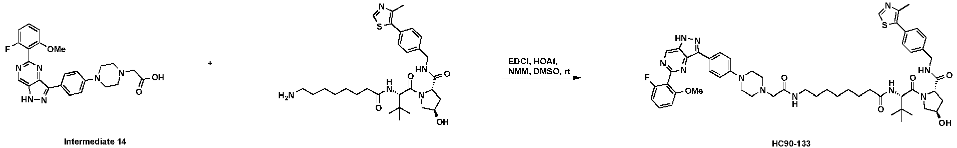

HC90-133 was synthesized following the standard procedure for preparing HC90-86 from Intermediate 14 (5.8 mg, 0.01 mmol, 1.0 equiv), (2S,4R)-1-((S)-2-(8-aminooctanamido)-3,3-dimethylbutanoyl)-4-hydroxy-N-(4-(4-methylthiazol-5-yl)benzyl)pyrrolidine-2-carboxamide (7.5 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-133 was obtained as yellow solid in TFA salt form (4.7 mg, 42%). ¹H NMR (800 MHz, Methanol-*d*₄) δ 9.31 (s, 1H), 8.89 (s, 1H), 8.43 (d, *J* = 8.3 Hz, 2H), 7.50 (q, *J =* 8.2 Hz, 1H), 7.47 (d, *J* = 7.8 Hz, 2H), 7.42 (d, *J* = 7.9 Hz, 2H), 7.17 (d, *J =* 8.5 Hz, 2H), 7.01 (d, *J* = 8.5 Hz, 1H), 6.90 (t, *J* = 8.7 Hz, 1H), 4.66 (s, 1H), 4.58 (t, *J =* 8.1 Hz, 1H), 4.56 - 4.51 (m, 2H), 4.38 (d, *J =* 15.5 Hz, 1H), 4.01 (s, 2H), 3.92 (d, *J* = 10.9 Hz, 1H), 3.85 - 3.79 (m, 4H), 3.65 - 3.43 (m, 8H), 3.30 (t, *J* = 7.1 Hz, 2H), 2.48 (s, 3H), 2.36 - 2.21 (m, 3H), 2.10 (ddd, *J* = 13.4, 9.1, 4.5 Hz, 1H), 1.69 - 1.61 (m, 2H), 1.61 - 1.54 (m, 2H), 1.38 (s, 6H), 1.05 (s, 9H). HRMS calcd for C₅₄H₆₇FN₁₁O₆S⁺ [M + H⁺] 1016.4975, found 1016.4961.

### Example 249

### Synthesis of HC90-134

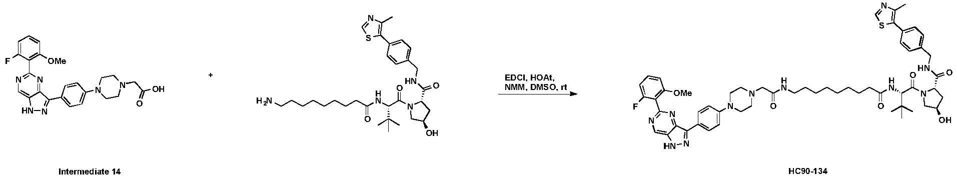

HC90-134 was synthesized following the standard procedure for preparing HC90-86 from Intermediate 14 (5.8 mg, 0.01 mmol, 1.0 equiv), (2S,4R)-1-((S)-2-(9-aminononanamido)-3,3-dimethylbutanoyl)-4-hydroxy-N-(4-(4-methylthiazol-5-yl)benzyl)pyrrolidine-2-carboxamide (7.7 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-134 was obtained as yellow solid in TFA salt form (9.5mg, 83%). ¹H NMR (800 MHz, Methanol-*d*₄) δ 9.31 (s, 1H), 8.89 (s, 1H), 8.42 (d, *J* = 8.3 Hz, 2H), 7.50 (q, *J* = 7.9 Hz, 1H), 7.47 (d, *J* = 8.0 Hz, 2H), 7.42 (d, *J =* 7.8 Hz, 2H), 7.16 (d, *J =* 8.3 Hz, 2H), 7.01 (d, *J =* 8.5 Hz, 1H), 6.90 (t, *J =* 8.6 Hz, 1H), 4.67 (s, 1H), 4.59 (t, *J =* 8.3 Hz, 1H), 4.56 - 4.50 (m, 2H), 4.38 (d, *J =* 15.4 Hz, 1H), 4.02 (s, 2H), 3.92 (d, *J* = 11.0 Hz, 1H), 3.83 (dd, *J* = 11.5, 3.9 Hz, 1H), 3.81 (s, 3H), 3.63 - 3.48 (m, 8H), 3.29 (t, *J* = 7.2 Hz, 2H), 2.48 (s, 3H), 2.37 - 2.29 (m, 1H), 2.29 - 2.22 (m, 2H), 2.10 (ddd, *J* = 13.4, 9.1, 4.6 Hz, 1H), 1.68 - 1.60 (m, 2H), 1.59 - 1.54 (m, 2H), 1.37 (s, 8H), 1.06 (s, 9H). HRMS calcd for C₅₅H₆₉FN₁₁O₆S⁺ [M + H⁺] 1030.5132, found 1030.5119.

### Example 250

### Synthesis of HC90-135

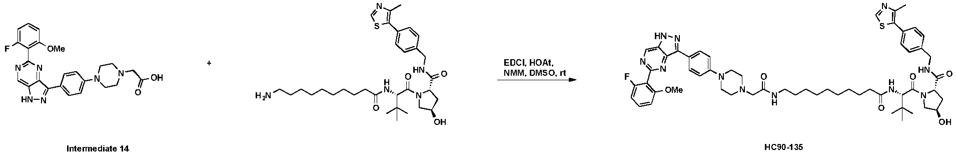

HC90-135 was synthesized following the standard procedure for preparing HC90-86 from Intermediate 14 (5.8 mg, 0.01 mmol, 1.0 equiv), (2*S*,4*R*)-1-((*S*)-2-(10-aminodecanamido)-3,3-dimethylbutanoyl)-4-hydroxy-*N*-(4-(4-methylthiazol-5-yl)benzyl)pyrrolidine-2-carboxamide (7.8 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-135 was obtained as yellow solid in TFA salt form (8.1 mg, 70%). ¹H NMR (800 MHz, Methanol-*d*₄) δ 9.31 (s, 1H), 8.90 (s, 1H), 8.43 (d, *J =* 8.3 Hz, 2H), 7.54 - 7.45 (m, 3H), 7.42 (d, *J =* 7.8 Hz, 2H), 7.17 (d, *J =* 8.4 Hz, 2H), 7.01 (d, *J =* 8.5 Hz, 1H), 6.90 (t, *J* = 8.6 Hz, 1H), 4.66 (s, 1H), 4.59 (t, *J =* 8.4 Hz, 1H), 4.55 (d, *J* = 15.5 Hz, 1H), 4.53 - 4.50 (m, 1H), 4.38 (d, *J* = 15.4 Hz, 1H), 4.02 (s, 2H), 3.92 (d, *J =* 11.0 Hz, 1H), 3.86 - 3.78 (m, 4H), 3.67 - 3.42 (m, 8H), 3.29 (t, *J =* 7.2 Hz, 2H), 2.49 (s, 3H), 2.37 - 2.29 (m, 1H), 2.29 - 2.21 (m, 2H), 2.10 (ddd, *J* = 13.2, 8.9, 4.5 Hz, 1H), 1.68 - 1.5 3 (m, 4H), 1.41 - 1.32 (m, 10H), 1.06 (s, 9H). HRMS calcd for C₅₆H₇₁FN₁₁O₆S⁺ [M + H⁺] 1044.5288, found 1044.5279.

### Example 251

### Synthesis of HC90-136

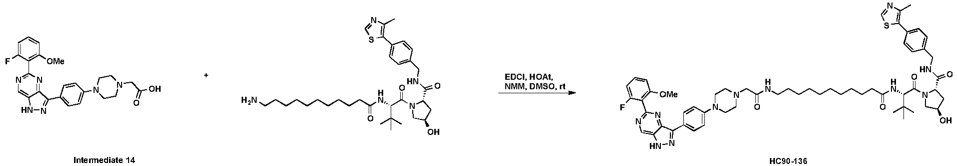

HC90-136 was synthesized following the standard procedure for preparing HC90-86 from Intermediate 14 (5.8 mg, 0.01 mmol, 1.0 equiv), (2*S*,4*R*)-1-((*S*)-2-(11-aminoundecanamido)-3,3-dimethylbutanoyl)-4-hydroxy-*N*-(4-(4-methylthiazol-5-yl)benzyl)pyrrolidine-2-carboxamide (8.0 mg, 0.011 mmol, 1.1 equiv), EDCI (3.8 mg, 0.02 mmol, 2.0 equiv), HOAt (2.7 mg, 0.02 mmol, 2.0 equiv), and NMM (5.1 mg, 0.05 mmol, 5.0 equiv) in DMSO (1 mL). HC90-136 was obtained as yellow solid in TFA salt form (9.1 mg, 78%). ¹H NMR (800 MHz, Methanol-*d*₄) δ 9.31 (s, 1H), 8.90 (s, 1H), 8.43 (d, *J =* 8.3 Hz, 2H), 7.53 - 7.46 (m, 3H), 7.42 (d, *J =* 7.8 Hz, 2H), 7.17 (d*, J =* 8.4 Hz, 2H), 7.01 (d, *J =* 8.5 Hz, 1H), 6.90 (t, *J* = 8.6 Hz, 1H), 4.66 (s, 1H), 4.59 (t, *J =* 8.3 Hz, 1H), 4.55 (d, *J* = 15.4 Hz, 1H), 4.52 (s, 1H), 4.38 (d, *J* = 15.4 Hz, 1H), 4.02 (s, 2H), 3.92 (d, *J =* 10.9 Hz, 1H), 3.83 (dd, *J* = 11.2, 4.0 Hz, 1H), 3.81 (s, 3H), 3.66 - 3.44 (m, 8H), 3.29 (t, *J* = 7.2 Hz, 2H), 2.49 (s, 3H), 2.36 - 2.29 (m, 1H), 2.30 - 2.22 (m, 2H), 2.11 (ddd, *J =* 13.3, 9.1, 4.6 Hz, 1H), 1.59 (dtd, *J* = 52.8, 15.7, 8.2 Hz, 4H), 1.41 - 1.29 (m, 12H), 1.06 (s, 9H). HRMS calcd for C₅₇H₇₃FN₁₁O₆S⁺ [M + H⁺] 1058.5445, found 1058.5434.

### Example 252

### Synthesis of HC90-60

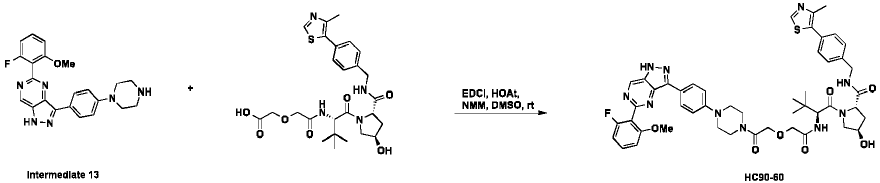

HC90-60 was synthesized following the standard procedure for preparing HC90-33 from Intermediate 13 (6.5 mg, 0.0125 mmol, 1.0 equiv), 2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)acetic acid (6.9 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-60 was obtained as yellow solid in TFA salt form (7.4 mg, 57%). HRMS calcd for C₄₈H₅₄FN₁₀O₇S⁺ [M + H⁺] 933.3876, found 933.3865.

### Example 253

### Synthesis of HC90-62

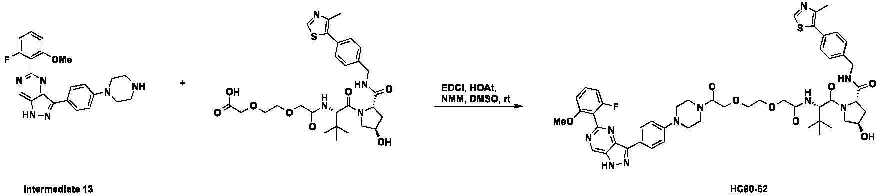

HC90-62 was synthesized following the standard procedure for preparing HC90-33 from Intermediate 13 (6.5 mg, 0.0125 mmol, 1.0 equiv), 2-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)acetic acid (7.4 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-62 was obtained as yellow solid in TFA salt form (8.0 mg, 59%). HRMS calcd for C₅₀H₅₈FN₁₀O₈S⁺ [M + H⁺] 977.4138, found 977.4129.

### Example 254

### Synthesis of HC90-63

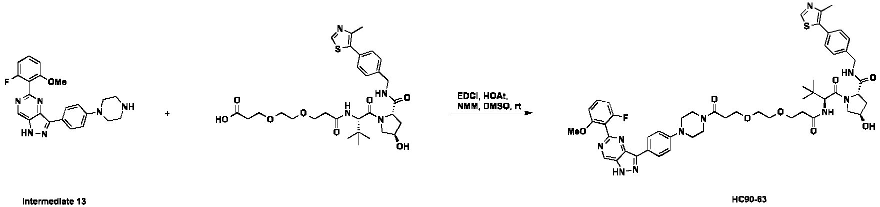

HC90-63 was synthesized following the standard procedure for preparing HC90-33 from Intermediate 13 (6.5 mg, 0.0125 mmol, 1.0 equiv), 3-(2-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)ethoxy)propanoic acid (7.8 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-63 was obtained as yellow solid in TFA salt form (6.6 mg, 47%). HRMS calcd for C₅₂H₆₂FN₁₀O₈S⁺ [M + H⁺] 1005.4451, found 1005.4440.

### Example 255

### Synthesis of HC90-64

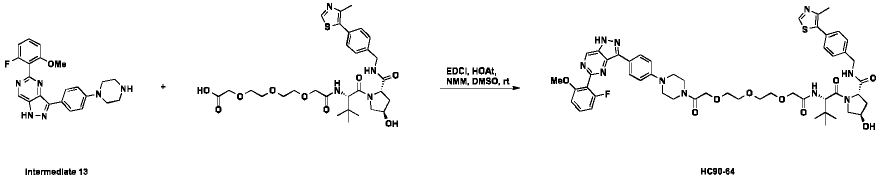

HC90-64 was synthesized following the standard procedure for preparing HC90-33 from Intermediate 13 (6.5 mg, 0.0125 mmol, 1.0 equiv), (*S*)-13-((2*S*,4*R*)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecanoic acid (8.0 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-64 was obtained as yellow solid in TFA salt form (8.8 mg, 62%). HRMS calcd for C₅₂H₆₂FN₁₀O₉S⁺ [M + H⁺] 1021.4400, found 1021.4388.

### Example 256

### Synthesis of HC90-65

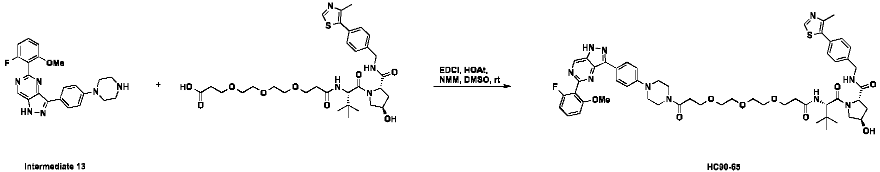

HC90-65 was synthesized following the standard procedure for preparing HC90-33 from Intermediate 13 (6.5 mg, 0.0125 mmol, 1.0 equiv), (*S*)-15-((2*S*,4*R*)-4-hydroxy-2-((4-(4-methylthiazol-5-yl)benzyl)carbamoyl)pyrrolidine-1 -carbonyl)-16,16-dimethyl-13-oxo-4,7,10-trioxa-14-azaheptadecanoic acid (8.3 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-65 was obtained as yellow solid in TFA salt form (7.2 mg, 50%). HRMS calcd for C₅₄H₆₆FN₁₀O₉S⁺ [M + H⁺] 1049.4713, found 1049.4703.

### Example 257

### Synthesis of HC90-67

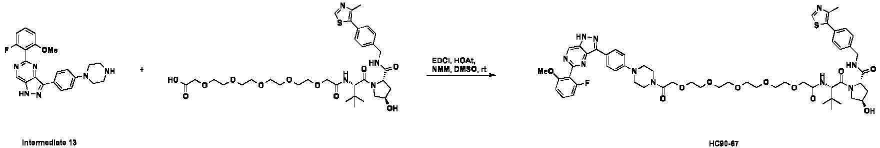

HC90-67 was synthesized following the standard procedure for preparing HC90-33 from Intermediate 13 (6.5 mg, 0.0125 mmol, 1.0 equiv), (*S*)-19-((2*S*,4*R*)-4-hydroxy-2-((4-(4-methylthiazol-5-yl)benzyl)carbamoyl)pyrrolidine-1-carbonyl)-20,20-dimethyl-17-oxo-3,6,9,12,15-pentaoxa-18-azahenicosanoic acid (9.1 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-67 was obtained as yellow solid in TFA salt form (9.4 mg, 61%). HRMS calcd for C₅₆H₇₀FN₁₀O₁₁S⁺ [M + H⁺] 1109.4925, found 1109.4918.

### Example 258

### Synthesis of HC90-68

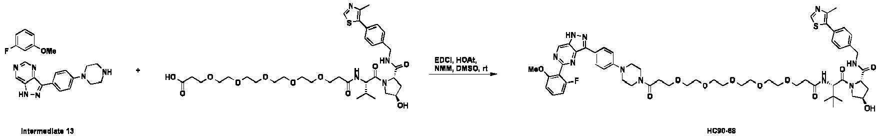

HC90-68 was synthesized following the standard procedure for preparing HC90-33 from Intermediate 13 (6.5 mg, 0.0125 mmol, 1.0 equiv), (*S*)-21-((2*S*,4*R*)-4-hydroxy-2-((4-(4-methylthiazol-5-yl)benzyl)carbamoyl)pyrrolidine-1-carbonyl)-22,22-dimethyl-1 9-oxo-4,7,10,13,16-pentaoxa-20-azatricosanoic acid (9.4 mg, 0.0125 mmol, 1.0 equiv), EDCI (3.6 mg, 0.019 mmol, 1.5 equiv), HOAt (2.6 mg, 0.019 mmol, 1.5 equiv), and NMM (5.0 mg, 0.05 mmol, 4.0 equiv) in DMSO (1 mL). HC90-68 was obtained as yellow solid in TFA salt form (8.8 mg, 56%). HRMS calcd for C₅₈H₇₄FN₁₀O₁₁S⁺ [M + H⁺] 1137.5238, found 1137.5229.

### Example 259

### Synthesis of intermediate 15

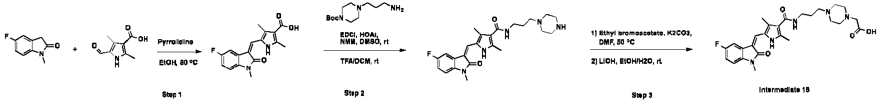

### Step 1: (Z)-5-((5-fluoro-1-methyl-2-oxoindolin-3-ylidene)methyl)-2,4-dimethyl-1H-pyrrole-3-carboxylic acid

A solution of 5-fluoro-1-methylindolin-2-one (82 mg, 0.5 mmol, 1.0 eq), 5-formyl-2,4-dimethyl-1*H-*pyrrole-3-carboxylic acid (84 g, 0.5 mmol, 1.0 eq), and pyrrolidine (71 mg, 1.0 mmol, 2.0 eq) in ethanol (2 mL) was heated to 80 °C for 3 h. Upon cooling to room temperature, HCl (1 M, 1.5 mL) was added to the suspension and the resulted crude precipitate was recovered by suction filtration, washed with ethanol (2 mL) and petroleum ether (10 mL) to afford (*Z*)-5-((5-fluoro-1-methyl-2-oxoindolin-3-ylidene)methyl)-2,4-dimethyl-1*H*-pyrrole-3-carboxylic acid as a yellow powder (155 mg, 99% yield). ESI m/z =315.1 [M + H⁺].

### Step 2: (Z)-5-((5-fluoro-1-methyl-2-oxoindolin-3-ylidene)methyl)-2,4-dimethyl-N (3-(piperazin-1-yl)propyl)-1H-pyrrole-3-carboxamide

To a solution of (*Z*)-5-((5-fluoro-1-methyl-2-oxoindolin-3-ylidene)methyl)-2,4-dimethyl-1*H-*pyrrole-3-carboxylic acid (79 mg, 0.25 mmol, 1.0 equiv) in DMSO (2 mL) were added *tert*-butyl 4-(3-aminopropyl)piperazine-1-carboxylate (73 mg, 0.30 mmol, 1.2 equiv), EDCI (1-ethyl-3-(3-dimethylaminopropyl)carbodiimide) (96 mg, 0.50 mmol, 2.0 equiv), HOAt (1-hydroxy-7-azabenzo-triazole) (68 mg, 0.50 mmol, 2.0 equiv), and NMM (N-Methylmorpholine) (101 mg, 1.00 mmol, 4.0 equiv). After being stirred overnight at room temperature, H₂O (10 mL) was added to the solution and precipitates was filtered. The solid was washed with water and dried under vacuum. The obtained solid was dissolved in DCM (4 mL). To the resulting solution was added TFA (2 ml). After being stirred for 1 h at room temperature, the reaction mixture was concentrated and the residue was purified by reverse phase C18 column (10% - 100% methanol / 0.1% TFA in H₂O) to afford (*Z*)-5-((5-fluoro-1-methyl-2-oxoindolin-3-ylidene)methyl)-2,4-dimethyl-*N*-(3-(piperazin-1-yl)propyl)-1*H*-pyrrole-3-carboxamide as yellow solid in TFA salt form (127 mg, 76% for two steps). ESI m/z =440.2 [M + H⁺].

### Step 3: intermediate 15

To a solution of (*Z*)-5-((5-fluoro-1-methyl-2-oxoindolin-3-ylidene)methyl)-2,4-dimethyl-N-(3-(piperazin-1-yl)propyl)-1*H*-pyrrole-3-carboxamide (127 mg, 0.19 mmol, 1.0 equiv) in DMF (2 mL) was added potassium carbonate (131 mg, 0.95 mmol, 5.0 equiv) and tert-butyl bromoacetate (44 mg, 0.23 mmol, 1.2 equiv). Then the mixture was stirred at 40 °C for 2 h. Water (10 mL) was added to quench the reaction. The mixture was extracted with ethyl acetate (3 x 10 mL). The organic layer was combined and washed with brine. Dried over Na₂SO₄, filtered and evaporated to afford oil. The obtained residue was dissolved in DCM (4 mL). To the solution was added TFA (2 mL). Stirred at room temperature for 1 h and concentrated under vacuum. The residue was purified preparative HPLC (10%-100% acetonitrile/ 0.1% TFA in H₂O) to afford intermediate 15 as a yellow solid (82 mg, 59% for two steps) in TFA salt form. ¹H NMR (600 MHz, Methanol-*d*₄) δ 7.64 (s, 1H), 7.51 (d, *J* = 8.8 Hz, 1H), 7.04 - 6.95 (m, 2H), 3.51 (t, *J* = 6.5 Hz, 2H), 3.46 (s, 2H), 3.38 (s, 3H), 3.35 - 3.26 (m, 8H), 3.22 (t, *J=* 7.5 Hz, 2H), 2.55 (s, 3H), 2.50 (s, 3H), 2.12 - 2.04 (m, 2H). ESI m/z =498.3 [M + H⁺].

### Example 260

### Synthesis of HC90-114

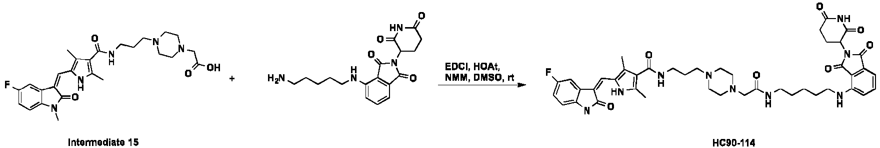

HC90-114 was synthesized following the standard procedure for preparing HC58-53 from Intermediate 15 (9.4 mg, 0.013 mmol, 1.0 equiv), 4-((5-aminooctyl)amino)-2-(2,6-dioxopiperidin-3-yl)isoindoline-1,3-dione (6.8 mg, 0.0143 mmol, 1.1 equiv), EDCI (5.0 mg, 0.026 mmol, 2.0 equiv), HOAt (3.5 mg, 0.026 mmol, 2.0 equiv), and NMM (8.0 mg, 0.078 mmol, 6.0 equiv) in DMSO (1 mL). HC90-114 was obtained as yellow solid in TFA salt form (8.9 mg, 64%). ¹H NMR (600 MHz, Methanol-*d*₄) δ 7.58 - 7.53 (m, 2H), 7.45 (dd, *J* = 8.9, 2.2 Hz, 1H), 7.04 (t, *J* = 7.5 Hz, 2H), 6.98 - 6.92 (m, 2H), 5.06 (dd, *J* = 12.7, 5.5 Hz, 1H), 3.51 (t, *J =* 6.5 Hz, 2H), 3.39 - 3.31 (m, 13H), 3.29 (t, *J =* 6.7 Hz, 2H), 3.24 - 3.19 (m, 4H), 2.85 (ddd, *J =* 17.5, 13.9, 5.3 Hz, 1H), 2.79 - 2.66 (m, 2H), 2.53 (s, 3H), 2.47 (s, 3H), 2.15 - 2.02 (m, 3H), 1.71 (p, *J =* 6.9 Hz, 2H), 1.62 (p, *J* = 7.0 Hz, 2H), 1.52 - 1.43 (m, 2H). HRMS calcd for C₄₄H₅₃FN₉O₇⁺ [M + H⁺] 838.4046, found 838.4037.

### Example 261

### Synthesis of HC90-115

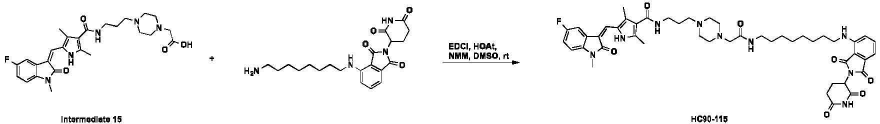

HC90-115 was synthesized following the standard procedure for preparing HC58-53 from Intermediate 15 (9.4 mg, 0.013 mmol, 1.0 equiv), 4-((8-aminooctyl)amino)-2-(2,6-dioxopiperidin-3-yl)isoindoline-1,3-dione (7.4 mg, 0.0143 mmol, 1.1 equiv), EDCI (5.0 mg, 0.026 mmol, 2.0 equiv), HOAt (3.5 mg, 0.026 mmol, 2.0 equiv), and NMM (8.0 mg, 0.078 mmol, 6.0 equiv) in DMSO (1 mL). HC90-115 was obtained as yellow solid in TFA salt form (9.3 mg, 65%). ¹H NMR (600 MHz, Methanol-*d*₄) δ 7.57 - 7.48 (m, 2H), 7.43 (dd, *J =* 8.8, 2.3 Hz, 1H), 7.01 (dd, *J =* 7.8, 2.4 Hz, 2H), 6.97-6.88 (m, 2H), 5.06 (dd, *J* = 12.8, 5.5 Hz, 1H), 3.51 (t, *J* = 6.6 Hz, 2H), 3.37 (s, 3H), 3.34 - 3.28 (m, 10H), 3.26 - 3.19 (m, 6H), 2.87 (ddd, *J =* 17.5, 13.9, 5.3 Hz, 1H), 2.80 - 2.69 (m, 2H), 2.51 (s, 3H), 2.46 (s, 3H), 2.15 - 2.05 (m, 3H), 1.66 (p, *J* = 7.1 Hz, 2H), 1.54 (p, *J =* 7.1 Hz, 2H), 1.47 - 1.29 (m, 8H). HRMS calcd for C₄₇H₅₉FN₉O₇⁺ [M + H⁺] 880.4516, found 880.4515.

### Example 262

### Synthesis of HC90-118

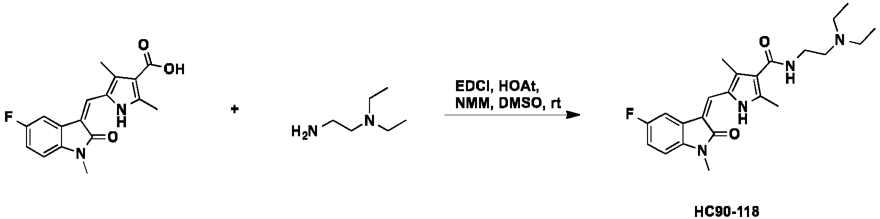

To a solution of (*Z*)-5-((5-fluoro-1-methyl-2-oxoindolin-3-ylidene)methyl)-2,4-dimethyl-1*H-*pyrrole-3-carboxylic acid (9.4 mg, 0.03 mmol, 1.0 equiv) in DMSO (1 mL) were added *N*¹,*N*¹-diethylethane-1,2-diamine (7.0 mg, 0.06 mmol, 2.0 equiv), EDCI (1-ethyl-3-(3-dimethylaminopropyl)carbodiimide) (11.5 mg, 0.06 mmol, 2.0 equiv), HOAt (1-hydroxy-7-azabenzo-triazole) (8.2 mg, 0.06 mmol, 2.0 equiv), and NMM (N-Methylmorpholine) (9.1 mg, 0.09 mmol, 3.0 equiv). After being stirred overnight at room temperature, the resulting mixture was purified by preparative HPLC (10%-100% methanol / 0.1% TFA in H₂O) to afford HC90-118 as yellow solid in TFA salt form (12.0 mg, 76%). ¹H NMR (600 MHz, Acetone-*d*₆) δ 10.67 (s, 1H), 7.89 (s, 1H), 7.74 (s, 1H), 7.67 - 7.54 (m, 1H), 7.02 - 6.98 (m, 2H), 3.90 (q, *J* = 5.5 Hz, 2H), 3.61 - 3.54 (m, 2H), 3.52 - 3.44 (m, 4H), 3.36 (s, 3H), 2.59 (s, 3H), 2.53 (s, 3H), 1.44 (t, *J* = 7.2 Hz, 6H). ESI m/z =413.2 [M + H⁺].

### Example 263

### Synthesis of HC90-168

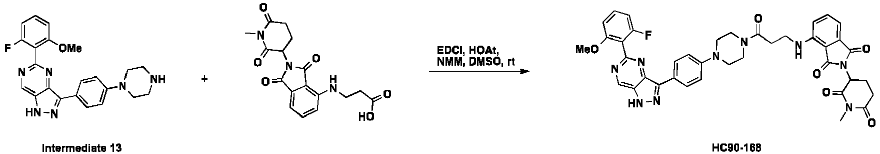

HC90-168 was synthesized following the standard procedure for preparing HC90-33 from Intermediate 13 (5.2 mg, 0.01 mmol, 1.0 equiv), 3-((2-(1-methyl-2,6-dioxopiperidin-3-yl)-1,3-dioxoisoindolin-4-yl)amino)propanoic acid (3.6 mg, 0.01 mmol, 1.0 equiv), EDCI (3.8 mg, 0.02 mmol, 2.0 equiv), HOAt (2.7 mg, 0.02 mmol, 2.0 equiv), and NMM (4.0 mg, 0.04 mmol, 4.0 equiv) in DMSO (1 mL). HC90-168 was obtained as yellow solid in TFA salt form (4.8 mg, 64%). ¹H NMR (600 MHz, Acetone-*d*₆) δ 9.40 (s, 1H), 8.45 (d, *J =* 8.9 Hz, 2H), 7.61 (dd, *J =* 8.6, 7.1 Hz, 1H), 7.50 (td, *J* = 8.5, 6.7 Hz, 1H), 7.18 (d, *J* = 8.6 Hz, 1H), 7.15 - 7.09 (m, 2H), 7.08 - 7.00 (m, 2H), 6.91 (t, *J* = 8.7 Hz, 1H), 5.07 (dd, *J* = 13.1, 5.4 Hz, 1H), 3.79 (s, 3H), 3.77 - 3.68 (m, 6H), 3.31 (t, *J =* 5.3 Hz, 2H), 3.27 (t, *J =* 5.3 Hz, 2H), 3.09 (s, 3H), 2.97 (ddd, *J =* 17.3, 14.0, 5.3 Hz, 1H), 2.90 - 2.83 (m, 3H), 2.73 (qd, *J =* 13.2, 4.6 Hz, 1H), 2.23 - 2.13 (m, 1H). HRMS calcd for C₃₉H₃₇FN₉O₆+ [M + H⁺] 746.2845, found 746.2834.

### Example 264

### Synthesis of HC90-169

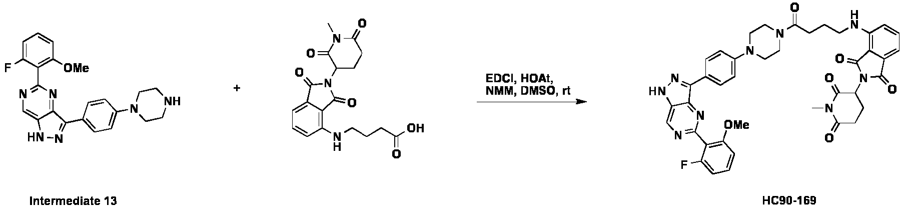

HC90-169 was synthesized following the standard procedure for preparing HC90-33 from Intermediate 13 (5.2 mg, 0.01 mmol, 1.0 equiv), 4-((2-(1-methyl-2,6-dioxopiperidin-3-yl)-1,3-dioxoisoindolin-4-yl)amino)butanoic acid (3.8 mg, 0.01 mmol, 1.0 equiv), EDCI (3.8 mg, 0.02 mmol, 2.0 equiv), HOAt (2.7 mg, 0.02 mmol, 2.0 equiv), and NMM (4.0 mg, 0.04 mmol, 4.0 equiv) in DMSO (1 mL). HC90-169 was obtained as yellow solid in TFA salt form (4.7 mg, 54%). ¹H NMR (600 MHz, Acetone-d₆) δ 9.41 (s, 1H), 8.48 - 8.44 (m, 2H), 7.61 (ddd, *J* = 8.4, 6.9, 1.2 Hz, 1H), 7.50 (dt, *J =* 9.4, 7.6 Hz, 1H), 7.23 (d, *J =* 8.5 Hz, 1H), 7.13 (d, *J =* 8.4 Hz, 2H), 7.03 (t, *J =* 7.3 Hz, 2H), 6.91 (t, *J =* 8.7 Hz, 1H), 5.08 (dd, *J =* 13.0, 5.4 Hz, 1H), 3.79 (s, 3H), 3.77 - 3.72 (m, 4H), 3.49 (t, *J* = 7.1 Hz, 2H), 3.34 - 3.25 (m, 4H), 3.10 (s, 3H), 2.99 (ddd, *J =* 18.6, 13.9, 5.3 Hz, 1H), 2.89 (dt, *J =* 17.4, 3.5 Hz, 1H), 2.75 (qd, *J =* 13.3, 4.5 Hz, 1H), 2.60 (t, *J* = 6.8 Hz, 2H), 2.24 - 2.15 (m, 1H), 2.02 (p, *J* = 7.0 Hz, 2H). HRMS calcd for C₄₀H₃₉FN₉O₆⁺ [M + H⁺] 760.3002, found 760.2991.
Certain compounds disclosed herein have the structures shown in Table 1. Compounds shown in Table 1 that do not fall within the scope of the claims, are not part of the invention.

**Table 1**

| Examples | Compound code | Structure | Chemical Name |
|---|---|---|---|
| 2 | HC58-18 | 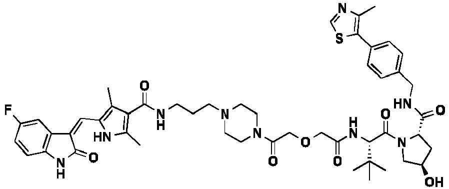 | 5-(((*Z*)-5-fluoro-2-oxoindolin-3-yiidene)methyl)-*N*-(3-(4-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)acetyl)piperazin-1-yl)propyl)-2,4-dimethyl-1*H*-pyrrole-3-carboxamide |
| 3 | HC58-19 | 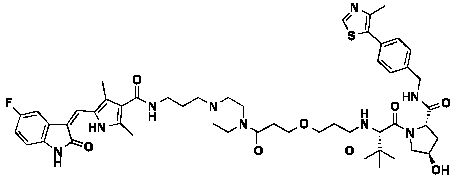 | 5-(((*Z*)-5-fluoro-2-oxoindolin-3-yiidene)methyl)-*N*-(3-(4-(3-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)propanoyl)piperazin-1-yl)propyl)-2,4-dimethyl-1*H-*pyrrole-3-carboxamide |
| 4 | HC58-20 | 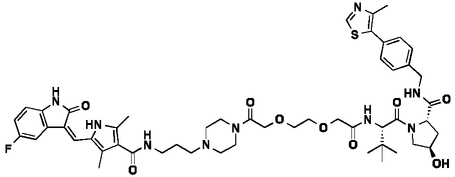 | 5-(((*Z*)-5-fluoro-2-oxoindolin-3-ylidene)methyl)-*N*-(3-(4-(2-(2-(2-(((*S*)-1-((2*S*,4*R*)-4-hydrooy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)acetyl)piperazin -1-yl)propyl)-2,4-dimethyl-1*H-*pyrrole-3-carboxamide |
| 5 | HC58-22 | 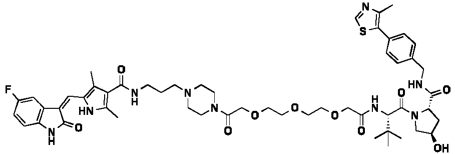 | 5-(((*Z*)-5-fluoro-2-oxoindolin-3-ylidene)methyl)-*N*-(3-(4-((*S*)-13-((2*S*,4*R*)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecanoyl)piperazin-1-yl)propyl)-2,4-dimethyl-1*H*-pyrrole-3-carboxamide |
| 6 | HC58-23 | 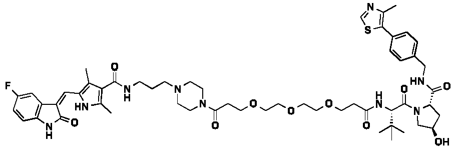 | 5-(((*Z*)-5-fluoro-2-oxoindolin-3-ylidene)methyl)-*N*-(3-(4-((*S*)-15-((2*S*,4*R*)-4-hydroxy-2-((4-(4-methylthiazol-5-yl)benzyl)carbamoyl)pyrrolidine-1-carbonyl)-16,16-dimethyl-13-oxo-4,7,10-trioxa-14-azaheptadecanoyl)piperazin-1-yl)propyl)-2,4-dimethyl-1*H*-pyrrole-3-carboxamide |
| 7 | HC58-24 | 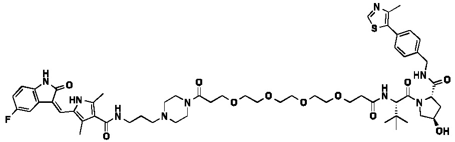 | 5-(((*Z*)-5-fluoro-2-oxoindolin-3-ylidene)methyl)-*N*-(3-(4-((*S*)-18-((2*S*,4*R*)-4-hydroxy-2-((4-(4-methylthiazol-5-yl)benzyl)carbamoyl)pyrrolidine-1-carbonyl)-19,19-dimethyl-16-oxo-4,7,10,13-tetraoxa-17-azaicosanoyl)piperazin-1-yl)propyl)-2,4-dimethyl-1*H*-pyrrole-3-carboxamide |
| 8 | HC58-25 | 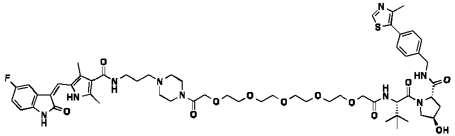 | 5-(((*Z*)-5-fluoro-2-oxoindolin-3-ylidene)methyl)-*N*-(3-(4-((*S*)-19-((2*S*,4*R*)-4-hydroxy-2-((4-(4-methylthiazol-5-yl)benzyl)carbamoyl)pyrrolidine-1-carbonyl)-20,20-dimethyl-17-oxo-3,6,9,12,15-pentaoxa-18-azahenicosanoyl)piperazin-1-yl)propyl)-2,4-dimethyl-1*H*-pyrrole-3-carboxamide |
| 9 | HC58-26 | 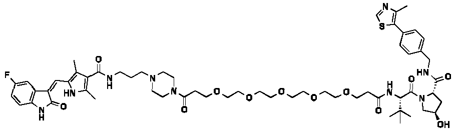 | 5-(((*Z*)-5-fluoro-2-oxoindolin-3-ylidene)methyl)-*N*-(3-(4-((*S*)-21-((2*S*,4*R*)-4-hydroxy-2-((4-(4-methylthiazol-5-yl)benzyl)carbamoyl)pyrrolidine-1-carbonyl)-22,22-dimethyl-19-oxo-4,7,10,13,16-pentaoxa-20-azatricosanoyl)piperazin-1-yl)propyl)-2,4-dimethyl-1*H*-pyrrole-3-carboxamide |
| 10 | HC58-27 | 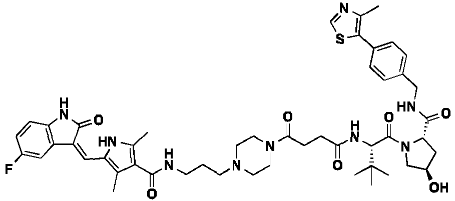 | 5-(((*Z*)-5-fluoro-2-oxoindolin-3-y[idene)methyl)-*N*-(3-(4-(4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutanoyl)piperazin-1-yl)propyl)-2,4-dimethyl-1*H*-pyrrole-3-carboxamide |
| 11 | HC58-28 | 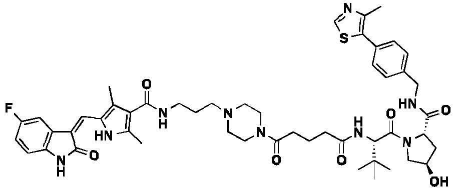 | 5-(((*Z*)-5-fluoro-2-oxoindolin-3-ylidene)methyl)-*N*-(3-(4-(5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentanoyl)piperazin-1-yl)propyl)-2,4-dimethyl-1*H*-pyrrole-3-carboxamide |
| 12 | HC58-29 | 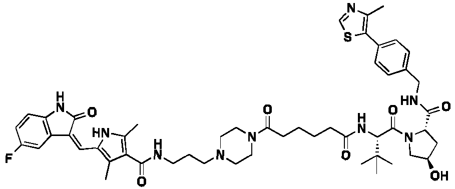 | 5-(((*Z*)-5-fluoro-2-oxoindolin-3-ylidene)methyl)-*N*-(3-(4-(6-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-6-oxohexanoyl)piperazin-1-yl)propyl)-2,4-dimethyl-1*H*-pyrrole-3-carboxamide |
| 13 | HC58-30 | 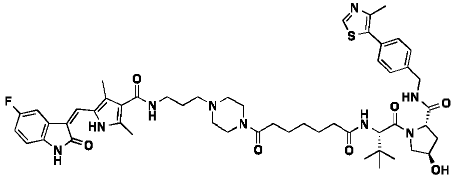 | 5-(((*Z*)-5-fluoro-2-oxoindolin-3-ylidene)methyl)-*N*-(3-(4-(7-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-7-oxoheptanoyl)piperazin-1-yl)propyl)-2,4-dimethyl-1*H*-pyrrole-3-carboxamide |
| 14 | HC58-31 | 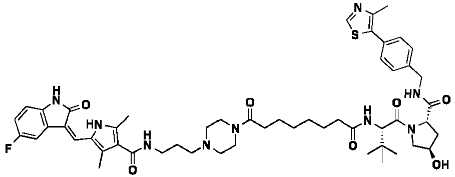 | 5-(((*Z*)-5-fluoro-2-oxoindolin-3-ylidene)methyl)-*N*-(3-(4-(8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctanoyl)piperazin-1-yl)propyl)-2,4-dimethyl-1*H-*pyrrole-3-carboxamide |
| 15 | HC58-32 | 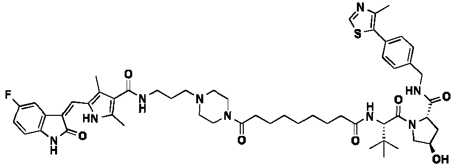 | 5-(((*Z*)-5-fluoro-2-oxoindolin-3-ylidene)methyl)-*N*-(3-(4-(9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononanoyl)piperazin-1-yl)propyl)-2,4-dimethyl-1*H*-pyrrole-3-carboxamide |
| 16 | HC58-33 | 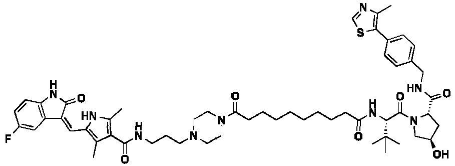 | 5-(((*Z*)-5-fluoro-2-oxoindolin-3-ylidene)methyl)-*N*-(3-(4-(10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecanoyl)piperazin-1-yl)propyl)-2,4-dimethyl-1*H*-pyrrole-3-carboxamide |
| 17 | HC58-34 | 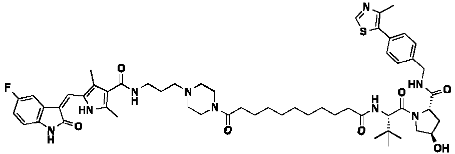 | 5-(((*Z*)-5-fluoro-2-oxoindolin-3-ylidene)methyl)-*N*-(3-(4-(11-(((S)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecanoyl)piperazin-1-yl)propyl)-2,4-dimethyl-1*H*-pyrrole-3-carboxamide |
| 18 | HC58-35 | 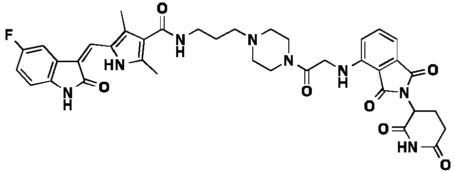 | (Z)-*N*-(3-(4-((2-(2,6-dioxopiperidin-3-yl)-1,3-dioxoisoindolin-4-yl)glycyl)piperazin-1-yl)propyl)-5-((5-fluoro-2-oxoindolin-3-ylidene)methyl)-2,4-dimethyl-1*H-*pyrrole-3-carboxamide |
| 19 | HC58-36 | 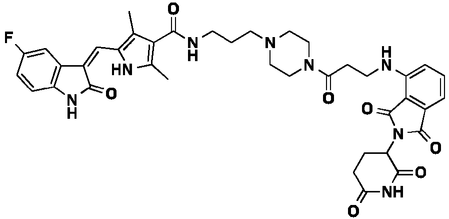 | (*Z*)-*N*-(3-(4-(3-((2-(2,6-dioxopiperidin-3-yl)-1,3-dioxoisoindolin-4-yl)amino)propanoyl)piperazin-1-yl)propyl)-5-((5-fluoro-2-oxoindolin-3-ylidene)methyl)-2,4-dimethyl-1*H*-pyrrole-3-carboxamide |
| 20 | HC58-37 | 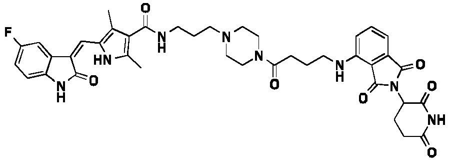 | (*Z*)-*N*-(3-(4-(4-((2-(2,6-dioxopiperidin-3-yl)-1,3-dioxoisoindolin-4-yl)amino)butanoyl)piperazin-1-yl)propyl)-5-((5-fluoro-2-oxoindolin-3-ylidene)methyl)-2,4-dimethyl-1*H*-pyrrole-3-carboxamide |
| 21 | HC58-38 | 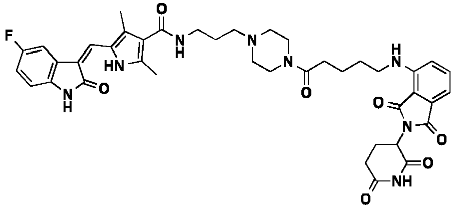 | (*Z*)-*N*-(3-(4-(5-((2-(2,6-dioxopiperidin-3-yl)-1,3-dioxoisoindolin-4-yl)amino)pentanoyl)piperazin-1-yl)propyl)-5-((5-fluoro-2-oxoindolin-3-ylidene)methyl)-2,4-dimethyl-1*H*-pyrrole-3-carboxamide |
| 22 | HC58-39 | 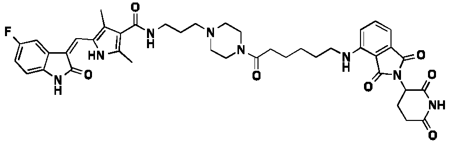 | (*Z*)-*N*-(3-(4-(6-((2-(2,6-dioxopiperidin-3-yl)-1,3-dioxoisoindolin-4-yl)amino)hexanoyl)piperazin-1-yl)propyl)-5-((5-fluoro-2-oxoindolin-3-ylidene)methyl)-2,4-dimethyl-1*H*-pyrrole-3-carboxamide |
| 23 | HC58-40 | 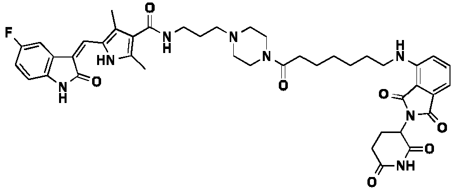 | (*Z*)-*N*-(3-(4-(7-((2-(2,6-dioxopiperidin-3-yl)-1,3-dioxoisoindolin-4-yl)amino)heptanoyl) piperazin-1-yl)propyl)-5-((5-fluoro-2-oxoindolin-3-ylidene)methyl)-2,4-dimethyl-1*H*-pyrrole-3-carboxamide |
| 24 | HC58-41 | 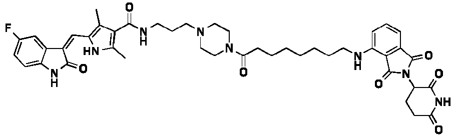 | (*Z*)-*N*-(3-(4-(8-((2-(2,6-dioxopiperidin-3-yl)-1,3-dioxoisoindolin-4-yl)amino)octanoyl)piperazin-1-yl)propyl)-5-((5-fluoro-2-oxoindolin-3-ylidene)methyl)-2,4-dimethyl-1*H*-pyrrole-3-carboxamide |
| 25 | HC58-43 | 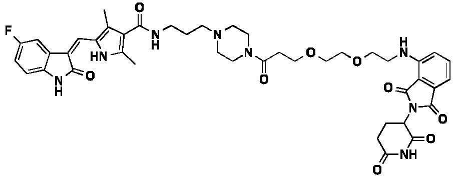 | (*Z*)-*N*-(3-(4-(3-(2-(2-((2-(2,6-dioxopiperidin-3-yl)-1,3-dioxoisoindolin-4-yl)amino)ethoxy)ethoxy)propanoyl) piperazin-1-yl)propyl)-5-((5-fluoro-2-oxoindolin-3-ylidene)methyl)-2,4-dimethyl-1*H*-pyrrole-3-carboxamide |
| 26 | HC58-44 | 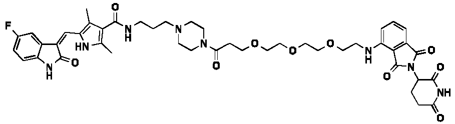 | (*Z*)-*N*-(3-(4-(3-(2-(2-(2-((2-(2,6-dioxopiperidin-3-yl)-1,3-dioxoisoindolin-4-yl)amino)ethoxy)ethoxy)ethoxy)pro panoyl)piperazin-1-yl)propyl)-5-((5-fluoro-2-oxoindolin-3-ylidene)methyl)-2,4-dimethyl-1*H-*pyrrole-3-carboxamide |
| 27 | HC58-45 | 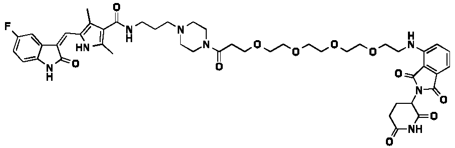 | (Z)-*N*-(3-(4-(1-((2-(2,6-dioxopiperidin-3-yl)-1,3-dioxoisoindolin-4-yl)amino)-3,6,9,12-tetraoxapentadecan-15-oyl)piperazin-1-yl)propyl)-5-((5-fluoro-2-oxo indolin-3-ylidene)methyl)-2,4-dimethyl-1*H-*pyrrole-3-carboxamide |
| 28 | HC58-46 | 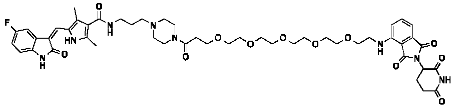 | (Z)-*N*-(3-(4-(1-((2-(2,6-dioxopiperidin-3-yl)-1,3-dioxoisoindolin-4-yl)amino)-3,6,9,12,15-pentaoxaoctadecan-18-oyl)piperazin-1-yl)propyl)-5-((5-fluoro-2-oxoindolin-3-ylidene)methyl)-2,4-dimethyl-1*H-*pyrrole-3-carboxamide |
| 30 | HC58-53 | 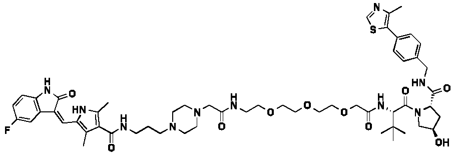 | 5-(((*Z*)-5-fluoro-2-oxoindolin-3-ylidene)methyl)-*N*-(3-(4-((*S*)-16-((2*S*,4*R*)-4-hydroxy-2-((4-(4-methylthiazol-5-yl)benzyl)carbamoyl)pyrrolidine-1-carbonyl)-17,17-dimethyl-2,14-dioxo-6,9,12-trioxa-3,15-diazaoctadecyl)piperazin-1-yl)propyl)-2,4-dimethyl-1*H*-pyrrole-3-carboxamide |
| 31 | HC58-57 | 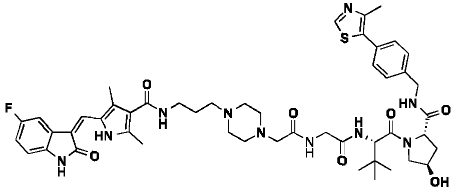 | 5-(((*Z*)-5-fluoro-2-oxoindolin-3-yl ide n e) methyl)-N-(3-(4-(2-((2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethyl)amino)-2-oxoethyl)piperazin-1-yl)propyl)-2,4-dimethyl-1*H*-pyrrole-3-carboxamide |
| 32 | HC58-58 | 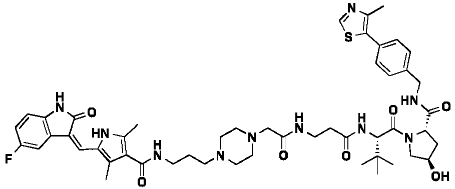 | 5-(((*Z*)-5-fluoro-2-oxoindolin-3-yl ide n e) methyl)-*N*-(3-(4-(2-((3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)amino)-2-oxoethyl)piperazin-1-yl)propyl)-2,4-dimethyl-1*H*-pyrrole-3-carboxamide |
| 33 | HC58-59 | 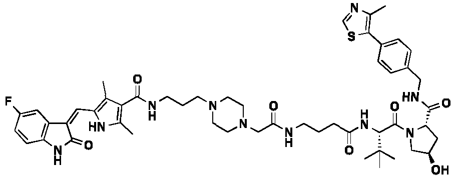 | 5-(((*Z*)-5-fluoro-2-oxoindolin-3-ylidene)methyl)-*N*-(3-(4-(2-((4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutyl)amino)-2-oxoethyl)piperazin-1-yl)propyl)-2,4-dimethyl-1*H*-pyrrole-3-carboxamide |
| 34 | HC58-60 | 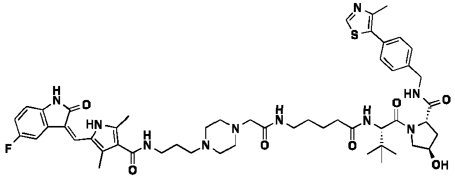 | 5-(((*Z*)-5-fluoro-2-oxoindolin-3-ylidene)methyl)-*N*-(3-(4-(2-((5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentyl)amino)-2-oxoethyl)piperazin-1-yl)propyl)-2,4-dimethyl-1*H*-pyrrole-3-carboxamide |
| 35 | HC58-63 | 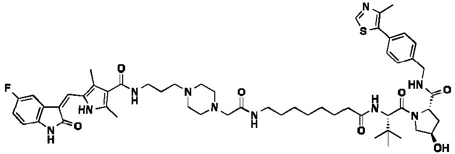 | 5-(((*Z*)-5-fluoro-2-oxoindolin-3-ylidene)methyl)-*N*-(3-(4-(2-((8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctyl)amino)-2-oxoethyl)piperazin-1-yl)propyl)-2,4-dimethyl-1*H*-pyrrole-3-carboxamide |
| 36 | HC58-64 | 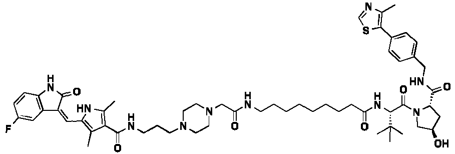 | 5-(((*Z*)-5-fluoro-2-oxoindolin-3-ylidene)methyl)-*N*-(3-(4-(2-((9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononyl)amino)-2-oxoethyl)piperazin-1-yl)propyl)-2,4-dimethyl-1*H*-pyrrole-3-carboxamide |
| 37 | HC58-65 | 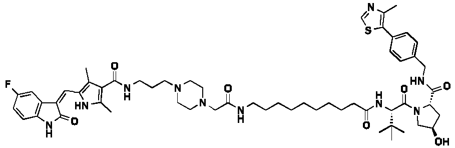 | 5-(((*Z*)-5-fluoro-2-oxoindolin-3-ylidene)methyl)-*N*-(3-(4-(2-((10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecyl)amino)-2-oxoethyl)piperazin-1-yl)propyl)-2,4-dimethyl-1*H*-pyrrole-3-carboxamide |
| 38 | HC58-66 | 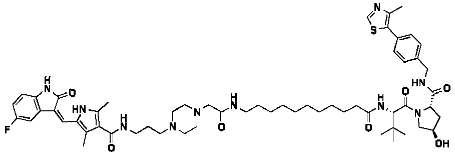 | 5-(((*Z*)-5-fluoro-2-oxoindolin-3-ylidene)methyl)-*N*-(3-(4-(2-((11-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecyl)amino)-2-oxoethyl)piperazin-1-yl)propyl)-2,4-dimethyl-1*H*-pyrrole-3-carboxamide |
| 39 | HC58-67 | 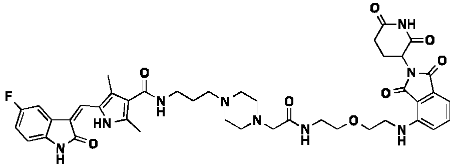 | (Z)-*N*-(3-(4-(2-((2-(2-((2-(2,6-dioxopiperidin-3-yl)-1,3-dioxoisoindolin-4-yl)amino)ethoxy)ethyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 40 | HC58-68 | 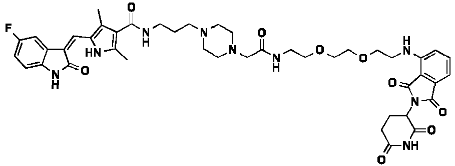 | (*Z*)-*N*-(3-(4-(2-((2-(2-(2-((2-(2,6-dioxopiperidin-3-yl)-1,3-dioxoisoindolin-4-yl)amino)ethoxy)ethoxy)ethyl)amin o)-2-oxoethyl)piperazin-1-yl)propyl)-5-((5-fluoro-2-oxoindolin-3-ylidene)methyl)-2,4-dimethyl-1*H*-pyrrole-3-carboxamide |
| 41 | HC58-69 | 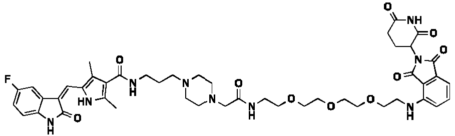 | (Z)-*N*-(3-(4-(14-((2-(2,6-dioxopiperidin-3-yl)-1,3-dioxoisoindolin-4-yl)amino)-2-oxo-6,9,12-trioxa-3-azatetradecyl)piperazin-1-yl)propyl)-5-((5-fluoro-2-oxoindolin-3-ylidene)methyl)-2,4-dimethyl-1*H*-pyrrole-3-carboxamide |
| 42 | HC58-70 | 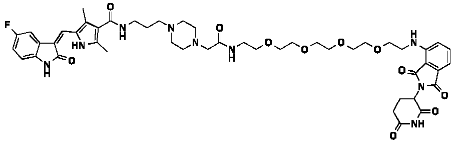 | (*Z*)-*N*-(3-(4-(17-((2-(2,6-dioxopiperidin-3-yl)-1,3-dioxoisoindolin-4-yl)amino)-2-oxo-6,9,12,15-tetraoxa-3-azaheptadecyl)piperazin-1-yl)propyl)-5-((5-fluoro-2-oxoindolin-3-ylidene)methyl)-2,4-dimethyl-1*H*-pyrrole-3-carboxamide |
| 43 | HC58-71 | 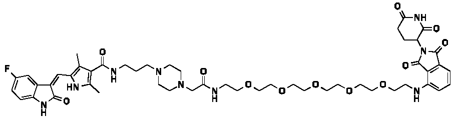 | (Z)-*N*-(3-(4-(20-((2-(2,6-dioxopiperidin-3-yl)-1,3-dioxoisoindolin-4-yl)amino)-2-oxo-6,9,12,15,18-pentaoxa-3-azaicosyl)piperazin-1-yl)propyl)-5-((5-fluoro-2-oxoindolin-3-ylidene)methyl)-2,4-dimethyl-1*H-*pyrrole-3-carboxamide |
| 44 | HC58-73 | 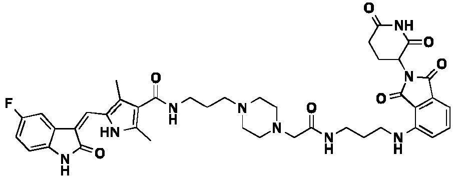 | (Z)-*N*-(3-(4-(2-((3-((2-(2,6-dioxopiperidin-3-yl)-1,3-dioxoisoindolin-4-yl)amino)propyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 45 | HC58-74 | 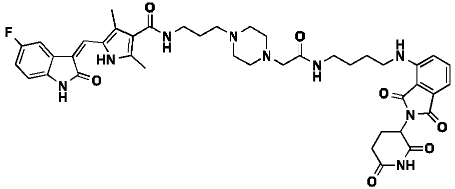 | (*Z*)-*N*-(3-(4-(2-((4-((2-(2,6-dioxopiperidin-3-yl)-1,3-dioxoisoindolin-4-yl)amino)butyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 46 | HC58-75 | 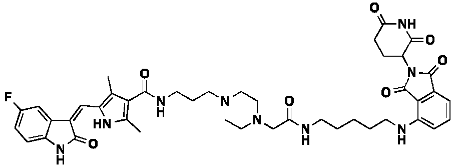 | (*Z*)-*N*-(3-(4-(2-((5-((2-(2,6-dioxopiperidin-3-yl)-1,3-dioxoisoindolin-4-yl)amino)pentyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 47 | HC58-76 | 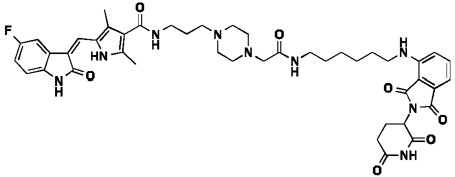 | (*Z*)-*N*-(3-(4-(2-((6-((2-(2,6-dioxopiperidin-3-yl)-1,3-dioxoisoindolin-4-yl)amino)hexyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 48 | HC58-77 | 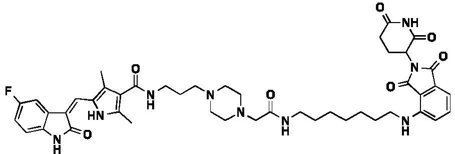 | (*Z*)-*N*-(3-(4-(2-((7-((2-(2,6-dioxopiperidin-3-yl)-1,3-dioxoisoindolin-4-yl)amino)heptyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 49 | HC58-78 | 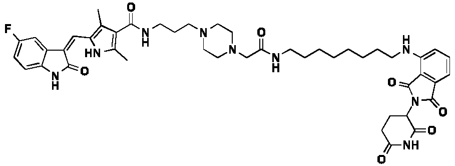 | (*Z*)-*N*-(3-(4-(2-((8-((2-(2,6-dioxopiperidin-3-yl)-1,3-dioxoisoindolin-4-yl)amino)octyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 51 | HC58-133 | 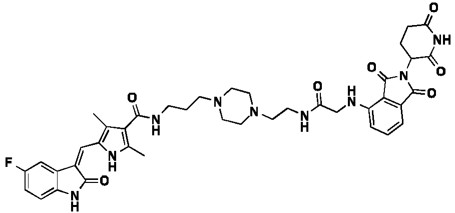 | (*Z*)-*N*-(3-(4-(2-(2-((2-(2,6-dioxopiperidin-3-yl)-1,3-dioxoisoindolin-4-yl)amino)acetamido)ethyl)piperazi n-1 -yl)propyl)-5-((5-fluoro-2-oxoindolin-3-ylidene)methyl)-2,4-dimethyl-1*H*-pyrrole-3-carboxamide |
| 52 | HC58-134 | 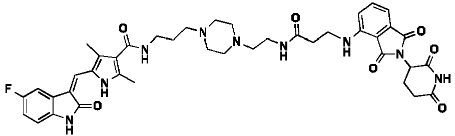 | (*Z*)-*N*-(3-(4-(2-(3-((2-(2,6-dioxopiperidin-3-yl)-1,3-dioxoisoindolin-4-yl)amino)propanamido)ethyl)piper azin-1-yl)propyl)-5-((5-fluoro-2-oxoindolin-3-ylidene)methyl)-2,4-dimethyl-1*H*-pyrrole-3-carboxamide |
| 53 | HC58-135 | 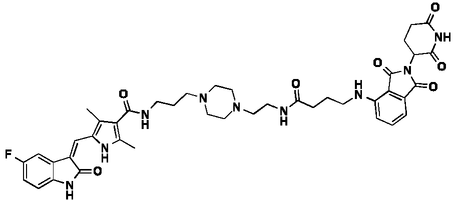 | (*Z*)-*N*-(3-(4-(2-(4-((2-(2,6-dioxopiperidin-3-yl)-1,3-dioxoisoindolin-4-yl)amino)butanamido)ethyl)piperaz in-1-yl)propyl)-5-((5-fluoro-2-oxoindolin-3-ylidene)methyl)-2,4-dimethyl-1*H*-pyrrole-3-carboxamide |
| 54 | HC58-136 | 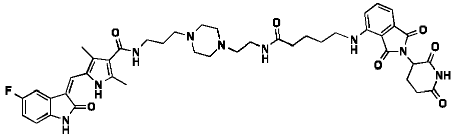 | (*Z*)-*N*-(3-(4-(2-(5-((2-(2,6-dioxopiperidin-3-yl)-1,3-dioxoisoindolin-4-yl)amino)pentanamido)ethyl)piper azin-1-yl)propyl)-5-((5-fluoro-2-oxoindolin-3-ylidene)methyl)-2,4-dimethyl-1*H*-pyrrole-3-carboxamide |
| 55 | HC58-137 | 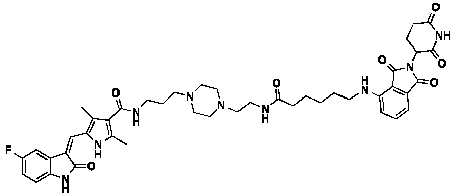 | (*Z*)-*N*-(3-(4-(2-(6-((2-(2,6-dioxopiperidin-3-yl)-1,3-dioxoisoindolin-4-yl)amino)hexanamido)ethyl)pipera zin-1-yl)propyl)-5-((5-fluoro-2-oxoindolin-3-ylidene)methyl)-2,4-dimethyl-1*H*-pyrrole-3-carboxamide |
| 56 | HC58-138 | 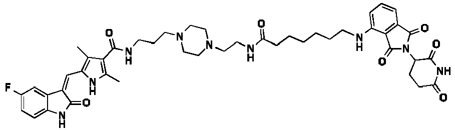 | (*Z*)-*N*-(3-(4-(2-(7-((2-(2,6-dioxopiperidin-3-yl)-1,3-dioxoisoindolin-4-yl)amino)heptanamido)ethyl)piper azin-1-yl)propyl)-5-((5-fluoro-2-oxoindolin-3-ylidene)methyl)-2,4-dimethyl-1*H*-pyrrole-3-carboxamide |
| 57 | HC58-139 | 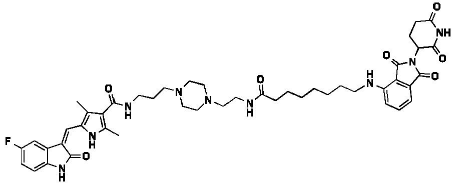 | (*Z*)-*N*-(3-(4-(2-(8-((2-(2,6-dioxopiperidin-3-yl)-1,3-dioxoisoindolin-4-yl)amino)octanamido)ethyl)piperaz in-1-yl)propyl)-5-((5-fluoro-2-oxoindolin-3-ylidene)methyl)-2,4-dimethyl-1*H*-pyrrole-3-carboxamide |
| 59 | HC58-144 | 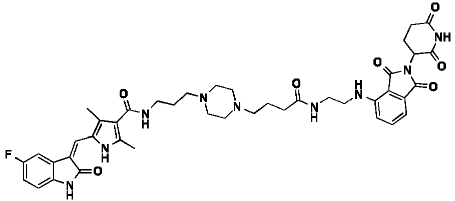 | (*Z*)-*N*-(3-(4-(4-((2-((2-(2,6-dioxopiperidin-3-yl)-1,3-dioxoisoindolin-4-yl)amino)ethyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 60 | HC58-145 | 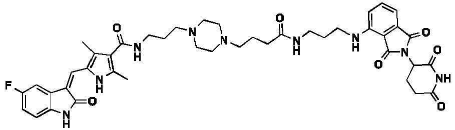 | (*Z*)-*N*-(3-(4-(4-((3-((2-(2,6-dioxopiperidin-3-yl)-1,3-dioxoisoindolin-4-yl)amino)propyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 61 | HC58-146 | 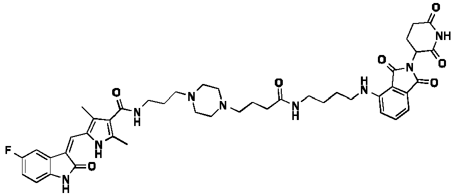 | (*Z*)-*N*-(3-(4-(4-((4-((2-(2,6-dioxopiperidin-3-yl)-1,3-dioxoisoindolin-4-yl)amino)butyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 62 | HC58-147 | 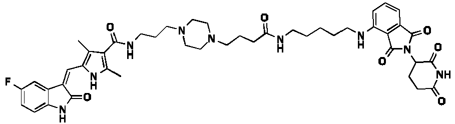 | (*Z*)-*N*-(3-(4-(4-((5-((2-(2,6-dioxopiperidin-3-yl)-1,3-dioxoisoindolin-4-yl)amino)pentyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 63 | HC58-148 | 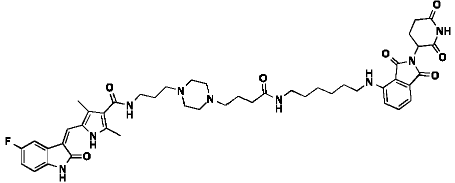 | (*Z*)-*N*-(3-(4-(4-((6-((2-(2,6-dioxopiperidin-3-yl)-1,3-dioxoisoindolin-4-yl)amino)hexyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 64 | HC58-149 | 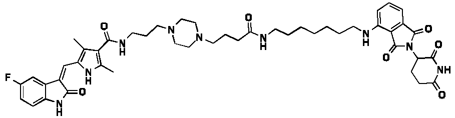 | (*Z*)-*N*-(3-(4-(4-((7-((2-(2,6-dioxopiperidin-3-yl)-1,3-dioxoisoindolin-4-yl)amino)heptyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 65 | HC58-150 | 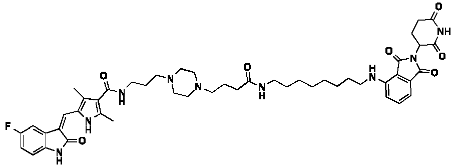 | (*Z*)-*N-*(3-(4-(4-((8-((2-(2,6-dioxopiperidin-3-yl)-1,3-dioxoisoindolin-4-yl)amino)octyl)amino)-4-oxobutyl)piperazin-1-yl)propyl)-5-((5-fluoro-2-oxoindolin-3-ylidene)methyl)-2,4-dimethyl-1*H-*pyrrole-3-carboxamide |
| 66 | HC58-158 | 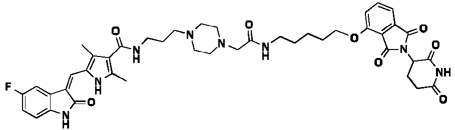 | (*Z*)-*N*-(3-(4-(2-((5-((2-(2,6-dioxopiperidin-3-yl)-1,3-dioxoisoindolin-4-yl)oxy)pentyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 67 | HC58-159 | 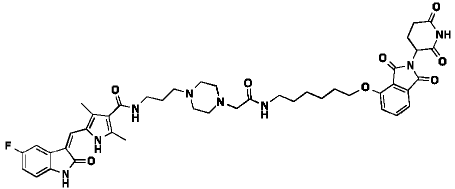 | (*Z*)-*N*-(3-(4-(2-((6-((2-(2,6-dioxopiperidin-3-yl)-1,3-dioxoisoindolin-4-yl)oxy)hexyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 68 | HC58-160 | 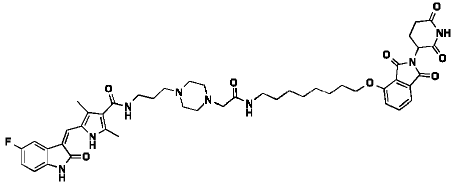 | (*Z*)-*N*-(3-(4-(2-((8-((2-(2,6-dioxopiperidin-3-yl)-1,3-dioxoisoindolin-4-yl)oxy)octyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 69 | HC58-161 | 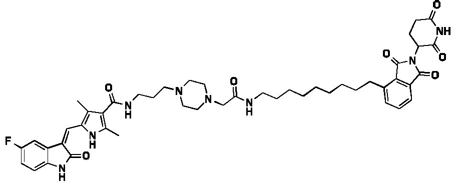 | (*Z*)-*N*-(3-(4-(2-((9-(2-(2,6-dioxopiperidin-3-yl)-1,3-dioxoisoindolin-4-yl)nonyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 70 | HC58-164 | 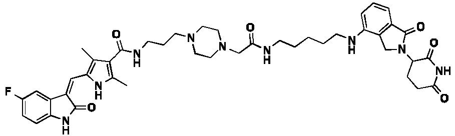 | (*Z*)-*N*-(3-(4-(2-((5-((2-(2,6-dioxopiperidin-3-yl)-1-oxoisoindolin-4-yl)amino)pentyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 71 | HC58-165 | 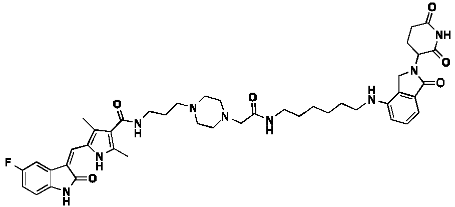 | (*Z*)-*N*-(3-(4-(2-((6-((2-(2,6-dioxopiperidin-3-yl)-1-oxoisoindolin-4-yl)amino)hexyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 72 | HC58-167 | 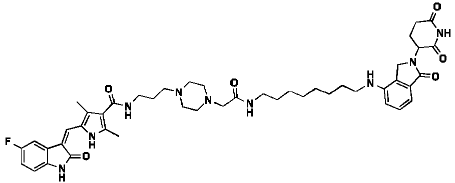 | (*Z*)-*N*-(3-(4-(2-((8-((2-(2,6-dioxopiperidin-3-yl)-1-oxoisoindolin-4-yl)amino)octyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 73 | HC58-178 | 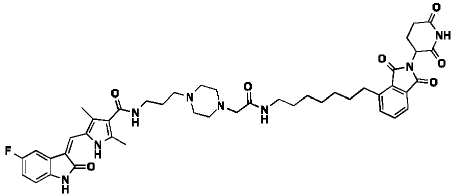 | (*Z*)-*N*-(3-(4-(2-((7-(2-(2,6-dioxopiperidin-3-yl)-1,3-dioxoisoindolin-4-yl)heptyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 75 | HC58-179 | 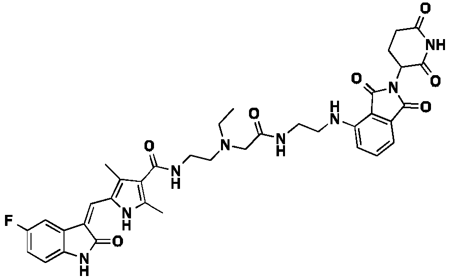 | (*Z*)-*N*-(2-((2-((2-((2-(2,6-dioxopiperidin-3-yl)-1,3-dioxoisoindolin-4-yl)amino)ethyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 76 | HC58-180 | 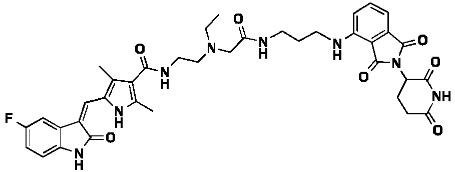 | (*Z*)-*N*-(2-((2-((3-((2-(2,6-dioxopiperidin-3-yl)-1,3-dioxoisoindolin-4-yl)amino)propyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 77 | HC58-181 | 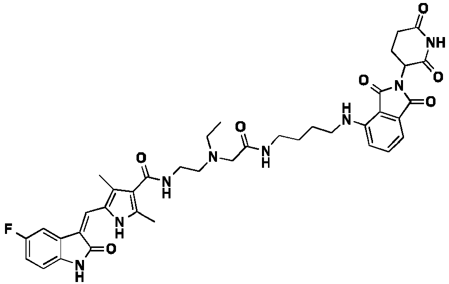 | (*Z*)-*N*-(2-((2-((4-((2-(2,6-dioxopiperidin-3-yl)-1,3-dioxoisoindolin-4-yl)amino)butyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 78 | HC58-182 | 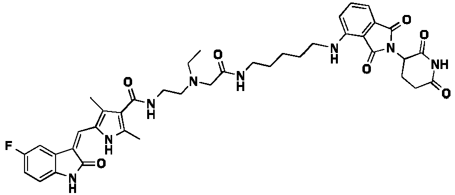 | (*Z*)-*N*-(2-((2-((5-((2-(2,6-dioxopiperidin-3-yl)-1,3-dioxoisoindolin-4-yl)amino)pentyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 79 | HC58-183 | 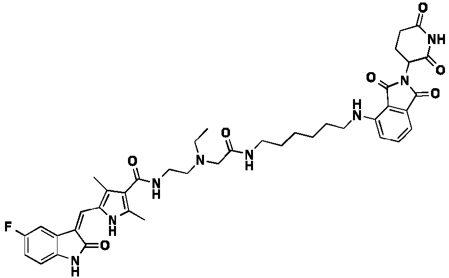 | (*Z*)-*N*-(2-((2-((6-((2-(2,6-dioxopiperidin-3-yl)-1,3-dioxoisoindolin-4-yl)amino)hexyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 80 | HC58-184 | 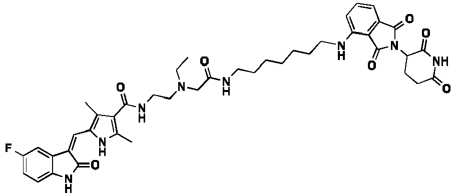 | (*Z*)-*N*-(2-((2-((7-((2-(2,6-dioxopiperidin-3-yl)-1,3-dioxoisoindolin-4-yl)amino)heptyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 81 | HC58-185 | 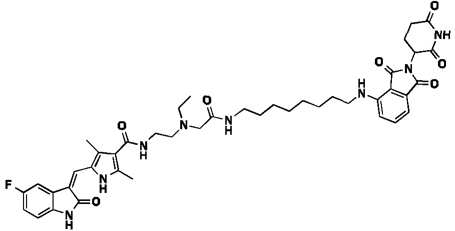 | (*Z*)-*N*-(2-((2-((8-((2-(2,6-dioxopiperidin-3-yl)-1,3-dioxoisoindolin-4-yl)amino)octyl)amino)-2-oxoethyl)(ethyl)amino)ethyl)-5-((5-fluoro-2-oxo indolin-3-ylidene)methyl)-2,4-dimethyl-1*H-*pyrrole-3-carboxamide |
| 83 | HC65-2 | 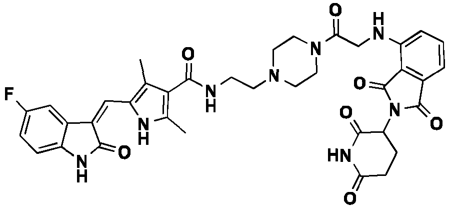 | (*Z*)-*N*-(2-(4-((2-(2,6-dioxopiperidin-3-yl)-1,3-dioxoisoindolin-4-yl)glycyl)piperazin-1-yl)ethyl)-5-((5-fluoro-2-oxoindolin-3-ylidene)methyl)-2,4-dimethyl-1*H-*pyrrole-3-carboxamide |
| 84 | HC65-3 | 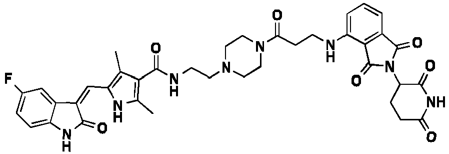 | (*Z*)-*N*-(2-(4-(3-((2-(2,6-dioxopiperidin-3-yl)-1,3-dioxoisoindolin-4-yl)amino)propanoyl)piperazin-1-yl)ethyl)-5-((5-fluoro-2-oxoindolin-3-ylidene)methyl)-2,4-dimethyl-1*H-*pyrrole-3-carboxamide |
| 85 | HC65-4 | 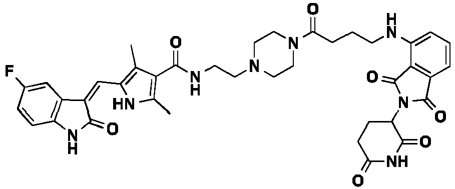 | (*Z*)-*N*-(2-(4-(4-((2-(2,6-dioxopiperidin-3-yl)-1,3-dioxoisoindolin-4-yl)amino)butanoyl)piperazin-1-yl)ethyl)-5-((5-fluoro-2-oxoindolin-3-ylidene)methyl)-2,4-dimethyl-1*H-*pyrrole-3-carboxamide |
| 86 | HC65-5 | 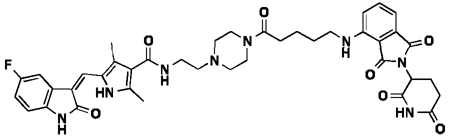 | (*Z*)-*N*-(2-(4-(5-((2-(2,6-dioxopiperidin-3-yl)-1,3-dioxoisoindolin-4-yl)amino)pentanoyl)piperazin-1-yl)ethyl)-5-((5-fluoro-2-oxoindolin-3-ylidene)methyl)-2,4-dimethyl-1*H-*pyrrole-3-carboxamide |
| 87 | HC65-6 | 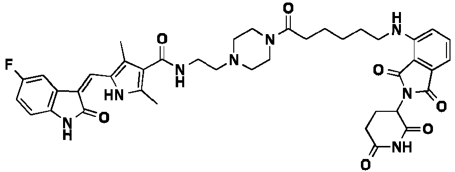 | (Z)-/V-(2-(4-(6-((2-(2,6-dioxopiperidin-3-yl)-1,3-dioxoisoindolin-4-yl)amino)hexanoyl)piperazin-1-yl)ethyl)-5-((5-fluoro-2-oxoindolin-3-ylidene)methyl)-2,4-dimethyl-1H-pyrrole-3-carboxamide |
| 88 | HC65-7 | 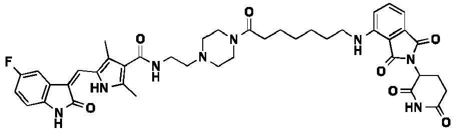 | (*Z*)-*N*-(2-(4-(7-((2-(2,6-dioxopiperidin-3-yl)-1,3-dioxoisoindolin-4-yl)amino)heptanoyl) piperazin-1-yl)ethyl)-5-((5-fluoro-2-oxoindolin-3-ylidene)methyl)-2,4-dimethyl-1*H-*pyrrole-3-carboxamide |
| 89 | HC65-8 | 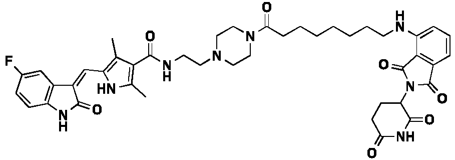 | (*Z*)-*N*-(2-(4-(8-((2-(2,6-dioxopiperidin-3-yl)-1,3-dioxoisoindolin-4-yl)amino)octanoyl)piperazin-1-yl)ethyl)-5-((5-fluoro-2-oxoindolin-3-ylidene)methyl)-2,4-dimethyl-1*H-*pyrrole-3-carboxamide |
| 91 | HC65-13 | 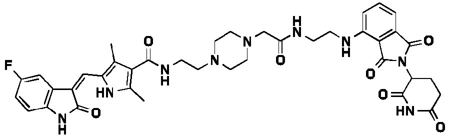 | (*Z*)-*N*-(2-(4-(2-((2-((2-(2,6-dioxopiperidin-3-yl)-1,3-dioxoisoindolin-4-yl)amino)ethyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 92 | HC65-14 | 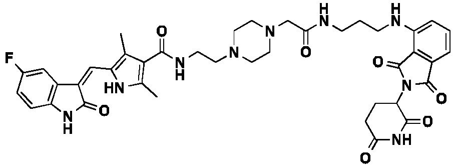 | (*Z*)-*N*-(2-(4-(2-((3-((2-(2,6-dioxopiperidin-3-yl)-1,3-dioxoisoindolin-4-yl)amino)propyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 93 | HC65-15 | 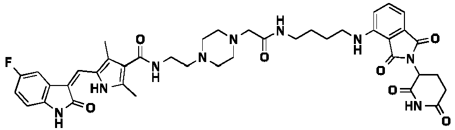 | (*Z*)-*N*-(2-(4-(2-((4-((2-(2,6-dioxopiperidin-3-yl)-1,3-dioxoisoindolin-4-yl)amino)butyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 94 | HC65-16 | 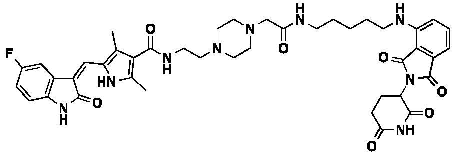 | (*Z*)-*N*-(2-(4-(2-((5-((2-(2,6-dioxopiperidin-3-yl)-1,3-dioxoisoindolin-4-yl)amino)pentyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 95 | HC65-17 | 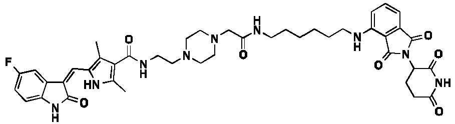 | (*Z*)-*N*-(2-(4-(2-((6-((2-(2,6-dioxopiperidin-3-yl)-1,3-dioxoisoindolin-4-yl)amino)hexyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 96 | HC65-18 | 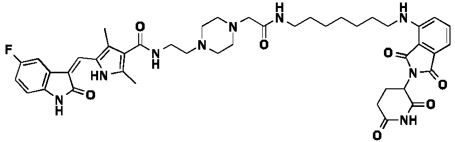 | (*Z*)-*N*-(2-(4-(2-((7-((2-(2,6-dioxopiperidin-3-yl)-1,3-dioxoisoindolin-4-yl)amino)heptyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 97 | HC65-19 | 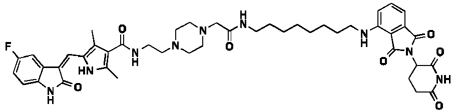 | (*Z*)-*N*-(2-(4-(2-((8-((2-(2,6-dioxopiperidin-3-yl)-1,3-dioxoisoindolin-4-yl)amino)octyl)amino)-2-oxoethyl)piperazin-1-yl)ethyl)-5-((5-fluoro-2-oxoindolin-3-ylidene)methyl)-2,4-dimethyl-1*H-*pyrrole-3-carboxamide |
| 99 | HC65-24 | 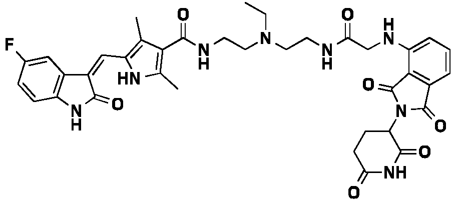 | (*Z*)-*N*-(2-((2-(2-((2-(2,6-dioxopiperidin-3-yl)-1,3-dioxoisoindolin-4-yl)amino)acetamido)ethyl)(ethyl)a mino)ethyl)-5-((5-fluoro-2-oxoindolin-3-ylidene)methyl)-2,4-dimethyl-1*H*-pyrrole-3-carboxamide |
| 100 | HC65-25 | 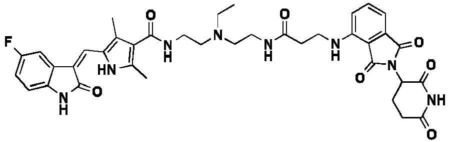 | (*Z*)-*N*-(2-((2-(3-((2-(2,6-dioxopiperidin-3-yl)-1,3-dioxoisoindolin-4-yl)amino)propanamido)ethyl)(ethyl )amino)ethyl)-5-((5-fluoro-2-oxoindolin-3-ylidene)methyl)-2,4-dimethyl-1*H*-pyrrole-3-carboxamide |
| 101 | HC65-26 | 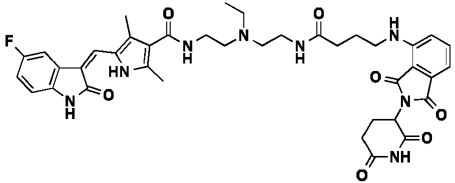 | (*Z*)-*N*-(2-((2-(4-((2-(2,6-dioxopiperidin-3-yl)-1,3-dioxoisoindolin-4-yl)amino)butanamido)ethyl)(ethyl) amino)ethyl)-5-((5-fluoro-2-oxoindolin-3-ylidene)methyl)-2,4-dimethyl-1*H*-pyrrole-3-carboxamide |
| 102 | HC65-27 | 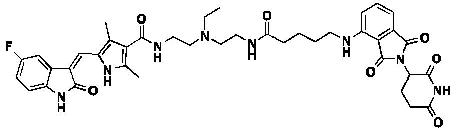 | (*Z*)-*N*-(2-((2-(5-((2-(2,6-dioxopiperidin-3-yl)-1,3-dioxoisoindolin-4-yl)amino)pentanamido)ethyl)(ethyl )amino)ethyl)-5-((5-fluoro-2-oxoindolin-3-ylidene)methyl)-2,4-dimethyl-1*H*-pyrrole-3-carboxamide |
| 103 | HC65-28 | 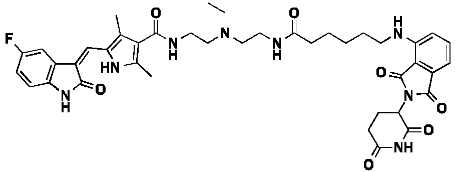 | (*Z*)-*N*-(2-((2-(6-((2-(2,6-dioxopiperidin-3-yl)-1,3-dioxoisoindolin-4-yl)amino)hexanamido)ethyl)(ethyl) amino)ethyl)-5-((5-fluoro-2-oxoindolin-3-ylidene)methyl)-2,4-dimethyl-1*H*-pyrrole-3-carboxamide |
| 104 | HC65-29 | 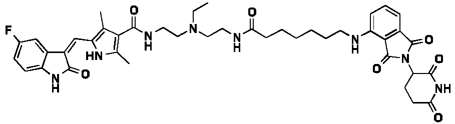 | (*Z*)-*N*-(2-((2-(7-((2-(2,6-dioxopiperidin-3-yl)-1,3-dioxoisoindolin-4-yl)amino)heptanamido)ethyl)(ethyl )amino)ethyl)-5-((5-fluoro-2-oxoindolin-3-ylidene)methyl)-2,4-dimethyl-1*H*-pyrrole-3-carboxamide |
| 105 | HC65-30 | 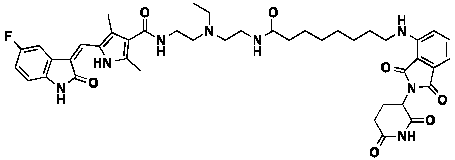 | (*Z*)-*N*-(2-((2-(8-((2-(2,6-dioxopiperidin-3-yl)-1,3-dioxoisoindolin-4-yl)amino)octanamido)ethyl)(ethyl)a mino)ethyl)-5-((5-fluoro-2-oxoindolin-3-ylidene)methyl)-2,4-dimethyl-1*H*-pyrrole-3-carboxamide |
| 106 | HC65-33 | 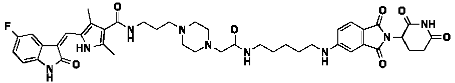 | (*Z*)-*N*-(3-(4-(2-((5-((2-(2,6-dioxopiperidin-3-yl)-1,3-dioxoisoindolin-5-yl)amino)pentyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 107 | HC65-34 | 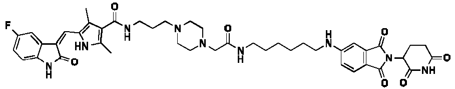 | (*Z*)-*N*-(3-(4-(2-((6-((2-(2,6-dioxopiperidin-3-yl)-1,3-dioxoisoindolin-5-yl)amino)hexyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 108 | HC65-35 | 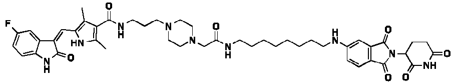 | (*Z*)-*N*-(3-(4-(2-((8-((2-(2,6-dioxopiperidin-3-yl)-1,3-dioxoisoindolin-5-yl)amino)octyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 109 | HC65-37 | 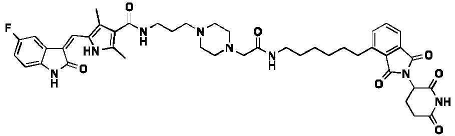 | (*Z*)-*N*-(3-(4-(2-((6-(2-(2,6-dioxopiperidin-3-yl)-1,3-dioxoisoindolin-4-yl)hexyl)amino)-2-oxoethyl)piperazin-1-yl)propyl)-5-((5-fluoro-2-oxoindolin-3-ylidene)methyl)-2,4-dimethyl-1*H-*pyrrole-3-carboxamide |
| 110 | HC65-74 | 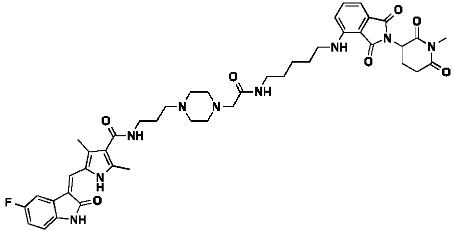 | (*Z*)-5-((5-fluoro-2-oxoindolin-3-ylidene)methyl)-2,4-dimethyl-*N*-(3-(4-(2-((5-((2-(1-methyl-2,6-dioxopiperidin-3-yl)-1,3-dioxoisoindolin-4-yl)amino)pentyl)amino)-2-oxoethyl)piperazin-1-yl)propyl)-1*H-*pyrrole-3-carboxamide |
| 111 | HC65-75 | 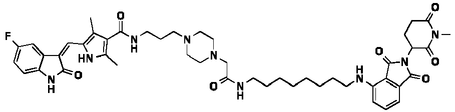 | (*Z*)-5-((5-fluoro-2-oxoindolin-3-ylidene)methyl)-2,4-dimethyl-*N*-(3-(4-(2-((8-((2-(1-methyl-2,6-dioxopiperidin-3-yl)-1,3-dioxoisoindolin-4-yl)amino)octyl)amino)-2-oxoethyl)piperazin-1-yl)propyl)-1*H-*pyrrole-3-carboxamide |
| 115 | HC65-175 | 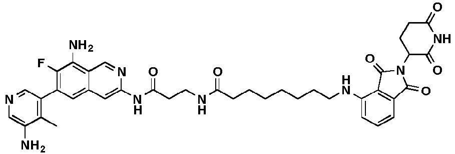 | *N*-(3-((8-amino-6-(5-amino-4-methylpyridin-3-yl)-7-fluoroisoquinolin-3-yl)amino)-3-oxopropyl)-8-((2-(2,6-dioxopiperidin-3-yl)-1,3-dioxoisoindolin-4-yl)amino)octanamide |
| 116 | HC65-183 | 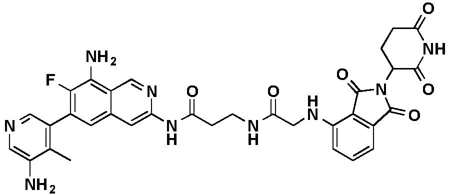 | *N*-(8-amino-6-(5-amino-4-methylpyridin-3-yl)-7-fluoroisoquinolin-3-yl)-3-(2-((2-(2,6-dioxopiperidin-3-yl)-1,3-dioxoisoindolin-4-yl)amino)acetamido)propanamide |
| 117 | HC65-184 | 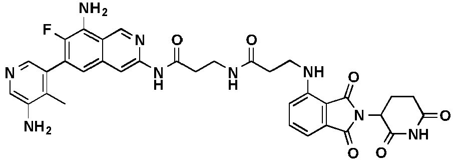 | *N*-(8-amino-6-(5-amino-4-methylpyridin-3-yl)-7-fluoroisoquinolin-3-yl)-3-(3-((2-(2,6-dioxopiperidin-3-yl)-1,3-dioxoisoindolin-4-yl)amino)propanamido)propanami de |
| 118 | HC65-185 | 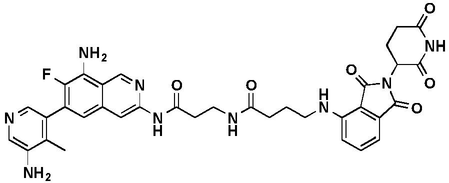 | *N*-(3-((8-amino-6-(5-amino-4-methylpyridin-3-yl)-7-fluoroisoquinolin-3-yl)amino)-3-oxopropyl)-4-((2-(2,6-dioxopiperidin-3-yl)-1,3-dioxoisoindolin-4-yl)amino)butanamide |
| 119 | HC65-186 | 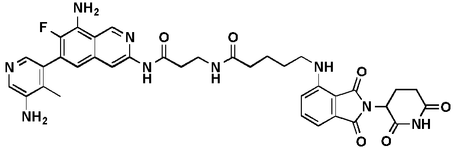 | *N*-(3-((8-amino-6-(5-amino-4-methylpyridin-3-yl)-7-fluoroisoquinolin-3-yl)amino)-3-oxopropyl)-5-((2-(2,6-dioxopiperidin-3-yl)-1,3-dioxoisoindolin-4-yl)amino)pentanamide |
| 120 | HC75-1 | 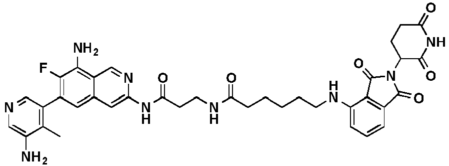 | *N*-(3-((8-amino-6-(5-amino-4-methylpyridin-3-yl)-7-fluoroisoquinolin-3-yl)amino)-3-oxopropyl)-6-((2-(2,6-dioxopiperidin-3-yl)-1,3-dioxoisoindolin-4-yl)amino)hexanamide |
| 121 | HC75-2 | 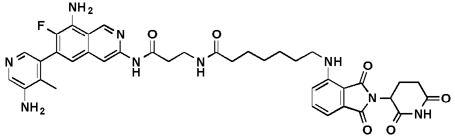 | *N*-(3-((8-amino-6-(5-amino-4-methylpyridin-3-yl)-7-fluoroisoquinolin-3-yl)amino)-3-oxopropyl)-7-((2-(2,6-dioxopiperidin-3-yl)-1,3-dioxoisoindolin-4-yl)amino)heptanamide |
| 122 | HC75-3 | 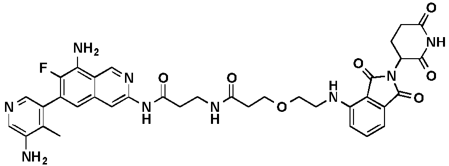 | *N*-(8-amino-6-(5-amino-4-methylpyridin-3-yl)-7-fluoroisoquinolin-3-yl)-3-(3-(2-((2-(2,6-dioxopiperidin-3-yl)-1,3-dioxoisoindolin-4-yl)amino)ethoxy)propanamido)pro panamide |
| 123 | HC75-4 | 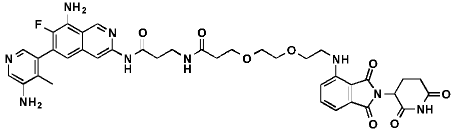 | *N*-(8-amino-6-(5-amino-4-methylpyridin-3-yl)-7-fluoroisoquinolin-3-yl)-3-(3-(2-(2-((2-(2,6-dioxopiperidin-3-yl)-1,3-dioxoisoindolin-4-yl)amino)ethoxy)ethoxy)propanami do)propanamide |
| 124 | HC75-5 | 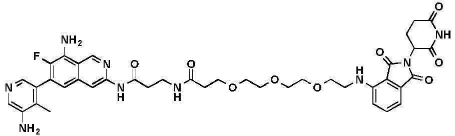 | *N*-(8-amino-6-(5-amino-4-methylpyridin-3-yl)-7-fluoroisoquinolin-3-yl)-3-(3-(2-(2-(2-((2-(2,6-dioxopiperidin-3-yl)-1,3-dioxoisoindolin-4-yl)amino)ethoxy)ethoxy)ethoxy)pro panamido)propanamide |
| 125 | HC75-6 | 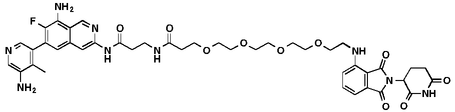 | *N*-(3-((8-amino-6-(5-amino-4-methylpyridin-3-yl)-7-fluoroisoquinolin-3-yl)amino)-3-oxopropyl)-1-((2-(2,6-dioxopiperidin-3-yl)-1,3-dioxoisoindolin-4-yl)amino)-3,6,9,12-tetraoxa pentadecan-15-amide |
| 126 | HC75-7 | 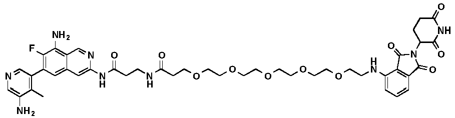 | *N*-(3-((8-amino-6-(5-amino-4-methylpyridin-3-yl)-7-fluoroisoquinolin-3-yl)amino)-3-oxopropyl)-1-((2-(2,6-dioxopiperidin-3-yl)-1,3-dioxoisoindolin-4-yl)amino)-3,6,9,12,15-pentaoxaoctadecan-18-amide |
| 127 | HC75-8 | 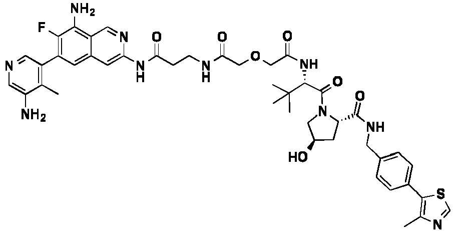 | (2*S*,4*R*)-1-((*S*)-2-(2-(2-((3-((8-amino-6-(5-amino-4-methylpyridin-3-yl)-7-fluoroisoquinolin-3-yl)amino)-3-oxopropyl)amino)-2-oxoethoxy)acetamido)-3,3-dimethylbutanoyl)-4-hydroxy-*N*-(4-(4-methylthiazol-5-yl)benzyl)pyrrolidine-2-carboxamide |
| 128 | HC75-9 | 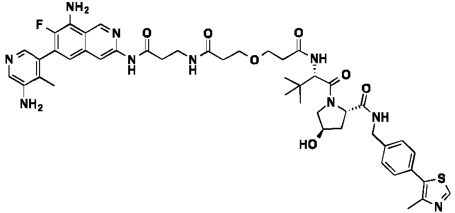 | (2*S*,4*R*)-1-((*S*)-2-(3-(3-((3-((8-amino-6-(5-amino-4-methylpyridin-3-yl)-7-fluoroisoquinolin-3-yl)amino)-3-oxopropyl)amino)-3-oxopropoxy)propanamido)-3,3-dimethylbutanoyl)-4-hydroxy-*N*-(4-(4-methylthiazol-5-yl)benzyl)pyrrolidine-2-carboxamide |
| 129 | HC75-10 | 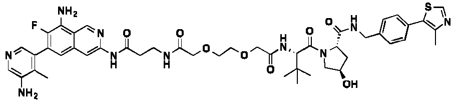 | (2*S*,4*R*)-1-((S)-15-((8-amino-6-(5-amino-4-methylpyridin-3-yl)-7-fluoroisoquinolin-3-yl)amino)-2-(*tert*-butyl)-4,11,15-trioxo-6,9-dioxa-3,12-diazapentadecanoyl)-4-hydroxy-*N*-(4-(4-methylthiazol-5-yl)benzyl)pyrrolidine-2-carboxamide |
| 130 | HC75-11 | 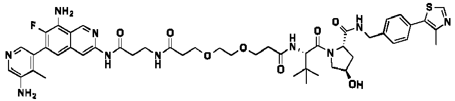 | (2*S*,4*R*)-1-((*S*)-17-((8-amino-6-(5-amino-4-methylpyridin-3-yl)-7-fluoroisoquinolin-3-yl)amino)-2-(fert-butyl)-4,13,17-trioxo-7,10-dioxa-3,14-diazaheptadecanoyl)-4-hydroxy-*N*-(4-(4-methylthiazol-5-yl)benzyl)pyrrolidine-2-carboxamide |
| 131 | HC75-12 | 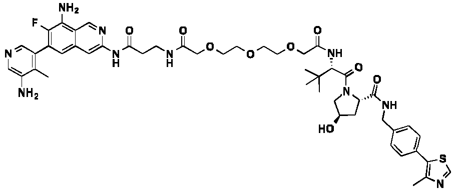 | (2*S*,4*R*)-1-((*S*)-18-((8-amino-6-(5-amino-4-methylpyridin-3-yl)-7-fluoroisoquinolin-3-yl)amino)-2-(fert-butyl)-4,14,18-trioxo-6,9,12-trioxa-3,15-diazaoctadecanoyl)-4-hydroxy-*N*-(4-(4-methylthiazol-5-yl)benzyl)pyrrolidine-2-carboxamide |
| 132 | HC75-13 | 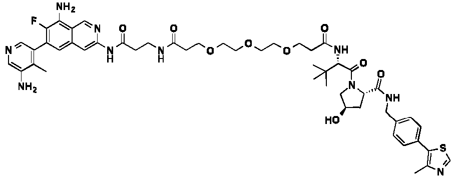 | (2*S*,4*R*)-1-((*S*)-20-((8-amino-6-(5-amino-4-methylpyridin-3-yl)-7-fluoroisoquinolin-3-yl)amino)-2-(tert-butyl)-4,16,20-trioxo-7,10,13-trioxa-3,17-diazaicosanoyl)-4-hydroxy-*N*-(4-(4-methylthiazol-5-yl)benzyl)pyrrolidine-2-carboxamide |
| 133 | HC75-14 | 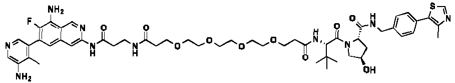 | *N*¹-(3-((8-amino-6-(5-amino-4-methylpyridin-3-yl)-7-fluoroisoquinolin-3-yl)amino)-3-oxopropyl)-*N*¹⁶-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)-4,7,10,13-tetraoxahexadecanediamide |
| 134 | HC75-15 | 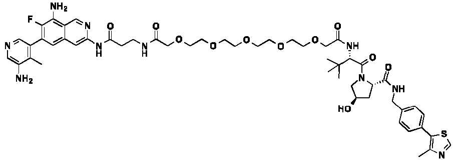 | *N*¹-(3-((8-amino-6-(5-amino-4-methylpyridin-3-yl)-7-fluoroisoquinolin-3-yl)amino)-3-oxopropyl)-*N*¹⁷-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)-3,6,9,12,15-pentaoxaheptadecanediamide |
| 135 | HC75-16 | 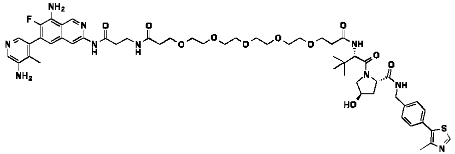 | *N*¹-(3-((8-amino-6-(5-amino-4-methylpyridin-3-yl)-7-fluoroisoquinolin-3-yl)amino)-3-oxopropyl)-*N*¹⁹-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)-4,7,10,13,16-pentaoxanonadecanediamide |
| 136 | HC75-17 | 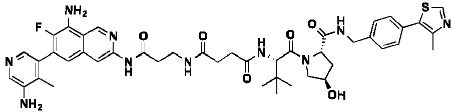 | *N*¹-(3-((8-amino-6-(5-amino-4-methylpyridin-3-yl)-7-fluoroisoquinolin-3-yl)amino)-3-oxopropyl)-*N*⁴-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)succinamide |
| 137 | HC75-18 | 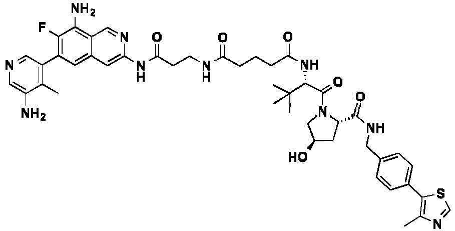 | *N*¹-(3-((8-amino-6-(5-amino-4-methylpyridin-3-yl)-7-fluoroisoquinolin-3-yl)amino)-3-oxopropyl)-*N*⁵-((*S*)-1*-*((*2*S*,*4*R*)-4-hydroxy-2-((4-(4-methylthiazol-5-yl)benzyl)carbamoyl)pyrrolidin-1-yl)-3,3-dimethyl-1-oxobutan-2-yl)glutaramide |
| 138 | HC75-19 | 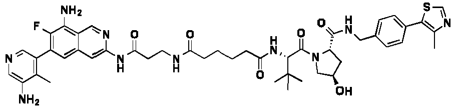 | *N*¹-(3-((8-amino-6-(5-amino-4-methylpyridin-3-yl)-7-fluoroisoquinolin-3-yl)amino)-3-oxopropyl)-*N*⁶-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)adipamide |
| 139 | HC75-20 | 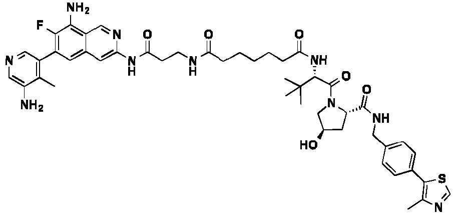 | *N*¹-(3-((8-amino-6-(5-amino-4-methylpyridin-3-yl)-7-fluoroisoquinolin-3-yl)amino)-3-oxopropyl)-*N*⁷-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)heptanediamide |
| 140 | HC75-21 | 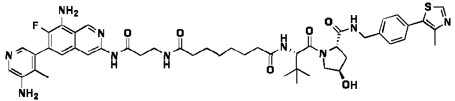 | *N*¹-(3-((8-amino-6-(5-amino-4-methylpyridin-3-yl)-7-fluoroisoquinolin-3-yl)amino)-3-oxopropyl)-*N*⁸-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)octanediamide |
| 141 | HC75-22 | 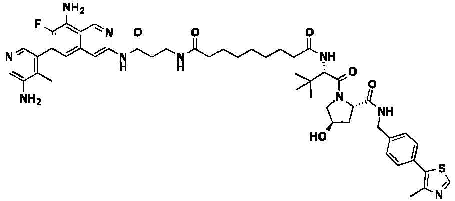 | *N*¹-(3-((8-amino-6-(5-amino-4-methylpyridin-3-yl)-7-fluoroisoquinolin-3-yl)amino)-3-oxopropyl)-*N*⁹-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)nonanediamide |
| 142 | HC75-23 | 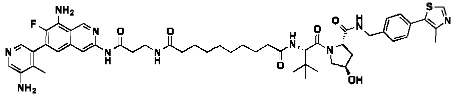 | *N*¹-(3-((8-amino-6-(5-amino-4-methylpyridin-3-yl)-7-fluoroisoquinolin-3-yl)amino)-3-oxopropyl)-*N*¹⁰-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)decanediamide |
| 143 | HC75-24 | 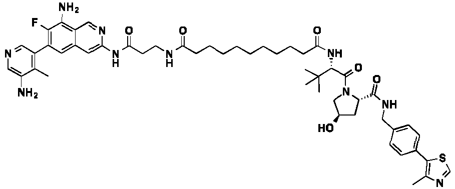 | *N*¹-(3-((8-amino-6-(5-amino-4-methylpyridin-3-yl)-7-fluoroisoquinolin-3-yl)amino)-3-oxopropyl)-*N*¹¹-((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)undecanediamide |
| 144 | HC75-29 | 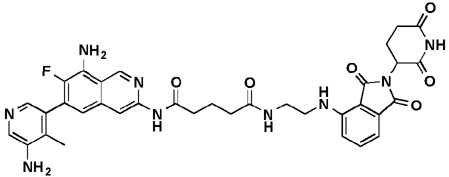 | *N*¹-(8-amino-6-(5-amino-4-methylpyridin-3-yl)-7-fluoroisoquinolin-3-yl)-*N*⁵-(2-((2-(2,6-dioxopiperidin-3-yl)-1,3-dioxoisoindolin-4-yl)amino)ethyl)glutaramide |
| 145 | HC75-31 | 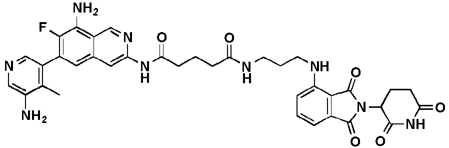 | *N*¹-(8-amino-6-(5-amino-4-methylpyridin-3-yl)-7-fluoroisoquinolin-3-yl)-*N*⁵-(3-((2-(2,6-dioxopiperidin-3-yl)-1,3-dioxoisoindolin-4-yl)amino)propyl)glutaramide |
| 146 | HC75-34 | 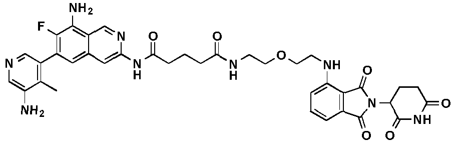 | *N*¹-(8-amino-6-(5-amino-4-methylpyridin-3-yl)-7-fluoroisoquinolin-3-yl)-*N*⁵-(2-(2-((2-(2,6-dioxopiperidin-3-yl)-1,3-dioxoisoindolin-4-yl)amino)ethoxy)ethyl)glutaramide |
| 147 | HC75-35 | 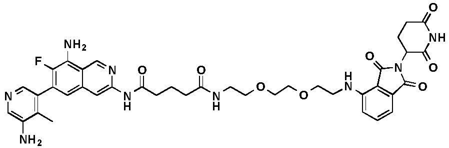 | *N*¹-(8-amino-6-(5-amino-4-methylpyridin-3-yl)-7-fluoroisoquinolin-3-yl)-*N*⁵-(2-(2-(2-((2-(2,6-dioxopiperidin-3-yl)-1,3-dioxoisoindolin-4-yl)amino)ethoxy)ethoxy)ethyl)gluta ramide |
| 148 | HC75-36 | 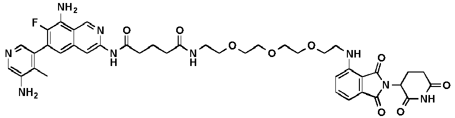 | *N*¹-(8-amino-6-(5-amino-4-methylpyridin-3-yl)-7-fluoroisoquinolin-3-yl)-*N*⁵-(2-(2-(2-(2-((2-(2,6-dioxopiperidin-3-yl)-1,3-dioxoisoindolin-4-yl)amino)ethoxy)ethoxy)ethoxy)eth yl)glutaramide |
| 149 | HC75-37 | 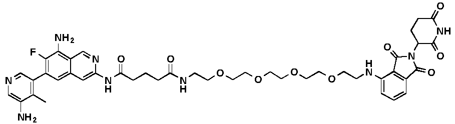 | *N*¹-(8-amino-6-(5-amino-4-methylpyridin-3-yl)-7-fluoroisoquinolin-3-yl)-*N*⁵-(14-((2-(2,6-dioxopiperidin-3-yl)-1,3-dioxoisoindolin-4-yl)amino)-3,6,9,12-tetraoxatetradecyl)glutaramide |
| 150 | HC75-38 | 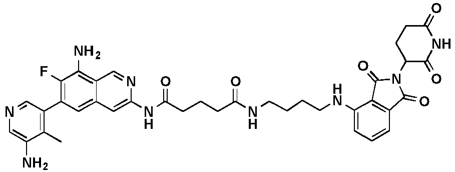 | *N*¹-(8-amino-6-(5-amino-4-methylpyridin-3-yl)-7-fluoroisoquinolin-3-yl)-*N*⁵-(4-((2-(2,6-dioxopiperidin-3-yl)-1,3-dioxoisoindolin-4-yl)amino)butyl)glutaramide |
| 151 | HC75-39 | 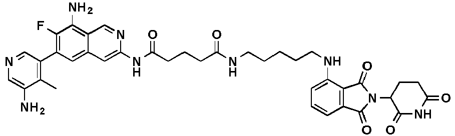 | *N*¹-(8-amino-6-(5-amino-4-methylpyridin-3-yl)-7-fluoroisoquinolin-3-yl)-*N*⁵-(5-((2-(2,6-dioxopiperidin-3-yl)-1,3-dioxoisoindolin-4-yl)amino)pentyl)glutaramide |
| 152 | HC75-40 | 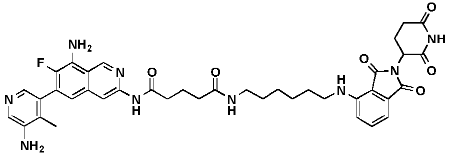 | *N*¹-(8-amino-6-(5-amino-4-methylpyridin-3-yl)-7-fluoroisoquinolin-3-yl)-*N*⁵-(6-((2-(2,6-dioxopiperidin-3-yl)-1,3-dioxoisoindolin-4-yl)amino)hexyl)glutaramide |
| 153 | HC75-41 | 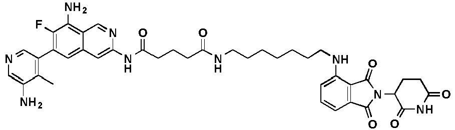 | *N*¹-(8-amino-6-(5-amino-4-methylpyridin-3-yl)-7-fluoroisoquinolin-3-yl)-*N*⁵-(7-((2-(2,6-dioxopiperidin-3-yl)-1,3-dioxoisoindolin-4-yl)amino)heptyl)glutaramide |
| 154 | HC75-42 | 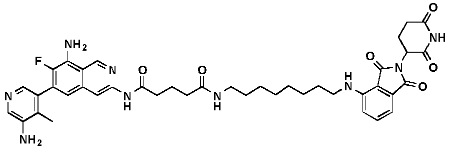 | *N*¹-(8-amino-6-(5-amino-4-methylpyridin-3-yl)-7-fluoroisoquinolin-3-yl)-*N*⁵-(8-((2-(2,6-dioxopiperidin-3-yl)-1,3-dioxoisoindolin-4-yl)amino)octyl)glutaramide |
| 155 | HC75-43 | 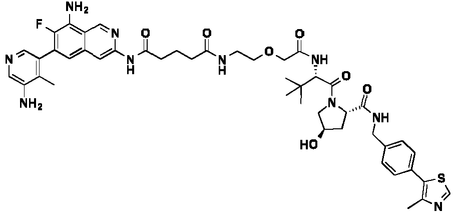 | *N*¹-(8-amino-6-(5-amino-4-methylpyridin-3-yl)-7-fluoroisoquinolin-3-yl)-*N*⁵-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethyl)glutaramide |
| 156 | HC75-44 | 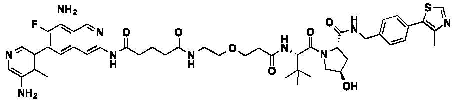 | *N*¹-(8-amino-6-(5-amino-4-methylpyridin-3-yl)-7-fluoroisoquinolin-3-yl)-*N*⁵-(2-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)ethyl)glutaramide |
| 157 | HC75-45 | 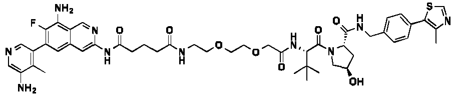 | *N*¹-(8-amino-6-(5-amino-4-methylpyridin-3-yl)-7-fluoroisoquinolin-3-yl)-*N*⁵-(2-(2-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)glutaramid e |
| 158 | HC75-46 | 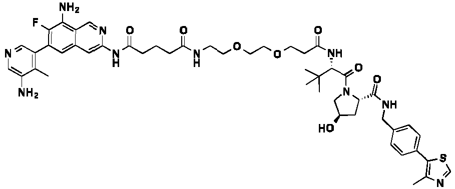 | *N*¹-(8-amino-6-(5-amino-4-methylpyridin-3-yl)-7-fluoroisoquinolin-3-yl)-*N*⁵-(2-(2-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropoxy)ethoxy)ethyl)glutarami de |
| 159 | HC75-47 | 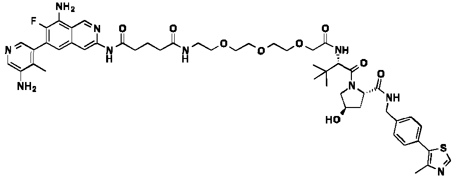 | *N*¹-(8-amino-6-(5-amino-4-methylpyridin-3-yl)-7-fluoroisoquinolin-3-yl)-*N*⁵-((*S*)-13-((2*S*,4*R*)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecyhglutaramide |
| 160 | HC75-48 | 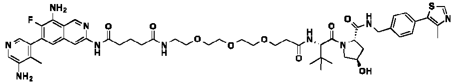 | *N*¹-(8-amino-6-(5-amino-4-methylpyridin-3-yl)-7-fluoroisoquinolin-3-yl)-*N*⁵-((*S*)-14-((2*S*,4*R*)-4-hydroxy-2-((4-(4-methylthiazol-5-yl)benzyl)carbamoyl)pyrrolidine-1-carbonyl)-15,15-dimethyl-12-oxo-3,6,9-trioxa-13-azahexadecyl)glutaramide |
| 161 | HC75-49 | 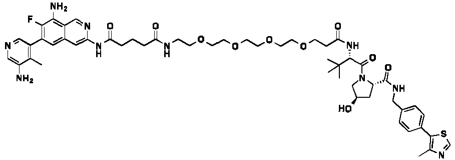 | *N*¹-(8-amino-6-(5-amino-4-methylpyridin-3-yl)-7-fluoroisoquinolin-3-yl)-*N*⁵-((*S*)-17-((2*S*,4*R*)-4-hydroxy-2-((4-(4-methylthiazol-5-yl)benzyl)carbamoyl)pyrrolidine-1-carbonyl)-18,18-dimethyl-15-oxo-3,6,9,12-tetraoxa-16-azanonadecyl)glutaramide |
| 162 | HC75-50 | 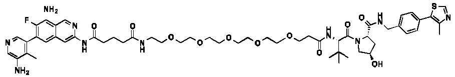 | *N*¹-(8-amino-6-(5-amino-4-methylpyridin-3-yl)-7-fluoroisoquinolin-3-yl)-*N*⁵-((*S*)-20-((2*S*,4*R*)-4-hydroxy-2-((4-(4-methylthiazol-5-yl)benzyl)carbamoyl)pyrrolidine-1-carbonyl)-21,21-dimethyl-18-oxo-3,6,9,12,15-pentaoxa-19-azadocosyl)glutaramide |
| 163 | HC75-52 | 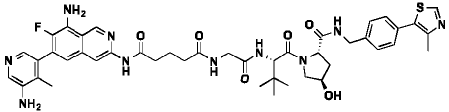 | *N*¹-(8-amino-6-(5-amino-4-methylpyridin-3-yl)-7-fluoroisoquinolin-3-yl)-*N*⁵-(2-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-2-oxoethyl)glutaramide |
| 164 | HC75-53 | 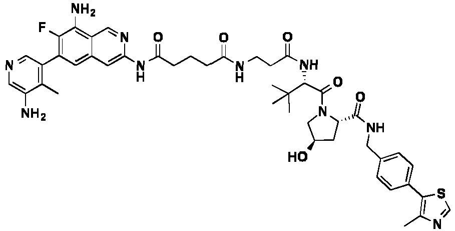 | *N*¹-(8-amino-6-(5-amino-4-methylpyridin-3-yl)-7-fluoroisoquinolin-3-yl)-*N*⁵-(3-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-3-oxopropyl)glutaramide |
| 165 | HC75-54 | 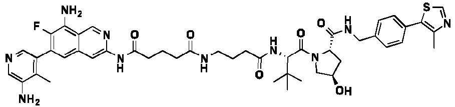 | *N*¹-(8-amino-6-(5-amino-4-methylpyridin-3-yl)-7-fluoroisoquinolin-3-yl)-*N*⁵-(4-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-4-oxobutyl)glutaramide |
| 166 | HC75-55 | 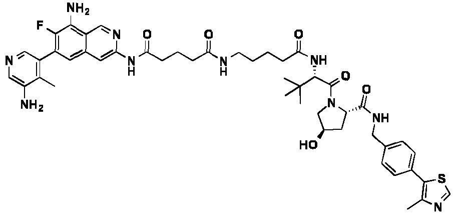 | *N*¹-(8-amino-6-(5-amino-4-methylpyridin-3-yl)-7-fluoroisoquinolin-3-yl)-*N*⁵-(5-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-5-oxopentyl)glutaramide |
| 167 | HC75-56 | 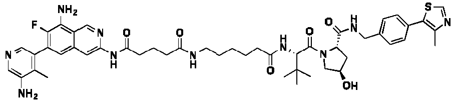 | *N*¹-(8-amino-6-(5-amino-4-methylpyridin-3-yl)-7-fluoroisoquinolin-3-yl)-*N*⁵-(6-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-6-oxohexyl)glutaramide |
| 168 | HC75-57 | 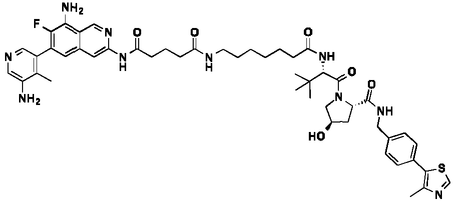 | *N*¹-(8-amino-6-(5-amino-4-methylpyridin-3-yl)-7-fluoroisoquinolin-3-yl)-*N*⁵-(7-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-7-oxoheptyl)glutaramide |
| 169 | HC75-58 | 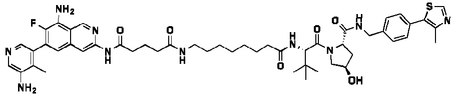 | *N*¹-(8-amino-6-(5-amino-4-methylpyridin-3-yl)-7-fluoroisoquinolin-3-yl)-*N*⁵-(8-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-8-oxooctyl)qlutaramide |
| 170 | HC75-59 | 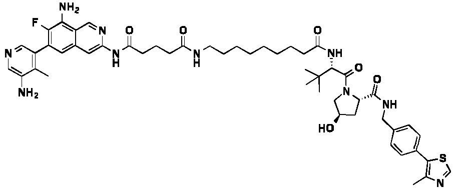 | *N*¹-(8-amino-6-(5-amino-4-methylpyridin-3-yl)-7-fluoroisoquinolin-3-yl)-*N*⁵-(9-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-9-oxononyl)glutaramide |
| 171 | HC75-60 | 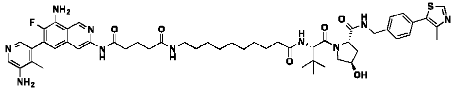 | *N*¹-(8-amino-6-(5-amino-4-methylpyridin-3-yl)-7-fluoroisoquinolin-3-yl)-*N*⁵-(10-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-10-oxodecyl)glutaramide |
| 172 | HC75-61 | 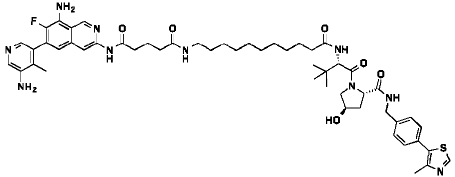 | *N*¹-(8-amino-6-(5-amino-4-methylpyridin-3-yl)-7-fluoroisoquinolin-3-yl)-N⁵-(11-(((*S*)-1-((2*S*,4*R*)-4-hydroxy-2-((4-(4-methylthiazol-5-yl)benzyl)carbamoyl)pyrrolidin-1-yl)-3,3-dimethyl-1-oxobutan-2-yl)amino)-11-oxoundecyl)glutaramide |
| 173 | HC75-62 | 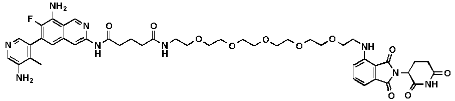 | *N*¹-(8-amino-6-(5-amino-4-methylpyridin-3-yl)-7-fluoroisoquinolin-3-yl)-*N*⁵-(17-((2-(2,6-dioxopiperidin-3-yl)-1,3-dioxoisoindolin-4-yl)amino)-3,6,9,12,15-pentaoxaheptadecyl)glutaramide |
| 175 | HC90-33 | 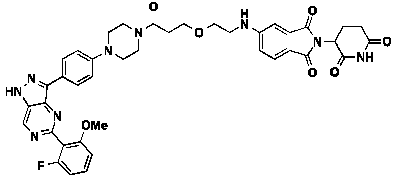 | 2-(2,6-dioxopiperidin-3-yl)-5-((2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethyl)amino)isoindoli ne-1,3-dione |
| 176 | HC90-34 | 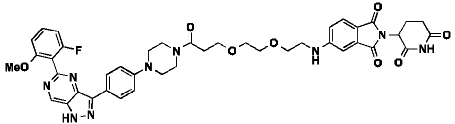 | 2-(2,6-dioxopiperidin-3-yl)-5-((2-(2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethoxy)ethyl)amino)i soindoline-1,3-dione |
| 177 | HC90-35 | 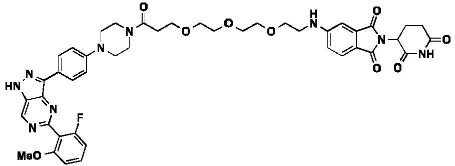 | 2-(2,6-dioxopiperidin-3-yl)-5-((2-(2-(2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethoxy)ethoxy)ethyl) amino)isoindoline-1,3-dione |
| 178 | HC90-36 | 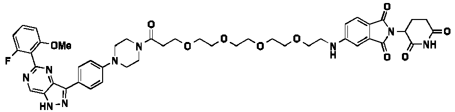 | 2-(2,6-dioxopiperidin-3-yl)-5-((15-(4-(4-(5-(2-fluoro-6-methoxyphenyl)-1*H*-pyrazolo[4,3-*d*]pyrimidin-3-yl)phenyl)piperazin-1-yl)-15-oxo-3,6,9,12-tetraoxapentadecyl)amino)isoind oline-1,3-dione |
| 179 | HC90-37 | 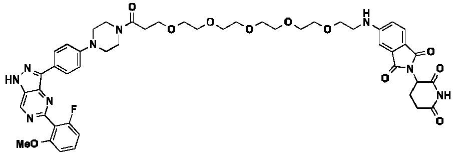 | 2-(2,6-dioxopiperidin-3-yl)-5-((18-(4-(4-(5-(2-fluoro-6-methoxyphenyl)-1*H*-pyrazolo[4,3-*d*]pyrimidin-3-yl)phenyl)piperazin-1-yl)-18-oxo-3,6,9,12,15-pentaoxaoctadecyl)amino)isoindo line-1,3-dione |
| 180 | HC90-41 | 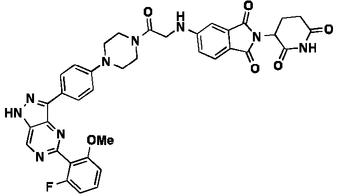 | 2-(2,6-dioxopiperidin-3-yl)-5-((2-(4-(4-(5-(2-fluoro-6-methoxyphenyl)-1*H*-pyrazolo[4,3-*d*]pyrimidin-3-yl)phenyl)piperazin-1-yl)-2-oxoethyl)amino)isoindoline-1,3-dione |
| 181 | HC90-42 | 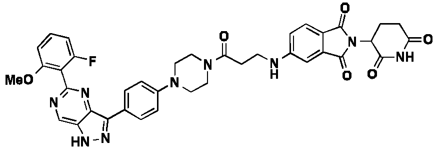 | 2-(2,6-dioxopiperidin-3-yl)-5-((3-(4-(4-(5-(2-fluoro-6-methoxyphenyl)-1*H*-pyrazolo[4,3-*d*]pyrimidin-3-yl)phenyl)piperazin-1-yl)-3-oxopropyl)amino)isoindoline-1,3-dione |
| 182 | HC90-43 | 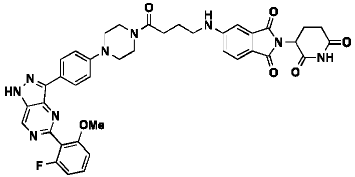 | 2-(2,6-dioxopiperidin-3-yl)-5-((4-(4-(4-(5-(2-fluoro-6-methoxyphenyl)-1*H*-pyrazolo[4,3-*d*]pyrimidin-3-yl)phenyl)piperazin-1-yl)-4-oxobutyl)amino)isoindoline-1,3-dione |
| 183 | HC90-44 | 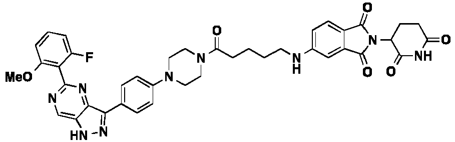 | 2-(2,6-dioxopiperidin-3-yl)-5-((5-(4-(4-(5-(2-fluoro-6-methoxyphenyl)-1*H*-pyrazolo[4,3-*d*]pyrimidin-3-yl)phenyl)piperazin-1-yl)-5-oxopentyl)amino)isoindoline-1,3-dione |
| 184 | HC90-45 | 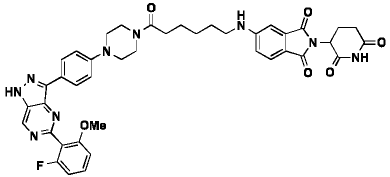 | 2-(2,6-dioxopiperidin-3-yl)-5-((6-(4-(4-(5-(2-fluoro-6-methoxyphenyl)-1*H*-pyrazolo[4,3-*d*]pyrimidin-3-yl)phenyl)piperazin-1-yl)-6-oxohexyl)amino)isoindoline-1,3-dione |
| 185 | HC90-46 | 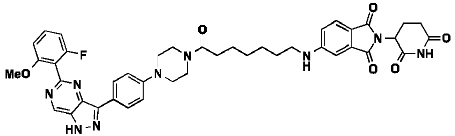 | 2-(2,6-dioxopiperidin-3-yl)-5-((7-(4-(4-(5-(2-fluoro-6-methoxyphenyl)-1*H*-pyrazolo[4,3-*d*]pyrimidin-3-yl)phenyl)piperazin-1-yl)-7-oxoheptyl)amino)isoindoline-1,3-dione |
| 186 | HC90-47 | 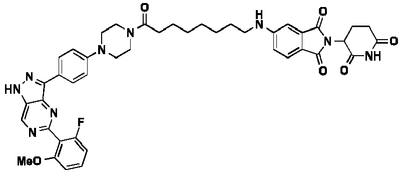 | 2-(2,6-dioxopiperidin-3-yl)-5-((8-(4-(4-(5-(2-fluoro-6-methoxyphenyl)-1*H*-pyrazolo[4,3-*d*]pyrimidin-3-yl)phenyl)piperazin-1-yl)-8-oxooctyl)amino)isoindoline-1,3-dione |
| 187 | HC90-49 | 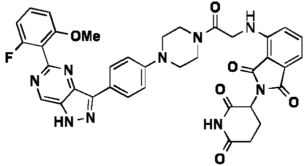 | 2-(2,6-dioxopiperidin-3-yl)-4-((2-(4-(4-(5-(2-fluoro-6-methoxyphenyl)-1*H*-pyrazolo[4,3-*d*]pyrimidin-3-yl)phenyl)piperazin-1-yl)-2-oxoethyl)amino)isoindoline-1,3-dione |
| 188 | HC90-50 | 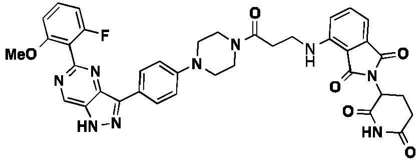 | 2-(2,6-dioxopiperidin-3-yl)-4-((3-(4-(4-(5-(2-fluoro-6-methoxyphenyl)-1*H*-pyrazolo[4,3-*d*]pyrimidin-3-yl)phenyl)piperazin-1-yl)-3-oxopropyl)amino)isoindoline-1,3-dione |
| 189 | HC90-51 | 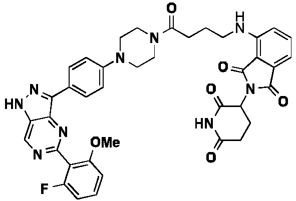 | 2-(2,6-dioxopiperidin-3-yl)-4-((4-(4-(4-(5-(2-fluoro-6-methoxyphenyl)-1*H*-pyrazolo[4,3-*d*]pyrimidin-3-yl)phenyl)piperazin-1-yl)-4-oxobutyl)amino)isoindoline-1,3-dione |
| 190 | HC90-52 | 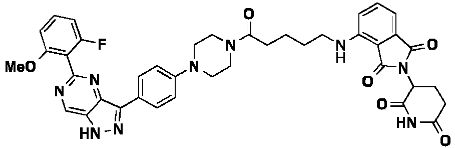 | 2-(2,6-dioxopiperidin-3-yl)-4-((5-(4-(4-(5-(2-fluoro-6-methoxyphenyl)-1*H*-pyrazolo[4,3-*d*]pyrimidin-3-yl)phenyl)piperazin-1-yl)-5-oxopentyl)amino)isoindoline-1,3-dione |
| 191 | HC90-53 | 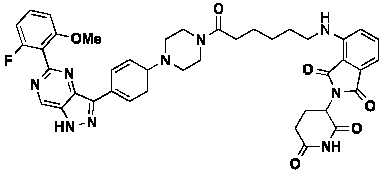 | 2-(2,6-dioxopiperidin-3-yl)-4-((6-(4-(4-(5-(2-fluoro-6-methoxyphenyl)-1*H*-pyrazolo[4,3-*d*]pyrimidin-3-yl)phenyl)piperazin-1-yl)-6-oxohexyl)amino)isoindoline-1,3-dione |
| 192 | HC90-54 | 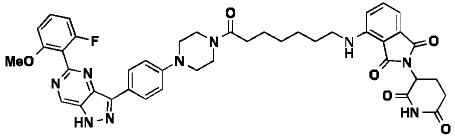 | 2-(2,6-dioxopiperidin-3-yl)-4-((7-(4-(4-(5-(2-fluoro-6-methoxyphenyl)-1*H*-pyrazolo[4,3-*d*]pyrimidin-3-yl)phenyl)piperazin-1-yl)-7-oxoheptyl)amino)isoindoline-1,3-dione |
| 193 | HC90-55 | 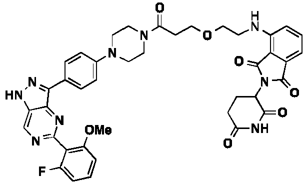 | 2-(2,6-dioxopiperidin-3-yl)-4-((2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethyl)amino)isoindoli ne-1,3-dione |
| 194 | HC90-56 | 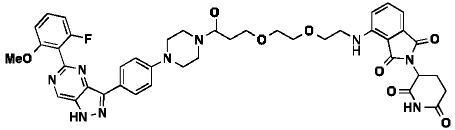 | 2-(2,6-dioxopiperidin-3-yl)-4-((2-(2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethoxy)ethyl)amino)i soindoline-1,3-dione |
| 195 | HC90-57 | 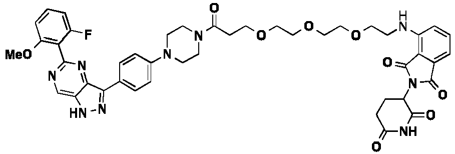 | 2-(2,6-dioxopiperidin-3-yl)-4-((2-(2-(2-(3-(4-(4-(5-(2-fluoro-6-methoxyphenyl)-1*H*-pyrazolo[4,3-*d*]pyrimidin-3-yl)phenyl)piperazin-1-yl)-3-oxopropoxy)ethoxy)ethoxy)ethyl) amino)isoindoline-1,3-dione |
| 196 | HC90-58 | 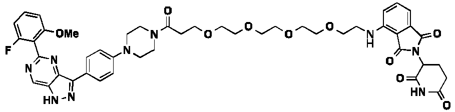 | 2-(2,6-dioxopiperidin-3-yl)-4-((15-(4-(4-(5-(2-fluoro-6-methoxyphenyl)-1*H*-pyrazolo[4,3-*d*]pyrimidin-3-yl)phenyl)piperazin-1-yl)-15-oxo-3,6,9,12-tetraoxapentadecyl)amino)isoind oline-1,3-dione |
| 197 | HC90-59 | 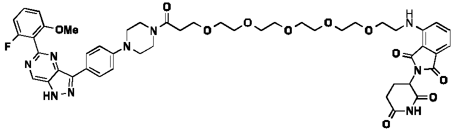 | 2-(2,6-dioxopiperidin-3-yl)-4-((18-(4-(4-(5-(2-fluoro-6-methoxyphenyl)-1*H*-pyrazolo[4,3-*d*]pyrimidin-3-yl)phenyl)piperazin-1-yl)-18-oxo-3,6,9,12,15-pentaoxaoctadecyl)amino)isoindo line-1,3-dione |
| 198 | HC90-61 | 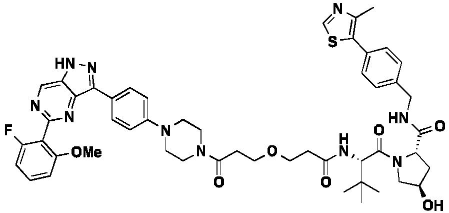 | (2*S*,4*R*)-1-((*S*)-2-(3-(3-(4-(4-(5-(2-fluoro-6-methoxyphenyl)-1*H-*pyrazolo[4,3-*d*]pyrimidin-3-yl)phenyl)piperazin-1-yl)-3-oxopropoxy)propanamido)-3,3-dimethylbutanoyl)-4-hydroxy-*N-*(4-(4-methylthiazol-5-yl)benzyl)pyrrolidine-2-carboxamide |
| 199 | HC90-66 | 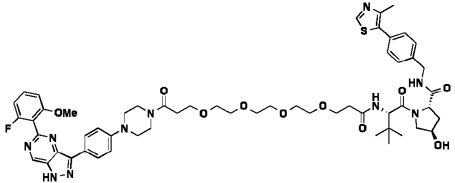 | (2*S*,4*R*)-1-((S)-2-(*tert*-butyl)-19-(4-(4-(5-(2-fluoro-6-methoxyphenyl)-1*H*-pyrazolo[4,3-*d*]pyrimidin-3-yl)phenyl)piperazin-1-yl)-4,19-dioxo-7,10,13,16-tetraoxa-3-azanonadecanoyl)-4-hydroxy-*N*-(4-(4-methylthiazol-5-yl)benzyl)pyrrolidine-2-carboxamide |
| 200 | HC90-69 | 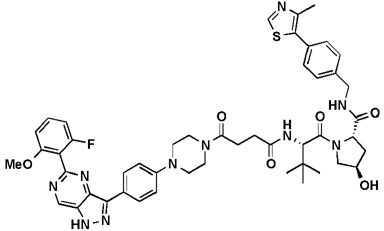 | (2*S*,4*R*)-1-((*S*)-2-(4-(4-(4-(5-(2-fluoro-6-methoxyphenyl)-1*H-*pyrazolo[4,3-*d*]pyrimidin-3-yl)phenyl)piperazin-1-yl)-4-oxobutanamido)-3,3-dimethylbutanoyl)-4-hydroxy-*N-*(4-(4-methylthiazol-5-yl)benzyl)pyrrolidine-2-carboxamide |
| 201 | HC90-70 | 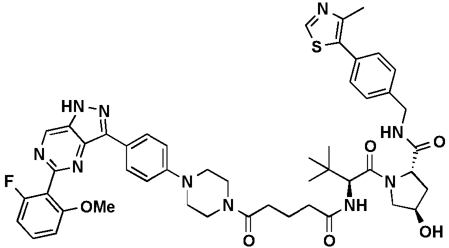 | (2*S*,4*R*)-1-((*S*)-2-(5-(4-(4-(5-(2-fluoro-6-methoxyphenyl)-1*H-*pyrazolo[4,3-*d*]pyrimidin-3-yl)phenyl)piperazin-1-yl)-5-oxopentanamido)-3,3-dimethylbutanoyl)-4-hydroxy-*N-*(4-(4-methylthiazol-5-yl)benzyl)pyrrolidine-2-carboxamide |
| 202 | HC90-71 | 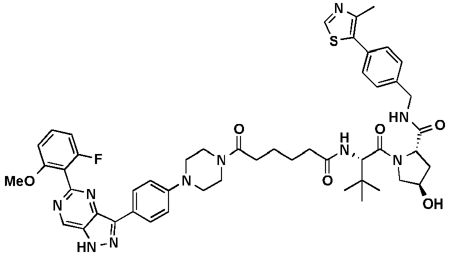 | (2*S*,4*R*)-1-((*S*)-2-(6-(4-(4-(5-(2-fluoro-6-methoxyphenyl)-1*H-*pyrazolo[4,3-*d*]pyrimidin-3-yl)phenyl)piperazin-1-yl)-6-oxohexanamido)-3,3-dimethylbutanoyl)-4-hydroxy-*N-*(4-(4-methylthiazol-5-yl)benzyl)pyrrolidine-2-carboxamide |
| 203 | HC90-72 | 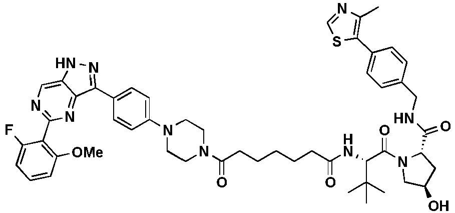 | (2*S*,4*R*)-1-((*S*)-2-(7-(4-(4-(5-(2-fluoro-6-methoxyphenyl)-1*H-*pyrazolo[4,3-*d*]pyrimidin-3-yl)phenyl)piperazin-1-yl)-7-oxoheptanamido)-3,3-dimethylbutanoyl)-4-hydroxy-*N-*(4-(4-methylthiazol-5-yl)benzyl)pyrrolidine-2-carboxamide |
| 204 | HC90-73 | 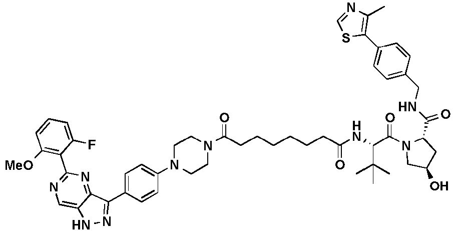 | (2S,4R)-1-((S)-2-(8-(4-(4-(5-(2-fluoro-6-methoxyphenyl)-1H-pyrazolo[4,3-d]pyrimidin-3-yl)phenyl)piperazin-1-yl)-8-oxooctanamido)-3,3-dimethylbutanoyl)-4-hydroxy-N-(4-(4-methylthiazol-5-yl)benzyl)pyrrolidine-2-carboxamide |
| 205 | HC90-74 | 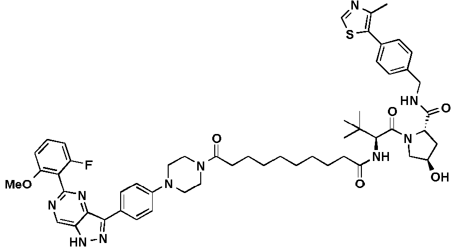 | (2S,4R)-1-((S)-2-(10-(4-(4-(5-(2-fluoro-6-methoxyphenyl)-1H-pyrazolo[4,3-d]pyrimidin-3-yl)phenyl)piperazin-1-yl)-10-oxodecanamido)-3,3-dimethylbutanoyl)-4-hydroxy-N-(4-(4-methylthiazol-5-yl)benzyl)pyrrolidine-2-carboxamide |
| 206 | HC90-84 | 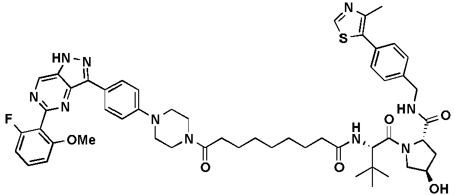 | (2*S*,4*R*)-1-((*S*)-2-(9-(4-(4-(5-(2-fluoro-6-methoxyphenyl)-1*H-*pyrazolo[4,3-*d*]pyrimidin-3-yl)phenyl)piperazin-1-yl)-9-oxononanamido)-3,3-dimethylbutanoyl)-4-hydroxy-*N-*(4-(4-methylthiazol-5-yl)benzyl)pyrrolidine-2-carboxamide |
| 207 | HC90-85 | 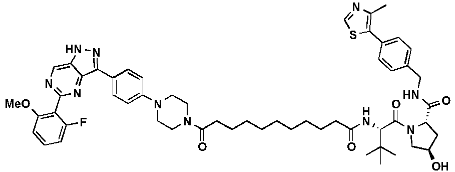 | (2S,4R)-1-((S)-2-(11-(4-(4-(5-(2-fluoro-6-methoxyphenyl)-1H-pyrazolo[4,3-d]pyrimidin-3-yl)phenyl)piperazin-1-yl)-11-oxoundecanamido)-3,3-dimethylbutanoyl)-4-hydroxy-N-(4-(4-methylthiazol-5-yl)benzyl)pyrrolidine-2-carboxamide |
| 209 | HC90-86 | 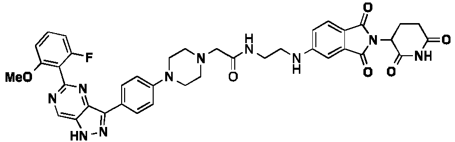 | *N*-(2-((2-(2,6-dioxopiperidin-3-yi)-1,3-dioxoisoindolin-5-yl)amino)ethyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 210 | HC90-87 | 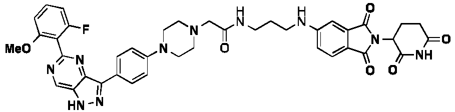 | *N*-(3-((2-(2,6-dioxopiperidin-3-yl)-1,3-dioxoisoindolin-5-yl)amino)propyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 211 | HC90-88 | 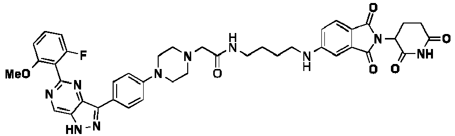 | *N*-(4-((2-(2,6-d ioxopiperid in-3-yi)-1,3-dioxoisoindolin-5-yl)amino)butyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 212 | HC90-89 | 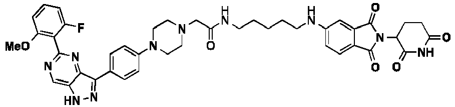 | *N*-(5-((2-(2,6-dioxopiperidin-3-yl)-1,3-dioxoisoindolin-5-yl)amino)pentyl)-2-(4-(4-(5-(2-fluoro-6-methoxyphenyl)-1*H-*pyrazolo[4,3-*d*]pyrimidin-3-yl)phenyl)piperazin-1-vl)acetamide |
| 213 | HC90-90 | 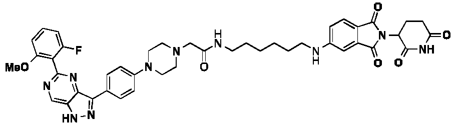 | *N*-(6-((2-(2,6-dioxopiperidin-3-yl)-1,3-dioxoisoindolin-5-yl)amino)hexyl)-2-(4-(4-(5-(2-fluoro-6-methoxyphenyl)-1*H-*pyrazolo[4,3-*d*]pyrimidin-3-yl)phenyl)piperazin-1-vl)acetamide |
| 214 | HC90-91 | 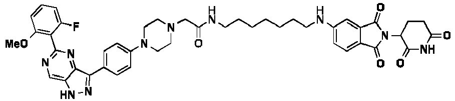 | *N*-(7-((2-(2,6-dioxopiperidin-3-yl)-1,3-dioxoisoindolin-5-yl)amino)heptyl)-2-(4-(4-(5-(2-fluoro-6-methoxyphenyl)-1*H-*pyrazolo[4,3-*d*]pyrimidin-3-yl)phenyl)piperazin-1-vl)acetamide |
| 215 | HC90-92 | 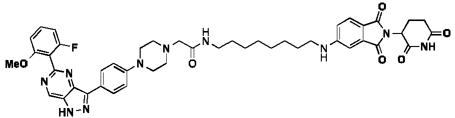 | *N*-(8-((2-(2,6-dioxopiperidin-3-yl)-1,3-dioxoisoindolin-5-yl)amino)octyl)-2-(4-(4-(5-(2-fluoro-6-methoxyphenyl)-1*H-*pyrazolo[4,3-*d*]pyrimidin-3-yl)phenyl)piperazin-1-vl)acetamide |
| 216 | HC90-93 | 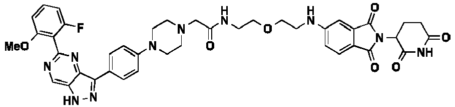 | N-(2-(2-((2-(2,6-dioxopiperidin-3-yl)-1,3-dioxoisoindolin-5-yl)amino)ethoxy)ethyl)-2-(4-(4-(5-(2-fluoro-6-methoxyphenyl)-1H-pyrazolo[4,3-d]pyrimidin-3-yl)phenyl)piperazin-1-vl)acetamide |
| 217 | HC90-94 | 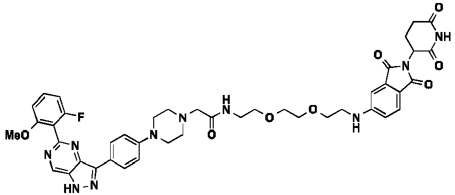 | N-(2-(2-(2-((2-(2,6-dioxopiperidin-3-yl)-1,3-dioxoisoindolin-5-yl)amino)ethoxy)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide |
| 218 | HC90-95 | 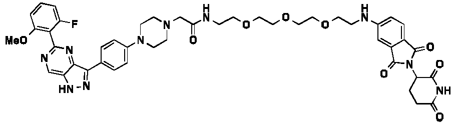 | N-(2-(2-(2-(2-((2-(2,6-dioxopiperidin-3-yl)-1,3-dioxoisoindolin-5-yl)amino)ethoxy)ethoxy)ethoxy)et hyl)-2-(4-(4-(5-(2-fluoro-6-methoxyphenyl)-1H-pyrazolo[4,3-d]pyrimidin-3-yl)phenyl)piperazin-1-yl)acetamide |
| 219 | HC90-96 | 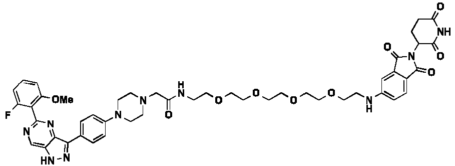 | N-(14-((2-(2,6-dioxopiperidin-3-yl)-1,3-dioxoisoindolin-5-yl)amino)-3,6,9,12-tetraoxatetradecyl)-2-(4-(4-(5-(2-fluoro-6-methoxyphenyl)-1H-pyrazolo[4,3-d]pyrimidin-3-yl)phenyl)piperazin-1-yl)acetamide |
| 220 | HC90-97 | 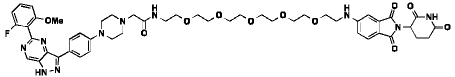 | N-(17-((2-(2,6-dioxopiperidin-3-yl)-1,3-dioxoisoindolin-5-yl)amino)-3,6,9,12,15-pentaoxaheptadecyl)-2-(4-(4-(5-(2-fluoro-6-methoxyphenyl)-1H-pyrazolo[4,3-d]pyrimidin-3-yl)phenyl)piperazin-1-yl)acetamide |
| 221 | HC90-102 | 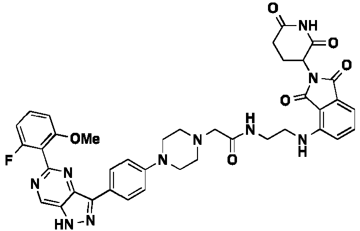 | *N*-(2-((2-(2,6-dioxopiperidin-3-yl)-1,3-dioxoisoindolin-4-yl)amino)ethyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 222 | HC90-103 | 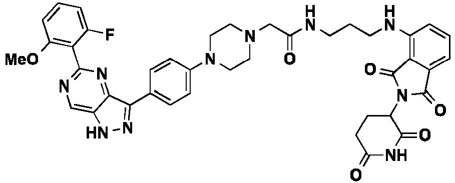 | *N*-(3-((2-(2,6-dioxopiperidin-3-yl)-1,3-dioxoisoindolin-4-yl)amino)propyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 223 | HC90-104 | 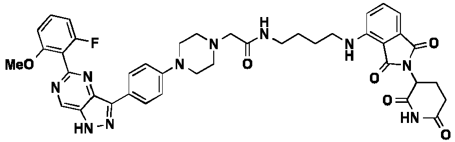 | *N*-(4-((2-(2,6-dioxopiperidin-3-yl)-1,3-dioxoisoindolin-4-yl)amino)butyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 224 | HC90-105 | 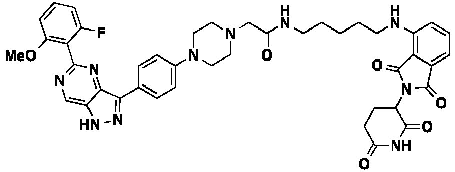 | *N*-(5-((2-(2,6-dioxopiperidin-3-yl)-1,3-dioxoisoindolin-4-yl)amino)pentyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 225 | HC90-106 | 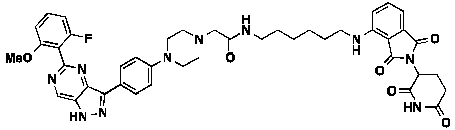 | *N*-(6-((2-(2,6-dioxopiperidin-3-yi)-1,3-dioxoisoindolin-4-yl)amino)hexyl)-2-(4-(4-(5-(2-fluoro-6-methoxyphenyl)-1*H-*pyrazolo[4,3-*d*]pyrimidin-3-yl)phenyl)piperazin-1-vl)acetamide |
| 226 | HC90-107 | 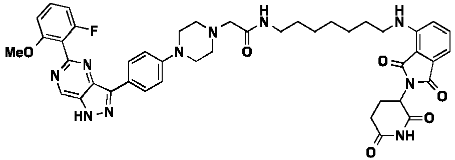 | *N*-(7-((2-(2,6-dioxopiperidin-3-yl)-1,3-dioxoisoindolin-4-yl)amino)heptyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 227 | HC90-108 | 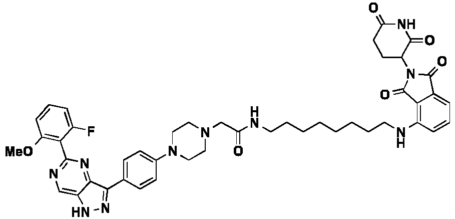 | *N*-(8-((2-(2,6-dioxopiperidin-3-yl)-1,3-dioxoisoindolin-4-yl)amino)octyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 228 | HC90-109 | 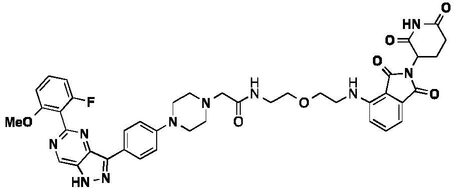 | *N*-(2-(2-((2-(2,6-dioxopiperidin-3-yl)-1,3-dioxoisoindolin-4-yl)amino)ethoxy)ethyl)-2-(4-(4-(5-(2-fluoro-6-methoxyphenyl)-1*H-*pyrazolo[4,3-d]pyrimidin-3-yl)phenyl)piperazin-1-yl)acetamide |
| 229 | HC90-110 | 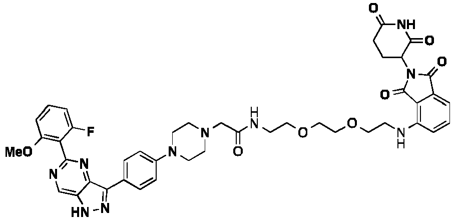 | *N*-(2-(2-(2-((2-(2,6-dioxopiperidin-3-yl)-1,3-dioxoisoindolin-4-yl)amino)ethoxy)ethoxy)ethyl)-2-(4-(4-(5-(2-fluoro-6-methoxyphenyl)-1*H*-pyrazolo[4,3-*d*]pyrimidin-3-yl)phenyl)piperazin-1-yl)acetamide |
| 230 | HC90-111 | 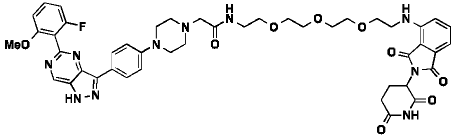 | *N*-(2-(2-(2-(2-((2-(2,6-dioxopiperidin-3-yl)-1,3-dioxoisoindolin-4-yl)amino)ethoxy)ethoxy)ethoxy)et hyl)-2-(4-(4-(5-(2-fluoro-6-methoxyphenyl)-1*H*-pyrazolo[4,3-*d*]pyrimidin-3-yl)phenyl)piperazin-1-vl)acetamide |
| 231 | HC90-112 | 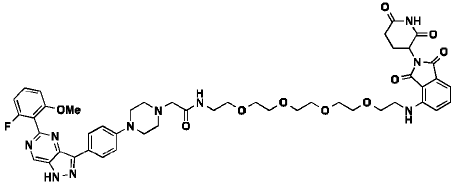 | *N*-(14-((2-(2,6-dioxopiperidin-3-yl)-1,3-dioxoisoindolin-4-yl)amino)-3,6,9,12-tetraoxatetradecyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 232 | HC90-113 | 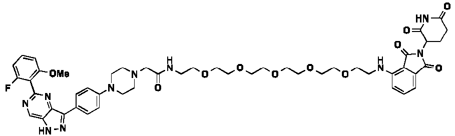 | *N*-(17-((2-(2,6-dioxopiperidin-3-yl)-1,3-dioxoisoindolin-4-yl)amino)-3,6,9,12,15-pentaoxaheptadecyl)-2-(4-(4-(5-(2-fluoro-6-methoxyphenyl)-1*H-*pyrazolo[4,3-*d*]pyrimidin-3-yl)phenyl)piperazin-1-yl)acetamide |
| 233 | HC90-117 | 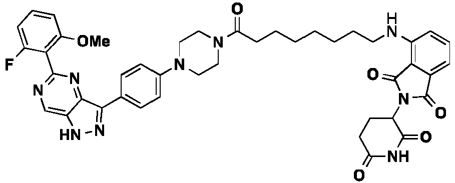 | 2-(2,6-dioxopiperidin-3-yl)-4-((8-(4-(4-(5-(2-fluoro-6-methoxyphenyl)-1*H*-pyrazolo[4,3-*d*]pyrimidin-3-yl)phenyl)piperazin-1-yl)-8-oxooctyl)amino)isoindoline-1,3-dione |
| 234 | HC90-119 | 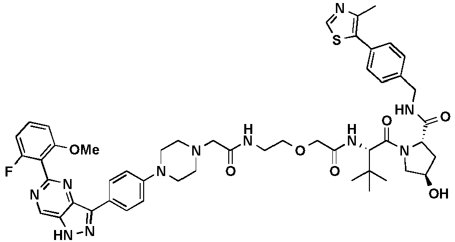 | (2*S*,4*R*)-1-((*S*)-2-(2-(2-(2-(4-(4-(5-(2-fluoro-6-methoxyphenyl)-1*H-*pyrazolo[4,3-*d*]pyrimidin-3-yl)phenyl)piperazin-1-yl)aceta mido)ethoxy)acetamido)-3,3-dimethylbutanoyl)-4-hydroxy-*N*-(4-(4-methylthiazol-5-yl)benzyl)pyrrolidine-2-carboxamide |
| 235 | HC90-120 | 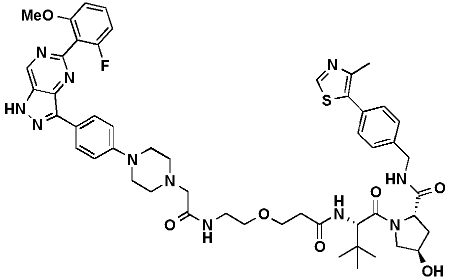 | (2*S*,4*R*)-1-((*S*)-2-(3-(2-(2-(4-(4-(5-(2-fluoro-6-methoxyphenyl)-1*H-*pyrazolo[4,3-*d*]pyrimidin-3-yl)phenyl)piperazin-1-yl)acetamido)ethoxy)propanamid o)-3,3-dimethylbutanoyl)-4-hydroxy-*N*-(4-(4-methylthiazol-5-yl)benzyl)pyrrolidine-2-carboxamide |
| 236 | HC90-121 | 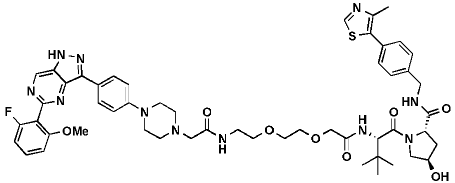 | (2*S*,4*R*)-1-((*S*)-2-(*tert*-butyl)-14-(4-(4-(5-(2-fluoro-6-methoxyphenyl)-1*H*-pyrazolo[4,3-*d*]pyrimidin-3-yl)phenyl)piperazin-1-yl)-4,13-dioxo-6,9-dioxa-3,12-diazatetradecanoyl)-4-hydroxy-*N-*(4-(4-methylthiazol-5-yl)benzyl)pyrrolidine-2-carboxamide |
| 237 | HC90-122 | 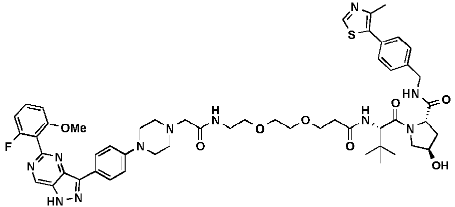 | (2*S*,4*R*)-1-((*S*)-14-(*tert*-butyl)-1-(4-(4-(5-(2-fluoro-6-methoxyphenyl)-1*H*-pyrazolo[4,3-*d*]pyrimidin-3-yl)phenyl)piperazin-1-yl)-2,12-dioxo-6,9-dioxa-3,13-diazapentadecan-15-oyl)-4-hydroxy-*N*-(4-(4-methylthiazol-5-yl)benzyl)pyrrolidine-2-carboxamide |
| 238 | HC90-123 | 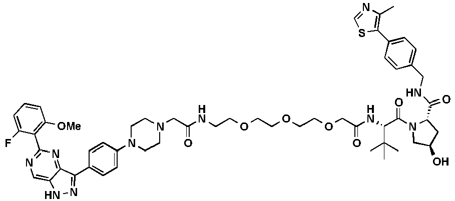 | (2*S*,4*R*)-1-((*S*)-2-(*tert*-butyl)-17-(4-(4-(5-(2-fluoro-6-methoxyphenyl)-1*H*-pyrazolo[4,3-*d*]pyrimidin-3-yl)phenyl)piperazin-1-yl)-4,16-dioxo-6,9,12-trioxa-3,15-diazaheptadecanoyl)-4-hydroxy-*N*-(4-(4-methylthiazol-5-yl)benzyl)pyrrolidine-2-carboxamide |
| 239 | HC90-124 | 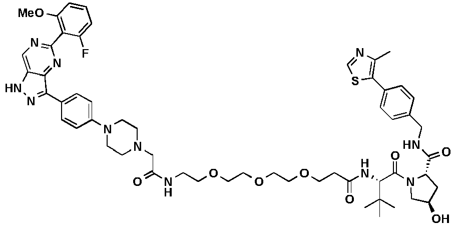 | (2*S*,4*R*)-1-((*S*)-17-(*tert*-butyl)-1-(4-(4-(5-(2-fluoro-6-methoxyphenyl)-1*H*-pyrazolo[4,3-*d*]pyrimidin-3-yl)phenyl)piperazin-1-yl)-2,15-dioxo-6,9,12-trioxa-3,16-diazaoctadecan-18-oyl)-4-hydroxy-*N*-(4-(4-methylthiazol-5-yl)benzyl)pyrrolidine-2-carboxamide |
| 240 | HC90-125 | 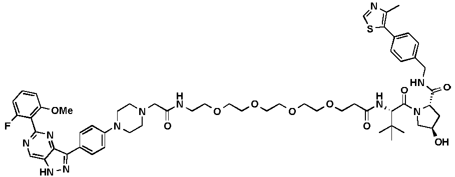 | (2*S*,4*R*)-1-((*S*)-20-(*tert*-butyl)-1-(4-(4-(5-(2-fluoro-6-methoxyphenyl)-1*H*-pyrazolo[4,3-*d*]pyrimidin-3-yl)phenyl)piperazin-1-yl)-2,18-dioxo-6,9,12,15-tetraoxa-3,19-diazahenicosan-21-oyl)-4-hydroxy-*N*-(4-(4-methylthiazol-5-yl)benzyl)pyrrolidine-2-carboxamide |
| 241 | HC90-126 | 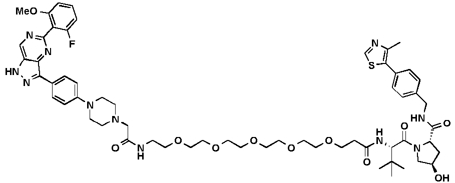 | (2*S*,4*R*)-1-((*S*)-23-(*tert*-butyl)-1-(4-(4-(5-(2-fluoro-6-methoxyphenyl)-1*H*-pyrazolo[4,3-*d*]pyrimidin-3-yl)phenyl)piperazin-1-yl)-2,21-dioxo-6,9,12,15,18-pentaoxa-3,22-diazatetracosan-24-oyl)-4-hydroxy-*N*-(4-(4-methylthiazol-5-yl)benzyl)pyrrolidine-2-carboxamide |
| 242 | HC90-127 | 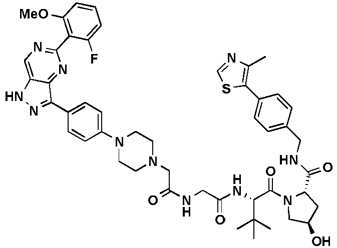 | (2*S*,4*R*)-1-((*S*)-2-(2-(2-(4-(4-(5-(2-fluoro-6-methoxyphenyl)-1*H-*pyrazolo[4,3-*d*]pyrimidin-3-yl)phenyl)piperazin-1-yl)acetamido)acetamido)-3,3-dimethylbutanoyl)-4-hydroxy-*N-*(4-(4-methylthiazol-5-yl)benzyl)pyrrolidine-2-carboxamide |
| 243 | HC90-128 | 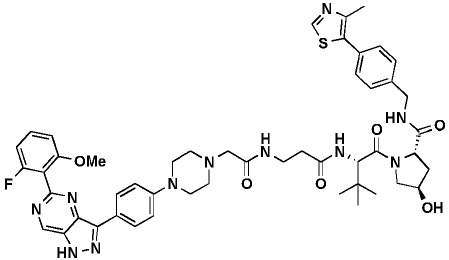 | (2*S*,4*R*)-1-((*S*)-2-(3-(2-(4-(4-(5-(2-fluoro-6-methoxyphenyl)-1*H-*pyrazolo[4,3-*d*]pyrimidin-3-yl)phenyl)piperazin-1-yl)acetamido)propanamido)-3,3-dimethylbutanoyl)-4-hydroxy-*N-*(4-(4-methylthiazol-5-yl)benzyl)pyrrolidine-2-carboxamide |
| 244 | HC90-129 | 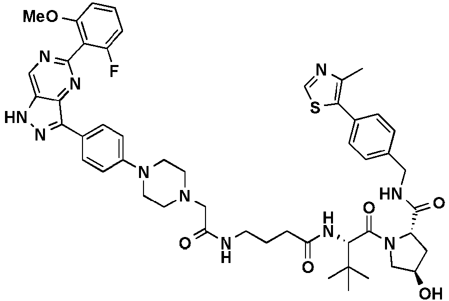 | (2*S*,4*R*)-1-((*S*)-2-(4-(2-(4-(4-(5-(2-fluoro-6-methoxyphenyl)-1*H-*pyrazolo[4,3-*d*]pyrimidin-3-yl)phenyl)piperazin-1-yl)acetamido)butanamido)-3,3-dimethylbutanoyl)-4-hydroxy-*N-*(4-(4-methylthiazol-5-yl)benzyl)pyrrolidine-2-carboxamide |
| 245 | HC90-130 | 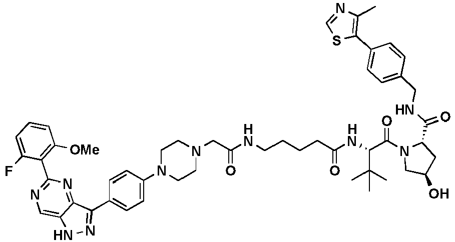 | (2*S*,4*R*)-1-((*S*)-2-(5-(2-(4-(4-(5-(2-fluoro-6-methoxyphenyl)-1*H-*pyrazolo[4,3-*d*]pyrimidin-3-yl)phenyl)piperazin-1-yl)acetamido)pentanamido)-3,3-dimethylbutanoyl)-4-hydroxy-*N-*(4-(4-methylthiazol-5-yl)benzyl)pyrrolidine-2-carboxamide |
| 246 | HC90-131 | 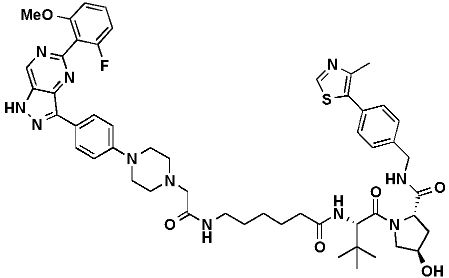 | (2*S*,4*R*)-1-((*S*)-2-(6-(2-(4-(4-(5-(2-fluoro-6-methoxyphenyl)-1*H-*pyrazolo[4,3-*d*]pyrimidin-3-yl)phenyl)piperazin-1-yl)acetamido)hexanamido)-3,3-dimethylbutanoyl)-4-hydroxy-*N-*(4-(4-methylthiazol-5-yl)benzyl)pyrrolidine-2-carboxamide |
| 247 | HC90-132 | 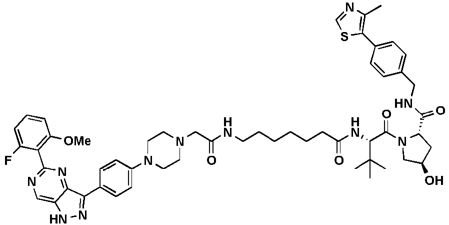 | (2*S*,4*R*)-1-((*S*)-2-(7-(2-(4-(4-(5-(2-fluoro-6-methoxyphenyl)-1*H-*pyrazolo[4,3-*d*]pyrimidin-3-yl)phenyl)piperazin-1-yl)acetamido)heptanamido)-3,3-dimethylbutanoyl)-4-hydroxy-*N-*(4-(4-methylthiazol-5-yl)benzyl)pyrrolidine-2-carboxamide |
| 248 | HC90-133 | 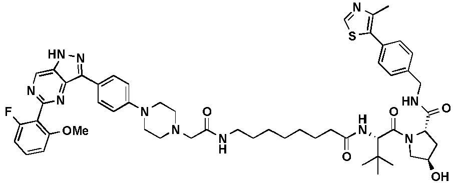 | (2*S*,4*R*)-1-((*S*)-2-(8-(2-(4-(4-(5-(2-fluoro-6-methoxyphenyl)-1*H-*pyrazolo[4,3-*d*]pyrimidin-3-yl)phenyl)piperazin-1-yl)acetamido)octanamido)-3,3-dimethylbutanoyl)-4-hydroxy-*N-*(4-(4-methylthiazol-5-yl)benzyl)pyrrolidine-2-carboxamide |
| 249 | HC90-134 | 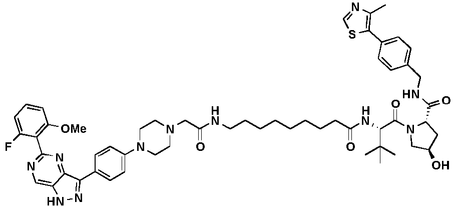 | (2*S*,4*R*)-1-((*S*)-2-(9-(2-(4-(4-(5-(2-fluoro-6-methoxyphenyl)-1*H-*pyrazolo[4,3-*d*]pyrimidin-3-yl)phenyl)piperazin-1-yl)acetamido)nonanamido)-3,3-dimethylbutanoyl)-4-hydroxy-*N-*(4-(4-methylthiazol-5-yl)benzyl)pyrrolidine-2-carboxamide |
| 250 | HC90-135 | 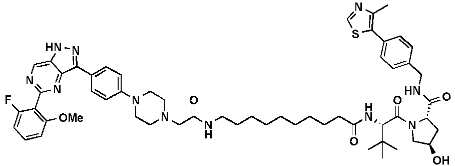 | (2*S*,4*R*)-1-((*S*)-2-(10-(2-(4-(4-(5-(2-fluoro-6-methoxyphenyl)-1*H-*pyrazolo[4,3-*d*]pyrimidin-3-yl)phenyl)piperazin-1-yl)acetamido)decanamido)-3,3-dimethylbutanoyl)-4-hydroxy-*N-*(4-(4-methylthiazol-5-yl)benzyl)pyrrolidine-2-carboxamide |
| 251 | HC90-136 | 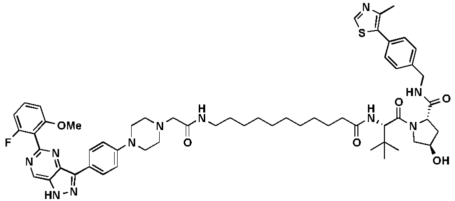 | (2*S*,4*R*)-1-((*S*)-2-(11-(2-(4-(4-(5-(2-fluoro-6-methoxyphenyl)-1*H-*pyrazolo[4,3-*d*]pyrimidin-3-yl)phenyl)piperazin-1-yl)acetamido)undecanamido)-3,3-dimethylbutanoyl)-4-hydroxy-*N*-(4-(4-methylthiazol-5-yl)benzyl)pyrrolidine-2-carboxamide |
| 252 | HC90-60 | 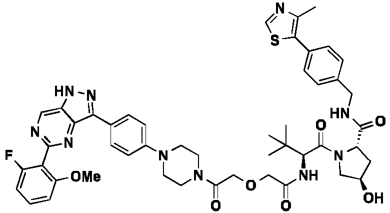 | (2*S*,4*R*)-1-((*S*)-2-(2-(2-(4-(4-(5-(2-fluoro-6-methoxyphenyl)-1*H-*pyrazolo[4,3-*d*]pyrimidin-3-yl)phenyl)piperazin-1-yl)-2-oxoethoxy)acetamido)-3,3-dimethylbutanoyl)-4-hydroxy-*N-*(4-(4-methylthiazol-5-yl)benzyl)pyrrolidine-2-carboxamide |
| 253 | HC90-62 | 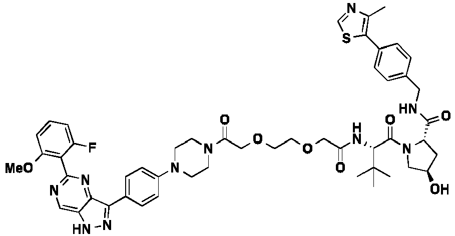 | (2*S*,4*R*)-1-((*S*)-2-(2-(2-(2-(4-(4-(5-(2-fluoro-6-methoxyphenyl)-1*H-*pyrazolo[4,3-*d*]pyrimidin-3-yl)phenyl)piperazin-1-yl)-2-oxoethoxy)ethoxy)acetamido)-3,3-dimethylbutanoyl)-4-hydroxy-*N*-(4-(4-methylthiazol-5-yl)benzyl)pyrrolidine-2-carboxamide |
| 254 | HC90-63 | 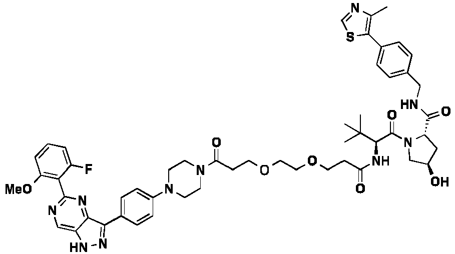 | (2*S*,4*R*)-1-((*S*)-2-(3-(2-(3-(4-(4-(5-(2-fluoro-6-methoxyphenyl)-1*H-*pyrazolo[4,3-*d*]pyrimidin-3-yl)phenyl)piperazin-1-yl)-3-oxopropoxy)ethoxy)propanamido )-3,3-dimethylbutanoyl)-4-hydroxy-*N*-(4-(4-methylthiazol-5-yl)benzyl)pyrrolidine-2-carboxamide |
| 255 | HC90-64 | 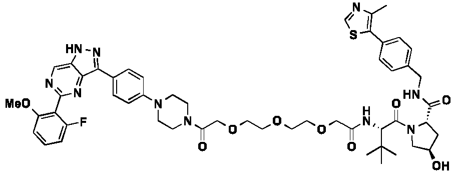 | (2*S*,4*R*)-1-((*S*)-2-(*tert*-butyl)-14-(4-(4-(5-(2-fluoro-6-methoxyphenyl)-1*H*-pyrazolo[4,3-*d*]pyrimidin-3-yl)phenyl)piperazin-1-yl)-4,14-dioxo-6,9,12-trioxa-3-azatetradecanoyl)-4-hydroxy-*N-*(4-(4-methylthiazol-5-yl)benzyl)pyrrolidine-2-carboxamide |
| 256 | HC90-65 | 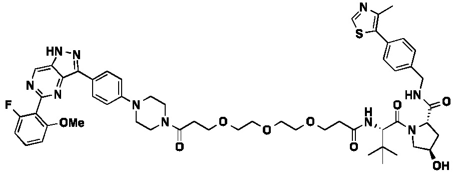 | (2*S*,4*R*)-1-((*S*)-2-(*tert*-butyl)-16-(4-(4-(5-(2-fluoro-6-methoxyphenyl)-1*H*-pyrazolo[4,3-*d*]pyrimidin-3-yl)phenyl)piperazin-1-yl)-4,16-dioxo-7,10,13-trioxa-3-azahexadecanoyl)-4-hydroxy-*N-*(4-(4-methylthiazol-5-yl)benzyl)pyrrolidine-2-carboxamide |
| 257 | HC90-67 | 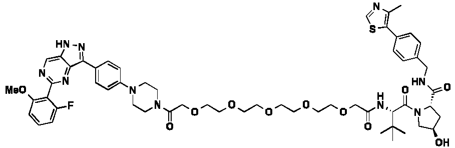 | (2*S*,4*R*)-1-((*S*)-2-(*tert*-butyl)-20-(4-(4-(5-(2-fluoro-6-methoxyphenyl)-1*H*-pyrazolo[4,3-*d*]pyrimidin-3-yl)phenyl)piperazin-1-yl)-4,20-dioxo-6,9,12,15,18-pentaoxa-3-azaicosanoyl)-4-hydroxy-*N*-(4-(4-methylthiazol-5-yl)benzyl)pyrrolidine-2-carboxamide |
| 258 | HC90-68 | 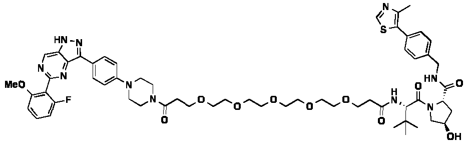 | (2*S*,4*R*)-1-((*S*)-2-(*tert*-butyl)-22-(4-(4-(5-(2-fluoro-6-methoxyphenyl)-1*H*-pyrazolo[4,3-*d*]pyrimidin-3-yl)phenyl)piperazin-1-yl)-4,22-dioxo-7,10,13,16,19-pentaoxa-3-azadocosanoyl)-4-hydroxy-*N*-(4-(4-methylthiazol-5-yl)benzyl)pyrrolidine-2-carboxamide |
| 260 | HC90-114 | 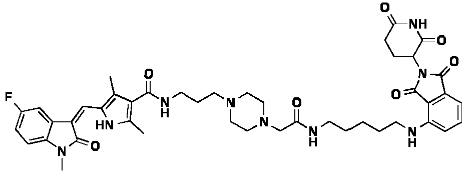 | (*Z*)-*N*-(3-(4-(2-((5-((2-(2,6-dioxopiperidin-3-yl)-1,3-dioxoisoindolin-4-yl)amino)pentyl)amino)-2-oxoethyl)piperazin-1-yl)propyl)-5-((5-fluoro-1-methyl-2-oxoindolin-3-ylidene)methyl)-2,4-dimethyl-1*H*-pyrrole-3-carboxamide |
| 261 | HC90-115 | 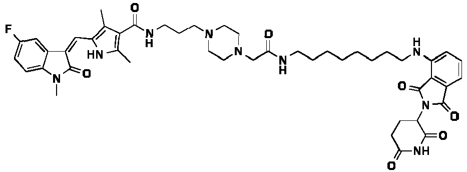 | (*Z*)-*N*-(3-(4-(2-((8-((2-(2,6-dioxopiperidin-3-yl)-1,3-dioxoisoindolin-4-yl)amino)octyl)amino)-2-oxoethyl)piperazin-1-yl)propyl)-5-((5-fluoro-1-methyl-2-oxoindolin-3-ylidene)methyl)-2,4-dimethyl-1*H*-pyrrole-3-carboxamide |
| 263 | HC90-168 | 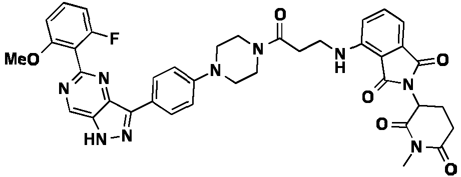 | 4-((3-(4-(4-(5-(2-fluoro-6-methoxyphenyl)-1*H*-pyrazolo[4,3-*d*]pyrimidin-3-yl)phenyl)piperazin-1-yl)-3-oxopropyl)amino)-2-(1-methyl-2,6-dioxopiperidin-3-yl)isoindoline-1,3-dione |
| 264 | HC90-169 | 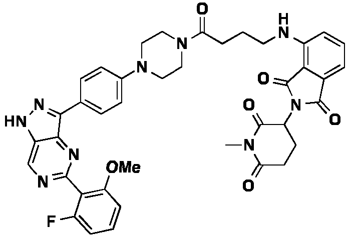 | 4-((4-(4-(4-(5-(2-fluoro-6-methoxyphenyl)-1*H*-pyrazolo[4,3-*d*]pyrimidin-3-yl)phenyl)piperazin-1-yl)-4-oxobutyl)amino)-2-(1-methyl-2,6-dioxopiperidin-3-yl)isoindoline-1,3-dione |

Compounds shown in Table 2 that do not fall within the scope of the claims are not part of the invention.

**Table 2.**

| Examples | Structure | Chemical Name |
|---|---|---|
| 265 | 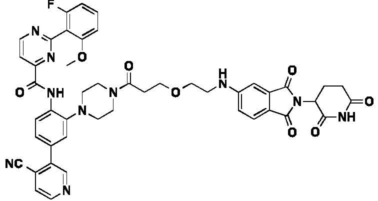 | *N*-(4-(4-cyanopyridin-3-yl)-2-(4-(3-(2-((2-(2,6-dioxopiperidin-3-yl)-1,3-dioxoisoindolin-5-yl)amino)ethoxy)propanoyl)pi perazin-1-yl)phenyl)-2-(2-fluoro-6-methoxyphenyl)pyrimidine-4-carboxamide |
| 266 | 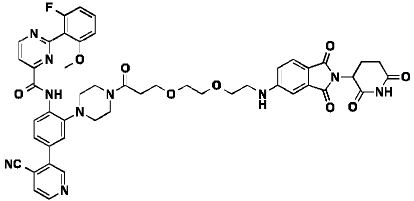 | *N*-(4-(4-cyanopyridin-3-yl)-2-(4-(3-(2-(2-((2-(2,6-dioxopiperidin-3-yl)-1,3-dioxoisoindolin-5-yl)amino)ethoxy)ethoxy)prop anoyl)piperazin-1-yl)phenyl)-2-(2-fluoro-6-methoxyphenyl)pyrimidine-4-carboxamide |
| 267 | 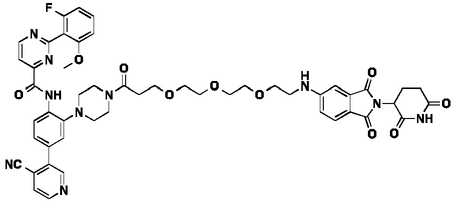 | *N*-(4-(4-cyanopyridin-3-yl)-2-(4-(3-(2-(2-(2-((2-(2,6-dioxopiperidin-3-yl)-1,3-dioxoisoindolin-5-yl)amino)ethoxy)ethoxy)etho xy)propanoyl)piperazin-1-yl)phenyl)-2-(2-fluoro-6-methoxyphenyl)pyrimidine-4-carboxamide |
| 268 | 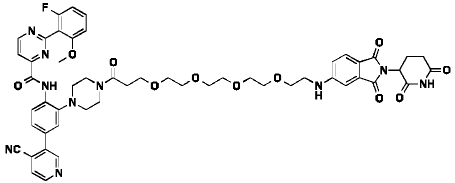 | *N*-(4-(4-cyanopyridin-3-yl)-2-(4-(1-((2-(2,6-dioxopiperidin-3-yl)-1,3-dioxoisoindolin-5-yl)amino)-3,6,9,12-tetraoxa pentadecan-15-oyl)piperazin-1-yl)phenyl)-2-(2-fluoro-6-methoxyphenyl)pyrimidine-4-carboxamide |
| 269 | 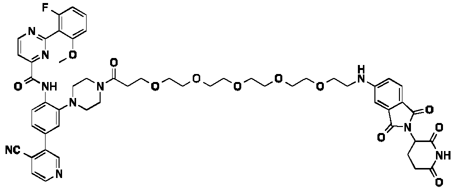 | *N*-(4-(4-cyanopyridin-3-yl)-2-(4-(1-((2-(2,6-dioxopiperidin-3-yl)-1,3-dioxoisoindolin-5-yl)amino)-3,6,9,12,15-pentaoxaoctadecan-18-oyl)piperazin-1-yl)phenyl)-2-(2-fluoro-6-methoxyphenyl)pyrimidine-4-carboxamide |
| 270 | 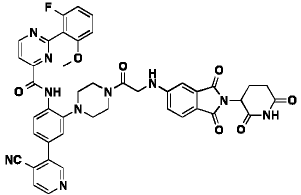 | *N*-(4-(4-cyanopyridin-3-yl)-2-(4-((2-(2,6-dioxopiperidin-3-yl)-1,3-dioxoisoindolin-5-yl)glycyl)piperazin-1-yl)phenyl)-2-(2-fluoro-6-methoxyphenyl)pyrimidine-4-carboxamide |
| 271 | 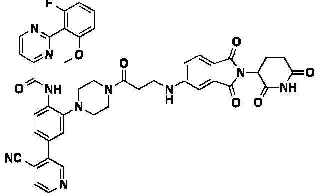 | *N*-(4-(4-cyanopyridin-3-yl)-2-(4-(3-((2-(2,6-dioxopiperidin-3-yl)-1,3-dioxoisoindolin-5-yl)amino)propanoyl)piperazin -1-yl)phenyl)-2-(2-fluoro-6-methoxyphenyl)pyrimidine-4-carboxamide |
| 272 | 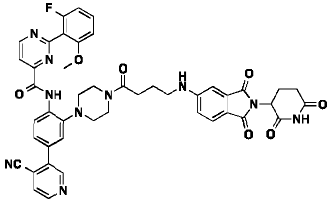 | *N*-(4-(4-cyanopyridin-3-yl)-2-(4-(4-((2-(2,6-dioxopiperidin-3-yl)-1,3-dioxoisoindolin-5-yl)amino)butanoyl)piperazin-1-yl)phenyl)-2-(2-fluoro-6-methoxyphenyl)pyrimidine-4-carboxamide |
| 273 | 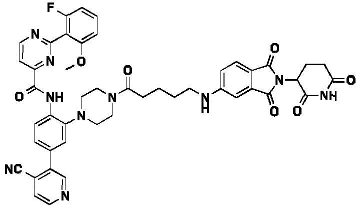 | *N*-(4-(4-cyanopyridin-3-yl)-2-(4-(5-((2-(2,6-dioxopiperidin-3-yl)-1,3-dioxoisoindolin-5-yl)amino)pentanoyl)piperazin -1-yl)phenyl)-2-(2-fluoro-6-methoxyphenyl)pyrimidine-4-carboxamide |
| 274 | 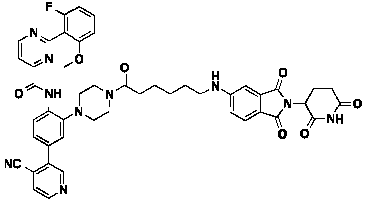 | *N*-(4-(4-cyanopyridin-3-yl)-2-(4-(6-((2-(2,6-dioxopiperidin-3-yl)-1,3-dioxoisoindolin-5-yl)amino)hexanoyl)piperazin-1-yl)phenyl)-2-(2-fluoro-6-methoxyphenyl)pyrimidine-4-carboxamide |
| 275 | 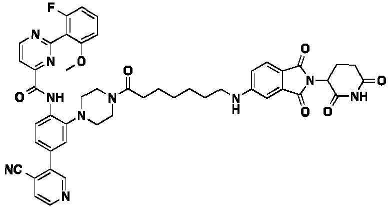 | *N*-(4-(4-cyanopyridin-3-yl)-2-(4-(7-((2-(2,6-dioxopiperidin-3-yl)-1,3-dioxoisoindolin-5-yl)amino)heptanoyl)piperazin -1-yl)phenyl)-2-(2-fluoro-6-methoxyphenyl)pyrimidine-4-carboxamide |
| 276 | 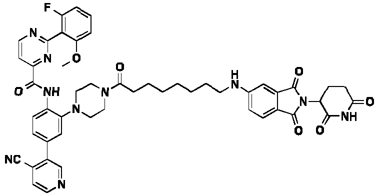 | *N*-(4-(4-cyanopyridin-3-yl)-2-(4-(8-((2-(2,6-dioxopiperidin-3-yl)-1,3-dioxoisoindolin-5-yl)amino)octanoyl)piperazin-1-yl)phenyl)-2-(2-fluoro-6-methoxyphenyl)pyrimidine-4-carboxamide |
| 277 | 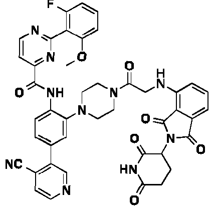 | *N*-(4-(4-cyanopyridin-3-yl)-2-(4-((2-(2,6-dioxopiperidin-3-yl)-1,3-dioxoisoindolin-4-yl)glycyl)piperazin-1-yl)phenyl)-2-(2-fluoro-6-methoxyphenyl)pyrimidine-4-carboxamide |
| 278 | 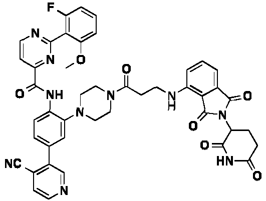 | *N*-(4-(4-cyanopyridin-3-yl)-2-(4-(3-((2-(2,6-dioxopiperidin-3-yl)-1,3-dioxoisoindolin-4-yl)amino)propanoyl)piperazin -1-yl)phenyl)-2-(2-fluoro-6-methoxyphenyl)pyrimidine-4-carboxamide |
| 279 | 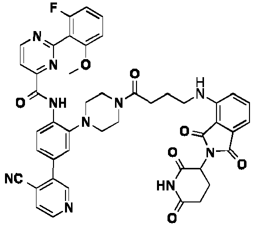 | *N*-(4-(4-cyanopyridin-3-yl)-2-(4-(4-((2-(2,6-dioxopiperidin-3-yl)-1,3-dioxoisoindolin-4-yl)amino)butanoyl)piperazin-1-yl)phenyl)-2-(2-fluoro-6-methoxyphenyl)pyrimidine-4-carboxamide |
| 280 | 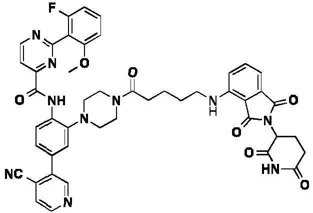 | *N*-(4-(4-cyanopyridin-3-yl)-2-(4-(5-((2-(2,6-dioxopiperidin-3-yl)-1,3-dioxoisoindolin-4-yl)amino)pentanoyl)piperazin -1-yl)phenyl)-2-(2-fluoro-6-methoxyphenyl)pyrimidine-4-carboxamide |
| 281 | 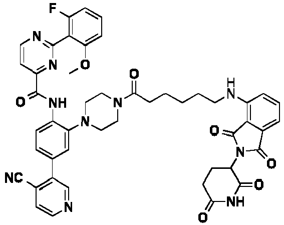 | *N*-(4-(4-cyanopyridin-3-yl)-2-(4-(6-((2-(2,6-dioxopiperidin-3-yl)-1,3-dioxoisoindolin-4-yl)amino)hexanoyl)piperazin-1-yl)phenyl)-2-(2-fluoro-6-methoxyphenyl)pyrimidine-4-carboxamide |
| 282 | 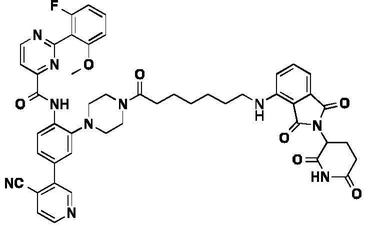 | *N*-(4-(4-cyanopyridin-3-yl)-2-(4-(7-((2-(2,6-dioxopiperidin-3-yl)-1,3-dioxoisoindolin-4-yl)amino)heptanoyl)piperazin -1-yl)phenyl)-2-(2-fluoro-6-methoxyphenyl)pyrimidine-4-carboxamide |
| 283 | 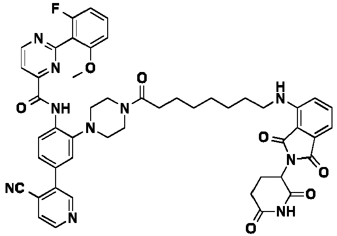 | *N*-(4-(4-cyanopyridin-3-yl)-2-(4-(8-((2-(2,6-dioxopiperidin-3-yl)-1,3-dioxoisoindolin-4-yl)amino)octanoyl)piperazin-1-yl)phenyl)-2-(2-fluoro-6-methoxyphenyl)pyrimidine-4-carboxamide |
| 284 | 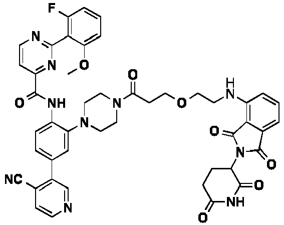 | *N*-(4-(4-cyanopyridin-3-yl)-2-(4-(3-(2-((2-(2,6-dioxopiperidin-3-yl)-1,3-dioxoisoindolin-4-yl)amino)ethoxy)propanoyl)pi perazin-1-yl)phenyl)-2-(2-fluoro-6-methoxyphenyl)pyrimidine-4-carboxamide |
| 285 | 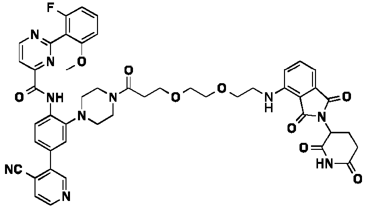 | *N*-(4-(4-cyanopyridin-3-yl)-2-(4-(3-(2-(2-((2-(2,6-dioxopiperidin-3-yl)-1,3-dioxoisoindolin-4-yl)amino)ethoxy)ethoxy)prop anoyl)piperazin-1-yl)phenyl)-2-(2-fluoro-6-methoxyphenyl)pyrimidine-4-carboxamide |
| 286 | 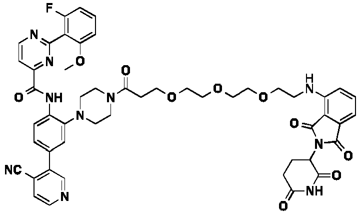 | *N*-(4-(4-cyanopyridin-3-yl)-2-(4-(3-(2-(2-(2-((2-(2,6-dioxopiperidin-3-yl)-1,3-dioxoisoindolin-4-yl)amino)ethoxy)ethoxy)etho xy)propanoyl)piperazin-1-yl)phenyl)-2-(2-fluoro-6-methoxyphenyl)pyrimidine-4-carboxamide |
| 287 | 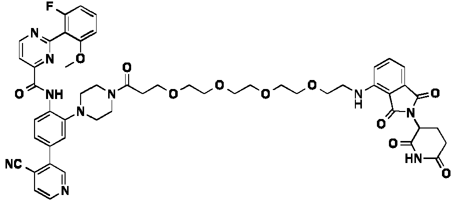 | *N*-(4-(4-cyanopyridin-3-yl)-2-(4-(1-((2-(2,6-dioxopiperidin-3-yl)-1,3-dioxoisoindolin-4-yl)amino)-3,6,9,12-tetraoxapentadecan-15-oyl)piperazin-1-yl)phenyl)-2-(2-fluoro-6-methoxyphenyl)pyrimidine-4-carboxamide |
| 288 | 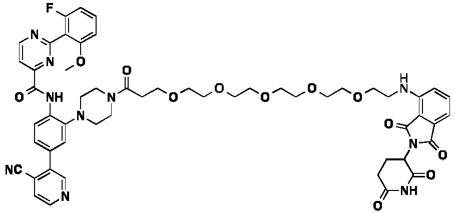 | *N*-(4-(4-cyanopyridin-3-yl)-2-(4-(1-((2-(2,6-dioxopiperidin-3-yl)-1,3-dioxoisoindolin-4-yl)amino)-3,6,9,12,15-pentaoxaoctadecan-18-oyl)piperazin-1-yl)phenyl)-2-(2-fluoro-6-methoxyphenyl)pyrimidine-4-carboxamide |
| 289 | 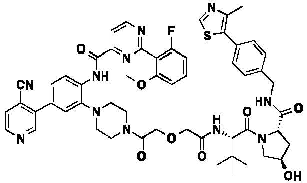 | *N*-(4-(4-cyanopyridin-3-yl)-2-(4-(2-(2-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-2-oxoethoxy)acetyl)piperazin-1-yl)phenyl)-2-(2-fluoro-6-methoxyphenyl)pyrimidine-4-carboxamide |
| 290 | 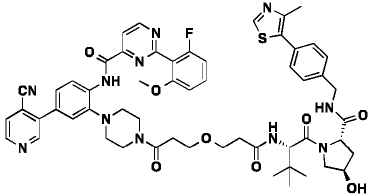 | *N*-(4-(4-cyanopyridin-3-yl)-2-(4-(3-(3-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-3-oxopropoxy)propanoyl)pipera zin-1-yl)phenyl)-2-(2-fluoro-6-methoxyphenyl)pyrimidine-4-carboxamide |
| 291 | 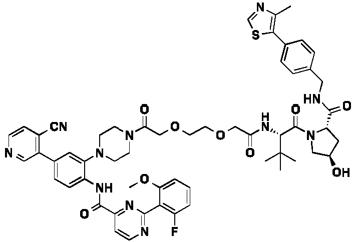 | *N*-(4-(4-cyanopyridin-3-yl)-2-(4-(2-(2-(2-(((S)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-2-oxoethoxy)ethoxy)acetyl)pipe razin-1-yl)phenyl)-2-(2-fluoro-6-methoxyphenyl)pyrimidine-4-carboxamide |
| 292 | 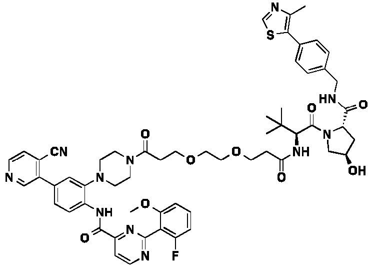 | *N*-(4-(4-cyanopyridin-3-yl)-2-(4-(3-(2-(3-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-3-oxopropoxy)ethoxy)propanoy l)piperazin-1-yl)phenyl)-2-(2-fluoro-6-methoxyphenyl)pyrimidine-4-carboxamide |
| 293 | 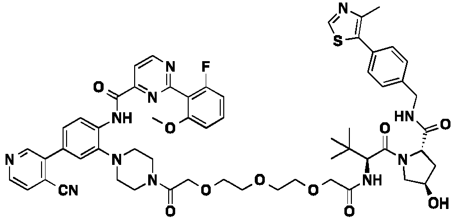 | *N*-(4-(4-cyanopyridin-3-yl)-2-(4-((*S*)-13-((2*S*,4*R*)-4-hydroxy-2-((4-(4-methylthiazol-5-yl)benzyl)carbamoyl)pyrrolidi ne-1-carbonyl)-14,14-dimethyl-11-oxo-3,6,9-trioxa-12-azapentadecanoyl)piperazin-1-yl)phenyl)-2-(2-fluoro-6-methoxyphenyl)pyrimidine-4-carboxamide |
| 294 | 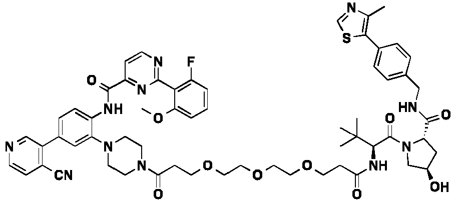 | *N*-(4-(4-cyanopyridin-3-yl)-2-(4-((*S*)-15-((2*S*,4*R*)-4-hydroxy-2-((4-(4-methylthiazol-5-yl)benzyl)carbamoyl)pyrrolidi ne-1-carbonyl)-16,16-dimethyl-13-oxo-4,7,10-trioxa-14-azaheptadecanoyl)piperazin-1-yl)phenyl)-2-(2-fluoro-6-methoxyphenyl)pyrimidine-4-carboxamide |
| 295 | 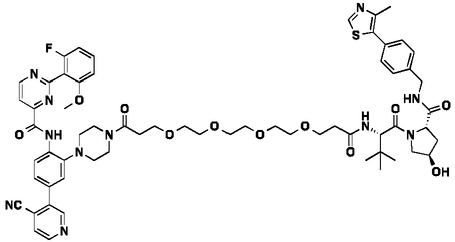 | *N*-(4-(4-cyanopyridin-3-yl)-2-(4-((*S*)-18-((2*S*,4*R*)-4-hydroxy-2-((4-(4-methylthiazol-5-yl)benzyl)carbamoyl)pyrrolidi ne-1-carbonyl)-19,19-dimethyl-16-oxo-4,7,10,13-tetraoxa-17-azaicosanoyl)piperazin-1-yl)phenyl)-2-(2-fluoro-6-methoxyphenyl)pyrimidine-4-carboxamide |
| 296 | 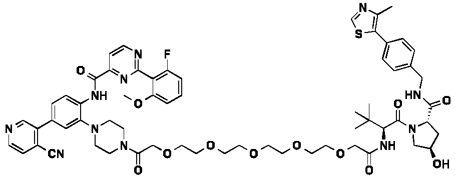 | *N*-(4-(4-cyanopyridin-3-yl)-2-(4-((*S*)-19-((2*S*,4*R*)-4-hydroxy-2-((4-(4-methylthiazol-5-yl)benzyl)carbamoyl)pyrrolidi ne-1-carbonyl)-20,20-dimethyl-17-oxo-3,6,9,12,15-pentaoxa-18-azahenicosanoyl)piperazin-1-yl)phenyl)-2-(2-fluoro-6-methoxyphenyl)pyrimidine-4-carboxamide |
| 297 | 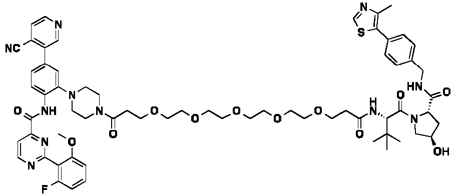 | *N*-(4-(4-cyanopyridin-3-yl)-2-(4-((*S*)-21-((2*S*,4*R*)-4-hydroxy-2-((4-(4-methylthiazol-5-yl)benzyl)carbamoyl)pyrrolidi ne-1-carbonyl)-22,22-dimethyl-19-oxo-4,7,10,13,16-pentaoxa-20-azatricosanoyl)piperazin-1-yl)phenyl)-2-(2-fluoro-6-methoxyphenyl)pyrimidine-4-carboxamide |
| 298 | 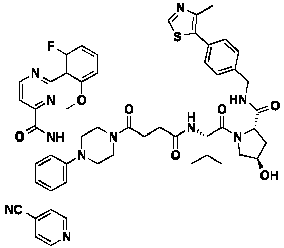 | *N*-(4-(4-cyanopyridin-3-yl)-2-(4-(4-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-4-oxobutanoyl)piperazin-1-yl)phenyl)-2-(2-fluoro-6-methoxyphenyl)pyrimidine-4-carboxamide |
| 299 | 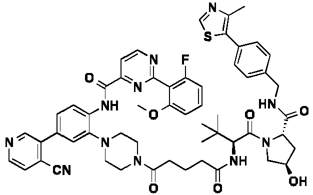 | *N*-(4-(4-cyanopyridin-3-yl)-2-(4-(5-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-5-oxopentanoyl)piperazin-1-yl)phenyl)-2-(2-fluoro-6-methoxyphenyl)pyrimidine-4-carboxamide |
| 300 | 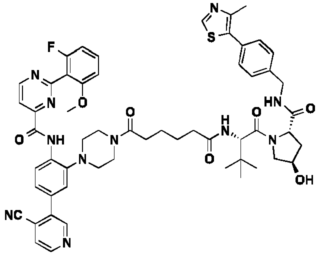 | *N*-(4-(4-cyanopyridin-3-yl)-2-(4-(6-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-6-oxohexanoyl)piperazin-1-yl)phenyl)-2-(2-fluoro-6-methoxyphenyl)pyrimidine-4-carboxamide |
| 301 | 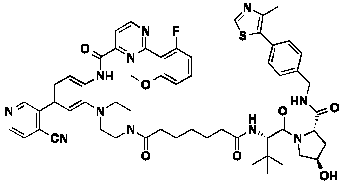 | *N*-(4-(4-cyanopyridin-3-yl)-2-(4-(7-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-7-oxoheptanoyl)piperazin-1-yl)phenyl)-2-(2-fluoro-6-methoxyphenyl)pyrimidine-4-carboxamide |
| 302 | 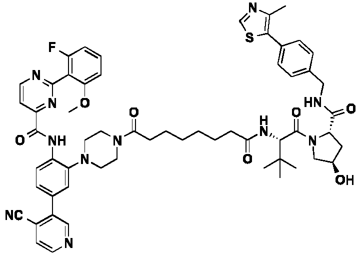 | *N*-(4-(4-cyanopyridin-3-yl)-2-(4-(8-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-8-oxooctanoyl)piperazin-1-yl)phenyl)-2-(2-fluoro-6-methoxyphenyl)pyrimidine-4-carboxamide |
| 303 | 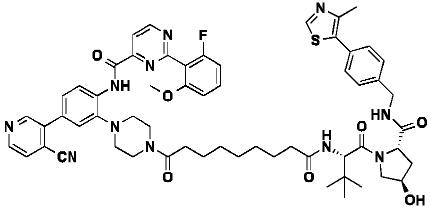 | *N*-(4-(4-cyanopyridin-3-yl)-2-(4-(9-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-9-oxononanoyl)piperazin-1-yl)phenyl)-2-(2-fluoro-6-methoxyphenyl)pyrimidine-4-carboxamide |
| 304 | 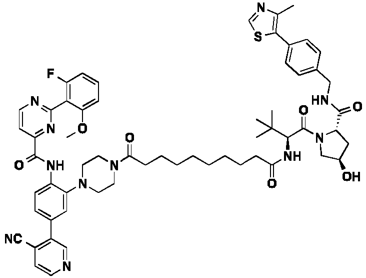 | *N*-(4-(4-cyanopyridin-3-yl)-2-(4-(10-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-10-oxodecanoyl)piperazin-1-yl)phenyl)-2-(2-fluoro-6-methoxyphenyl)pyrimidine-4-carboxamide |
| 305 | 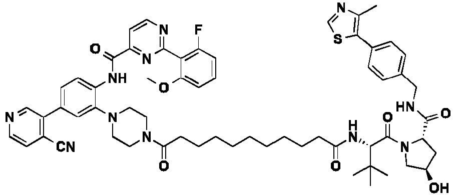 | *N*-(4-(4-cyanopyridin-3-yl)-2-(4-(11-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-11-oxoundecanoyl)piperazin-1-yl)phenyl)-2-(2-fluoro-6-methoxyphenyl)pyrimidine-4-carboxamide |
| 306 | 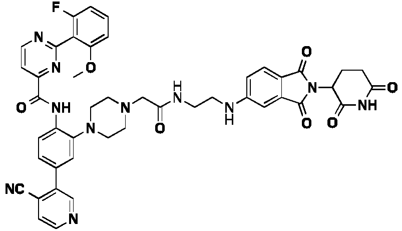 | *N*-(4-(4-cyanopyridin-3-yl)-2-(4-(2-((2-((2-(2,6-dioxopiperidin-3-yl)-1,3-dioxoisoindolin-5-yl)amino)ethyl)amino)-2-oxoethyl)piperazin-1-yl)phenyl)-2-(2-fluoro-6-methoxyphenyl)pyrimidine-4-carboxamide |
| 307 | 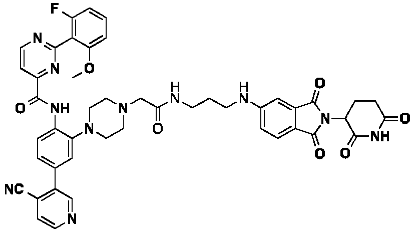 | *N*-(4-(4-cyanopyridin-3-yl)-2-(4-(2-((3-((2-(2,6-dioxopiperidin-3-yl)-1,3-dioxoisoindolin-5-yl)amino)propyl)amino)-2-oxoethyl)piperazin-1-yl)phenyl)-2-(2-fluoro-6-methoxyphenyl)pyrimidine-4-carboxamide |
| 308 | 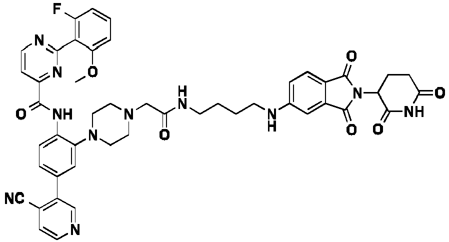 | *N*-(4-(4-cyanopyridin-3-yl)-2-(4-(2-((4-((2-(2,6-dioxopiperidin-3-yl)-1,3-dioxoisoindolin-5-yl)amino)butyl)amino)-2-oxoethyl)piperazin-1-yl)phenyl)-2-(2-fluoro-6-methoxyphenyl)pyrimidine-4-carboxamide |
| 309 | 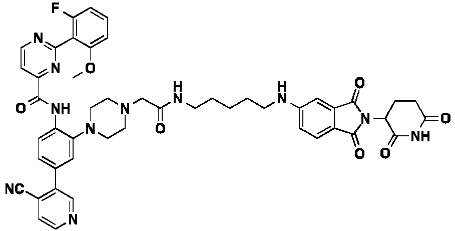 | *N*-(4-(4-cyanopyridin-3-yl)-2-(4-(2-((5-((2-(2,6-dioxopiperidin-3-yl)-1,3-dioxoisoindolin-5-yl)amino)pentyl)amino)-2-oxoethyl)piperazin-1-yl)phenyl)-2-(2-fluoro-6-methoxyphenyl)pyrimidine-4-carboxamide |
| 310 | 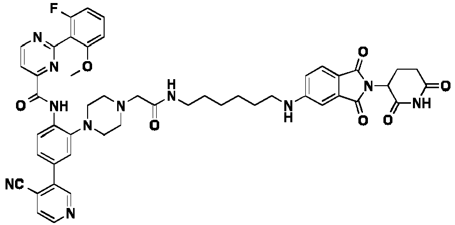 | *N*-(4-(4-cyanopyridin-3-yl)-2-(4-(2-((6-((2-(2,6-dioxopiperidin-3-yl)-1,3-dioxoisoindolin-5-yl)amino)hexyl)amino)-2-oxoethyl)piperazin-1-yl)phenyl)-2-(2-fluoro-6-methoxyphenyl)pyrimidine-4-carboxamide |
| 311 | 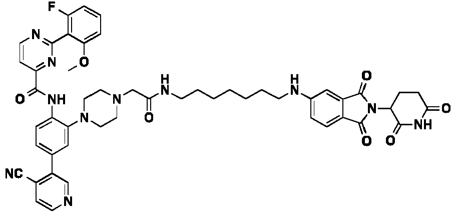 | *N*-(4-(4-cyanopyridin-3-yl)-2-(4-(2-((7-((2-(2,6-dioxopiperidin-3-yl)-1,3-dioxoisoindolin-5-yl)amino)heptyl)amino)-2-oxoethyl)piperazin-1-yl)phenyl)-2-(2-fluoro-6-methoxyphenyl)pyrimidine-4-carboxamide |
| 312 | 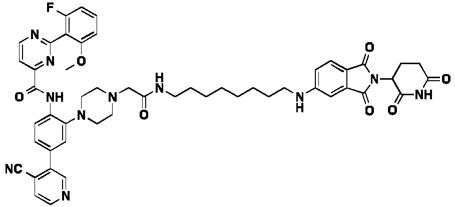 | *N*-(4-(4-cyanopyridin-3-yl)-2-(4-(2-((8-((2-(2,6-dioxopiperidin-3-yl)-1,3-dioxoisoindolin-5-yl)amino)octyl)amino)-2-oxoethyl)piperazin-1-yl)phenyl)-2-(2-fluoro-6-methoxyphenyl)pyrimidine-4-carboxamide |
| 313 | 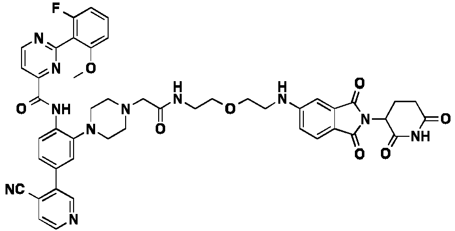 | *N*-(4-(4-cyanopyridin-3-yl)-2-(4-(2-((2-(2-((2-(2,6-dioxopiperidin-3-yl)-1,3-dioxoisoindolin-5-yl)amino)ethoxy)ethyl)amino) -2-oxoethyl)piperazin-1-yl)phenyl)-2-(2-fluoro-6-methoxyphenyl)pyrimidine-4-carboxamide |
| 314 | 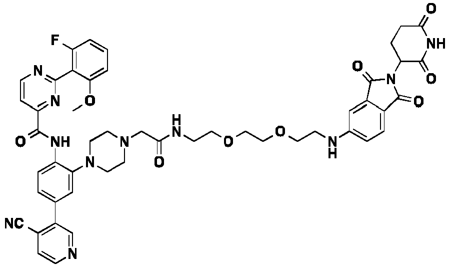 | *N*-(4-(4-cyanopyridin-3-yl)-2-(4-(2-((2-(2-(2-((2-(2,6-dioxopiperidin-3-yl)-1,3-dioxoisoindolin-5-yl)amino)ethoxy)ethoxy)ethyl )amino)-2-oxoethyl)piperazin-1-yl)phenyl)-2-(2-fluoro-6-methoxyphenyl)pyrimidine-4-carboxamide |
| 315 | 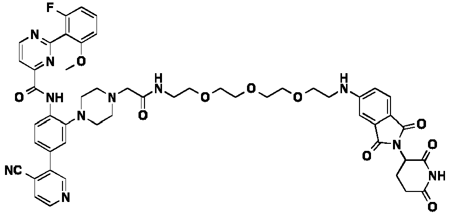 | *N*-(4-(4-cyanopyridin-3-yl)-2-(4-(14-((2-(2,6-dioxopiperidin-3-yl)-1,3-dioxoisoindolin-5-yl)amino)-2-oxo-6,9,12-trioxa-3-azatetradecyl)piperazin-1-yl)phenyl)-2-(2-fluoro-6-methoxyphenyl)pyrimidine-4-carboxamide |
| 316 | 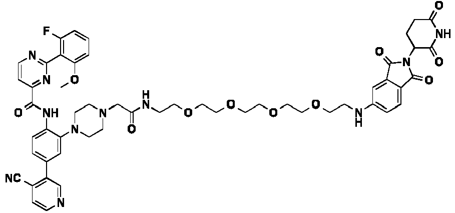 | *N*-(4-(4-cyanopyridin-3-yl)-2-(4-(17-((2-(2,6-dioxopiperidin-3-yl)-1,3-dioxoisoindolin-5-yl)amino)-2-oxo-6,9,12,15-tetraoxa-3-azaheptadecyl)piperazin-1-yl)phenyl)-2-(2-fluoro-6-methoxyphenyl)pyrimidine-4-carboxamide |
| 317 | 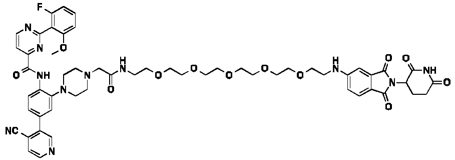 | *N*-(4-(4-cyanopyridin-3-yl)-2-(4-(20-((2-(2,6-dioxopiperidin-3-yl)-1,3-dioxoisoindolin-5-yl)amino)-2-oxo-6,9,12,15,18-pentaoxa-3-azaicosyl)piperazin-1-yl)phenyl)-2-(2-fluoro-6-methoxyphenyl)pyrimidine-4-carboxamide |
| 318 | 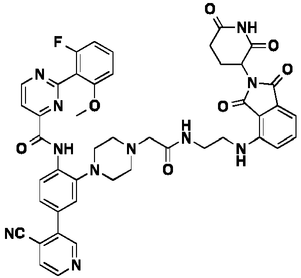 | *N*-(4-(4-cyanopyridin-3-yl)-2-(4-(2-((2-((2-(2,6-dioxopiperidin-3-yl)-1,3-dioxoisoindolin-4-yl)amino)ethyl)amino)-2-oxoethyl)piperazin-1-yl)phenyl)-2-(2-fluoro-6-methoxyphenyl)pyrimidine-4-carboxamide |
| 319 | 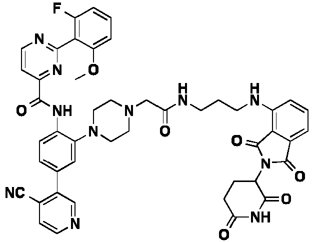 | *N*-(4-(4-cyanopyridin-3-yl)-2-(4-(2-((3-((2-(2,6-dioxopiperidin-3-yl)-1,3-dioxoisoindolin-4-yl)amino)propyl)amino)-2-oxoethyl)piperazin-1-yl)phenyl)-2-(2-fluoro-6-methoxyphenyl)pyrimidine-4-carboxamide |
| 320 | 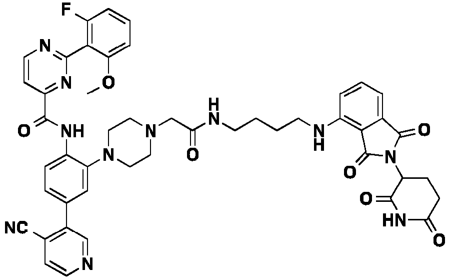 | *N*-(4-(4-cyanopyridin-3-yl)-2-(4-(2-((4-((2-(2,6-dioxopiperidin-3-yl)-1,3-dioxoisoindolin-4-yl)amino)butyl)amino)-2-oxoethyl)piperazin-1-yl)phenyl)-2-(2-fluoro-6-methoxyphenyl)pyrimidine-4-carboxamide |
| 321 | 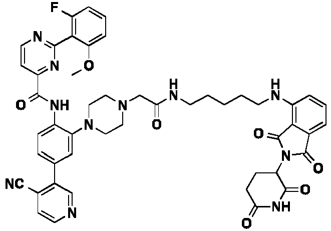 | *N*-(4-(4-cyanopyridin-3-yl)-2-(4-(2-((5-((2-(2,6-dioxopiperidin-3-yl)-1,3-dioxoisoindolin-4-yl)amino)pentyl)amino)-2-oxoethyl)piperazin-1-yl)phenyl)-2-(2-fluoro-6-methoxyphenyl)pyrimidine-4-carboxamide |
| 322 | 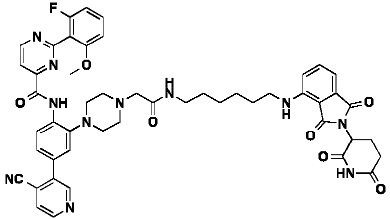 | *N*-(4-(4-cyanopyridin-3-yl)-2-(4-(2-((6-((2-(2,6-dioxopiperidin-3-yl)-1,3-dioxoisoindolin-4-yl)amino)hexyl)amino)-2-oxoethyl)piperazin-1-yl)phenyl)-2-(2-fluoro-6-methoxyphenyl)pyrimidine-4-carboxamide |
| 323 | 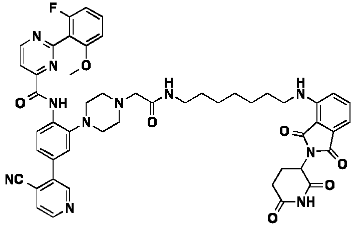 | *N*-(4-(4-cyanopyridin-3-yl)-2-(4-(2-((7-((2-(2,6-dioxopiperidin-3-yl)-1,3-dioxoisoindolin-4-yl)amino)heptyl)amino)-2-oxoethyl)piperazin-1-yl)phenyl)-2-(2-fluoro-6-methoxyphenyl)pyrimidine-4-carboxamide |
| 324 | 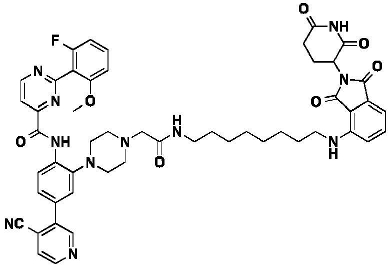 | *N*-(4-(4-cyanopyridin-3-yl)-2-(4-(2-((8-((2-(2,6-dioxopiperidin-3-yl)-1,3-dioxoisoindolin-4-yl)amino)octyl)amino)-2-oxoethyl)piperazin-1-yl)phenyl)-2-(2-fluoro-6-methoxyphenyl)pyrimidine-4-carboxamide |
| 325 | 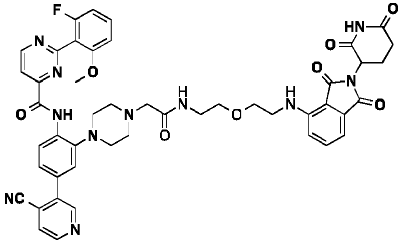 | *N*-(4-(4-cyanopyridin-3-yl)-2-(4-(2-((2-(2-((2-(2,6-dioxopiperidin-3-yl)-1,3-dioxoisoindolin-4-yl)amino)ethoxy)ethyl)amino) -2-oxoethyl)piperazin-1-yl)phenyl)-2-(2-fluoro-6-methoxyphenyl)pyrimidine-4-carboxamide |
| 326 | 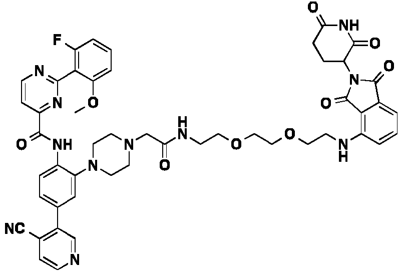 | *N*-(4-(4-cyanopyridin-3-yl)-2-(4-(2-((2-(2-(2-((2-(2,6-dioxopiperidin-3-yl)-1,3-dioxoisoindolin-4-yl)amino)ethoxy)ethoxy)ethyl )amino)-2-oxoethyl)piperazin-1-yl)phenyl)-2-(2-fluoro-6-methoxyphenyl)pyrimidine-4-carboxamide |
| 327 | 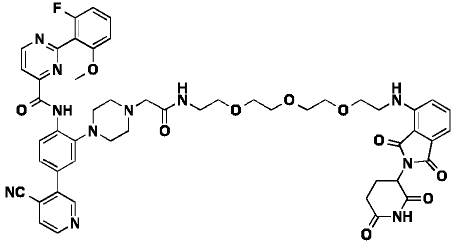 | *N*-(4-(4-cyanopyridin-3-yl)-2-(4-(14-((2-(2,6-dioxopiperidin-3-yl)-1,3-dioxoisoindolin-4-yl)amino)-2-oxo-6,9,12-trioxa-3-azatetradecyl)piperazin-1-yl)phenyl)-2-(2-fluoro-6-methoxyphenyl)pyrimidine-4-carboxamide |
| 328 | 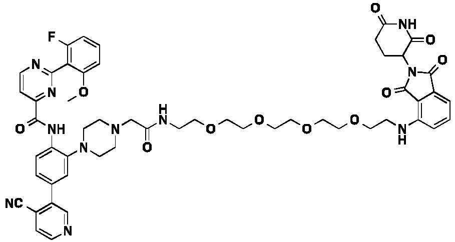 | *N*-(4-(4-cyanopyridin-3-yl)-2-(4-(17-((2-(2,6-dioxopiperidin-3-yl)-1,3-dioxoisoindolin-4-yl)amino)-2-oxo-6,9,12,15-tetraoxa-3-azaheptadecyl)piperazin-1-yl)phenyl)-2-(2-fluoro-6-methoxyphenyl)pyrimidine-4-carboxamide |
| 329 | 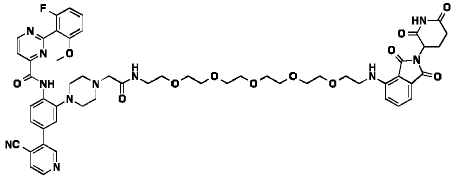 | *N*-(4-(4-cyanopyridin-3-yl)-2-(4-(20-((2-(2,6-dioxopiperidin-3-yl)-1,3-dioxoisoindolin-4-yl)amino)-2-oxo-6,9,12,15,18-pentaoxa-3-azaicosyl)piperazin-1-yl)phenyl)-2-(2-fluoro-6-methoxyphenyl)pyrimidine-4-carboxamide |
| 330 | 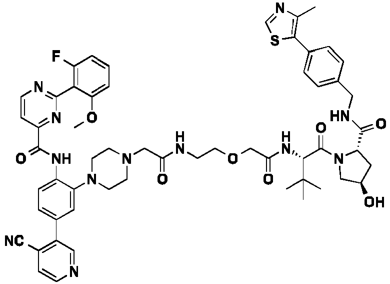 | *N*-(4-(4-cyanopyridin-3-yl)-2-(4-(2-((2-(2-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-2-oxoethoxy)ethyl)amino)-2-oxoethyl)piperazin-1-yl)phenyl)-2-(2-fluoro-6-methoxyphenyl)pyrimidine-4-carboxamide |
| 331 | 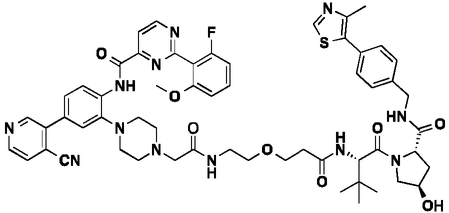 | *N*-(4-(4-cyanopyridin-3-yl)-2-(4-(2-((2-(3-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-3-oxopropoxy)ethyl)amino)-2-oxoethyl)piperazin-1-yl)phenyl)-2-(2-fluoro-6-methoxyphenyl)pyrimidine-4-carboxamide |
| 332 | 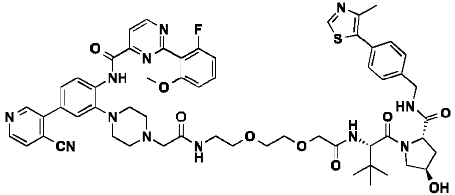 | *N*-(4-(4-cyanopyridin-3-yl)-2-(4-((*S*)-13-((2*S*,4*R*)-4-hydroxy-2-((4-(4-methylthiazol-5-yl)benzyl)carbamoyl)pyrrolidi ne-1-carbonyl)-14,14-dimethyl-2,11-dioxo-6,9-dioxa-3,12-diazapentadecyl)piperazin-1-yl)phenyl)-2-(2-fluoro-6-methoxyphenyl)pyrimidine-4-carboxamide |
| 333 | 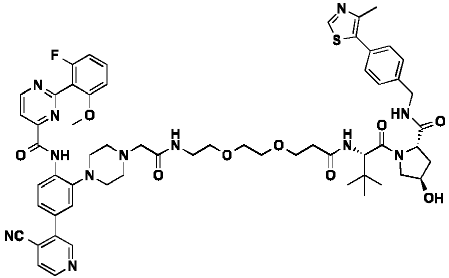 | *N*-(4-(4-cyanopyridin-3-yl)-2-(4-((*S*)-14-((2*S*,4*R*)-4-hydroxy-2-((4-(4-methylthiazol-5-yl)benzyl)carbamoyl)pyrrolidi ne-1-carbonyl)-15,15-dimethyl-2,12-dioxo-6,9-dioxa-3,13-diazahexadecyl)piperazin-1-yl)phenyl)-2-(2-fluoro-6-methoxyphenyl)pyrimidine-4-carboxamide |
| 334 | 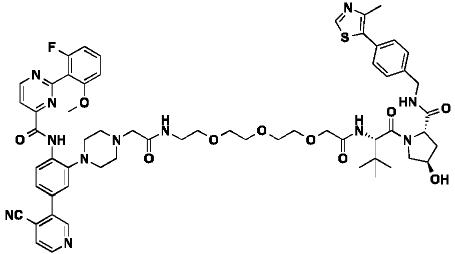 | *N-*(4-(4-cyanopyridin-3-yl)-2-(4-((*S*)-16-((2*S*,4*R*)-4-hydroxy-2-((4-(4-methylthiazol-5-yl)benzyl)carbamoyl)pyrrolidi ne-1-ca rbonyl)-17,17-dimethyl-2,14-dioxo-6,9,12-trioxa-3,15-diazaoctadecyl)piperazin-1-yl)phenyl)-2-(2-fluoro-6-methoxyphenyl)pyrimidine-4-carboxamide |
| 335 | 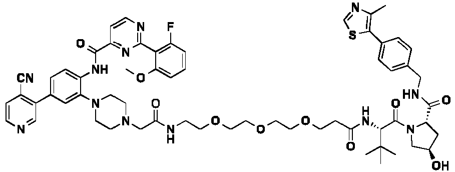 | *N*-(4-(4-cyanopyridin-3-yl)-2-(4-((*S*)-17-((2*S*,4*R*)-4-hydroxy-2-((4-(4-methylthiazol-5-yl)benzyl)carbamoyl)pyrrolidi ne-1-carbonyl)-18,18-dimethyl-2,15-dioxo-6,9,12-trioxa-3,16-diazanonadecyl)piperazin-1-yl)phenyl)-2-(2-fluoro-6-methoxyphenyl)pyrimidine-4-carboxamide |
| 336 | 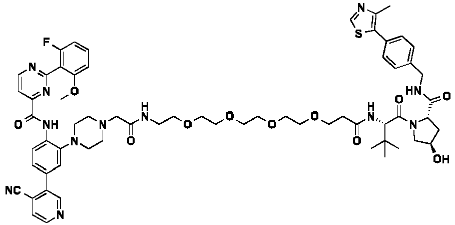 | *N*-(4-(4-cyanopyridin-3-yl)-2-(4-((*S*)-20-((2*S*,4*R*)-4-hydroxy-2-((4-(4-methylthiazol-5-yl)benzyl)carbamoyl)pyrrolidi ne-1-carbonyl)-21,21-dimethyl-2,18-dioxo-6,9,12,15-tetraoxa-3,19-diazadocosyl) piperazin-1-yl)phenyl)-2-(2-fluoro-6-methoxyphenyl)pyrimidine-4-carboxamide |
| 337 | 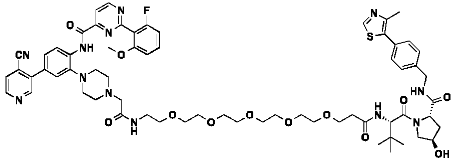 | *N*-(4-(4-cyanopyridin-3-yl)-2-(4-((*S*)-23-((2*S*,4*R*)-4-hydroxy-2-((4-(4-methylthiazol-5-yl)benzyl)carbamoyl)pyrrolidi ne-1-carbonyl)-24,24-dimethyl-2,21-dioxo-6,9,12,15,18-pentaoxa-3,22-diazapentacosyl)piperazin-1-yl)phenyl)-2-(2-fluoro-6-methoxyphenyl)pyrimidine-4-carboxamide |
| 338 | 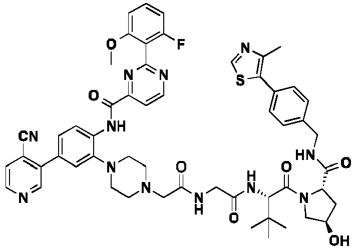 | *N*-(4-(4-cyanopyridin-3-yl)-2-(4-(2-((2-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-2-oxoethyl)amino)-2-oxoethyl)piperazin-1-yl)phenyl)-2-(2-fluoro-6-methoxyphenyl)pyrimidine-4-carboxamide |
| 339 | 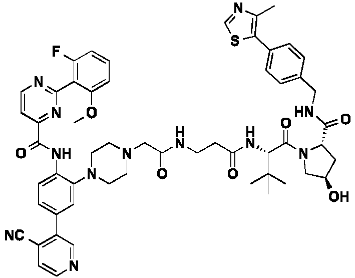 | *N*-(4-(4-cyanopyridin-3-yl)-2-(4-(2-((3-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-3-oxopropyl)amino)-2-oxoethyl)piperazin-1-yl)phenyl)-2-(2-fluoro-6-methoxyphenyl)pyrimidine-4-carboxamide |
| 340 | 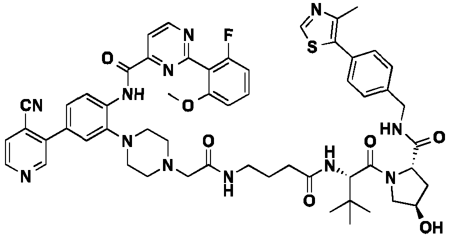 | *N*-(4-(4-cyanopyridin-3-yl)-2-(4-(2-((4-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-4-oxobutyl)amino)-2-oxoethyl)piperazin-1-yl)phenyl)-2-(2-fluoro-6-methoxyphenyl)pyrimidine-4-carboxamide |
| 341 | 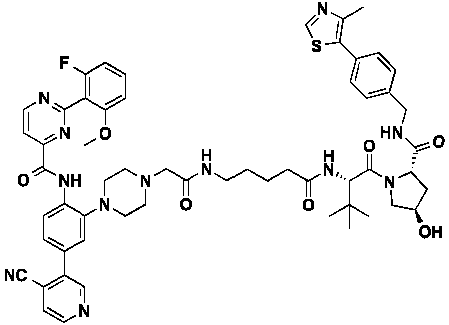 | *N*-(4-(4-cyanopyridin-3-yl)-2-(4-(2-((5-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-5-oxopentyl)amino)-2-oxoethyl)piperazin-1-yl)phenyl)-2-(2-fluoro-6-methoxyphenyl)pyrimidine-4-carboxamide |
| 342 | 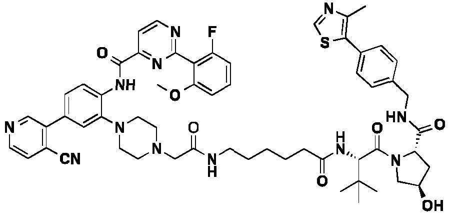 | *N*-(4-(4-cyanopyridin-3-yl)-2-(4-(2-((6-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-6-oxohexyl)amino)-2-oxoethyl)piperazin-1-yl)phenyl)-2-(2-fluoro-6-methoxyphenyl)pyrimidine-4-carboxamide |
| 343 | 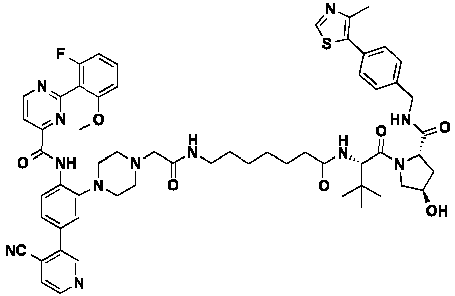 | *N*-(4-(4-cyanopyridin-3-yl)-2-(4-(2-((7-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-7-oxoheptyl)amino)-2-oxoethyl)piperazin-1-yl)phenyl)-2-(2-fluoro-6-methoxyphenyl)pyrimidine-4-carboxamide |
| 344 | 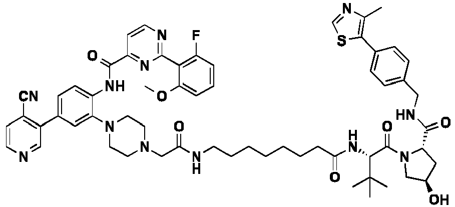 | *N*-(4-(4-cyanopyridin-3-yl)-2-(4-(2-((8-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-8-oxooctyl)amino)-2-oxoethyl)piperazin-1-yl)phenyl)-2-(2-fluoro-6-methoxyphenyl)pyrimidine-4-carboxamide |
| 345 | 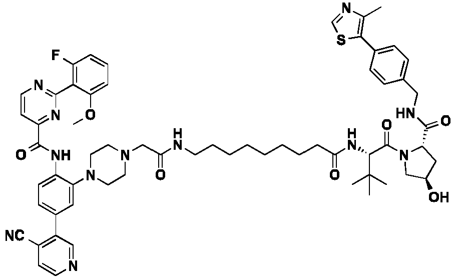 | *N*-(4-(4-cyanopyridin-3-yl)-2-(4-(2-((9-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-9-oxononyl)amino)-2-oxoethyl)piperazin-1-yl)phenyl)-2-(2-fluoro-6-methoxyphenyl)pyrimidine-4-carboxamide |
| 346 | 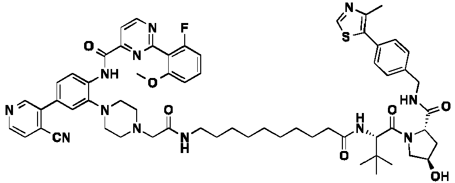 | *N*-(4-(4-cyanopyridin-3-yl)-2-(4-(2-((10-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-10-oxodecyl)amino)-2-oxoethyl)piperazin-1-yl)phenyl)-2-(2-fluoro-6-methoxyphenyl)pyrimidine-4-carboxamide |
| 347 | 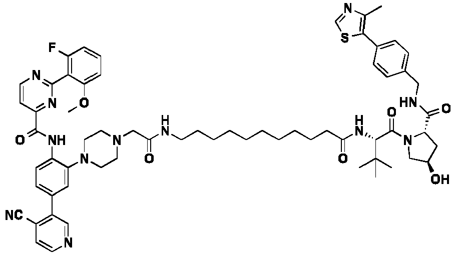 | *N*-(4-(4-cyanopyridin-3-yl)-2-(4-(2-((11-(((*S*)-1-((2*S*,4*R*)-4-hydroxy-2-((4-(4-methylthiazol-5-yl)benzyl)carbamoyl)pyrrolidi n-1-yl)-3,3-dimethyl-1-oxobutan-2-yl)amino)-11-oxoundecyl)amino)-2-oxoethyl)piperazin-1-yl)phenyl)-2-(2-fluoro-6-methoxyphenyl)pyrimidine-4-carboxamide |

In Table 1 and Table 2, the left portion of the structure of the HPK1 disruptors/degraders binds to HPK1 and the right portion of the structure recruits the ubiquitination machinery to HPK1, which induces poly-ubiquitination and degradation of HPK1 at the proteasome.

Compounds corresponding to Examples 1- 264 have been synthesized and final compounds are provided with a Compound Code in Table 1. Compounds in Table 2 corresponding to Examples 265 - 347 have not been synthesized and are not provided with a Compound Code. These compounds may be synthesized according to the schemes set forth above.

As used herein, in case of discrepancy between the structure and chemical name provided for a particular compound, the given structure shall control.

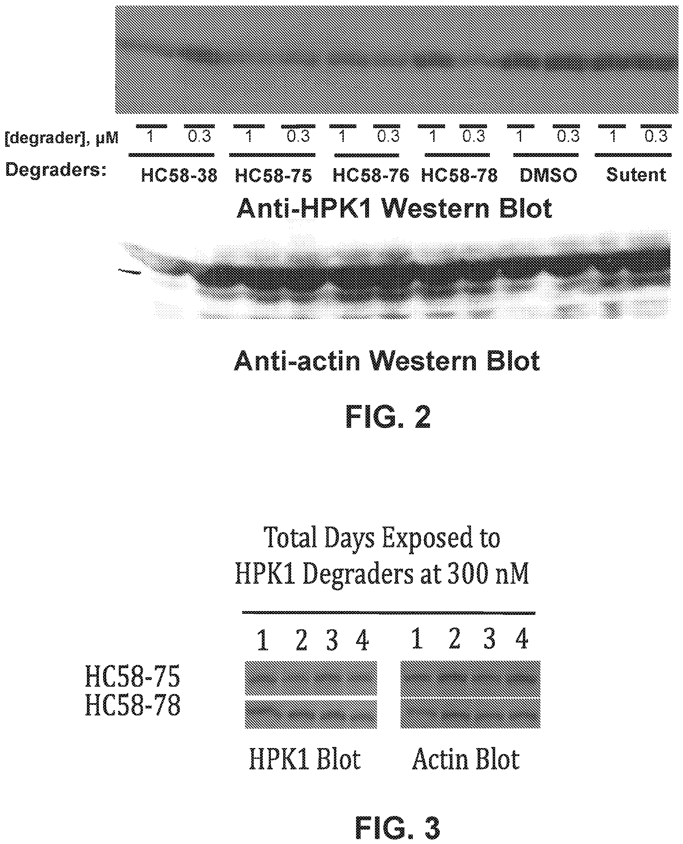

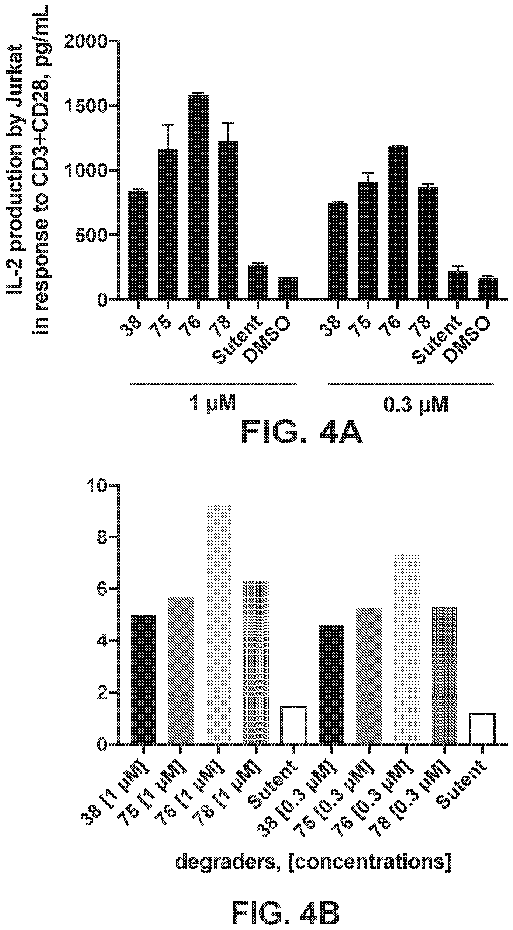

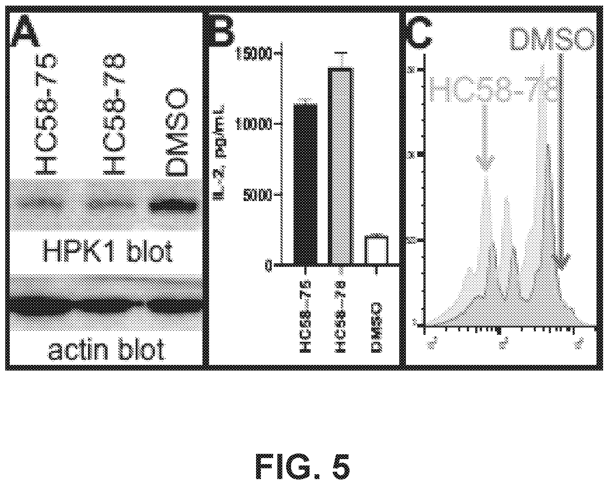

**EXAMPLE 348.** Several compounds according to the present invention employing the indolin-2-one HPK1 binding base, (which henceforth shall be referred to as the HC58 series, the HC65 series and the HC75 series) were assessed on their abilities to degrade HPK1 and produce an elevated IL-2 response profile upon stimulation by anti-CD3- and anti-CD28-mAb-mediated (CD3XCD28) stimulation (Figures 1-4). The elevated IL-2 response profile induced by TCR engagement after exposure to HC58 series of HPK1 degraders is shown in Figure 1. Western blot analysis revealed that some degraders were able to reduce the expression level of HPK1 protein in Jurkat T cell line by approximately 75% (Figures 2 and 3). Furthermore, Jurkat cells treated with these HPK1 degraders also exhibited elevated TCR-induced IL-2 response profile - nearly 10 fold higher than the DMSO treated control - that is similar to the profile observed in *HPK1*^{*-*/*-*} T cells (Figure 4). A select sub-set of the lead HPK1 degraders from the HC58 series were further characterized to determine whether they could degrade primary mouse T cells. Evidence was found which suggests that either 300 nM HC58-76 or 300 nM HC58-78 HPK1 degraders could degrade more HPK1 protein in primary T cells than in Jurkat T cells (Figure 5A). Both the HC58-75- and HC58-78-treated T cells produced more IL-2 than the DMSO-treated counterpart (Figure 5B). Lastly, HC58-78 could induce primary murine T cells to proliferate much more quickly than the DMSO-treated counterpart (Figure 5C). Similar results were found in HC58-75-treated T cells (data not shown).

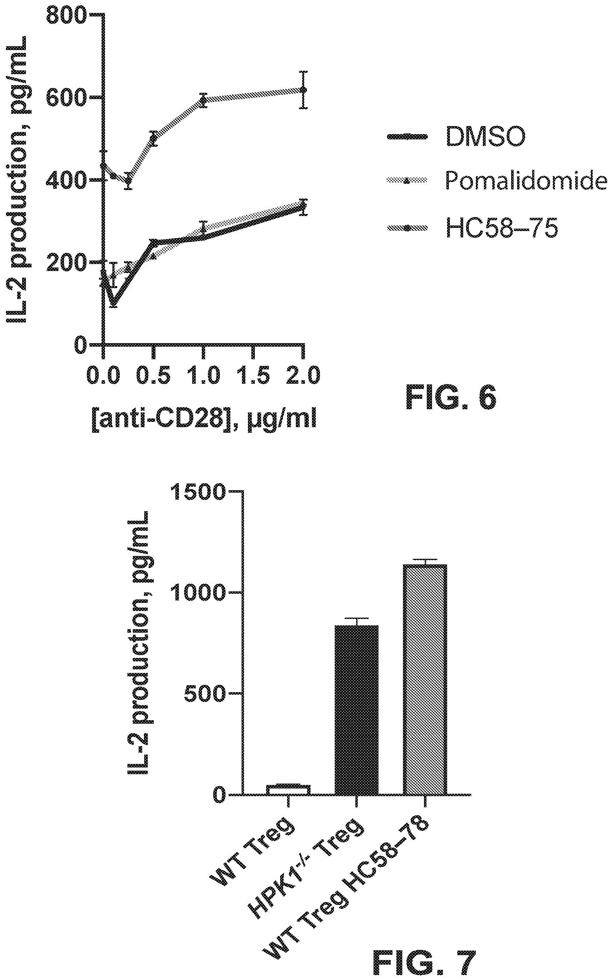

**EXAMPLE 349.** I was observed that *HPK1*^{*-*/*-*} T cells, unlike their wild type counterparts, could readily produce IL-2 in response to CD3 □ crosslinking alone (data not shown). To assess whether HPK1 degraders could confer the same ability to produce IL-2 in a CD28-independent manner, CD4⁺ primary murine T cells prepared as described in Figure 5 were treated with 500 nM of HC58-75 HPK1 degrader or DMSO or Pomalidomide as controls. Their abilities to produce IL-2 in response a fixed concentration of plate-bound anti-CD3 □ mAb (5 µg/mL) in conjunction with varying soluble concentration of anti-CD28 mAb was then assessed. Quantitative IL-2 ELISA analysis revealed that the HC58-75-treated T cells could produce IL-2 without the help of CD28 co-receptor engagement, which is similar to the IL-2 response produced by *HPK1*^{*-*/*-*} T cells (Figure 6 and data not shown).

**EXAMPLE 350.** It was observed that *HPK1*^{*-*/*-*} regulatory T cells (Treg), unlike their wild type counterparts, could readily produce IL-2 in response to CD3XCD28 stimulation. Taking advantage of the FoxP3-driven eGFP reporter mice that express eGFP only in the FoxP3⁺ regulatory T cells (Tregs), bonafide Tregs were purified from spleens and lymph nodes by FACS-assisted sorting of CD4⁺eGFP⁺ Tregs and used as the source of *ex vivo* functional studies. The ability of the wild type Treg, the *HPK1*^{*-*/*-*} Tregs and the HC58-78-treated wild type Tregs to produce IL-2 in response to CD3XCD28 stimulation was compared. Analysis of quantitative IL-2 ELISA revealed that the HC58-78-treated Tregs produced IL-2 at level comparable to that produced by *HPK1*^{*-*/*-*} Tregs (Figure 7, the black and grey histogram bars and data not shown). Wild type regulatory T cells could not produce the IL-2 in response to the same stimulation due to the transcriptional repression of the IL-2 locus by the FoxP3 transcription factor (Figure 7, the white histogram bar).

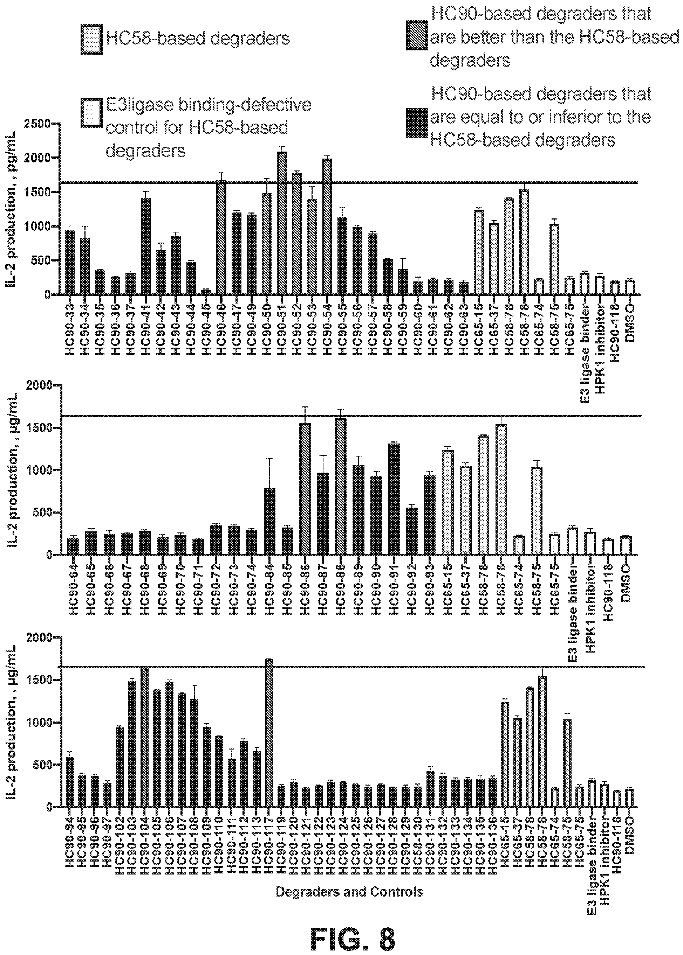

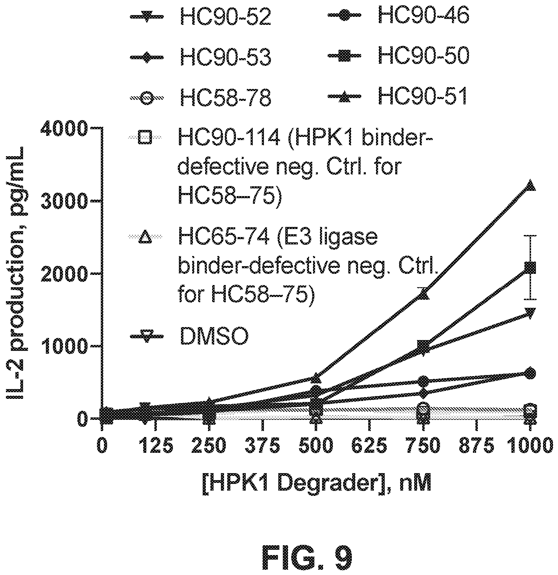

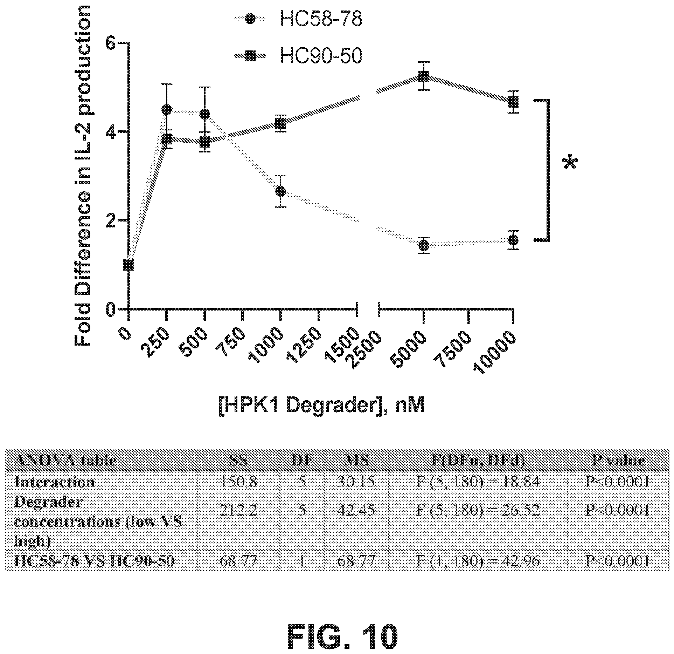

**EXAMPLE 351.** In an effort to improve the potency of HPK1 degraders, additional HPK1 degraders that utilize a different HPK1 binding scaffold (pyrazolo-pyrimidine) were designed and synthesized (referred to as the HC90 series). Jurkat cells that were pre-treated with each member of the HC90 HPK1 degrader series (500 nM) were screened for the amount of IL-2 produced in response CD3XCD28 stimulation. The levels of IL-2 produced were compared to the level produced by T cells that were treated by selected compounds from the HC58 series (Figure 8). Compounds that endowed Jurkat cells with either comparable or superior IL-2 response profiles when compared to the lead HC58 compounds (Figure 8, red histogram bars) were selected for confirmatory assessment in the presence of a wider ranges of compound concentrations (Figure 9). The top two compounds from the HC90 series, the HC90-50 and HC90-51 (Figure 9, black triangle and black square) were selected as lead compounds for the HC90 series and were subjected to additional analysis. Furthermore, compound HC58-78 and HC90-50 were also assessed for their abilities to enhance the CD3XCD28-induced IL-2 response by primary human PBMC (Figure 10).

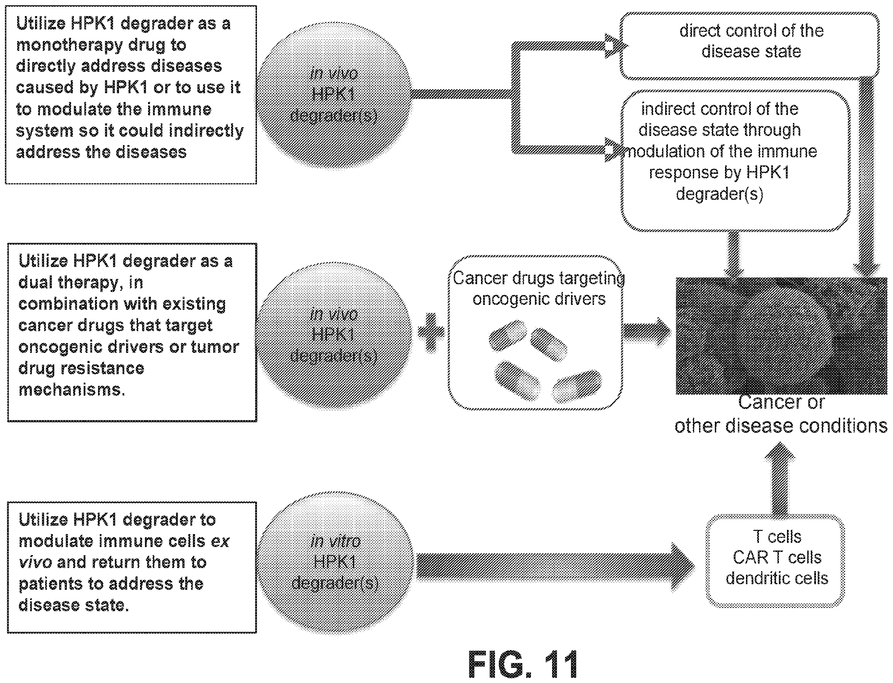

**EXAMPLE 352.** In an aspect, this disclosure provides several methods of treating HPK1-mediated diseases or diseases that could be controlled by the reduction of HPK1 expression level through administration of the compounds (HPK1 degraders) described herein. Compounds that could alter the expression level of HPK1 could be administered *in vivo* systematically alone, or in conjunction with other drugs, to directly or indirectly address the disease state. Alternatively, the HPK1 degraders could be administered externally to the *ex vivo* cells that had been prepared for therapeutic use, and then returned to the patients to address the disease state *in vivo.* These therapeutic concepts are schematically depicted in Figure 11.

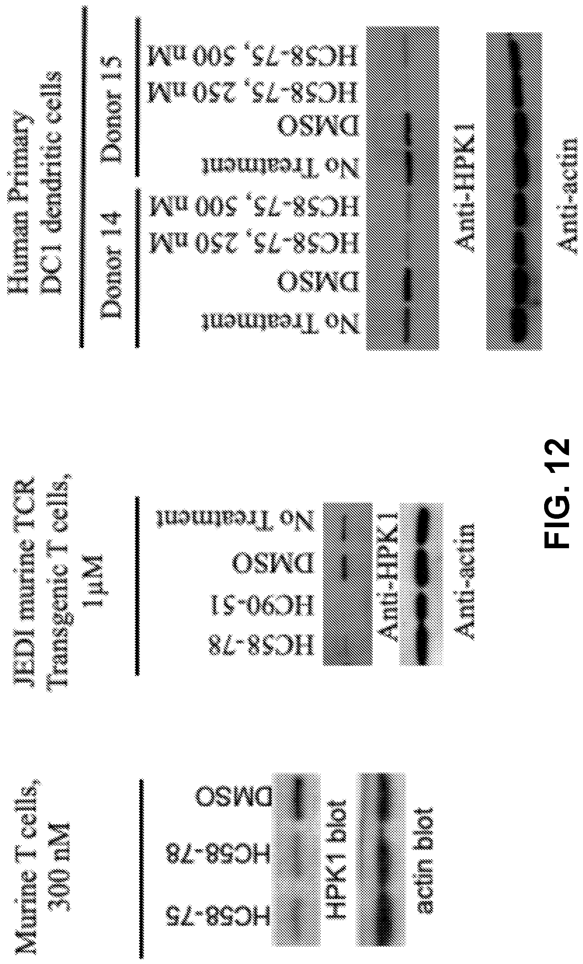

**EXAMPLE 353.** HPK1 is expressed in all hematopoietic cells examined to date, including T cells, B cells, dendritic cells and neutrophils. Because the breath of therapeutic approaches might involve the use of Chimeric Antigen Receptor (CAR) T cells, dendritic cells, TCR transgenic T cells and Tumor Infiltrating Lymphocytes (TILs), selected HPK1 degraders were evaluated to determine whether they could degrade HPK1 effectively in some of these cell types. Figure 12 revealed that HPK1 degraders could degrade HPK1 in murine primary T cells and in the JEDI TCR transgenic T cells as well. Potent degradation of HPK1 by HPK1 degraders was also observed in the human DC1 dendritic cells.

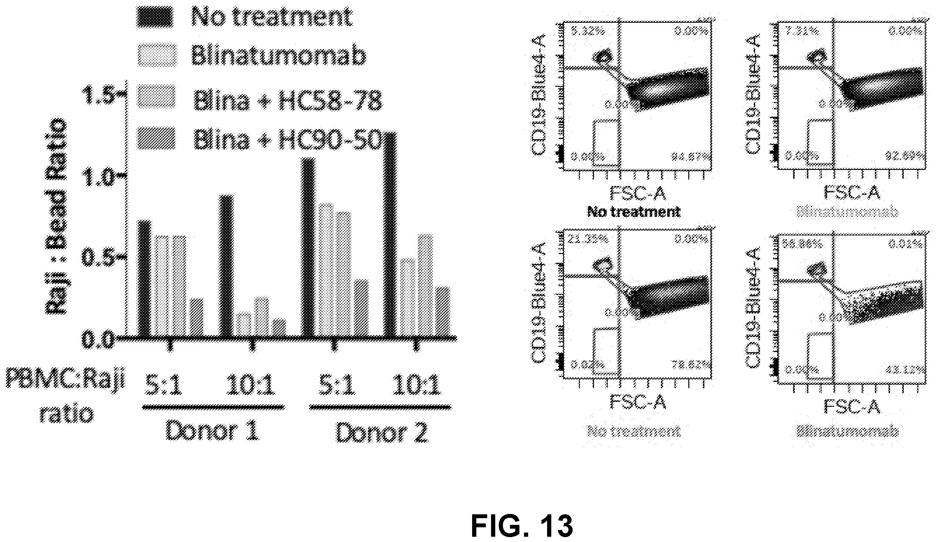

**EXAMPLE 354.** One of the approaches schematically depicted in Figure 11 is the use of HPK1 degraders in conjunction with other drugs. The ability of HPK1 degraders to enhance the T cell-mediated killing of CD19⁺ B-Cell Acute Lymphoblastic Leukemia treated with blinatumomab was assessed.. It was demonstrated that human PBMC that were treated with HPK1 degraders could aid blinatumomab in killing more human CD19⁺ B Cell Acute Lymphoblastic Leukemia cells, Raji (Figure 13).

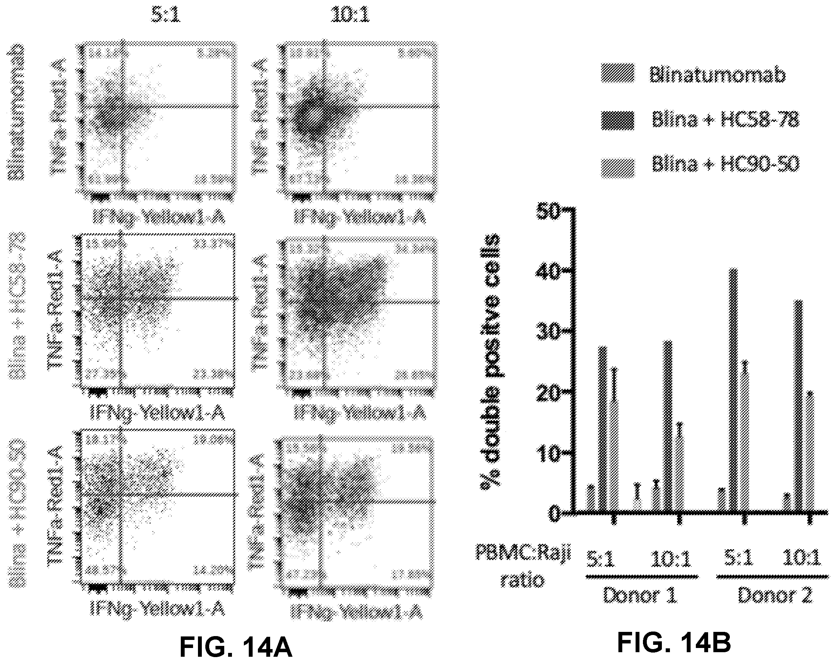

**EXAMPLE 355.** One of the mechanisms employed by PBMC to kill their target cells is by releasing IFNγ and TNFα production. These inflammatory cytokines are known have potent cytolytic effects on cancer cells. FACS analysis of anti-IFNy and TNFα intracellular staining revealed that combining either HC58-78 or HC90-50 with blinatumomab significantly increased the percentage of human PBMCs that express high level of IFNγ and TNFα (Figure 14A and 14B). These data suggest that the elevated level of IFNγ and TNFα produced by blinatumomab-treated PBMC might be responsible for the more effective killing of the CD19⁺ B Cell Acute Lymphoblastic Leukemia cells. Mediated PBMC killing of the Raji cells is most evident at PBMC:Raji ratio of 5:1 (Figure 13).

### Materials And Methods:

### General Chemistry Methods

For the synthesis of intermediates and examples (1-89) below, HPLC spectra for all compounds were acquired using an Agilent 1200 Series system with DAD detector. Chromatography was performed on a 2.1×150 mm Zorbax 300SB-C18 5 µm column with water containing 0.1% formic acid as solvent A and acetonitrile containing 0.1% formic acid as solvent B at a flow rate of 0.4 ml/min. The gradient program was as follows: 1% B (0-1 min), 1-99% B (1-4 min), and 99% B (4-8 min). High-resolution mass spectra (HRMS) data were acquired in positive ion mode using an Agilent G1969A API-TOF with an electrospray ionization (ESI) source. Nuclear Magnetic Resonance (NMR) spectra were acquired on a Bruker DRX-600 spectrometer with 600 MHz for proton (¹H NMR) and 150 MHz for carbon (¹³C NMR); chemical shifts are reported in (δ). Preparative HPLC was performed on Agilent Prep 1200 series with UV detector set to 254 nm. Samples were injected onto a Phenomenex Luna 250 × 30 mm, 5 µm, Cis column at room temperature. The flow rate was 40 ml/min. A linear gradient was used with 10% (or 50%) of MeOH (A) in H₂O (with 0.1 % TFA) (B) to 100% of MeOH (A). HPLC was used to establish the purity of target compounds. All final compounds had > 95% purity using the HPLC methods described above.

### Cell Lines and Tissue Culture

The Jurkat (sub-clone E6-1 [ATCC^{®} TIB-152^{™}]) is a human acute T cell leukemia cell line that was established from the peripheral blood of a 14 year old boy. (Weiss 1984) (Gillis 1980). Schneider U, et al. Characterization of EBV-genome negative "null" and "T" cell lines derived from children with acute lymphoblastic leukemia and leukemic transformed non-Hodgkin lymphoma. Int. J. Cancer 19: 621-626, 1977. PubMed: 68013. Clone E6-1 cells is a preferred cellular platform for studying T cell activation in response to T cell receptor engagement because it produces the cytokine IL-2, which could be used as proxy readout for the level of T cell activation. While the Jurkat cell would produce IL-2 when stimulated with phorbol esters and either lectins or monoclonal antibodies CD3ε itself, the stimulation that most mimic T cell activation *in vivo* is to use anti-CD3ε monoclonal antibody in conjunction with anti-CD28 monoclonal antibody. It is this activation method that was used to stimulate Jurkat T cells to that had been treated with degraders of interest. Specifically, the anti-CD3ε monoclonal antibody (OKT3 clone, 5 µg per mL) is absorbed onto 96-well polystyrene tissue culture plate to form a planar surface that mimics the surface of antigen presentating cells. Excess non-absorbed antibody is washed off with phosphate buffer saline 3 times and would be used to intiate stimulation of Jurkat T cells that had been pre-treated with indicated concentration of the degraders. Jurkat cells were resuspended in culture medium (RPMI-1640, 10% PCS, 1 × L-glutamine and 1 × penicillin and streptomycin). Fifty thousands Jurkat cells were added to each well in duplicate, in the presence of (5 µg per mL of anti-CD28 monoclonal antibody). The plate is then placed into a humidity controlled, 5% CO₂ incubator for 24 hours. The indicated concentration of degrader is maintained through out the stimulation period of 24 hours. Supernatant from Jukat cells were collected and the amount of IL-2 present in the supernatant was assessed by standard anti-human IL-2 ELISA.

The ability of degraders to degrade HPK protein level in treated Jukat cels was determined by Western blot, using anti-human HPK1 polyclonal antibody as probe (CSL). Degrader-treated Jurkat cells were lysed in standard NP-40 lysis buffer and lysate equalvalent to 1 × 10⁶ Jurkat cells were loaded into each well of the 10% poly-acrylamide gel and subjected to electrophoretic separation by SDS-PAGE. Proteins in lysates that had been resolved by SDS-PAGE were transferred to PVDF support matrix, blocked by 4% BSA containing solution and probed with anti-human HPK1 antibody, using standard Western blotting procedure.

To determine the number of CD19⁺ Raji cells killed by human PBMC that were activated by the Blinatumomab-mediated activation, we used CountBright cell counting beads to estimate the number of cells remained after 72 hours of co-culturing of PBMC and Raji. As for the intracellular staining of the TNFα and the IFNγ, the standard para-formaldehyde-based "fix and perm" procedure was used in order to quantitate the amount of intracellular cytokine trapped inside the cells by brefeldin A. FACS analysis of stained cells is performed by BD's LSR II FACS .

### REFERENCES

Alzabin, S., Bhardwaj, N., Kiefer, F., Sawasdikosol, S., and Burakoff, S. (2009). Hematopoietic progenitor kinase 1 is a negative regulator of dendritic cell activation. J Immunol 182, 6187-6194.
Alzabin, S., Pyarajan, S., Yee, H., Kiefer, F., Suzuki, A., Burakoff, S., and Sawasdikosol, S. (2010). Hematopoietic progenitor kinase 1 is a critical component of prostaglandin E2-mediated suppression of the anti-tumor immune response. Cancer Immunol Immunother 59, 419-429.
Bondeson, D.P., Mares, A., Smith, I.E., Ko, E., Campos, S., Miah, A.H., Mulholland, K.E., Routly, N., Buckley, D.L., Gustafson, J.L., et al. (2015). Catalytic in vivo protein knockdown by small-molecule PROTACs. Nat Chem Biol 11, 611-617.
Buckley, D.L., and Crews, C.M. (2014). Small-molecule control of intracellular protein levels through modulation of the ubiquitin proteasome system. Angew Chem Int Ed Engl 53, 2312-2330.
Buckley, D.L., Gustafson, J.L., Van Molle, I., Roth, A.G., Tae, H.S., Gareiss, P.C., Jorgensen, W.L., Ciulli, A., and Crews, C.M. (2012a). Small-molecule inhibitors of the interaction between the E3 ligase VHL and HIF1alpha. Angew Chem Int Ed Engl 51, 11463-11467.
Buckley, D.L., Raina, K., Darricarrere, N., Hines, J., Gustafson, J.L., Smith, I.E., Miah, A.H., Harling, J.D., and Crews, C.M. (2015). HaloPROTACS: Use of Small Molecule PROTACs to Induce Degradation of HaloTag Fusion Proteins. ACS Chem Biol 10, 1831-1837.
Buckley, D.L., Van Molle, I., Gareiss, P.C., Tae, H.S., Michel, J., Noblin, D.J., Jorgensen, W.L., Ciulli, A., and Crews, C.M. (2012b). Targeting the von Hippel-Lindau E3 ubiquitin ligase using small molecules to disrupt the VHL/HIF-1alpha interaction. J Am Chem Soc 134, 4465-4468.
Burnet, F.M. (1970). The concept of immunological surveillance. Progress in experimental tumor research 13, 1-27.
Chamberlain, P.P., Lopez-Girona, A., Miller, K., Carmel, G., Pagarigan, B., Chie-Leon, B., Rychak, E., Corral, L.G., Ren, Y.J., Wang, M., et al. (2014). Structure of the human Cereblon-DDB1-lenalidomide complex reveals basis for responsiveness to thalidomide analogs. Nat Struct Mol Biol 21, 803-809.
Corthay, A. (2014). Does the immune system naturally protect against cancer? Frontiers in immunology 5, 197.
Davies, T.G., Wixted, W.E., Coyle, IE., Griffiths-Jones, C., Heam, K., Mcl\fonamin, R., Norton, D., Rich, S.J., Richardson, C., Saxty, G., et al. (2016). Monoacidic Inhibitors of the Kelch-like ECH-Associated Protein 1: Nuclear Factor Erythroid 2-Related Factor 2 (KEAP1:NRF2) Protein-Protein Interaction with High Cell Potency Identified by Fragment-Based Discovery. J Med Chem 59, 3991-4006.
Fischer, E.S., Bohm, K., Lydeard, J.R., Yang, H., Stadler, M.B., Cavadini, S., Nagel, J., Serluca, F., Acker, V., Lingaraju, G.M., et al. (2014). Structure of the DDB1-CRBN E3 ubiquitin ligase in complex with thalidomide. Nature 512, 49-53.
Galdeano, C., Gadd, M.S., Soares, P., Scaffidi, S., Van Molle, I., Birced, I., Hewitt, S., Dias, D.M., and Ciulli, A. (2014). Structure-guided design and optimization of small molecules targeting the protein-protein interaction between the von Hippel-Lindau (VHL) E3 ubiquitin ligase and the hypoxia inducible factor (HIF) alpha subunit with in vitro nanomolar affinities. J Med Chem 57, 8657-8663.
Gillis, S., and J. Watson. 1980. Biochemical and biological characterization of lymphocyte regulatory molecules. V. Identification of an interleukin 2-producing human leukemia T cell line. The Journal of experimental medicine 152: 1709-1719.
Hernandez, S., Qing, J., Thibodeau, R.H., Du, X., Park, S., Lee, H.M., Xu, M., Oh, S., Navarro, A., Roose-Girma, M., et al. (2018). The Kinase Activity of Hematopoietic Progenitor Kinase 1 Is Essential for the Regulation of T Cell Function. Cell reports 25, 80-94.
Hu, M.C., Qiu, W.R., Wang, X., Meyer, C.F., and Tan, T.H. (1996). Human HPK1, a novel human hematopoietic progenitor kinase that activates the JNK/SAPK kinase cascade. Genes Dev 10, 2251-2264.
Ito, T., Ando, H., Suzuki, T., Ogura, T., Hotta, K., Imamura, Y., Yamaguchi, Y., and Handa, H. (2010). Identification of a primary target of thalidomide teratogenicity. Science 327, 1345-1350. Kiefer, F., Tibbles, L.A., Anafi, M., Janssen, A., Zanke, B.W., Lassam, N., Pawson, T.,
Woodgett, J.R., and Iscove, N.N. (1996). HPK1, a hematopoietic protein kinase activating the SAPK/JNK pathway. EMBO J 15, 7013-7025.
Lai, A.C., Toure, M., Hellerschmied, D., Salami, J., Jaime-Figueroa, S., Ko, E., Hines, J., and Crews, C.M. (2016). Modular PROTAC Design for the Degradation of Oncogenic BCR-ABL. Angew Chem Int Ed Engl 55, 807-810.
Ling, P., Meyer, C.F., Redmond, L.P., Shui, J.W., Davis, B., Rich, R.R., Hu, M.C., Wange, R.L., and Tan, T.H. (2001). Involvement of hematopoietic progenitor kinase 1 in T cell receptor signaling. The Journal of biological chemistry 276, 18908-18914.
Liou, J., Kiefer, F., Dang, A., Hashimoto, A., Cobb, M.H., Kurosaki, T., and Weiss, A. (2000). HPK1 is activated by lymphocyte antigen receptors and negatively regulates AP-1. Immunity 12, 399-408.
Liu, J., Curtin, J., You, D., Hillerman, S., Li-Wang, B., Eraslan, R., Xie, J., Swanson, J., Ho, C.P., Oppenheimer, S., etal. (2019). Critical role of kinase activity of hematopoietic progenitor kinase 1 in anti-tumor immune surveillance. PloS one 14, e0212670.
Lu, J., Qian, Y., Altieri, M., Dong, H., Wang, J., Raina, K., Hines, J., Winkler, J.D., Crew, A.P., Coleman, K., et al. (2015). Hijacking the E3 Ubiquitin Ligase Cereblon to Efficiently Target BRD4. Chemistry & biology 22, 755-763.
Ohoka, N, Okuhira, K., Ito, M, Nagai, K, Shibata, N., Hattori, T., Ujikawa, O, Shimokawa, K., Sano, O., Koyama, R., et al (2017). In Vivo Knockdown of Pathogenic Proteins via Specific and Nongenetic Inhibitor of Apoptosis Protein (IAP)-dependent Protein Erasers (SNIPERs). J Biol Chem 292, 4556-4570.
Okuhira, K., Ohoka, N., Sai, K., Nishimaki-Mogami, T., Itoh, Y., Ishikawa, M., Hashimoto, Y., and Naito, M. (2011). Specific degradation of CRABP-II via cIAPI-mediated ubiquitylationinduced by hybrid molecules that crosslink clAPl and the target protein. FEBS Lett 585, 1147-1152.
Ribas, A., and Wolchok, J.D. (2018). Cancer immunotherapy using checkpoint blockade. Science (New York, NY 359, 1350-1355.
Sawasdikosol, S., Pyarajan, S., Alzabin, S., Matejovic, G., and Burakoff, S.J. (2007). Prostaglandin E2 activates HPK1 kinase activity via a PKA-dependent pathway. The Journal of biological chemistry 282, 34693-34699.
Sawasdikosol, S., Russo, K.M., and Burakoff, S.J. (2003). Hematopoietic progenitor kinase 1 (HPK1) regulates prostaglandin E2-induced fos gene transcription. Blood 101, 3687-3689. Sawasdikosol, S., Zha, R., Yang, B., and Burakoff, S. (2012). HPK1 as a novel target for cancer immunotherapy. Immunologic research 54, 262-265.
Shibata, N., Miyamoto, N., Nagai, K., Shimokawa, K., Sameshima, T., Ohoka, N., Hattori, T., Imaeda, Y, Nara, H., Cho, N., et al. (2017). Development of protein degradation inducers of oncogenic BCR-ABL protein by conjugation of ABL kinase inhibitors and IAP ligands. Cancer Sci 108, 1657-1666.
Shui, J.W., Boomer, J.S., Han, J., Xu, J., Dement, G.A., Zhou, G., and Tan, T.H. (2007). Hematopoietic progenitor kinase 1 negatively regulates T cell receptor signaling and T cell-mediated immune responses. Nat Immunol 8, 84-91.
Sun, D., Li, Z, Rew, Y., Gribble, M, Bartberger, M.D., Beck, H.P., Canon, J., Chen, A., Chen, X, Chow, D., et al. (2014). Discovery of AMG 232, a potent, selective, and orally bioavailable MDM2-p53 inhibitor in clinical development. J Med Chem 57, 1454-1472.
Weiss, A., R. L. Wiskocil, and J. D. Stobo. 1984. The role of T3 surface molecules in the activation of human T cells: a two-stimulus requirement for IL 2 production reflects events occurring at a pre-translational level. J Immunol 133: 123-128.
Winter, G.E., Buckley, D.L., Paulk, J., Roberts, J.M., Souza, A., Dhe-Paganon, S., and Bradner, J.E. (2015). Phthalimide conjugation as a strategy for in vivo target protein degradation. Science 348, 1376-1381.
Xie, T., Lim, S.M., Westover, K.D., Dodge, M.E., Ercan, D., Ficarro, S.B., Udayakumar, D., Gurbani, D., Tae, H.S., Riddle, S.M., et al. (2014). Pharmacological targeting of the pseudokinase Her3. Nat Chem Biol 10, 1006-1012.
Zengerle, M., Chan, K.H., and Ciulli, A. (2015). Selective Small Molecule Induced Degradation of the BET Bromodomain Protein BRD4. ACS Chem Biol 10, 1770-1777.

## Claims

1. A bivalent compound comprising a hematopoietic progenitor kinase 1 (HPK1) ligand conjugated to a degradation/disruption tag, wherein the bivalent compound has the structure: 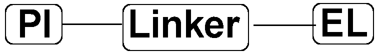
**(I)** wherein **PI** comprises an HPK1 ligand and EL comprises a degradation/disruption tag, wherein **PI** is a moiety selected from the group consisting of:
**(I-A)** a moiety according to FORMULA 3L: 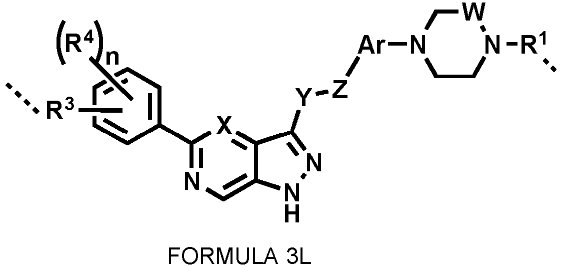 wherein
the "Linker" moiety of the bivalent compound is attached independently to R¹ or R³;
X is selected from CR² or N;
R¹ and R³ are independently selected from null, hydrogen, C(O)R⁶, C(O)OR⁶, C(O)NR⁶R⁷, S(O)R⁶, S(O)₂R⁶, S(O)₂NR⁶R⁷, NR⁸C(O)OR⁶, NR⁸C(O)R⁶, NR⁸C(O)NR⁶R⁷, NR⁸S(O)R⁶, NR⁸S(O)₂R⁶, NR⁸S(O)₂NR⁶R⁷ , optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
R⁶ is null, or a bivalent moiety selected from optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted C₃-C₈ cycloalkoxy,
optionally substituted 3-8 membered heterocyclyl, optionally substituted aryl, and optionally substituted heteroaryl;
R⁷, and R⁸ are independently selected from hydrogen, optionally substituted C₁-C₈ alkyl, optionally substituted C₁-C₈alkoxyC₁-C₈alkyl, optionally substituted C₁-C₈alkylaminoC₁-C₈alkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
R⁶ and R⁷ together with the atom to which they are connected form an optionally substituted 3-20 membered cycloalkyl or heterocyclyl ring;
R² is independently selected from hydrogen, halogen, oxo, CN, NO₂, OR⁹, SR⁹, NR⁹R¹⁰, C(O)R⁹, C(O)OR⁹, C(O)NR⁹R¹⁰, S(O)R⁹, S(O)₂R⁹, S(O)₂NR⁹R¹⁰, NR¹¹C(O)OR⁹, NR¹¹C(O)R⁹, NR¹¹C(O)NR⁹R¹⁰, NR¹¹S(O)R⁹, NR¹¹S(O)₂R⁹, NR¹¹S(O)₂NR⁹R¹⁰ , optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-CsalkylaminoCi-Csalkyl, optionally substituted 3-8 membered cycloalkyl, optionally substituted C₃-C₈ cycloalkoxy, optionally substituted 3-8 membered heterocyclyl, optionally substituted aryl, and optionally substituted heteroaryl; wherein
R⁹, R¹⁰, and R¹¹ are independently selected from hydrogen, optionally substituted C₁-C₈ alkyl, optionally substituted C₁-C₈alkoxyC₁-C₈alkyl, optionally substituted C₁-C₈alkylaminoC₁-C₈alkyl, optionally substituted C₃-C₁₀ cycloalkyl, optionally substituted 3-20 membered heterocyclyl, optionally substituted C₂-C₈ alkenyl, optionally substituted C₂-C₈ alkynyl, optionally substituted aryl, and optionally substituted heteroaryl; or
R⁹ and R¹⁰, R⁹ and R¹¹, R¹⁰ and R¹¹ together with the atom to which they are connected form an optionally substituted 3-20 membered cycloalkyl or heterocyclyl ring;
each R⁴ is independently selected from null, hydrogen, halogen, oxo, CN, NO₂, OR¹⁸, SR¹⁸, NR¹⁸R¹⁹, OCOR¹⁸, OCO₂R¹⁸, OCONR¹⁸R¹⁹, COR¹⁸, CO₂R¹⁸, CONR¹⁸R¹⁹, SOR¹⁸, SO₂R¹⁸, SO₂NR¹⁸R¹⁹, NR²⁰CO₂R¹⁸, NR²⁰COR¹⁸, NR²⁰C(O)NR¹⁸R¹⁹, NR²⁰SOR¹⁸, NR²⁰SO₂R¹⁸, NR⁷SO₂NR⁵R⁶, optionally substituted C₁-C₈ alkyl, optionally substituted C₂-C₈ alkenyl, optionally substituted C₂-C₈ alkynyl, optionally substituted C₁-C₈ alkoxy, optionally substituted C₁-CsalkoxyCi-Csalkyl, optionally substituted C₁-C₈alkylaminoC₁-C₈alkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; wherein
R¹⁸ , R¹⁹ and R²⁰ are independently selected from hydrogen, optionally substituted C₁-C₈ alkyl, optionally substituted C₂-C₈ alkenyl, optionally substituted C₂-C₈ alkynyl, optionally substituted C₁-C₈ alkoxy, optionally substituted C₁-C₈alkoxyC₁-C₈alkyl, optionally substituted C₁-CsalkylaminoCi-Csalkyl, optionally substituted 3-8 membered cycloalkyl, optionally substituted C₃-C₈ cycloalkoxy, optionally substituted 4-8 membered heterocyclyl, optionally substituted aryl, and optionally substituted heteroaryl; or
R¹⁸ and R¹⁹, R¹⁸ and R²⁰ together with the atom to which they are connected form a 4-20 membered heterocyclyl ring;
Ar is selected from null, aryl and heteroaryl;
n is independently selected from 0, 1, 2, 3, 4 and 5;
Y, Z at each occurrence, are independently selected from null, CO, CO₂, CH₂, CR²⁴R²⁵, C(O)NR²⁴, C(S)NR²⁴, O, S, SO, SO₂, SO₂NR²⁴, NR²⁴, NR²⁴CO, NR²⁴CONR²⁴, NR²⁴C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
R²⁴ and R²⁵ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
W, at each occurrence, is independently selected from null, CO, CH₂, (CH₂)ₘCR²⁶R²⁷, (CR²⁶R²⁷)ₘ, SO, SO₂
wherein
R²⁶ and R²⁷ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
and
m = 0-5;
**(I-B)** a moiety according to FORMULA 3M: 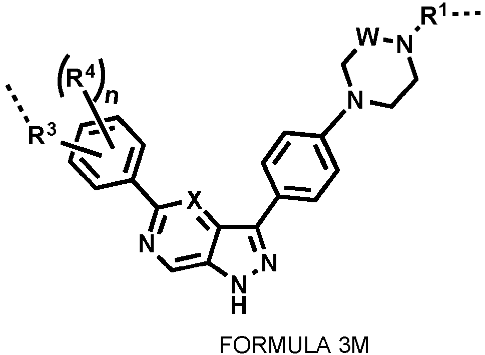 wherein
the "Linker" moiety of the bivalent compound is attached independently to R¹ or R³; X is selected from CR² or N;
the definitions of W, R¹, R², R³ and R⁴ are the same as for FORMULA 3L;
n is independently selected from 0, 1, 2, 3, 4 and 5;
and pharmaceutically acceptable salts thereof;
**(II)** wherein EL is a moiety selected from the group consisting of:
**(II-A)** a moiety according to FORMULAE 12A, 12B, 12C and 12D: 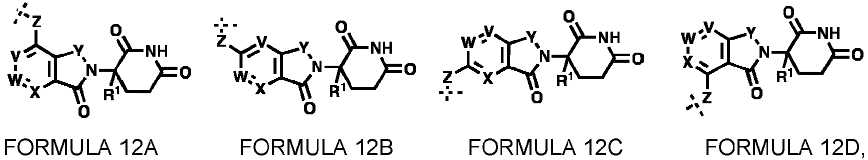 wherein
V, W, and X are independently selected from CR² and N;
Y is selected from CO, CR³R⁴, and N=N;
Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
**(II-B)** a moiety according to one of FORMULAE 12E, 12F, 12G, 12H, and 12I: 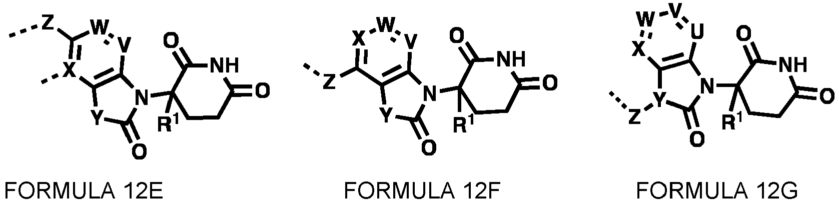 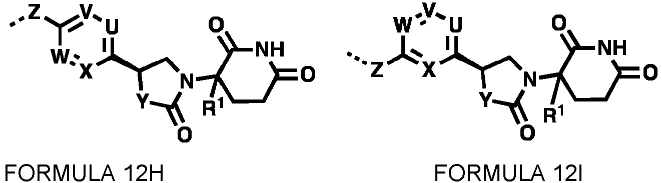 wherein
U, V, W, and X are independently selected from CR² and N;
Y is selected from CR³R⁴, NR³ and O; preferably, Y is selected from CH₂, NH, NCH₃ and O;
Z is selected from null, CO, CR⁵R⁶, NR⁵, O, optionally substituted C₁-C₁₀ alkylene, optionally substituted C₁-C₁₀ alkenylene, optionally substituted C₁-C₁₀ alkynylene, optionally substituted 3-10 membered carbocyclyl, optionally substituted 4-10 membered heterocyclyl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, optionally substituted C₃-C₁₃ spiro heterocyclyl, optionally substituted aryl, and optionally substituted heteroaryl; preferably, Z is selected from null, CH₂, CH=CH, C=C, NH and O;
R¹, and R² are independently selected from hydrogen, halogen, cyano, nitro, optionally substituted C₁-C₆ alkyl, optionally substituted 3 to 6 membered carbocyclyl, and optionally substituted 4 to 6 membered heterocyclyl;
R³, and R⁴ are independently selected from hydrogen, halogen, cyano, nitro, optionally substituted C₁-C₆ alkyl, optionally substituted 3 to 6 membered carbocyclyl, and optionally substituted 4 to 6 membered heterocyclyl; or R³ and R⁴ together with the atom to which they are connected form a 3-6 membered carbocyclyl, or 4-6 membered heterocyclyl; and
R⁵ and R⁶ are independently selected from null, hydrogen, halogen, oxo, hydroxyl, amino, cyano, nitro, optionally substituted C₁-C₆ alkyl, optionally substituted 3 to 6 membered carbocyclyl, and optionally substituted 4 to 6 membered heterocyclyl; or R⁵ and R⁶ together with the atom to which they are connected form a 3-6 membered carbocyclyl, or 4-6 membered heterocyclyl;
**(II-C)** a moiety according to FORMULA 13A: 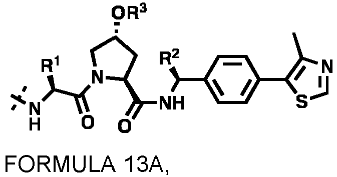 wherein
R¹ and R² are independently selected from hydrogen, optionally substituted C₁-C₈ alkyl, optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; and
R³ is hydrogen, optionally substituted C(O)Ci-Cs alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC,-CaalkoxyCi-Csalkyl, optionally substituted C(O)NC₁-C₈ haloalkyl, optionally substituted C(O)NC₁-C₈ hydroxyalkyl, optionally substituted C(O)NC₁-C₈ aminoalkyl, optionally substituted C(O)NC,-C₈alkylaminoC₁-C₈alkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂;
**(II-D)** a moiety according to FORMULAE 13B, 13C, 13D, 13E and 13F: 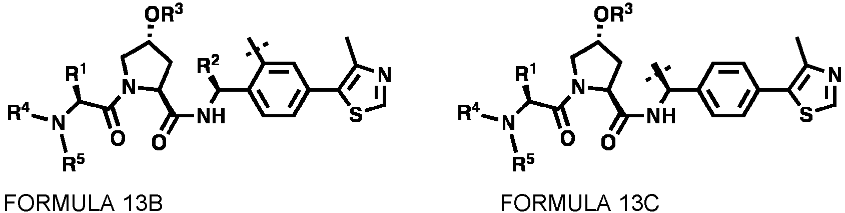 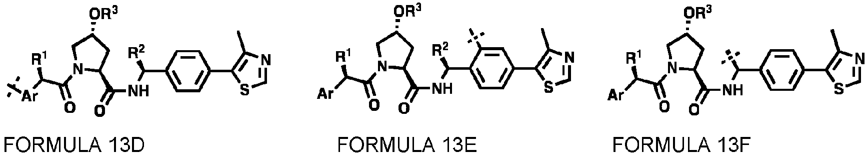 wherein
R¹ and R² are independently selected from hydrogen, halogen, OH, NH₂, CN, optionally substituted C₁-C₈ alkyl, optionally substituted C₁-C₈alkoxyC₁-C₈alkyl, optionally substituted C₁-C₈ haloalkyl, optionally substituted C₁-C₈ hydroxyalkyl, optionally substituted C₁-C₈ aminoalkyl, optionally substituted C₁-C₈alkylaminoC₁-C₈alkyl, optionally substituted C₃-C₇ cycloalkyl, optionally substituted 3-7 membered heterocyclyl, optionally substituted C₂-C₈ alkenyl, and optionally substituted C₂-C₈ alkynyl; (preferably, R¹ is selected from iso-propyl or tert-butyl; and R² is selected from hydrogen or methyl);
R³ is hydrogen, optionally substituted C(O)C₁-C₈ alkyl, optionally substituted C(O)C₁-C₈alkoxyC₁-C₈alkyl, optionally substituted C(O)C₁-C₈ haloalkyl, optionally substituted C(O)C₁-C₈ hydroxyalkyl, optionally substituted C(O)C₁-C₈ aminoalkyl, optionally substituted C(O)C₁-C₈alkylaminoC₁-C₈alkyl, optionally substituted C(O)C₃-C₇ cycloalkyl, optionally substituted C(O)(3-7 membered heterocyclyl), optionally substituted C(O)C₂-C₈ alkenyl, optionally substituted C(O)C₂-C₈ alkynyl, optionally substituted C(O)OC₁-C₈alkoxyC₁-C₈alkyl, optionally substituted C(O)OC₁-C₈ haloalkyl, optionally substituted C(O)OC₁-C₈ hydroxyalkyl, optionally substituted C(O)OC₁-C₈ aminoalkyl, optionally substituted C(O)OC₁-C₈alkylaminoC₁-C₈alkyl, optionally substituted C(O)OC₃-C₇ cycloalkyl, optionally substituted C(O)O(3-7 membered heterocyclyl), optionally substituted C(O)OC₂-C₈ alkenyl, optionally substituted C(O)OC₂-C₈ alkynyl, optionally substituted C(O)NC₁-C₈alkoxyC₁-C₈alkyl, optionally substituted C(O)NC₁-C₈ haloalkyl, optionally substituted C(O)NC,-C₈ hydroxyalkyl, optionally substituted C(O)NC₁-C₈ aminoalkyl, optionally substituted C(O)NC,-CsalkylaminoCi-Csalkyl, optionally substituted C(O)NC₃-C₇ cycloalkyl, optionally substituted C(O)N(3-7 membered heterocyclyl), optionally substituted C(O)NC₂-C₈ alkenyl, optionally substituted C(O)NC₂-C₈ alkynyl, optionally substituted P(O)(OH)₂, optionally substituted P(O)(OC₁-C₈ alkyl)₂, and optionally substituted P(O)(OC₁-C₈ aryl)₂; and
R⁴ and R⁵ are independently selected from hydrogen, COR⁶, CO₂R⁶, CONR⁶R⁷, SOR⁶, SO₂R⁶, SO₂NR⁶R⁷, optionally substituted C₁-C₈ alkyl, optionally substituted C₁-C₈alkoxyC₁-C₈alkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; wherein
R⁶ and R⁷ are independently selected from hydrogen, optionally substituted C₁-C₈ alkyl, optionally substituted C₁-C₈ alkoxy, optionally substituted C₁-C₈alkoxyC₁-C₈alkyl, optionally substituted C₁-C₈alkylaminoC₁-C₈alkyl, optionally substituted 3-8 membered cycloalkyl, optionally substituted 3-8 membered heterocyclyl, optionally substituted aryl, and optionally substituted heteroaryl; or
R⁴ and R⁵; R⁶ and R⁷ together with the atom to which they are connected form a 4-8 membered cycloalkyl or heterocyclyl ring;
Ar is selected from aryl and heteroaryl, each of which is optionally substituted with one or more substituents independently selected from F, Cl, CN, NO₂, OR⁸, NR⁸R⁹, COR⁸, CO₂R⁸, CONR⁸R⁹, SOR⁸, SO₂R⁸, SO₂NR⁹R¹⁰, NR⁹COR¹⁰, NR⁸C(O)NR⁹R¹⁰, NR⁹SOR¹⁰, NR⁹SO₂R¹⁰, optionally substituted C₁-C₆ alkyl, optionally substituted C₁-C₆ alkoxyalkyl, optionally substituted C₁-C₆ haloalkyl, optionally substituted C₁-C₆ hydroxyalkyl, optionally substituted C₁-C₆alkylaminoC₁-C₆alkyl, optionally substituted C₃-C₇ cycloalkyl, optionally substituted 3-7 membered heterocyclyl, optionally substituted C₂-C₆ alkenyl, optionally substituted C₂-C₆ alkynyl, optionally substituted aryl, and optionally substituted C₄-C₅ heteroaryl; wherein
R⁸, R⁹, and R¹⁰ are independently selected from null, hydrogen, optionally substituted C₁-C₆ alkyl, optionally substituted C₂-C₆ alkenyl, optionally substituted C₂-C₆ alkynyl, optionally substituted C₃-C₇ cycloalkyl, optionally substituted 3-7 membered heterocyclyl, optionally substituted aryl, and optionally substituted heteroaryl; or
R⁸ and R⁹; R⁹ and R¹⁰ together with the atom to which they are connected form a 4-8 membered cycloalkyl or heterocyclyl ring;
and pharmaceutically acceptable salts thereof;
**(III)** wherein the linker is:
(III-A) a moiety selected from the group consisting of:
(III-A-a) a moiety according to FORMULA 16: 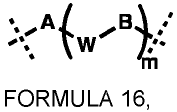 wherein
A, W, and B, at each occurrence, are independently selected from null, CO, CO₂, C(O)NR¹, C(S)NR¹, O, S, SO, SO₂, SO₂NR¹, NR¹, NR¹CO, NR¹CONR², NR¹C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy,optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
R¹ and R² are independently selected from hydrogen, optionally substituted C₁-C₈ alkyl, optionally substituted C₃-C₈ cycloalkyl, optionally substituted 3-8 membered cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl; and
m is 0 to 15;
**(III-A-b)** a moiety according to FORMULA 16A: 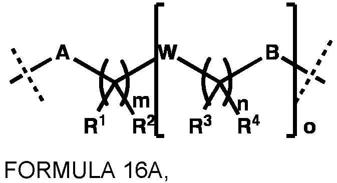 wherein
R¹, R², R³, and R⁴, at each occurrence, are independently selected from hydrogen, halogen, CN, OH, NH₂, optionally substituted C₁-C₈alkyl, optionally substituted C₃-C₈ cycloalkyl, optionally substituted C₃-C₈ cycloalkoxy, optionally substituted 3-8 membered membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
A, W, and B, at each occurrence, are independently selected from null, CO, CO₂, C(O)NR⁵, C(S)NR⁵, O, S, SO, SO₂, SO₂NR⁵, NR⁵, NR⁵CO, NR⁵CONR⁶, NR⁵C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
R⁵ and R⁶ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈alkylaminoC₁-C₈alkyl;
m is 0 to 15;
n, at each occurrence, is 0 to 15; and
o is 0 to 15;
**(III-A-c)** a moiety according to FORMULA 16B: 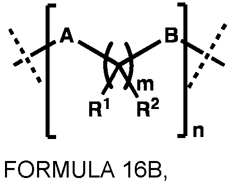 wherein
R¹ and R², at each occurrence, are independently selected from hydrogen, halogen, CN, OH, NH₂, and optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
A and B, at each occurrence, are independently selected from null, CO, CO₂, C(O)NR³, C(S)NR³, O, S, SO, SO₂, SO₂NR³, NR³, NR³CO, NR³CONR⁴, NR³C(S), and optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, or C₃-C₁₃ spiro heterocyclyl; wherein
R³ and R⁴ are independently selected from hydrogen, and optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, or C₁-C₈alkylaminoC₁-C₈alkyl;
each m is 0 to 15; and
n is 0 to 15;
**(III-A-d)** a moiety according to FORMULA 16C: 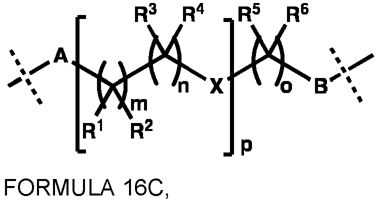 wherein
X is selected from O, NH, and NR⁷;
R¹, R², R³, R⁴, R⁵, and R⁶, at each occurrence, are independently selected from hydrogen, halogen, CN, OH, NH₂, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
A and B, at each occurrence, are independently selected from null, CO, NH, NH-CO, CO-NH, CH₂-NH-CO, CH₂-CO-NH, NH-CO-CH₂, CO-NH-CH₂, CH₂-NH-CH₂-CO-NH, CH₂-NH-CH₂-NH-CO, -CO-NH, CO-NH- CH₂-NH-CH₂, CH₂-NH-CH₂, CO₂, C(O)NR⁷, C(S)NR⁷, O, S, SO, SO₂, SO₂NR⁷, NR⁷, NR⁷CO, NR⁷CONR⁸, NR⁷C(S), optionally substituted C₁-C₈ alkyl, optionally substituted C₁-C₈ alkoxy, optionally substituted C₁-C₈alkoxyC₁-C₈alkyl, optionally substituted C₁-C₈ haloalkyl, optionally substituted C₁-C₈ hydroxyalkyl, optionally substituted C₂-C₈ alkenyl, optionally substituted C₂-C₈ alkynyl, optionally substituted 3-8 membered cycloalkyl, optionally substituted C₃-C₈ cycloalkoxy, optionally substituted 3-8 membered heterocyclyl, optionally substituted aryl, optionally substituted heteroaryl, optionally substituted C₃-C₁₃ fused cycloalkyl, optionally substituted C₃-C₁₃ fused heterocyclyl, optionally substituted C₃-C₁₃ bridged cycloalkyl, optionally substituted C₃-C₁₃ bridged heterocyclyl, optionally substituted C₃-C₁₃ spiro cycloalkyl, and optionally substituted C₃-C₁₃ spiro heterocyclyl; wherein
R⁷ and R⁸ are independently selected from hydrogen, optionally substituted C₁-C₈ alkyl, optionally substituted 3-8 membered cycloalkyl, optionally substituted C₃-C₈ cycloalkoxy, optionally substituted 3-8 membered heterocyclyl, optionally substituted C₁-C₈ alkoxy, optionally substituted C₁-C₈ alkoxyalkyl, optionally substituted C₁-C₈ haloalkyl, optionally substituted C₁-C₈ hydroxyalkyl, optionally substituted C₁-C₈ alkylamino, and optionally substituted C₁-C₈ alkylaminoC₁-C₈ alkyl;
m, at each occurrence, is 0 to 15;
n, at each occurrence, is 0 to 15;
o is 0 to 15; and
p is 0 to 15;
and pharmaceutically acceptable salts thereof; or
**(III-B)** a moiety selected from the group consisting of: a ring selected from the group consisting of a 3 to 13 membered ring; a 3 to 13 membered fused ring; a 3 to 13 membered bridged ring; and a 3 to13 membered spiro ring; and pharmaceutically acceptable salts thereof; or
(III-C) a moiety selected from the group consisting of one of FORMULAE C1, C2, C3, C4 and C5: 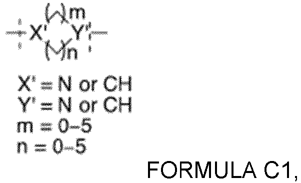 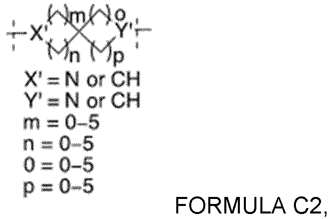 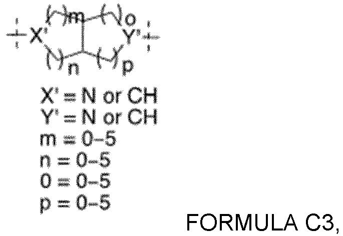 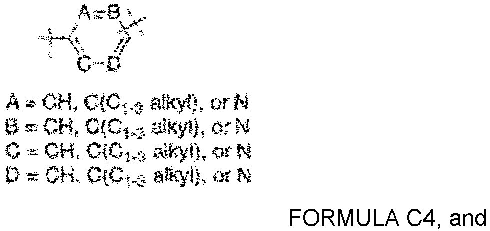 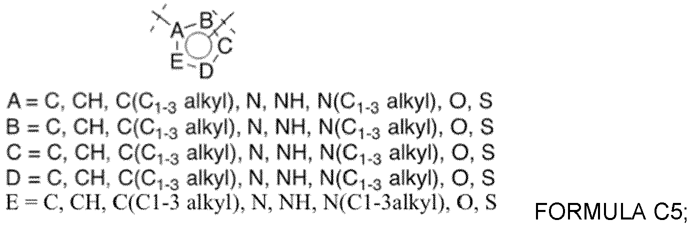 and pharmaceutically acceptable salts thereof.

2. The compound of claim 1, wherein the compound is selected from the group consisting of:
a. 2-(2,6-dioxopiperidin-3-yl)-4-((3-(4-(4-(5-(2-fluoro-6-methoxyphenyl)-1*H*-pyrazolo[4,3-d]pyrimidin-3-yl)phenyl)piperazin-1-yl)-3-oxopropyl)amino)isoindoline-1,3-dione (HC90-50); and
b. 2-(2,6-dioxopiperidin-3-yl)-4-((4-(4-(4-(5-(2-fluoro-6-methoxyphenyl)-1*H*-pyrazolo[4,3-d]pyrimidin-3-yl)phenyl)piperazin-1-yl)-4-oxobutyl)amino)isoindoline-1,3-dione (HC90-51), and
pharmaceutically acceptable salts thereof.

3. The bivalent compound of claim 1, wherein **PI** is: 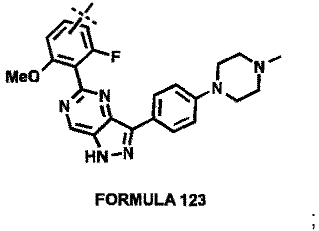 and pharmaceutically acceptable salts thereof.

4. The bivalent compound of claim 1(II-A)-(II-B), wherein **EL** is a moiety selected from the group consisting of: 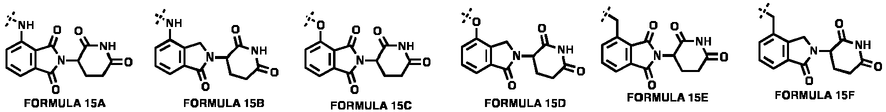 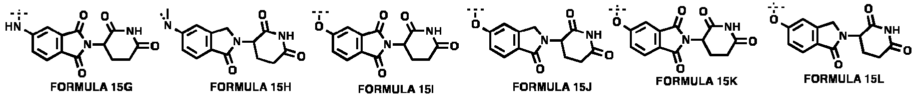 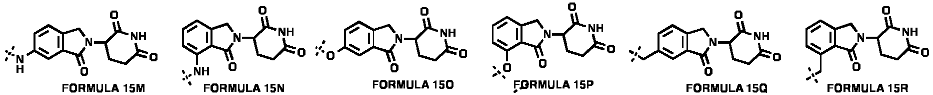 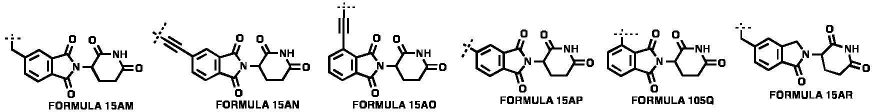 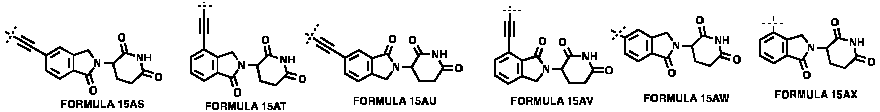 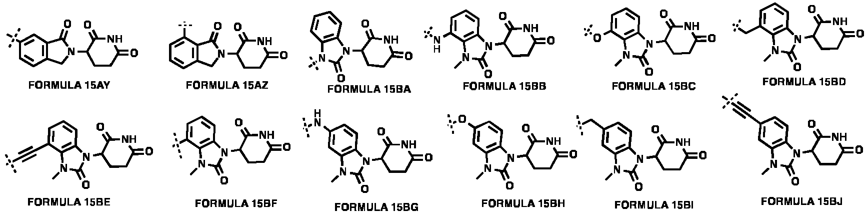 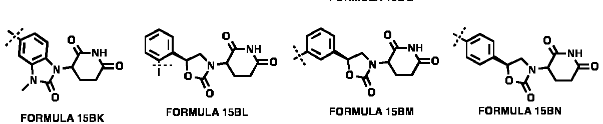 and pharmaceutically acceptable salts thereof.

5. The bivalent compound of claim 1, wherein the compound is selected from the group consisting of:
2-(2,6-dioxopiperidin-3-yl)-5-((2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethyl)amino)isoindoline-1,3-dione (HC90-33);
2-(2,6-dioxopiperidin-3-yl)-5-((2-(2-(3-(4-(4-(5-(2-fluoro-6-methoxyphenyl)-1 H-pyrazolo[4,3-d]pyrimidin-3-yl)phenyl)piperazin-1-yl)-3-oxopropoxy)ethoxy)ethyl)amino)isoindoline-1,3-dione (HC90-34);
2-(2,6-dioxopiperidin-3-yl)-5-((2-(2-(2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethoxy)ethoxy)ethyl)amino)isoindoline-1,3-dione (HC90-35);
2-(2,6-dioxopiperidin-3-yl)-5-((15-(4-(4-(5-(2-fluoro-6-methoxyphenyl)-1H-pyrazolo[4,3-d]pyrimidin-3-yl)phenyl)piperazin-1-yl)-15-oxo-3,6,9,12-tetraoxapentadecyl)amino)isoindoline-1,3-dione (HC90-36);
2-(2,6-dioxopiperidin-3-yl)-5-((18-(4-(4-(5-(2-fluoro-6-methoxyphenyl)-1H-pyrazolo[4,3-d]pyrimidin-3-yl)phenyl)piperazin-1-yl)-18-oxo-3,6,9,12,15-pentaoxaoctadecyl)amino)isoindoline-1,3-dione (HC90-37);
2-(2,6-dioxopiperidin-3-yl)-5-((2-(4-(4-(5-(2-fluoro-6-methoxyphenyl)-1H-pyrazolo[4,3-d]pyrimidin-3-yl)phenyl)piperazin-1-yl)-2-oxoethyl)amino)isoindoline-1,3-dione (HC90-41);
2-(2,6-dioxopiperidin-3-yl)-5-((3-(4-(4-(5-(2-fluoro-6-methoxyphenyl)-1H-pyrazolo[4,3-d]pyrimidin-3-yl)phenyl)piperazin-1-yl)-3-oxopropyl)amino)isoindoline-1,3-dione (HC90-42);
2-(2,6-dioxopiperidin-3-yl)-5-((4-(4-(4-(5-(2-fluoro-6-methoxyphenyl)-1H-pyrazolo[4,3-d]pyrimidin-3-yl)phenyl)piperazin-1-yl)-4-oxobutyl)amino)isoindoline-1,3-dione (HC90-43);
2-(2,6-dioxopiperidin-3-yl)-5-((5-(4-(4-(5-(2-fluoro-6-methoxyphenyl)-1H-pyrazolo[4,3-d]pyrimidin-3-yl)phenyl)piperazin-1-yl)-5-oxopentyl)amino)isoindoline-1,3-dione (HC90-44);
2-(2,6-dioxopiperidin-3-yl)-5-((6-(4-(4-(5-(2-fluoro-6-methoxyphenyl)-1H-pyrazolo[4,3-d]pyrimidin-3-yl)phenyl)piperazin-1-yl)-6-oxohexyl)amino)isoindoline-1,3-dione (HC90-45);
2-(2,6-dioxopiperidin-3-yl)-5-((7-(4-(4-(5-(2-fluoro-6-methoxyphenyl)-1H-pyrazolo[4,3-d]pyrimidin-3-yl)phenyl)piperazin-1-yl)-7-oxoheptyl)amino)isoindoline-1,3-dione (HC90-46);
2-(2,6-dioxopiperidin-3-yl)-5-((8-(4-(4-(5-(2-fluoro-6-methoxyphenyl)-1H-pyrazolo[4,3-d]pyrimidin-3-yl)phenyl)piperazin-1-yl)-8-oxooctyl)amino)isoindoline-1,3-dione (HC90-47);
2-(2,6-dioxopiperidin-3-yl)-4-((2-(4-(4-(5-(2-fluoro-6-methoxyphenyl)-1H-pyrazolo[4,3-d]pyrimidin-3-yl)phenyl)piperazin-1-yl)-2-oxoethyl)amino)isoindoline-1,3-dione (HC90-49);
2-(2,6-dioxopiperidin-3-yl)-4-((3-(4-(4-(5-(2-fluoro-6-methoxyphenyl)-1H-pyrazolo[4,3-d]pyrimidin-3-yl)phenyl)piperazin-1-yl)-3-oxopropyl)amino)isoindoline-1,3-dione (HC90-50);
2-(2,6-dioxopiperidin-3-yl)-4-((4-(4-(4-(5-(2-fluoro-6-methoxyphenyl)-1H-pyrazolo[4,3-d]pyrimidin-3-yl)phenyl)piperazin-1-yl)-4-oxobutyl)amino)isoindoline-1,3-dione (HC90-51);
2-(2,6-dioxopiperidin-3-yl)-4-((5-(4-(4-(5-(2-fluoro-6-methoxyphenyl)-1H-pyrazolo[4,3-d]pyrimidin-3-yl)phenyl)piperazin-1-yl)-5-oxopentyl)amino)isoindoline-1,3-dione (HC90-52);
2-(2,6-dioxopiperidin-3-yl)-4-((6-(4-(4-(5-(2-fluoro-6-methoxyphenyl)-1H-pyrazolo[4,3-d]pyrimidin-3-yl)phenyl)piperazin-1-yl)-6-oxohexyl)amino)isoindoline-1,3-dione (HC90-53);_
2-(2,6-dioxopiperidin-3-yl)-4-((7-(4-(4-(5-(2-fluoro-6-methoxyphenyl)-1H-pyrazolo[4,3-d]pyrimidin-3-yl)phenyl)piperazin-1-yl)-7-oxoheptyl)amino)isoindoline-1,3-dione (HC90-54);
2-(2,6-dioxopiperidin-3-yl)-4-((2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethyl)amino)isoindoline-1,3-dione (HC90-55);
2-(2,6-dioxopiperidin-3-yl)-4-((2-(2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethoxy)ethyl)amino)isoindoline-1,3-dione (HC90-56);
2-(2,6-dioxopiperidin-3-yl)-4-((2-(2-(2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethoxy)ethoxy)ethyl)amino)isoindoline-1,3-dione (HC90-57);
2-(2,6-dioxopiperidin-3-yl)-4-((15-(4-(4-(5-(2-fluoro-6-methoxyphenyl)-1H-pyrazolo[4,3-d]pyrimidin-3-yl)phenyl)piperazin-1-yl)-15-oxo-3,6,9,12-tetraoxapentadecyl)amino)isoindoline-1,3-dione (HC90-58);
2-(2,6-dioxopiperidin-3-yl)-4-((18-(4-(4-(5-(2-fluoro-6-methoxyphenyl)-1H-pyrazolo[4,3-d]pyrimidin-3-yl)phenyl)piperazin-1-yl)-18-oxo-3,6,9,12,15-pentaoxaoctadecyl)amino)isoindoline-1,3-dione (HC90-59);
(2S,4R)-1-((S)-2-(3-(3-(4-(4-(5-(2-fluoro-6-methoxyphenyl)-1H-pyrazolo[4,3-d]pyrimidin-3-yl)phenyl)piperazin-1-yl)-3-oxopropoxy)propanamido)-3,3-dimethylbutanoyl)-4-hydroxy-N-(4-(4-methylthiazol-5-yl)benzyl)pyrrolidine-2-carboxamide (HC90-61);
(2S,4R)-1-((S)-2-(tert-butyl)-19-(4-(4-(5-(2-fluoro-6-methoxyphenyl)-1H-pyrazolo[4,3-d]pyrimidin-3-yl)phenyl)piperazin-1-yl)-4,19-dioxo-7,10,13,16-tetraoxa-3-azanonadecanoyl)-4-hydroxy-N-(4-(4-methylthiazol-5-yl)benzyl)pyrrolidine-2-carboxamide (HC90-66);
(2S,4R)-1-((S)-2-(4-(4-(4-(5-(2-fluoro-6-methoxyphenyl)-1H-pyrazolo[4,3-d]pyrimidin-3-yl)phenyl)piperazin-1-yl)-4-oxobutanamido)-3,3-dimethylbutanoyl)-4-hydroxy-N-(4-(4-methylthiazol-5-yl)benzyl)pyrrolidine-2-carboxamide (HC90-69);
(2S,4R)-1-((S)-2-(5-(4-(4-(5-(2-fluoro-6-methoxyphenyl)-1H-pyrazolo[4,3-d]pyrimidin-3-yl)phenyl)piperazin-1-yl)-5-oxopentanamido)-3,3-dimethylbutanoyl)-4-hydroxy-N-(4-(4-methylthiazol-5-yl)benzyl)pyrrolidine-2-carboxamide (HC90-70);
(2S,4R)-1-((S)-2-(6-(4-(4-(5-(2-fluoro-6-methoxyphenyl)-1H-pyrazolo[4,3-d]pyrimidin-3-yl)phenyl)piperazin-1-yl)-6-oxohexanamido)-3,3-dimethylbutanoyl)-4-hydroxy-N-(4-(4-methylthiazol-5-yl)benzyl)pyrrolidine-2-carboxamide (HC90-71);
(2S,4R)-1-((S)-2-(7-(4-(4-(5-(2-fluoro-6-methoxyphenyl)-1H-pyrazolo[4,3-d]pyrimidin-3-yl)phenyl)piperazin-1-yl)-7-oxoheptanamido)-3,3-dimethylbutanoyl)-4-hydroxy-N-(4-(4-methylthiazol-5-yl)benzyl)pyrrolidine-2-carboxamide (HC90-72);
(2S,4R)-1-((S)-2-(8-(4-(4-(5-(2-fluoro-6-methoxyphenyl)-1H-pyrazolo[4,3-d]pyrimidin-3-yl)phenyl)piperazin-1-yl)-8-oxooctanamido)-3,3-dimethylbutanoyl)-4-hydroxy-N-(4-(4-methylthiazol-5-yl)benzyl)pyrrolidine-2-carboxamide (HC90-73);
(2S,4R)-1-((S)-2-(10-(4-(4-(5-(2-fluoro-6-methoxyphenyl)-1H-pyrazolo[4,3-d]pyrimidin-3-yl)phenyl)piperazin-1-yl)-10-oxodecanamido)-3,3-dimethylbutanoyl)-4-hydroxy-N-(4-(4-methylthiazol-5-yl)benzyl)pyrrolidine-2-carboxamide (HC90-74);
(2S,4R)-1-((S)-2-(9-(4-(4-(5-(2-fluoro-6-methoxyphenyl)-1H-pyrazolo[4,3-d]pyrimidin-3-yl)phenyl)piperazin-1-yl)-9-oxononanamido)-3,3-dimethylbutanoyl)-4-hydroxy-N-(4-(4-methylthiazol-5-yl)benzyl)pyrrolidine-2-carboxamide (HC90-84);
(2S,4R)-1-((S)-2-(11-(4-(4-(5-(2-fluoro-6-methoxyphenyl)-1H-pyrazolo[4,3-d]pyrimidin-3-yl)phenyl)piperazin-1-yl)-11-oxoundecanamido)-3,3-dimethylbutanoyl)-4-hydroxy-N-(4-(4-methylthiazol-5-yl)benzyl)pyrrolidine-2-carboxamide (HC90-85);
N-(2-((2-(2,6-dioxopiperidin-3-yl)-1,3-dioxoisoindolin-5-yl)amino)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-86);
N-(3-((2-(2,6-dioxopiperidin-3-yl)-1,3-dioxoisoindolin-5-yl)amino)propyl)-2-(4-(4-(5-(2-fluoro-6-methoxyphenyl)-1H-pyrazolo[4,3-d]pyrimidin-3-yl)phenyl)piperazin-1-yl)acetamide (HC90-87);
N-(4-((2-(2,6-dioxopiperidin-3-yl)-1,3-dioxoisoindolin-5-yl)amino)butyl)-2-(4-(4-(5-(2-fluoro-6-methoxyphenyl)-1H-pyrazolo[4,3-d]pyrimidin-3-yl)phenyl)piperazin-1-yl)acetamide (HC90-88);
N-(5-((2-(2,6-dioxopiperidin-3-yl)-1,3-dioxoisoindolin-5-yl)amino)pentyl)-2-(4-(4-(5-(2-fluoro-6-methoxyphenyl)-1H-pyrazolo[4,3-d]pyrimidin-3-yl)phenyl)piperazin-1-yl)acetamide (HC90-89);
N-(6-((2-(2,6-dioxopiperidin-3-yl)-1,3-dioxoisoindolin-5-yl)amino)hexyl)-2-(4-(4-(5-(2-fluoro-6-methoxyphenyl)-1H-pyrazolo[4,3-d]pyrimidin-3-yl)phenyl)piperazin-1-yl)acetamide (HC90-90);
N-(7-((2-(2,6-dioxopiperidin-3-yl)-1,3-dioxoisoindolin-5-yl)amino)heptyl)-2-(4-(4-(5-(2-fluoro-6-methoxyphenyl)-1H-pyrazolo[4,3-d]pyrimidin-3-yl)phenyl)piperazin-1-yl)acetamide (HC90-91);
N-(8-((2-(2,6-dioxopiperidin-3-yl)-1,3-dioxoisoindolin-5-yl)amino)octyl)-2-(4-(4-(5-(2-fluoro-6-methoxyphenyl)-1H-pyrazolo[4,3-d]pyrimidin-3-yl)phenyl)piperazin-1-yl)acetamide (HC90-92);
N-(2-(2-((2-(2,6-dioxopiperidin-3-yl)-1,3-dioxoisoindolin-5-yl)amino)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-93);
N-(2-(2-(2-((2-(2,6-dioxopiperidin-3-yl)-1,3-dioxoisoindolin-5-yl)amino)ethoxy)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-94);
N-(2-(2-(2-(2-((2-(2,6-dioxopiperidin-3-yl)-1,3-dioxoisoindolin-5-yl)amino)ethoxy)ethoxy)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-95);
N-(14-((2-(2,6-dioxopiperidin-3-yl)-1,3-dioxoisoindolin-5-yl)amino)-3,6,9,12-tetraoxatetradecyl)-2-(4-(4-(5-(2-fluoro-6-methoxyphenyl)-1H-pyrazolo[4,3-d]pyrimidin-3-yl)phenyl)piperazin-1-yl)acetamide (HC90-96);
N-(17-((2-(2,6-dioxopiperidin-3-yl)-1,3-dioxoisoindolin-5-yl)amino)-3,6,9,12,15-pentaoxaheptadecyl)-2-(4-(4-(5-(2-fluoro-6-methoxyphenyl)-1H-pyrazolo[4,3-d]pyrimidin-3-yl)phenyl)piperazin-1-yl)acetamide (HC90-97);
N-(2-((2-(2,6-dioxopiperidin-3-yl)-1,3-dioxoisoindolin-4-yl)amino)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-102);
N-(3-((2-(2,6-dioxopiperidin-3-yl)-1,3-dioxoisoindolin-4-yl)amino)propyl)-2-(4-(4-(5-(2-fluoro-6-methoxyphenyl)-1H-pyrazolo[4,3-d]pyrimidin-3-yl)phenyl)piperazin-1-yl)acetamide (HC90-103);
N-(4-((2-(2,6-dioxopiperidin-3-yl)-1,3-dioxoisoindolin-4-yl)amino)butyl)-2-(4-(4-(5-(2-fluoro-6-methoxyphenyl)-1H-pyrazolo[4,3-d]pyrimidin-3-yl)phenyl)piperazin-1-yl)acetamide (HC90-104);
N-(5-((2-(2,6-dioxopiperidin-3-yl)-1,3-dioxoisoindolin-4-yl)amino)pentyl)-2-(4-(4-(5-(2-fluoro-6-methoxyphenyl)-1H-pyrazolo[4,3-d]pyrimidin-3-yl)phenyl)piperazin-1-yl)acetamide (HC90-105);
N-(6-((2-(2,6-dioxopiperidin-3-yl)-1,3-dioxoisoindolin-4-yl)amino)hexyl)-2-(4-(4-(5-(2-fluoro-6-methoxyphenyl)-1H-pyrazolo[4,3-d]pyrimidin-3-yl)phenyl)piperazin-1-yl)acetamide (HC90-106);
N-(7-((2-(2,6-dioxopiperidin-3-yl)-1,3-dioxoisoindolin-4-yl)amino)heptyl)-2-(4-(4-(5-(2-fluoro-6-methoxyphenyl)-1H-pyrazolo[4,3-d]pyrimidin-3-yl)phenyl)piperazin-1-yl)acetamide (HC90-107);
N-(8-((2-(2,6-dioxopiperidin-3-yl)-1,3-dioxoisoindolin-4-yl)amino)octyl)-2-(4-(4-(5-(2-fluoro-6-methoxyphenyl)-1H-pyrazolo[4,3-d]pyrimidin-3-yl)phenyl)piperazin-1-yl)acetamide (HC90-108);
N-(2-(2-((2-(2,6-dioxopiperidin-3-yl)-1,3-dioxoisoindolin-4-yl)amino)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-109);
N-(2-(2-(2-((2-(2,6-dioxopiperidin-3-yl)-1,3-dioxoisoindolin-4-yl)amino)ethoxy)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-110);
N-(2-(2-(2-(2-((2-(2,6-dioxopiperidin-3-yl)-1,3-dioxoisoindolin-4-yl)amino)ethoxy)ethoxy)ethoxy)ethyl)-2-(4-(4-(5-(2-fluoro-6-methoxyphenyl)-1H-pyrazolo[4,3-d]pyrimidin-3-yl)phenyl)piperazin-1-yl)acetamide (HC90-111);
N-(14-((2-(2,6-dioxopiperidin-3-yl)-1,3-dioxoisoindolin-4-yl)amino)-3,6,9,12-tetraoxatetradecyl)-2-(4-(4-(5-(2-fluoro-6-methoxyphenyl)-1H-pyrazolo[4,3-d]pyrimidin-3-yl)phenyl)piperazin-1-yl)acetamide (HC90-112);
N-(17-((2-(2,6-dioxopiperidin-3-yl)-1,3-dioxoisoindolin-4-yl)amino)-3,6,9,12,15-pentaoxaheptadecyl)-2-(4-(4-(5-(2-fluoro-6-methoxyphenyl)-1H-pyrazolo[4,3-d]pyrimidin-3-yl)phenyl)piperazin-1-yl)acetamide (HC90-113);
2-(2,6-dioxopiperidin-3-yl)-4-((8-(4-(4-(5-(2-fluoro-6-methoxyphenyl)-1H-pyrazolo[4,3-d]pyrimidin-3-yl)phenyl)piperazin-1-yl)-8-oxooctyl)amino)isoindoline-1,3-dione (HC90-117);
(2S,4R)-1-((S)-2-(2-(2-(2-(4-(4-(5-(2-fluoro-6-methoxyphenyl)-1H-pyrazolo[4,3-d]pyrimidin-3-yl)phenyl)piperazin-1-yl)acetamido)ethoxy)acetamido)-3,3-dimethylbutanoyl)-4-hydroxy-N-(4-(4-methylthiazol-5-yl)benzyl)pyrrolidine-2-carboxamide (HC90-119);
(2S,4R)-1-((S)-2-(3-(2-(2-(4-(4-(5-(2-fluoro-6-methoxyphenyl)-1H-pyrazolo[4,3-d]pyrimidin-3-yl)phenyl)piperazin-1-yl)acetamido)ethoxy)propanamido)-3,3-dimethylbutanoyl)-4-hydroxy-N-(4-(4-methylthiazol-5-yl)benzyl)pyrrolidine-2-carboxamide (HC90-120);
(2S,4R)-1-((S)-2-(tert-butyl)-14-(4-(4-(5-(2-fluoro-6-methoxyphenyl)-1H-pyrazolo[4,3-d]pyrimidin-3-yl)phenyl)piperazin-1-yl)-4,13-dioxo-6,9-dioxa-3,12-diazatetradecanoyl)-4-hydroxy-N-(4-(4-methylthiazol-5-yl)benzyl)pyrrolidine-2-carboxamide (HC90-121);
(2S,4R)-1-((S)-14-(tert-butyl)-1-(4-(4-(5-(2-fluoro-6-methoxyphenyl)-1H-pyrazolo[4,3-d]pyrimidin-3-yl)phenyl)piperazin-1-yl)-2,12-dioxo-6,9-dioxa-3,13-diazapentadecan-15-oyl)-4-hydroxy-N-(4-(4-methylthiazol-5-yl)benzyl)pyrrolidine-2-carboxamide (HC90-122);
(2S,4R)-1-((S)-2-(tert-butyl)-17-(4-(4-(5-(2-fluoro-6-methoxyphenyl)-1H-pyrazolo[4,3-d]pyrimidin-3-yl)phenyl)piperazin-1-yl)-4,16-dioxo-6,9,12-trioxa-3,15-diazaheptadecanoyl)-4-hydroxy-N-(4-(4-methylthiazol-5-yl)benzyl)pyrrolidine-2-carboxamide (HC90-123);
(2S,4R)-1-((S)-17-(tert-butyl)-1-(4-(4-(5-(2-fluoro-6-methoxyphenyl)-1H-pyrazolo[4,3-d]pyrimidin-3-yl)phenyl)piperazin-1-yl)-2,15-dioxo-6,9,12-trioxa-3,16-diazaoctadecan-18-oyl)-4-hydroxy-N-(4-(4-methylthiazol-5-yl)benzyl)pyrrolidine-2-carboxamide (HC90-124);
(2S,4R)-1-((S)-20-(tert-butyl)-1-(4-(4-(5-(2-fluoro-6-methoxyphenyl)-1H-pyrazolo[4,3-d]pyrimidin-3-yl)phenyl)piperazin-1-yl)-2,18-dioxo-6,9,12,15-tetraoxa-3,19-diazahenicosan-21-oyl)-4-hydroxy-N-(4-(4-methylthiazol-5-yl)benzyl)pyrrolidine-2-carboxamide (HC90-125);
(2S,4R)-1-((S)-23-(tert-butyl)-1-(4-(4-(5-(2-fluoro-6-methoxyphenyl)-1H-pyrazolo[4,3-d]pyrimidin-3-yl)phenyl)piperazin-1-yl)-2,21-dioxo-6,9,12,15,18-pentaoxa-3,22-diazatetracosan-24-oyl)-4-hydroxy-N-(4-(4-methylthiazol-5-yl)benzyl)pyrrolidine-2-carboxamide (HC90-126);
(2S,4R)-1-((S)-2-(2-(2-(4-(4-(5-(2-fluoro-6-methoxyphenyl)-1H-pyrazolo[4,3-d]pyrimidin-3-yl)phenyl)piperazin-1-yl)acetamido)acetamido)-3,3-dimethylbutanoyl)-4-hydroxy-N-(4-(4-methylthiazol-5-yl)benzyl)pyrrolidine-2-carboxamide (HC90-127);
(2S,4R)-1-((S)-2-(3-(2-(4-(4-(5-(2-fluoro-6-methoxyphenyl)-1H-pyrazolo[4,3-d]pyrimidin-3-yl)phenyl)piperazin-1-yl)acetamido)propanamido)-3,3-dimethylbutanoyl)-4-hydroxy-N-(4-(4-methylthiazol-5-yl)benzyl)pyrrolidine-2-carboxamide (HC90-128);
(2S,4R)-1-((S)-2-(4-(2-(4-(4-(5-(2-fluoro-6-methoxyphenyl)-1H-pyrazolo[4,3-d]pyrimidin-3-yl)phenyl)piperazin-1-yl)acetamido)butanamido)-3,3-dimethylbutanoyl)-4-hydroxy-N-(4-(4-methylthiazol-5-yl)benzyl)pyrrolidine-2-carboxamide (HC90-129);
(2S,4R)-1-((S)-2-(5-(2-(4-(4-(5-(2-fluoro-6-methoxyphenyl)-1H-pyrazolo[4,3-d]pyrimidin-3-yl)phenyl)piperazin-1-yl)acetamido)pentanamido)-3,3-dimethylbutanoyl)-4-hydroxy-N-(4-(4-methylthiazol-5-yl)benzyl)pyrrolidine-2-carboxamide (HC90-130);
(2S,4R)-1-((S)-2-(6-(2-(4-(4-(5-(2-fluoro-6-methoxyphenyl)-1H-pyrazolo[4,3-d]pyrimidin-3-yl)phenyl)piperazin-1-yl)acetamido)hexanamido)-3,3-dimethylbutanoyl)-4-hydroxy-N-(4-(4-methylthiazol-5-yl)benzyl)pyrrolidine-2-carboxamide (HC90-131);
(2S,4R)-1-((S)-2-(7-(2-(4-(4-(5-(2-fluoro-6-methoxyphenyl)-1H-pyrazolo[4,3-d]pyrimidin-3-yl)phenyl)piperazin-1-yl)acetamido)heptanamido)-3,3-dimethylbutanoyl)-4-hydroxy-N-(4-(4-methylthiazol-5-yl)benzyl)pyrrolidine-2-carboxamide (HC90-132);
(2S,4R)-1-((S)-2-(8-(2-(4-(4-(5-(2-fluoro-6-methoxyphenyl)-1H-pyrazolo[4,3-d]pyrimidin-3-yl)phenyl)piperazin-1-yl)acetamido)octanamido)-3,3-dimethylbutanoyl)-4-hydroxy-N-(4-(4-methylthiazol-5-yl)benzyl)pyrrolidine-2-carboxamide (HC90-133);
(2S,4R)-1-((S)-2-(9-(2-(4-(4-(5-(2-fluoro-6-methoxyphenyl)-1H-pyrazolo[4,3-d]pyrimidin-3-yl)phenyl)piperazin-1-yl)acetamido)nonanamido)-3,3-dimethylbutanoyl)-4-hydroxy-N-(4-(4-methylthiazol-5-yl)benzyl)pyrrolidine-2-carboxamide (HC90-134);
(2S,4R)-1-((S)-2-(10-(2-(4-(4-(5-(2-fluoro-6-methoxyphenyl)-1H-pyrazolo[4,3-d]pyrimidin-3-yl)phenyl)piperazin-1-yl)acetamido)decanamido)-3,3-dimethylbutanoyl)-4-hydroxy-N-(4-(4-methylthiazol-5-yl)benzyl)pyrrolidine-2-carboxamide (HC90-135);
(2S,4R)-1-((S)-2-(11-(2-(4-(4-(5-(2-fluoro-6-methoxyphenyl)-1H-pyrazolo[4,3-d]pyrimidin-3-yl)phenyl)piperazin-1-yl)acetamido)undecanamido)-3,3-dimethylbutanoyl)-4-hydroxy-N-(4-(4-methylthiazol-5-yl)benzyl)pyrrolidine-2-carboxamide (HC90-136);
(2S,4R)-1-((S)-2-(2-(2-(4-(4-(5-(2-fluoro-6-methoxyphenyl)-1H-pyrazolo[4,3-d]pyrimidin-3-yl)phenyl)piperazin-1-yl)-2-oxoethoxy)acetamido)-3,3-dimethylbutanoyl)-4-hydroxy-N-(4-(4-methylthiazol-5-yl)benzyl)pyrrolidine-2-carboxamide (HC90-60);
(2S,4R)-1-((S)-2-(2-(2-(2-(4-(4-(5-(2-fluoro-6-methoxyphenyl)-1H-pyrazolo[4,3-d]pyrimidin-3-yl)phenyl)piperazin-1-yl)-2-oxoethoxy)ethoxy)acetamido)-3,3-dimethylbutanoyl)-4-hydroxy-N-(4-(4-methylthiazol-5-yl)benzyl)pyrrolidine-2-carboxamide (HC90-62);
(2S,4R)-1-((S)-2-(3-(2-(3-(4-(4-(5-(2-fluoro-6-methoxyphenyl)-1H-pyrazolo[4,3-d]pyrimidin-3-yl)phenyl)piperazin-1-yl)-3-oxopropoxy)ethoxy)propanamido)-3,3-dimethylbutanoyl)-4-hydroxy-N-(4-(4-methylthiazol-5-yl)benzyl)pyrrolidine-2-carboxamide (HC90-63);
(2S,4R)-1-((S)-2-(tert-butyl)-14-(4-(4-(5-(2-fluoro-6-methoxyphenyl)-1H-pyrazolo[4,3-d]pyrimidin-3-yl)phenyl)piperazin-1-yl)-4,14-dioxo-6,9,12-trioxa-3-azatetradecanoyl)-4-hydroxy-N-(4-(4-methylthiazol-5-yl)benzyl)pyrrolidine-2-carboxamide (HC90-64);
(2S,4R)-1-((S)-2-(tert-butyl)-16-(4-(4-(5-(2-fluoro-6-methoxyphenyl)-1H-pyrazolo[4,3-d]pyrimidin-3-yl)phenyl)piperazin-1-yl)-4,16-dioxo-7,10,13-trioxa-3-azahexadecanoyl)-4-hydroxy-N-(4-(4-methylthiazol-5-yl)benzyl)pyrrolidine-2-carboxamide (HC90-65);
(2S,4R)-1-((S)-2-(tert-butyl)-20-(4-(4-(5-(2-fluoro-6-methoxyphenyl)-1H-pyrazolo[4,3-d]pyrimidin-3-yl)phenyl)piperazin-1-yl)-4,20-dioxo-6,9,12,15,18-pentaoxa-3-azaicosanoyl)-4-hydroxy-N-(4-(4-methylthiazol-5-yl)benzyl)pyrrolidine-2-carboxamide (HC90-67); and
(2S,4R)-1-((S)-2-(tert-butyl)-22-(4-(4-(5-(2-fluoro-6-methoxyphenyl)-1H-pyrazolo[4,3-d]pyrimidin-3-yl)phenyl)piperazin-1-yl)-4,22-dioxo-7,10,13,16,19-pentaoxa-3-azadocosanoyl)-4-hydroxy-N-(4-(4-methylthiazol-5-yl)benzyl)pyrrolidine-2-carboxamide (HC90-68);
and pharmaceutically acceptable salts thereof.

6. The bivalent compound of any one of claims 1-5 for use in a method of treating an HPK1-mediated disease in a subject in need thereof,
preferably the HPK1-mediated disease is selected from the group consisting of cancer, chronic infections that produce exhausted immune response, infection-mediated immune suppression, age-related decline in immune response, age-related decline in cognitive function and infertility, more preferably the cancer is selected from the group consisting of cancers of the breast, respiratory tract, brain, reproductive organs, digestive tract, urinary tract, eye, liver, skin, head and neck, thyroid, parathyroid and their distant metastases, lymphomas, sarcomas, and leukaemias.

7. A pharmaceutical composition including the bivalent compound of any one of claims 1-5 for use in a method of treating an HPK1-mediated disease in a subject in need thereof.

8. The bivalent compound according to any one of claims 1-5 for use in a method of reducing HPK1 expression in cells, wherein the method comprises:
isolating cells from the body of a subject;
treating the cells with the bivalent compound; and
reintroducing the treated cells to the body of the subject,
preferably
**(i)** the cells are selected from the group consisting of autologous bone marrow cells, cord blood stem cells and peripheral blood or bone marrow stem cells, or
**(ii)** the cells are immune cells selected from the group consisting of T cells, genetically engineered T cells, Chimeric Antigen Receptor (CAR) T cells, tumor infiltrating lymphocytes, dendritic cells, macrophage, mast cells, granulocytes (include basophils, eosinophils, and neutrophils), natural killer cells, NK T cells and B cells.

9. The bivalent compound of any one of claims 1-5 for use in a method for increasing secretion of interleukin-2 from T cells, wherein the method comprises:
isolating T cells from the body of a subject;
treating the T cells with the bivalent compound; and
reintroducing the T cells into the body of the subject.

10. A pharmaceutical composition including the bivalent compound of any one of claims 1-5 for use in a method of treating cancer in a subject in need thereof, wherein the method comprises:
administering to a subject having cancer,
the pharmaceutical composition and a pharmaceutically acceptable carrier, and
a pharmaceutical composition including a checkpoint inhibitor and a pharmaceutically acceptable carrier.

11. The pharmaceutical composition for use according to claim 10, wherein the checkpoint inhibitor is selected from the group consisting of anti-PD-1, anti-PD-L1 and anti-CTLA-4.

12. The pharmaceutical composition for use according to claim 11,
**(i)** wherein the checkpoint inhibitor is anti-PD-1, preferably the anti-PD-1 is selected from the group consisting of pembrolizumab, nivolumab and cemiplimab, more preferably the anti-PD-1 is selected from the group consisting of pembrolizumab, nivolumab and cemiplimab and the cancer is selected from the group consisting of melanoma, lung cancer, renal cell carcinoma, Hodgkin lymphoma, head and neck cancer, colon cancer and liver cancer;
**(ii)** wherein the checkpoint inhibitor is anti-PD-L1, preferably the anti-PD-L1 is selected from the group consisting of atezolizumab, avelumab and durvalumab, more preferably the anti-PD-L1 is selected from the group consisting of atezolizumab, avelumab and durvalumab and the cancer is selected from the group consisting of non-small cell lung carcinoma, multiple myeloma, urothelial cancer and head and neck cancer; or
**(iii)** wherein the checkpoint inhibitor is anti-CDLA-4, preferably the anti-CDLA-4 is selected from the group consisting of pembrolizumab, nivolumab and cemiplimab, more preferably the anti-CDLA-4 is selected from the group consisting of pembrolizumab, nivolumab and cemiplimab and the cancer is selected from the group consisting of melanoma, lung cancer, renal cell carcinoma, glioblastoma, hepatocellular carcinoma large B cell lymphoma, Hodgkin lymphoma, head and neck cancer, colon cancer and liver cancer.

13. A pharmaceutical composition including the bivalent compound of any one of claims 1-5 for use in a method of treating cancer with an elevated expression of cyclooxygenase-2 in a subject in need thereof, wherein the method comprises:
administering to a subject having cancer with an elevated expression of
cyclooxygenase-2 the pharmaceutical composition and a pharmaceutically acceptable carrier,
preferably the cancer is selected from the group consisting of colon cancer, lung cancer, sarcoma and breast cancer.

14. The pharmaceutical composition for use according to claim 10, wherein the two compositions are administered simultaneously or sequentially.

## Patentansprüche

1. Bivalente Verbindung, umfassend einen Liganden der hämatopoetischen Vorläufer-Kinase 1 (HPK1), der mit einer Abbau-/Störungs-Markierung konjugiert ist, wobei die bivalente Verbindung folgende Struktur hat: 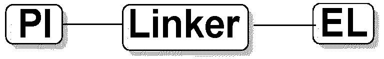
**(I)** wobei **PI** einen HPK1-Liganden und **EL** eine Abbau-/Störungs-Markierung umfasst, wobei **PI** ein Rest ist, der ausgewählt ist aus einer Gruppe bestehend aus:
**(I-A)** ein Rest gemäß FORMEL 3L: 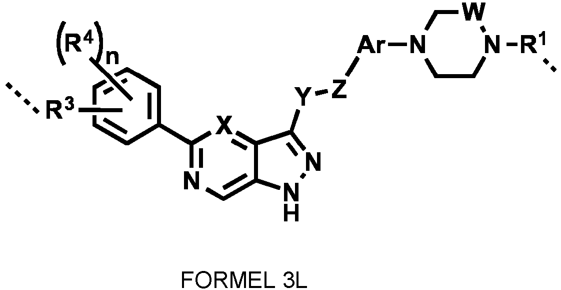 wobei
der "Linker"-Rest der bivalenten Verbindung unabhängig an R¹ oder R³ gebunden ist;
X ausgewählt ist aus CR² oder N;
R¹ und R³ unabhängig ausgewählt sind aus Null, Wasserstoff, C(O)R⁶, C(O)OR⁶, C(O)NR⁶R⁷, S(O)R⁶, S(O)₂R⁶, S(O)₂NR⁶R⁷, NR⁸C(O)OR⁶, NR⁸C(O)R⁶, NR⁸C(O)NR⁶R⁷, NR⁸S(O)R⁶, NR⁸S(O)₂R⁶, NR⁸S(O)₂NR⁶R⁷ , gegebenenfalls substituiertem C₁-C₈-Alkyl, gegebenenfalls substituiertem C₂-C₈-Alkenyl, gegebenenfalls substituiertem C₂-C₈-Alkinyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-bis 8-gliedrigem Cycloalkyl, gegebenenfalls substituiertem C₃-C₈-Cycloalkoxy, gegebenenfalls substituiertem 3- bis 8-gliedrigem Heterocyclyl, gegebenenfalls substituiertem Aryl, und gegebenenfalls substituiertem Heteroaryl; wobei
R⁶ Null ist oder ein bivalenter Rest, der ausgewählt ist aus gegebenenfalls substituiertem C₁-C₈-Alkyl, gegebenenfalls substituiertem C₂-C₈-Alkenyl, gegebenenfalls substituiertem C₂-C₈-Alkinyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, und gegebenenfalls substituiertem Heteroaryl;
R⁷ und R⁸ unabhängig voneinander ausgewählt sind aus Wasserstoff, gegebenenfalls substituiertem C₁-C₈-Alkyl, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem C₃-C_{1O}-Cycloalkyl, gegebenenfalls substituiertem 3-20-gliedrigem Heterocyclyl, gegebenenfalls substituiertem C₂-C₈-Alkenyl, gegebenenfalls substituiertem C₂-C₈-Alkinyl, gegebenenfalls substituiertem Aryl, und gegebenenfalls substituiertem Heteroaryl; oder
R⁶ und R⁷ gemeinsam mit dem Atom, an welches sie gebunden sind, einen gegebenenfalls substituierten 3-20-gliedrigen Cycloalkyl- oder Heterocyclyl-Ring bilden;
R² unabhängig ausgewählt ist aus Wasserstoff, Halogen, Oxo, CN, NO₂, OR⁹, SR⁹, NR⁹R¹⁰, C(O)R⁹, C(O)OR⁹, C(O)NR⁹R¹⁰, S(O)R⁹, S(O)₂R⁹, S(O)₂NR⁹R¹⁰, NR¹¹C(O)OR⁹, NR¹¹C(O)R⁹, NR¹¹C(O)NR⁹R¹⁰, NR¹¹S(O)R⁹, NR¹¹S(O)₂R⁹, NR¹¹S(O)₂NR⁹R¹⁰ , gegebenenfalls substituiertem C₁-C₈-Alkyl, gegebenenfalls substituiertem C₂-C₈-Alkenyl, gegebenenfalls substituiertem C₂-C₈-Alkinyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, und gegebenenfalls substituiertem Heteroaryl; wobei
R⁹, R¹⁰, und R¹¹ unabhängig ausgewählt sind aus Wasserstoff, gegebenenfalls substituiertem C₁-C₈-Alkyl, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem C₃-C₁₀-Cycloalkyl, gegebenenfalls substituiertem 3-20-gliedrigem Heterocyclyl, gegebenenfalls substituiertem C₂-C₈-Alkenyl, gegebenenfalls substituiertem C₂-C₈-Alkinyl, gegebenenfalls substituiertem Aryl, und gegebenenfalls substituiertem Heteroaryl; oder
R⁹ und R¹⁰, R⁹ und R¹¹, R¹⁰ und R¹¹ zusammen mit dem Atom, an welches sie gebunden sind, einen gegebenenfalls substituierten 3-20-gliedrigen Cycloalkyl- oder Heterocyclyl-Ring bilden;
jedes R⁴ ist unabhängig ausgewählt aus Null, Wasserstoff, Halogen, Oxo, CN, NO₂, OR¹⁸, SR¹⁸, NR¹⁸R¹⁹, OCOR¹⁸, OCO₂R¹⁸, OCONR¹⁸R¹⁹, COR¹⁸, CO₂R¹⁸, CONR¹⁸R¹⁹, SOR¹⁸, SO₂R¹⁸, SO₂NR¹⁸R¹⁹, NR²⁰CO₂R¹⁸, NR²⁰COR¹⁸, NR²⁰C(O)NR¹⁸R¹⁹, NR²⁰SOR¹⁸, NR²⁰SO₂R¹⁸, NR⁷SO₂NR⁵R⁶, gegebenenfalls substituiertem C₁-C₈-Alkyl, gegebenenfalls substituiertem C₂-C₈-Alkenyl, gegebenenfalls substituiertem C₂-C₈-Alkinyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 4-8-gliedrigem Heterocyclyl, gegebenenfalls substituiertem Aryl, und gegebenenfalls substituiertem Heteroaryl; wobei
R¹⁸, R¹⁹ und R²⁰ unabhängig ausgewählt sind aus Wasserstoff, gegebenenfalls substituiertem C₁-C₈-Alkyl, gegebenenfalls substituiertem C₂-C₈-Alkenyl, gegebenenfalls substituiertem C₂-C₈-Alkinyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 4-8-gliedrigem Heterocyclyl, gegebenenfalls substituiertem Aryl, und gegebenenfalls substituiertem Heteroaryl; oder
R¹⁸ und R¹⁹, R¹⁸ und R²⁰ zusammen mit dem Atom, an welches sie gebunden sind, einen 4-20-gliedrigen Heterocyclyl-Ring bilden;
Ar ausgewählt ist aus Null, Aryl und Heteroaryl;
n unabhängig ausgewählt ist aus 0, 1, 2, 3, 4 und 5;
Y, Z bei jedem Vorkommen unabhängig ausgewählt sind aus Null, CO, CO₂, CH₂, CR²⁴R²⁵, C(O)NR²⁴, C(S)NR²⁴, O, S, SO, SO₂, SO₂NR²⁴, NR²⁴, NR²⁴CO, NR²⁴CONR²⁴, NR²⁴C(S), gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₂-C₈-Alkenyl, gegebenenfalls substituiertem C₂-C₈-Alkinyl, gegebenenfalls substituiertem 3-8-gliedrigem Cycloalkyl, gegebenenfalls substituiertem Cs-Cs-Cycloalkoxy, gegebenenfalls substituiertem 3-8-gliedrigem Heterocyclyl, gegebenenfalls substituiertem Aryl, gegebenenfalls substituiertem Heteroaryl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃ Spirocycloalkyl, und gegebenenfalls substituiertem C₃-C₁₃ Spiroheterocyclyl; wobei
R²⁴ und R²⁵ unabhängig ausgewählt sind aus Wasserstoff, gegebenenfalls substituiertem C₁-C₈-Alkyl, gegebenenfalls substituiertem 3-8-gliedrigem Cycloalkyl, gegebenenfalls substituiertem Cs-Cs-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
W, bei jedem Auftreten unabhängig ausgewählt ist aus Null, CO, CH₂, (CH₂)ₘ CR²⁶R²⁷, (CR²⁶R²⁷)ₘ, SO, SO₂
wobei
R²⁶ und R²⁷ unabhängig ausgewählt sind aus Wasserstoff, gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
und
m = 0-5;
**(I-B)** eine Gruppe gemäß FORMEL 3M: 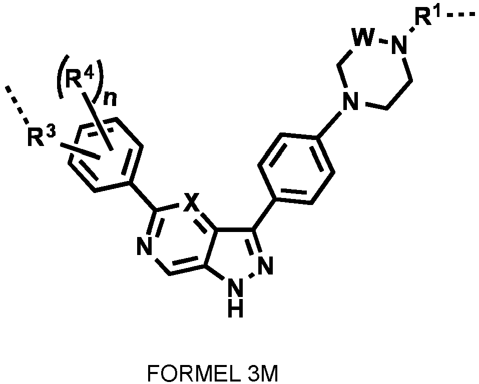 wobei
der "Linker"-Rest der bivalenten Verbindung unabhängig an R¹ und R³ gebunden ist; X ausgewählt ist aus CR² oder N;
die Definitionen von W, R¹, R², R³ und R⁴ dieselben sind wie für FORMEL 3L;
n unabhängig ausgewählt ist aus 0, 1, 2, 3, 4 und 5;
und ein pharmazeutisch akzeptables Salz davon;
**(II)** wobei **EL** ein Rest ist, die ausgewählt ist aus einer Gruppe bestehend aus:
**(II-A)** einer Gruppe gemäß FORMEL 12A, 12B, 12C und 12D 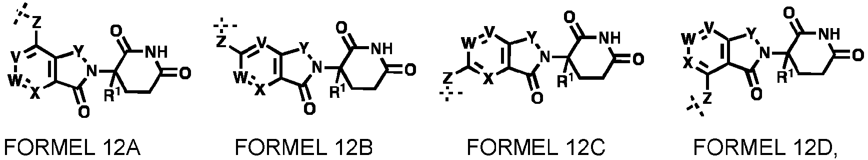 wobei
V, W, und X unabhängig ausgewählt sind aus CR² und N;
Y ausgewählt ist aus CO, CR³R⁴, und N=N;
Z ausgewählt ist aus Null, CO, CR⁵R⁶, NR⁵, O, gegebenenfalls substituiertem C₁-C₁₀-Alkylene, gegebenenfalls substituiertem C₁-C₁₀-Alkenylene, gegebenenfalls substituiertem C₁-C₁₀-Alkinylene, gegebenenfalls substituiertem 3-10-gliedrigem Carbocyclyl, gegebenenfalls substituiertem 4-10-gliedrigem Heterocyclyl, gegebenenfalls substituiertem C₃-C₁₃-fusioniertem Cycloalkyl, gegebenenfalls substituiertem C₃-C₁₃-fusioniertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃ Spirocycloalkyl, gegebenenfalls substituiertem C₃-C₁₃ Spiroheterocyclyl, gegebenenfalls substituiertem Aryl, und gegebenenfalls substituiertem Heteroaryl; vorzugsweise ist Z ausgewählt aus Null, CH₂, CH=CH, C=C, NH und O;
R¹ und R² unabhängig ausgewählt sind aus Wasserstoff, Halogen, Cyano, Nitro, gegebenenfalls substituiertem C₁-C₆-Alkyl, gegebenenfalls substituiertem 3-6-gliedrigem Carbocyclyl und gegebenenfalls substituiertem 4-6-gliedrigem Heterocyclyl;
R³ und R⁴ unabhängig voneinander ausgewählt sind aus Wasserstoff, Halogen, Cyano, Nitro, gegebenenfalls substituiertem C₁-C₆-Alkyl, gegebenenfalls substituiertem 3-6-gliedrigem Carbocyclyl und gegebenenfalls substituiertem 4-6-gliedrigem Heterocyclyl; oder R³ und R⁴ zusammen mit dem Atom, an welches sie gebunden sind, ein 3-6-gliedriges Carbocyclyl oder 4-6-gliedriges Heterocyclyl bilden; und
R⁵ und R⁶ unabhängig voneinander ausgewählt sind aus Null, Wasserstoff, Halogen, Oxo, Hydroxyl, Amino, Cyano, Nitro, gegebenenfalls substituiertem C₁-C₆-Alkyl, gegebenenfalls substituiertem 3- bis 6-gliedrigem Carbocyclyl, und gegebenenfalls substituiertem 4- bis 6-gliedrigem Heterocyclyl; oder R⁵ und R⁶ gemeinsam mit dem Atom, an welches sie gebunden sind, ein 3-6-gliedriges Carbocyclyl oder 4-6-gliedriges Heterocyclyl bilden;
**(II-B)** eine Gruppe gemäß einer der FORMELN 12E, 12F, 12G, 12H, und 12I: 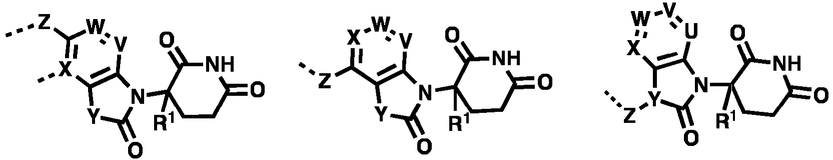 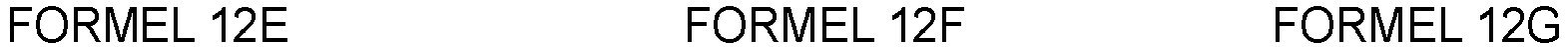 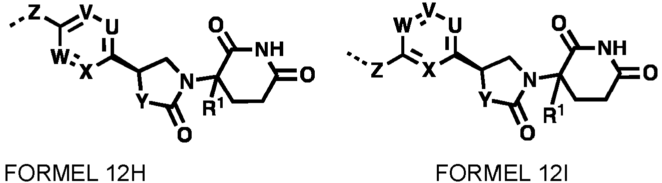 wobei
U, V, W und X unabhängig voneinander ausgewählt sind aus CR² und N;
Y ausgewählt ist aus CR³R⁴, NR³ und O; vorzugsweise ist Y ausgewählt aus CH₂, NH, NCH₃ und O;
Z ausgewählt ist aus Null, CO, CR⁵R⁶, NR⁵, O, gegebenenfalls substituiertem C₁-C₁₀-Alkylene, gegebenenfalls substituiertem C₁-C₁₀-Alkenylene, gegebenenfalls substituiertem C₁-C₁₀-Alkinylene, gegebenenfalls substituiertem 3-10-gliedrigem Carbocyclyl, gegebenenfalls substituiertem 4-10-gliedrigem Heterocyclyl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃-Spirocycloalkyl, gegebenenfalls substituiertem C₃-C₁₃-Spiroheterocyclyl, gegebenenfalls substituiertem Aryl, und gegebenenfalls substituiertem Heteroaryl; vorzugsweise ist Z ausgewählt aus Null, CH₂, CH=CH, C=C, NH und O;
R¹ und R² unabhängig voneinander ausgewählt sind aus Wasserstoff, Halogen, Cyano, Nitro, gegebenenfalls substituiertem C₁-C₆-Alkyl, gegebenenfalls substituiertem 3- bis 6-gliedrigem Carbocyclyl, und gegebenenfalls substituiertem 4- bis 6-gliedrigem Heterocyclyl;
R³ und R⁴ unabhängig voneinander ausgewählt sind aus Wasserstoff, Halogen, Cyano, Nitro, gegebenenfalls substituiertem C₁-C₆-Alkyl, gegebenenfalls substituiertem 3- bis 6-gliedrigem Carbocyclyl, und gegebenenfalls substituiertem 4- bis 6-gliedrigem Heterocyclyl; oder R³ und R⁴ zusammen mit dem Atom, an welches sie gebunden sind, ein 3-6-gliedriges Carbocyclyl oder ein 4-6-gliedriges Heterocyclyl bilden;
R⁵ und R⁶ unabhängig voneinander ausgewählt sind aus Null, Wasserstoff, Halogen, Oxo, Hydroxyl, Amino, Cyano, Nitro, gegebenenfalls substituiertem C₁-C₆-Alkyl, gegebenenfalls substituiertem 3- bis 6-gliedrigem Carbocyclyl, und gegebenenfalls substituiertem 4- bis 6-gliedrigem Heterocyclyl; oder R⁵ und R⁶ zusammen mit dem Atom, an welches sie gebunden sind, eine 3-6-gliedriges Carbocyclyl oder 4-6-gliedriges Heterocyclyl bilden;
(II-C) eine Gruppe gemäß FORMEL 13A: 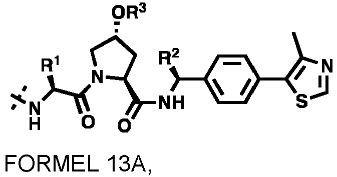 wobei
R¹ und R² unabhängig voneinander ausgewählt sind aus Wasserstoff, gegebenenfalls substituiertem C₁-C₈-Alkyl, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₁-C₈-Aminoalkyl, gegebenenfalls substituiertem C₁-C₈-Alkylamino-C₁-C₈-Alkyl, gegebenenfalls substituiertem C₃-C₇-Cycloalkyl, gegebenenfalls substituiertem 3-7-gliedrigem Heterocyclyl, gegebenenfalls substituiertem C₂-C₈ Alkenyl, und gegebenenfalls substituiertem C₂-C₈-Alkinyl; und
R³ ist Wasserstoff, gegebenenfalls substituiertes C(O)C₁-C₈-Alkyl, gegebenenfalls substituiertes C(O)C₁-C₈-Alkoxy-C₁-C₈-Alkyl, gegebenenfalls substituiertes C(O)C₁-C₈-Haloalkyl, gegebenenfalls substituiertes C(O)C₁-C₈-Hydroxyalkyl, gegebenenfalls substituiertes C(O)C₁-C₈-Aminoalkyl, gegebenenfalls substituiertes C(O)C₁-C₈-Alkylamino-C₁-C₈-Alkyl, gegebenenfalls substituiertes C(O)C₃-C₇-Cycloalkyl, gegebenenfalls substituiertes C(O)(3-7-gliedriges Heterocyclyl), gegebenenfalls substituiertes C(O)C₂-C₈-Alkenyl, gegebenenfalls substituiertes C(O)C₂-C₈-Alkinyl, gegebenenfalls substituiertes C(O)OC₁-C₈-Alkoxy-C₁-C₈-Alkyl, gegebenenfalls substituiertes C(O)OC₁-C₈-Haloalkyl, gegebenenfalls substituiertes C(O)OC₁-C₈-Hydroxyalkyl, gegebenenfalls substituiertes C(O)OC₁-C₈-Aminoalkyl, gegebenenfalls substituiertes C(O)OC₁-C₈-Alkylamino-C₁-C₈-Alkyl, gegebenenfalls substituiertes C(O)OC₃-C₇-Cycloalkyl, gegebenenfalls substituiertes C(O)O(3-7-gliedriges Heterocyclyl), gegebenenfalls substituiertes C(O)OC₂-C₈-Alkenyl, gegebenenfalls substituiertes C(O)OC₂-C₈-Alkinyl, gegebenenfalls substituiertes C(O)NC₁-C₈-Alkoxy-C₁-C₈-Alkyl, gegebenenfalls substituiertes C(O)NC₁-C₈ Haloalkyl, gegebenenfalls substituiertes C(O)NC₁-C₈ Hydroxyalkyl, gegebenenfalls substituiertes C(O)NC₁C₈-Aminoalkyl, gegebenenfalls substituiertes C(O)NC₁C₈-Alkylamino-C₁-C₈-Alkyl, gegebenenfalls substituiertes C(O)NC₃-C₇-Cycloalkyl, gegebenenfalls substituiertes C(O)N(3-7-gliedriges Heterocyclyl), gegebenenfalls substituiertes C(O)NC₂-C₈-Alkenyl, gegebenenfalls substituiertes C(O)NC₂-C₈-Alkinyl, gegebenenfalls substituiertes P(O)(OH)₂, gegebenenfalls substituiertes P(O)(OC₁-C₈ alkyl)₂, und gegebenenfalls substituiertes P(O)(OC₁-C₈ aryl)₂;
**(II-D)** eine Gruppe gemäß FORMEL 13B, 13C, 13D, 13E und 13F: 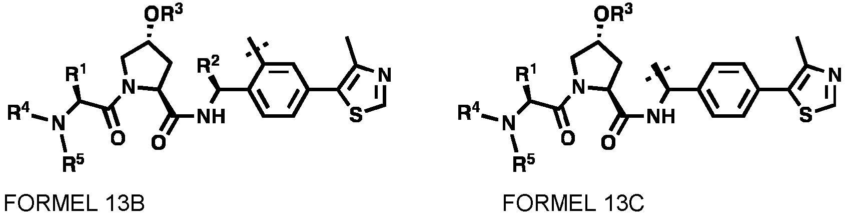 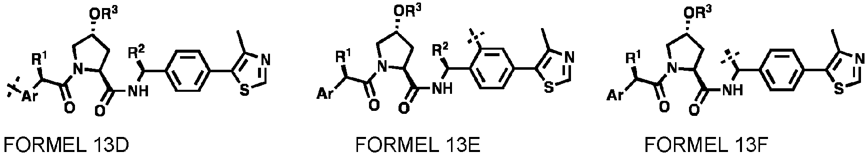 wobei
R¹ und R² unabhängig voneinander ausgewählt sind aus Wasserstoff, Halogen, OH, NH₂, CN, gegebenenfalls substituiertem C₁-C₈-Alkyl, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₁-C₈-Aminoalkyl, gegebenenfalls substituiertem C₁-C₈-Alkylamino-C₁-C₈-Alkyl, gegebenenfalls substituiertem C₃-C₇-Cycloalkyl, gegebenenfalls substituiertem 3-7-gliedrigen Heterocyclyl, gegebenenfalls substituiertem C₂-C₈-Alkenyl, und gegebenenfalls substituiertem C₂-C₈-Alkinyl; (vorzugsweise ist R¹ ausgewählt aus iso-Propyl oder tert-Butyl; und R² ist ausgewählt aus Wasserstoff oder Methyl);
R³ ausgewählt ist aus Wasserstoff, gegebenenfalls substituiertem C(O)C₁-C₈-Alkyl, gegebenenfalls substituiertem C(O)C₁-C₈-Alkoxy-C₁-C₈-Alkyl, gegebenenfalls substituiertem C(O)C₁-C₈-Haloalkyl, gegebenenfalls substituiertem C(O)C₁-C₈-Hydroxyalkyl, gegebenenfalls substituiertem C(O)C₁-C₈-Aminoalkyl, gegebenenfalls substituiertem C(O)C₁-C₈-Alkylamino-C₁-C₈-Alkyl, gegebenenfalls substituiertem C(O)C₃-C₇-Cycloalkyl, gegebenenfalls substituiertem C(O)(3-7-gliedrigem Heterocyclyl), gegebenenfalls substituiertem C(O)C₂-C₈-Alkenyl, gegebenenfalls substituiertem C(O)C₂-C₈-Alkinyl, gegebenenfalls substituiertem C(O)OC₁-C₈-Alkoxy-C₁-C₈-Alkyl, gegebenenfalls substituiertem C(O)OC₁-C₈-Haloalkyl, gegebenenfalls substituiertem C(O)OC₁-C₈-Hydroxyalkyl, gegebenenfalls substituiertem C(O)OC₁-C₈-Aminoalkyl, gegebenenfalls substituiertem C(O)OC₁-C₈-Alkylamino-C₁-C₈-Alkyl, gegebenenfalls substituiertem C(O)OC₃-C₇-Cycloalkyl, gegebenenfalls substituiertem C(O)O(3-7-gliedrigem Heterocyclyl), gegebenenfalls substituiertem C(O)OC₂-C₈-Alkenyl, gegebenenfalls substituiertem C(O)OC₂-C₈-Alkinyl, gegebenenfalls substituiertem C(O)NC₁-C₈-Alkoxy-C₁-C₈-Alkyl, gegebenenfalls substituiertem C(O)NC₁-C₈-Haloalkyl, gegebenenfalls substituiertem C(O)NC₁-C₈-Hydroxyalkyl, gegebenenfalls substituiertem C(O)NC₁-C₈-Aminoalkyl, gegebenenfalls substituiertem C(O)NC₁-C₈-Alkylamino-C₁-C₈-Alkyl, gegebenenfalls substituiertem C(O)NC₃-C₇-Cycloalkyl, gegebenenfalls substituiertem C(O)N(3-7-gliedrigem Heterocyclyl), gegebenenfalls substituiertem C(O)NC₂-C₈-Alkenyl, gegebenenfalls substituiertem C(O)NC₂-C₈-Alkinyl, gegebenenfalls substituiertem P(O)(OH)₂, gegebenenfalls substituiertem P(O)(OC₁-C₈-Alkyl)₂, und gegebenenfalls substituiertem P(O)(OC₁-C₈-Aryl)₂; und
R⁴ und R⁵ unabhängig voneinander ausgewählt sind aus Wasserstoff, COR⁶, CO₂R⁶, CONR⁶R⁷, SOR⁶, SO₂R⁶, SO₂NR⁶R⁷, gegebenenfalls substituiertem C₁-C₈-Alkyl, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem 3-8-gliedrigem Heterocyclyl, gegebenenfalls substituiertem Aryl, und gegebenenfalls substituiertem Heteroaryl; wobei
R⁶ und R⁷ unabhängig voneinander ausgewählt sind aus Wasserstoff, gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Alkylamino-C₁-C₈-Alkyl, gegebenenfalls substituiertem 3-8-gliedrigem Cycloalkyl, gegebenenfalls substituiertem 3-8-gliedrigem Heterocyclyl, gegebenenfalls substituiertem Aryl, und gegebenenfalls substituiertem Heteroaryl; oder
R⁴ und R⁵; R⁶ und R⁷ zusammen mit dem Atom, an welches sie gebunden sind, einen 4-8-gliedrigen Cycloalkyl- oder Heterocyclyl-Ring bilden;
Ar ausgewählt ist aus Aryl und Heteroaryl, wobei jedes davon gegebenenfalls substituiert ist mit einem oder mehreren Substituenten, die unabhängig voneinander ausgewählt werden aus F, Cl, CN, NO₂, OR⁸, NR⁸R⁹, COR⁸, CO2R⁸, CONR⁸R⁹, SOR⁸, SO₂R⁸, SO₂NR⁹R¹⁰, NR⁹COR¹⁰, NR⁸C(O)NR⁹R¹⁰, NR⁹SOR¹⁰, NR⁹SO₂R¹⁰, gegebenenfalls substituiertem C₁-C₆-Alkyl, gegebenenfalls substituiertem C₁-C₆-Alkoxyalkyl, gegebenenfalls substituiertem C₁-C₆-Haloalkyl, gegebenenfalls substituiertem C₁-C₆-Hydroxyalkyl, gegebenenfalls substituiertem C₁-C₆-Alkylamino-C₁-C₆-Alkyl, gegebenenfalls substituiertem C₃-C₇-Cycloalkyl, gegebenenfalls substituiertem 3-7-gliedrigem Heterocyclyl, gegebenenfalls substituiertem C₂-C₆-Alkenyl, gegebenenfalls substituiertem C₂-C₆-Alkinyl, gegebenenfalls substituiertem Aryl, und gegebenenfalls substituiertem C₄-C₅-Heteroaryl; wobei
R⁸, R⁹, und R¹⁰ unabhängig voneinander ausgewählt sind aus Null, Wasserstoff, gegebenenfalls substituiertem C₁-C₆-Alkyl, gegebenenfalls substituiertem C₂-C₆-Alkenyl, gegebenenfalls substituiertem C₂-C₆-Alkinyl, gegebenenfalls substituiertem C₃-C₇-Cycloalkyl, gegebenenfalls substituiertem 3-7-gliedrigem Heterocyclyl, gegebenenfalls substituiertem Aryl, und gegebenenfalls substituiertem Heteroaryl; oder
R⁸ und R⁹; R⁹ und R¹⁰ zusammen mit dem Atom, an welches sie gebunden sind, einen 4-8-gliedrigen Cycloalkyl- oder Heterocyclyl-Ring bilden;
und pharmazeutisch akzeptable Salze davon;
**(III)** wobei der **Linker** ist:
**(III-A)** ein Rest, der ausgewählt ist aus einer Gruppe bestehend aus:
**(III-A-a)** eine Gruppe gemäß FORMEL 16: 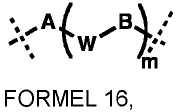 wobei
A, W, und B, bei jedem Auftreten unabhängig voneinander ausgewählt sind aus Null, CO, CO₂, C(O)NR¹, C(S)NR¹, O, S, SO, SO₂, SO₂NR¹, NR¹, NR¹CO, NR¹CONR², NR¹C(S), gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₂-C₈-Alkenyl, gegebenenfalls substituiertem C₂-C₈-Alkinyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, gegebenenfalls substituiertem Heteroaryl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃-Spirocycloalkyl, und gegebenenfalls substituiertem C₃-C₁₃-Spiroheterocyclyl; wobei
R¹ und R² unabhängig voneinander ausgewählt sind aus Wasserstoff, gegebenenfalls substituiertem C₁-C₈-Alkyl, gegebenenfalls substituiertem C₃-C₈-Cycloalkyl, gegebenenfalls substituiertem 3-8-gliedrigem Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl; und
m 0 bis 15 ist;
**(III-A-b)** eine Gruppe gemäß FORMEL 16A: 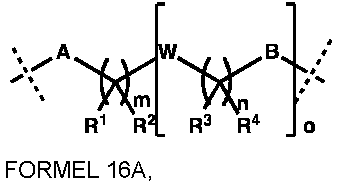 wobei
R¹, R², R³, und R⁴ bei jedem Auftreten unabhängig voneinander ausgewählt sind aus Wasserstoff, Halogen, CN, OH, NH₂, gegebenenfalls substituiertem C₁-C₈-Alkyl, gegebenenfalls substituiertem C₃-C₈-Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
A, W, und B bei jedem Auftreten unabhängig voneinander ausgewählt sind aus Null, CO, CO₂, C(O)NR⁵, C(S)NR⁵, O, S, SO, SO₂, SO₂NR⁵, NR⁵, NR⁵CO, NR⁵CONR⁶, NR⁵C(S), gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₂-C₈-Alkenyl, gegebenenfalls substituiertem C₂-C₈-Alkinyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, gegebenenfalls substituiertem Heteroaryl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃Spirocycloalkyl, und gegebenenfalls substituiertem C₃-C₁₃-Spiroheterocyclyl; wobei
R⁵ und R⁶ unabhängig voneinander ausgewählt sind aus Wasserstoff, gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
m 0 bis 15 ist;
n bei jedem Auftreten 0 bis 15 ist; und
o 0 bis 15 ist;
**(III-A-c)** eine Gruppe gemäß FORMEL 16B: 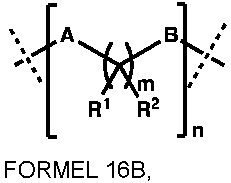 wobei
R¹ und R² bei jedem Auftreten unabhängig voneinander ausgewählt sind aus Wasserstoff, Halogen, CN, OH, NH₂, und gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, oder C₁-C₈-Alkylamino-C₁-C₈-Alkyl;
A und B bei jedem Auftreten unabhängig voneinander ausgewählt sind aus Null, CO, CO₂, C(O)NR³, C(S)NR³, O, S, SO, SO₂, SO₂NR³, NR³, NR³CO, NR³CONR⁴, NR³C(S), und gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₂-C₈-Alkenyl, gegebenenfalls substituiertem C₂-C₈-Alkinyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, gegebenenfalls substituiertem Heteroaryl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃-Spirocycloalkyl, oder C₃-C₁₃-Spiroheterocyclyl; wobei
R³ und R⁴ unabhängig voneinander ausgewählt sind aus Wasserstoff, und gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, oder C₁-C₈-Alkylamino-C₁-C₈-Alkyl;
jedes m 0 bis 15 ist; und
n 0 bis 15 ist;
**(III-A-d)** eine Gruppe gemäß FORMEL 16C: 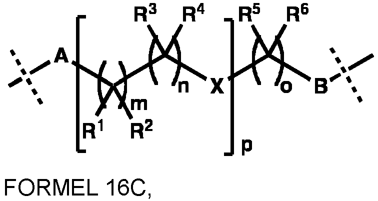 wobei
X ausgewählt ist aus O, NH, und NR⁷;
R¹, R², R³, R⁴, R⁵, und R⁶ bei jedem Auftreten unabhängig voneinander ausgewählt sind aus Wasserstoff, Halogen, CN, OH, NH₂, gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
A und B bei jedem Auftreten unabhängig voneinander ausgewählt sind aus Null, CO, NH, NH-CO, CO-NH, CH₂-NH-CO, CH₂-CO-NH, NH-CO-CH₂, CO-NH-CH₂, CH₂-NH-CH₂-CO-NH, CH₂-NH-CH₂-NH-CO, -CO-NH, CO-NH- CH₂-NH-CH₂, CH₂-NH-CH₂, CO₂, C(O)NR⁷, C(S)NR⁷, O, S, SO, SO₂, SO₂NR⁷, NR⁷, NR⁷CO, NR⁷CONR⁸, NR⁷C(S), gegebenenfalls substituiertem C₁-C₈-Alkyl, gegebenenfalls substituiertem C₁-C₈-Alkoxy, gegebenenfalls substituiertem C₁-C₈-Alkoxy-C₁-C₈-Alkyl, gegebenenfalls substituiertem C₁-C₈-Haloalkyl, gegebenenfalls substituiertem C₁-C₈-Hydroxyalkyl, gegebenenfalls substituiertem C₂-C₈-Alkenyl, gegebenenfalls substituiertem C₂-C₈-Alkinyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem Aryl, gegebenenfalls substituiertem Heteroaryl, gegebenenfalls substituiertem C₃-C₁₃-kondensiertem Cycloalkyl, gegebenenfalls substituiertem C₃-C₁₃-kondensiertem Heterocyclyl, gegebenenfalls substituiertem C₃-C₁₃-verbrücktem Cycloalkyl, gegebenenfalls substituiertem C₃-C₁₃-verbrücktem Heterocyclyl, gegebenenfalls substituiertem C₃-C₁₃-Spirocycloalkyl, und gegebenenfalls substituiertem C₃-C₁₃-Spiroheterocyclyl; wobei
R⁷ und R⁸ unabhängig voneinander ausgewählt sind aus Wasserstoff, gegebenenfalls substituiertem C₁-C₈-Alkyl, gegebenenfalls substituiertem 3-8-gliedrigem Cycloalkyl, gegebenenfalls substituiertem C₃-C₈-Cycloalkoxy, gegebenenfalls substituiertem 3-8-gliedrigem Heterocyclyl, gegebenenfalls substituiertem C₁-C₈-Alkoxy, gegebenenfalls substituiertem C₁-C₈-Alkoxyalkyl, gegebenenfalls substituiertem C₁-C₈-Haloalkyl, gegebenenfalls substituiertem C₁-C₈-Hydroxyalkyl, gegebenenfalls substituiertem C₁-C₈-Alkylamino, und gegebenenfalls substituiertem C₁-C₈-Alkylamino-C₁-C₈-Alkyl;
m bei jedem Auftreten 0 bis 15 ist;
n bei jedem Auftreten 0 bis 15 ist;
o 0 bis 15 ist; und
p 0 bis 15 ist;
und pharmazeutisch akzeptable Salze davon; oder
**(III-B)** ein Rest, der ausgewählt ist aus einer Gruppe bestehend aus: einem Ring ausgewählt aus der Gruppe bestehend aus einem 3- bis 13- gliedrigem Ring; einem 3-bis 13-gliedrigem kondensierten Ring; einem 3- bis 13-gliedrigem verbrückten Ring; und einem 3- bis 13-gliedrigem Spiroring; und pharmazeutisch akzeptable Salze davon; oder
**(III-C)** ein Rest, der ausgewählt ist aus einer Gruppe bestehend aus einer der FORMELN C1, C2, C3, C4 und C5: 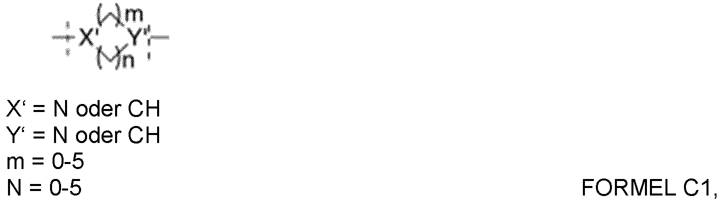 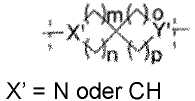 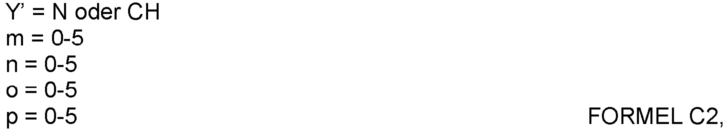 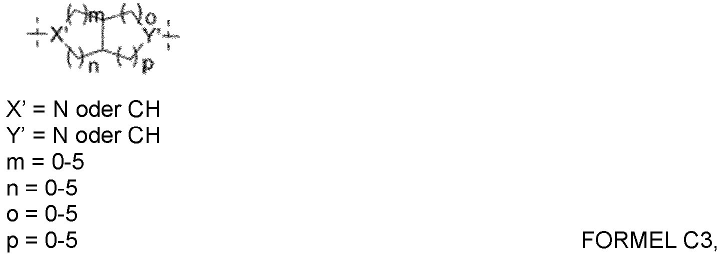 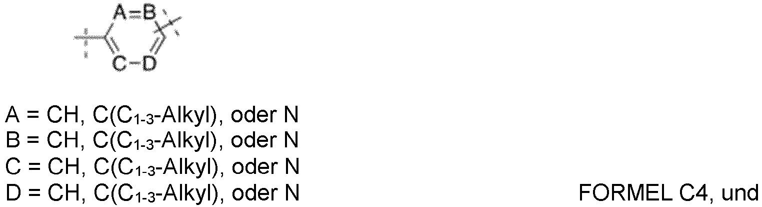 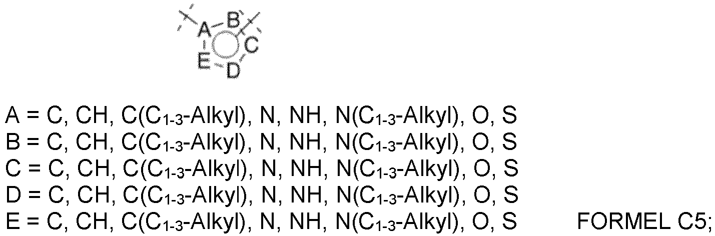
und pharmazeutisch akzeptable Salze davon.

2. Die Verbindung gemäß Anspruch 1, wobei die Verbindung ausgewählt ist aus der Gruppe bestehend aus:
a. 2-(2,6-Dioxopiperidin-3-yl)-4-((3-(4-(4-(5-(2-Fluor-6-methoxyphenyl)-1*H*-pyrazolo[4,3-*d*]pyrimidin-3-yl)phenyl)piperazin-1-yl)-3-oxopropyl)amino)isoindolin-1,3-dion (HC90-50); und
b. 2-(2,6-Dioxopiperidin-3-yl)-4-((4-(4-(4-(5-(2-Fluor-6-methoxyphenyl)-1*H*-pyrazolo[4,3-d]pyrimidin-3-yl)phenyl)piperazin-1-yl)-4-oxobutyl)amino)isoindolin-1,3-dion (HC90-51), und
pharmazeutisch akzeptablen Salzen davon.

3. Die bivalente Verbindung nach Anspruch 1, wobei **PI** ist: 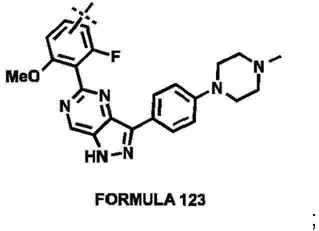 und pharmazeutisch akzeptable Salze davon.

4. Die bivalente Verbindung gemäß Anspruch 1(I I-A)-(I I-B), wobei **EL** ein Rest ist, der ausgewählt ist aus einer Gruppe bestehend aus: 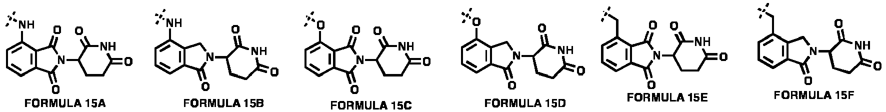 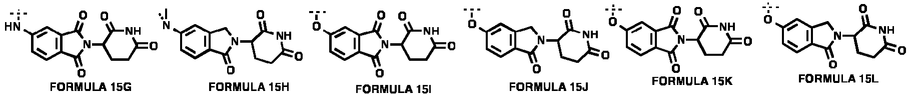 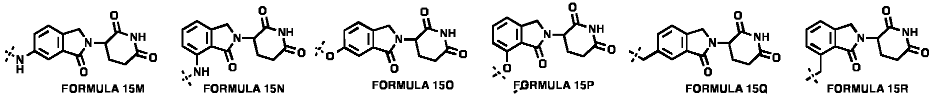 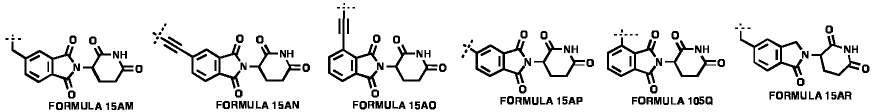 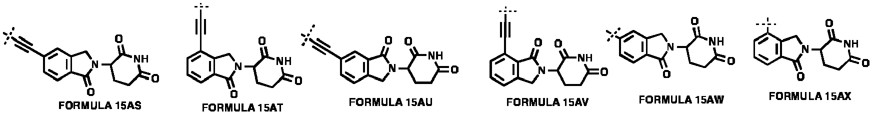 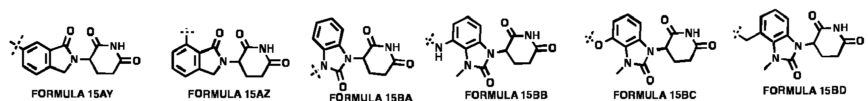 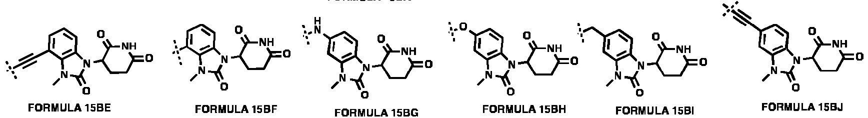 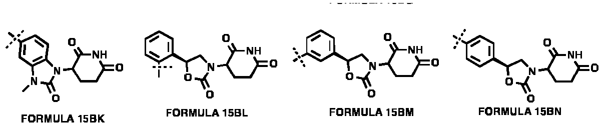 und pharmazeutisch akzeptablen Salzen davon.

5. Die bivalente Verbindung gemäß Anspruch 1, wobei die Verbindung ausgewählt ist aus der Gruppe bestehend aus:
2-(2,6-Dioxopiperidin-3-yl)-5-((2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethyl)amino)isoindolin-1,3-dion (HC90-33);
2-(2,6-Dioxopiperidin-3-yl)-5-((2-(2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethoxy)ethyl)amino)isoindolin-1,3-dion (HC90-34);
2-(2,6-Dioxopiperidin-3-yl)-5-((2-(2-(2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethoxy)ethoxy)ethyl)amino)isoindolin-1,3-dion (HC90-35);
2-(2,6-Dioxopiperidin-3-yl)-5-((15-(4-(4-(5-(2-Fluor-6-methoxyphenyl)-1H-pyrazolo[4,3-d]pyrimidin-3-yl)phenyl)piperazin-1-yl)-15-oxo-3,6,9,12-tetraoxapentadecyl)amino)isoindolin-1,3-dion (HC90-36);
2-(2,6-Dioxopiperidin-3-yl)-5-((18-(4-(4-(5-(2-Fluor-6-methoxyphenyl)-1H-pyrazolo[4,3-d]pyrimidin-3-yl)phenyl)piperazin-1-yl)-18-oxo-3,6,9,12,15-pentaoxaoctadecyl)amino)isoindolin-1,3-dion (HC90-37);
2-(2,6-Dioxopiperidin-3-yl)-5-((2-(4-(4-(5-(2-Fluor-6-methoxyphenyl)-1H-pyrazolo[4,3-d]pyrimidin-3-yl)phenyl)piperazin-1-yl)-2-oxoethyl)amino)isoindolin-1,3-dion (HC90-41);
2-(2,6-Dioxopiperidin-3-yl)-5-((3-(4-(4-(5-(2-Fluor-6-methoxyphenyl)-1H-pyrazolo[4,3-d]pyrimidin-3-yl)phenyl)piperazin-1-yl)-3-oxopropyl)amino)isoindolin-1,3-dion (HC90-42);
2-(2,6-Dioxopiperidin-3-yl)-5-((4-(4-(4-(5-(2-Fluor-6-methoxyphenyl)-1H-pyrazolo[4,3-d]pyrimidin-3-yl)phenyl)piperazin-1-yl)-4-oxobutyl)amino)isoindolin-1,3-dion (HC90-43);
2-(2,6-Dioxopiperidin-3-yl)-5-((5-(4-(4-(5-(2-Fluor-6-methoxyphenyl)-1H-pyrazolo[4,3-d]pyrimidin-3-yl)phenyl)piperazin-1-yl)-5-oxopentyl)amino)isoindolin-1,3-dion (HC90-44);
2-(2,6-Dioxopiperidin-3-yl)-5-((6-(4-(4-(5-(2-Fluor-6-methoxyphenyl)-1H-pyrazolo[4,3-d]pyrimidin-3-yl)phenyl)piperazin-1-yl)-6-oxohexyl)amino)isoindolin-1,3-dion (HC90-45);
2-(2,6-Dioxopiperidin-3-yl)-5-((7-(4-(4-(5-(2-Fluor-6-methoxyphenyl)-1H-pyrazolo[4,3-d]pyrimidin-3-yl)phenyl)piperazin-1-yl)-7-oxoheptyl)amino)isoindolin-1,3-dion (HC90-46);
2-(2,6-Dioxopiperidin-3-yl)-5-((8-(4-(4-(5-(2-Fluor-6-methoxyphenyl)-1H-pyrazolo[4,3-d]pyrimidin-3-yl)phenyl)piperazin-1-yl)-8-oxooctyl)amino)isoindolin-1,3-dion (HC90-47);
2-(2,6-Dioxopiperidin-3-yl)-4-((2-(4-(4-(5-(2-Fluor-6-methoxyphenyl)-1H-pyrazolo[4,3-d]pyrimidin-3-yl)phenyl)piperazin-1-yl)-2-oxoethyl)amino)isoindolin-1,3-dion (HC90-49);
2-(2,6-Dioxopiperidin-3-yl)-4-((3-(4-(4-(5-(2-Fluor-6-methoxyphenyl)-1H-pyrazolo[4,3-d]pyrimidin-3-yl)phenyl)piperazin-1-yl)-3-oxopropyl)amino)isoindolin-1,3-dion (HC90-50);
2-(2,6-Dioxopiperidin-3-yl)-4-((4-(4-(4-(5-(2-Fluor-6-methoxyphenyl)-1H-pyrazolo[4,3-d]pyrimidin-3-yl)phenyl)piperazin-1-yl)-4-oxobutyl)amino)isoindolin-1,3-dion (HC90-51);
2-(2,6-Dioxopiperidin-3-yl)-4-((5-(4-(4-(5-(2-Fluor-6-methoxyphenyl)-1H-pyrazolo[4,3-d]pyrimidin-3-yl)phenyl)piperazin-1-yl)-5-oxopentyl)amino)isoindolin-1,3-dion (HC90-52);
2-(2,6-Dioxopiperidin-3-yl)-4-((6-(4-(4-(5-(2-Fluor-6-methoxyphenyl)-1H-pyrazolo[4,3-d]pyrimidin-3-yl)phenyl)piperazin-1-yl)-6-oxohexyl)amino)isoindolin-1,3-dion (HC90-53);_
2-(2,6-Dioxopiperidin-3-yl)-4-((7-(4-(4-(5-(2-Fluor-6-methoxyphenyl)-1H-pyrazolo[4,3-d]pyrimidin-3-yl)phenyl)piperazin-1-yl)-7-oxoheptyl)amino)isoindolin-1,3-dion (HC90-54);
2-(2,6-Dioxopiperidin-3-yl)-4-((2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethyl)amino)isoindolin-1,3-dion (HC90-55);
2-(2,6-Dioxopiperidin-3-yl)-4-((2-(2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethoxy)ethyl)amino)isoindolin-1,3-dion (HC90-56);
2-(2,6-Dioxopiperidin-3-yl)-4-((2-(2-(2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethoxy)ethoxy)ethyl)amino)isoindolin-1,3-dion (HC90-57);
2-(2,6-Dioxopiperidin-3-yl)-4-((15-(4-(4-(5-(2-Fluor-6-methoxyphenyl)-1H-pyrazolo[4,3-d]pyrimidin-3-yl)phenyl)piperazin-1-yl)-15-oxo-3,6,9,12-tetraoxapentadecyl)amino)isoindolin-1,3-dion (HC90-58);
2- (2,6-Dioxopiperidin-3-yl)-4-((18-(4-(4-(5-(2-Fluor-6-methoxyphenyl)-1H-pyrazolo[4,3-d]pyrimidin-3-yl)phenyl)piperazin-1-yl)-18-oxo-3,6,9,12,15-pentaoxaoctadecyl)amino)isoindolin-1,3-dion (HC90-59);
(2S,4R)-1-((S)-2-(3-(3-(4-(4-(5-(2-Fluor-6-methoxyphenyl)-1H-pyrazolo[4,3-d]pyrimidin-3-yl)phenyl)piperazin-1-yl)-3-oxopropoxy)propanamido)-3,3-dimethylbutanoyl)-4-hydroxy-N-(4-(4-methylthiazol-5-yl)benzyl)pyrrolidin-2-carboxamid (HC90-61);
(2S,4R)-1-((S)-2-(tert-Butyl)-19-(4-(4-(5-(2-Fluor-6-methoxyphenyl)-1H-pyrazolo[4,3-d]pyrimidin-3-yl)phenyl)piperazin-1-yl)-4,19-dioxo-7,10,13,16-tetraoxa-3-azanonadecanoyl)-4-hydroxy-N-(4-(4-methylthiazol-5-yl)benzyl)pyrrolidin-2-carboxamid (HC90-66);
(2S,4R)-1-((S)-2-(4-(4-(4-(5-(2-Fluor-6-methoxyphenyl)-1H-pyrazolo[4,3-d]pyrimidin-3-yl)phenyl)piperazin-1-yl)-4-oxobutanamido)-3,3-dimethylbutanoyl)-4-hydroxy-N-(4-(4-methylthiazol-5-yl)benzyl)pyrrolidin-2-carboxamid (HC90-69);
(2S,4R)-1-((S)-2-(5-(4-(4-(5-(2-Fluor-6-methoxyphenyl)-1H-pyrazolo[4,3-d]pyrimidin-3-yl)phenyl)piperazin-1-yl)-5-oxopentanamido)-3,3-dimethylbutanoyl)-4-hydroxy-N-(4-(4-methylthiazol-5-yl)benzyl)pyrrolidin-2-carboxamid (HC90-70);
(2S,4R)-1-((S)-2-(6-(4-(4-(5-(2-Fluor-6-methoxyphenyl)-1H-pyrazolo[4,3-d]pyrimidin-3-yl)phenyl)piperazin-1-yl)-6-oxohexanamido)-3,3-dimethylbutanoyl)-4-hydroxy-N-(4-(4-methylthiazol-5-yl)benzyl)pyrrolidin-2-carboxamid (HC90-71);
(2S,4R)-1-((S)-2-(7-(4-(4-(5-(2-Fluor-6-methoxyphenyl)-1H-pyrazolo[4,3-d]pyrimidin-3-yl)phenyl)piperazin-1-yl)-7-oxoheptanamido)-3,3-dimethylbutanoyl)-4-hydroxy-N-(4-(4-methylthiazol-5-yl)benzyl)pyrrolidin-2-carboxamid (HC90-72);
(2S,4R)-1-((S)-2-(8-(4-(4-(5-(2-Fluor-6-methoxyphenyl)-1H-pyrazolo[4,3-d]pyrimidin-3-yl)phenyl)piperazin-1-yl)-8-oxooctanamido)-3,3-dimethylbutanoyl)-4-hydroxy-N-(4-(4-methylthiazol-5-yl)benzyl)pyrrolidin-2-carboxamid (HC90-73);
(2S,4R)-1-((S)-2-(10-(4-(4-(5-(2-Fluor-6-methoxyphenyl)-1H-pyrazolo[4,3-d]pyrimidin-3-yl)phenyl)piperazin-1-yl)-10-oxodecanamido)-3,3-dimethylbutanoyl)-4-hydroxy-N-(4-(4-methylthiazol-5-yl)benzyl)pyrrolidin-2-carboxamid (HC90-74);
(2S,4R)-1-((S)-2-(9-(4-(4-(5-(2-Fluor-6-methoxyphenyl)-1H-pyrazolo[4,3-d]pyrimidin-3-yl)phenyl)piperazin-1-yl)-9-oxononanamido)-3,3-dimethylbutanoyl)-4-hydroxy-N-(4-(4-methylthiazol-5-yl)benzyl)pyrrolidin-2-carboxamid (HC90-84);
(2S,4R)-1-((S)-2-(11-(4-(4-(5-(2-Fluor-6-methoxyphenyl)-1H-pyrazolo[4,3-d]pyrimidin-3-yl)phenyl)piperazin-1-yl)-11-oxoundecanamido)-3,3-dimethylbutanoyl)-4-hydroxy-N-(4-(4-methylthiazol-5-yl)benzyl)pyrrolidin-2-carboxamid (HC90-85);
N-(2-((2-(2,6-Dioxopiperidin-3-yl)-1,3-dioxoisoindolin-5-yl)amino)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-86);
N-(3-((2-(2,6-Dioxopiperidin-3-yl)-1,3-dioxoisoindolin-5-yl)amino)propyl)-2-(4-(4-(5-(2-Fluor-6-methoxyphenyl)-1H-pyrazolo[4,3-d]pyrimidin-3-yl)phenyl)piperazin-1-yl)acetamid (HC90-87);
N-(4-((2-(2,6-Dioxopiperidin-3-yl)-1,3-dioxoisoindolin-5-yl)amino)butyl)-2-(4-(4-(5-(2-Fluor-6-methoxyphenyl)-1H-pyrazolo[4,3-d]pyrimidin-3-yl)phenyl)piperazin-1-yl)acetamid (HC90-88);
N-(5-((2-(2,6-Dioxopiperidin-3-yl)-1,3-dioxoisoindolin-5-yl)amino)pentyl)-2-(4-(4-(5-(2-Fluor-6-methoxyphenyl)-1H-pyrazolo[4,3-d]pyrimidin-3-yl)phenyl)piperazin-1-yl)acetamid (HC90-89);
N-(6-((2-(2,6-Dioxopiperidin-3-yl)-1,3-dioxoisoindolin-5-yl)amino)hexyl)-2-(4-(4-(5-(2-Fluor-6-methoxyphenyl)-1H-pyrazolo[4,3-d]pyrimidin-3-yl)phenyl)piperazin-1-yl)acetamid (HC90-90);
N-(7-((2-(2,6-Dioxopiperidin-3-yl)-1,3-dioxoisoindolin-5-yl)amino)heptyl)-2-(4-(4-(5-(2-Fluor-6-methoxyphenyl)-1H-pyrazolo[4,3-d]pyrimidin-3-yl)phenyl)piperazin-1-yl)acetamid (HC90-91);
N-(8-((2-(2,6-Dioxopiperidin-3-yl)-1,3-dioxoisoindolin-5-yl)amino)octyl)-2-(4-(4-(5-(2-Fluor-6-methoxyphenyl)-1H-pyrazolo[4,3-d]pyrimidin-3-yl)phenyl)piperazin-1-yl)acetamid (HC90-92);
N-(2-(2-((2-(2,6-Dioxopiperidin-3-yl)-1,3-dioxoisoindolin-5-yl)amino)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-93);
N-(2-(2-(2-((2-(2,6-Dioxopiperidin-3-yl)-1,3-dioxoisoindolin-5-yl)amino)ethoxy)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-94);
N-(2-(2-(2-(2-((2-(2,6-Dioxopiperidin-3-yl)-1,3-dioxoisoindolin-5-yl)amino)ethoxy)ethoxy)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-95);
N-(14-((2-(2,6-Dioxopiperidin-3-yl)-1,3-dioxoisoindolin-5-yl)amino)-3,6,9,12-tetraoxatetradecyl)-2-(4-(4-(5-(2-Fluor-6-methoxyphenyl)-1H-pyrazolo[4,3-d]pyrimidin-3-yl)phenyl)piperazin-1-yl)acetamid (HC90-96);
N-(17-((2-(2,6-Dioxopiperidin-3-yl)-1,3-dioxoisoindolin-5-yl)amino)-3,6,9,12,15-pentaoxaheptadecyl)-2-(4-(4-(5-(2-Fluor-6-methoxyphenyl)-1H-pyrazolo[4,3-d]pyrimidin-3-yl)phenyl)piperazin-1-yl)acetamid (HC90-97);
N-(2-((2-(2,6-Dioxopiperidin-3-yl)-1,3-dioxoisoindolin-4-yl)amino)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-102);
N-(3-((2-(2,6-Dioxopiperidin-3-yl)-1,3-dioxoisoindolin-4-yl)amino)propyl)-2-(4-(4-(5-(2-Fluor-6-methoxyphenyl)-1H-pyrazolo[4,3-d]pyrimidin-3-yl)phenyl)piperazin-1-yl)acetamid (HC90-103);
N-(4-((2-(2,6-Dioxopiperidin-3-yl)-1,3-dioxoisoindolin-4-yl)amino)butyl)-2-(4-(4-(5-(2-Fluor-6-methoxyphenyl)-1H-pyrazolo[4,3-d]pyrimidin-3-yl)phenyl)piperazin-1-yl)acetamid (HC90-104);
N-(5-((2-(2,6-Dioxopiperidin-3-yl)-1,3-dioxoisoindolin-4-yl)amino)pentyl)-2-(4-(4-(5-(2-Fluor-6-methoxyphenyl)-1H-pyrazolo[4,3-d]pyrimidin-3-yl)phenyl)piperazin-1-yl)acetamid (HC90-105);
N-(6-((2-(2,6-Dioxopiperidin-3-yl)-1,3-dioxoisoindolin-4-yl)amino)hexyl)-2-(4-(4-(5-(2-Fluor-6-methoxyphenyl)-1H-pyrazolo[4,3-d]pyrimidin-3-yl)phenyl)piperazin-1-yl)acetamid (HC90-106);
N-(7-((2-(2,6-Dioxopiperidin-3-yl)-1,3-dioxoisoindolin-4-yl)amino)heptyl)-2-(4-(4-(5-(2-Fluor-6-methoxyphenyl)-1H-pyrazolo[4,3-d]pyrimidin-3-yl)phenyl)piperazin-1-yl)acetamid (HC90-107);
N-(8-((2-(2,6-Dioxopiperidin-3-yl)-1,3-dioxoisoindolin-4-yl)amino)octyl)-2-(4-(4-(5-(2-Fluor-6-methoxyphenyl)-1H-pyrazolo[4,3-d]pyrimidin-3-yl)phenyl)piperazin-1-yl)acetamid (HC90-108);
N-(2-(2-((2-(2,6-Dioxopiperidin-3-yl)-1,3-dioxoisoindolin-4-yl)amino)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-109);
N-(2-(2-(2-((2-(2,6-Dioxopiperidin-3-yl)-1,3-dioxoisoindolin-4-yl)amino)ethoxy)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-110);
N-(2-(2-(2-(2-((2-(2,6-Dioxopiperidin-3-yl)-1,3-dioxoisoindolin-4-yl)amino)ethoxy)ethoxy)ethoxy)ethyl)-2-(4-(4-(5-(2-Fluor-6-methoxyphenyl)-1H-pyrazolo[4,3-d]pyrimidin-3-yl)phenyl)piperazin-1-yl)acetamid (HC90-111);
N-(14-((2-(2,6-Dioxopiperidin-3-yl)-1,3-dioxoisoindolin-4-yl)amino)-3,6,9,12-tetraoxatetradecyl)-2-(4-(4-(5-(2-Fluor-6-methoxyphenyl)-1H-pyrazolo[4,3-d]pyrimidin-3-yl)phenyl)piperazin-1-yl)acetamid (HC90-112);
N-(17-((2-(2,6-Dioxopiperidin-3-yl)-1,3-dioxoisoindolin-4-yl)amino)-3,6,9,12,15-pentaoxaheptadecyl)-2-(4-(4-(5-(2-Fluor-6-methoxyphenyl)-1H-pyrazolo[4,3-d]pyrimidin-3-yl)phenyl)piperazin-1-yl)acetamid (HC90-113);
2- (2,6-Dioxopiperidin-3-yl)-4-((8-(4-(4-(5-(2-Fluor-6-methoxyphenyl)-1H-pyrazolo[4,3-d]pyrimidin-3-yl)phenyl)piperazin-1-yl)-8-oxooctyl)amino)isoindolin-1,3-dion (HC90-117);
(2S,4R)-1-((S)-2-(2-(2-(2-(4-(4-(5-(2-Fluor-6-methoxyphenyl)-1H-pyrazolo[4,3-d]pyrimidin-3-yl)phenyl)piperazin-1-yl)acetamido)ethoxy)acetamido)-3,3-dimethylbutanoyl)-4-hydroxy-N-(4-(4-methylthiazol-5-yl)benzyl)pyrrolidin-2-carboxamid (HC90-119);
(2S,4R)-1-((S)-2-(3-(2-(2-(4-(4-(5-(2-Fluor-6-methoxyphenyl)-1H-pyrazolo[4,3-d]pyrimidin-3-yl)phenyl)piperazin-1-yl)acetamido)ethoxy)propanamido)-3,3-dimethylbutanoyl)-4-hydroxy-N-(4-(4-methylthiazol-5-yl)benzyl)pyrrolidin-2-carboxamid (HC90-120);
(2S,4R)-1-((S)-2-(tert-Butyl)-14-(4-(4-(5-(2-Fluor-6-methoxyphenyl)-1H-pyrazolo[4,3-d]pyrimidin-3-yl)phenyl)piperazin-1-yl)-4,13-dioxo-6,9-dioxa-3,12-diazatetradecanoyl)-4-hydroxy-N-(4-(4-methylthiazol-5-yl)benzyl)pyrrolidin-2-carboxamid (HC90-121);
(2S,4R)-1-((S)-14-(tert-Butyl)-1-(4-(4-(5-(2-Fluor-6-methoxyphenyl)-1H-pyrazolo[4,3-d]pyrimidin-3-yl)phenyl)piperazin-1-yl)-2,12-dioxo-6,9-dioxa-3,13-diazapentadecan-15-oyl)-4-hydroxy-N-(4-(4-methylthiazol-5-yl)benzyl)pyrrolidin-2-carboxamid (HC90-122);
(2S,4R)-1-((S)-2-(tert-Butyl)-17-(4-(4-(5-(2-Fluor-6-methoxyphenyl)-1H-pyrazolo[4,3-d]pyrimidin-3-yl)phenyl)piperazin-1-yl)-4,16-dioxo-6,9,12-trioxa-3,15-diazaheptadecanoyl)-4-hydroxy-N-(4-(4-methylthiazol-5-yl)benzyl)pyrrolidin-2-carboxamid (HC90-123);
(2S,4R)-1-((S)-17-(tert-Butyl)-1-(4-(4-(5-(2-Fluor-6-methoxyphenyl)-1H-pyrazolo[4,3-d]pyrimidin-3-yl)phenyl)piperazin-1-yl)-2,15-dioxo-6,9,12-trioxa-3,16-diazaoctadecan-18-oyl)-4-hydroxy-N-(4-(4-methylthiazol-5-yl)benzyl)pyrrolidin-2-carboxamid (HC90-124);
(2S,4R)-1-((S)-20-(tert-Butyl)-1-(4-(4-(5-(2-Fluor-6-methoxyphenyl)-1H-pyrazolo[4,3-d]pyrimidin-3-yl)phenyl)piperazin-1-yl)-2,18-dioxo-6,9,12,15-tetraoxa-3,19-diazahenicosan-21-oyl)-4-hydroxy-N-(4-(4-methylthiazol-5-yl)benzyl)pyrrolidin-2-carboxamid (HC90-125);
(2S,4R)-1-((S)-23-(tert-Butyl)-1-(4-(4-(5-(2-Fluor-6-methoxyphenyl)-1H-pyrazolo[4,3-d]pyrimidin-3-yl)phenyl)piperazin-1-yl)-2,21-dioxo-6,9,12,15,18-pentaoxa-3,22-diazatetracosan-24-oyl)-4-hydroxy-N-(4-(4-methylthiazol-5-yl)benzyl)pyrrolidin-2-carboxamid (HC90-126);
(2S,4R)-1-((S)-2-(2-(2-(4-(4-(5-(2-Fluor-6-methoxyphenyl)-1H-pyrazolo[4,3-d]pyrimidin-3-yl)phenyl)piperazin-1-yl)acetamido)acetamido)-3,3-dimethylbutanoyl)-4-hydroxy-N-(4-(4-methylthiazol-5-yl)benzyl)pyrrolidin-2-carboxamid (HC90-127);
(2S,4R)-1-((S)-2-(3-(2-(4-(4-(5-(2-Fluor-6-methoxyphenyl)-1H-pyrazolo[4,3-d]pyrimidin-3-yl)phenyl)piperazin-1-yl)acetamido)propanamido)-3,3-dimethylbutanoyl)-4-hydroxy-N-(4-(4-methylthiazol-5-yl)benzyl)pyrrolidin-2-carboxamid (HC90-128);
(2S,4R)-1-((S)-2-(4-(2-(4-(4-(5-(2-Fluor-6-methoxyphenyl)-1H-pyrazolo[4,3-d]pyrimidin-3-yl)phenyl)piperazin-1-yl)acetamido)butanamido)-3,3-dimethylbutanoyl)-4-hydroxy-N-(4-(4-methylthiazol-5-yl)benzyl)pyrrolidin-2-carboxamid (HC90-129);
(2S,4R)-1-((S)-2-(5-(2-(4-(4-(5-(2-Fluor-6-methoxyphenyl)-1H-pyrazolo[4,3-d]pyrimidin-3-yl)phenyl)piperazin-1-yl)acetamido)pentanamido)-3,3-dimethylbutanoyl)-4-hydroxy-N-(4-(4-methylthiazol-5-yl)benzyl)pyrrolidin-2-carboxamid (HC90-130);
(2S,4R)-1-((S)-2-(6-(2-(4-(4-(5-(2-Fluor-6-methoxyphenyl)-1H-pyrazolo[4,3-d]pyrimidin-3-yl)phenyl)piperazin-1-yl)acetamido)hexanamido)-3,3-dimethylbutanoyl)-4-hydroxy-N-(4-(4-methylthiazol-5-yl)benzyl)pyrrolidin-2-carboxamid (HC90-131);
(2S,4R)-1-((S)-2-(7-(2-(4-(4-(5-(2-Fluor-6-methoxyphenyl)-1H-pyrazolo[4,3-d]pyrimidin-3-yl)phenyl)piperazin-1-yl)acetamido)heptanamido)-3,3-dimethylbutanoyl)-4-hydroxy-N-(4-(4-methylthiazol-5-yl)benzyl)pyrrolidin-2-carboxamid (HC90-132);
(2S,4R)-1-((S)-2-(8-(2-(4-(4-(5-(2-Fluor-6-methoxyphenyl)-1H-pyrazolo[4,3-d]pyrimidin-3-yl)phenyl)piperazin-1-yl)acetamido)octanamido)-3,3-dimethylbutanoyl)-4-hydroxy-N-(4-(4-methylthiazol-5-yl)benzyl)pyrrolidin-2-carboxamid (HC90-133);
(2S,4R)-1-((S)-2-(9-(2-(4-(4-(5-(2-Fluor-6-methoxyphenyl)-1H-pyrazolo[4,3-d]pyrimidin-3-yl)phenyl)piperazin-1-yl)acetamido)nonanamido)-3,3-dimethylbutanoyl)-4-hydroxy-N-(4-(4-methylthiazol-5-yl)benzyl)pyrrolidin-2-carboxamid (HC90-134);
(2S,4R)-1-((S)-2-(10-(2-(4-(4-(5-(2-Fluor-6-methoxyphenyl)-1H-pyrazolo[4,3-d]pyrimidin-3-yl)phenyl)piperazin-1-yl)acetamido)decanamido)-3,3-dimethylbutanoyl)-4-hydroxy-N-(4-(4-methylthiazol-5-yl)benzyl)pyrrolidin-2-carboxamid (HC90-135);
(2S,4R)-1-((S)-2-(11-(2-(4-(4-(5-(2-Fluor-6-methoxyphenyl)-1H-pyrazolo[4,3-d]pyrimidin-3-yl)phenyl)piperazin-1-yl)acetamido)undecanamido)-3,3-dimethylbutanoyl)-4-hydroxy-N-(4-(4-methylthiazol-5-yl)benzyl)pyrrolidin-2-carboxamid (HC90-136);
(2S,4R)-1-((S)-2-(2-(2-(4-(4-(5-(2-Fluor-6-methoxyphenyl)-1H-pyrazolo[4,3-d]pyrimidin-3-yl)phenyl)piperazin-1-yl)-2-oxoethoxy)acetamido)-3,3-dimethylbutanoyl)-4-hydroxy-N-(4-(4-methylthiazol-5-yl)benzyl)pyrrolidin-2-carboxamid (HC90-60);
(2S,4R)-1-((S)-2-(2-(2-(2-(4-(4-(5-(2-Fluor-6-methoxyphenyl)-1H-pyrazolo[4,3-d]pyrimidin-3-yl)phenyl)piperazin-1-yl)-2-oxoethoxy)ethoxy)acetamido)-3,3-dimethylbutanoyl)-4-hydroxy-N-(4-(4-methylthiazol-5-yl)benzyl)pyrrolidin-2-carboxamid (HC90-62);
(2S,4R)-1-((S)-2-(3-(2-(3-(4-(4-(5-(2-Fluor-6-methoxyphenyl)-1H-pyrazolo[4,3-d]pyrimidin-3-yl)phenyl)piperazin-1-yl)-3-oxopropoxy)ethoxy)propanamido)-3,3-dimethylbutanoyl)-4-hydroxy-N-(4-(4-methylthiazol-5-yl)benzyl)pyrrolidin-2-carboxamid (HC90-63);
(2S,4R)-1-((S)-2-(tert-Butyl)-14-(4-(4-(5-(2-Fluor-6-methoxyphenyl)-1H-pyrazolo[4,3-d]pyrimidin-3-yl)phenyl)piperazin-1-yl)-4,14-dioxo-6,9,12-trioxa-3-azatetradecanoyl)-4-hydroxy-N-(4-(4-methylthiazol-5-yl)benzyl)pyrrolidin-2-carboxamid (HC90-64);
(2S,4R)-1-((S)-2-(tert-Butyl)-16-(4-(4-(5-(2-Fluor-6-methoxyphenyl)-1H-pyrazolo[4,3-d]pyrimidin-3-yl)phenyl)piperazin-1-yl)-4,16-dioxo-7,10,13-trioxa-3-azahexadecanoyl)-4-hydroxy-N-(4-(4-methylthiazol-5-yl)benzyl)pyrrolidin-2-carboxamid (HC90-65);
(2S,4R)-1-((S)-2-(tert-Butyl)-20-(4-(4-(5-(2-Fluor-6-methoxyphenyl)-1H-pyrazolo[4,3-d]pyrimidin-3-yl)phenyl)piperazin-1-yl)-4,20-dioxo-6,9,12,15,18-pentaoxa-3-azaicosanoyl)-4-hydroxy-N-(4-(4-methylthiazol-5-yl)benzyl)pyrrolidin-2-carboxamid (HC90-67); und
(2S,4R)-1-((S)-2-(tert-Butyl)-22-(4-(4-(5-(2-Fluor-6-methoxyphenyl)-1H-pyrazolo[4,3-d]pyrimidin-3-yl)phenyl)piperazin-1-yl)-4,22-dioxo-7,10,13,16,19-pentaoxa-3-azadocosanoyl)-4-hydroxy-N-(4-(4-methylthiazol-5-yl)benzyl)pyrrolidin-2-carboxamid (HC90-68);
und pharmazeutisch akzeptable Salze davon.

6. Die bivalente Verbindung gemäß einem der Ansprüche 1-5 zur Verwendung in einem Verfahren zur Behandlung einer HPK1-vermittelten Erkrankung bei einem Patienten, der einer Behandlung bedarf,
vorzugsweise ist die HPK1-vermittelte Erkrankung ausgewählt aus der Gruppe bestehend aus Krebs, chronischen Infektionen die eine erschöpfte Immunantwort hervorrufen, infektionsbedingter Immunsuppression, altersbedingter Abnahme der Immunantwort, altersbedingter Abnahme kognitiver Funktionen und Unfruchtbarkeit,
stärker bevorzugt ist der Krebs ausgewählt aus einer Gruppe bestehend aus Krebserkrankungen der Brust, des Respirationstrakts, des Gehirns, der Fortpflanzungsorgane, des Verdauungstrakts, des Harntrakts, des Auges, der Leber, der Haut, des Kopfes und des Halses, der Schilddrüse, der Nebenschilddrüse, und deren fernen Metastasen, Lymphomen, Sarkomen und Leukämien.

7. Pharmazeutische Zusammensetzung, die die bivalente Verbindung gemäß einem der Ansprüche 1-5 enthält, zur Verwendung in einem Verfahren zur Behandlung einer HPK1-vermittelten Erkrankung in einem Patienten, der einer Behandlung bedarf.

8. Die bivalente Verbindung gemäß einem der Ansprüche 1-5 zur Verwendung in einem Verfahren zur Reduktion der HPK1-Expression in Zellen, wobei das Verfahren Folgendes umfasst:
Isolieren der Zellen aus dem Körper des Probanden;
Behandeln der Zellen mit der bivalenten Verbindung; und
Wiedereinführen der behandelten Zellen in den Körper des Probanden,
vorzugsweise
**(i)** die Zellen sind ausgewählt aus der Gruppe bestehend aus autologen Knochenmarkzellen, Nabelschnurrstammzellen und peripheren Blut- oder Knochenmarkstammzellen, oder
**(ii)** die Zellen sind Immunzellen, die ausgewählt sind aus der Gruppe bestehend aus T-Zellen, genetisch veränderten T-Zellen, chimären Antigenrezeptor (CAR) T-Zellen,
Tumorinfiltrierenden Lymphozyten, dendritischen Zellen, Makrophagen, Mastzellen,
Granulozyten (einschließlich Basophilen, Eosinophilen und Neutrophilen), natürliche Killerzellen, NK T-Zellen und B-Zellen.

9. Die bivalente Verbindung gemäß einem der Ansprüche 1-5 zur Verwendung in einem Verfahren zur Steigerung der Sekretion von Interleukin-2 aus T-Zellen, wobei das Verfahren Folgendes umfasst:
Isolieren der T-Zellen aus dem Körper eines Probanden;
Behandeln der T-Zellen mit der bivalenten Verbindung; und
Wiedereinführen der behandelten Zellen in den Körper des Probanden.

10. Eine pharmazeutische Zusammensetzung, die die bivalente Verbindung gemäß einem der Ansprüche 1-5 enthält, zur Verwendung in einem Verfahren zur Behandlung von Krebs in einem Probanden, der einer Behandlung bedarf, wobei das Verfahren folgendes umfasst:
Verabreichung an einen Probanden mit Krebs,
die pharmazeutische Zusammensetzung und einem pharmakologisch akzeptablen Träger, und
eine pharmazeutische Zusammensetzung, die einen Checkpoint-Inhibitor und einen pharmakologisch akzeptablen Träger enthält.

11. Pharmazeutische Zusammensetzung zur Verwendung gemäß Anspruch 10, wobei der Checkpoint-Inhibitor ausgewählt ist aus einer Liste bestehend aus anti-PD-1, anti-PD-L1 und anti-CTLA-4.

12. Die pharmazeutische Zusammensetzung zur Verwendung gemäß Anspruch 11,
**(i)** wobei der Checkpoint-Inhibitor anti-PD-1 ist, vorzugsweise ist anti-PD-1 ausgewählt aus der Gruppe bestehend aus Pembrolizumab, Nivolumab und Cemiplimab, stärker bevorzugt ist anti-PD-1 ausgewählt aus der Gruppe bestehend aus Pembrolizumab, Nivolumab und Cemiplimab und der Krebs ist ausgewählt aus der Gruppe bestehend aus Melanom, Lungenkrebs, Nierenzellkarzinom, Hodgkin-Lymphom, Kopf- und Hals-Krebs, Dickdarmkrebs und Leberkrebs;
**(ii)** wobei der Checkpoint-Inhibitor anti-PD-L1 ist, vorzugsweise wobei anti-PD-L1 ausgewählt ist aus der Gruppe bestehend aus Atezolizumab, Avelumab und Durvalumab, stärker bevorzugt ist anti-PD-L1 ausgewählt aus der Gruppe bestehend aus Atezolizumab, Avelumab und Durvalumab und der Krebs ist ausgewählt aus der Gruppe bestehend aus nicht-kleinzelligem Lungenkarzinom, multiplem Myelom, Urothelkarzinom und Kopf- und Halskrebs; oder
**(iii)** wobei der Checkpoint-Inhibitor anti-CTLA-4 ist, vorzugsweise wobei anti-CTLA-4 ausgewählt ist aus der Gruppe bestehend aus Pembrolizumab, Nivolumab und Cemiplimab, stärker bevorzugt ist anti-CDLA-4 ausgewählt aus der Gruppe bestehend aus Pembrolizuman, Nivolumab und Cemiplimab und der Krebs ist ausgewählt aus der Gruppe bestehend aus Melanom, Lungenkrebs, Nierenzellkarzinom, Glioblastom, hepatozelluläres Karzinom, großzelligem B-Zell-Lymphom, Hodgin-Lymphom, Kopf- und Halskrebs, Dickdarmkrebs und Leberkrebs.

13. Eine pharmazeutische Zusammensetzung, die die bivalente Verbindung gemäß einem der Ansprüche 1-5 enthält, zur Verwendung in einem Verfahren zur Behandlung von Krebs mit einer erhöhten Expression von Cyclooxygenase-2 bei einem Patienten, der einer Behandlung bedarf, wobei das Verfahren folgendes umfasst:
Verabreichung der pharmazeutischen Zusammensetzung und eines pharmazeutisch akzeptablen Trägers an einen Patienten mit Krebs und erhöhter Expression von Cyclooxygenase-2,
vorzugsweise wobei der Krebs ausgewählt ist aus einer Gruppe bestehend aus Dickdarmkrebs, Lungenkrebs, Sarkom und Brustkrebs.

14. Die pharmazeutische Zusammensetzung zur Verwendung gemäß Anspruch 10, wobei die beiden Zusammensetzungen gleichzeitig oder nacheinander verabreicht werden.

## Revendications

1. Composé bivalent comprenant un ligand kinase des cellules progénitrices hématopoïétiques 1 (HPK1) conjugué à un marqueur de dégradation/disruption, dans lequel le composé bivalent ayant la structure suivante : 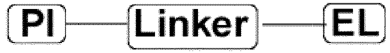
**(I)** dans lequel PI comprend un ligand HPK1 et EL comprend un marqueur de dégradation/disruption, PI étant un fragment choisi dans le groupe constitué par :
(I-A) un fragment selon la Formule 3L : 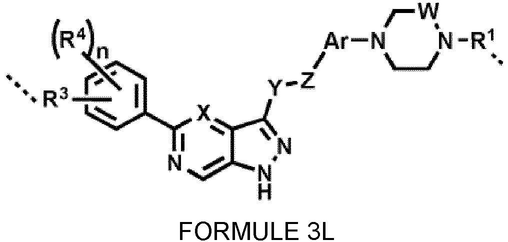 dans laquelle
le fragment « Linker » du composé bivalent est fixé indépendamment à R¹ ou R³ ;
X est choisi parmi CR² ou N ;
R¹ et R³ sont choisis indépendamment parmi rien, un hydrogène, C(O)R⁶, C(O)OR⁶, C(O)NR⁶R⁷, S(O)R⁶, S(O)₂R⁶, S(O)₂NR⁶R⁷, NR⁸C(O)OR⁶, NR⁸C(O)R⁶, NR⁸C(O)NR⁶R⁷, NR⁸S(O)R⁶, NR⁸S(O)₂R, NR⁸S(O)₂NR⁶R⁷, alkyle en C₁ à C₈ éventuellement substitué, alcényle en C₂ à C₈ éventuellement substitué, alcynyle en C₂ à C₈ éventuellement substitué, alcoxy en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué et hétéroaryle éventuellement substitué ; dans lesquels
R⁶ est rien, ou un fragment bivalent choisi parmi un alkyle en C₁ à C₈ éventuellement substitué, alcényle en C₂ à C₈ éventuellement substitué, alcynyle en C₂ à C₈ éventuellement substitué, alcoxy en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué et hétéroaryle éventuellement substitué ;
R⁷ et R⁸ sont choisis indépendamment parmi un hydrogène, alkyle en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle en C₃ à C₁₀ éventuellement substitué, hétérocyclyle de 3 à 20 chaînons éventuellement substitué, alcényle en C₂ à C₈ éventuellement substitué, alcynyle en C₂ à C₈ éventuellement substitué, aryle éventuellement substitué et hétéroaryle éventuellement substitué ; ou
R⁶ et R⁷ forment ensemble, avec l'atome auquel ils sont liés, un noyau cycloalkyle ou hétérocyclyle de 3 à 20 chaînons éventuellement substitué ;
R² est choisi indépendamment parmi un hydrogène, halogène, oxo, CN, NO₂, OR⁹, SR⁹, NR⁹R¹⁰, C(O)R⁹, C(O)OR⁹, C(O)NR⁹R¹⁰, S(O)R⁹, S(O)₂R⁹, S(O)₂NR⁹R¹⁰, NR¹¹C(O)OR⁹, NR¹¹C(O)R⁹, NR¹¹C(O)NR⁹R¹⁰, NR¹¹S(O)R⁹, NR¹¹S(O)₂R, NR¹¹S(O)₂NR⁹R¹⁰, alkyle en C₁ à C₈ éventuellement substitué, alcényle en C₂ à C₈ éventuellement substitué, alcynyle en C₂ à C₈ éventuellement substitué, alcoxy en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué et hétéroaryle éventuellement substitué ; dans lesquels
R⁹, R¹⁰ et R¹¹ sont choisis indépendamment parmi un hydrogène, alkyle en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle en C₃ à C₁₀ éventuellement substitué, hétérocyclyle de 3 à 20 chaînons éventuellement substitué, alcényle en C₂ à C₈ éventuellement substitué, alcynyle en C₂ à C₈ éventuellement substitué, aryle éventuellement substitué et hétéroaryle éventuellement substitué ; ou
R⁹ et R¹⁰, R⁹ et R¹¹, R¹⁰ et R¹¹ forment ensemble, avec l'atome auquel ils sont liés, un noyau cycloalkyle ou hétérocyclyle de 3 à 20 chaînons éventuellement substitué ;
chaque R⁴ est choisi indépendamment parmi rien, un hydrogène, halogène, oxo, CN, NO₂, OR¹⁸, SR¹⁸, NR¹⁸R¹⁹, OCOR¹⁸, OCO₂R¹⁸, OCONR¹⁸R¹⁹, COR¹⁸, CO₂R¹⁸, CONR¹⁸R¹⁹, SOR¹⁸, SO₂R¹⁸, SO₂NR¹⁸R¹⁹, NR²⁰CO₂R¹⁸, NR²⁰CO₂R¹⁸, NR²⁰C(O)NR¹⁸R¹⁹, NR²⁰SOR¹⁸, NR²⁰SO₂R¹⁸, NR⁷S(O)₂NR⁵R⁶, alkyle en C₁ à C₈ éventuellement substitué, alcényle en C₂ à C₈ éventuellement substitué, alcynyle en C₂ à C₈ éventuellement substitué, alcoxy en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, cycloalcoxy en C₃ à C₈ éventuellement substitué, hétérocyclyle de 4 à 8 chaînons éventuellement substitué, aryle éventuellement substitué et hétéroaryle éventuellement substitué ; dans lesquels
R¹⁸, R¹⁹ et R²⁰ sont choisis indépendamment parmi un hydrogène, alkyle en C₁ à C₈ éventuellement substitué, alcényle en C₂ à C₈ éventuellement substitué, alcynyle en C₂ à C₈ éventuellement substitué, alcoxy en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, cycloalcoxy en C₃ à C₈ éventuellement substitué, hétérocyclyle de 4 à 8 chaînons éventuellement substitué, aryle éventuellement substitué et hétéroaryle éventuellement substitué ; ou
R¹⁸ et R¹⁹, R¹⁸ et R²⁰ forment ensemble, avec l'atome auquel ils sont liés, un noyau hétérocyclyle de 4 à 20 chaînons ;
Ar est choisi parmi rien, un aryle et un hétéroaryle ;
n est choisi indépendamment parmi 0, 1, 2, 3, 4 et 5 ;
Y, Z, à chaque occurrence, sont choisis indépendamment parmi rien, CO, CO₂, CH₂, CR²⁴R²⁵, C(O)NR²⁴, C(S)NR²⁴, O, S, SO, SO₂, SO₂NR²⁴, NR²⁴, NR²⁴CO, NR²⁴CONR²⁴, NR²⁴C(S), alkyle en C₁ à C₈ éventuellement substitué, alcoxy en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, alcényle en C₂ à C₈ éventuellement substitué, alcynyle en C₂ à C₈ éventuellement substitué, cycloalkyle de 3 à 8 chaînons éventuellement substitué, cycloalcoxy en Cs à C₈ éventuellement substitué, hétérocyclyle de 3 à 8 chaînons éventuellement substitué, aryle éventuellement substitué, hétéroaryle éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué et spirohétérocyclyle en C₃ à C₁₃ éventuellement substitué ; dans laquelle
R²⁴ et R²⁵ sont choisis indépendamment parmi un hydrogène, alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
W, à chaque occurrence, est choisi indépendamment parmi rien, CO, CH₂, (CH₂)ₘ, CR²⁶R²⁷, (CR²⁶R²⁷)ₘ, SO, SO₂
dans lesquels
R²⁶ et R²⁷ sont choisis indépendamment parmi un hydrogène, alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
et
m = 0 à 5 ;
(I-B) un fragment selon la Formule 3M : 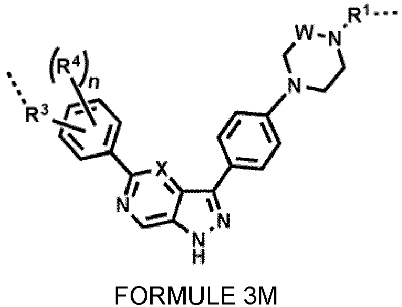 dans laquelle
le fragment « Linker » du composé bivalent est fixé indépendamment à R¹ ou R³ ;
X est choisi parmi CR² ou N ;
les définitions de W, R¹, R², R³ et R⁴ sont les mêmes que pour la Formule 3L;
n est choisi indépendamment parmi 0, 1, 2, 3, 4 et 5 ;
et des sels pharmaceutiquement acceptables de ceux-ci ;
(II) dans lequel EL est un fragment choisi dans le groupe constitué par :
(II-A) un fragment selon les formules 12A, 12B, 12C et 12D : 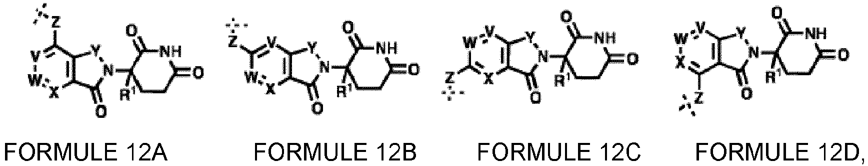 dans lesquelles
V, W et X sont choisis indépendamment parmi CR² et N ;
Y est choisi parmi CO, CR³R⁴ et N=N ;
Z est choisi parmi rien, CO, CR⁵R⁶, NR⁵, O, un alkylène en C₁ à C₁₀ éventuellement substitué, alcénylène en C₁ à C₁₀ éventuellement substitué, alcynylène en C₁ à C₁₀ éventuellement substitué, carbocyclyle de 3 à 10 chaînons éventuellement substitué, hétérocyclyle de 4 à 10 chaînons éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué, spirohétérocyclyle en C₃ à C₁₃ éventuellement substitué, aryle éventuellement substitué et hétéroaryle éventuellement substitué ; de préférence, Z est choisi parmi rien, CH₂, CH=CH, C=C, NH et O ;
R¹ et R² sont choisis indépendamment parmi un hydrogène, halogène, cyano, nitro, alkyle en C₁ à C₆ éventuellement substitué, carbocyclyle de 3 à 6 chaînons éventuellement substitué et hétérocyclyle de 4 à 6 chaînons éventuellement substitué ;
R³ et R⁴ sont choisis indépendamment parmi un hydrogène, halogène, cyano, nitro, alkyle en C₁ à C₆ éventuellement substitué, carbocyclyle de 3 à 6 chaînons éventuellement substitué et hétérocyclyle de 4 à 6 chaînons éventuellement substitué ; ou bien R³ et R⁴ forment ensemble, avec l'atome auquel ils sont liés, un carbocyclyle de 3 à 6 chaînons, ou un hétérocyclyle de 4 à 6 chaînons ; et
R⁵ et R⁶ sont choisis indépendamment parmi rien, un hydrogène, halogène, oxo, hydroxyle, amino, cyano, nitro, alkyle en C₁ à C₆ éventuellement substitué, carbocyclyle de 3 à 6 chaînons éventuellement substitué et hétérocyclyle de 4 à 6 chaînons éventuellement substitué ; ou bien R⁵ et R⁶ forment ensemble, avec l'atome auquel ils sont liés, un carbocyclyle de 3 à 6 chaînons, ou un hétérocyclyle de 4 à 6 chaînons ;
(II-B) un fragment selon l'une des formules 12E, 12F, 12G, 12H et 121 : 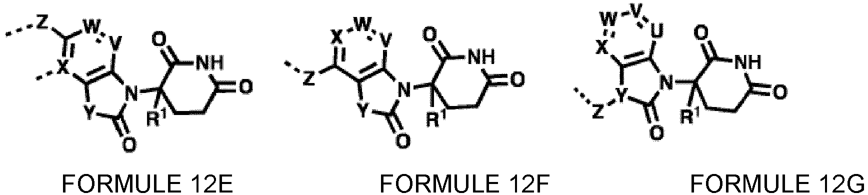 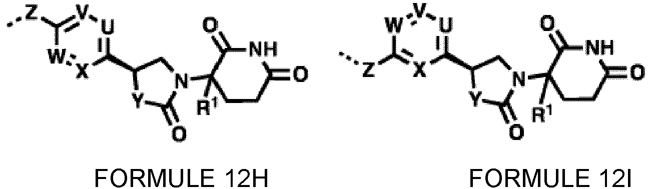 dans lesquelles
U, V, W et X sont choisis indépendamment parmi CR² et N ;
Y est choisi parmi CR³R⁴, NR³ et O ; de préférence, Y est choisi parmi CH₂, NH, NCH₃ et O ;
Z est choisi parmi rien, CO, CR⁵R⁶, NR⁵, O, un alkylène en C₁ à C₁₀ éventuellement substitué, alcénylène en C₁ à C₁₀ éventuellement substitué, alcynylène en C₁ à C₁₀ éventuellement substitué, carbocyclyle de 3 à 10 chaînons éventuellement substitué, hétérocyclyle de 4 à 10 chaînons éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué, spirohétérocyclyle en C₃ à C₁₃ éventuellement substitué, aryle éventuellement substitué et hétéroaryle éventuellement substitué ; de préférence, Z est choisi parmi rien, CH₂, CH=CH, C=C, NH et O ;
R¹ et R² sont choisis indépendamment parmi un hydrogène, halogène, cyano, nitro, alkyle en C₁ à C₆ éventuellement substitué, carbocyclyle de 3 à 6 chaînons éventuellement substitué et hétérocyclyle de 4 à 6 chaînons éventuellement substitué ;
R³ et R⁴ sont choisis indépendamment parmi un hydrogène, halogène, cyano, nitro, alkyle en C₁ à C₆ éventuellement substitué, carbocyclyle de 3 à 6 chaînons éventuellement substitué et hétérocyclyle de 4 à 6 chaînons éventuellement substitué ; ou R³ et R⁴ forment ensemble, avec l'atome auquel ils sont liés, un carbocyclyle de 3 à 6 chaînons, ou un hétérocyclyle de 4 à 6 chaînons ; et
R⁵ et R⁶ sont choisis indépendamment parmi rien, un hydrogène, halogène, oxo, hydroxyle, amino, cyano, nitro, alkyle en C₁ à C₆ éventuellement substitué, carbocyclyle de 3 à 6 chaînons éventuellement substitué et hétérocyclyle de 4 à 6 chaînons éventuellement substitué ; ou bien R⁵ et R⁶ forment ensemble, avec l'atome auquel ils sont liés, un carbocyclyle de 3 à 6 chaînons, ou un hétérocyclyle de 4 à 6 chaînons ;
(II-C) un fragment selon la Formule 13A : 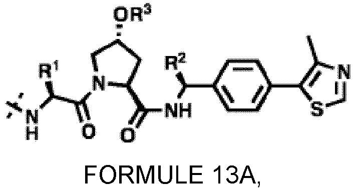 dans laquelle
R¹ et R² sont choisis indépendamment parmi un hydrogène, alkyle en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, aminoalkyle en C₁ à C₈ éventuellement substitué, aminoalkyle en C₁ à C₈-alkyle en C₁ à C₈ éventuellement substitué, cycloalkyle en C₃ à C₇ éventuellement substitué, hétérocyclyle de 3 à 7 chaînons éventuellement substitué, alcényle en C₂ à C₈ éventuellement substitué et alcynyle en C₂ à C₈ éventuellement substitué ; et
R³ est un hydrogène, C(O)-alkyle en C₁ à C₈ éventuellement substitué, C(O)-alcoxy en C₁ à C₈-alkyle en C₁ à C₈ éventuellement substitué, C(O)-haloalkyle en C₁ à C₈ éventuellement substitué, C(O)-hydroxyalkyle en C₁ à C₈ éventuellement substitué, C(O)-aminoalkyle en C₁ à C₈ éventuellement substitué, C(O)-alkylamino en C₁ à C₈-alkyle en C₁ à C₈ éventuellement substitué, C(O)-cycloalkyle en C₃ à C₇ éventuellement substitué, C(O)-hétérocyclyle de 3 à 7 chaînons éventuellement substitué, C(O)-alcényle en C₂ à C₈ éventuellement substitué, C(O)-alcynyle en C₂ à C₈ éventuellement substitué, C(O)O-alcoxy en C₁ à C₈-alkyle en C₁ à C₈) éventuellement substitué, C(O)O-haloalkyle en C₁ à C₈ éventuellement substitué, C(O)O-hydroxyalkyle en C₁ à C₈ éventuellement substitué, C(O)O-aminoalkyle en C₁ à C₈ éventuellement substitué, C(O)O-alkylamino en C₁ à C₈-alkyle en C₁ à C₈ éventuellement substitué, C(O)O-cycloalkyle en C₃ à C₇ éventuellement substitué, C(O)O-hétérocyclyle de 3 à 7 chaînons éventuellement substitué, C(O)O-alcényle en C₂ à C₈ éventuellement substitué, C(O)O-alcynyle en C₂ à C₈ éventuellement substitué, C(O)N-alcoxy en C₁ à C₈-alkyle en C₁ à C₈ éventuellement substitué, C(O)N-haloalkyle en C₁ à C₈ éventuellement substitué, C(O)N-hydroxyalkyle en C₁ à C₈ éventuellement substitué, C(O)N-aminoalkyle en C₁ à C₈ éventuellement substitué, C(O)N-alkylamino en C₁ à C₈-alkyle en C₁ à C₈ éventuellement substitué, C(O)N-cycloalkyle en C₃ à C₇ éventuellement substitué, C(O)N-hétérocyclyle de 3 à 7 chaînons éventuellement substitué, C(O)N-alcényle en C₂ à C₈ éventuellement substitué, C(O)N-alcynyle en C₂ à C₈ éventuellement substitué, P(O)(OH)₂ éventuellement substitué, P(O)(O-alkyle en C₁ à C₈)₂ éventuellement substitué et P(O)(O-aryle en C₁ à C₈)₂ éventuellement substitué ;
(II-D) un fragment selon les Formules 13B, 13C, 13D, 13E et 13F : 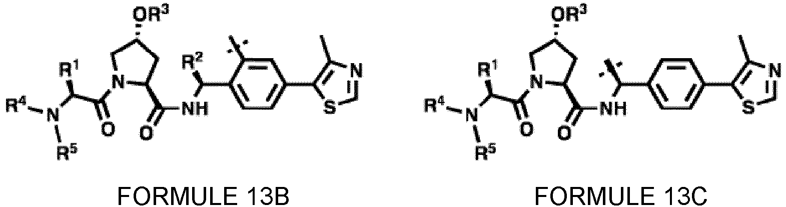 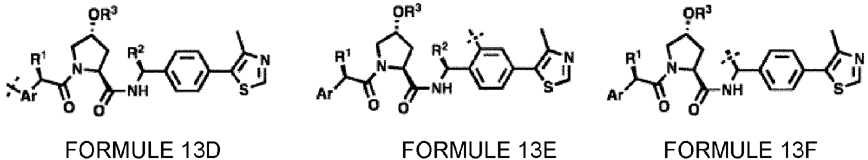 dans lesquelles
R¹ et R² sont choisis indépendamment parmi un hydrogène, halogène, OH, NH₂, CN, alkyle en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, aminoalkyle en C₁ à C₈ éventuellement substitué, alkylamino en C₁ à C₈-alkyle en C₁ à C₈ éventuellement substitué, cycloalkyle en C₃ à C₇ éventuellement substitué, hétérocyclyle de 3 à 7 chaînons éventuellement substitué, alcényle en C₂ à C₈ éventuellement substitué et alcynyle en C₂ à C₈ éventuellement substitué ; (de préférence, R¹ est choisi parmi l'isopropyle ou le tert-butyle ; et R² est choisi parmi l'hydrogène ou le méthyle) ;
R³ est un hydrogène, C(O)-alkyle en C₁ à C₈ éventuellement substitué, C(O)-alcoxy en C₁ à C₈-alkyle en C₁ à C₈ éventuellement substitué, C(O)-haloalkyle en C₁ à C₈ éventuellement substitué, C(O)-hydroxyalkyle en C₁ à C₈ éventuellement substitué, C(O)-aminoalkyle en C₁ à C₈ éventuellement substitué, C(O)-alkylamino en C₁ à C₈-alkyle en C₁ à C₈ éventuellement substitué, C(O)-cycloalkyle en C₃ à C₇ éventuellement substitué, C(O)-hétérocyclyle de 3 à 7 chaînons éventuellement substitué, C(O)-alcényle en C₂ à C₈ éventuellement substitué, C(O)-alcynyle en C₂ à C₈ éventuellement substitué, C(O)O-alcoxy en C₁ à C₈-alkyle en C₁ à C₈) éventuellement substitué, C(O)O-haloalkyle en C₁ à C₈ éventuellement substitué, C(O)O-hydroxyalkyle en C₁ à C₈ éventuellement substitué, C(O)O-aminoalkyle en C₁ à C₈ éventuellement substitué, C(O)O-alkylamino en C₁ à C₈-alkyle en C₁ à C₈ éventuellement substitué, C(O)O-cycloalkyle en C₃ à C₇ éventuellement substitué, C(O)O-hétérocyclyle de 3 à 7 chaînons éventuellement substitué, C(O)O-alcényle en C₂ à C₈ éventuellement substitué, C(O)O-alcynyle en C₂ à C₈ éventuellement substitué, C(O)N-alcoxy en C₁ à C₈-alkyle en C₁ à C₈ éventuellement substitué, C(O)N-haloalkyle en C₁ à C₈ éventuellement substitué, C(O)N-hydroxyalkyle en C₁ à C₈ éventuellement substitué, C(O)N-aminoalkyle en C₁ à C₈ éventuellement substitué, C(O)N-alkylamino en C₁ à C₈-alkyle en C₁ à C₈ éventuellement substitué, C(O)N-cycloalkyle en C₃ à C₇ éventuellement substitué, C(O)N-hétérocyclyle de 3 à 7 chaînons éventuellement substitué, C(O)N-alcényle en C₂ à C₈ éventuellement substitué, C(O)N-alcynyle en C₂ à C₈ éventuellement substitué, P(O)(OH)₂ éventuellement substitué, P(O)(O-alkyle en C₁ à C₈)₂ éventuellement substitué et P(O)(O-aryle en C₁ à C₈)₂ éventuellement substitué ; et
R⁴ et R⁵ sont choisis indépendamment parmi un hydrogène, COR⁶, CO₂R⁶, CONR⁶R⁷, SOR⁶, SO₂R⁶, SO₂NR⁶R⁷, alkyle en C₁ à C₈ éventuellement substitué, alcoxy en C₁ à C₈-alkyle en C₁ à C₈ éventuellement substitué, alkylamino en C₁ à C₈-alkyle en C₁ à C₈ éventuellement substitué, cycloalkyle de 3 à 8 chaînons éventuellement substitué, hétérocyclyle de 3 à 8 chaînons éventuellement substitué, aryle éventuellement substitué et hétéroaryle éventuellement substitué ; dans lesquels
R⁶ et R⁷ sont choisis indépendamment parmi un hydrogène, alkyle en C₁ à C₈ éventuellement substitué, alcoxy en C₁ à C₈ éventuellement substitué, alcoxy en C₁ à C₈-alkyle en C₁ à C₈ éventuellement substitué, alkyle en C₁ à Cs-aminoalkyle en C₁ à C₈ éventuellement substitué, cycloalkyle de 3 à 8 chaînons éventuellement substitué, hétérocyclyle de 3 à 8 chaînons éventuellement substitué, aryle éventuellement substitué et hétéroaryle éventuellement substitué ; ou
R⁴ et R⁵ ; R⁶ et R⁷ forment ensemble, avec l'atome auquel ils sont liés, un noyau cycloalkyle ou hétérocyclyle de 4 à 8 chaînons ;
Ar est choisi parmi un aryle et un hétéroaryle, chacun étant éventuellement substitué par un ou plusieurs substituants choisis indépendamment parmi F, Cl, CN, NO₂, OR⁸, NR⁸R⁹, COR⁸, CO₂R⁸, CONR⁸R⁹, SOR⁸, SO₂R⁸, SO₂NR⁹R¹⁰, NR⁹COR¹⁰, NR⁸C(O)NR⁹R¹⁰, NR⁹SOR¹⁰, NR⁹SO₂R¹⁰, alkyle en C₁ à C₆ éventuellement substitué, alcoxyalkyle en C₁ à C₆ éventuellement substitué, haloalkyle en C₁ à C₆ éventuellement substitué, hydroxyalkyle en C₁ à C₆ éventuellement substitué, alkylamino en C₁ à C₈-alkyle en C₁ à C₈ éventuellement substitué, cycloalkyle en C₃ à C₇ éventuellement substitué, hétérocyclyle de 3 à 7 chaînons éventuellement substitué, alcényle en C₂ à C₆ éventuellement substitué, alcynyle en C₂ à C₆ éventuellement substitué, aryle éventuellement substitué et hétéroaryle en C₄ à C₅ éventuellement substitué ; dans lesquels
R⁸, R⁹ et R¹⁰ sont choisis indépendamment parmi rien, un hydrogène, alkyle en C₁ à C₆ éventuellement substitué, alcényle en C₂ à C₆ éventuellement substitué, alcynyle en C₂ à C₆ éventuellement substitué, cycloalkyle en C₃ à C₇ éventuellement substitué, hétérocyclyle de 3 à 7 chaînons éventuellement substitué, aryle éventuellement substitué et hétéroaryle éventuellement substitué ; ou
R⁸ et R⁹ ; R⁹ et R¹⁰ forment ensemble, avec l'atome auquel ils sont liés, un noyau cycloalkyle ou hétérocyclyle de 4 à 8 chaînons ;
et des sels pharmaceutiquement acceptables de ceux-ci ;
(III) dans laquelle le « Linker » est :
(III-A) un fragment choisi dans le groupe constitué par :
(III-A-a) un fragment selon la Formule 16 : 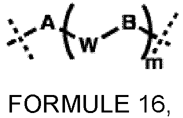 dans laquelle
A, W et B, à chaque occurrence, sont choisis indépendamment parmi rien, CO, CO₂, C(O)NR¹, C(S)NR¹, O, S, SO, SO₂, SO₂NR¹, NR¹, NR¹CO, NR¹CONR², NR¹C(S), alkyle en C₁ à C₈ éventuellement substitué, alcoxy en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, alcényle en C₂ à C₈ éventuellement substitué, alcynyle en C₂ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué, hétéroaryle éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué et spirohétérocyclyle en C₃ à C₁₃ éventuellement substitué ; dans lesquels
R¹ et R² sont choisis indépendamment parmi un hydrogène, alkyle en C₁ à C₈ éventuellement substitué, cycloalkyle en C₃ à C₈ éventuellement substitué, cycloalcoxy de 3 à 8 chaînons éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ; et
m va de 0 à 15 ;
(III-A-b) un fragment selon la Formule 16A : 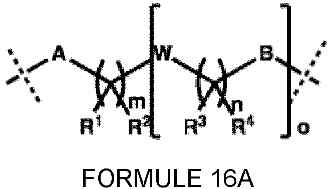 dans laquelle
R¹, R², R³ et R⁴, à chaque occurrence, sont choisis indépendamment parmi un hydrogène, halogène, CN, OH, NH₂, alkyle en C₁ à C₈ éventuellement substitué, cycloalkyle en C₃ à C₈ éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
A, W et B, à chaque occurrence, sont choisis indépendamment parmi rien, CO, CO₂, C(O)NR⁵, C(S)NR⁵, O, S, SO, SO₂, SO₂NR⁵, NR⁵, NR⁵CO, NR⁵CONR⁶, NR⁵C(S), alkyle en C₁ à C₈ éventuellement substitué, alcoxy en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, alcényle en C₂ à C₈ éventuellement substitué, alcynyle en C₂ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué, hétéroaryle éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué et spirohétérocyclyle en C₃ à C₁₃ éventuellement substitué ; dans lesquels
R⁵ et R⁶ sont choisis indépendamment parmi un hydrogène, alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
m va de 0 à 15 ;
n, à chaque occurrence, va de 0 à 15 ; et
o va de 0 à 15 ;
(III-A-c) un fragment selon la Formule 16B : 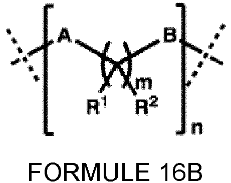 dans laquelle
R¹ et R², à chaque occurrence, sont choisis indépendamment parmi un hydrogène, halogène, CN, OH, NH₂ et alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ ;
A et B, à chaque occurrence, sont choisis indépendamment parmi rien, CO, CO₂, C(O)NR³, C(S)NR³, O, S, SO, SO₂, SO₂NR³, NR³, NR³CO, NR³CONR⁴, NR³C(S) et alkyle en C₁ à C₈ éventuellement substitué, alcoxy en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, alcényle en C₂ à C₈ éventuellement substitué, alcynyle en C₂ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué, hétéroaryle éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué et spirohétérocyclyle en C₃ à C₁₃ éventuellement substitué ; dans lesquels
R³ et R⁴ sont choisis indépendamment parmi un hydrogène et un alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué ou alkylamino en C₁ à C₈-alkyle en C₁ à C₈ ;
chaque m va de 0 à 15 ; et
n va de 0 à 15 ;
(III-A-d) un fragment selon la Formule 16C : 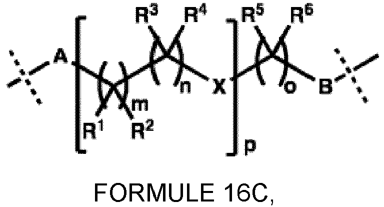 dans laquelle
X est choisi parmi O, NH et NR⁷ ;
R¹, R², R³, R⁴, R⁵ et R⁶, à chaque occurrence, sont choisis indépendamment parmi un hydrogène, halogène, CN, OH, NH₂, alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
A et B, à chaque occurrence, sont choisis indépendamment parmi rien, CO, NH, NH-CO, CO-NH, CH₂-NH-CO, CH₂-CO-NH, NH-CO-CH₂, CO-NH-CH₂, CH₂-NH-CH₂-CO-NH, CH₂-NH-CH₂-NH-CO, -CO-NH, CO-NH- CH₂-NH-CH₂, CH₂-NH-CH₂, CO₂, C(O)NR⁷, C(S)NR⁷, O, S, SO, SO₂, SO₂NR⁷, NR⁷, NR⁷CO, NR⁷CONR⁸, NR⁷C(S), alkyle en C₁ à C₈ éventuellement substitué, alcoxy en C₁ à C₈ éventuellement substitué, alcoxy en C₁ à C₈-alkyle en C₁ à C₈ éventuellement substitué, haloalkyle en C₁ à C₈ éventuellement substitué, hydroxyalkyle en C₁ à C₈ éventuellement substitué, alcényle en C₂ à C₈ éventuellement substitué, alcynyle en C₂ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, aryle éventuellement substitué, hétéroaryle éventuellement substitué, cycloalkyle fusionné en C₃ à C₁₃ éventuellement substitué, hétérocyclyle fusionné en C₃ à C₁₃ éventuellement substitué, cycloalkyle ponté en C₃ à C₁₃ éventuellement substitué, hétérocyclyle ponté en C₃ à C₁₃ éventuellement substitué, spirocycloalkyle en C₃ à C₁₃ éventuellement substitué et spirohétérocyclyle en C₃ à C₁₃ éventuellement substitué ; dans lesquels
R⁷ et R⁸ sont choisis indépendamment parmi un hydrogène, alkyle en C₁ à C₈ éventuellement substitué, cycloalkyle de 3 à 8 chaînons éventuellement substitué, cycloalcoxy en C₃ à C₈ éventuellement substitué, hétérocyclyle de 3 à 8 chaînons éventuellement substitué, alcoxy en C₁ à C₈ éventuellement substitué, alcoxyalkyle en C₁ à C₈ éventuellement substitué, haloalkyle en C₁ à C₈ éventuellement substitué, hydroxyalkyle en C₁ à C₈ éventuellement substitué, alkylamino en C₁ à C₈ éventuellement substitué et alkylamino en C₁ à C₈-alkyle en C₁ à C₈ éventuellement substitué ;
m, à chaque occurrence, va de 0 à 15 ;
n, à chaque occurrence, va de 0 à 15 ;
o va de 0 à 15 ; et
p va de 0 à 15 ;
et des sels pharmaceutiquement acceptables de ceux-ci ; ou
(III-B) un fragment choisi dans le groupe constitué par : un noyau choisi dans le groupe constitué par un noyau de 3 à 13 chaînons ; un noyau fusionné de 3 à 13 chaînons ; un noyau ponté de 3 à 13 chaînons ; et un noyau spiro de 3 à 13 chaînons ; et des sels pharmaceutiquement acceptables de ceux-ci ; ou
(III-C) un fragment choisi dans le groupe constitué par l'une parmi les Formules C1, C2, C3, C4 et C5 : 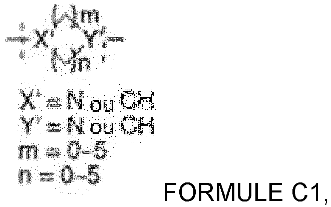 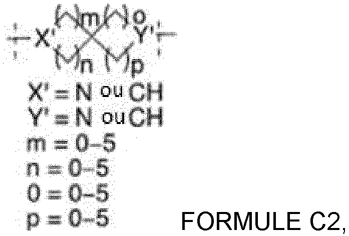 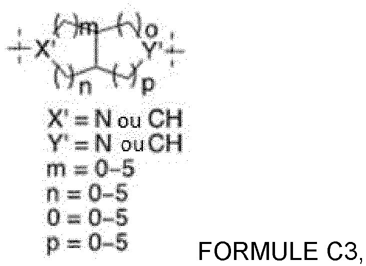 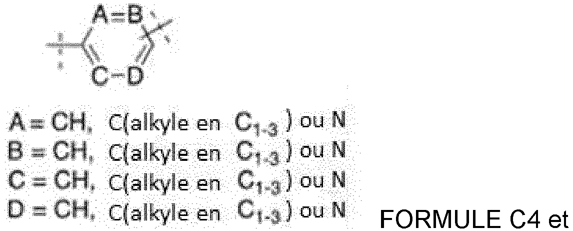 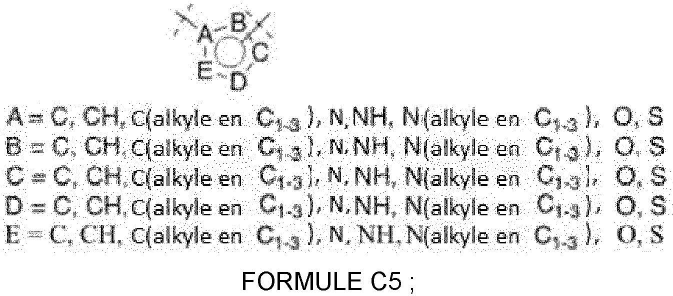 et des sels pharmaceutiquement acceptables de ceux-ci.

2. Composé selon la revendication 1, le composé étant choisi dans le groupe constitué par :
a. 2-(2,6-dioxopipéridin-3-yl)-4-((3-(4-(4-(5-(2-fluoro-6-méthoxyphényl)-1H-pyrazolo[4,3-d]pyrimidin-3-yl)phényl)pipérazin-1-yl)-3-oxopropyl)amino)isoindoline-1,3-dione (HC90-50) ; et
b. 2-(2,6-dioxopipéridin-3-yl)-4-((4-(4-(4-(5-(2-fluoro-6-méthoxyphényl)-1H-pyrazolo[4,3-d]pyrimidin-3-yl)phényl)pipérazin-1-yl)-4-oxobutyl)amino)isoindoline-1,3-dione (HC90-51), et
sels pharmaceutiquement acceptables de celui-ci.

3. Composé bivalent selon la revendication 1, dans lequel PI est : 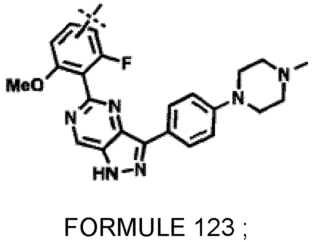 et
sels pharmaceutiquement acceptables de celui-ci.

4. Composé bivalent selon la revendication 1 (II-A)-(II-B), dans lequel EL est un fragment choisi dans le groupe constitué par : 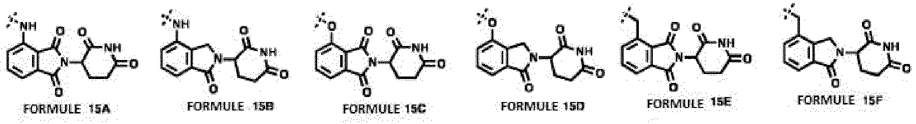 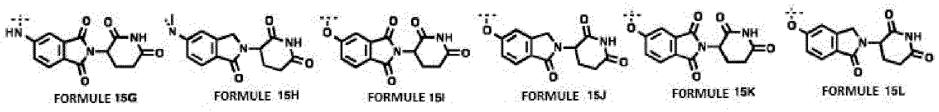 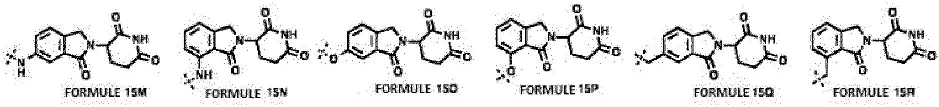 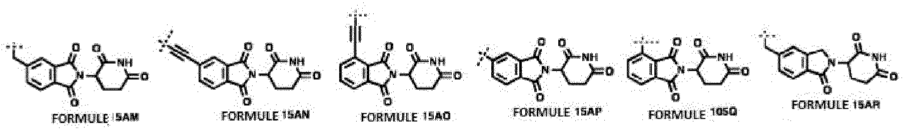 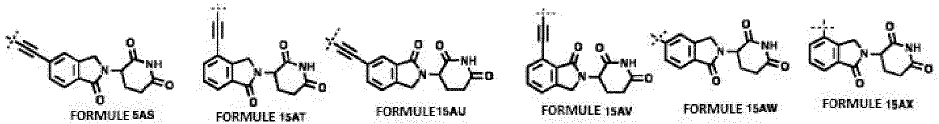 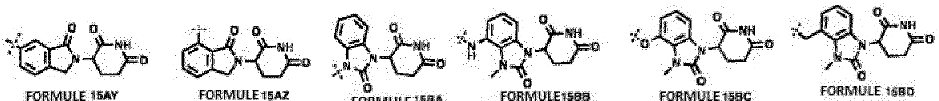 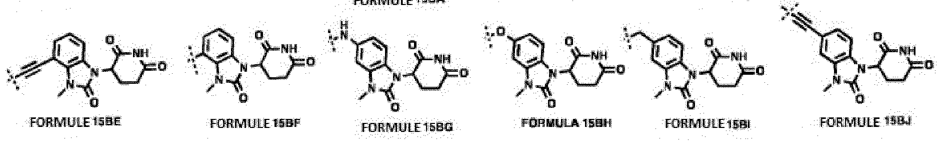 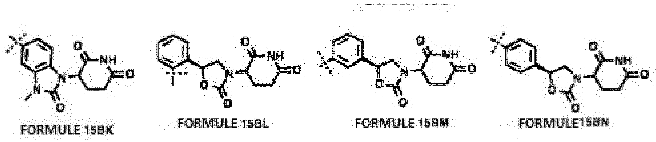 et sels pharmaceutiquement acceptables de celui-ci.

5. Composé bivalent selon la revendication 1, le composé étant choisi dans le groupe constitué par :
2-(2,6-dioxopipéridin-3-yl)-5-((2-(3-(4-(4-(5-(2-fluoro-6-méthoxyphényl)-1H-pyrazolo[4,3-d]pyrimidin-3-yl)phényl)pipérazin-1-yl)-3-oxopro-poxy)éthyl)amino)isoindoline-1,3-dione (HC90-33) ;
2-(2,6-dioxopipéridin-3-yl)-5-((2-(2-(3-(4-(4-(5-(2-fluoro-6-méthoxyphényl)-1H-pyrazolo[4,3-d]pyrimidin-3-yl)phényl)pipérazin-1-yl)-3-oxopro-poxy)éthoxy)éthyl)amino)isoindoline-1,3-dione (HC90-34) ;
2-(2,6-dioxopipéridin-3-yl)-5-((2(-2-(2-(3-(4-(4-(5-(2-fluoro-6-méthoxyphé-nyl)-1H-pyrazolo[4,3-d]pyrimidin-3-yl)phényl)pipérazin-1-yl)-3-oxopro-poxy)éthoxy)éthoxy)éthyl)amino)isoindoline-1,3-dione (HC90-35) ;
2-(2,6-dioxopipéridin-3-yl)-5-((15-(4-(4-(5-(2-fluoro-6-méthoxyphényl)-1H-pyrazolo[4,3-d]pyrimidin-3-yl)phényl)pipérazin-1-yl)-15-oxo-3,6,9,12-tétraoxapen-tadécyl)amino)isoindoline-1,3-dione (HC90-36) ;
2-(2,6-dioxopipéridin-3-yl)-5-((18-(4-(4-(5-(2-fluoro-6-méthoxyphényl)-1H-pyrazolo[4,3-d]pyrimidin-3-yl)phényl)pipérazin-1-yl)-18-oxo-3, 6, 9,12, 15-pen-taoxaoctadécyl)amino)isoindoline-1,3-dione (HC90-37) ;
2-(2,6-dioxopipéridin-3-yl)-5-((2-(4-(4-(5-(2-fluoro-6-méthoxyphényl)-1H-pyrazolo[4,3-d]pyrimidin-3-yl)phényl)pipérazin-1-yl)-2-oxoéthyl)amino)isoindoline-1,3-dione (HC90-41) ;
2-(2,6-dioxopipéridin-3-yl)-5-((3-(4-(4-(5-(2-fluoro-6-méthoxyphényl)-1H-pyrazolo[4,3-d]pyrimidin-3-yl)phényl)pipérazin-1-yl)-3-oxopropyl)amino)isoindoline-1,3-dione (HC90-42) ;
2-(2,6-dioxopipéridin-3-yl)-5-((4-(4-(4-(5-(2-fluoro-6-méthoxyphényl)-1H-pyrazolo[4,3-d]pyrimidin-3-yl)phényl)pipérazin-1-yl)-4-oxobutyl)amino)isoindoline-1,3-dione (HC90-43) ;
2-(2,6-dioxopipéridin-3-yl)-5-((5-(4-(4-(5-(2-fluoro-6-méthoxyphényl)-1H-pyrazolo[4,3-d]pyrimidin-3-yl)phényl)pipérazin-1-yl)-5-oxopentyl)amino)isoindoline-1,3-dione (HC90-44) ;
2-(2,6-dioxopipéridin-3-yl)-5-((6-(4-(4-(5-(2-fluoro-6-méthoxyphényl)-1H-pyrazolo[4,3-d]pyrimidin-3-yl)phényl)pipérazin-1-yl)-6-oxohexyl)amino)isoindoline-1,3-dione (HC90-45) ;
2-(2,6-dioxopipéridin-3-yl)-5-((7-(4-(4-(5-(2-fluoro-6-méthoxyphényl)-1H-pyrazolo[4,3-d]pyrimidin-3-yl)phényl)pipérazin-1-yl)-7-oxoheptyl)amino)isoindoline-1,3-dione (HC90-46) ;
2-(2,6-dioxopipéridin-3-yl)-5-((8-(4-(4-(5-(2-fluoro-6-méthoxyphényl)-1H-pyrazolo[4,3-d]pyrimidin-3-yl)phényl)pipérazin-1-yl)-8-oxooctyl)amino)isoindoline-1,3-dione (HC90-47) ;
2-(2,6-dioxopipéridin-3-yl)-4-((2-(4-(4-(5-(2-fluoro-6-méthoxyphényl)-1H-pyrazolo[4,3-d]pyrimidin-3-yl)phényl)pipérazin-1-yl)-2-oxoéthyl)amino)isoindoline-1,3-dione (HC90-49) ;
2-(2,6-dioxopipéridin-3-yl)-4-((3-(4-(4-(5-(2-fluoro-6-méthoxyphényl)-1H-pyrazolo[4,3-d]pyrimidin-3-yl)phényl)pipérazin-1-yl)-3-oxopropyl)amino)isoindoline-1,3-dione (HC90-50) ;
2-(2,6-dioxopipéridin-3-yl)-4-((4-(4-(4-(5-(2-fluoro-6-méthoxyphényl)-1H-pyrazolo[4,3-d]pyrimidin-3-yl)phényl)pipérazin-1-yl)-4-oxobutyl)amino)isoindoline-1,3-dione (HC90-51) ;
2-(2,6-dioxopipéridin-3-yl)-4-((5-(4-(4-(5-(2-fluoro-6-méthoxyphényl)-1H-pyrazolo[4,3-d]pyrimidin-3-yl)phényl)pipérazin-1-yl)-5-oxopentyl)amino)isoindoline-1,3-dione (HC90-52) ;
2-(2,6-dioxopipéridin-3-yl)-4-((6-(4-(4-(5-(2-fluoro-6-méthoxyphényl)-1H-pyrazolo[4,3-d]pyrimidin-3-yl)phényl)pipérazin-1-yl)-6-oxohexyl)amino)isoindoline-1,3-dione (HC90-53) ;
2-(2,6-dioxopipéridin-3-yl)-4-((7-(4-(4-(5-(2-fluoro-6-méthoxyphényl)-1H-pyrazolo[4,3-d]pyrimidin-3-yl)phényl)pipérazin-1-yl)-7-oxoheptyl)amino)isoindoline-1,3-dione (HC90-54) ;
2-(2,6-dioxopipéridin-3-yl)-4-((2-(3-(4-(4-(5-(2-fluoro-6-méthoxyphényl)-1H-pyrazolo[4,3-d]pyrimidin-3-yl)phényl)pipérazin-1-yl)-3-oxopro-poxy)éthyl)amino)isoindoline-1,3-dione (HC90-55) ;
2-(2,6-dioxopipéridin-3-yl)-4-((2-(2-(3-(4-(4-(5-(2-fluoro-6-méthoxyphényl)-1H-pyrazolo[4,3-d]pyrimidin-3-yl)phényl)pipérazin-1-yl)-3-oxopro-poxy)éthoxy)éthyl)amino)isoindoline-1,3-dione (HC90-56) ;
2-(2,6-dioxopipéridin-3-yl)-4-((2-(2-(2-(3-(4-(4-(5-(2-fluoro-6-méthoxyphé-nyl)-1H-pyrazolo[4,3-d]pyrimidin-3-yl)phényl)pipérazin-1-yl)-3-oxopro-poxy)éthoxy)éthoxy)éthyl)amino)isoindoline-1,3-dione (HC90-57) ;
2-(2,6-dioxopipéridin-3-yl)-4-((15-(4-(4-(5-(2-fluoro-6-méthoxyphényl)-1H-pyrazolo[4,3-d]pyrimidin-3-yl)phényl)pipérazin-1-yl)-15-oxo-3,6,9,12-tétraoxapen-tadécyl)amino)isoindoline-1,3-dione (HC90-58) ;
2-(2,6-dioxopipéridin-3-yl)-4-((18-(4-(4-(5-(2-fluoro-6-méthoxyphényl)-1H-pyrazolo[4,3-d]pyrimidin-3-yl)phényl)pipérazin-1-yl)-18-oxo-3,6,9,12,15-pen-taoxaoctadécyl)amino)isoindoline-1,3-dione (HC90-59) ;
(2S,4R)-1-((S)-2-(3-(3-(4-(4-(5-(2-fluoro-6-méthoxyphényl)-1H-pyra-zolo[4,3-d]pyrimidin-3-yl)phényl)pipérazin-1-yl)-3-oxopropoxy)propanamido)-3,3-diméthylbutanoyl)-4-hydroxy-N-(4-(4-méthylthiazol-5-yl)benzyl)pyrrolidine-2-car-boxamide (HC90-61) ;
(2S,4R)-1-((S)-2-(tert-butyl)-19-(4-(4-(5-(2-fluoro-6-méthoxyphényl)-1H-py-razolo[4,3-d]pyrimidin-3-yl)phényl)pipérazin-1-yl)-4,19-dioxo-7,10,13,16-tétraoxa-3-azanonadécanoyl)-4-hydroxy-N-(4-(4-méthylthiazol-5-yl)benzyl)pyrrolidine-2-car-boxamide (HC90-66) ;
(2S,4R)-1-((S)-2-(4-(4-(4-(5-(2-fluoro-6-méthoxyphényl)-1H-pyrazolo[4,3-d]pyrimidin-3-yl)phényl)pipérazin-1-yl)-4-oxobutanamido)-3,3-diméthylbutanoyl)-4-hydroxy-N-(4-(4-méthylthiazol-5-yl)benzyl)pyrrolidine-2-carboxamide (HC90-69) ;
(2S,4R)-1-((S)-2-(5-(4-(4-(5-(2-fluoro-6-méthoxyphényl)-1H-pyrazolo[4,3-d]pyrimidin-3-yl)phényl)pipérazin-1-yl)-5-oxopentanamido)-3,3-diméthylbutanoyl)-4-hydroxy-N-(4-(4-méthylthiazol-5-yl)benzyl)pyrrolidine-2-carboxamide (HC90-70) ;
(2S,4R)-1-((S)-2-(6-(4-(4-(5-(2-fluoro-6-méthoxyphényl)-1H-pyrazolo[4,3-d]pyrimidin-3-yl)phényl)pipérazin-1-yl)-6-oxohexanamido)-3,3-diméthylbutanoyl)-4-hydroxy-N-(4-(4-méthylthiazol-5-yl)benzyl)pyrrolidine-2-carboxamide (HC90-71) ;
(2S,4R)-1-((S)-2-(7-(4-(4-(5-(2-fluoro-6-méthoxyphényl)-1H-pyrazolo[4,3-d]pyrimidin-3-yl)phényl)pipérazin-1-yl)-7-oxoheptanamido)-3,3-diméthylbutanoyl)-4-hydroxy-N-(4-(4-méthylthiazol-5-yl)benzyl)pyrrolidine-2-carboxamide (HC90-72) ;
(2S,4R)-1-((S)-2-(8-(4-(4-(5-(2-fluoro-6-méthoxyphényl)-1H-pyrazolo[4,3-d]pyrimidin-3-yl)phényl)pipérazin-1-yl)-8-oxooctanamido)-3,3-diméthylbutanoyl)-4-hydroxy-N-(4-(4-méthylthiazol-5-yl)benzyl)pyrrolidine-2-carboxamide (HC90-73) ;
(2S,4R)-1-((S)-2-(10-(4-(4-(5-(2-fluoro-6-méthoxyphényl)-1H-pyrazolo[4,3-d]pyrimidin-3-yl)phényl)pipérazin-1-yl)-10-oxodécanamido)-3,3-diméthylbutanoyl)-4-hydroxy-N-(4-(4-méthylthiazol-5-yl)benzyl)pyrrolidine-2-carboxamide (HC90-74) ;
(2S,4R)-1-((S)-2-(9-(4-(4-(5-(2-fluoro-6-méthoxyphényl)-1H-pyrazolo[4,3-d]pyrimidin-3-yl)phényl)pipérazin-1-yl)-9-oxononanamido)-3,3-diméthylbutanoyl)-4-hydroxy-N-(4-(4-méthylthiazol-5-yl)benzyl)pyrrolidine-2-carboxamide (HC90-84) ;
(2S,4R)-1-((S)-2-(11-(4-(4-(5-(2-fluoro-6-méthoxyphényl)-1H-pyrazolo[4,3-d]pyrimidin-3-yl)phényl)pipérazin-1-yl)-11-oxoundécanamido)-3,3-diméthylbuta-noyl)-4-hydroxy-N-(4-(4-méthylthiazol-5-yl)benzyl)pyrrolidine-2-carboxam ide (HC90-85) ;
N-(2-((2-(2,6-dioxopipéridin-3-yl)-1,3-dioxoisoindolin-5-yl)amino)éthyl)-2-(4-(4-(5-(2-fluoro-6-méthoxyphényl)-1H-pyrazolo[4,3-d]pyrimidin-3-yl)phényl)pipé-razin-1-yl)acétamide (HC90-86) ;
N-(3-((2-(2,6-dioxopipéridin-3-yl)-1,3-dioxoisoindolin-5-yl)amino)propyl)-2-(4-(4-(5-(2-fluoro-6-méthoxyphényl)-1H-pyrazolo[4,3-d]pyrimidin-3-yl)phényl)pipé-razin-1-yl)acétamide (HC90-87) ;
N-(4-((2-(2,6-dioxopipéridin-3-yl)-1,3-dioxoisoindolin-5-yl)amino)butyl)-2-(4-(4-(5-(2-fluoro-6-méthoxyphényl)-1H-pyrazolo[4,3-d]pyrimidin-3-yl)phényl)pipé-razin-1-yl)acétamide (HC90-88) ;
N-(5-((2-(2,6-dioxopipéridin-3-yl)-1,3-dioxoisoindolin-5-yl)amino)pentyl)-2-(4-(4-(5-(2-fluoro-6-méthoxyphényl)-1H-pyrazolo[4,3-d]pyrimidin-3-yl)phényl)pipé-razin-1-yl)acétamide (HC90-89) ;
N-(6-((2-(2,6-dioxopipéridin-3-yl)-1,3-dioxoisoindolin-5-yl)amino)hexyl)-2-(4-(4-(5-(2-fluoro-6-méthoxyphényl)-1H-pyrazolo[4,3-d]pyrimidin-3-yl)phényl)pipé-razin-1-yl)acétamide (HC90-90) ;
N-(7-((2-(2,6-dioxopipéridin-3-yl)-1,3-dioxoisoindolin-5-yl)amino)heptyl)-2-(4-(4-(5-(2-fluoro-6-méthoxyphényl)-1H-pyrazolo[4,3-d]pyrimidin-3-yl)phényl)pipé-razin-1-yl)acétamide (HC90-91) ;
N-(8-((2-(2,6-dioxopipéridin-3-yl)-1,3-dioxoisoindolin-5-yl)amino)octyl)-2-(4-(4-(5-(2-fluoro-6-méthoxyphényl)-1H-pyrazolo[4,3-d]pyrimidin-3-yl)phényl)pipé-razin-1-yl)acétamide (HC90-92) ;
N-(2-(2-((2-(2,6-dioxopipéridin-3-yl)-1,3-dioxoisoindolin-5-yl)amino)éthoxy)éthyl)-2-(4-(4-(5-(2-fluoro-6-méthoxyphényl)-1H-pyrazolo[4,3-d]pyrimidin-3-yl)phényl)pipérazin-1-yl)acétamide (HC90-93) ;
N-(2-(2-(2-((2-(2,6-dioxopipéridin-3-yl)-1,3-dioxoisoindolin-5-yl)amino)éthoxy)éthoxy)éthyl)-2-(4-(4-(5-(2-fluoro-6-méthoxyphényl)-1H-pyra-zolo[4,3-d]pyrimidin-3-yl)phényl)pipérazin-1-yl)acétamide (HC90-94) ;
N-(2-(2-(2-(2-((2-(2,6-dioxopipéridin-3-yl)-1,3-dioxoisoindolin-5-yl)amino)éthoxy)éthoxy)éthoxy)éthyl)-2-(4-(4-(5-(2-fluoro-6-méthoxyphényl)-1H-pyrazolo[4,3-d]pyrimidin-3-yl)phényl)pipérazin-1-yl)acétamide (HC90-95) ;
N-(14-((2-(2,6-dioxopipéridin-3-yl)-1,3-dioxoisoindolin-5-yl)amino)-3,6,9,12-tétraoxatétradécyl)-2-(4-(4-(5-(2-fluoro-6-méthoxyphényl)-1H-pyra-zolo[4,3-d]pyrimidin-3-yl)phényl)pipérazin-1-yl)acétamide (HC90-96) ;
N-(17-((2-(2,6-dioxopipéridin-3-yl)-1,3-dioxoisoindolin-5-yl)amino)-3,6,9,12,15-pentaoxaheptadécyl)-2-(4-(4-(5-(2-fluoro-6-méthoxyphényl)-1H-pyra-zolo[4,3-d]pyrimidin-3-yl)phényl)pipérazin-1-yl)acétamide (HC90-97) ;
N-(2-((2-(2,6-dioxopipéridin-3-yl)-1,3-dioxoisoindolin-4-yl)amino)éthyl)-2-(4-(4-(5-(2-fluoro-6-méthoxyphényl)-1H-pyrazolo[4,3-d]pyrimidin-3-yl)phényl)pipé-razin-1-yl)acétamide (HC90-102) ;
N-(3-((2-(2,6-dioxopipéridin-3-yl)-1,3-dioxoisoindolin-4-yl)amino)propyl)-2-(4-(4-(5-(2-fluoro-6-méthoxyphényl)-1H-pyrazolo[4,3-d]pyrimidin-3-yl)phényl)pipé-razin-1-yl)acétamide (HC90-103) ;
N-(4-((2-(2,6-dioxopipéridin-3-yl)-1,3-dioxoisoindolin-4-yl)amino)butyl)-2-(4-(4-(5-(2-fluoro-6-méthoxyphényl)-1H-pyrazolo[4,3-d]pyrimidin-3-yl)phényl)pipérazin-1-yl)acétamide (HC90-104) ;
N-(5-((2-(2,6-dioxopipéridin-3-yl)-1,3-dioxoisoindolin-4-yl)amino)pentyl)-2-(4-(4-(5-(2-fluoro-6-méthoxyphényl)-1H-pyrazolo[4,3-d]pyrimidin-3-yl)phényl)pipé-razin-1-yl)acétamide (HC90-105) ;
N-(6-((2-(2,6-dioxopipéridin-3-yl)-1,3-dioxoisoindolin-4-yl)amino)hexyl)-2-(4-(4-(5-(2-fluoro-6-méthoxyphényl)-1H-pyrazolo[4,3-d]pyrimidin-3-yl)phényl)pipé-razin-1-yl)acétamide (HC90-106) ;
N-(7-((2-(2,6-dioxopipéridin-3-yl)-1,3-dioxoisoindolin-4-yl)amino)heptyl)-2-(4-(4-(5-(2-fluoro-6-méthoxyphényl)-1H-pyrazolo[4,3-d]pyrimidin-3-yl)phényl)pipé-razin-1-yl)acétamide (HC90-107) ;
N-(8-((2-(2,6-dioxopipéridin-3-yl)-1,3-dioxoisoindolin-4-yl)amino)octyl)-2-(4-(4-(5-(2-fluoro-6-méthoxyphényl)-1H-pyrazolo[4,3-d]pyrimidin-3-yl)phényl)pipé-razin-1-yl)acétamide (HC90-108) ;
N-(2-(2-((2-(2,6-dioxopipéridin-3-yl)-1,3-dioxoisoindolin-4-yl)amino)éthoxy)éthyl)-2-(4-(4-(5-(2-fluoro-6-méthoxyphényl)-1H-pyrazolo[4,3-d]pyrimidin-3-yl)phényl)pipérazin-1-yl)acétamide (HC90-109) ;
N-(2-(2-(2-((2-(2,6-dioxopipéridin-3-yl)-1,3-dioxoisoindolin-4-yl)amino)éthoxy)éthoxy)éthyl)-2-(4-(4-(5-(2-fluoro-6-méthoxyphényl)-1H-pyra-zolo[4,3-d]pyrimidin-3-yl)phényl)pipérazin-1-yl)acétamide (HC90-110) ;
N-(2-(2-(2-(2-((2-(2,6-dioxopipéridin-3-yl)-1,3-dioxoisoindolin-4-yl)amino)éthoxy)éthoxy)éthoxy)éthyl)-2-(4-(4-(5-(2-fluoro-6-méthoxyphényl)-1H-pyrazolo[4,3-d]pyrimidin-3-yl)phényl)pipérazin-1-yl)acétamide (HC90-111) ;
N-(14-((2-(2,6-dioxopipéridin-3-yl)-1,3-dioxoisoindolin-4-yl)amino)-3,6,9,12-tétraoxatétradécyl)-2-(4-(4-(5-(2-fluoro-6-méthoxyphényl)-1H-pyra-zolo[4,3-d]pyrimidin-3-yl)phényl)pipérazin-1-yl)acétamide (HC90-112) ;
N-(17-((2-(2,6-dioxopipéridin-3-yl)-1,3-dioxoisoindolin-4-yl)amino)-3,6,9,12,15-pentaoxaheptadécyl)-2-(4-(4-(5-(2-fluoro-6-méthoxyphényl)-1H-pyra-zolo[4,3-d]pyrimidin-3-yl)phényl)pipérazin-1-yl)acétamide (HC90-113) ;
2-(2,6-dioxopipéridin-3-yl)-4-((8-(4-(4-(5-(2-fluoro-6-méthoxyphényl)-1H-pyrazolo[4,3-d]pyrimidin-3-yl)phényl)pipérazin-1-yl)-8-oxooctyl)amino)isoindoline-1,3-dione (HC90-117) ;
(2S,4R)-1-((S)-2-(2-(2-(2-(4-(4-(5-(2-fluoro-6-méthoxyphényl)-1H-pyra-zolo[4,3-d]pyrimidin-3-yl)phényl)pipérazin-1-yl)acétamido)éthoxy)acétamido)-3,3-diméthylbutanoyl)-4-hydroxy-N-(4-(4-méthylthiazol-5-yl)benzyl)pyrrolidine-2-car-boxamide (HC90-119) ;
(2S,4R)-1-((S)-2-(3-(2-(2-(4-(4-(5-(2-fluoro-6-méthoxyphényl)-1H-pyra-zolo[4,3-d]pyrimidin-3-yl)phényl)pipérazin-1-yl)acétamido)éthoxy)propanamido)-3,3-diméthylbutanoyl)-4-hydroxy-N-(4-(4-méthylthiazol-5-yl)benzyl)pyrrolidine-2-carboxamide (HC90-120) ;
(2S,4R)-1-((S)-2-(tert-butyl)-14-(4-(4-(5-(2-fluoro-6-méthoxyphényl)-1H-py-razolo[4,3-d]pyrimidin-3-yl)phényl)pipérazin-1-yl)-4,13-dioxo-6,9-dioxa-3,12-dia-zatétradécanoyl)-4-hydroxy-N-(4-(4-méthylthiazol-5-yl)benzyl)pyrrolidine-2-car-boxamide (HC90-121) ;
(2S,4R)-1-((S)-14-(tert-butyl)-1-(4-(4-(5-(2-fluoro-6-méthoxyphényl)-1H-py-razolo[4,3-d]pyrimidin-3-yl)phényl)pipérazin-1-yl)-2,12-dioxo-6,9-dioxa-3,13-diaza-pentadécan-15-oyl)-4-hydroxy-N-(4-(4-méthylthiazol-5-yl)benzyl)pyrrolidine-2-car-boxamide (HC90-122) ;
(2S,4R)-1-((S)-2-(tert-butyl)-17-(4-(4-(5-(2-fluoro-6-méthoxyphényl)-1H-py-razolo[4,3-d]pyrimidin-3-yl)phényl)pipérazin-1-yl)-4,16-dioxo-6, 9,12-trioxa-3,15-diazaheptadécanoyl)-4-hydroxy-N-(4-(4-méthylthiazol-5-yl)benzyl)pyrrolidine-2-carboxamide (HC90-123) ;
(2S,4R)-1-((S)-17-(tert-butyl)-1-(4-(4-(5-(2-fluoro-6-méthoxyphényl)-1H-py-razolo[4,3-d]pyrimidin-3-yl)phényl)pipérazin-1-yl)-2,15-dioxo-6,9,12-trioxa-3,16-diazaoctadécan-18-oyl)-4-hydroxy-N-(4-(4-méthylthiazol-5-yl)benzyl)pyrrolidine-2-carboxamide (HC90-124) ;
(2S,4R)-1-((S)-20-(tert-butyl)-1-(4-(4-(5-(2-fluoro-6-méthoxyphényl)-1H-py-razolo[4,3-d]pyrimidin-3-yl)phényl)pipérazin-1-yl)-2,18-dioxo-6,9,12,15-tétraoxa-3,19-diazahénicosan-21-oyl)-4-hydroxy-N-(4-(4-méthylthiazol-5-yl)benzyl)pyrroli-dine-2-carboxamide (HC90-125) ;
(2S,4R)-1-((S)-23-(tert-butyl)-1-(4-(4-(5-(2-fluoro-6-méthoxyphényl)-1H-py-razolo[4,3-d]pyrimidin-3-yl)phényl)pipérazin-1-yl)-2,21-dioxo-6,9,12,15,18-pen-taoxa-3,22-diazatétracosan-24-oyl)-4-hydroxy-N-(4-(4-méthylthiazol-5-yl)ben-zyl)pyrrolidine-2-carboxamide (HC90-126) ;
(2S,4R)-1-((S)-2-(2-(2-(4-(4-(5-(2-fluoro-6-méthoxyphényl)-1H-pyra-zolo[4,3-d]pyrimidin-3-yl)phényl)pipérazin-1-yl)acétamido)acétamido)-3,3-diméthyl-butanoyl)-4-hydroxy-N-(4-(4-méthylthiazol-5-yl)benzyl)pyrrolidine-2-carboxam ide (HC90-127) ;
(2S,4R)-1-((S)-2-(3-(2-(4-(4-(5-(2-fluoro-6-méthoxyphényl)-1H-pyra-zolo[4,3-d]pyrimidin-3-yl)phényl)pipérazin-1-yl)acétamido)propanamido)-3,3-dimé-thylbutanoyl)-4-hydroxy-N-(4-(4-méthylthiazol-5-yl)benzyl)pyrrolidine-2-carboxa-mide (HC90-128) ;
(2S,4R)-1-((S)-2-(4-(2-(4-(4-(5-(2-fluoro-6-méthoxyphényl)-1H-pyra-zolo[4,3-d]pyrimidin-3-yl)phényl)pipérazin-1-yl)acétamido)butanamido)-3,3-dimé-thylbutanoyl)-4-hydroxy-N-(4-(4-méthylthiazol-5-yl)benzyl)pyrrolidine-2-carboxa-mide (HC90-129) ;
(2S,4R)-1-((S)-2-(5-(2-(4-(4-(5-(2-fluoro-6-méthoxyphényl)-1H-pyra-zolo[4,3-d]pyrimidin-3-yl)phényl)pipérazin-1-yl)acétamido)pentanamido)-3,3-dimé-thylbutanoyl)-4-hydroxy-N-(4-(4-méthylthiazol-5-yl)benzyl)pyrrolidine-2-carboxa-mide (HC90-130) ;
(2S,4R)-1-((S)-2-(6-(2-(4-(4-(5-(2-fluoro-6-méthoxyphényl)-1H-pyra-zolo[4,3-d]pyrimidin-3-yl)phényl)pipérazin-1-yl)acétamido)hexanamido)-3,3-dimé-thylbutanoyl)-4-hydroxy-N-(4-(4-méthylthiazol-5-yl)benzyl)pyrrolidine-2-carboxa-mide (HC90-131) ;
(2S,4R)-1-((S)-2-(7-(2-(4-(4-(5-(2-fluoro-6-méthoxyphényl)-1H-pyra-zolo[4,3-d]pyrimidin-3-yl)phényl)pipérazin-1-yl)acétamido)heptanamido)-3,3-dimé-thylbutanoyl)-4-hydroxy-N-(4-(4-méthylthiazol-5-yl)benzyl)pyrrolidine-2-carboxa-mide (HC90-132) ;
(2S,4R)-1-((S)-2-(8-(2-(4-(4-(5-(2-fluoro-6-méthoxyphényl)-1H-pyra-zolo[4,3-d]pyrimidin-3-yl)phényl)pipérazin-1-yl)acétamido)octanamido)-3,3-dimé-thylbutanoyl)-4-hydroxy-N-(4-(4-méthylthiazol-5-yl)benzyl)pyrrolidine-2-carboxa-mide (HC90-133) ;
(2S,4R)-1-((S)-2-(9-(2-(4-(4-(5-(2-fluoro-6-méthoxyphényl)-1H-pyra-zolo[4,3-d]pyrimidin-3-yl)phényl)pipérazin-1-yl)acétamido)nonanamido)-3,3-dimé-thylbutanoyl)-4-hydroxy-N-(4-(4-méthylthiazol-5-yl)benzyl)pyrrolidine-2-carboxa-mide (HC90-134) ;
(2S,4R)-1-((S)-2-(10-(2-(4-(4-(5-(2-fluoro-6-méthoxyphényl)-1H-pyra-zolo[4,3-d]pyrimidin-3-yl)phényl)pipérazin-1-yl)acétamido)décanamido)-3,3-dimé-thylbutanoyl)-4-hydroxy-N-(4-(4-méthylthiazol-5-yl)benzyl)pyrrolidine-2-carboxa-mide (HC90-135) ;
(2S,4R)-1-((S)-2-(11-(2-(4-(4-(5-(2-fluoro-6-méthoxyphényl)-1H-pyra-zolo[4,3-d]pyrimidin-3-yl)phényl)pipérazin-1-yl)acétamido)undécanamido)-3,3-di-méthylbutanoyl)-4-hydroxy-N-(4-(4-méthylthiazol-5-yl)benzyl)pyrrolidine-2-car-boxamide (HC90-136) ;
(2S,4R)-1-((S)-2-(2-(2-(4-(4-(5-(2-fluoro-6-méthoxyphényl)-1H-pyra-zolo[4,3-d]pyrimidin-3-yl)phényl)pipérazin-1-yl)-2-oxoéthoxy)acétamido)-3,3-dimé-thylbutanoyl)-4-hydroxy-N-(4-(4-méthylthiazol-5-yl)benzyl)pyrrolidine-2-carboxa-mide (HC90-60) ;
(2S,4R)-1-((S)-2-(2-(2-(2-(4-(4-(5-(2-fluoro-6-méthoxyphényl)-1H-pyra-zolo[4,3-d]pyrimidin-3-yl)phényl)pipérazin-1-yl)-2-oxoéthoxy)éthoxy)acétamido)-3,3-diméthylbutanoyl)-4-hydroxy-N-(4-(4-méthylthiazol-5-yl)benzyl)pyrrolidine-2-carboxamide (HC90-62) ;
(2S,4R)-1-((S)-2-(3-(2-(3-(4-(4-(5-(2-fluoro-6-méthoxyphényl)-1H-pyra-zolo[4,3-d]pyrimidin-3-yl)phényl)pipérazin-1-yl)-3-oxopropoxy)éthoxy)propana-mido)-3,3-diméthylbutanoyl)-4-hydroxy-N-(4-(4-méthylthiazol-5-yl)benzyl)pyrroli-dine-2-carboxamide (HC90-63) ;
(2S,4R)-1-((S)-2-(tert-butyl)-14-(4-(4-(5-(2-fluoro-6-méthoxyphényl)-1H-py-razolo[4,3-d]pyrimidin-3-yl)phényl)pipérazin-1-yl)-4,14-dioxo-6,9,12-trioxa-3-azaté-tradécanoyl)-4-hydroxy-N-(4-(4-méthylthiazol-5-yl)benzyl)pyrrolidine-2-carboxa-mide (HC90-64) ;
(2S,4R)-1-((S)-2-(tert-butyl)-16-(4-(4-(5-(2-fluoro-6-méthoxyphényl)-1H-py-razolo[4,3-d]pyrimidin-3-yl)phényl)pipérazin-1-yl)-4,16-dioxo-7,10,13-trioxa-3-azahexadécanoyl)-4-hydroxy-N-(4-(4-méthylthiazol-5-yl)benzyl)pyrrolidine-2-car-boxamide (HC90-65) ;
(2S,4R)-1-((S)-2-(tert-butyl)-20-(4-(4-(5-(2-fluoro-6-méthoxyphényl)-1H-py-razolo[4,3-d]pyrimidin-3-yl)phényl)pipérazin-1-yl)-4,20-dioxo-6,9,12,15,18-pen-taoxa-3-azaicosanoyl)-4-hydroxy-N-(4-(4-méthylthiazol-5-yl)benzyl)pyrrolidine-2-carboxamide (HC90-67) ; et
(2S,4R)-1-((S)-2-(tert-butyl)-22-(4-(4-(5-(2-fluoro-6-méthoxyphényl)-1H-py-razolo[4,3-d]pyrimidin-3-yl)phényl)pipérazin-1-yl)-4,22-dioxo-7,10,13,16,19-pen-taoxa-3-azadocosanoyl)-4-hydroxy-N-(4-(4-méthylthiazol-5-yl)benzyl)pyrrolidine-2-carboxamide (HC90-68) ;
et sels pharmaceutiquement acceptables de celui-ci.

6. Composé bivalent selon l'une quelconque des revendications 1 à 5 destiné à être utilisé dans un procédé de traitement d'une maladie médiée par HPKI chez un sujet le nécessitant,
la maladie médiée par HPKI étant de préférence choisie dans le groupe constitué par un cancer, des infections chroniques qui entrainent une réponse immunitaire épuisée, une immunosuppression due à une infection, le déclin de la réponse immunitaire lié à l'âge, un déclin des fonctions cognitives lié à l'âge et l'infertilité, le cancer étant plus préférablement choisi dans le groupe constitué par des cancers du sein, des voies respiratoires, du cerveau, des organes reproducteurs, des voies digestives, des voies urinaires, des yeux, du foie, de la peau, de la tête et du cou, de la thyroïde, des parathyroïdes et leurs métastases à distance, des lymphomes, des sarcomes et des leucémies.

7. Composition pharmaceutique comportant le composé bivalent selon l'une quelconque des revendications 1 à 5 destinée à être utilisée dans un procédé de traitement d'une maladie médiée par HPKI chez un sujet le nécessitant.

8. Composé bivalent selon l'une quelconque des revendications 1 à 5 destiné à être utilisé dans un procédé de réduction de l'expression de HPK1 dans des cellules, le procédé comprenant:
l'isolement de cellules provenant du corps d'un sujet ;
le traitement des cellules au moyen du composé bivalent ; et
la réintroduction des cellules traitées dans le corps du sujet,
de préférence
(i) les cellules sont choisies dans le groupe constitué par des cellules de moelle osseuse autologues, des cellules souches de sang de cordon et des cellules souches de sang périphérique ou de moelle osseuse, ou
(ii) les cellules sont des cellules immunitaires choisies dans le groupe constitué par des cellules T, des cellules T génétiquement modifiées, des cellules T de récepteur d'antigène chimérique (CAR, *chimeric antigen receptor*), des lymphocytes d'infiltration tumorale, des cellules dendritiques, des macrophages, des mastocytes, des granulocytes (y compris des basophiles, des éosinophiles et des neutrophiles), des cellules T NK et des cellules B.

9. Composé bivalent selon l'une quelconque des revendications 1 à 5 destiné à être utilisé dans un procédé d'augmentation de la sécrétion d'interleukine-2 par les cellules T, le procédé comprenant:
l'isolement de cellules T provenant du corps d'un sujet ;
le traitement des cellules T au moyen du composé bivalent ; et
la réintroduction des cellules T dans le corps du sujet.

10. Composition pharmaceutique comportant le composé bivalent selon l'une quelconque des revendications 1 à 5 destinée à être utilisée dans un procédé de traitement d'un cancer chez un sujet le nécessitant, le procédé comprenant :
l'administration, à un sujet ayant un cancer, de la composition pharmaceutique et d'un véhicule pharmaceutiquement acceptable, et d'une composition pharmaceutique comportant un inhibiteur du point de contrôle et un véhicule pharmaceutiquement acceptable.

11. Composition pharmaceutique destinée à être utilisée selon la revendication 10, dans laquelle l'inhibiteur du point de contrôle est choisi dans le groupe constitué par un anti-PD-1, un anti-PD-L1 et un anti-CTLA-4.

12. Composition pharmaceutique destinée à être utilisée selon la revendication 11,
(i) dans laquelle l'inhibiteur du point de contrôle est un anti-PD-1, de préférence l'anti-PD-1 est choisi dans le groupe constitué par le pembrolizumab, le nivolumab et le cemiplimab, plus préférablement l'anti-PD-1 est choisi dans le groupe constitué par le pembrolizumab, le nivolumab et le cemiplimab et le cancer est choisi dans le groupe constitué par un mélanome, un cancer du poumon, un carcinome des cellules rénales, un lymphome de Hodgkin, un cancer de la tête et du cou, un cancer du côlon et un cancer du foie ;
(ii) dans laquelle l'inhibiteur du point de contrôle est un anti-PD-L1, de préférence l'anti-PD-L1 est choisi dans le groupe constitué par l'atezolizumab, l'avelumab et le durvalumab, plus préférablement l'anti-PD-L1 est choisi dans le groupe constitué par l'atezolizumab, l'avelumab et le durvalumab et le cancer est choisi dans le groupe constitué par un carcinome non à petites cellules, un carcinome du poumon, un myélome multiple, un cancer urothélial et un cancer de la tête et du cou ; ou
(iii) dans laquelle l'inhibiteur du point de contrôle est un anti-CDLA-4, de préférence l'anti-CDLA-4 est choisi dans le groupe constitué par le pembrolizumab, le nivolumab et le cemiplimab, plus préférablement l'anti-CDLA-4 est choisi dans le groupe constitué par le pembrolizumab, le nivolumab et le cemiplimab et le cancer est choisi dans le groupe constitué par un mélanome, un cancer du poumon, un carcinome des cellules rénales, un glioblastome, un carcinome hépatocellulaire, un lymphome à grandes cellules B, un lymphome de Hodgkin, un cancer de la tête et du cou, un cancer du côlon et un cancer du foie.

13. Composition pharmaceutique comportant le composé bivalent selon l'une quelconque des revendications 1 à 5 destinée à être utilisée dans un procédé de traitement d'un cancer présentant une expression élevée de la cyclooxygénase-2 chez un sujet le nécessitant, le procédé comprenant :
l'administration, à un sujet ayant un cancer présentant une expression élevée de la cyclooxygénase-2, de la composition pharmaceutique et d'un véhicule pharmaceutiquement acceptable,
le cancer étant de préférence choisi dans le groupe constitué par un cancer du côlon, un cancer du poumon, un sarcome et un cancer du sein.

14. Composition pharmaceutique destinée à être utilisée selon la revendication 10, dans laquelle les deux compositions sont administrées de manière simultanée ou séquentielle.
